(19) Europäisches Patentamt
European Patent Office
Office européen des brevets

(11) EP 2 615 115 A2

(12) EUROPEAN PATENT APPLICATION

(43) Date of publication:
**17.07.2013 Bulletin 2013/29**

(21) Application number: **13154451.2**

(22) Date of filing: **28.11.2008**

(51) Int Cl.:
*C07K 16/46* (2006.01) *A61K 39/395* (2006.01)
*A61P 35/00* (2006.01) *A61P 29/00* (2006.01)
*C12N 15/13* (2006.01) *C12N 5/10* (2006.01)
*C07K 16/22* (2006.01) *C07K 16/24* (2006.01)
*C07K 16/28* (2006.01) *C07K 16/32* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.11.2007 US 991449 P**
**12.02.2008 US 27858 P**
**21.04.2008 US 46572 P**
**16.07.2008 US 81191 P**
**29.07.2008 US 84431 P**

(62) Document number(s) of the earlier application(s) in accordance with Art. 76 EPC:
**08854122.2 / 2 222 709**

(71) Applicant: **Glaxo Group Limited**
**Brentford, Middlesex TW8 9GS (GB)**

(72) Inventors:
- **Ashman, Claire**
**Stevenage**
**Hertfordshire SG1 2NY (GB)**
- **Batuwangala, Thil**
**Cambridge**
**Cambridgeshire CB4 0WG (GB)**
- **Burden, Michael Neil**
**Stevenage**
**Hertfordshire SG1 2NY (GB)**
- **Clegg, Stephanie Jane**
**Stevenage**
**Hertfordshire SG1 2NY (GB)**
- **De Wildt, Rudolf Maria**
**Cambridge**
**Cambridgeshire CB4 0WG (GB)**
- **Ellis, Jonathan Henry**
**Stevenage**
**Cambridgeshire SG1 2NY (GB)**
- **Hamblin, Paul Andrew**
**Stevenage**
**Cambridgeshire SG1 2NY (GB)**
- **Hussain, Farhana**
**Stevenage**
**Cambridgeshire SG1 2NY (GB)**
- **Jespers, Laurent**
**Cambridge**
**Cambridgeshire CB4 0WG (GB)**
- **Lewis, Alan**
**Stevenage**
**Hertfordshire SG1 2NY (GB)**
- **Orecchia, Martin Anibal**
**Stevenage**
**Hertfordshire SG1 2NY (GB)**
- **Shah, Radha**
**Stevenage**
**Hertfordshire SG1 2NY (GB)**
- **Steward, Michael**
**Cambridge**
**Cambridgeshire CB4 0WG (GB)**

(74) Representative: **Fowler, Gavin James**
**GlaxoSmithKline**
**Global Patents CN925.1**
**980 Great West Road**
**Brentford, Middlesex TW8 9GS (GB)**

Remarks:
•This application was filed on 07-02-2013 as a divisional application to the application mentioned under INID code 62.
•Claims filed after the date of filing of the application (Rule 68(4) EPC).

# (54) ANTIGEN-BINDING CONSTRUCTS

(57) The invention relates to antigen-binding constructs comprising a protein scaffold which are linked to one or more epitope-binding domains wherein the antigen-binding construct has at least two antigen binding sites at least one of which is from an epitope binding domain and at least one of which is from a paired VH/VL domain, methods of making such constructs and uses thereof.

EP 2 615 115 A2

## Description

## Background

Antibodies are well known for use in therapeutic applications.

Antibodies are heteromultimeric glycoproteins comprising at least two heavy and two light chains. Aside from IgM, intact antibodies are usually heterotetrameric glycoproteins of approximately 150Kda, composed of two identical light (L) chains and two identical heavy (H) chains. Typically, each light chain is linked to a heavy chain by one covalent disulfide bond while the number of disulfide linkages between the heavy chains of different immunoglobulin isotypes varies. Each heavy and light chain also has intrachain disulfide bridges. Each heavy chain has at one end a variable domain (VH) followed by a number of constant regions. Each light chain has a variable domain (VL) and a constant region at its other end; the constant region of the light chain is aligned with the first constant region of the heavy chain and the light chain variable domain is aligned with the variable domain of the heavy chain. The light chains of antibodies from most vertebrate species can be assigned to one of two types called Kappa and Lambda based on the amino acid sequence of the constant region. Depending on the amino acid sequence of the constant region of their heavy chains, human antibodies can be assigned to five different classes, IgA, IgD, IgE, IgG and IgM. IgG and IgA can be further subdivided into subclasses, IgG1, IgG2, IgG3 and IgG4; and IgA1 and IgA2. Species variants exist with mouse and rat having at least IgG2a, IgG2b. The variable domain of the antibody confers binding specificity upon the antibody with certain regions displaying particular variability called complementarity determining regions (CDRs). The more conserved portions of the variable region are called Framework regions (FR). The variable domains of intact heavy and light chains each comprise four FR connected by three CDRs. The CDRs in each chain are held together in close proximity by the FR regions and with the CDRs from the other chain contribute to the formation of the antigen binding site of antibodies. The constant regions are not directly involved in the binding of the antibody to the antigen but exhibit various effector functions such as participation in antibody dependent cell-mediated cytotoxicity (ADCC), phagocytosis via binding to Fcγ receptor, half-life/clearance rate via neonatal Fc receptor (FcRn) and complement dependent cytotoxicity via the C1q component of the complement cascade.

The nature of the structure of an IgG antibody is such that there are two antigen-binding sites, both of which are specific for the same epitope. They are therefore, monospecific.

A bispecific antibody is an antibody having binding specificities for at least two different epitopes. Methods of making such antibodies are known in the art. Traditionally, the recombinant production of bispecific antibodies is based on the coexpression of two immunoglobulin H chain-L chain pairs, where the two H chains have different binding specificities see Millstein et al, Nature 305 537-539 (1983), WO93/08829 and Traunecker et al EMBO, 10, 1991, 3655-3659. Because of the random assortment of H and L chains, a potential mixture of ten different antibody structures are produced of which only one has the desired binding specificity. An alternative approach involves fusing the variable domains with the desired binding specificities to heavy chain constant region comprising at least part of the hinge region, CH2 and CH3 regions. It is preferred to have the CH1 region containing the site necessary for light chain binding present in at least one of the fusions. DNA encoding these fusions, and if desired the L chain are inserted into separate expression vectors and are then cotransfected into a suitable host organism. It is possible though to insert the coding sequences for two or all three chains into one expression vector. In one approach, a bispecific antibody is composed of a H chain with a first binding specificity in one arm and a H-L chain pair, providing a second binding specificity in the other arm, see WO94/04690. Also see Suresh et al Methods in Enzymology 121, 210, 1986. Other approaches include antibody molecules which comprise single domain binding sites which is set out in WO2007/095338.

## Summary of invention

The present invention relates to an antigen-binding construct comprising a protein scaffold which is linked to one or more epitope-binding domains wherein the antigen-binding construct has at least two antigen binding sites at least one of which is from an epitope binding domain and at least one of which is from a paired VH/VL domain.

The invention further relates to antigen-binding constructs comprising at least one homodimer comprising two or more structures of formula I:

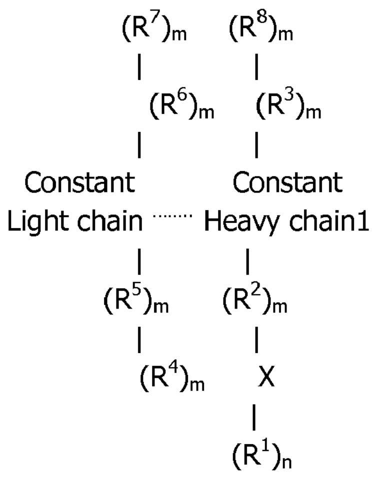


(I)

wherein
X represents a constant antibody region comprising constant heavy domain 2 and constant heavy domain 3;
$R^1$, $R^4$, $R^7$ and $R^8$ represent a domain independently selected from an epitope-binding domain;
$R^2$ represents a domain selected from the group consisting of constant heavy chain 1, and an epitope-binding domain;
$R^3$ represents a domain selected from the group consisting of a paired VH and an epitope-binding domain;
$R^5$ represents a domain selected from the group consisting of constant light chain, and an epitope-binding domain;
$R^6$ represents a domain selected from the group consisting of a paired VL and an epitope-binding domain;
n represents an integer independently selected from: 0, 1, 2, 3 and 4;
m represents an integer independently selected from: 0 and 1,
wherein the Constant Heavy chain 1 and the Constant Light chain domains are associated;
wherein at least one epitope binding domain is present;
and when $R^3$ represents a paired VH domain, $R^6$ represents a paired VL domain, so that the two domains are together capable of binding antigen.

The invention relates to IgG based structures which comprise monoclonal antibodies, or fragments linked to one or more domain antibodies, and to methods of making such constructs and uses thereof, particularly uses in therapy.

The invention also provides a polynucleotide sequence encoding a heavy chain of any of the antigen binding constructs described herein, and a polynucleotide encoding a light chain of any of the antigen binding constructs described herein. Such polynucleotides represent the coding sequence which corresponds to the equivalent polypeptide sequences, however it will be understood that such polynucleotide sequences could be cloned into an expression vector along with a start codon, an appropriate signal sequence and a stop codon.

The invention also provides a recombinant transformed or transfected host cell comprising one or more polynucleotides encoding a heavy chain and a light chain of any of the antigen binding constructs described herein.

The invention further provides a method for the production of any of the antigen binding constructs described herein which method comprises the step of culturing a host cell comprising a first and second vector, said first vector comprising a polynucleotide encoding a heavy chain of any of the antigen binding constructs described herein and said second vector comprising a polynucleotide encoding a light chain of any of the antigen binding constructs described herein, in a serum- free culture media.

The invention further provides a pharmaceutical composition comprising an antigen binding construct as described herein a pharmaceutically acceptable carrier.

The invention also provides a domain antibody comprising or consisting of the polypeptide sequence set out in SEQ ID NO: 2 or SEQ ID NO: 3. In one aspect the invention provides a protein which is expressed from the polynucleotide sequence set out in SEQ ID NO: 60 or SEQ ID NO: 61.

**Definitions**

The term 'Protein Scaffold' as used herein includes but is not limited to an immunoglobulin (Ig) scaffold, for example an IgG scaffold, which may be a four chain or two chain antibody, or which may comprise only the Fc region of an antibody, or which may comprise one or more constant regions from an antibody, which constant regions may be of human or primate origin, or which may be an artificial chimera of human and primate constant regions. Such protein scaffolds may comprise antigen-binding sites in addition to the one or more constant regions, for example where the

protein scaffold comprises a full IgG. Such protein scaffolds will be capable of being linked to other protein domains, for example protein domains which have antigen-binding sites, for example epitope-binding domains or ScFv domains.

A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. A "single antibody variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The phrase "immunoglobulin single variable domain" refers to an antibody variable domain ($V_H$, $V_{HH}$, $V_L$) that specifically binds an antigen or epitope independently of a different V region or domain. An immunoglobulin single variable domain can be present in a format (*e.g.*, homo- or hetero-multimer) with other, different variable regions or variable domains where the other regions or domains are not required for antigen binding by the single immunoglobulin variable domain (*i.e.,* where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004), nurse shark and *Camelid* $V_{HH}$ dAbs. Camelid $V_{HH}$ are immunoglobulin single variable domain polypeptides that are derived from species including camel, llama, alpaca, dromedary, and guanaco, which produce heavy chain antibodies naturally devoid of light chains. Such $V_{HH}$ domains may be humanised according to standard techniques available in the art, and such domains are still considered to be "domain antibodies" according to the invention. As used herein "$V_H$ includes camelid $V_{HH}$ domains. NARV are another type of immunoglobulin single variable domain which were identified in cartilaginous fish including the nurse shark. These domains are also known as Novel Antigen Receptor variable region (commonly abbreviated to V(NAR) or NARV). For further details see Mol. Immunol. 44, 656-665 (2006) and US20050043519A.

The term "Epitope-binding domain" refers to a domain that specifically binds an antigen or epitope independently of a different V region or domain, this may be a domain antibody (dAb), for example a human, camelid or shark immunoglobulin single variable domain or itmay be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand.

CTLA-4 (Cytotoxic T Lymphocyte-associated Antigen 4) is a CD28-family receptor expressed on mainly CD4+ T-cells. Its extracellular domain has a variable domain-like Ig fold. Loops corresponding to CDRs of antibodies can be substituted with heterologous sequence to confer different binding properties. CTLA-4 molecules engineered to have different binding specificities are also known as Evibodies. For further details see Journal of Immunological Methods 248 (1-2), 31-45 (2001)

Lipocalins are a family of extracellular proteins which transport small hydrophobic molecules such as steroids, bilins, retinoids and lipids. They have a rigid β-sheet secondary structure with a numer of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633

An affibody is a scaffold derived from Protein A of *Staphylococcus aureus* which can be engineered to bind to antigen. The domain consists of a three-helical bundle of approximately 58 amino acids. Libraries have been generated by randomisation of surface residues. For further details see Protein Eng. Des. Sel. 17, 455-462 (2004) and EP1641818A1

Avimers are multidomain proteins derived from the A-domain scaffold family. The native domains of approximately 35 amino acids adopt a defined disulphide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007)

A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two α-helices and a β-turn. They can be engineered to bind different target antigens by randomising residues in the first α-helix and a β-turn of each repeat. Their binding interface can be increased by increasing the number of modules

(a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the β-sandwich can be engineered to enable an Adnectin to specifically recognize a therapeutic target of interest. For further details see Protein Eng. Des. Sel. 18, 435-444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataB1 and conotoxin and knottins. The microproteins have a loop which can be engineered to include upto 25 amino acids without affecting the overall fold of the microprotein. For further details of engineered knottin domains, see WO2008098796.

Other epitope binding domains include proteins which have been used as a scaffold to engineer different target antigen binding properties include human γ-crystallin and human ubiquitin (affilins), kunitz type domains of human protease inhibitors, PDZ-domains of the Ras-binding protein AF-6, scorpion toxins (charybdotoxin), C-type lectin domain (tetranectins) are reviewed in Chapter 7 - Non-Antibody Scaffolds from Handbook of Therapeutic Antibodies (2007, edited by Stefan Dubel) and Protein Science 15:14-27 (2006). Epitope binding domains of the present invention could be derived from any of these alternative protein domains.

As used herein, the terms "paired VH domain", "paired VL domain", and "paired VH/VL domains" refer to antibody variable domains which specifically bind antigen only when paired with their partner variable domain. There is always one VH and one VL in any pairing, and the term "paired VH domain" refers to the VH partner, the term "paired VL domain" refers to the VL partner, and the term "paired VH/VL domains" refers to the two domains together.

In one embodiment of the invention the antigen binding site binds to antigen with a Kd of at least 1mM, for example a Kd of 10nM, 1nM, 500pM, 200pM, 100pM, to each antigen as measured by Biacore™, such as the Biacore™ method as described in method 4 or 5.

As used herein, the term "antigen binding site" refers to a site on a construct which is capable of specifically binding to antigen, this may be a single domain, for example an epitope-binding domain, or it may be paired VH/VL domains as can be found on a standard antibody. In some aspects of the invention single-chain Fv (ScFv) domains can provide antigen-binding sites.

The terms "mAb/dAb" and dAb/mAb" are used herein to refer to antigen-binding constructs of the present invention. The two terms can be used interchangeably, and are intended to have the same meaning as used herein.

The term "constant heavy chain 1" is used herein to refer to the CH1 domain of an immunoglobulin heavy chain.

The term "constant light chain" is used herein to refer to the constant domain of an immunoglobulin light chain.

### Detailed description of Invention

The present invention relates to an antigen-binding construct comprising a protein scaffold which is linked to one or more epitope-binding domains wherein the antigen-binding construct has at least two antigen binding sites at least one of which is from an epitope binding domain and at least one of which is from a paired VH/VL domain.

Such antigen-binding constructs comprise a protein scaffold, for example an Ig scaffold such as IgG, for example a monoclonal antibody, which is linked to one or more epitope-binding domains, for example a domain antibody, wherein the binding construct has at least two antigen binding sites, at least one of which is from an epitope binding domain, and to methods of producing and uses thereof, particularly uses in therapy.

Some examples of antigen-binding constructs according to the invention are set out in Figure 1.

The antigen-binding constructs of the present invention are also referred to as mAbdAbs.

In one embodiment the protein scaffold of the antigen-binding construct of the present invention is an Ig scaffold, for example an IgG scaffold or IgA scaffold. The IgG scaffold may comprise all the domains of an antibody (i.e. CH1, CH2, CH3, VH, VL). The antigen-binding construct of the present invention may comprise an IgG scaffold selected from IgG1, IgG2, IgG3, IgG4 or IgG4PE.

The antigen-binding construct of the present invention has at least two antigen binding sites, for examples it has two binding sites, for example where the first binding site has specificity for a first epitope on an antigen and the second binding site has specificity for a second epitope on the same antigen. In a further embodiment there are 4 antigen binding sites, or 6 antigen binding sites, or 8 antigen binding sites, or 10 or more antigen-binding sites. In one embodiment the antigen binding construct has specificity for more than one antigen, for example two antigens, or for three antigens, or for four antigens.

In another aspect the invention relates to an antigen-binding construct comprising at least one homodimer

comprising two or more structures of formula I:

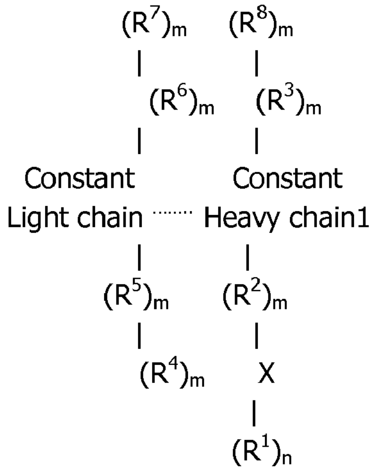


(I)

wherein
X represents a constant antibody region comprising constant heavy domain 2 and constant heavy domain 3;
$R^1$, $R^4$ , $R^7$ and $R^8$ represent a domain independently selected from an epitope-binding domain;
$R^2$ represents a domain selected from the group consisting of constant heavy chain 1, and an epitope-binding domain;
$R^3$ represents a domain selected from the group consisting of a paired VH and an epitope-binding domain; $R^5$ represents a domain selected from the group consisting of constant light chain, and an epitope-binding domain; $R^6$ represents a domain selected from the group consisting of a paired VL and an epitope-binding domain;
n represents an integer independently selected from: 0, 1, 2, 3 and 4;
m represents an integer independently selected from: 0 and 1,
wherein the Constant Heavy chain 1 and the Constant Light chain domains are associated;
wherein at least one epitope binding domain is present;
and when $R^3$ represents a paired VH domain, $R^6$ represents a paired VL domain, so that the two domains are together capable of binding antigen.

In one embodiment $R^6$ represents a paired VL and $R^3$ represents a paired VH.

In a further embodiment either one or both of $R^7$ and $R^8$ represent an epitope binding domain.

In yet a further embodiment either one or both of $R^1$ and $R^4$ represent an epitope binding domain.

In one embodiment $R^4$ is present.

In one embodiment $R^1$ $R^7$ and $R^8$ represent an epitope binding domain.

In one embodiment $R^1$ $R^7$ and $R^8$, and $R^4$ represent an epitope binding domain.

In one embodiment $(R^1)_n$, $(R^2)_m$, $(R^4)_m$ and $(R^5)_m$ = 0, i.e. are not present, $R^3$ is a paired VH domain, $R^6$ is a paired VL domain, $R^8$ is a VH dAb, and $R^7$ is a VL dAb.

In another embodiment $(R^1)_n$, $(R^2)_m$, $(R^4)_m$ and $(R^5)_m$ are 0, i.e. are not present, $R^3$ is a paired VH domain, $R^6$ is a paired VL domain, $R^8$ is a VH dAb, and $(R^7)_m$ = 0 i.e. not present.

In another embodiment $(R^2)_m$, and $(R^5)_m$ are 0, i.e. are not present, $R^1$ is a dAb, $R^4$ is a dAb, $R^3$ is a paired VH domain, $R^6$ is a paired VL domain, $(R^8)_m$ and $(R^7)_m$ = 0 i.e. not present.

In one embodiment of the present invention the epitope binding domain is a dAb.

It will be understood that any of the antigen-binding constructs described herein will be capable of neutralising one or more antigens.

The term "neutralises" and grammatical variations thereof as used throughout the present specification in relation to antigen binding constructs of the invention means that a biological activity of the target is reduced, either totally or partially, in the presence of the antigen binding constructs of the present invention in comparison to the activity of the target in the absence of such antigen binding constructs. Neutralisation may be due to but not limited to one or more of blocking ligand binding, preventing the ligand activating the receptor, down regulating the receptor or affecting effector functionality.

Levels of neutralisation can be measured in several ways, for example by use of any of the assays as set out in the examples and methods below, for example in an assay which measures inhibition of ligand binding to receptor which may be carried out for example as described in any one of Methods 12, 19 or 21 or Example 32. The neutralisation of VEGF, IL-4, IL-13 or HGF in these assays is measured by assessing the decreased binding between the ligand and

its receptor in the presence of neutralising antigen binding construct.

Levels of neutralisation can also be measured, for example in a TF1 assay which may be carried out for example as described in Method 8, 9, 10, 20 or 21. The neutralisation of IL-13, IL-4 or both of these cytokines in this assay is measured by assessing the inhibition of TF1 cell proliferation in the presence of neutralising antigen binding construct. Alternatively neutralisation could be measured in an EGFR phosphorylation assay which may be carried out for example as described in Method 13. The neutralisation of EGFR in this assay is measured by assessing the inhibition of tyrosine kinase phosphorylation of the receptor in the presence of neutralising antigen binding construct. Or, neutralisation could be measured in an IL-8 secretion assay in MRC-5 cells which may be carried out for example as described in Method 14 or 15. The neutralisation of TNF$\alpha$ or IL-1R1 in this assay is measured by assessing the inhibition of IL-8 secretion in the presence of neutralising antigen binding construct.

Other methods of assessing neutralisation, for example, by assessing the decreased binding between the ligand and its receptor in the presence of neutralising antigen binding construct are known in the art, and include, for example, Biacore™ assays.

In an alternative aspect of the present invention there is provided antigen binding constructs which have at least substantially equivalent neutralising activity to the antibodies exemplified herein, for example antigen binding constructs which retain the neutralising activity of 586H-TVAAPS-210, or PascoH-G4S-474, or PascoH-474, PascoH-474 GS removed, PascoL-G4S-474 or PascoHL-G4S-474 in the TF1 cell proliferation assay, or inhibition of pSTAT6 signalling assay as set out in Examples 4 and 20 respectively, or for example antigen binding constructs which retain the neutralising activity of BPC1603, BPC1604, BPC1605, BPC1606 in the VEGFR binding assay or inhibition of IGF-1R receptor phosphorylation as set out in Examples 14.6 and 14.7.

The antigen binding constructs of the invention include those which have specificity for IL-13, for example which comprise an epitope-binding domain which is capable of binding to IL-13, or which comprise a paired VH/VL which binds to IL-13. The antigen binding construct may comprise an antibody which is capable of binding to IL-13. The antigen binding construct may comprise a dAb which is capable of binding to IL-13.

In one embodiment the antigen-binding construct of the present invention has specificity for more than one antigen, for example where it is capable of binding two or more antigens selected from IL-13, IL-5, and IL-4, for example where it is capable of binding IL-13 and IL-4, or where it is capable of binding IL-13 and IL-5, or where it is capable of binding IL-5 and IL-4.

In one embodiment the antigen-binding construct of the present invention has specificity for more than one antigen, for example where it is capable of binding two or more antigens selected from IL-13, IL-5, and IL-4, for example where it is capable of binding IL-13 and IL-4 simultaneously, or where it is capable of binding IL-13 and IL-5 simultaneously, or where it is capable of binding IL-5 and IL-4 simultaneously.

It will be understood that any of the antigen-binding constructs described herein may be capable of binding two or more antigens simultaneously, for example, as determined by stochiometry analysis by using a suitable assay such as that described in the Examples section, method 7.

Examples of antigen-binding constructs of the invention include IL-13 antibodies which have an epitope binding domain with a specificity for IL-4, for example an anti-IL-4 dAb, attached to the c-terminus or the n-terminus of the heavy chain or the c-terminus or n-terminus of the light chain, for example the mAbdAb having the heavy chain sequence set out in SEQ ID NO:16 to 39, SEQ ID NO:41 to 43, SEQ ID NO:87 to 90, SEQ ID NO:151, SEQ ID NO:152 or SEQ ID NO:155. Antigen binding constructs of the present invention include IL-13 antibodies with an IL-4 epitope binding domain attached to the n-terminus of the heavy chain. Antigen binding constructs of the present invention include IL-13 antibodies with an IL-4 epitope binding domain attached to the n-terminus of the light chain. Antigen binding constructs of the present invention include IL-13 antibodies with an IL-4 epitope binding domain attached to the c-terminus of the heavy chain. Antigen binding constructs of the present invention include IL-13 antibodies with an IL-4 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-4 antibodies which have an epitope binding domain with a specificity for IL-13, for example an anti-IL-13 dAb, attached to the c-terminus or the n-terminus of the heavy chain or the c-terminus or n-terminus of the light chain, for example the mAbdAb having the heavy chain sequence set out in SEQ ID NO:48 to 53, SEQ ID NO:91 SEQ ID NO:92, SEQ ID NO: 149, SEQ ID NO: 150, or SEQ ID NO: 157 to 160, and/or the light chain sequence set out in SEQ ID NO:54 to 59.

Antigen binding constructs of the present invention include IL-4 antibodies with an IL-13 epitope binding domain attached to the n-terminus of the heavy chain. Antigen binding constructs of the present invention include IL-4 antibodies with an IL-13 epitope binding domain attached to the n-terminus of the light chain. Antigen binding constructs of the present invention include IL-4 antibodies with an IL-13 epitope binding domain attached to the c-terminus of the heavy chain. Antigen binding constructs of the present invention include IL-4 antibodies with an IL-13 epitope binding domain attached to the c-terminus of the light chain. Such antigen-binding constructs may also have one or more further epitope

binding domains with the same or different antigen-specificity attached to the c-terminus and/or the n-terminus of the heavy chain and/ or the c-terminus and/or n-terminus of the light chain.

Examples of such antigen-binding constructs include IL-13 antibodies which have an epitope binding domain with a specificity for IL-5, for example an anti-IL-5 dAb, attached to the c-terminus or the n-terminus of the heavy chain or the c-terminus or n-terminus of the light chain.. Antigen binding constructs of the present invention include IL-13 antibodies with an IL-5 epitope binding domain attached to the n-terminus of the heavy chain. Antigen binding constructs of the present invention include IL-13 antibodies with an IL-5 epitope binding domain attached to the n-terminus of the light chain. Antigen binding constructs of the present invention include IL-13 antibodies with an IL-5 epitope binding domain attached to the c-terminus of the heavy chain. Antigen binding constructs of the present invention include IL-13 antibodies with an IL-5 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-5 antibodies which have an epitope binding domain with a specificity for IL-13, for example an anti-IL-13 dAb, attached to the c-terminus or the n-terminus of the heavy chain or the c-terminus or n-terminus of the light chain, for example the mAbdAb having the light chain sequence set out in SEQ ID NO: 72.

Antigen binding constructs of the present invention include IL-5 antibodies with an IL-13 epitope binding domain attached to the n-terminus of the heavy chain. Antigen binding constructs of the present invention include IL-5 antibodies with an IL-13 epitope binding domain attached to the n-terminus of the light chain. Antigen binding constructs of the present invention include IL-5 antibodies with an IL-13 epitope binding domain attached to the c-terminus of the heavy chain. Antigen binding constructs of the present invention include IL-5 antibodies with an IL-13 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-4 antibodies which have an epitope binding domain with a specificity for IL-5, for example an anti-IL-5 dAb, attached to the c-terminus or the n-terminus of the heavy chain or the c-terminus or n-terminus of the light chain.. Antigen binding constructs of the present invention include IL-4 antibodies with an IL-5 epitope binding domain attached to the n-terminus of the heavy chain. Antigen binding constructs of the present invention include IL-4 antibodies with an IL-5 epitope binding domain attached to the n-terminus of the light chain. Antigen binding constructs of the present invention include IL-4 antibodies with an IL-5 epitope binding domain attached to the c-terminus of the heavy chain. Antigen binding constructs of the present invention include IL-4 antibodies with an IL-5 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-5 antibodies which have an epitope binding domain with a specificity for IL-4, for example an anti-IL-4 dAb, attached to the c-terminus or the n-terminus of the heavy chain or the c-terminus or n-terminus of the light chain, for example the mAbdAb having the heavy chain sequence set out in SEQ ID NO: 71.

Antigen binding constructs of the present invention include IL-5 antibodies with an IL-4 epitope binding domain attached to the n-terminus of the heavy chain. Antigen binding constructs of the present invention include IL-5 antibodies with an IL-4 epitope binding domain attached to the n-terminus of the light chain. Antigen binding constructs of the present invention include IL-5 antibodies with an IL-4 epitope binding domain attached to the c-terminus of the heavy chain. Antigen binding constructs of the present invention include IL-5 antibodies with an IL-4 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

The invention also provides a trispecific binding construct which is capable of binding to IL-4, IL-13 and IL-5.

Examples of such antigen-binding constructs include IL-5 antibodies which have an epitope binding domain with a specificity for IL-4, for example an anti-IL-4 dAb, attached to the c-terminus or the n-terminus of the heavy chain or the c-terminus or n-terminus of the light chain and an epitope binding domain with a specificity for IL-13, for example an anti-IL-13 dAb, attached to the c-terminus or the n-terminus of the heavy chain or the c-terminus or n-terminus of the light chain.

Antigen binding constructs of the present invention include IL-5 antibodies with an IL-4 epitope binding domain attached to the n-terminus of the heavy chain and an IL-13 epitope binding domain attached to the n-terminus of the light chain.

Antigen binding constructs of the present invention include IL-5 antibodies with an IL-4 epitope binding domain attached to the n-terminus of the heavy chain and an IL-13 epitope binding domain attached to the c-terminus of the light chain.

Antigen binding constructs of the present invention include IL-5 antibodies with an IL-4 epitope binding domain attached to the n-terminus of the heavy chain and an IL-13 epitope binding domain attached to the c-terminus of the heavy chain.

Antigen binding constructs of the present invention include IL-5 antibodies with an IL-4 epitope binding domain attached to the n-terminus of the light chain and an IL-13 epitope binding domain attached to the c-terminus of the light chain.

Antigen binding constructs of the present invention include IL-5 antibodies with an IL-4 epitope binding domain attached to the n-terminus of the light chain and an IL-13 epitope binding domain attached to the c-terminus of the heavy chain.

Antigen binding constructs of the present invention include IL-5 antibodies with an IL-4 epitope binding domain attached to the n-terminus of the light chain and an IL-13 epitope binding domain attached to the n-terminus of the heavy chain.

Antigen binding constructs of the present invention include IL-5 antibodies with an IL-4 epitope binding domain attached to the c-terminus of the heavy chain and an IL-13 epitope binding domain attached to the c-terminus of the light chain.

Antigen binding constructs of the present invention include IL-5 antibodies with an IL-4 epitope binding domain attached to the c-terminus of the heavy chain and an IL-13 epitope binding domain attached to the n-terminus of the light chain.

Antigen binding constructs of the present invention include IL-5 antibodies with an IL-4 epitope binding domain attached to the c-terminus of the heavy chain and an IL-13 epitope binding domain attached to the n-terminus of the heavy chain.

Antigen binding constructs of the present invention include IL-5 antibodies with an IL-4 epitope binding domain attached to the c-terminus of the light chain and an IL-13 epitope binding domain attached to the c-terminus of the heavy chain.

Antigen binding constructs of the present invention include IL-5 antibodies with an IL-4 epitope binding domain attached to the c-terminus of the light chain and an IL-13 epitope binding domain attached to the n-terminus of the heavy chain.

Antigen binding constructs of the present invention include IL-5 antibodies with an IL-4 epitope binding domain attached to the c-terminus of the light chain and an IL-13 epitope binding domain attached to the n-terminus of the light chain.

Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for IL-18, for example which comprises an epitope-binding domain which is capable of binding to IL-18, or which comprises a paired VH/VL which binds to IL-18.

The antigen binding construct may comprise an antibody which is capable of binding to IL-18. The antigen binding construct may comprise a dAb which is capable of binding to IL-18.

The invention also provides a trispecific binding construct which is capable of binding to IL-4, IL-13 and IL-18.

Examples of such antigen-binding constructs include IL-18 antibodies which have an epitope binding domain with a specificity for IL-4, for example an anti-IL-4 dAb, attached to the c-terminus or the n-terminus of the heavy chain or the c-terminus or n-terminus of the light chain and an epitope binding domain with a specificity for IL-13, for example an anti-IL-13 dAb, attached to the c-terminus or the n-terminus of the heavy chain or the c-terminus or n-terminus of the light chain. Antigen binding constructs of the present invention include IL-18 antibodies with an IL-4 epitope binding domain attached to the n-terminus of the heavy chain and an IL-13 epitope binding domain attached to the n-terminus of the light chain.

Antigen binding constructs of the present invention include IL-18 antibodies with an IL-4 epitope binding domain attached to the n-terminus of the heavy chain and an IL-13 epitope binding domain attached to the c-terminus of the light chain.

Antigen binding constructs of the present invention include IL-18 antibodies with an IL-4 epitope binding domain attached to the n-terminus of the heavy chain and an IL-13 epitope binding domain attached to the c-terminus of the heavy chain.

Antigen binding constructs of the present invention include IL-18 antibodies with an IL-4 epitope binding domain attached to the n-terminus of the light chain and an IL-13 epitope binding domain attached to the c-terminus of the light chain.

Antigen binding constructs of the present invention include IL-18 antibodies with an IL-4 epitope binding domain attached to the n-terminus of the light chain and an IL-13 epitope binding domain attached to the c-terminus of the heavy chain.

Antigen binding constructs of the present invention include IL-18 antibodies with an IL-4 epitope binding domain attached to the n-terminus of the light chain and an IL-13 epitope binding domain attached to the n-terminus of the heavy

chain.

Antigen binding constructs of the present invention include IL-18 antibodies with an IL-4 epitope binding domain attached to the c-terminus of the heavy chain and an IL-13 epitope binding domain attached to the c-terminus of the light chain.

Antigen binding constructs of the present invention include IL-18 antibodies with an IL-4 epitope binding domain attached to the c-terminus of the heavy chain and an IL-13 epitope binding domain attached to the n-terminus of the light chain.

Antigen binding constructs of the present invention include IL-18 antibodies with an IL-4 epitope binding domain attached to the c-terminus of the heavy chain and an IL-13 epitope binding domain attached to the n-terminus of the heavy chain.

Antigen binding constructs of the present invention include IL-18 antibodies with an IL-4 epitope binding domain attached to the c-terminus of the light chain and an IL-13 epitope binding domain attached to the c-terminus of the heavy chain.

Antigen binding constructs of the present invention include IL-18 antibodies with an IL-4 epitope binding domain attached to the c-terminus of the light chain and an IL-13 epitope binding domain attached to the n-terminus of the heavy chain.

Antigen binding constructs of the present invention include IL-18 antibodies with an IL-4 epitope binding domain attached to the c-terminus of the light chain and an IL-13 epitope binding domain attached to the n-terminus of the light chain.

Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for TNF$\alpha$, for example which comprises an epitope-binding domain which is capable of binding to TNF$\alpha$, or which comprises a paired VH/VL which binds to TNF$\alpha$.

The antigen binding construct may comprise an antibody which is capable of binding to TNF$\alpha$. The antigen binding construct may comprise a dAb which is capable of binding to TNF$\alpha$.

In one embodiment the antigen-binding construct of the present invention has specificity for more than one antigen, for example where it is capable of binding two or more antigens selected from TNF$\alpha$, EGFR and VEGF, for example where it is capable of binding TNF$\alpha$ and EGFR, or where it is capable of binding TNF$\alpha$ and VEGF, or where it is capable of binding EGFR and VEGF. Examples of such antigen-binding constructs include TNF$\alpha$ antibodies which have an epitope binding domain with a specificity for EGFR, for example an anti-EGFR dAb, attached to the c-terminus or the n-terminus of the heavy chain or the c-terminus or n-terminus of the light chain, for example a mAbdAb having the heavy chain sequence set out in SEQ ID NO: 74, and/or the light chain sequence set out in SEQ ID NO: 79.

Antigen binding constructs of the present invention include TNF$\alpha$ antibodies with an EGFR epitope binding domain attached to the n-terminus of the heavy chain. Antigen binding constructs of the present invention include TNF$\alpha$ antibodies with an EGFR epitope binding domain attached to the n-terminus of the light chain. Antigen binding constructs of the present invention include TNF$\alpha$ antibodies with an EGFR epitope binding domain attached to the c-terminus of the heavy chain. Antigen binding constructs of the present invention include TNF$\alpha$ antibodies with an EGFR epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Antigen binding constructs of the present invention include EGFR antibodies with an TNF$\alpha$ epitope binding domain attached to the n-terminus of the heavy chain. Antigen binding constructs of the present invention include EGFR antibodies with an TNF$\alpha$ epitope binding domain attached to the n-terminus of the light chain. Antigen binding constructs of the present invention include EGFR antibodies with an TNF$\alpha$ epitope binding domain attached to the c-terminus of the heavy chain. Antigen binding constructs of the present invention include EGFR antibodies with an TNF$\alpha$ epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include TNF$\alpha$ antibodies which have an epitope binding domain with a specificity for VEGF, for example an anti-VEGF dAb, attached to the c-terminus or the n-terminus of the heavy chain or the c-terminus or n-terminus of the light chain, for example a mAbdAb having the heavy chain sequence set out in SEQ ID NO: 75, 78 or 185.

The antigen-binding construct of the present invention may have specificity for more than one antigen, for example where it is capable of binding TNF$\alpha$, and one or both antigens selected from IL-4 and IL-13, for example where it is capable of binding TNF$\alpha$ and IL-4, or where it is capable of binding TNF$\alpha$ and IL-13, or where it is capable of binding TNF$\alpha$ and IL-13 and IL-4. Examples of such antigen-binding constructs include IL-13 antibodies which have an epitope

binding domain with a specificity for TNFα, for example an anti- TNFα adnectin, attached to the c-terminus or the n-terminus of the heavy chain or the c-terminus or n-terminus of the light chain, for example a mAbdAb having the heavy chain sequence set out in SEQ ID NO: 134 or 135. Other examples of such antigen-binding constructs include IL-4 antibodies which have an epitope binding domain with a specificity for TNFα, for example an anti- TNFα adnectin, attached to the c-terminus or the n-terminus of the heavy chain or the c-terminus or n-terminus of the light chain, for example a mAbdAb having the heavy chain sequence set out in SEQ ID NO: 146 or 147.

Antigen binding constructs of the present invention include TNFα antibodies with an VEGF epitope binding domain attached to the n-terminus of the heavy chain. Antigen binding constructs of the present invention include TNFα antibodies with an VEGF epitope binding domain attached to the n-terminus of the light chain. Antigen binding constructs of the present invention include TNFα antibodies with an VEGF epitope binding domain attached to the c-terminus of the heavy chain. Antigen binding constructs of the present invention include TNFα antibodies with an VEGF epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Antigen binding constructs of the present invention include VEGF antibodies with an TNFα epitope binding domain attached to the n-terminus of the heavy chain. Antigen binding constructs of the present invention include VEGF antibodies with an TNFα epitope binding domain attached to the n-terminus of the light chain. Antigen binding constructs of the present invention include VEGF antibodies with an TNFα epitope binding domain attached to the c-terminus of the heavy chain. Antigen binding constructs of the present invention include VEGF antibodies with an TNFα epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for CD-20, for example which comprises an epitope-binding domain which is capable of binding to CD-20, or which comprises a paired VH/VL which binds to CD-20.

The antigen binding construct may comprise an antibody which is capable of binding to CD-20, for example it may comprise an antibody having the heavy and light chain sequences of SEQ ID NO: 120 and 117. The antigen binding construct may comprise a dAb which is capable of binding to CD-20. Examples of mAbdAbs with specificity for CD-20 are those having the heavy chain sequence set out in SEQ ID NO: 116, 118 or those having the light chain sequence set out in SEQ ID NO: 119 or 121.

Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for IL1R1, for example which comprise an epitope-binding domain which is capable of binding to IL1R1, or which comprises a paired VH/VL which binds to IL1R1.

The antigen binding construct may comprise an antibody which is capable of binding to IL1R1. The antigen binding construct may comprise a dAb which is capable of binding to IL1R1.

In one embodiment the antigen-binding construct of the present invention has specificity for more than one antigen, for example where it is capable of binding IL1R1 and a second antigen, for example where it is capable of binding IL1R1 and VEGF. Examples of such antigen-binding constructs include IL1R1 antibodies which have an epitope binding domain with a specificity for VEGF, for example an anti- VEGF dAb, attached to the c-terminus or the n-terminus of the heavy chain or the c-terminus or n-terminus of the light chain, for example a mAbdAb having the light chain sequence set out in SEQ ID NO: 77.

Antigen binding constructs of the present invention include IL1R1 antibodies with an VEGF epitope binding domain attached to the n-terminus of the heavy chain. Antigen binding constructs of the present invention include IL1R1 antibodies with an VEGF epitope binding domain attached to the n-terminus of the light chain. Antigen binding constructs of the present invention include IL1R1 antibodies with an VEGF epitope binding domain attached to the c-terminus of the heavy chain. Antigen binding constructs of the present invention include IL1R1 antibodies with an VEGF epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Antigen binding constructs of the present invention include VEGF antibodies with an IL1R1 epitope binding domain attached to the n-terminus of the heavy chain. Antigen binding constructs of the present invention include VEGF antibodies with an IL1R1 epitope binding domain attached to the n-terminus of the light chain. Antigen binding constructs of the present invention include VEGF antibodies with an IL1R1 epitope binding domain attached to the c-terminus of the heavy chain. Antigen binding constructs of the present invention include VEGF antibodies with an IL1R1 epitope binding domain attached to the c-terminus of the light chain. Such antigen-binding constructs may also have one or

more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for EGFR, for example which comprises an epitope-binding domain which is capable of binding to EGFR, or which comprises a paired VH/VL which binds to EGFR.

The antigen binding construct may comprise an antibody which is capable of binding to EGFR. The antigen binding construct may comprise a dAb which is capable of binding to EGFR. Some examples of such antigen binding construct will be capable of binding to an epitope on EGFR comprising SEQ ID NO:103, for example an antigen binding construct comprising one or more of the CDRs set out in SEQ ID NO: 97 to SEQ ID NO: 102 and SEQ ID NO: 104 to SEQ ID NO: 107.

In one embodiment the antigen-binding construct of the present invention has specificity for more than one antigen, for example where it is capable of binding two or more antigens selected from EGFR, IGF-1R, VEGFR2 and VEGF, for example where it is capable of binding EGFR and IGF-1R, or where it is capable of binding EGFR and VEGF, or where it is capable of binding VEGF and IGF-1R, or where it is capable of binding EGFR and VEGFR2, or where it is capable of binding IGF-1R and VEGFR2, or where it is capable of binding VEGF and VEGFR2, or where it is capable of binding EGFR, IGF-1R and VEGFR2, or where it is capable of binding VEGF, IGF-1R and VEGFR2, or where it is capable of binding EGFR, VEGF and VEGFR2, or where it is capable of binding EGFR, VEGF and IGF1R. Examples of such antigen-binding constructs include EGFR antibodies which have an epitope binding domain with a specificity for VEGFR2, for example an anti-VEGFR2 adnectin, attached to the c-terminus or the n-terminus of the heavy chain or the c-terminus or n-terminus of the light chain, for example the mAbdAb having the heavy chain sequence set out in SEQ ID NO: 136, 140 or 144 and/or the light chain sequence set out in SEQ ID NO: 138, 142 or 145.

Examples of such antigen-binding constructs include EGFR antibodies which have an epitope binding domain with a specificity for VEGF, for example an anti-VEGF dAb, attached to the c-terminus or the n-terminus of the heavy chain or the c-terminus or n-terminus of the light chain, for example the mAbdAb having the heavy chain sequence set out in SEQ ID NO: 165, 174, 176, 178, 184 or 186 and/or the light chain sequence set out in SEQ ID NO: 188 or 190.

Examples of such antigen-binding constructs include VEGF antibodies which have an epitope binding domain with a specificity for EGFR, for example an anti-EGFR dAb, attached to the c-terminus or the n-terminus of the heavy chain or the c-terminus or n-terminus of the light chain, for example the mAbdAb having the heavy chain sequence set out in SEQ ID NO: 180. Such mAbdAbs may also comprise the light chain sequence set out in SEQ ID NO: 182.

Examples of such antigen-binding constructs include IGF-1R antibodies which have an epitope binding domain with a specificity for VEGF, for example an anti-VEGF lipocalin, attached to the c-terminus or the n-terminus of the heavy chain or the c-terminus or n-terminus of the light chain, for example the mAbdAb having the heavy chain sequence set out in SEQ ID NO: 123 or 125. Such mAbdAbs may also comprise the light chain sequence set out in SEQ ID NO:113

Examples of such antigen-binding constructs include IGF-1R antibodies which have an epitope binding domain with a specificity for VEGFR2, for example an anti-VEGFR2 adnectin, attached to the c-terminus or the n-terminus of the heavy chain or the c-terminus or n-terminus of the light chain, for example the mAbdAb having the heavy chain sequence set out in SEQ ID NO: 124 or 133. Such mAbdAbs may also comprise the light chain sequence set out in SEQ ID NO: 113.

The antigen binding constructs of the invention include those which have specificity for IL-23, for example which comprises an epitope-binding domain which is capable of binding to IL-23, or which comprises a paired VH/VL which binds to IL-23.

The antigen binding construct may comprise an antibody which is capable of binding to IL-23. The antigen binding construct may comprise a dAb which is capable of binding to IL-23.

In one embodiment the antigen-binding construct of the present invention has specificity for more than one antigen, for example where it is capable of binding two or more antigens selected from TH17 type cytokines, for example. IL-17, IL-22, or IL-21, for example where it is capable of binding IL-23 and IL-17, or where it is capable of binding IL-23 and IL-21, or where it is capable of binding IL-23 and IL-22. Examples of such antigen-binding constructs include IL-23 antibodies which have an epitope binding domain with a specificity for IL-17, for example an anti-IL-17 dAb, attached to the c-terminus or the n-terminus of the heavy chain or the c-terminus or n-terminus of the light chain.

The antigen binding constructs of the invention include those which have specificity for PDGFR$\alpha$, for example which comprises an epitope-binding domain which is capable of binding to PDGFR$\alpha$, or which comprises a paired VH/VL which binds to PDGFR$\alpha$. The antigen binding construct may comprise an antibody which is capable of binding to PDGFR$\alpha$. The antigen binding construct may comprise a dAb which is capable of binding to PDGFR$\alpha$.

The antigen binding constructs of the invention include those which have specificity for FGFR1, for example which comprises an epitope-binding domain which is capable of binding to FGFR1, or which comprises a paired VH/VL which binds to FGFR1. The antigen binding construct may comprise an antibody which is capable of binding to FGFR1. The antigen binding construct may comprise a dAb which is capable of binding to FGFR1.

The antigen binding constructs of the invention include those which have specificity for FGFR3, for example which comprises an epitope-binding domain which is capable of binding to FGFR3, or which comprises a paired VH/VL

which binds to FGFR3. The antigen binding construct may comprise an antibody which is capable of binding to FGFR3. The antigen binding construct may comprise a dAb which is capable of binding to FGFR3.

The antigen binding constructs of the invention include those which have specificity for VEGFR2, for example which comprises an epitope-binding domain which is capable of binding to VEGFR2, or which comprises a paired VH/VL which binds to VEGFR2.

The antigen binding construct may comprise an antibody which is capable of binding to VEGFR2. The antigen binding construct may comprise a dAb which is capable of binding to VEGFR2. The antigen binding constructs of the invention include those which have specificity for VEGFR3, for example which comprises an epitope-binding domain which is capable of binding to VEGFR3, or which comprises a paired VH/VL which binds to VEGFR3.

The antigen binding construct may comprise an antibody which is capable of binding to VEGFR3. The antigen binding construct may comprise a dAb which is capable of binding to VEGFR3. The antigen binding constructs of the invention include those which have specificity for VE cadherin, for example which comprises an epitope-binding domain which is capable of binding to VE cadherin, or which comprises a paired VH/VL which binds to VE cadherin. The antigen binding construct may comprise an antibody which is capable of binding to VE cadherin. The antigen binding construct may comprise a dAb which is capable of binding to VE cadherin.

The antigen binding constructs of the invention include those which have specificity for neuropilin, for example which comprises an epitope-binding domain which is capable of binding to neuropilin, or which comprises a paired VH/VL which binds to neuropilin.

The antigen binding construct may comprise an antibody which is capable of binding to neuropilin. The antigen binding construct may comprise a dAb which is capable of binding to neuropilin.

The antigen binding constructs of the invention include those which have specificity for Flt-3, for example which comprises an epitope-binding domain which is capable of binding to Flt-3, or which comprises a paired VH/VL which binds to Flt-3.

The antigen binding construct may comprise an antibody which is capable of binding to Flt-3. The antigen binding construct may comprise a dAb which is capable of binding to Flt-3.

The antigen binding constructs of the invention include those which have specificity for ron, for example which comprises an epitope-binding domain which is capable of binding ron, or which comprises a paired VH/VL which binds to ron.

The antigen binding construct may comprise an antibody which is capable of binding to ron. The antigen binding construct may comprise a dAb which is capable of binding to ron.

The antigen binding constructs of the invention include those which have specificity for Trp-1, for example which comprises an epitope-binding domain which is capable of binding Trp-1, or which comprises a paired VH/VL which binds to Trp-1.

The antigen binding construct may comprise an antibody which is capable of binding to Trp-1. The antigen binding construct may comprise a dAb which is capable of binding to Trp-1.

In one embodiment the antigen-binding construct of the present invention has specificity for more than one antigen, for example where it is capable of binding two or more antigens which are implicated in cancer, for example where it is capable of binding two or more antigens selected from PDGFRα, FGFR1, FGFR3, VEGFR2, VEGFR3, IGF1R, EGFR and VEGF, VE cadherin, neuropilin, Flt-3, ron, Trp-1, CD-20 for example where it is capable of binding PDGFRα and FGFR1, or where it is capable of binding PDGFRα and VEGF, or where it is capable of binding PDGFRα and FGFR3, or where it is capable of binding PDGFRα and VEGFR2, or where it is capable of binding PDGFRα and VEGFR3, or where it is capable of binding PDGFRα and IGF1R, or where it is capable of binding PDGFRα and EGFR, or where it is capable of binding PDGFRα and VEGF, or where it is capable of binding PDGFRα and VE cadherin, or where it is capable of binding PDGFRα and neuropilin, or where it is capable of binding PDGFRα and Flt-3, or where it is capable of binding PDGFRα and ron, or where it is capable of binding PDGFRα and Trp1, or where it is capable of binding PDGFRα and CD-20, or where it is capable of binding FGFR1 and FGFR3, or where it is capable of binding FGFR1 and VEGFR2, or where it is capable of binding FGFR1 and VEGR3, or where it is capable of binding FGFR1 and IGF1R, or where it is capable of binding FGFR1 and EGFR, or where it is capable of binding FGFR1 and VEGF, or where it is capable of binding FGFR1 and VE cadherin, or where it is capable of binding FGFR1 and neuropilin, or where it is capable of binding FGFR1 and Flt-3, or where it is capable of binding FGFR1 and ron, or where it is capable of binding FGFR1 and Trp-1, or where it is capable of binding FGFR1 and CD-20, or where it is capable of binding FGFR3 and VEGFR2, or where it is capable of binding FGFR3 and VEGFR3, or where it is capable of binding FGFR3 and IGF1R, or where it is capable of binding FGFR3 and EGFR, or where it is capable of binding FGFR3 and VEGF, or where it is capable of binding FGFR3 and VE cadherin, or where it is capable of binding FGFR3 and neuropilin, or where it is capable of binding FGFR3 and Flt-3, or where it is capable of binding FGFR3 and ron, or where it is capable of binding FGFR3 and Trp-1, or where it is capable of binding FGFR3 and CD-20, or where it is capable of binding VEGFR2 and VEGFR3, or or where it is capable of binding VEGFR2 and IGF1R, or where it is capable of binding VEGFR2 and EGFR, or where it is capable of binding VEGFR2 and VEGF, or where it is capable of binding VEGFR2 and VE cadherin, or

where it is capable of binding VEGFR2 and neuropilin, or where it is capable of binding VEGFR2 and Flt-3, or where it is capable of binding VEGFR2 and ron, or where it is capable of binding VEGFR2 and Trp-1, or where it is capable of binding VEGFR2 and CD-20, or where it is capable of binding VEGFR3 and IGF-1R, or where it is capable of binding VEGFR3 and EGFR, or where it is capable of binding VEGFR3 and VEGF, or where it is capable of binding VEGFR3 and VE cadherin, or where it is capable of binding VEGFR3 and neuropilin, or where it is capable of binding VEGFR3 and Flt-3, or where it is capable of binding VEGFR3 and Trp-1, or where it is capable of binding VEGFR3 and CD-20, or where it is capable of binding IGF1R and EGFR, or where it is capable of binding IGF1R and VEGF, or where it is capable of binding IGF1R and VE cadherin, or where it is capable of binding IGF1R and neuropilin, or where it is capable of binding IGF1R and Flt-3, or where it is capable of binding IGF1R and ron, or where it is capable of binding IGF1R and Trp-1, or where it is capable of binding IGF1R and CD-20, or where it is capable of binding EGFR and VEGF, or where it is capable of binding EGFR and VE cadherin, or where it is capable of binding EGFR and neuropilin, or where it is capable of binding EGFR and Flt-3, or where it is capable of binding EGFR and ron, or where it is capable of binding EGFR and Trp-1, or where it is capable of binding EGFR and CD-20, or where it is capable of binding VEGF and VE cadherin, or where it is capable of binding VEGF and neuropilin, or where it is capable of binding VEGF and Flt-3, or where it is capable of binding VEGF and ron, or where it is capable of binding VEGF and Trp-1, or where it is capable of binding VEGF and CD-20, or where it is capable of binding VE cadherin and neuropilin, or where it is capable of binding VE cadherin and Flt-3, or where it is capable of binding VE cadherin and ron, or where it is capable of binding VE cadherin and Trp-1, or where it is capable of binding VE cadherin and CD-20, or where it is capable of binding neuropilin and Flt-3, or where it is capable of binding neuropilin and ron, or where it is capable of binding neuropilin and Trp-1, or where it is capable of binding neuropilin and CD-20, or where it is capable of binding Flt-3 and ron, or where it is capable of binding Flt-3 and Trp-1, or where it is capable of binding Flt-3 and CD-20, or where it is capable of binding ron and Trp-1, or where it is capable of binding ron and CD-20, and or where it is capable of binding Trp-1 and CD-20.

Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for beta-amyloid, for example which comprise an epitope-binding domain which is capable of binding to beta-amyloid, or which comprises a paired VH/VL which binds to beta-amyloid.

The antigen binding construct may comprise an antibody which is capable of binding to beta-amyloid. The antigen binding construct may comprise a dAb which is capable of binding to beta-amyloid.

The antigen binding constructs of the invention include those which have specificity for CD-3, for example which comprise an epitope-binding domain which is capable of binding to CD-3, or which comprises a paired VH/VL which binds to CD-3.

The antigen binding construct may comprise an antibody which is capable of binding to CD-3. The antigen binding construct may comprise a dAb which is capable of binding to CD-3.

The antigen binding constructs of the invention include those which have specificity for gpIIIb/IIa, for example which comprise an epitope-binding domain which is capable of binding to gpIIIb/IIa, or which comprises a paired VH/VL which binds to gpIIIb/IIa.

The antigen binding construct may comprise an antibody which is capable of binding to gpIIIb/IIa. The antigen binding construct may comprise a dAb which is capable of binding to gpIIIb/IIa.

The antigen binding constructs of the invention include those which have specificity for TGFbeta, for example which comprise an epitope-binding domain which is capable of binding to TGFbeta, or which comprises a paired VH/VL which binds to TGFbeta.

The antigen binding construct may comprise an antibody which is capable of binding to TGFbeta. The antigen binding construct may comprise a dAb which is capable of binding to TGFbeta.

In one embodiment of the present invention there is provided an antigen binding construct according to the invention described herein and comprising a constant region such that the antibody has reduced ADCC and/or complement activation or effector functionality. In one such embodiment the heavy chain constant region may comprise a naturally disabled constant region of IgG2 or IgG4 isotype or a mutated IgG1 constant region. Examples of suitable modifications are described in EP0307434. One example comprises the substitutions of alanine residues at positions 235 and 237 (EU index numbering).

In one embodiment the antigen-binding constructs of the present invention will retain Fc functionality for example will be capable of one or both of ADCC and CDC activity. Such antigen-binding constructs may comprise an epitope-binding domain located on the light chain, for example on the c-terminus of the light chain.

The invention also provides a method of maintaining ADCC and CDC function of antigen-binding constructs by positioning of the epitope binding domain on the light chain of the antibody in particular, by positioning the epitope binding domain on the c-terminus of the light chain. Such ADCC and CDC function can be measured by any suitable assay, for example the ADCC assay set out in Example 15.3 and the CDC assay set out in Example 15.4.

The invention also provides a method of reducing CDC function of antigen-binding constructs by positioning of the epitope binding domain on the heavy chain of the antibody, in particular, by positioning the epitope binding domain on the c-terminus of the heavy chain. Such CDC function can be measured by any suitable assay, for example the CDC assay set out in Example 15.4.

In a further embodiment the antigen-binding construct of the present invention is capable of binding two or more antigens selected from VEGF, IGF-1R and EGFR, for example it is capable of binding EGFR and VEGF, or EGFR and IGF1R, or IGF1R and VEGF, or for example it is capable of binding to TNF and IL1-R. In embodiments of the invention which comprise an IGF-1R binding site, the IGF-1R binding site of the antigen-binding construct of the invention may comprise a paired VH/VL domain in the protein scaffold, which paired VH/VL domain may comprise one or more of the CDRs selected from those set out in SEQ ID NO: 80, SEQ ID NO: 81, SEQ ID NO: 82, SEQ ID NO: 83, SEQ ID NO: 84, SEQ ID NO: 85 and SEQ ID NO: 86, for example it may comprise at least CDRH3 as set out in SEQ ID NO:80, for example it may comprise all the CDRs set out in SEQ ID NO: 80, SEQ ID NO: 81, SEQ ID NO: 82, SEQ ID NO: 83, SEQ ID NO: 85, and SEQ ID NO: 86.

In embodiments of the invention which comprise an EGFR binding site, the antigen-binding construct of the present invention may bind to an epitope comprising residues 273-501 of the mature or normal or wild type EGFR sequence, for example it may bind to an epitope comprising residues 287-302 of the mature or normal or wildtype EGFR (SEQ ID N0:103).

In one embodiment, the EGFR binding site of the antigen-binding construct of the invention may comprise a paired VH/VL domain in the protein scaffold, which paired VH/VL domain may comprise one or more of the CDRs selected from those set out in SEQ ID NO: 104, SEQ ID NO: 105, SEQ ID NO: 106, SEQ ID NO: 100, SEQ ID NO: 101, and SEQ ID NO: 102, for example, it may comprise CDRH3 as set out in SEQ ID NO: 106, or it may comprise all six CDRs set out in SEQ ID NO: 104, SEQ ID NO: 105, SEQ ID NO: 106, SEQ ID NO: 100, SEQ ID NO: 101, and SEQ ID NO: 102. Such paired VH/VL domain may further comprise additional residues, particularly in the heavy chain CDRs, and in one embodiment, CDRH1 may comprise SEQ ID NO: 104 plus up to five additional residues, for example one or more of the five additional residues which are set out in SEQ ID NO: 97, CDRH2 may comprise SEQ ID NO: 105 plus up to two additional residues, for example one or both of the two additional residues which are set out in SEQ ID NO: 98 and SEQ ID NO: 107, and CDRH3 may comprise SEQ ID NO: 106 plus up to two additional residues, for example one or both of the two additional residues which are set out in SEQ ID NO: 99. In one such embodiment, the paired VH/VL comprises one or more of the CDRs set out in SEQ ID NO: 97, SEQ ID NO: 98, SEQ ID NO: 99, SEQ ID NO: 100, SEQ ID NO: 101, and SEQ ID NO: 102, for example it may comprise at least CDRH3 as set out in SEQ ID NO:99, for example it may comprise all six CDRs set out in SEQ ID NO: 97, SEQ ID NO: 98, SEQ ID NO: 99, SEQ ID NO: 100, SEQ ID NO: 101 and SEQ ID NO: 102 (more detail of suitable antibodies can be found in WO02/092771 and WO2005/081854).

In one embodiment, the antigen binding constructs comprise an epitope-binding domain which is a domain antibody (dAb), for example the epitope binding domain may be a human VH or human VL, or a camelid $V_{HH}$ or a shark dAb (NARV).

In one embodiment the antigen binding constructs comprise an epitope-binding domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand.

The antigen binding constructs of the present invention may comprise a protein scaffold attached to an epitope binding domain which is an adnectin, for example an IgG scaffold with an adnectin attached to the c-terminus of the heavy chain, or it may comprise a protein scaffold attached to an adnectin, for example an IgG scaffold with an adnectin attached to the n-terminus of the heavy chain, or it may comprise a protein scaffold attached to an adnectin, for example an IgG scaffold with an adnectin attached to the c-terminus of the light chain, or it may comprise a protein scaffold attached to an adnectin, for example an IgG scaffold with an adnectin attached to the n-terminus of the light chain.

In other embodiments it may comprise a protein scaffold, for example an IgG scaffold, attached to an epitope binding domain which is CTLA-4, for example an IgG scaffold with CTLA-4 attached to the n-terminus of the heavy chain, or it may comprise for example an IgG scaffold with CTLA-4 attached to the c-terminus of the heavy chain, or it may comprise for example an IgG scaffold with CTLA-4 attached to the n-terminus of the light chain, or it may comprise an IgG scaffold with CTLA-4 attached to the c-terminus of the light chain.

In other embodiments it may comprise a protein scaffold, for example an IgG scaffold, attached to an epitope binding domain which is a lipocalin, for example an IgG scaffold with a lipocalin attached to the n-terminus of the heavy chain, or it may comprise for example an IgG scaffold with a lipocalin attached to the c-terminus of the heavy chain, or it may comprise for example an IgG scaffold with a lipocalin attached to the n-terminus of the light chain, or it may

comprise an IgG scaffold with a lipocalin attached to the c-terminus of the light chain.

In other embodiments it may comprise a protein scaffold, for example an IgG scaffold, attached to an epitope binding domain which is an SpA, for example an IgG scaffold with an SpA attached to the n-terminus of the heavy chain, or it may comprise for example an IgG scaffold with an SpA attached to the c-terminus of the heavy chain, or it may comprise for example an IgG scaffold with an SpA attached to the n-terminus of the light chain, or it may comprise an IgG scaffold with an SpA attached to the c-terminus of the light chain.

In other embodiments it may comprise a protein scaffold, for example an IgG scaffold, attached to an epitope binding domain which is an affibody, for example an IgG scaffold with an affibody attached to the n-terminus of the heavy chain, or it may comprise for example an IgG scaffold with an affibody attached to the c-terminus of the heavy chain, or it may comprise for example an IgG scaffold with an affibody attached to the n-terminus of the light chain, or it may comprise an IgG scaffold with an affibody attached to the c-terminus of the light chain.

In other embodiments it may comprise a protein scaffold, for example an IgG scaffold, attached to an epitope binding domain which is an affimer, for example an IgG scaffold with an affimer attached to the n-terminus of the heavy chain, or it may comprise for example an IgG scaffold with an affimer attached to the c-terminus of the heavy chain, or it may comprise for example an IgG scaffold with an affimer attached to the n-terminus of the light chain, or it may comprise an IgG scaffold with an affimer attached to the c-terminus of the light chain.

In other embodiments it may comprise a protein scaffold, for example an IgG scaffold, attached to an epitope binding domain which is a GroEI, for example an IgG scaffold with a GroEI attached to the n-terminus of the heavy chain, or it may comprise for example an IgG scaffold with a GroEI attached to the c-terminus of the heavy chain, or it may comprise for example an IgG scaffold with a GroEI attached to the n-terminus of the light chain, or it may comprise an IgG scaffold with a GroEI attached to the c-terminus of the light chain.

In other embodiments it may comprise a protein scaffold, for example an IgG scaffold, attached to an epitope binding domain which is a transferrin, for example an IgG scaffold with a transferrin attached to the n-terminus of the heavy chain, or it may comprise for example an IgG scaffold with a transferrin attached to the c-terminus of the heavy chain, or it may comprise for example an IgG scaffold with a transferrin attached to the n-terminus of the light chain, or it may comprise an IgG scaffold with a transferrin attached to the c-terminus of the light chain.

In other embodiments it may comprise a protein scaffold, for example an IgG scaffold, attached to an epitope binding domain which is a GroES, for example an IgG scaffold with a GroES attached to the n-terminus of the heavy chain, or it may comprise for example an IgG scaffold with a GroES attached to the c-terminus of the heavy chain, or it may comprise for example an IgG scaffold with a GroES attached to the n-terminus of the light chain, or it may comprise an IgG scaffold with a GroES attached to the c-terminus of the light chain.

In other embodiments it may comprise a protein scaffold, for example an IgG scaffold, attached to an epitope binding domain which is a DARPin, for example an IgG scaffold with a DARPin attached to the n-terminus of the heavy chain, or it may comprise for example an IgG scaffold with a DARPin attached to the c-terminus of the heavy chain, or it may comprise for example an IgG scaffold with a DARPin attached to the n-terminus of the light chain, or it may comprise an IgG scaffold with a DARPin attached to the c-terminus of the light chain.

In other embodiments it may comprise a protein scaffold, for example an IgG scaffold, attached to an epitope binding domain which is a peptide aptamer, for example an IgG scaffold with a peptide aptamer attached to the n-terminus of the heavy chain, or it may comprise for example an IgG scaffold with a peptide aptamer attached to the c-terminus of the heavy chain, or it may comprise for example an IgG scaffold with a peptide aptamer attached to the n-terminus of the light chain, or it may comprise an IgG scaffold with a peptide aptamer attached to the c-terminus of the light chain.

In one embodiment of the present invention there are four epitope binding domains, for example four domain antibodies, two of the epitope binding domains may have specificity for the same antigen, or all of the epitope binding domains present in the antigen-binding construct may have specificity for the same antigen.

Protein scaffolds of the present invention may be linked to epitope-binding domains by the use of linkers. Examples of suitable linkers include amino acid sequences which may be from 1 amino acid to 150 amino acids in length, or from 1 amino acid to 140 amino acids, for example, from 1 amino acid to 130 amino acids, or from 1 to 120 amino acids, or from 1 to 80 amino acids, or from 1 to 50 amino acids, or from 1 to 20 amino acids, or from 1 to 10 amino acids, or from 5 to 18 amino acids. Such sequences may have their own tertiary structure, for example, a linker of the present invention may comprise a single variable domain. The size of a linker in one embodiment is equivalent to a single variable domain. Suitable linkers may be of a size from 1 to 20 angstroms, for example less than 15 angstroms, or less than 10 angstroms, or less than 5 angstroms.

In one embodiment of the present invention at least one of the epitope binding domains is directly attached to the Ig scaffold with a linker comprising from 1 to 150 amino acids, for example 1 to 20 amino acids, for example 1 to 10 amino acids. Such linkers may be selected from any one of those set out in SEQ ID NO: 6 to 11, 'STG' (serine, threonine, glycine), 'GSTG' or 'RS', for example the linker may be 'TVAAPS', or the linker may be 'GGGGS'. Linkers of use in the antigen binding constructs of the present invention may comprise alone or in addition to other linkers, one or more sets

of GS residues, for example 'GSTVAAPS' or 'TVAAPSGS' or 'GSTVAAPSGS'. In another embodiment there is no linker between the epitope binding domain, for example the dAb, and the Ig scaffold. In another embodiment the epitope binding domain, for example a dAb, is linked to the Ig scaffold by the linker 'TVAAPS'. In another embodiment the epitope binding domain, for example a dAb, is linked to the Ig scaffold by the linker 'TVAAPSGS'. In another embodiment the epitope binding domain, for example a dAb, is linked to the Ig scaffold by the linker 'GS'.

In one embodiment, the antigen-binding construct of the present invention comprises at least one epitope binding domain which is capable of binding human serum albumin.

In one embodiment, there are at least 3 antigen binding sites, for example there are 4, or 5 or 6 or 8 or 10 antigen binding sites and the antigen binding construct is capable of binding at least 3 or 4 or 5 or 6 or 8 or 10 antigens, for example it is capable of binding 3 or 4 or 5 or 6 or 8 or 10 antigens simultaneously.

The invention also provides the antigen-binding constructs for use in medicine, for example for use in the manufacture of a medicament for treating cancer or inflammatory diseases such as asthma, rheumatoid arthritis, or osteoarthritis.

The invention provides a method of treating a patient suffering from cancer or inflammatory diseases such as asthma, rheumatoid arthritis, or osteoarthritis, comprising administering a therapeutic amount of an antigen-binding construct of the invention.

The antigen-binding constructs of the invention may be used for the treatment of cancer or inflammatory diseases such as asthma, rheumatoid arthritis, or osteoarthritis.

The antigen-binding constructs of the invention may have some effector function. For example if the protein scaffold contains an Fc region derived from an antibody with effector function, for example if the protein scaffold comprises CH2 and CH3 from IgG1. Levels of effector function can be varied according to known techniques, for example by mutations in the CH2 domain, for example wherein the IgG1 CH2 domain has one or more mutations at positions selected from 239 and 332 and 330, for example the mutations are selected from S239D and I332E and A330L such that the antibody has enhanced effector function, and/or for example altering the glycosylation profile of the antigen-binding construct of the invention such that there is a reduction in fucosylation of the Fc region.

Protein scaffolds of use in the present invention include full monoclonal antibody scaffolds comprising all the domains of an antibody, or protein scaffolds of the present invention may comprise a non-conventional antibody structure, such as a monovalent antibody. Such monovalent antibodies may comprise a paired heavy and light chain wherein the hinge region of the heavy chain is modified so that the heavy chain does not homodimerise, such as the monovalent antibody described in WO2007059782. Other monovalent antibodies may comprise a paired heavy and light chain which dimerises with a second heavy chain which is lacking a functional variable region and CH1 region, wherein the first and second heavy chains are modified so that they will form heterodimers rather than homodimers, resulting in a monovalent antibody with two heavy chains and one light chain such as the monovalent antibody described in WO2006015371. Such monovalent antibodies can provide the protein scaffold of the present invention to which epitope binding domains can be linked, for example such as the antigen binding constructs describe in Example 32.

Epitope-binding domains of use in the present invention are domains that specifically bind an antigen or epitope independently of a different V region or domain, this may be a domain antibody or may be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand. In one embodiment this may be an domain antibody or other suitable domains such as a domain selected from the group consisting of CTLA-4, lipocallin, SpA, an Affibody, an avimer, GroEl, transferrin, GroES and fibronectin. In one embodiment this may be selected from a dAb, an Affibody, an ankyrin repeat protein (DARPin) and an adnectin. In another embodiment this may be selected from an Affibody, an ankyrin repeat protein (DARPin) and an adnectin. In another embodiment this may be a domain antibody, for example a domain antibody selected from a human, camelid or shark (NARV) domain antibody.

Epitope-binding domains can be linked to the protein scaffold at one or more positions. These positions include the C-terminus and the N-terminus of the protein scaffold, for example at the C-terminus of the heavy chain and/or the C-terminus of the light chain of an IgG, or for example the N-terminus of the heavy chain and/or the N-terminus of the light chain of an IgG.

In one embodiment, a first epitope binding domain is linked to the protein scaffold and a second epitope binding domain is linked to the first epitope binding domain, for example where the protein scaffold is an IgG scaffold, a first epitope binding domain may be linked to the c-terminus of the heavy chain of the IgG scaffold, and that epitope binding domain can be linked at its c-terminus to a second epitope binding domain, or for example a first epitope binding domain may be linked to the c-terminus of the light chain of the IgG scaffold, and that first epitope binding domain may be further linked at its c-terminus to a second epitope binding domain, or for example a first epitope binding domain may be linked

to the n-terminus of the light chain of the IgG scaffold, and that first epitope binding domain may be further linked at its n-terminus to a second epitope binding domain, or for example a first epitope binding domain may be linked to the n-terminus of the heavy chain of the IgG scaffold, and that first epitope binding domain may be further linked at its n-terminus to a second epitope binding domain. Examples of such antigen binding constructs are described in Example 31.

When the epitope-binding domain is a domain antibody, some domain antibodies may be suited to particular positions within the scaffold.

Domain antibodies of use in the present invention can be linked at the C-terminal end of the heavy chain and/or the light chain of conventional IgGs. In addition some dAbs can be linked to the C-terminal ends of both the heavy chain and the light chain of conventional antibodies.

In constructs where the N-terminus of dAbs are fused to an antibody constant domain (either $C_H3$ or CL), a peptide linker may help the dAb to bind to antigen. Indeed, the N-terminal end of a dAb is located closely to the complementarity-determining regions (CDRS) involved in antigen-binding activity. Thus a short peptide linker acts as a spacer between the epitope-binding, and the constant domain fo the protein scaffold, which may allow the dAb CDRs to more easily reach the antigen, which may therefore bind with high affinity.

The surroundings in which dAbs are linked to the IgG will differ depending on which antibody chain they are fused to:

> When fused at the C-terminal end of the antibody light chain of an IgG scaffold, each dAb is expected to be located in the vicinity of the antibody hinge and the Fc portion. It is likely that such dAbs will be located far apart from each other. In conventional antibodies, the angle between Fab fragments and the angle between each Fab fragment and the Fc portion can vary quite significantly. It is likely that - with mAbdAbs - the angle between the Fab fragments will not be widely different, whilst some angular restrictions may be observed with the angle between each Fab fragment and the Fc portion.

When fused at the C-terminal end of the antibody heavy chain of an IgG scaffold, each dAb is expected to be located in the vicinity of the $C_H3$ domains of the Fc portion. This is not expected to impact on the Fc binding properties to Fc receptors (e.g. FcγRI, II, III an FcRn) as these receptors engage with the $C_H2$ domains (for the FcγRI, II and III class of receptors) or with the hinge between the $C_H2$ and $C_H3$ domains (e.g. FcRn receptor). Another feature of such antigen-binding constructs is that both dAbs are expected to be spatially close to each other and provided that flexibility is provided by provision of appropriate linkers, these dAbs may even form homodimeric species, hence propagating the 'zipped' quaternary structure of the Fc portion, which may enhance stability of the construct.

Such structural considerations can aid in the choice of the most suitable position to link an epitope-binding domain, for example a dAb, on to a protein scaffold, for example an antibody.

The size of the antigen, its localization (in blood or on cell surface), its quaternary structure (monomeric or multimeric) can vary. Conventional antibodies are naturally designed to function as adaptor constructs due to the presence of the hinge region, wherein the orientation of the two antigen-binding sites at the tip of the Fab fragments can vary widely and hence adapt to the molecular feature of the antigen and its surroundings. In contrast dAbs linked to an antibody or other protein scaffold, for example a protein scaffold which comprises an antibody with no hinge region, may have less structural flexibility either directly or indirectly.

Understanding the solution state and mode of binding at the dAb is also helpful. Evidence has accumulated that *in vitro* dAbs can predominantly exist in monomeric, homo-dimeric or multimeric forms in solution (Reiter et al. (1999) J Mol Biol 290 p685-698; Ewert et al (2003) J Mol Biol 325, p531-553, Jespers et al (2004) J Mol Biol 337 p893-903; Jespers et al (2004) Nat Biotechnol 22 p1161-1165; Martin et al (1997) Protein Eng. 10 p607-614; Sepulvada et al (2003) J Mol Biol 333 p355-365). This is fairly reminiscent to multimerisation events observed *in vivo* with Ig domains such as Bence-Jones proteins (which are dimers of immunoglobulin light chains (Epp et al (1975) Biochemistry 14 p4943-4952; Huan et al (1994) Biochemistry 33 p14848-14857; Huang et al (1997) Mol immunol 34 p1291-1301) and amyloid fibers (James et al. (2007) J Mol Biol. 367:603-8).

For example, it may be desirable to link domain antibodies that tend to dimerise in solution to the C-terminal end of the Fc portion in preference to the C-terminal end of the light chain as linking to the C-terminal end of the Fc will allow those dAbs to dimerise in the context of the antigen-binding construct of the invention.

The antigen-binding constructs of the present invention may comprise antigen-binding sites specific for a single antigen, or may have antigen-binding sites specific for two or more antigens, or for two or more epitopes on a single antigen, or there may be antigen-binding sites each of which is specific for a different epitope on the same or different antigens.

The antigen-binding sites can each have binding specificity for an antigen, such as human or animal proteins, including cytokines, growth factors, cytokine receptors, growth factor receptors, enzymes (*e.g.*, proteases), co-factors for enzymes, DNA binding proteins, lipids and carbohydrates. Suitable targets, including cytokines, growth factors, cytokine receptors, growth factor receptors and other proteins include but are not limited to: ApoE, Apo-SAA, BDNF,

Cardiotrophin-1, CEA, CD40, CD40 Ligand, CD56, CD38, CD138, EGF, EGF receptor, ENA-78, Eotaxin, Eotaxin-2, Exodus-2, FAPα, FGF-acidic, FGF-basic, fibroblast growth factor-10, FLT3 ligand, Fractalkine (CX3C), GDNF, G-CSF, GM-CSF, GF-β1, human serum albumin, insulin, IFN-γ, IGF-I, IGF-II, IL-1α, IL-1β, IL-1 receptor, IL-1 receptor type 1, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, c-fms, v-fmsMDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF A, VEGF B, VEGF C, VEGF D, VEGF receptor 1, VEGF receptor 2, VEGF receptor 3, GCP-2, GRO/MGSA, GRO-β, GRO-γ, HCC1, 1-309, HER 1, HER 2, HER 3, HER 4, serum albumin, vWF, amyloid proteins (*e.g.*, amyloid alpha), MMP12, PDK1, IgE, and other targets disclosed herein. It will be appreciated that this list is by no means exhaustive.

In some embodiments, the protease resistant peptide or polypeptide binds a target in pulmonary tissue, such as a target selected from the group consisting of TNFR1, IL-1, IL-1R, IL-4, IL-4R, IL-5, IL-6, IL-6R, IL-8, IL-8R, IL-9, IL-9R, IL-10, IL-12 IL-12R, IL-13, IL-13Ra1, IL-13Ra2, IL-15, IL-15R, IL-16, IL-17R, IL-17, IL-18, IL-18R, IL-23 IL-23R, IL-25, CD2, CD4, CD11a, CD23, CD25, CD27, CD28, CD30, CD40, CD40L, CD56, CD138, ALK5, EGFR, FcER1, TGFb, CCL2, CCL18, CEA, CR8, CTGF, CXCL12 (SDF-1), chymase, FGF, Furin, Endothelin-1, Eotaxins (*e.g.*, Eotaxin, Eotaxin-2, Eotaxin-3), GM-CSF, ICAM-1, ICOS, IgE, IFNa, I-309, integrins, L-selectin, MIF, MIP4, MDC, MCP-1, MMPs, neutrophil elastase, osteopontin, OX-40, PARC, PD-1, RANTES, SCF, SDF-1, siglec8, TARC, TGFb, Thrombin, Tim-1, TNF, TRANCE, Tryptase, VEGF, VLA-4, VCAM, α4β7, CCR2, CCR3, CCR4, CCR5, CCR7, CCR8, alphavbeta6, alphavbeta8, cMET, CD8, vWF, amyloid proteins (*e.g.*, amyloid alpha), MMP12, PDK1, and IgE.

In particular, the antigen-binding constructs of the present invention may be useful in treating diseases associated with IL-13, IL-5 and IL-4, for example atopic dermatitis, allergic rhinitis, crohn's disease, COPD, fibrotic diseases or disorders such as idiopathic pulmonary fibrosis, progressive systemic sclerosis, hepatic fibrosis, hepatic granulomas, schistosomiasis, leishmaniasis, diseases of cell cycle regulation such as Hodgkins disease, B cell chronic lymphocytic leukaemia, for example the constructs may be useful in treating asthma.

Antigen-binding constructs of the present invention may be useful in treating diseases associated with growth factors such as IGF-1R, VEGF, and EGFR, for example cancer or rheumatoid arthritis, examples of types of cancer in which such therapies may be useful are breast cancer, prostrate cancer, lung cancer and myeloma.

Antigen-binding constructs of the present invention may be useful in treating diseases associated with TNF, for example arthritis, for example rheumatoid arthritis or osteoarthritis.

Antigen-binding constructs of the present invention may be useful in treating diseases associated with IL1-R, for example arthritis, for example rheumatoid arthritis or osteoarthritis.

Antigen-binding constructs of the present invention may be useful in treating diseases associated with CD-20, for example autoimmune diseases such as psoriasis, inflammatory bowel disease, ulcerative colitis, crohns disease, rheumatoid arthritis, juvenile rheumatoid arthritis, systemic lupus erythematosus, neurodegenerative diseases, for example multiple sclerosis, neutrophil driven diseases, for example COPD , Wegeners vasculitis, cystic fibrosis, Sjogrens syndrome, chronic transplant rejection, type 1 diabetes graft versus host disease, asthma, allergic diseases atoptic dermatitis, eczematous dermatitis, allergic rhinitis, autoimmune diseases other including thyroiditis, spondyloarthropathy, ankylosing spondylitis, uveitis, polychonritis or scleroderma, or cancer e.g. B- cell lymphomas or mature B cell neoplasm such as CLL or SLL.

Antigen-binding constructs of the present invention may be useful in treating diseases associated with IL-17 and IL-23, for example psoriasis, inflammatory bowel disease, ulcerative colitis, crohns disease, rheumatoid arthritis, juvenile rheumatoid arthritis, systemic lupus erythematosus, neurodegenerative diseases, for example multiple sclerosis, neutrophil driven diseases, for example COPD , Wegeners vasculitis, cystic fibrosis, Sjogrens syndrome, chronic transplant rejection, type 1 diabetes graft versus host disease, asthma, allergic diseases atoptic dermatitis, eczematous dermatitis, allergic rhinitis, autoimmune diseases other including thyroiditis, spondyloarthropathy, ankylosing spondylitis, uveitis, polychonritis or scleroderma.

The antigen binding constructs of the present invention may be produced by transfection of a host cell with an expression vector comprising the coding sequence for the antigen binding construct of the invention. An expression vector or recombinant plasmid is produced by placing these coding sequences for the antigen binding construct in operative association with conventional regulatory control sequences capable of controlling the replication and expression in, and/or secretion from, a host cell. Regulatory sequences include promoter sequences, e.g., CMV promoter, and signal sequences which can be derived from other known antibodies. Similarly, a second expression vector can be produced having a DNA sequence which encodes a complementary antigen binding construct light or heavy chain. In certain embodiments this second expression vector is identical to the first except insofar as the coding sequences and

selectable markers are concerned, so to ensure as far as possible that each polypeptide chain is functionally expressed. Alternatively, the heavy and light chain coding sequences for the antigen binding construct may reside on a single vector, for example in two expression cassettes in the same vector.

A selected host cell is co-transfected by conventional techniques with both the first and second vectors (or simply transfected by a single vector) to create the transfected host cell of the invention comprising both the recombinant or synthetic light and heavy chains. The transfected cell is then cultured by conventional techniques to produce the engineered antigen binding construct of the invention. The antigen binding construct which includes the association of both the recombinant heavy chain and/or light chain is screened from culture by appropriate assay, such as ELISA or RIA. Similar conventional techniques may be employed to construct other antigen binding constructs. Suitable vectors for the cloning and subcloning steps employed in the methods and construction of the compositions of this invention may be selected by one of skill in the art. For example, the conventional pUC series of cloning vectors may be used. One vector, pUC19, is commercially available from supply houses, such as Amersham (Buckinghamshire, United Kingdom) or Pharmacia (Uppsala, Sweden). Additionally, any vector which is capable of replicating readily, has an abundance of cloning sites and selectable genes (e.g., antibiotic resistance), and is easily manipulated may be used for cloning. Thus, the selection of the cloning vector is not a limiting factor in this invention.

The expression vectors may also be characterized by genes suitable for amplifying expression of the heterologous DNA sequences, e.g., the mammalian dihydrofolate reductase gene (DHFR). Other preferable vector sequences include a poly A signal sequence, such as from bovine growth hormone (BGH) and the betaglobin promoter sequence (betaglopro). The expression vectors useful herein may be synthesized by techniques well known to those skilled in this art.

The components of such vectors, e.g. replicons, selection genes, enhancers, promoters, signal sequences and the like, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression may also be selected for this purpose.

The present invention also encompasses a cell line transfected with a recombinant plasmid containing the coding sequences of the antigen binding constructs of the present invention. Host cells useful for the cloning and other manipulations of these cloning vectors are also conventional. However, cells from various strains of E. coli may be used for replication of the cloning vectors and other steps in the construction of antigen binding constructs of this invention.

Suitable host cells or cell lines for the expression of the antigen binding constructs of the invention include mammalian cells such as NS0, Sp2/0, CHO (e.g. DG44), COS, HEK, a fibroblast cell (e.g., 3T3), and myeloma cells, for example it may be expressed in a CHO or a myeloma cell. Human cells may be used, thus enabling the molecule to be modified with human glycosylation patterns. Alternatively, other eukaryotic cell lines may be employed. The selection of suitable mammalian host cells and methods for transformation, culture, amplification, screening and product production and purification are known in the art. See, e.g., Sambrook et al., cited above. Bacterial cells may prove useful as host cells suitable for the expression of the recombinant Fabs or other embodiments of the present invention (see, e.g., Plückthun, A., Immunol. Rev., 130:151-188 (1992)). However, due to the tendency of proteins expressed in bacterial cells to be in an unfolded or improperly folded form or in a non-glycosylated form, any recombinant Fab produced in a bacterial cell would have to be screened for retention of antigen binding ability. If the molecule expressed by the bacterial cell was produced in a properly folded form, that bacterial cell would be a desirable host, or in alternative embodiments the molecule may express in the bacterial host and then be subsequently re-folded. For example, various strains of E. coli used for expression are well-known as host cells in the field of biotechnology. Various strains of B. subtilis, Streptomyces, other bacilli and the like may also be employed in this method.

Where desired, strains of yeast cells known to those skilled in the art are also available as host cells, as well as insect cells, e.g. Drosophila and Lepidoptera and viral expression systems. See, e.g. Miller et al., Genetic Engineering, 8:277-298, Plenum Press (1986) and references cited therein.

The general methods by which the vectors may be constructed, the transfection methods required to produce the host cells of the invention, and culture methods necessary to produce the antigen binding construct of the invention from such host cell may all be conventional techniques. Typically, the culture method of the present invention is a serum-free culture method, usually by culturing cells serum-free in suspension. Likewise, once produced, the antigen binding constructs of the invention may be purified from the cell culture contents according to standard procedures of the art, including ammonium sulfate precipitation, affinity columns, column chromatography, gel electrophoresis and the like. Such techniques are within the skill of the art and do not limit this invention. For example, preparation of altered antibodies are described in WO 99/58679 and WO 96/16990.

Yet another method of expression of the antigen binding constructs may utilize expression in a transgenic animal, such as described in U. S. Patent No. 4,873,316. This relates to an expression system using the animal's casein promoter which when transgenically incorporated into a mammal permits the female to produce the desired recombinant protein in its milk.

In a further aspect of the invention there is provided a method of producing an antibody of the invention which

method comprises the step of culturing a host cell transformed or transfected with a vector encoding the light and/or heavy chain of the antibody of the invention and recovering the antibody thereby produced.

In accordance with the present invention there is provided a method of producing an antigen binding construct of the present invention which method comprises the steps of;

(a) providing a first vector encoding a heavy chain of the antigen binding construct;
(b) providing a second vector encoding a light chain of the antigen binding construct;
(c) transforming a mammalian host cell (e.g. CHO) with said first and second vectors;
(d) culturing the host cell of step (c) under conditions conducive to the secretion of the antigen binding construct from said host cell into said culture media;
(e) recovering the secreted antigen binding construct of step (d).

Once expressed by the desired method, the antigen binding construct is then examined for in vitro activity by use of an appropriate assay. Presently conventional ELISA assay formats are employed to assess qualitative and quantitative binding of the antigen binding construct to its target. Additionally, other in vitro assays may also be used to verify neutralizing efficacy prior to subsequent human clinical studies performed to evaluate the persistence of the antigen binding construct in the body despite the usual clearance mechanisms.

The dose and duration of treatment relates to the relative duration of the molecules of the present invention in the human circulation, and can be adjusted by one of skill in the art depending upon the condition being treated and the general health of the patient. It is envisaged that repeated dosing (e.g. once a week or once every two weeks) over an extended time period (e.g. four to six months) maybe required to achieve maximal therapeutic efficacy.

The mode of administration of the therapeutic agent of the invention may be any suitable route which delivers the agent to the host. The antigen binding constructs, and pharmaceutical compositions of the invention are particularly useful for parenteral administration, i.e., subcutaneously (s.c.), intrathecally, intraperitoneally, intramuscularly (i.m.), intravenously (i.v.), or intranasally.

Therapeutic agents of the invention may be prepared as pharmaceutical compositions containing an effective amount of the antigen binding construct of the invention as an active ingredient in a pharmaceutically acceptable carrier. In the prophylactic agent of the invention, an aqueous suspension or solution containing the antigen binding construct, preferably buffered at physiological pH, in a form ready for injection is preferred. The compositions for parenteral administration will commonly comprise a solution of the antigen binding construct of the invention or a cocktail thereof dissolved in a pharmaceutically acceptable carrier, preferably an aqueous carrier. A variety of aqueous carriers may be employed, e.g., 0.9% saline, 0.3% glycine, and the like. These solutions may be made sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antigen binding construct of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition of the invention for intramuscular injection could be prepared to contain 1 mL sterile buffered water, and between about 1 ng to about 100 mg, e.g. about 50 ng to about 30 mg or more preferably, about 5 mg to about 25 mg, of an antigen binding construct of the invention. Similarly, a pharmaceutical composition of the invention for intravenous infusion could be made up to contain about 250 ml of sterile Ringer's solution, and about 1 to about 30 and preferably 5 mg to about 25 mg of an antigen binding construct of the invention per ml of Ringer's solution. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania. For the preparation of intravenously administrable antigen binding construct formulations of the invention see Lasmar U and Parkins D "The formulation of Biopharmaceutical products", Pharma. Sci.Tech.today, page 129-137, Vol.3 (3rd April 2000), Wang, W "Instability, stabilisation and formulation of liquid protein pharmaceuticals", Int. J. Pharm 185 (1999) 129-188, Stability of Protein Pharmaceuticals Part A and B ed Ahern T.J., Manning M.C., New York, NY: Plenum Press (1992), Akers,M.J. "Excipient-Drug interactions in Parenteral Formulations", J.Pharm Sci 91 (2002) 2283-2300, Imamura, K et al "Effects of types of sugar on stabilization of Protein in the dried state", J Pharm Sci 92 (2003) 266-274,Izutsu, Kkojima, S. "Excipient crystalinity and its protein-structure-stabilizing effect during freeze-drying", J Pharm. Pharmacol, 54 (2002) 1033-1039, Johnson, R, "Mannitol-sucrose mixtures-versatile formulations for protein lyophilization", J. Pharm. Sci, 91 (2002) 914-922.

Ha,E Wang W, Wang Y.j. "Peroxide formation in polysorbate 80 and protein stability", J. Pharm Sci, 91, 2252-2264,(2002) the entire contents of which are incorporated herein by reference and to which the reader is specifically referred.

It is preferred that the therapeutic agent of the invention, when in a pharmaceutical preparation, be present in

unit dose forms. The appropriate therapeutically effective dose will be determined readily by those of skill in the art. Suitable doses may be calculated for patients according to their weight, for example suitable doses may be in the range of 0.01 to 20mg/kg, for example 0.1 to 20mg/kg, for example 1 to 20mg/kg, for example 10 to 20mg/kg or for example 1 to 15mg/kg, for example 10 to 15mg/kg. To effectively treat conditions of use in the present invention in a human, suitable doses may be within the range of 0.01 to 1000 mg, for example 0.1 to 1000mg, for example 0.1 to 500mg, for example 500mg, for example 0.1 to 100mg, or 0.1 to 80mg, or 0.1 to 60mg, or 0.1 to 40mg, or for example 1 to 100mg, or 1 to 50mg, of an antigen binding construct of this invention, which may be administered parenterally, for example subcutaneously, intravenously or intramuscularly. Such dose may, if necessary, be repeated at appropriate time intervals selected as appropriate by a physician.

The antigen binding constructs described herein can be lyophilized for storage and reconstituted in a suitable carrier prior to use. This technique has been shown to be effective with conventional immunoglobulins and art-known lyophilization and reconstitution techniques can be employed.

There are several methods known in the art which can be used to find epitope-binding domains of use in the present invention.

The term "library" refers to a mixture of heterogeneous polypeptides or nucleic acids. The library is composed of members, each of which has a single polypeptide or nucleic acid sequence. To this extent, "library" is synonymous with "repertoire." Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. In one example, each individual organism or cell contains only one or a limited number of library members. Advantageously, the nucleic acids are incorporated into expression vectors, in order to allow expression of the polypeptides encoded by the nucleic acids. In a one aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of diverse polypeptides. A "universal framework" is a single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. There may be a single framework, or a set of such frameworks, which has been found to permit the derivation of virtually any binding specificity though variation in the hypervariable regions alone.

Amino acid and nucleotide sequence alignments and homology, similarity or identity, as defined herein are in one embodiment prepared and determined using the algorithm BLAST 2 Sequences, using default parameters (Tatusova, T. A. et al., FEMS Microbiol Lett, 174:187-188 (1999)).

The epitope binding domain(s) and antigen binding sites can each have binding specificity for a generic ligand or any desired target ligand, such as human or animal proteins, including cytokines, growth factors, cytokine receptors, growth factor receptors, enzymes (*e.g.*, proteases), co-factors for enzymes, DNA binding proteins, lipids and carbohydrates. Suitable targets, including cytokines, growth factors, cytokine receptors, growth factor receptors and other proteins include but are not limited to: ApoE, Apo-SAA, BDNF, Cardiotrophin-1, CEA, CD40, CD40 Ligand, CD56, CD38, CD138, EGF, EGF receptor, ENA-78, Eotaxin, Eotaxin-2, Exodus-2, FAP$\alpha$, FGF-acidic, FGF-basic, fibroblast growth factor-10, FLT3 ligand, Fractalkine (CX3C), GDNF, G-CSF, GM-CSF, GF-$\beta$1, human serum albumin, insulin, IFN-$\gamma$, IGF-I, IGF-II, IL-1$\alpha$, IL-1$\beta$, IL-1 receptor, IL-1 receptor type 1, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin $\alpha$, Inhibin $\beta$, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, c-fms, v-fmsMDC (67 a.a.), MDC (69 a.a.), MCP-1 (MCAF), MCP-2, MCP-3, MCP-4, MDC (67 a.a.), MDC (69 a.a.), MIG, MIP-1$\alpha$, MIP-1$\beta$, MIP-3$\alpha$, MIP-3$\beta$, MIP-4, myeloid progenitor inhibitor factor-1 (MPIF-1), NAP-2, Neurturin, Nerve growth factor, $\beta$-NGF, NT-3, NT-4, Oncostatin M, PDGF-AA, PDGF-AB, PDGF-BB, PF-4, RANTES, SDF1$\alpha$, SDF1$\beta$, SCF, SCGF, stem cell factor (SCF), TARC, TGF-$\alpha$, TGF-$\beta$, TGF-$\beta$2, TGF-$\beta$3, tumour necrosis factor (TNF), TNF-$\alpha$, TNF-$\beta$, TNF receptor I, TNF receptor II, TNIL-1, TPO, VEGF, VEGF A, VEGF B, VEGF C, VEGF D, VEGF receptor 1, VEGF receptor 2, VEGF receptor 3, GCP-2, GRO/MGSA, GRO-$\beta$, GRO-$\gamma$, HCC1, 1-309, HER 1, HER 2, HER 3, HER 4, serum albumin, vWF, amyloid proteins (*e.g.*, amyloid alpha), MMP12, PDK1, IgE, and other targets disclosed herein. It will be appreciated that this list is by no means exhaustive.

In some embodiments, binding is to a target in pulmonary tissue, such as a target selected from the group consisting of TNFR1, IL-1, IL-1R, IL-4, IL-4R, IL-5, IL-6, IL-6R, IL-8, IL-8R, IL-9, IL-9R, IL-10, IL-12 IL-12R, IL-13, IL-13R$\alpha$1, IL-13Ra2, IL-15, IL-15R, IL-16, IL-17R, IL-17, IL-18, IL-18R, IL-23 IL-23R, IL-25, CD2, CD4, CD11a, CD23, CD25, CD27, CD28, CD30, CD40, CD40L, CD56, CD138, ALK5, EGFR, FcER1, TGFb, CCL2, CCL18, CEA, CR8, CTGF, CXCL12 (SDF-1), chymase, FGF, Furin, Endothelin-1, Eotaxins (*e.g.*, Eotaxin, Eotaxin-2, Eotaxin-3), GM-CSF, ICAM-1, ICOS, IgE, IFNa, I-309, integrins, L-selectin, MIF, MIP4, MDC, MCP-1, MMPs, neutrophil elastase, osteopontin, OX-40, PARC, PD-1, RANTES, SCF, SDF-1, siglec8, TARC, TGFb, Thrombin, Tim-1, TNF, TRANCE, Tryptase, VEGF,

VLA-4, VCAM, α4β7, CCR2, CCR3, CCR4, CCR5, CCR7, CCR8, alphavbeta6, alphavbeta8, cMET, CD8, vWF, amyloid proteins (*e.g.*, amyloid alpha), MMP12, PDK1, and IgE. When a display system (*e.g.*, a display system that links coding function of a nucleic acid and functional characteristics of the peptide or polypeptide encoded by the nucleic acid) is used in the methods described herein, eg in the selection of a dAb or other epitope binding domain, it is frequently advantageous to amplify or increase the copy number of the nucleic acids that encode the selected peptides or polypeptides. This provides an efficient way of obtaining sufficient quantities of nucleic acids and/or peptides or polypeptides for additional rounds of selection, using the methods described herein or other suitable methods, or for preparing additional repertoires *(e.g.,* affinity maturation repertoires). Thus, in some embodiments, the methods of selecting epitope binding domains comprises using a display system (*e.g.*, that links coding function of a nucleic acid and functional characteristics of the peptide or polypeptide encoded by the nucleic acid, such as phage display) and further comprises amplifying or increasing the copy number of a nucleic acid that encodes a selected peptide or polypeptide. Nucleic acids can be amplified using any suitable methods, such as by phage amplification, cell growth or polymerase chain reaction.

In one example, the methods employ a display system that links the coding function of a nucleic acid and physical, chemical and/or functional characteristics of the polypeptide encoded by the nucleic acid. Such a display system can comprise a plurality of replicable genetic packages, such as bacteriophage or cells (bacteria). The display system may comprise a library, such as a bacteriophage display library. Bacteriophage display is an example of a display system. A number of suitable bacteriophage display systems (*e.g.*, monovalent display and multivalent display systems) have been described. (See, *e.g.,* Griffiths et al., U.S. Patent No. 6,555,313 B1 (incorporated herein by reference); Johnson et al., U.S. Patent No. 5,733,743 (incorporated herein by reference); McCafferty et al., U.S. Patent No. 5,969,108 (incorporated herein by reference); Mulligan-Kehoe, U.S. Patent No. 5,702,892 (Incorporated herein by reference); Winter, G. et al., Annu. Rev. Immunol. 12:433-455 (1994); Soumillion, P. et al., Appl. Biochem. Biotechnol. 47(2-3): 175-189 (1994); Castagnoli, L. et al., Comb. Chem. High Throughput Screen, 4(2):121-133 (2001).) The peptides or polypeptides displayed in a bacteriophage display system can be displayed on any suitable bacteriophage, such as a filamentous phage (*e.g.*, fd, M13, F1), a lytic phage (*e.g.*, T4, T7, lambda), or an RNA phage (*e.g.*, MS2), for example.

Generally, a library of phage that displays a repertoire of peptides or phagepolypeptides, as fusion proteins with a suitable phage coat protein (*e.g.*, fd pIII protein), is produced or provided. The fusion protein can display the peptides or polypeptides at the tip of the phage coat protein, or if desired at an internal position. For example, the displayed peptide or polypeptide can be present at a position that is amino-terminal to domain 1 of pIII. (Domain 1 of pIII is also referred to as N1.) The displayed polypeptide can be directly fused to pIII (*e.g.*, the N-terminus of domain 1 of pIII) or fused to pIII using a linker. If desired, the fusion can further comprise a tag (*e.g.*, myc epitope, His tag). Libraries that comprise a repertoire of peptides or polypeptides that are displayed as fusion proteins with a phage coat protein, can be produced using any suitable methods, such as by introducing a library of phage vectors or phagemid vectors encoding the displayed peptides or polypeptides into suitable host bacteria, and culturing the resulting bacteria to produce phage (*e.g.*, using a suitable helper phage or complementing plasmid if desired). The library of phage can be recovered from the culture using any suitable method, such as precipitation and centrifugation.

The display system can comprise a repertoire of peptides or polypeptides that contains any desired amount of diversity. For example, the repertoire can contain peptides or polypeptides that have amino acid sequences that correspond to naturally occurring polypeptides expressed by an organism, group of organisms, desired tissue or desired cell type, or can contain peptides or polypeptides that have random or randomized amino acid sequences. If desired, the polypeptides can share a common core or scaffold. For example, all polypeptides in the repertoire or library can be based on a scaffold selected from protein A, protein L, protein G, a fibronectin domain, an anticalin, CTLA4, a desired enzyme (*e.g.*, a polymerase, a cellulase), or a polypeptide from the immunoglobulin superfamily, such as an antibody or antibody fragment (*e.g.*, an antibody variable domain). The polypeptides in such a repertoire or library can comprise defined regions of random or randomized amino acid sequence and regions of common amino acid sequence. In certain embodiments, all or substantially all polypeptides in a repertoire are of a desired type, such as a desired enzyme (*e.g.*, a polymerase) or a desired antigen-binding fragment of an antibody (*e.g.*, human $V_H$ or human $V_L$). In some embodiments, the polypeptide display system comprises a repertoire of polypeptides wherein each polypeptide comprises an antibody variable domain. For example, each polypeptide in the repertoire can contain a $V_H$, a $V_L$ or an Fv (*e.g.*, a single chain Fv). Amino acid sequence diversity can be introduced into any desired region of a peptide or polypeptide or scaffold using any suitable method. For example, amino acid sequence diversity can be introduced into a target region, such as a complementarity determining region of an antibody variable domain or a hydrophobic domain, by preparing a library of nucleic acids that encode the diversified polypeptides using any suitable mutagenesis methods (*e.g.*, low fidelity PCR, oligonucleotide-mediated or site directed mutagenesis, diversification using NNK codons) or any other suitable method. If desired, a region of a polypeptide to be diversified can be randomized. The size of the polypeptides that make up the repertoire is largely a matter of choice and uniform polypeptide size is not required. The polypeptides in the repertoire may have at least tertiary structure (form at least one domain).

Selection/Isolation/Recovery

An epitope binding domain or population of domains can be selected, isolated and/or recovered from a repertoire or library (*e.g.*, in a display system) using any suitable method. For example, a domain is selected or isolated based on a selectable characteristic (*e.g.*, physical characteristic, chemical characteristic, functional characteristic). Suitable selectable functional characteristics include biological activities of the peptides or polypeptides in the repertoire, for example, binding to a generic ligand (*e.g.*, a superantigen), binding to a target ligand (*e.g.*, an antigen, an epitope, a substrate), binding to an antibody (*e.g.*, through an epitope expressed on a peptide or polypeptide), and catalytic activity. (See, *e.g.,* Tomlinson et al., WO 99/20749; WO 01/57065; WO 99/58655.)

In some embodiments, the protease resistant peptide or polypeptide is selected and/or isolated from a library or repertoire of peptides or polypeptides in which substantially all domains share a common selectable feature. For example, the domain can be selected from a library or repertoire in which substantially all domains bind a common generic ligand, bind a common target ligand, bind (or are bound by) a common antibody, or possess a common catalytic activity. This type of selection is particularly useful for preparing a repertoire of domains that are based on a parental peptide or polypeptide that has a desired biological activity, for example, when performing affinity maturation of an immunoglobulin single variable domain.

Selection based on binding to a common generic ligand can yield a collection or population of domains that contain all or substantially all of the domains that were components of the original library or repertoire. For example, domains that bind a target ligand or a generic ligand, such as protein A, protein L or an antibody, can be selected, isolated and/or recovered by panning or using a suitable affinity matrix. Panning can be accomplished by adding a solution of ligand (*e.g.*, generic ligand, target ligand) to a suitable vessel (*e.g.*, tube, petri dish) and allowing the ligand to become deposited or coated onto the walls of the vessel. Excess ligand can be washed away and domains can be added to the vessel and the vessel maintained under conditions suitable for peptides or polypeptides to bind the immobilized ligand. Unbound domains can be washed away and bound domains can be recovered using any suitable method, such as scraping or lowering the pH, for example.

Suitable ligand affinity matrices generally contain a solid support or bead (*e.g.*, agarose) to which a ligand is covalently or noncovalently attached. The affinity matrix can be combined with peptides or polypeptides (*e.g.*, a repertoire that has been incubated with protease) using a batch process, a column process or any other suitable process under conditions suitable for binding of domains to the ligand on the matrix. domains that do not bind the affinity matrix can be washed away and bound domains can be eluted and recovered using any suitable method, such as elution with a lower pH buffer, with a mild denaturing agent (*e.g.*, urea), or with a peptide or domain that competes for binding to the ligand. In one example, a biotinylated target ligand is combined with a repertoire under conditions suitable for domains in the repertoire to bind the target ligand. Bound domains are recovered using immobilized avidin or streptavidin (*e.g.*, on a bead).

In some embodiments, the generic or target ligand is an antibody or antigen binding fragment thereof. Antibodies or antigen binding fragments that bind structural features of peptides or polypeptides that are substantially conserved in the peptides or polypeptides of a library or repertoire are particularly useful as generic ligands. Antibodies and antigen binding fragments suitable for use as ligands for isolating, selecting and/or recovering protease resistant peptides or polypeptides can be monoclonal or polyclonal and can be prepared using any suitable method.

LIBRARIES/REPERTOIRES

Libraries that encode and/or contain protease epitope binding domains can be prepared or obtained using any suitable method. A library can be designed to encode domains based on a domain or scaffold of interest (*e.g.*, a domain selected from a library) or can be selected from another library using the methods described herein. For example, a library enriched in domains can be prepared using a suitable polypeptide display system.

Libraries that encode a repertoire of a desired type of domain can readily be produced using any suitable method. For example, a nucleic acid sequence that encodes a desired type of polypeptide (*e.g.*, an immunoglobulin variable domain) can be obtained and a collection of nucleic acids that each contain one or more mutations can be prepared, for example by amplifying the nucleic acid using an error-prone polymerase chain reaction (PCR) system, by chemical mutagenesis (Deng et al., J. Biol. Chem., 269:9533 (1994)) or using bacterial mutator strains (Low et al., J. Mol. Biol., 260:359 (1996)).

In other embodiments, particular regions of the nucleic acid can be targeted for diversification. Methods for mutating selected positions are also well known in the art and include, for example, the use of mismatched oligonucleotides or degenerate oligonucleotides, with or without the use of PCR. For example, synthetic antibody libraries have been created by targeting mutations to the antigen binding loops. Random or semi-random antibody H3 and L3 regions have been appended to germline immunoblulin V gene segments to produce large libraries with unmutated framework regions (Hoogenboom and Winter (1992) supra; Nissim *et al.* (1994) supra; Griffiths *et al*. (1994) supra; DeKruif *et al.* (1995)

supra). Such diversification has been extended to include some or all of the other antigen binding loops (Crameri et al. (1996) Nature Med., 2:100; Riechmann et al. (1995) Bio/Technology, 13:475; Morphosys, WO 97/08320, supra). In other embodiments, particular regions of the nucleic acid can be targeted for diversification by, for example, a two-step PCR strategy employing the product of the first PCR as a "mega-primer." (See, *e.g.,* Landt, O. et al., Gene 96:125-128 (1990).) Targeted diversification can also be accomplished, for example, by SOE PCR. (See, *e.g.,* Horton, R.M. et al., Gene 77: 61-68 (1989).)

Sequence diversity at selected positions can be achieved by altering the coding sequence which specifies the sequence of the polypeptide such that a number of possible amino acids (*e.g.*, all 20 or a subset thereof) can be incorporated at that position. Using the IUPAC nomenclature, the most versatile codon is NNK, which encodes all amino acids as well as the TAG stop codon. The NNK codon may be used in order to introduce the required diversity. Other codons which achieve the same ends are also of use, including the NNN codon, which leads to the production of the additional stop codons TGA and TAA. Such a targeted approach can allow the full sequence space in a target area to be explored.

Some libraries comprise domains that are members of the immunoglobulin superfamily (*e.g.*, antibodies or portions thereof). For example the libraries can comprise domains that have a known main-chain conformation. (See, *e.g.,* Tomlinson et al., WO 99/20749.) Libraries can be prepared in a suitable plasmid or vector. As used herein, vector refers to a discrete element that is used to introduce heterologous DNA into cells for the expression and/or replication thereof. Any suitable vector can be used, including plasmids (*e.g.*, bacterial plasmids), viral or bacteriophage vectors, artificial chromosomes and episomal vectors. Such vectors may be used for simple cloning and mutagenesis, or an expression vector can be used to drive expression of the library. Vectors and plasmids usually contain one or more cloning sites (*e.g.*, a polylinker), an origin of replication and at least one selectable marker gene. Expression vectors can further contain elements to drive transcription and translation of a polypeptide, such as an enhancer element, promoter, transcription termination signal, signal sequences, and the like. These elements can be arranged in such a way as to be operably linked to a cloned insert encoding a polypeptide, such that the polypeptide is expressed and produced when such an expression vector is maintained under conditions suitable for expression (*e.g.*, in a suitable host cell).

Cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (*e.g.* SV40, adenovirus) are useful for cloning vectors in mammalian cells. Generally, the origin of replication is not needed for mammalian expression vectors, unless these are used in mammalian cells able to replicate high levels of DNA, such as COS cells.

Cloning or expression vectors can contain a selection gene also referred to as selectable marker. Such marker genes encode a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, *e.g.* ampicillin, neomycin, methotrexate or tetracycline, complement auxotrophic deficiencies, or supply critical nutrients not available in the growth media.

Suitable expression vectors can contain a number of components, for example, an origin of replication, a selectable marker gene, one or more expression control elements, such as a transcription control element (*e.g.*, promoter, enhancer, terminator) and/or one or more translation signals, a signal sequence or leader sequence, and the like. Expression control elements and a signal or leader sequence, if present, can be provided by the vector or other source. For example, the transcriptional and/or translational control sequences of a cloned nucleic acid encoding an antibody chain can be used to direct expression.

A promoter can be provided for expression in a desired host cell. Promoters can be constitutive or inducible. For example, a promoter can be operably linked to a nucleic acid encoding an antibody, antibody chain or portion thereof, such that it directs transcription of the nucleic acid. A variety of suitable promoters for procaryotic (*e.g.*, the β-lactamase and lactose promoter systems, alkaline phosphatase, the tryptophan (trp) promoter system, lac, tac, T3, T7 promoters for *E. coli*) and eucaryotic (*e.g.*, simian virus 40 early or late promoter, Rous sarcoma virus long terminal repeat promoter, cytomegalovirus promoter, adenovirus late promoter, EG-1a promoter) hosts are available.

In addition, expression vectors typically comprise a selectable marker for selection of host cells carrying the vector, and, in the case of a replicable expression vector, an origin of replication. Genes encoding products which confer antibiotic or drug resistance are common selectable markers and may be used in procaryotic (*e.g.,* β-lactamase gene (ampicillin resistance), *Tet* gene for tetracycline resistance) and eucaryotic cells (*e.g.,* neomycin (G418 or geneticin), gpt (mycophenolic acid), ampicillin, or hygromycin resistance genes). Dihydrofolate reductase marker genes permit selection with methotrexate in a variety of hosts. Genes encoding the gene product of auxotrophic markers of the host

(*e.g., LEU2, URA3, HIS3*) are often used as selectable markers in yeast. Use of viral (*e.g.,* baculovirus) or phage vectors, and vectors which are capable of integrating into the genome of the host cell, such as retroviral vectors, are also contemplated.

Suitable expression vectors for expression in prokaryotic (*e.g.,* bacterial cells such as *E. coli*) or mammalian cells include, for example, a pET vector (*e.g.,* pET-12a, pET-36, pET-37, pET-39, pET-40, Novagen and others), a phage vector (*e.g.,* pCANTAB 5 E, Pharmacia), pRIT2T (Protein A fusion vector, Pharmacia), pCDM8, pCDNA1.1/amp, pcDNA3.1, pRc/RSV, pEF-1 (Invitrogen, Carlsbad, CA), pCMV-SCRIPT, pFB, pSG5, pXT1 (Stratagene, La Jolla, CA), pCDEF3 (Goldman, L.A., et al., Biotechniques, 21:1013-1015 (1996)), pSVSPORT (GibcoBRL, Rockville, MD), pEF-Bos (Mizushima, S., et al., Nucleic Acids Res., 18:5322 (1990)) and the like. Expression vectors which are suitable for use in various expression hosts, such as prokaryotic cells *(E. coli*), insect cells (*Drosophila* Schnieder S2 cells, Sf9), yeast (*P. methanolica, P. pastoris, S. cerevisiae*) and mammalian cells (eg, COS cells) are available.

Some examples of vectors are expression vectors that enable the expression of a nucleotide sequence corresponding to a polypeptide library member. Thus, selection with generic and/or target ligands can be performed by separate propagation and expression of a single clone expressing the polypeptide library member. As described above, a particular selection display system is bacteriophage display. Thus, phage or phagemid vectors may be used, for example vectors may be phagemid vectors which have an *E. coli.* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector can contain a β-lactamase gene to confer selectivity on the phagemid and a lac promoter upstream of an expression cassette that can contain a suitable leader sequence, a multiple cloning site, one or more peptide tags, one or more TAG stop codons and the phage protein pIII. Thus, using various suppressor and non-suppressor strains of *E coli* and with the addition of glucose, iso-propyl thio-β-D-galactoside (IPTG) or a helper phage, such as VCS M13, the vector is able to replicate as a plasmid with no expression, produce large quantities of the polypeptide library member only or product phage, some of which contain at least one copy of the polypeptide-pIII fusion on their surface.

Antibody variable domains may comprise a target ligand binding site and/or a generic ligand binding site. In certain embodiments, the generic ligand binding site is a binding site for a superantigen, such as protein A, protein L or protein G. The variable domains can be based on any desired variable domain, for example a human $V_H$ (*e.g.,* $V_H$ 1a, $V_H$ 1b, $V_H$ 2, $V_H$ 3, $V_H$ 4, $V_H$ 5, $V_H$ 6), a human Vλ (*e.g.*, VλI, VλII, VλIII, VλIV, VλV, VλVI or Vλ1) or a human Vκ (*e.g.*, Vκ2, Vκ3, Vκ4, Vκ5, Vκ6, Vκ7, Vκ8, Vκ9 or Vκ10).

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

<u>Characterisation of the epitope binding domains.</u>

The binding of a domain to its specific antigen or epitope can be tested by methods which will be familiar to those skilled in the art and include ELISA. In one example, binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein.

The diversity of the selected phage monoclonal antibodies may also be assessed by gel electrophoresis of PCR products (Marks *et al.* 1991, *supra;* Nissim *et al.* 1994 *supra),* probing (Tomlinson et al., 1992) J. Mol. Biol. 227, 776) or by sequencing of the vector DNA.

E. <u>Structure of dAbs</u>

In the case that the dAbs are selected from V-gene repertoires selected for instance using phage display technology as herein described, then these variable domains comprise a universal framework region, such that is they may be recognised by a specific generic ligand as herein defined. The use of universal frameworks, generic ligands and

the like is described in WO99/20749.

Where V-gene repertoires are used variation in polypeptide sequence may be located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, 1994, Nature 370: 389-391 and U.S. Patent No. 6,297,053, both of which are incorporated herein by reference. Other methods of mutagenesis are well known to those of skill in the art.

Scaffolds for use in Constructing dAbs

i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main-chain conformations of H1, H2, L1, L2 and L3 found in the majority of human antibodies (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1).

The dAbs are advantageously assembled from libraries of domains, such as libraries of $V_H$ domains and/or libraries of $V_L$ domains. In one aspect, libraries of domains are designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human $V_\kappa$ domain, the L1 loop can adopt one of four canonical structures, the L2 loop has a single canonical structure and that 90% of human $V_\kappa$ domains adopt one of four or five canonical structures for the L3 loop (Tomlinson *et al.* (1995) supra); thus, in the $V_\kappa$ domain alone, different canonical structures can combine to create a range of different main-chain conformations. Given that the $V_\lambda$ domain encodes a different range of canonical structures for the L1, L2 and L3 loops and that $V_\kappa$ and $V_\lambda$ domains can pair with any $V_H$ domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a one particular aspect, the dAbs possess a single known main-chain conformation.

The single main-chain conformation that is chosen may be commonplace among molecules of the immunoglobulin superfamily type in question. A conformation is commonplace when a significant number of naturally occurring molecules are observed to adopt it. Accordingly, in one aspect, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. The desired combination of main-chain conformations for the different loops may be created by selecting germline gene segments which encode the desired main-chain conformations. In one example, the selected germline gene segments are frequently expressed in nature, and in particular they may be the most frequently expressed of all natural germline gene segments.

In designing libraries the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3

loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of $V_\kappa$(39%), L2 - CS 1 (100%), L3 - CS 1 of $V_\kappa$(36%) (calculation assumes a $\kappa$:$\lambda$ ratio of 70:30, Hood et al. (1967) Cold Spring Harbor Symp. Quant. Biol., 48: 133). For H3 loops that have canonical structures, a CDR3 length (Kabat et al. (1991) Sequences of proteins of immunological interest, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the $V_H$ segment 3-23 (DP-47), the $J_H$ segment JH4b, the $V_\kappa$ segment 02/O12 (DPK9) and the $J_\kappa$ segment $J_\kappa$1. $V_H$ segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five, or for all six of the antigen binding loops can be determined. Here, the chosen conformation may be commonplace in naturally occurring antibodies and may be observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

Diversification of the canonical sequence

Having selected several known main-chain conformations or a single known main-chain conformation, dAbs can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or they may be selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) J. Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomisation has the potential to create approximately more than $10^{15}$ structures for H3 alone and a similarly large number of variants for the other five loops, it is not feasible using current transformation technology or even by using cell free systems to produce a library representing all possible combinations. For example, in one of the largest libraries constructed to date, 6 x $10^{10}$ different antibodies, which is only a fraction of the potential diversity for a library of this design, were generated (Griffiths *et al.* (1994) supra).

In a one embodiment, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

In one aspect, libraries of dAbs are used in which only those residues in the antigen binding site are varied.

These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library.. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" or "dummy" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

It will be understood that the sequences described herein include sequences which are substantially identical, for example sequences which are at least 90% identical, for example which are at least 91%, or at least 92%, or at least 93%, or at least 94% or at least 95%, or at least 96%, or at least 97% or at least 98%, or at least 99% identical to the sequences described herein. For nucleic acids, the term "substantial identity" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial identity exists when the segments will hybridize under selective hybridization conditions, to the complement of the strand.

For nucleotide and amino acid sequences, the term "identical" indicates the degree of identity between two nucleic acid or amino acid sequences when optimally aligned and compared with appropriate insertions or deletions. Alternatively, substantial identity exists when the DNA segments will hybridize under selective hybridization conditions, to the complement of the strand.

The percent identity between two sequences is a function of the number of identical positions shared by the sequences (i.e., % identity = # of identical positions/total # of positions times 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package, using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6.

By way of example, a polynucleotide sequence of the present invention may be identical to the reference sequence of SEQ ID NO: 122, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO: 122 by the numerical percent of the respective percent identity(divided by 100) and subtracting that product from said total number of nucleotides in SEQ ID NO: 122, or:

$$nn \leq xn - (xn \bullet y),$$

wherein nn is the number of nucleotide alterations, xn is the total number of nucleotides in SEQ ID NO: 122, and y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and wherein any non-integer product of xn and y is rounded down to the nearest integer prior to subtracting it from xn. Alterations of the polynucleotide sequence of SEQ ID NO: 122 may create nonsense, missense or frameshift mutations in this coding sequence and thereby alter the polypeptide encoded by the polynucleotide following such alterations.

Similarly, in another example, a polypeptide sequence of the present invention may be identical to the reference sequence encoded by SEQ ID NO: 26, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the polypeptide sequence encoded by SEQ ID NO: 26 by the numerical percent of the respective percent identity (divided by 100) and then subtracting that product from said total number of amino acids in the polypeptide sequence encoded by SEQ ID NO: 26, or:

$$na \leq xa - (xa \bullet y),$$

wherein na is the number of amino acid alterations, xa is the total number of amino acids in the polypeptide sequence encoded by SEQ ID NO: 26, and y is, for instance 0.70 for 70%, 0.80 for 80%, 0.85 for 85% etc., and wherein any non-integer product of xa and y is rounded down to the nearest integer prior to subtracting it from xa.

The invention may further be described as in the following numbered sentences:

1. An antigen-binding construct comprising a protein scaffold which is linked to one or more epitope-binding domains wherein the antigen-binding construct has at least two antigen binding sites at least one of which is from an epitope binding domain and at least one of which is from a paired VH/VL domain.
2. An antigen-binding construct comprising at least one homodimer comprising two or more structures of formula I:

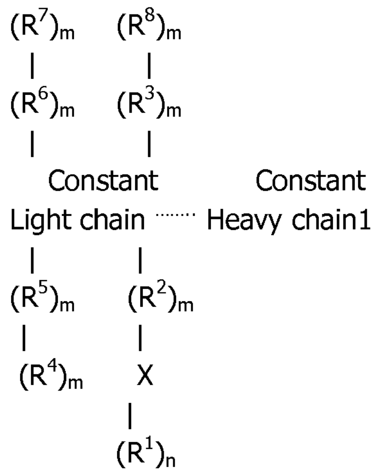


(I)

wherein

X represents a constant antibody region comprising constant heavy domain 2 and constant heavy domain 3;

$R^1$, $R^4$ , $R^7$ and $R^8$ represent a domain independently selected from an epitope-binding domain;

$R^2$ represents a domain selected from the group consisting of constant heavy chain 1, and an epitope-binding domain;

$R^3$ represents a domain selected from the group consisting of a paired VH and an epitope-binding domain;

$R^5$ represents a domain selected from the group consisting of constant light chain, and an epitope-binding domain;
$R^6$ represents a domain selected from the group consisting of a paired VL and an epitope-binding domain;
n represents an integer independently selected from: 0, 1, 2, 3 and 4;
m represents an integer independently selected from: 0 and 1,
wherein the Constant Heavy chain 1 and the Constant Light chain domains are associated; wherein at least one epitope binding domain is present;
and when $R^3$ represents a paired VH domain, $R^6$ represents a paired VL domain, so that the two domains are together capable of binding antigen.

3. An antigen-binding construct according to sentence 2 wherein and $R^6$ represents a paired VL and $R^3$ represents a paired VH.

4. An antigen-binding construct according to sentence 3 wherein either one or both of $R^7$ and $R^8$ represent an epitope binding domain.

5. An antigen-binding construct according to any one of claims 2 to 4 wherein either one or both of $R^1$ and $R^4$ represent an epitope binding domain.

6. An antigen-binding construct according to any one of sentences 2 to 4 wherein $R^4$ is present.

7. An antigen-binding construct according to any one of sentences 2 to 6 wherein $R^1$ $R^7$ and $R^8$ represent an epitope binding domain.

8. An antigen-binding construct according to any one of sentences 2 to 6 wherein $R^1$ $R^7$ and $R^8$, and $R^4$ represent an epitope binding domain.

9. An antigen-binding construct according to any preceding sentence wherein at least one epitope binding domain is a dAb.

10. An antigen-binding construct according to sentence 9 wherein the dAb is a human dAb.

11. An antigen-binding construct according to sentence 9 wherein the dAb is a camelid dAb.

12. An antigen-binding construct according to sentence 9 wherein the dAb is a shark dAb (NARV).

13. An antigen-binding construct according to any one of sentences 1 to 8 wherein at least one epitope binding domain is derived from a scaffold selected from CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human $\gamma$-crystallin and human ubiquitin (affilins); PDZ domains; scorpion toxinkunitz type domains of human protease inhibitors; and fibronectin (adnectin).

14. An antigen-binding construct according to sentence 13 wherein the epitope binding domain is derived from a scaffold selected from an Affibody, an ankyrin repeat protein (DARPin) and an adnectin.

15. An antigen-binding construct according to any one of sentences 1 to 8 wherein the epitope binding domain is selected from a dAb, an Affibody, an ankyrin repeat protein (DARPin) and an adnectin.

16. An antigen-binding construct of any preceding sentence wherein the binding construct has specificity for more than one antigen.

17. An antigen-binding construct according to any preceding sentence wherein the first binding site has specificity for a first epitope on an antigen and the second binding site has specificity for a second epitope on the same antigen.

18. An antigen-binding construct according to any preceding sentence wherein the antigen-binding construct is capable of binding IL-13.

19. An antigen-binding construct according to any preceding sentence wherein the antigen-binding construct is capable of binding two or more antigens selected from IL-13, IL-5, and IL-4.

20. An antigen-binding construct according sentence 19 wherein the antigen-binding construct is capable of binding IL-13 and IL-4 simultaneously.

21. An antigen-binding construct according to any preceding sentence wherein the antigen-binding construct is capable of binding two or more antigens selected from VEGF, IGF-1R and EGFR.

22. An antigen-binding construct according to any preceding sentence wherein the antigen-binding construct is capable of binding TNF.

23. An antigen-binding construct according to sentence 22 wherein the antigen-binding construct is capable of binding to TNF and IL1-R.

24. An antigen-binding construct according to any one of sentence 1 or sentences 9 to 23 wherein the protein scaffold is an Ig scaffold.

25. An antigen-binding construct according to sentence 24 wherein the Ig scaffold is an IgG scaffold.

26. An antigen-binding construct according to sentence 25 wherein the IgG scaffold is selected from IgG1, IgG2, IgG3 and IgG4.

27. An antigen-binding construct according to any one of sentence 1 or sentences 9 to 26 wherein the protein scaffold comprises a monovalent antibody.

28. An antigen-binding construct according to any one of sentences 25 to 27 wherein the IgG scaffold comprises

all the domains of an antibody.

29. An antigen-binding construct according to any one of sentences 9 to 12 or 15 to 28 which comprises four domain antibodies.

30. An antigen-binding construct according to sentence 29 wherein two of the domain antibodies have specificity for the same antigen.

31. An antigen-binding construct according to sentence 29 wherein all of the domain antibodies have specificity for the same antigen.

32. An antigen-binding construct according to any preceding sentence wherein at least one of the single variable domains is directly attached to the Ig scaffold with a linker comprising from 1 to 150 amino acids.

33. An antigen-binding construct according to sentence 32 wherein at least one of the single variable domains is directly attached to the Ig scaffold with a linker comprising from 1 to 20 amino acids.

34. An antigen-binding construct according to sentence 33 wherein at least one of the epitope binding domains is directly attached to the Ig scaffold with a linker selected from any one of those set out in SEQ ID NO: 6 to 11 or 'GS', or any combination thereof.

35. An antigen-binding construct according to any preceding sentence wherein at least one of the epitope binding domains binds human serum albumin.

36. An antigen-binding construct according to any one of sentences 21 to 33 comprising an epitope binding domain attached to the Ig scaffold at the N-terminus of the light chain.

37. An antigen-binding construct according to any one of sentences 21 to 33 comprising an epitope binding domain attached to the Ig scaffold at the N-terminus of the heavy chain.

38. An antigen-binding construct according to any one of sentences 21 to 33 comprising an epitope binding domain attached to the Ig scaffold at the C-terminus of the light chain.

39. An antigen-binding construct according to any one of sentences 21 to 33 comprising an epitope binding domain attached to the Ig scaffold at the C-terminus of the heavy chain.

40. An antigen-binding construct according to sentence 1 or 2 which has 4 antigen binding sites and which is capable of binding 4 antigens simultaneously.

41. An antigen-binding construct according to any preceding sentence for use in medicine.

42. An antigen-binding construct according to any preceding sentence for use in the manufacture of a medicament for treating cancer or inflammatory diseases such as asthma, rheumatoid arthritis or osteoarthritis.

43. A method of treating a patient suffering from cancer or an inflammatory disease such as asthma, rheumatoid arthritis or osteoarthritis, comprising administering a therapeutic amount of an antigen-binding construct according to any preceding sentence.

44. An antigen-binding construct according to any preceding sentence for the treatment of cancer or inflammatory diseases such as asthma, rheumatoid arthritis or osteoarthritis.

45. A polynucleotide sequence encoding a heavy chain of an antigen binding construct according to any one of sentences 1 to 40.

46. A polynucleotide encoding a light chain of an antigen binding construct according to any one of sentences 1 to 40.

47. A recombinant transformed or transfected host cell comprising one or more polynucleotide sequences encoding a heavy chain and a light chain of an antigen binding construct of any preceding sentence.

48. A method for the production of an antigen binding construct according to sentences 1 to 40 which method comprises the step of culturing a host cell of sentence 47 and isolating the antigen binding construct.

49. A pharmaceutical composition comprising an antigen binding construct of any one of sentences 1 to 38 and a pharmaceutically acceptable carrier.

## Examples

The following methods were used in the examples described herein.

### Method 1

### Binding to E.Coli-expressed recombinant human IL-13 by ELISA

mAbdAb molecules were assessed for binding to recombinant *E.coli*-expressed human IL-13 in a direct binding ELISA. In brief, 5μg/ml recombinant *E.coli*-expressed human IL-13 (made and purified at GSK) was coated to a 96-well ELISA plate. The wells were blocked for 1 hour at room temperature, mAbdAb constructs were then titrated out across the plate (usually from around 100nM in 3-fold dilutions to around 0.01nM). Binding was detected using an appropriate dilution of anti-human kappa light chain peroxidase conjugated antibody (catalogue number A7164, Sigma-Aldrich) or an appropriate dilution of anti-human IgG γ chain specific peroxidase conjugated detection antibody (catalogue number

A6029, Sigma-Aldrich).

**Method 2**

**Binding to E.Coli-expressed recombinant human IL-4 by ELISA**

mAbdAb constructs were assessed for binding to recombinant *E.coli*-expressed human IL-4 in a direct binding ELISA. In brief, 5μg/ml recombinant *E.coli*-expressed human IL-4 (made and purified at GSK) was coated to a 96-well ELISA plate. The wells were blocked for 1 hour at room temperature, mAbdAb constructs were then titrated out across the plate (usually from around 100nM in 3-fold dilutions to around 0.01nM). Binding was detected using an appropriate dilution of goat anti-human kappa light chain peroxidase conjugated antibody (catalogue number A7164, Sigma-Aldrich) or an appropriate dilution of anti-human IgG γ chain specific peroxidase conjugated detection antibody (catalogue number A6029, Sigma-Aldrich).

**Method 3**

**Binding to E.Coli-expressed recombinant human IL-18 by ELISA**

mAbdAb constructs were assessed for binding to recombinant *E.coli*-expressed human IL-18 in a direct binding ELISA. In brief, 5μg/ml recombinant *E.coli*-expressed human IL-18 (made and purified at GSK) was coated to a 96-well ELISA plate. The wells were blocked for 1 hour at room temperature, mAbdAb constructs were then titrated out across the plate (usually from around 100nM in 3-fold dilutions to around 0.01nM). Binding was detected using using a dilution of 1 in 2000 of anti-human kappa light chain peroxidase conjugated antibody (catalogue number A7164, Sigma-Aldrich) or using a dilution of 1 in 2000 of anti-human IgG γ chain specific peroxidase conjugated detection antibody (catalogue number A6029, Sigma-Aldrich).

**Method 4**

**Biacore™ binding affinity assessment for binding to E.Coli-expressed recombinant human IL-13**

The binding affinity of mAbdAb constructs for recombinant *E.Coli*-expressed human IL-13 were assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb constructs were then captured onto this surface and human IL-13 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-13 binding event. The anti-human IgG surface was regenerated either using similar conditions to the Protein A surface or by using 3M $MgCl_2$. The work was carried out on the Biacore™ 3000 and/or the T100 machine, data were analysed using the evaluation software in the machines and fitted to the 1:1 model of binding. Biacore™ runs were carried out at 25°C or 37°C.

**Method 5**

**Biacore™ binding affinity assessment for binding to E.Coli-expressed recombinant human IL-4**

The binding affinity of mAbdAb constructs for recombinant *E.Coli*-expressed human IL-4 were assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb constructs were then captured onto this surface and human IL-4 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-4 binding event. The anti-human IgG surface was regenerated either using similar conditions to the Protein A surface or by using 3M $MgCl_2$. The work was carried out on Biacore™ 3000 and / or the T100 and / or the A100 machine, data were analysed using the evaluation software in the machines and fitted to the 1:1 model of binding. Biacore™ runs were carried out at 25°C or 37°C.

**Method 6**

**Biacore™ binding affinity assessment for binding to E.Coli-expressed recombinant human IL-18**

The binding affinity of mAbdAb constructs for recombinant *E.Coli*-expressed human IL-18 was assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb constructs were then captured onto this surface and human IL-18 (made and purified at GSK) passed over at defined concentrations. The surface was regenerated back to the Protein A surface using mild acid elution conditions(such as 100mM phosphoric acid), this did not significantly affect the ability to capture antibody for a subsequent IL-18 binding event. The anti-human IgG surface was regenerated either using similar conditions to the Protein A surface or by using 3M $MgCl_2$. The work was carried out on Biacore™ 3000 and / or the T100 and / or the A100 machine, data were analysed using the evaluation software in the machines and fitted to the 1:1 model of binding. The Biacore™ run was carried out at 25°C.

**Method 7**

**Stoichiometry assessment of mAbdAb bispecific antibodies or trispecific antibody for IL-13, IL-4 or IL-18 (using Biacore™)**

Anti-human IgG was immobilised onto a CM5 biosensor chip by primary amine coupling. mAbdAb constructs were captured onto this surface after which a single concentration of IL-13, IL-4 or IL-18 cytokine was passed over, this concentration was enough to saturate the binding surface and the binding signal observed reached full R-max. Stoichiometries were then calculated using the given formula:

$$\text{Stoich}=\text{Rmax} * \text{Mw (ligand)} / \text{Mw (analyte)} * \text{R (ligand immobilised or captured)}$$

Where the stoichiometries were calculated for more than one analyte binding at the same time, the different cytokines were passed over sequentially at the saturating cytokine concentration and the stoichometries calculated as above. The work was carried out on the Biacore 3000, at 25°C using HBS-EP running buffer.

**Method 8**

**Neutralisation of E.Coli-expressed recombinant human IL-13 in a TF-1 cell proliferation bioassay**

TF-1 cells proliferate in response to a number of different cytokines including human IL-13. The proliferative response of these cells for IL-13 can therefore be used to measure the bioactivity of IL-13 and subsequently an assay has been developed to determine the IL-13 neutralisation potency (inhibition of IL-13 bioactivity) of mAbdAb constructs.

The assay was performed in sterile 96-well tissue culture plates under sterile conditions and all test wells were performed in triplicate. Approximately 14ng/ml recombinant E.Coli-expressed human IL-13 was pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 100nM titrated in 3-fold dilutions to 0.01nM), recombinant E.Coli-expressed human IL-13 (at a final concentration of 7ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed human IL-13 bioactivity was expressed as that concentration of the mAbdAb construct required to neutralise the bioactivity of the defined amount of human IL-13 (7ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 9**

**Neutralisation of E.Coli-expressed recombinant human IL-4 in a TF-1 cell proliferation bioassay**

TF-1 cells proliferate in response to a number of different cytokines including human IL-4. The proliferative response of these cells for IL-4 can therefore be used to measure the bioactivity of IL-4 and subsequently an assay has been developed to determine the IL-4 neutralisation potency (inhibition of IL-4 bioactivity) of mAbdAb constructs.

The assay was performed in sterile 96-well tissue culture plates under sterile conditions and all test wells were performed in triplicate. Approximately 2.2ng/ml recombinant E.Coli-expressed human IL-4 was pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 100nM titrated in 3-fold dilutions to 0.01nM), recombinant E.Coli-expressed human IL-4 (at a final concentration of 1.1ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed human IL-4 bioactivity was expressed as that concentration of the mAbdAb construct required to neutralise the bioactivity of the defined amount of human IL-4 (1.1ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 10**

**Dual neutralisation of E.Coli-expressed recombinant human IL-13 and E.Coli-expressed recombinant human IL-4 in a TF-1 cell proliferation bioassay**

TF-1 cells proliferate in response to a number of different cytokines including human IL-13 and human IL-4. The proliferative response of these cells for IL-13 and IL-4 can therefore be used to measure the bioactivity of IL-13 and IL-4 simultaneously and subsequently an assay has been developed to determine the dual IL-13 and IL-4 neutralisation potency (dual inhibition of IL-13 and IL-4 bioactivity) of mAbdAb constructs.

The assay was performed in sterile 96-well tissue culture plates under sterile conditions and all test wells were performed in triplicate. Approximately 14ng/ml recombinant E.Coli-expressed human IL-13 and approximately 2.2ng/ml recombinant E.Coli-expressed human IL-4 were pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume, contained various dilutions of mAbdAb constructs (at a final concentration of 100nM titrated in 3-fold dilutions to 0.01nM), recombinant E.Coli-expressed human IL-13 (at a final concentration of 7ng/ml), recombinant E.Coli-expressed human IL-4 (at a final concentration of 1.1ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

**Method 11**

**Biacore™ binding affinity assessment for binding to *Sf21*-expressed recombinant human IL-5**

The binding affinity of mAbdAb molecules for recombinant *Sf21*-expressed human IL-5 was assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb molecules were then captured onto this surface and human IL-5 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-5 binding event. The anti-human IgG surface was regenerated either using similar conditions to the Protein A surface or by using 3M $MgCl_2$. The work was carried out on Biacore™ 3000, T100 and A100 machines, data were analysed using

the evaluation software in the machines and fitted to the 1:1 model of binding. The Biacore™ run was carried out at 25°C.

**Method 12**

**VEGF Receptor Binding Assay.**

This assay measures the binding of $VEGF_{165}$ to VEGF R2 (VEGF receptor) and the ability of test molecules to block this interaction. ELISA plates were coated overnight with VEGF receptor (R&D Systems, Cat No: 357-KD-050) (0.5μg/ml final concentration in 0.2M sodium carbonate bicarbonate pH9.4), washed and blocked with 2% BSA in PBS. VEGF (R&D Systems, Cat No: 293-VE-050) and the test molecules (diluted in 0.1%BSA in 0.05% Tween 20™ PBS) were pre-incubated for one hour prior to addition to the plate (3ng/ml VEGF final concentration). Binding of VEGF to VEGF receptor was detected using biotinylated anti-VEGF antibody (0.5μg/ml final concentration) (R&D Systems, Cat No: BAF293) and a peroxidase conjugated anti-biotin secondary antibody (1:5000 dilution) (Stratech, Cat No: 200-032-096) and visualised at OD450 using a colorimetric substrate (Sure Blue TMB peroxidase substrate, KPL) after stopping the reaction with an equal volume of 1M HCl.

**Method 13**

**EGFR Kinase Assay**

Activation of EGFR expressed on the surface of A431 cells through its interaction with EGF leads to tyrosine kinase phosphorylation of the receptor. Reduction of EGFR tyrosine kinase phosphorylation was measured to determine potency of test molecules. A431 cells were allowed to adhere to 96 well tissue culture plates overnight then the test molecule was added and left for 1 hour and then incubated for 10 min with EGF (at 300ng/ml) (R&D Systems catalogue number 236-EG). The cells were lysed and the lysed preparation transferred to ELISA plates coated with anti-EGFR antibody (at 1ug/ml) (R&D Systems, cat # AF231). Both phosphorylated and non-phosphorylated EGFR present in the lysed cell solution was captured. After washing away unbound material phosphorylated EGFR was specifically detected using a HRP conjugated anti-phosphotyrosine antibody (1:2000 dilution) (Upstate Biotechnology, cat # 16-105). Binding was visualised using a colorimetric substrate.

**Method 14**

**MRC-5/TNF Assay**

The ability of test molecules to prevent human TNF-a binding to human TNFR1 and neutralise IL-8 secretion was determined using human lung fibroblast MRC-5 cells. A dilution series of test samples was incubated with TNF-a (500pg/ml) (Peprotech) for 1 hour. This was then diluted 1 in 2 with a suspension of MRC-5 cells (ATCC, Cat.# CCL-171) ($5x10^3$ cells/well). After an overnight incubation, samples were diluted 1 in 10, and IL-8 release was determined using an IL-8 ABI 8200 cellular detection assay (FMAT) where the IL-8 concentration was determined using anti-IL-8 (R&D systems, Cat# 208-IL) coated polystyrene beads, biotinylated anti-IL-8 (R&D systems, Cat# BAF208) and streptavidin Alexafluor 647 (Molecular Probes, Cat#S32357). The assay readout was localised fluorescence emission at 647nm and unknown IL-8 concentrations were interpolated using an IL-8 standard curve included in the assay.

**Method 15**

**MRC-5/IL-1 Assay.**

The ability of test molecules to prevent human IL-1a binding to human IL1-R and neutralise IL-8 secretion was determined using human lung fibroblast MRC-5 cells. MRC-5 cells (ATCC, Cat.# CCL-171) were trypsinised then incubated with the test samples for one hour as a suspension. IL-1a (200pg/ml final concentration) (R&D Systems cat no: 200-LA) was then added. After an overnight incubation IL-8 release was determined using an IL-8 quantification ELISA kit (R&D Systems) with anti-IL-8 coated ELISA plates, biotinylated anti-IL-8 and streptavidin-HRP. The assay readout is colourimetric absorbance at 450nm and unknown IL-8 concentrations are interpolated using an IL-8 standard curve included in the assay.

**Method 16**

**Neutralisation potency of E.Coli-expressed recombinant human IL-13 or IL-4 in a whole blood phospho-STAT6 bioassay**

Whole blood cells can be stimulated ex-vivo with recombinant E.Coli-expressed human IL-4 (rhIL-14) or IL-13 (rhIL-13) to express phospho STAT6 (pSTAT6). This assay was developed to quantitatively measure pSTAT6 and consequently determine the neutralisation potency (inhibition of IL-4 or IL-13 bioactivity) of mAbdAb constructs.

The assay was performed in sterile 96-well tissue culture plates under sterile conditions and all test wells were performed in triplicate. 12ng/ml of rhIL-13 or rhIL-4 was prepared in serum free cell culture medium and 31.2μL added to wells of a 96-well plate. A 9 point dilution curve of mAbdAb constructs or isotype control was prepared at 6x the final assay concentration and 31.2μL of each dilution added to wells containing either rhIL-4 or rhIL-13. 125μL of heparinized human whole blood was added to all wells and mixed on a shaker for 30 seconds. The final assay volume contained various dilutions of mAbdAb constructs together with rhIL-13 or rhIL-4 at a final concentration of 2ng/mL. The assay plate was incubated at 37°C, 5%$CO_2$ for 60 minutes.

The cells were then lysed by the addition of 62.5μL of 4x lysis buffer. The lysis buffer contained final assay concentrations of 50mM Tris hydrochloride, 300mM sodium chloride, 1% NP40, 0.5% sodium deoxycholate, 50mM sodium fluoride, 1mM sodium orthovanadate, 1mM EDTA, and protease inhibitor cocktail. The plates were placed on ice for 30 minutes then frozen at -80°C until assayed for pSTAT6.

The measurement of pSTAT6 in the whole blood samples was performed using an electro-chemiluminescent immuno-assay (Meso-Scale-Discovery, MSD). In brief, avidin coated 96-well MSD plates were blocked with 150μL per well of 5% MSD blocker A for 1 hour at room temperature on a shaker at 750rpm. The plate was washed 3 times with 150μL per well of MSD Tris wash buffer. 25μL per well of capture antibody (biotinylated mouse anti-human STAT6 monoclonal antibody) was added and the plates incubated overnight at 4°C. The capture antibody had been diluted to 4μg/mL in assay buffer consisting of 50mM Tris, 150mM sodium chloride, 0.2% BSA, 0.5% Tween 20, 1mM EDTA. The plate was washed 3 times with MSD tris wash buffer then blocked with 150μL of 5% MSD blocker A for 1 hour at room temperature on a shaker. Plates were washed 3 times as stated previously, then 25μL of whole blood lysate or pSTAT6 calibrator added per well. Plates were incubated for 3 hours at room temperature on a shaker. Plates were washed 3 times then 25μL of rabbit anti human pSTAT6 antibody (diluted 1 in 800 in assay buffer) was added and then incubated for 1 hour at room temperature. After further washing, 25μL per well of a 1 in 500 dilution of MSD TAG goat anti-rabbit IgG antibody was added and then incubated for 1 hour at room temperature on a shaker. Plates were washed again before addition of 150μL per well of 2x MSD read buffer T. Plates were read immediately on a MSD SECTOR imager.

The ability of the mAbdAb constructs to neutralise rhIL-13 or rhIL-4 bioactivity was expressed as the concentration of the mAbdAb construct required to neutralise 2ng/mL of human IL-4 or human IL-13 by 50% ($IC_{50}$). The lower the concentration of the mAbdAb construct required, the more potent the neutralisation capacity.

**Method 17**

**Binding to E.Coli-expressed recombinant cynomolgus IL-13 by ELISA**

mAbdAb molecules were assessed for binding to recombinant *E.coli*-expressed cynomolgus IL-13 in a direct binding ELISA. In brief, 5μg/ml recombinant *E.coli*-expressed cynomolgus IL-13 (made and purified at GSK) was coated to a 96-well ELISA plate. The wells were blocked for 1 hour at room temperature, mAbdAb molecules were then titrated out across the plate (usually from around 100nM in 3-fold dilutions to around 0.01nM). Binding was detected using an appropriate dilution of anti-human kappa light chain peroxidase conjugated antibody (catalogue number A7164, Sigma-Aldrich) or an appropriate dilution of anti-human IgG γ chain specific peroxidase conjugated detection antibody (catalogue number A6029, Sigma-Aldrich).

**Method 18**

Not used

**Method 19**

**Inhibition of human IL-4 binding to human IL4 receptor alpha (IL4Rα) by ELISA**

Unless otherwise stated all reagents were diluted to the required concentration in block solution (4% bovine serum albumin in tris-buffered saline and 0.05% Tween20) just prior to use. An ELISA plate was coated over-night at

4°C with 5μg/ml of recombinant human IL4Rα-Fc chimaera (R&D Systems, Cat. No. 604-4R) in phosphate buffered saline. All subsequent steps were carried out at room temperature. The plate was blocked for 2 hours in block solution before addition of 50μl of various concentrations of mAbdAb (or the positive control mAbs or dAbs) which had been pre-mixed with 0.02μg/ml of recombinant human IL-4 (made at GSK). Plates were incubated for 1 hour before washing 4 times in wash buffer (Tris buffered saline and 0.05% Tween20). 50μl of a 0.5μg/ml solution of biotinylated anti-human IL-4 (R&D Systems, Cat. No. BAF 204) was added to each well and incubated for 1 hour. The plate was washed x4 in wash buffer before addition of 50μl/well of a 1/2000 dilution of Extravadin (Sigma, Cat. No. E2886). After one hour the plate was washed 4 times and a colourimetric signal was detected by incubating with OPD peroxidase substrate (from Sigma), the reaction was stopped with the stop solution (3M $H_2SO_4$ acid) and absorbance data obtained by reading on a plate-reader at 490nm. Mean absorbance and standard error was plotted in GraphPad Prism and $IC_{50}$ values were calculated using Cambridge Soft BioAssay.

**Method 20**

**Neutralisation of E.Coli-expressed recombinant cynomolgus IL-13 in a TF-1 cell proliferation bioassay**

TF-1 cells proliferate in response to a number of different cytokines including cynomolgus IL-13. The proliferative response of these cells for IL-13 can therefore be used to measure the bioactivity of IL-13 and subsequently an assay has been developed to determine the IL-13 neutralisation potency (inhibition of IL-13 bioactivity) of mAbdAb constructs.

The assay was performed in sterile 96-well tissue culture plates under sterile conditions and all test wells were performed in triplicate. Approximately 14ng/ml recombinant E.Coli-expressed cynomolgus IL-13 was pre-incubated with various dilutions of mAbdAb constructs (usually from 1000nM or 200nM titrated in 3-fold dilutions to 1nM or 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 500nM or 100nM titrated in 3-fold dilutions to 0.5nM or 0.01nM), recombinant E.Coli-expressed cynomolgus IL-13 (at a final concentration of 7ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed cynomolgus IL-13 bioactivity was expressed as that concentration of the mAbdAb construct required to neutralise the bioactivity of the defined amount of cynomolgus IL-13 (7ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 21**

**Neutralisation of E.Coli-expressed recombinant cynomolgus IL-4 in a TF-1 cell proliferation bioassay**

TF-1 cells proliferate in response to a number of different cytokines including cynomolgus IL-4. The proliferative response of these cells for IL-4 can therefore be used to measure the bioactivity of IL-4 and subsequently an assay has been developed to determine the IL-4 neutralisation potency (inhibition of IL-4 bioactivity) of mAbdAb constructs.

The assay was performed in sterile 96-well tissue culture plates under sterile conditions and all test wells were performed in triplicate. Approximately 2.2ng/ml recombinant E.Coli-expressed cynomolgus IL-4 was pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 100nM titrated in 3-fold dilutions to 0.01nM), recombinant E.Coli-expressed cynomolgus IL-4 (at a final concentration of 1.1ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'cellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed cynomolgus IL-4 bioactivity was expressed as that concentration of the mAbdAb construct required to neutralise the bioactivity of the defined amount of cynomolgus IL-4 (1.1ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 22**

**Inhibition of human IL-13 bindingto human IL13 receptor alpha 2 (IL13Rα2) by ELISA**

Unless otherwise stated all reagents were diluted to the required concentration in block solution (1% bovine serum albumin in tris-buffered saline and 0.05% Tween20) just prior to use. An ELISA plate was coated overnight at 4°C with 5μg/ml of recombinant human IL13Rα2/Fc chimera expressed in Sf21 cells (R&D Systems, Cat. No. 614-IR) in a solution of coating buffer (0.05M bicarbonate pH9.6, Sigma C-3041). The plate was blocked for 1 hour at room temperature in block solution (1% BSA in TBST) before addition of various concentrations of mAbdAb (or the positive control mAbs or dAbs) which had been pre-incubated with 30ng/ml of recombinant human IL-13

(made at GSK) for 30 mins at 37°C. Plates were incubated for 1 hour at room temperature before washing 3 times in wash buffer (Tris buffered saline and 0.05% Tween20). 50μl of a 0.5μg/ml solution of biotinylated anti-human IL-13 (R&D Systems, Cat. No. BAF 213) was added to each well and incubated for 1 hour at room temperature. The plate was washed three times in wash buffer before addition of an appropriate dilution of Extravadin (Sigma, Cat. No. E2886). After one hour the plate was washed and a colourimetric signal was detected by incubating with OPD peroxidase substrate (from Sigma), the reaction was stopped with the stop solution (3M acid) and absorbance data obtained by reading on a plate-reader at 490nm. Mean absorbance and standard error was plotted in Excel sheet and $IC_{50}$ values were calculated using the Robosage software from Microsoft Excel.

**Method 23**

**Biacore™ binding affinity assessment for binding to E.Coli-expressed recombinant cynomolgus IL-13**

The binding affinity of mAbdAb (or mAb) molecules for recombinant *E.Coli*-expressed cynomolgus IL-13 was assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb (or mAb) molecules were then captured onto this surface and cynomolgus IL-13 (made and purified at GSK) passed over at defined concentrations. The surface was regenerated back to the Protein A surface using mild acid elution conditions, this did not significantly affect the ability to capture antibody for a subsequent IL-13 binding event. The work was carried out on BIAcore™ 3000 and / or the T100 machine, data were analysed using the evaluation software in the machines and fitted to the 1:1 model of binding. BIAcore™ runs were carried out at 25°C or 37°C.

**Method 24**

**BIAcore™ binding affinity assessment for binding to E.Coli-expressed recombinant cynomolgus IL-4**

The binding affinity of mAbdAb (or mAb) molecules for recombinant *E.Coli*-expressed cynomolgus IL-4 were assessed by BIAcore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb (or mAb) molecules were then captured onto this surface and cynomolgus IL-4 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-4 binding event. The anti-human IgG surface was regenerated either using similar conditions to the Protein A surface or by using 3M $MgCl_2$. The work was carried out on BIAcore™ 3000 and / or the T100 and / or the A100 machine, data were analysed using the evaluation software in the machines and fitted to the 1:1 model of binding. BIAcore™ runs were carried out at 25°C or 37°C.

**Method 25**

**IL-13 cell-based neutralisation assay**

The potency of mAbdAbs having specificity for IL13 was assayed in an IL-13 cell assay using the engineered reporter cell line HEK Blue-STAT6.

The transcription factor STAT6 is activated primarily by two cytokines with overlapping biologic functions, IL-4 and IL-13 which bind a receptor complex composed of the IL-4Ralpha and IL-13Ralpha1. Upon ligand binding, the receptor complex activates the receptor-associated Janus kinases (JAK1 and Tyk2) leading to the recruitment of STAT6

and its phosphorylation. Activated STAT6 forms a homodimer that translocates to the nucleus, inducing transcription of genes under the control of the responsive promoter. The HEK Blue-STAT6 line is engineered to express Secreted Embryonic Alkaline Phosphatase (SEAP) under the control of such a promoter.

Cells were plated into 96 well plates and incubated for 20-24 hours with pre-equilibrated human human IL-13 and test molecules. After this incubation period, the amount of SEAP produced by the cells as a result of IL-13 stimulation was then measured using the Quanti-blue system (Invivogen).

**Example 1**

**1. Generation of dual targeting mAbdAbs**

The dual targeting mAbdAbs set out in Tables 1-4 were constructed in the following way. Expression constructs were generated by grafting a sequence encoding a domain antibody on to a sequence encoding a heavy chain or a light chain (or both) of a monoclonal antibody such that when expressed the dAb is attached to the C-terminus of the heavy or light chain. For some constructs, linker sequences were used to join the domain antibody to heavy chain CH3 or light chain Cκ. In other constructs the domain antibody is joined directly to the heavy or light chain with no linker sequence. A general schematic diagram of these mAbdAb constructs is shown in Figure 8 (the mAb heavy chain is drawn in grey; the mAb light chain is drawn in white; the dAb is drawn in black).

An example of mAbdAb type 1 as set out in Figure 8 would be PascoH-G4S-474. An example of mAbdAb type 2 as set out in Figure 8 would be PascoL-G4S-474. An example of mAbdAb type 3 as set out in Figure 8 would be PascoHL-G4S-474. mAbdAb types 1 and 2 are tetravalent constructs, mAbdAb type 3 is a hexavalent construct.

A schematic diagram illustrating the construction of a mAbdAb heavy chain (top illustration) or a mAbdAb light chain (bottom illustration) is shown below. Unless otherwise stated, these restriction sites were used to construct the mAbdAbs described in Tables 1-4

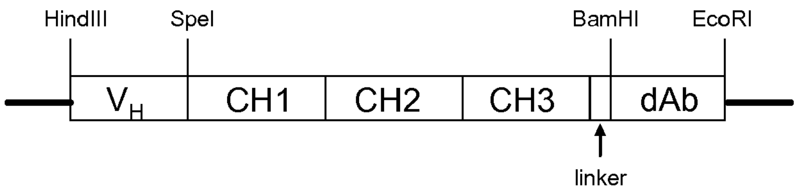


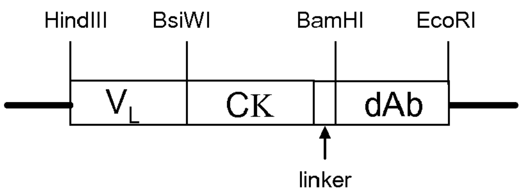


Note that for the heavy chain the term '$V_H$' is the monoclonal antibody variable heavy chain sequence; 'CH1, CH2 and CH3' are human IgG1 heavy chain constant region sequences; 'linker' is the sequence of the specific linker region used; 'dAb' is the domain antibody sequence. For the light chain the term '$V_L$' is the monoclonal antibody variable light chain sequence; 'Cκ' is the human light chain constant region sequence; 'linker' is the sequence of the specific linker region used; 'dAb' is the domain antibody sequence.

Some DNA expression constructs were made de novo by oligo build. And other DNA expression constructs were derived from exisiting constructs (which were made as described above) by restriction cloning or site-directed mutagenesis.

These constructs (mAbdAb heavy or light chains) were cloned into mammalian expression vectors (RIn, RId or pTT vector series) using standard molecular biology techniques. A mammalian amino acid signal sequence (as shown in SEQ ID NO: 64) was used in the construction of these constructs.

For expression of mAbdAbs where the dAb is joined to the C-terminal end of the heavy chain of the monoclonal antibody, the appropriate heavy chain mAbdAb expression vector was paired with the appropriate light chain expression vector for that monoclonal antibody. For expression of mAbdAbs where the dAb is joined to the C-terminal end of the light chain of the monoclonal antibody, the appropriate light chain mAbdAb expression vector was paired with the appropriate heavy chain expression vector for that monoclonal antibody.

For expression of mAbdAbs where the dAb is joined to the C-terminal end of the heavy chain of the monoclonal antibody and where the dAb is joined to the C-terminal end of the light chain of the monoclonal antibody, the appropriate heavy chain mAbdAb expression vector was paired with the appropriate light chain mAbdAb expression vector.

### 1.1 Nomenclature and abbreviations used

Monoclonal antibody (mAb)
Monoclonal antibodies (mAbs)
Domain antibody (dAb)
Domain antibodies (dAbs)
Heavy Chain (H chain)
Light chain (L chain)
Heavy chain variable region ($V_H$)
Light chain variable region ($V_L$)
Human IgG1 constant heavy region 1 (CH1)
Human IgG1 constant heavy region 2 (CH2)
Human IgG1 constant heavy region 3 (CH3)
Human kappa light chain constant region (Cκ)

### 1.2 Anti-IL13mAb-anti-IL4dAbs

Bispecific anti-IL13mAb-anti-IL4dAbs were constructed by as described above. A number of different linkers were used to join the anti-IL4 domain antibodies to the monoclonal antibody. Some constructs had no linker.

Note that a BamHI cloning site (which codes for amino acid residues G and S) was used to clone the linkers and dAbs either to CH3 of the mAb heavy chain or to Cκ of the mAb light chain. Thus in addition to the given linker sequence, additional G and S amino acid residues are present between the linker sequence and the domain antibody for both heavy chain and light chain expression constructs or between CH3 and the linker sequence in some but not all heavy chain expression constructs. However, when the G4S linker was placed between the mAb and dAb in the mAbdAb format, the BamHI cloning site was already present (due to the G and S amino acid residues inherent within the G4S linker sequence) and thus additional G and S amino acid residues were not present between CH3 or Cκ and the domain antibody in the constructs using this linker. When no linker sequence was between used n the mAb and dAb in the mAbdAb format, the BamHI cloning site (and hence the G and S amino acid residues) was still present between CH3 or Cκ and the domain antibody. Full details on the amino acid sequences of mAbdAb heavy and light chains are set out in Table 1.

Several of the following examples use an IL-4 mAb as a control antibody. The control IL-4 mAb used in these examples will either be the antibody having the heavy chain sequence of SEQ ID NO: 14 and the light chain sequence of SEQ ID NO: 15, or will be the antibody having the heavy chain sequence of SEQ ID NO: 166 and the light chain sequence of SEQ ID NO: 15. Both of these IL-4 mAbs share the same CDRs, and are expected to bind in the same way hence both of these antibodies are referred to as'Pascolizumab' or 'IL-4 mAb' in the following examples.

Several of the following examples use an IL-5 mAb as a control antibody. The control IL-5 mAb used in these examples will either be the antibody having the heavy chain sequence of SEQ ID NO: 65 and the light chain sequence of SEQ ID NO: 66, or the antibody having the heavy chain sequence of SEQ ID NO: 191 and the light chain sequence of SEQ ID NO: 66. Both of these IL-5 antibodies share the same CDRs, and are expected to bind in the same way hence both of these antibodies are referred to as 'Mepolizumab' or 'IL-5 mAb' in the following examples.

The mAbdAbs set out in table 1 were expressed transiently in CHOK1 cell supernatants. Following mAbdAb quantification these mAbdAb containing supernatants were analysed for activity in IL-13 and IL-4 binding ELISAs.

Table 1

| Name | Description | Sequence ID No. |
|---|---|---|
| 586H-25 | H chain = Anti-human IL-13 mAb heavy chain-GS-DOM9-155-25 dAb<br>L chain = Anti-human IL-13 mAb light chain | 16 (=H chain)<br>13 (=L chain) |
| 586H-G4S-25 | H chain = Anti-human IL-13 mAb heavy chain-G4S linker-DOM9-155-25 dAb<br>L chain = Anti-human IL-13 mAb light chain | 20 (=H chain)<br>13 (=L chain) |
| 586H-TVAAPS-25 | H chain = Anti-human IL-13 mAb heavy chain-TVAAPS linker-GS-DOM9-155-25 dAb<br>L chain = Anti-human IL-13 mAb light chain | 24 (=H chain)<br>13 (=L chain) |

(continued)

| Name | Description | Sequence ID No. |
|---|---|---|
| 586H-ASTKG-25 | H chain = Anti-human IL-13 mAb heavy chain-GS-ASTKGPT linker-GS-DOM9-155-25 dAb<br>L chain = Anti-human IL-13 mAb light chain | 28 (=H chain)<br>13 (=L chain) |
| 586H-EPKSC-25 | H chain = Anti-human IL-13 mAb heavy chain-GS-EPKSCDKTHTCPPCP linker-GS-DOM9-155-25 dAb<br>L chain = Anti-human IL-13 mAb light chain | 32 (=H chain)<br>13 (=L chain) |
| 586H-ELQLE-25 | H chain = Anti-human IL-13 mAb heavy chain-ELQLEESCAEAQDGELDG linker-GS-DOM9-155-25 dAb<br>L chain = Anti-human IL-13 mAb light chain | 36 (=H chain)<br>13 (=L chain) |
| 586H-147 | H chain = Anti-human IL-13 mAb heavy chain-GS-DOM9-155-147 dAb<br>L chain = Anti-human IL-13 mAb light chain | 17 (=H chain)<br>13 (=L chain) |
| 586H-G4S-147 | H chain = Anti-human IL-13 mAb heavy chain-G4S linker-DOM9-155-147 dAb<br>L chain = Anti-human IL-13 mAb light chain | 21 (=H chain)<br>13 (=L chain) |
| 586H-TVAAPS-147 | H chain = Anti-human IL-13 mAb heavy chain-TVAAPS linker-GS-DOM9-155-147 dAb<br>L chain = Anti-human IL-13 mAb light chain | 25 (=H chain)<br>13 (=L chain) |
| 586H-ASTKG-147 | H chain = Anti-human IL-13 mAb heavy chain-GS-ASTKGPT linker-DOM9-155-147 dAb<br>L chain = Anti-human IL-13 mAb light chain | 29 (=H chain)<br>13 (=L chain) |
| 586H-EPKSC-147 | H chain = Anti-human IL-13 mAb heavy chain-GS-EPKSCDKTHTCPPCP linker-GS-DOM9-155-147 dAb<br>L chain = Anti-human IL-13 mAb light chain | 33 (=H chain)<br>13 (=L chain) |
| 586H-ELQLE-147 | H chain = Anti-human IL-13 mAb heavy chain-GS-ELQLEESCAEAQDGELDG linker-GS-DOM9-155-147 dAb<br>L chain = Anti-human IL-13 mAb light chain | 37 (=H chain)<br>13 (=L chain) |
| 586H-154 | H chain = Anti-human IL-13 mAb heavy chain-GS-DOM9-155-154 dAb<br>L chain = Anti-human IL-13 mAb light chain | 18 (=H chain)<br>13 (=L chain) |
| 586H-G4S-154 | H chain = Anti-human IL-13 mAb heavy chain-G4S linker-DOM9-155-154 dAb<br>L chain = Anti-human IL-13 mAb light chain | 22 (=H chain)<br>13 (=L chain) |
| 586H-TVAAPS-154 | H chain = Anti-human IL-13 mAb heavy chain-TVAAPS linker-GS-DOM9-155-154 dAb<br>L chain = Anti-human IL-13 mAb light chain | 26 (=H chain)<br>13 (=L chain) |
| 586H-ASTKG-154 | H chain = Anti-human IL-13 mAb heavy chain-GS-ASTKGPT linker-GS-DOM9-155-154 dAb<br>L chain = Anti-human IL-13 mAb light chain | 30 (=H chain)<br>13 (=L chain) |
| 586H-EPKSC-154 | H chain = Anti-human IL-13 mAb heavy chain-GS-EPKSCDKTHTCPPCP linker-GS-DOM9-155-154 dAb<br>L chain = Anti-human IL-13 mAb light chain | 34 (=H chain)<br>13 (=L chain) |
| 586H-ELQLE-154 | H chain = Anti-human IL-13 mAb heavy chain-GS-ELQLEESCAEAQDGELDG linker-GS-DOM9-155-154 dAb<br>L chain = Anti-human IL-13 mAb light chain | 38 (=H chain)<br>13 (=L chain) |
| 586H-210 | H chain = Anti-human IL-13 mAb heavy chain-GS-DOM9-112-210 dAb<br>L chain = Anti-human IL-13 mAb light chain | 19 (=H chain)<br>13 (=L chain) |

(continued)

| Name | Description | Sequence ID No. |
|---|---|---|
| 586H-G4S-210 | H chain = Anti-human IL-13 mAb heavy chain-G4S linker-DOM9-112-210 dAb<br>L chain = Anti-human IL-13 mAb light chain | 23 (=H chain)<br>13 (=L chain) |
| 586H-TVAAPS-210 | H chain = Anti-human IL-13 mAb heavy chain-TVAAPS linker-GS-DOM9-112-210 dAb<br>L chain = Anti-human IL-13 mAb light chain | 27 (=H chain)<br>13 (=L chain) |
| 586H-ASTKG-210 | H chain = Anti-human IL-13 mAb heavy chain-GS-ASTKGPT linker-GS-DOM9-112-210 dAb<br>L chain = Anti-human IL-13 mAb light chain | 31 (=H chain)<br>13 (=L chain) |
| 586H-EPKSC-210 | H chain = Anti-human IL-13 mAb heavy chain-GS-EPKSCDKTHTCPPCP linker-GS-DOM9-112-210 dAb<br>L chain = Anti-human IL-13 mAb light chain | 35 (=H chain)<br>13 (=L chain) |
| 586H-ELQLE-210 | H chain = Anti-human IL-13 mAb heavy chain-GS-ELQLEESCAEAQDGELDG linker-GS-DOM9-112-210 dAb<br>L chain = Anti-human IL-13 mAb light chain | 39 (=H chain)<br>13 (=L chain) |
| 586H | H chain = Anti-human IL-13 mAb heavy chain-GS-<br>L chain = Anti-human IL-13 mAb light chain | 40 (=H chain)<br>13 (=L chain) |
| 586H-ASTKG | H chain = Anti-human IL-13 mAb heavy chain-GS-ASTKGPT linker-GS<br>L chain = Anti-human IL-13 mAb light chain | 41 (=H chain)<br>13 (=L chain) |
| 586H-EPKSC | H chain = Anti-human IL-13 mAb heavy chain-GS-EPKSCDKTHTCPPCP linker-GS<br>L chain = Anti-human IL-13 mAb light chain | 42 (=H chain)<br>13 (=L chain) |
| 586H-ELQLE | H chain = Anti-human IL-13 mAb heavy chain-GS-ELQLEESCAEAQDGELDG linker-GS<br>L chain = Anti-human IL-13 mAb light chain | 43 (=H chain)<br>13 (=L chain) |

The mAbdAbs set out in table 2 were expressed in one or both of CHOK1 or CHOE1a cell supernatants, purified and analysed in a number of IL-13 and IL-4 activity assays.

Table 2

| Name | Description | Sequence ID No. |
|---|---|---|
| 586H-TVAAPS-25 | H chain = Anti-human IL-13 mAb heavy chain-TVAAPS linker-GS-DOM9-155-25 dAb<br>L chain = Anti-human IL-13 mAb light chain | 24 (=H chain)<br>13 (=L chain) |
| 586H-TVAAPS-154 | H chain = Anti-human IL-13 mAb heavy chain-TVAAPS linker-GS-DOM9-155-154 dAb<br>L chain = Anti-human IL-13 mAb light chain | 26 (=H chain)<br>13 (=L chain) |
| 586H-TVAAPS-210 | H chain = Anti-human IL-13 mAb heavy chain-TVAAPS linker-GS-DOM9-112-210 dAb<br>L chain = Anti-human IL-13 mAb light chain | 27 (=H chain)<br>13 (=L chain) |

### 1.3 Anti-IL4mAb-anti-IL13dAbs

Bispecific anti-IL4mAb-anti-IL13dAbs were constructed as described above. A number of different linkers were used to join the anti-IL13 domain antibody to the monoclonal antibody. Some constructs had no linker.

Note that a BamHI cloning site (which codes for amino acid residues G and S) was used to clone the linkers and dAbs either to CH3 of the mAb heavy chain or to Cκ of the mAb light chain. Thus in addition to the given linker

sequence, additional G and S amino acid residues are present between the linker sequence and the domain antibody for both heavy chain and light chain expression constructs or between CH3 and the linker sequence in some but not all heavy chain expression constructs. However, when the G4S linker was placed between the mAb and dAb in the mAbdAb format, the BamHI cloning site was already present (due to the G and S amino acid residues inherent within the G4S linker sequence) and thus additional G and S amino acid residues were not present between CH3 or Cκ and the domain antibody in the constructs using this linker. When no linker sequence was between used n the mAb and dAb in the mAbdAb format, the BamHI cloning site (and hence the G and S amino acid residues) was still present between CH3 or Cκ and the domain antibody. Full details on the amino acid sequences of mAbdAb heavy and light chains are set out in table 3.

The mAbdAbs set out in table 3 were expressed transiently in CHOK1 cell supernatants. Following mAbdAb quantification these mAbdAb containing supernatants were analysed for activity in IL-13 and IL-4 binding ELISAs.

Table 3

| Name | Description | Sequence ID No. |
|---|---|---|
| PascoH-474 | H chain = Pascolizumab heavy chain-GS-DOM10-53-474 dAb<br>L chain = Pascolizumab light chain | 48 (=H chain)<br>15 (=L chain) |
| PascoH-G4S-474 | H chain = Pascolizumab heavy chain-G4S linker-DOM10-53-474 dAb<br>L chain = Pascolizumab light chain | 49 (=H chain)<br>15 (=L chain) |
| PascoH-TVAAPS-474 | H chain = Pascolizumab heavy chain-TVAAPS linker-GS-DOM10-53-474 dAb<br>L chain = Pascolizumab light chain | 50 (=H chain)<br>15 (=L chain) |
| PascoH-ASTKG-474 | H chain = Pascolizumab heavy chain-GS-ASTKGPT | 51 (=H chain) |
| | linker-GS-DOM10-53-474 dAb<br>L chain = Pascolizumab light chain | 15 (=L chain) |
| PascoH-EPKSC-474 | H chain = Pascolizumab heavy chain-GS-EPKSCDKTHTCPPCP linker-GS-DOM10-53-474 dAb<br>L chain = Pascolizumab light chain | 52 (=H chain)<br>15 (=L chain) |
| PascoH-ELQLE-474 | H chain = Pascolizumab heavy chain-GS-ELQLEESCAEAQDGELDG linker-GS-DOM10-53-474 dAb<br>L chain = Pascolizumab light chain | 53 (=H chain)<br>15 (=L chain) |
| PascoL-474 | H chain = Pascolizumab heavy chain<br>L chain = Pascolizumab light chain-GS-DOM10-53-474 dAb | 14 (=H chain)<br>54 (=L chain) |
| PascoL-G4S-474 | H chain = Pascolizumab heavy chain<br>L chain = Pascolizumab light chain-G4S linker-DOM10-53-474 dAb | 14 (=H chain)<br>55 (=L chain) |
| PascoL-TVAAPS-474 | H chain = Pascolizumab heavy chain<br>L chain = Pascolizumab light chain-TVAAPS linker-GS-DOM10-53-474 dAb | 14 (=H chain)<br>56 (=L chain) |
| PascoL-ASTKG-474 | H chain = Pascolizumab heavy chain<br>L chain = Pascolizumab light chain-ASTKGPT linker-GS-DOM10-53-474 dAb | 14 (=H chain)<br>57 (=L chain) |
| PascoL-EPKSC-474 | H chain = Pascolizumab heavy chain<br>L chain = Pascolizumab light chain-EPKSCDKTHTCPPCP linker-GS-DOM10-53-474 dAb | 14 (=H chain)<br>58 (=L chain) |
| PascoL-ELQLE-474 | H chain = Pascolizumab heavy chain<br>L chain = Pascolizumab light chain-ELQLEESCAEAQDGELDG linker-GS-DOM10-53-474 dAb | 14 (=H chain)<br>59 (=L chain) |

The mAbdAbs set out in Table 4 were expressed in one or more of CHOK1, CHOE1a or HEK293-6E cells.

Table 4

| Name | Description | Sequence ID No. |
|---|---|---|
| PascoH-G4S-474 | H chain = Pascolizumab heavy chain-G4S linker-DOM10-53-474 dAb<br>L chain = Pascolizumab light chain | 49 (=H chain)<br>15 (=L chain) |
| PascoH-474 | H chain = Pascolizumab heavy chain-GS-DOM10-53-474 dAb<br>L chain = Pascolizumab light chain | 48 (=H chain)<br>15 (=L chain) |
| PascoL-G4S-474 | H chain = Pascolizumab heavy chain<br>L chain = Pascolizumab light chain-G4S linker-DOM10-53-474 dAb | 14 (=H chain)<br>55 (=L chain) |
| PascoHL-G4S-474 | H chain = Pascolizumab heavy chain-G4S linker-DOM10-53-474 dAb<br>L chain = Pascolizumab light chain-G4S linker-DOM10-53-474 dAb | 49 (=H chain)<br>55 (=L chain) |

### 1.4 Sequence ID numbers for monoclonal antibodies, domain antibodies and linkers

Sequence IDs numbers for the monoclonal antibodies (mAb), domain antibodies (dAb) and linkers used to generate the mAbdAbs are shown below in table 5.

Table 5

| Name | Specificity | Sequence ID |
|---|---|---|
| Anti-human IL-13 monoclonal antibody (also known as 586) | Human IL-13 | 12 (H chain)<br>13 (L chain) |
| Anti-human IL-4 monoclonal antibody (also known as Pascolizumab) | Human IL-4 | 14 (H chain)<br>15 (L chain) |
| DOM10-53-474 domain antibody | Human IL-13 | 5 |
| Anti-human IL-13 monoclonal antibody (also known as 656) | Human IL-13 | 161 (H chain)<br>156 (L chain) |
| DOM9-112-210 domain antibody | Human IL-4 | 4 |
| DOM10-53-616 domain antibody | Human IL-13 | 148 |
| DOM9-155-25 domain antibody | Human IL-4 | 1 |
| DOM9-155-147 domain antibody | Human IL-4 | 2 |
| DOM9-155-154 domain antibody | Human IL-4 | 3 |
| ASTKGPS linker sequence | Derived from human IgG1 H chain (VH-CH1) | 9 |
| ASTKGPT linker sequence | Derived from human IgG1 H chain (VH-CH1), where the last amino acid residue in the native sequence (S) has been substituted for T | 8 |
| EPKSCDKTHTCPPCP linker sequence | Derived from human IgG1 H chain (CH1-CH2) | 10 |
| TVAAPS linker sequence | Derived from human K L chain (VL-CK) | 7 |
| ELQLEESCAEAQDGELDG linker sequence | Derived from human IgG1 CH3 tether | 11 |
| GGGGS linker sequence | A published linker sequence | 6 |

Mature human IL-13 amino acid sequence (without signal sequence) is given in SEQ ID NO: 63. Mature human IL-4 amino acid sequence (without signal sequence) is given in SEQ ID NO: 62.

### 1.5 Expression and purification of mAbdAbs

The mAbdAb expression constructs described in Example 1 were transfected into one or more of CHOK1 cells, CHOE1a cells or HEK293-6E cells, expressed at small (approximately 3mls) or medium (approximately 50mls to 100mls) or large (approximately 1 litre) scale and then some of the constructs were purified using immobilised Protein A columns

and quantified by reading absorbance at 280nm.

### 1.6 Size exclusion chromatography analyses of purified mAbdAbs

A number of mAbdAbs were analysed by size exclusion chromatography (SEC) and sodium dodecyl sulphate polyacrylamide gel electrophoresis (SDS PAGE). Representative data for some of these molecules (PascoH-G4S-474, PascoL-G4S-474, PascoH-474 and PascoHL-G4S-474.) are shown in Figures 9, 10, 11 and 12 respectively. Representative data showing SEC and SDS Page analysis for these molecules with the 'GS' motif removed are shown in Figures 90-98.

In some cases SEC was used to further purify these molecules to remove aggregates.

## Example 2

### Binding of mAbdAbs to human IL-13 and human IL-4 by ELISA

### 2.1 Binding of anti-IL13mAb-anti-IL4dAbs to IL-13 and IL-4

mAbdAb supernatants, were tested for binding to human IL-13 in a direct binding ELISA (as described in method 1). These data are shown in Figure 13.

Figure 13 shows that all of these anti-IL13mAb-anti-IL4dAbs bound IL-13. The binding activity of these mAbdAbs was also approximately equivalent (within 2-fold to 3-fold) to purified anti-human IL13 mAb alone, which was included in this assay as a positive control for IL-13 binding and in order to directly compare to the mAbdAbs. Purified anti-human IL4 mAb (Pascolizumab) was included as a negative control for IL-13 binding.

These molecules were also tested for binding to human IL-4 in a direct binding ELISA (as described in method 2). These data are shown in Figure 14.

Figure 14 shows that all of these anti-IL13mAb-anti-IL4dAbs bound IL-4, but some variation in IL-4 binding activity was observed. No binding to IL-4 was observed when no anti-IL4 dAb was present in the mAbdAb construct. Purified anti-human IL13 mAb was also included as a negative control for binding to IL-4. Note that the anti-IL-4 dAbs alone were not tested in this assay as the dAbs are not detected by the secondary detection antibody; instead, purified anti-human IL4 mAb (Pascolizumab) was used as a positive control to demonstrate IL-4 binding in this assay.

Purified samples of mAbdAbs, were also tested for binding to human IL-13 in a direct binding ELISA (as described in method 1). These data are shown in Figure 15.

These purified anti-IL13mAb-anti-IL4dAbs bound IL-13. The binding activity of these mAbdAbs for IL-13 was equivalent to that of purified anti-human IL13 mAb alone. An isotype-matched mAb (with specificity for an irrelevant antigen) was also included as a negative control for binding to IL-13 in this assay.

These purified mAbdAbs were also tested for binding to human IL-4 in a direct binding ELISA (as described in method 2). These data are shown in Figure 16.

All of these anti-IL13mAb-anti-IL4dAbs bound IL-4. Note that the anti-IL-4 dAbs alone were not tested in this assay as the dAbs are not detected by the secondary detection antibody; instead, purified anti-human IL4 mAb (Pascolizumab) was used as a positive control to demonstrate IL-4 binding in this assay. An isotype-matched mAb (with specificity for an irrelevant antigen) was also included as a negative control for binding to IL-4 in this assay.

### 2.2 Binding of anti-IL4mAb-anti-IL13dAbs to IL-13 and IL-4

mAbdAb supernatants were tested for binding to human IL-4 in a direct binding ELISA (as described in method 2). These data are shown in Figure 17 (some samples were prepared and tested in duplicate and this has been annotated as sample 1 and sample 2).

Figure 17 shows that all of these mAbdAbs bound IL-4. Purified anti-human IL4 mAb alone (Pascolizumab) was included in this assay but did not generate a binding curve as an error was made when diluting this mAb for use in the assay (Pascolizumab has been used successfully in all other subsequent IL-4 binding ELISAs). Purified anti-human IL13 mAb was included as a negative control for IL-4 binding.

The same mAbdAb supernatants were also tested for binding to human IL-13 in a direct binding ELISA (as described in method 1). These data are shown in Figure 18 (some samples were prepared and tested in duplicate and this has been annotated as sample 1 and sample 2).

Figure 18 shows that all of these anti-IL4mAb-anti-IL13dAbs bound IL-13. Purified anti-human IL13 mAb alone was included in this assay but did not generate a binding curve as an error was made when diluting this mAb for use in the assay (purified anti-human IL13 mAb has been used successfully in all other subsequent IL-13 binding ELISAs). Purified anti-IL4 mAb (Pascolizumab) was included as a negative control for binding to IL-13. Note that the anti-IL-13

dAb alone (DOM10-53-474) was not tested in this assay as this dAb is not detected by the secondary detection antibody.

The purified anti-IL4mAb-anti-IL13dAbs, 'PascoH-G4S-474', 'PascoH-474', 'PascoL-G4S-474' and 'PascoHL-G4S-474', were also tested for binding to human IL-4 in a direct binding ELISA (as described in method 2). These data are shown in Figure 19.

These purified anti-IL4mAb-anti-IL13dAbs bound IL-4. The binding activity of these mAbdAbs was approximately equivalent (within 2-fold) to purified anti-IL4 mAb alone (Pascolizumab). An isotype-matched mAb (with specificity for an irrelevant antigen) was also included as a negative control for binding to IL-4 in this assay.

These same purified anti-IL4mAb-anti-IL13dAbs, PascoH-G4S-474, PascoH-474, PascoL-G4S-474 and PascoHL-G4S-474, were also tested for binding to human IL-13 in a direct binding ELISA (as described in method 1). These data are shown in Figure 20A.

These purified anti-IL4mAb-anti-IL13dAbs bound IL-13. An isotype-matched mAb (with specificity for an irrelevant antigen) was also included as a negative control for binding to IL-13 in this assay. Note that the anti-IL-13 dAb alone (DOM10-53-474) was not tested in this assay as the dAb is not detected by the secondary detection antibody; instead, the anti-human IL13 mAb was used as a positive control to demonstrate IL-13 binding in this assay.

Purified PascoH-474, PascoH-TVAAPS-474, PascoH-ASTKG-474 and PascoH-ELQLE-474 were also tested for binding to cynomolgus IL-13 in a direct binding ELISA, as described in method 17 (PascoH-474 GS removed and PascoH-TVAAPS-474 GS removed were also included in this assay, the construction of these molecules is described in Example 18). A graph showing representative data is shown in Figure 20B.

Purified PascoH-474, PascoH-TVAAPS-474, PascoH-ASTKG-474 and PascoH-ELQLE-474 all bound cynomolgus IL-13. Purified anti-human IL4 mAb alone (Pascolizumab) was included in this assay as a negative control for binding to IL-13. Purified anti-human IL13 mAb was included as a positive control for cynomolgus IL-13 binding. Note that the anti-IL-13 dAb alone (DOM10-53-474) was not tested in this assay as the dAb is not detected by the secondary detection antibody; instead, the anti-human IL13 mAb was used as a positive control to demonstrate IL-13 binding in this assay.

## Example 3

### Binding of mAbdAbs to human IL-13 and human IL-4 by surface plasmon resonance (BIAcore™)

### 3.1 Binding of anti-IL13mAb-anti-IL4dAbs to IL-13 and IL-4 by BIAcore™

mAbdAbs (in CHO cell supernatants, prepared as described in section 1.5) were tested for binding to human IL-13 using BIAcore™ at 25°C (as described in method 4). For this data set, two IL-13 concentration curves (100nM and 1nM) were assessed and relative response capture levels of between 1000 and 1300 (approximately) were achieved for each mAbdAb construct. Due to the limited number of concentrations of IL-13 used, the data generated are more suitable for ranking of constructs rather than exact kinetic measurements. These data are shown in Table 6.

Table 6

| Antibody | Binding affinity KD (nM) |
|---|---|
| 586H-25 | 0.39 |
| 586H-G4S-25 | 0.41 |
| 586H-TVAAPS-25 | 0.5 |
| 586H-ASTKG-25 | 0.54 |
| 586H-EPKSC-25 | 0.55 |
| 586H-ELQLE-25 | 0.42 |
| 586H-147 | 0.46 |
| 586H-G4S-147 | 0.45 |
| 586H-TVAAPS-147 | 0.56 |
| 586H-ASTKG-147 | 0.44 |
| 586H-EPKSC-147 | 0.46 |
| 586H-ELQLE-147 | 0.51 |

(continued)

| Antibody | Binding affinity KD (nM) |
|---|---|
| 586H-154 | 0.46 |
| 586H-G4S-154 | 0.37 |
| 586H-TVAAPS-154 | 0.56 |
| 586H-ASTKG-154 | 0.44 |
| 586H-EPKSC-154 | 0.42 |
| 586H-ELQLE-154 | 0.44 |
| 586H-210 | 0.44 |
| 586H-G4S-210 | 0.42 |
| 586H-TVAAPS-210 | 0.4 |
| 586H-ASTKG-210 | 0.4 |
| 586H-EPKSC-210 | 0.43 |
| 586H-ELQLE-210 | 0.43 |
| 586H | 0.44 |
| 586H-ASTKG | 0.32 |
| 586H-ELQLE | 0.47 |
| 586H-EPKSC | 0.45 |
| Anti-human IL-13 mAb (purified) | 0.38 |
| Pascolizumab (purified) | no binding |

All of these anti-IL13mAb-anti-IL4dAbs bound IL-13 with similar binding affinities which were approximately equivalent to the binding affinity of purified anti-human IL13 mAb alone. These data suggested that the addition of linkers and/or anti-IL4 dAbs to the heavy chain of the anti-IL13 mAb, did not affect the IL-13 binding affinity of the mAb component within these mAbdAb constructs.

These mAbdAbs were also tested for binding to human IL-4 using BIAcore™ at 25°C (as described in method 5). These data are shown in Table 7. For this data set, four IL-4 concentration curves (512, 128, 32 and 8nM) were assessed and approximate relative response capture levels for each mAbdAb tested are indicated in the table. Note that the anti-IL-4 dAbs alone were not tested in this assay as the dAbs cannot be captured onto the Protein A or anti-human IgG coated CM5 chip; instead, the anti-human IL4 mAb (Pascolizumab) was used as a positive control to demonstrate IL-4 binding in this assay.

Table 7

| **Antibody** | **Capture Level** | **On rate (ka, $Ms^{-1}$)** | **Off rate (kd, $s^{-1}$)** | **Binding affinity KD (nM)** |
|---|---|---|---|---|
| 586H-25 | 864 | 6.13e3 | 4.11e-4 | 67 |
| 586H-G4S-25 | 1818 | 6.3e3 | 9.54e-4 | 151 |
| 586H-TVAAPS-25 | 673 | 1.27e5 | 1.2e-4 | 0.95 |
| 586H-ASTKG-25 | 809 | 5.4e5 | 1.20e-3 | 21.8 |
| 586H-EPKSC-25 | 748 | 4.79e4 | 1.42e-3 | 29.6 |
| 586H-ELQLE-25 | 603 | 1.26e6 | 1.63e-6 | 0.001* |
| 586H-147 | 1095 | 3.42e3 | 1.18e-3 | 344.8 |
| 586H-G4S-147 | 1200 | 4.21e3 | 4.57e-4 | 108.5 |
| 586H-TVAAPS-147 | 433 | 6.62e4 | 6.69e-7 | 0.011** |
| 586H-ASTKG-147 | 1248 | 3.67e4 | 6.9e-4 | 18.8 |

(continued)

| Antibody | Capture Level | On rate (ka, $Ms^{-1}$) | Off rate (kd, $s^{-1}$) | Binding affinity KD (nM) |
|---|---|---|---|---|
| 586H-EPKSC-147 | 878 | 2.54e4 | 6.71e-4 | 26.4 |
| 586H-ELQLE-147 | 676 | 7.01e5 | 1.52e-5 | 0.027* |
| 586H-154 | 436 | 6.1e3 | 1.74e-3 | 285 |
| 586H-G4S-154 | 1437 | 5.00e3 | 6.85e-4 | 137.8 |
| 586H-TVAAPS-154 | 1530 | 6.44e4 | 1.15e-7 | 0.002** |
| 586H-ASTKG-154 | 1373 | 3.26e4 | 2.84e-4 | 8.7 |
| 586H-EPKSC-154 | 794 | 3.03e4 | 5.7e-4 | 18.8 |
| 586H-ELQLE-154 | 795 | 1.25e6 | 3.57e-6 | 0.003* |
| 586H-210 | 1520 | not determined | not determined | --- |
| 586H-G4S-210 | 1448 | not determined | not determined | --- |
| 586H-TVAAPS-210 | 1693 | not determined | not determined | --- |
| 586H-ASTKG-210 | 1768 | not determined | not determined | --- |
| 586H-EPKSC-210 | 1729 | not determined | not determined | --- |
| 586H-ELQLE-210 | 1350 | not determined | not determined | --- |
| 586H | 1500 | no binding | no binding | --- |
| 586H-ASTKG | 1615 | no binding | no binding | --- |
| 586H-ELQLE | 343 | no binding | no binding | --- |
| 586H-EPKSC | 1416 | no binding | no binding | --- |
| Pascolizumab (purified) | 1092 | 2.04e6 | 1.23e-4 | 0.060 |

Caveats were observed for some of the above data sets. Poor curve fits were observed for some data sets (*), the actual binding affinity values that have been determined for these data should therefore be treated with caution. Positive dissociation was seen for some curves (**), the actual binding affinity values that have been determined for these data should therefore be treated with caution. In addition, BIAcore™ was unable (ie. not sensitive enough) to determine on and off rates for all mAbdAb constructs containing the DOM9-112-210 dAb, due to the exceptionally tight binding of these mAbdAbs to IL-4. Determination of binding kinetics for these mAbdAbs for IL-4 was further hampered by observed positive dissociation effects. These data are shown in Figure 21. Similar data was obtained in an additional experiment. These data are shown in Figure 22.

These 2 independent data sets indicated that all of the anti-IL13mAb-anti-IL4dAbs bound IL-4, but the binding affinities varied depending on the linker used to join the anti-IL4 dAb to the anti-IL13 mAb heavy chain. In this experiment, the presence of a linker was found to enhance the binding affinity for IL-4 of the anti-IL4 dAb component (when placed on the heavy chain) in the mAbdAb format. For example the molecules having TVAAPS or ELQLEESCAEAQDGELDG linkers appear to be more potent binders. No binding to IL-4 was observed when no anti-IL4 dAb was present in the mAbdAb construct. It was not possible to measure the binding affinity of the 586-linker-210 mAbdAbs for IL-4, due to the fact that the DOM9-112-210 component of these mAbdAbs binds very tightly and hence the off-rate is too small to determine using BIAcore™.

Purified anti-IL13mAb-anti-IL4dAbs were also tested for binding to human IL-13 and human IL-4 using BIAcore™ at 25°C (as described in methods 4 and 5). These data are shown in Table 8.

Table 8

| Construct | Binding affinity, KD (nM) | |
|---|---|---|
| | Human IL-13 | Human IL-4 |
| 586H-TVAAPS-25 | 0.38 | 1.1 |
| 586H-TVAAPS-154 | 0.41 | 0.49 |

(continued)

| Construct | Binding affinity, KD (nM) | |
|---|---|---|
| | Human IL-13 | Human IL-4 |
| 586H-TVAAPS-210 | 0.38 | very tight binder (unable to determine KD due to positive dissociation effects and sensitivity level of BIAcore™ technique) |
| Anti-human IL-13 mAb (purified) | 0.43 | --- |
| Pascolizumab (purified) | --- | 0.03 |

586H-TVAAPS-25, 586H-TVAAPS-154 and 586H-TVAAPS-210 all bound IL-13 with similar binding affinities and this was approximately equivalent to the binding affinity of purified anti-human IL13 mAb alone. 586H-TVAAPS-25, 586H-TVAAPS-154 and 586H-TVAAPS-210 all bound IL-4. It was not possible to measure the binding affinity of 586-TVAAPS-210 for IL-4, due to the fact that the DOM9-112-210 component of this mAbdAb bound very tightly and hence the off-rate was too small to determine using BIAcore™. Note that the anti-IL-4 dAbs alone (DOM9-155-25, DOM9-155-154 and DOM9-112-210) were not tested in this assay format as the dAbs cannot be captured onto the Protein A or anti-human IgG coated CM5 chip; instead, the anti-human IL4 mAb (Pascolizumab) was used as a positive control to demonstrate IL-4 binding in this assay.

### 3.2 Binding of anti-IL4mAb-anti-IL13dAbs to IL-4 and IL-13 by BIAcore™

mAbdAbs (in CHO cell supernatants prepared as described in section 1.5) were tested for binding to human IL-4 using BIAcore™ at 25°C (as described in method 5). These data are shown in Table 9 (some samples were prepared and tested in duplicate - this has been annotated as sample 1 and sample 2). For this data set, four IL-4 concentrations curves (100nM, 10nM, 1nM and 0.1nM) were assessed and approximate relative response capture levels for each mAbdAb tested are indicated in the table. An isotype-matched mAb (with specificity for an irrelevant antigen) was also included as a negative control for binding to IL-4 in this assay.

Table 9

| Antibody | Capture Level | On rate (ka, $Ms^{-1}$) | Off rate (kd, $s^{-1}$) | Binding affinity KD (nM) |
|---|---|---|---|---|
| **Experiment 1** | | | | |
| PascoH-G4S-474 | ~500 | 5.1e6 | 8.6e-5 | 0.02 |
| PascoH-TVAAPS-474 | ~500 | 5.5e6 | 9.7e-5 | 0.02 |
| PascoH-474 | ~500 | 4.8e6 | 9.4e-5 | 0.02 |
| PascoH-ASTKG-474 | ~500 | 5.3e6 | 8.6e-5 | 0.02 |
| PascoH-ELQLE-474 | ~500 | 5.1e6 | 1.1e-4 | 0.02 |
| PascoH-EPKSC-474 | ~500 | 4.9e6 | 9.8e-5 | 0.02 |
| Pascolizumab (purified) | ~700 | 5.3e6 | 1.6e-4 | 0.03 |
| **Experiment 2** | | | | |
| PascoL-G4S-474 (sample 1) | 1871 | 2.14e6 | 1.35e-4 | 0.063 |
| PascoL-G4S-474 (sample 2) | 1921 | 2.13e6 | 1.11e-4 | 0.052 |
| PascoL-TVAAPS-474 (sample 1) | 2796 | 2.48e6 | 2.12e-4 | 0.085 |
| PascoL-TVAAPS-474 (sample 2) | 3250 | 3.04e6 | 2.79e-4 | 0.092 |
| PascoL-474 (sample 1) | 3254 | 2.8e6 | 1.84e-4 | 0.065 |

(continued)

| | | | | |
|---|---|---|---|---|
| **Experiment 2** | | | | |
| PascoL-474 (sample 2) | 2756 | 2.53e6 | 1.22e-4 | 0.048 |
| PascoL-ASTKG-474 (sample 1) | 3037 | 2.95e6 | 1.21e-4 | 0.041 |
| PascoL-ASTKG-474 (sample 2) | 3784 | 2.54e6 | 1.52e-4 | 0.060 |
| PascoL-EPKSC-474 (sample 1) | 3238 | 1.86e6 | 2.58e-4 | 0.139 |
| PascoL-EPKSC-474 (sample 2) | 3276 | 2.51e6 | 3.18e-4 | 0.127 |
| Pascolizumab (purified) | 1152 | 2.04e6 | 1.23e-4 | 0.060 |
| Negative control mAb | 2976 | no binding | no binding | --- |

All of the anti-IL4mAb-anti-IL13dAbs tested bound IL-4 with similar binding affinities and this was approximately equivalent to the binding affinity of the anti-human IL4 mAb alone (Pascolizumab). PascoL-EPKSC-474 bound IL-4 approximately 2-fold less potently than Pascolizumab.These data suggested that the addition of linkers and the anti-IL13 dAb to either the heavy chain or the light chain of Pascolizumab, did not overtly affect the IL-4 binding affinity of the mAb component within the mAbdAb construct.

These mAbdAbs were also tested for binding to human IL-13 using BIAcore™ at 25°C (as described in method 4). These data are shown in Table 10 (some samples were prepared and tested in duplicate - this has been annotated as sample 1 and sample 2). For this data set, four IL-13 concentrations curves (128nM, 32nM, 8nM and 2nM) were assessed and approximate relative response capture levels for each mAbdAb tested are indicated in the table.

Table 10

| Antibody | Capture Level | On rate (ka, $Ms^{-}1$) | Off rate (kd, $s^{-1}$) | Binding affinity KD (nM) |
|---|---|---|---|---|
| **Experiment 1** | | | | |
| PascoH-474 | ~500 | 3.6e5 | 3.1e-4 | 0.84 |
| PascoH-G4S-474 | ~500 | 3.9e5 | 2.6e-4 | 0.67 |
| PascoH-TVAAPS-474 | ~500 | 4.5e5 | 4.2e-4 | 0.94 |
| PascoH-ASTKG-474 | ~500 | 3.1e5 | 4.6e-4 | 1.5 |
| PascoH-ELQLE-474 | ~500 | 3.4e5 | 6.2e-4 | 1.8 |
| PascoH-EPKSC-474 | ~500 | 3.5e5 | 4.0e-4 | 1.1 |
| Anti-human IL-13 mAb (purified) | ~650 | 8.6e5 | 4.9e-4 | 0.57 |
| **Experiment 2** | | | | |
| PascoL-474 (sample 1) | 3254 | 2.86e5 | 3.82e-4 | 1.34 |
| PascoL-474 (sample 2) | 2756 | 3.12e5 | 3.86e-4 | 1.24 |
| PascoL-G4S-474 (sample 1) | 1871 | 5.63e5 | 4.25e-4 | 0.756 |
| PascoL-G4S-474 (sample 2) | 1921 | 5.59e5 | 3.47e-4 | 0.621 |
| PascoL-TVAAPS-474 (sample 1) | 2796 | 7.42e5 | 2.58e-4 | 0.348 |

(continued)

| Experiment 2 | | | | |
|---|---|---|---|---|
| PascoL-TVAAPS-474 (sample 2) | 3250 | 6.22e5 | 1.71e-4 | 0.275 |
| PascoL-ASTKG-474 (sample 1) | 3037 | 5.26e5 | 2.38e-4 | 0.451 |
| PascoL-ASTKG-474 (sample 2) | 3784 | 5.38e5 | 3.20e-4 | 0.595 |
| PascoL-EPKSC-474 (sample 1) | 3238 | 4.17e5 | 3.34e-4 | 0.801 |
| PascoL-EPKSC-474 (sample 2) | 3276 | 3.51e5 | 2.86e-4 | 0.815 |
| Anti-human IL-13 mAb (purified) | 1373 | 9.12e4 | 6.11e-4 | 0.67 |
| Pascolizumab (purified) | 1152 | no binding | no binding | --- |
| Negative control mAb | 2976 | no binding | no binding | --- |

Binding affinity data for constructs tested in experiment 2 are also shown in figure 23.

All of the anti-IL4mAb-anti-IL13dAbs bound IL-13. In most cases the presence of a linker did not appear to enhance the binding affinity for IL-13 of the anti-IL13 dAb component when placed on the heavy chain of the anti-IL4 mAb. However, the presence of a linker did appear to enhance the binding affinity for IL-13 of the anti-IL13 dAb component when placed on the light chain of the anti-IL4 mAb. PascoL-TVAAPS-474 appeared to have the most potent IL-13 binding affinity in this experiment.

Note that the anti-IL-13 dAb alone (DOM10-53-474) was not tested in this assay as the dAb cannot be captured onto the Protein A or anti-human IgG coated CM5 chip; instead, purified anti-human IL13 mAb was used as a positive control to demonstrate IL-13 binding in this assay. An isotype-matched mAb (with specificity for an irrelevant antigen) was also included as a negative control for binding to IL-13 in this assay.

Purified anti-IL4mAb-anti-IL13dAbs were also tested for binding to human IL-4 and human IL-13 using BIAcore™ at 25°C (as described in methods 4 and 5). These data are shown in Table 11.

Table 11

| Construct | Binding affinity, KD (nM) | |
|---|---|---|
| | Human IL-4 | Human IL-13 |
| PascoH-G4S-474 | 0.036 | 0.58 |
| PascoH-474 | 0.037 | 0.71 |
| PascoL-G4S-474 | 0.028 | 1.2 |
| PascoHL-G4S-474 | 0.035 | 0.87 |
| Anti-human IL-13 mAb (purified) | --- | 0.41 |
| Pascolizumab (purified) | 0.037 | --- |

In this experiment PascoH-G4S-474, PascoH-474, PascoL-G4S-474 and PascoHL-G4S-474 all bound IL-4 with similar binding affinities and this was approximately equivalent to the binding affinity of the anti-human IL4 mAb alone (Pascolizumab). They also all bound IL-13. Note that the anti-IL-13 dAb alone (DOM10-53-474) was not tested in this assay as the dAb cannot be captured onto the Protein A or anti-human IgG coated CM5 chip; instead, the anti-human IL13 mAb was used as a positive control to demonstrate IL-13 binding in this assay.

### 3.3 Stoichiometry of binding of IL-13 and IL-4 to the anti-IL4mAb-anti-IL13dAbs using BIAcore™

Purified anti-IL4mAb-anti-IL13dAbs were evaluated for stoichiometry of binding for IL-13 and IL-4 using BIAcore™ (as described in method 7). These data are shown in Table 12.

Table 12

| Construct | Stoichiometry | |
|---|---|---|
| | Human IL-4 | Human IL-13 |
| PascoL-G4S-474 | 1.8 | 1.8 |
| PascoH-G4S-474 | 1.8 | 1.9 |
| Pasco-474 | 1.8 | 1.9 |
| PascoHL-G4S-474 | 1.7 | 3.5 |
| Anti-human IL-13 mAb (purified) | --- | 1.8 |
| Pascolizumab (purified) | 1.8 | --- |

PascoH-G4S-474, PascoH-474 and PascoL-G4S-474 were able to bind to nearly two molecules of IL-13 and two molecules of IL-4. PascoHL-G4S-474 was able to bind nearly two molecules of IL-4 and nearly four molecules of IL-13. These data indicated that the constructs tested could be fully occupied by the expected number of IL-13 or IL-4 molecules.

## <u>Example 4</u>

### <u>Neutralisation potency of mAbdAbs in IL-13 and IL-4 bioassays</u>

### 4.1 Anti-IL13mAb-anti-IL4dAbs

Purified anti-IL13mAb-anti-IL4dAbs were tested for neutralisation of human IL-13 in a TF-1 cell bioassay (as described in method 8). These data are shown in Figure 24.

Purified anti-IL13mAb-anti-IL4dAbs, 586H-TVAAPS-25, 586H-TVAAPS-154 and 586H-TVAAPS-210, fully neutralised the bioactivity of IL-13 in a TF-1 cell bioassay. The neutralisation potencies of these mAbdAbs were within 2-fold of purified anti-human IL-13 mAb alone. The purified anti-human IL-4 mAb (Pascolizumab) and purified anti-IL4 dAbs (DOM9-155-25, DOM9-155-154 or DOM9-112-210) were included as negative controls for neutralisation of IL-13 in this assay.

The purified anti-IL13mAb-anti-IL4dAbs, 586H-TVAAPS-25, 586H-TVAAPS-154 and 586H-TVAAPS-210, were also tested for neutralisation of human IL-4 in a TF-1 cell bioassay (as described in method 9). These data are shown in Figure 25.

586H-TVAAPS-210 fully neutralised the bioactivity of IL-4 in this TF-1 cell bioassay. The neutralisation potency of this mAbdAb was within 2-fold of purified anti-human IL-4 dAb alone (DOM9-112-210). 586H-TVAAPS-25 and 586H-TVAAPS-154 did not neutralise the bioactivity of IL-4 and this was in contrast to the purified anti-human IL-4 dAbs alone (DOM9-155-25 and DOM9-155-154). As demonstrated by BIAcore™, purified 586H-TVAAPS-25 and 586H-TVAAPS-154 had 1.1nM and 0.49nM binding affinities (respectively) for IL-4. IL-4 binds the IL-4 receptor very tightly (binding affinities of approximately 50pM have been reported in literature publications) and thus the observation that both 586H-TVAAPS-25 or 586H-TVAAPS-154 were unable to effectively neutralise the bioactivity of IL-4 in the TF-1 cell bioassay maybe a result of the relative lower affinity of these mAbdAbs for IL-4 compared to the potency of IL-4 for the IL-4 receptor.

Purified anti-human IL-4 mAb (Pascolizumab) was included as a positive control for neutralisation of IL-4 in this bioassay. Purified anti-human IL-13 mAb was included as a negative control for neutralisation of IL-4 in this bioassay.

### <u>4.2 Anti-IL4mAb-anti-IL13dAbs</u>

The 0purified anti-IL4mAb-anti-IL13dAbs, PascoH-G4S-474, PascoH-474, PascoL-G4S-474 and PascoHL-G4S-474, were tested for neutralisation of human IL-4 in a TF-1 cell bioassay (as described in method 9). These data are shown in Figure 26.

Purified anti-IL4mAb-anti-IL13dAbs, PascoH-G4S-474, PascoH-474, PascoL-G4S-474 and PascoHL-G4S-474,fully neutralised the bioactivity of IL-4 in a TF-1 cell bioassay. The neutralisation potencies of these mAbdAbs were

approximately equivalent to that of purified anti-human IL4 mAb alone (Pascolizumab), Purified anti-human IL-13 mAb, purified DOM10-53-474 dAb and a dAb with specificity for an irrelevant antigen (negative control dAb) were also included as negative controls for neutralisation of IL-4 in this bioassay.

The purified anti-IL4mAb-anti-IL13dAbs, PascoH-G4S-474, PascoH-474, PascoL-G4S-474 and PascoHL-G4S-474, were tested for neutralisation of human IL-13 in a TF-1 cell bioassay (as described in method 8). These data are shown in Figure 27.

Purified anti-IL4mAb-anti-IL13dAbs, PascoH-G4S-474, PascoH-474, PascoL-G4S-474 and PascoHL-G4S-474, fully neutralised the bioactivity of IL-13 in a TF-1 cell bioassay. The neutralisation potencies of these mAbdAbs were within 3-fold of purified anti-IL13 dAb alone (DOM10-53-474). Purified anti-human IL-13 mAb was also included as a positive control for IL-13 neutralisation in this bioassay. A dAb with specificity for an irrelevant antigen (negative control dAb) and purified anti-human IL4 mAb alone (Pascolizumab) were also included as negative controls for neutralisation of IL-4 in this bioassay.

The purified anti-IL4mAb-anti-IL13dAbs, PascoH-G4S-474, PascoH-474, PascoL-G4S-474 and PascoHL-G4S-474, were also tested for simultaneous neutralisation of human IL-4 and human IL-13 in a dual neutralisation TF-1 cell bioassay (as described in method 10). These data are shown in Figure 28.

Purified anti-IL4mAb-anti-IL13dAbs, PascoH-G4S-474, PascoH-474, PascoL-G4S-474 and PascoHL-G4S-474,fully neutralised the bioactivity of both IL-4 and IL-13 in a dual neutralisation TF-1 cell bioassay. The neutralisation potencies of these mAbdAbs were approximately equivalent to that of a combination of purified anti-human IL4 mAb (Pascolizumab) and purified anti-IL13 dAb (DOM10-53-474). Purified anti-human IL-13 mAb alone, purified anti-human IL-4 mAb alone (Pascolizumab) and the anti-human IL-13 dAb (DOM10-53-474) alone (which were included as negative controls) did not fully neutralise the bioactivity of both IL-4 and IL-13 in this dual IL-4 and IL-13 neutralisation bioassay.

## Example 5

## SEC-MALLS analysis of dAbs

Antigen-specific dAbs were characterized for their solution state by SEC-MALLS (size-exclusion chromatography - multi-angle laser light scattering) and the results are shown in Table 13: the DOM10-53-474, dAb exists as a monomer in solution whilst all DOM9 dAbs (DOM9-112-210, DOM9-155-25, DOM9-155-147 and DOM9-155-154) can form stable dimers (and in some instances tetramers).

### 5.1. Preparation of the proteins

Samples were purified and dialysed into appropriate buffer e.g. PBS. Samples were filtered after dialysis and the concentration determined (0.43mg/ml DOM-155-25), (1.35mg/ml DOM9-155-147) and 1.4mg/ml DOM9-155-159). DOM10-53-474 and DOM9-112-210 were adjusted to 1mg/ml. BSA was purchased from Sigma and used without further purification.

### 5.2. Size-Exclusion chromatography and Detector Set-up

Shimadzu LC-20AD Prominence HPLC system with an autosampler (SIL-20A) and SPD-20A Prominence UV/Vis detector was connected to Wyatt Mini Dawn Treos (MALLS, multi-angle laser light scattering detector) and Wyatt Optilab rEX DRI (differential refractive index) detector. The detectors were connected in the following order - LS-UV-RI. Both RI and LS instruments operated at a wavelength of 488nm. TSK2000 (Tosoh corporation)) column were used (silica-based HPLC column) with mobile phase of 50mM phosphate buffer (without salt), or 1xPBS, both at pH7.4. The flow rate used is 0.5 or 1ml/min, the time of the run was adjusted to reflect different flow rates (45 or 23 min) and was not expected to have significant impact onto separation of the molecules. Proteins were prepared in buffer to a concentration of 1mg/ml and injection volume was 100ul.

### 5.3. Detector Calibration

The light-scattering detector was calibrated with toluene according to manufacturer's instructions.

### 5.4. Detector Calibration with BSA

The UV detector output and RI detector output were connected to the light scattering instrument so that the signals from all three detectors could be simultaneously collected with the Wyatt ASTRA software. Several injections of BSA in a mobile phase of PBS (1ml/min) are run over a Tosoh TSK2000 column with UV, LS and RI signals collected

by the Wyatt software. The traces are then analysed using ASTRA software, and the signals are normalised aligned and corrected for band broadening following manufacturer's instructions. Calibration constants are then averaged and input into the template which is used for future sample runs.

### 5.5. Absolute molar mass calculations

100ul of each sample were injected onto appropriate pre-equilibrated column. After SEC column the sample passes through 3 on-line detectors - UV, MALLS (multi-angle laser light scattering) and DRI (differential refractive index) allowing absolute molar mass determination. The dilution that takes place on the column is about 10 fold, and the concentration at which in-solution state was determined as appropriate.

The basis of the calculations in ASTRA as well as of the Zimm plot technique, which is often implemented in a batch sample mode is the equation from Zimm [J. Chem. Phys. 16, 1093-1099 (1948)]:

$$\frac{R_\theta}{K^* c} = MP(\theta) - 2A_2cM^2P^2(\theta) \quad \text{(Eq. 1)}$$

where

- $c$ is the mass concentration of the solute molecules in the solvent (g/mL)
- $M$ is the weight average molar mass (g/mol)
- $A_2$ is the second virial coefficient (mol mL / $g^2$)
- $K^* = 4p^2 n_0^2 (dn/dc)^2 l_0^{-4} N_A^{-1}$ is an optical constant where $n_0$ is the refractive index of the solvent at the incident radiation (vacuum) wavelength, $l_0$ is the incident radiation (vacuum) wavelength, expressed in nanometers, $N_A$ is Avogadro's number, equal to $6.022 \times 10^{23}$ $mol^{-1}$, and *dn*/*dc* is the differential refractive index increment of the solvent-solute solution with respect to a change in solute concentration, expressed in mL/g (this factor must be measured independently using a dRI detector).
- $P$(q) is the theoretically-derived form factor, approximately equal to $1-2\mu^2\langle r^2\rangle/3!+\cdots$ , where $\mu=(4\pi/\lambda)\sin(\theta/2)$ , and $\langle r^2\rangle$ is the mean square radius. $P$(q) is a function of the molecules' *z*-average size, shape, and structure.
- $R$q is the excess Rayleigh ratio ($cm^{-1}$)

This equation assumes vertically polarized incident light and is valid to order $c^2$.
To perform calculations with the Zimm fit method, which is a fit to $R$q /$K^*c$ vs. $\sin^2$(q/2), we need to expand the reciprocal of Eq. 1 first order in $c$:
To perform calculations with the Zimm fit method, which is a fit to Rq /K*c vs. sin2(q/2), we need to expand the reciprocal of Eq. 1 to first order in c:

$$\frac{K^* c}{R_\theta} = \frac{1}{MP(\theta)} + 2A_2c \quad \text{Eq. 2}$$

The appropriate results in this case are

$$M = \left(\frac{K^* c}{R_0} - 2A_2c\right)^{-1} \quad \text{Eq. 3}$$

and

$$\langle r^2\rangle = \frac{3m_0\lambda^2 M}{16\pi^2} \quad \text{Eq. 4}$$

where

$$m_0 \equiv d\left[K^* c / R_\theta\right] / d\left[\sin^2(\theta / 2)\right]_{\theta \to 0} \quad \text{Eq. 5}$$

The calculations are performed automatically by ASTRA software, resulting in a plot with molar mass determined for each of the slices [Astra manual].

Molar mass obtained from the plot for each of the peaks observed on chromatogram was compared with expected molecular mass of a single unit of the protein. This provides a basis to form conclusions about in-solution state of the protein. Representative data is shown in Table 13.

**Table 13: Summary**

| dAb | SEC-MALLS | MW | Column & mobile phase |
|---|---|---|---|
| DOM10-53-474 | monomer | 14kDa | TSK2000, PBS, pH7.4, 0.5ml/min |
| DOM9-112-210 | dimer | 30kDa | TSK2000, PBS, pH7.4, 0.5ml/min |
| DOM9-155-25 | dimer | 28kDa | TSK2000, 50mM, phosphate buffer pH7.4, 1ml/min |
| DOM9-155-147 | dimer-tetramer equilibrium | 26-51kDa | TSK2000, 50mM phosphate buffer, pH7.4, 1ml/min |
| DOM9-155-159 | dimer | 28kDa | TSK2000, 50mM phosphate Buffer, pH7.4, 1ml/min |

**DOM10-53-474**

Single peak with the average molar mass defined as ~14kDa indicating a monomeric state in solution, shown in Figure 29

**DOM9-112-210**

Single peak with the molar mass defined as 30 kDa indicating a dimeric state in solution, shown in Figure 30

**DOM9-155-25**

Nice symmetrical peak but running at the buffer front. The mid part of the peak has been used for molar mass determination (see figure below with all three signals overlaid). Molar mass is 28 kDa which represents a dimeric dAb, shown in Figure 31.

Overlay of all three signals (Figure 32)

**DOM9-155-147**

The main peak is assigned with molar mass of 26kDa over the right part of the peak and increasing steeply over the left part of the peak up to 53kDa. The peak most likely represents a dimer and a smaller fraction of tetramer in a rapid equilibrium. A much smaller peak eluting at 7.6 min, represents tetrameric protein with molar mass of 51kDa (Figure 33).

**DOM9-155-159**

The protein runs as a single symmetric peak, with average molar mass assigned at ~28kDa indicating a dimeric state in solution (Figure 34)

**Control for MW assignment by SEC-MALLS: BSA**

Each BSA run for each of the experiments set out above resulted in the expected MW, e.g. 2 peaks with molar mass of 67and 145kDa (monomer and dimer) (Figure 35).

## Example 6

## Generation of trispecific mAbdAbs

Trispecific mAb-dAbs were constructed either by generating VH and VL sequences by assembly PCR which were then cloned into existing mAbdAb expression vectors or by sub-cloning existing VH and VL regions from mAb expression vectors into existing mAbdAb expression vectors, such that when expressed, the trispecific mAbdAb has dAbs attached to both the C-terminus of the heavy and light chains.

A linker sequence was used to join the domain antibody to heavy chain CH3 or light chain C□. A schematic diagram of a trispecific mAbdAb molecule is shown in Figure 36 (the mAb heavy chain is drawn in grey; the mAb light chain is drawn in white; the dAbs are drawn in black).

A schematic diagram illustrating the construction of a trispecific mAbdAb heavy chain (top illustration) and a trispecific mAbdAb light chain (bottom illustration) is shown below

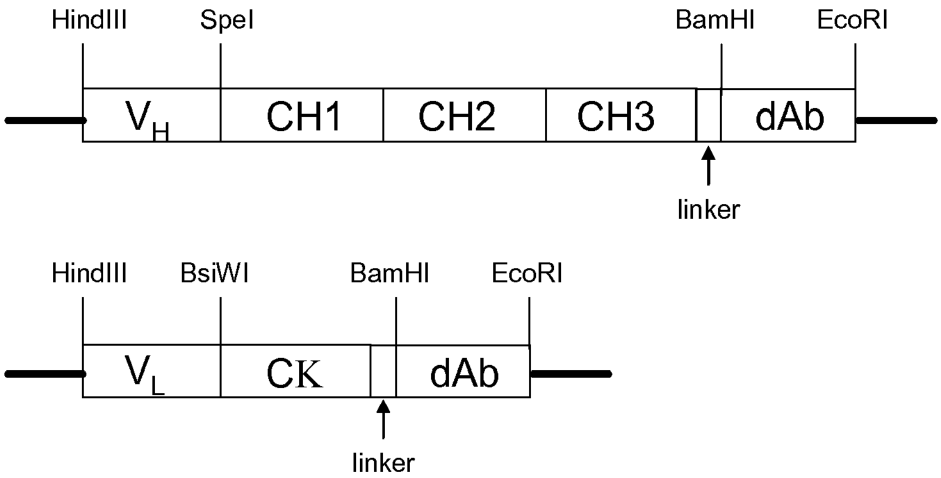


For the heavy chain the term 'V$_H$' is the monoclonal antibody variable heavy chain sequence; 'CH1, CH2 and CH3' are human IgG1 heavy chain constant region sequences; 'linker' is the sequence of the specific linker region used; 'dAb' is the domain antibody sequence. For the light chain: 'V$_L$' is the monoclonal antibody variable light chain sequence; 'C□' is the human light chain constant region sequence; 'linker' is the sequence of the specific linker region used; 'dAb' is the domain antibody sequence.

A mammalian amino acid signal sequence (as shown in SEQ ID NO: 64) was used in the generation of these constructs.

### 6.1 Trispecific anti-IL18mAb-anti-IL4dAb-anti-IL13dAb

A trispecific anti-IL18mAb-anti-IL4dAb-anti-IL13dAb (also known as IL18mAb-210-474) was constructed by grafting a sequence encoding an anti-human IL-4 domain antibody (DOM9-112-210) onto a sequence encoding the heavy chain and a sequence encoding an anti-IL13 domain antibody (DOM10-53-474) onto a sequence encoding the light chain of an anti-human IL-18 humanised monoclonal antibody. A G4S linker was used to join the anti-IL4 domain antibody onto the heavy chain of the monoclonal antibody. A G4S linker was also used to join the anti-IL13 domain antibody onto the light chain of the monoclonal antibody.

IL18mAb-210-474 was expressed transiently in CHOK1 cell supernatants, and following quantification of IL18mAb-210-474 in the cell supernatant, analysed in a number of IL-18, IL-4 and IL-13 binding assays.

| Name | Description | Sequence ID No. |
|---|---|---|
| IL18mAb-210-474 | H chain = Anti-human IL-18 mAb heavy chain-G4S linker-DOM9-112-210 dAb<br>L chain = Anti-human IL-18 mAb light chain-G4S linker-DOM10-53-474 dAb | 69 (=H chain)<br>70 (=L chain) |

### 6.2 Trispecific anti-IL5mAb-anti-IL4dAb-anti-IL13dAb

A trispecific anti-IL5mAb-anti-IL4dAb-anti-IL13dAb (also known as Mepo-210-474) was constructed by grafting a sequence encoding an anti-human IL-4 domain antibody DOM9-112-210 (SEQ ID NO: 4) onto a sequence encoding the heavy chain of an anti-human IL-5 humanised monoclonal antibody (SEQ ID NO: 65), and grafting a sequence encoding an anti-IL13 domain antibody DOM10-53-474 (SEQ ID NO: 5) onto a sequence encoding the light chain of an anti-human IL-5 humanised monoclonal antibody (SEQ ID NO: 66). A G4S linker was used to join the anti-IL4 domain antibody onto the heavy chain of the monoclonal antibody. A G4S linker was also used to join the anti-IL13 domain antibody onto the light chain of the monoclonal antibody.

This mAbdAb was expressed transiently in CHOK1 and HEK293-6E cell supernatants, and following quantification in the cell supernatant, analysed in a number of IL-4, IL-5 and IL-13 binding assavs.

| Name | Description | Sequence ID No. |
|---|---|---|
| Mepo-210-474 | H chain = Anti-human IL-5 mAb heavy chain-G4S linker-DOM9-112-210 dAb<br>L chain = Anti-human IL-5 mAb light chain-G4S linker-DOM10-53-474 dAb | 71 (=H chain)<br>72 (=L chain) |

### 6.3 Sequences of monoclonal antibodies, domain antibodies and linkers

The sequences for the monoclonal antibodies, domain antibodies and linkers used to generate the trispecific mAbdAbs (or used as control reagents in the following exemplifications) are shown below in table 14.

Table 14

| Name | Specificity | Sequence ID |
|---|---|---|
| DOM9-112-210 domain antibody | Human IL-4 | 4 |
| DOM10-53-474 domain antibody | Human IL-13 | 5 |
| GGGGS linker sequence | | 6 |
| Pascolizumab (Anti-human IL-4 monoclonal antibody) | Human IL-4 | 14 (=H chain)<br>15 (=L chain) |
| Mepolizumab (Anti-human IL-5 monoclonal antibody) | Human IL-5 | 65 (=H chain)<br>66 (=L chain) |
| Anti-human IL-13 (humanised) monoclonal antibody | Human IL-13 | 12 (=H chain)<br>13 (=L chain) |
| Anti-human IL-18 (humanised) monoclonal antibody | Human IL-18 | 67 (=H chain)<br>68 (=L chain) |

Mature human IL-4 amino acid sequence (without signal sequence) is given in SEQ ID NO: 62. Mature human IL-13 amino acid sequence (without signal sequence) is given in SEQ ID NO: 63.

### 6.4 Expression and purification of trispecific mAbdAbs

DNA sequences encoding trispecific mAbdAb molecules were cloned into mammalian expression vectors (RIn, RId or pTT) using standard molecular biology techniques. The trispecific mAbdAb expression constructs were transiently transfected into one or both of CHOK1 or HEK293-6E cells, expressed at small scale (3mls to 150mls).The expression procedures used to generate the trispecfic mAbdAbs were the same as those routinely used to express and monoclonal antibodies.

Some of the constructs were purified using immobilised Protein A columns and quantified by reading absorbance at 280nm.

## Example 7

### Binding of trispecific mAbdAbs to human IL-4, human IL-13 and human IL-18 by ELISA

#### 7.1 Binding of IL-18mAb-210-474 to IL-4, IL-13 and IL-18 by ELISA

Trispecific mAbdAb IL18mAb-210-474 (supernatants) prepared as described in Example 6 (SEQ ID NO: 69 and 70), was tested for binding to human IL-18, human IL-13 and human IL-4 in direct binding ELISAs (as described in methods 1, 2 and 3) and these data are shown in Figures 37, 38 and 39 respectively (IL18mAb-210-474 was prepared and tested a number of times and this has been annotated in the figures as sample 1, sample 2, sample 3, etc).

These figures show that IL18mAb-210-474 bound IL-4, IL-13 and IL-18 by ELISA. Purified anti-human IL18 mAb was included in the IL-18 binding ELISA as a positive control for IL-18 binding. The anti-IL-4 dAb (DOM9-112-210) was not tested in the IL-4 binding ELISA as this dAb is not detected by the secondary detection antibody; instead, purified anti-human IL4 mAb (Pascolizumab) was used as a positive control to demonstrate IL-4 binding in this ELISA. The anti-IL-13 dAb (DOM10-53-474) was not tested in the IL-13 binding ELISA as this dAb is not detected by the secondary detection antibody; instead, purified anti-human IL-13 mAb was included as a positive control to demonstrate IL-13 binding in this ELISA. As shown in the figures, negative control mAbs to an irrelevant antigen were included in each binding ELISA.

In each ELISA the binding curve for IL18mAb-210-474 sample 5 sits apart from the binding curves for the other IL18mAb-210-474 samples. The reason for this is unknown however, it maybe due to a quantification issue in the human IgG quantification ELISA for this particular IL18mAb-210-474 sample 5.

#### 7.2 Binding of Mepo-210-474 to IL-4 and IL-13 by ELISA

Trispecific mAbdAbs Mepo-210-474 (supernatant) prepared as described in section 1 (sequence ID numbers 71 and 72), were tested for binding to human IL-13 and human IL-4 in direct binding ELISAs (as described in methods 1 and 2 respectively) and these data are shown in Figures 40 and 41 respectively (Mepo-210-474 was prepared and tested in quadruplicate and this has been annotated as sample 1, sample 2, sample 3 and sample 4).

These figures illustrate that Mepo-210-474 bound IL-4 and IL-13 by ELISA. The anti-IL-4 dAb (DOM9-112-210) was not tested in the IL-4 binding ELISA as this dAb is not detected by the secondary detection antibody; instead, purified anti-human IL4 mAb (Pascolizumab) was used as a positive control to demonstrate IL-4 binding in this ELISA. The anti-IL-13 dAb (DOM10-53-474) was not tested in the IL-13 binding ELISA as this dAb is not detected by the secondary detection antibody; instead, purified anti-human IL-13 mAb was included as a positive control to demonstrate IL-13 binding in this ELISA. As shown in Figures 40 and 41, negative control mAbs to an irrelevant antigen were included in each binding ELISA.

Mepo-210-474 sample 1 and sample 2 were prepared in one transient transfection experiment and Mepo-210-474 sample 3 and sample 4 were prepared in another separate transient transfection experiment. All four samples bound IL-13 and IL-4 in IL-13 and IL-4 binding ELISAs. However, the reason for the different binding profiles of samples 1 and 2 verses samples 3 and 4 is unknown, but may reflect a difference in the quality of the mAbdAb (in the supernatant) generated in each transfection experiment.

## Example 8

### Binding of trispecific mAbdAbs to human IL-4, human IL-5, human IL-13 and human IL-18 by surface plasmon resonance (BIAcore™)

#### 8.1 Binding of IL-18mAb-210-474 to IL-4, IL-13 and IL-18 by BIAcore™

Trispecific mAbdAb IL18mAb-210-474 (supernatants) prepared as described in Example 6.1 (SEQ ID NO: 69 and 70), was tested for binding to human IL-4, human IL-13 and human IL-18 using BIAcore™ at 25°C (as described in methods 4, 5 and 6 respectively). Capture levels were within the range of approximately 400 to 850 Response Units. Three concentrations of each analyte were tested (256, 32 and 4nM). The resulting data are shown in Table 15 (samples were prepared and tested in triplicate, this has been annotated as sample 1, sample 2 and sample 3).

Table 15

| Construct | On rate (ka) | Off rate (kd) | Binding affinity, KD (nM) |
|---|---|---|---|
| **Binding to IL-18** | | | |
| IL18mAb-210-474 (sample 1) | 2.1e6 | 2.3e-5 | 0.011 |
| IL18mAb-210-474 (sample 2) | 2.1e6 | 2.8e-5 | 0.014 |
| IL18mAb-210-474 (sample 3) | 2.1e6 | 2.9e-5 | 0.014 |
| Anti-human IL-18 mAb (purified) | 1.9e6 | 6.8e-5 | 0.035 |
| **Binding to IL-13** | | | |
| IL18mAb-210-474 (sample 1) | 5.8e5 | 5.7e-4 | 0.99 |
| IL18mAb-210-474 (sample 2) | 6.2e5 | 6.1e-4 | 0.99 |
| IL18mAb-210-474 (sample 3) | 7.4e5 | 7.4e-4 | 1.0 |
| Anti-human IL-13 mAb (purified) | 1.2e6 | 5.0e-4 | 0.41 |
| **Binding to IL-4** | | | |
| IL18mAb-210-474 (sample 1) | --- | --- | very tight binder * |
| IL18mAb-210-474 (sample 2) | --- | --- | very tight binder * |
| IL18mAb-210-474 (sample 3) | --- | --- | very tight binder * |
| Pascolizumab (purified) | 4.6e6 | 1.7e-4 | 0.037 |
| *unable to determine KD due to positive dissociation effects and sensitivity level of BIAcore™ technique | | | |

The trispecific mAbdab bound IL-4, IL-13 and IL-18 using BIAcore™. The binding affinity of the mAbdAb for IL-18 was approximately equivalent to that of purified anti-human IL18 mAb alone, which was included in this assay as a positive control for IL-18 binding and in order to directly compare to the binding affinity of the mAbdab. It was not possible to determine the absolute binding affinity of the mAbdab for IL-4, due to the fact that the DOM9-112-210 component of this trispecific mAbdAb bound very tightly to IL-4 and hence the off-rate was too small to determine using BIAcore™. The anti-IL-4 dAb alone (DOM9-112-210) was not tested in this assay as this dAb cannot be captured onto the Protein A or anti-human IgG coated CM5 chip; instead, the anti-human IL4 mAb (Pascolizumab) was included as a positive control to demonstrate IL-4 binding in this assay. The anti-IL-13 dAb alone (DOM10-53-474) was not tested in this assay as this dAb cannot be captured onto the Protein A or anti-human IgG coated CM5 chip; instead, the anti-human IL13 mAb was included as a positive control to demonstrate IL-13 binding in this assay.

### 8.2 Binding of Mepo-210-474 to IL-4, IL-5 and IL-13 by BIAcore™

Trispecific mAbdAb Mepo-210-474 (supernatants) prepared as described in Example 6.2 (SEQ ID NO: 71 and 72), was tested for binding to human IL-4, human IL-5 and human IL-13 using BIAcore™ at 25°C (as described in methods 5, 11 and 4 respectively). Capture levels were within the range of approximately 550 to 900 Response Units. For IL-4 and IL-13 binding five concentrations of each analyte were tested (256, 64, 16, 4 and 1nM). For IL-5 binding four concentrations of each analyte were tested (64, 16, 4 and 1nM). The resulting data are shown in Table 16.

Table 16

| Construct | On rate (ka) | Off rate (kd) | Binding affinity, KD (nM) |
|---|---|---|---|
| **Binding to IL-5** | | | |
| Mepo-210-474 | 3.34e5 | 1.50e-4 | 0.45 |
| Mepolizumab (purified) | 3.78e4 | 1.30e-4 | 0.34 |

(continued)

| Binding to IL-13 | | | |
|---|---|---|---|
| Mepo-210-474 | 6.38e5 | 1.03e-3 | 1.62 |
| Anti-human IL-13 mAb (purified) | 1.51e6 | 5.68e-4 | 0.38 |
| **Binding to IL-4** | | | |
| Mepo-210-474 | --- | --- | very tight binder |
| | | | (unable to determine KD due to positive dissociation effects and sensitivity level of BIAcore™ technique) |
| Pascolizumab (purified) | 6.26e6 | 1.43e-4 | 0.02 |

Mepo-210-474 bound IL-4, IL-5 and IL-13 using BIAcore™. The binding affinity of Mepo-210-474 for IL-5 was approximately equivalent to that of purified anti-human IL5 mAb (Mepolizumab) alone, which was included in this assay as a positive control for IL-5 binding and in order to directly compare to the binding affinity of Mepo-210-474. It was not possible to determine the absolute binding affinity of Mepo-210-474 for IL-4, due to the fact that the DOM9-112-210 component of this trispecific mAbdAb bound very tightly to IL-4 and hence the off-rate was too small to determine using BIAcore™. The anti-IL-4 dAb alone (DOM9-112-210) was not tested in this assay as this dAb cannot be captured onto the Protein A or anti-human IgG coated CM5 chip; instead, the anti-human IL4 mAb (Pascolizumab) was included as a positive control to demonstrate IL-4 binding in this assay. The anti-IL-13 dAb alone (DOM10-53-474) was not tested in this assay as this dAb cannot be captured onto the Protein A or anti-human IgG coated CM5 chip; instead, the anti-human IL13 mAb was included as a positive control to demonstrate IL-13 binding in this assay.

## Example 9

## Stoichiometry

### 9.1 Stoichiometry of binding of IL-4, IL-13 and IL-18 to IL-18mAb-210-474 using BIAcore™

IL18mAb-210-474 (in CHO cell supernatants prepared as described in section 1) (SEQ ID NO: 69 and 70), were evaluated for stoichiometry of binding for IL-4, IL-13 and IL-18 using BIAcore™ (as described in method 7). These data are shown in Table 17 (R-max is the saturated binding response and this is required to calculate the stoichiometry, as per the given formulae in method 7). The concentration of each of the cytokines was 500nM. The injection position refers to the order in which each of the cytokines was added.

Table 17

| Cytokine | Injection position | R-max | Stoichiometry |
|---|---|---|---|
| IL-4 | 1st | 59 | 0.9 |
| IL-4 | 2nd | 56 | 0.9 |
| IL-4 | 3rd | 51 | 0.8 |
| IL-13 | 1st | 74 | 1.6 |
| IL-13 | 2nd | 77 | 1.7 |
| IL-13 | 3rd | 80 | 1.8 |
| IL-18 | 1st | 112 | 1.8 |
| IL-18 | 2nd | 113 | 1.8 |
| IL-18 | 3rd | 110 | 1.7 |

The stoichiometry data indicated that IL18mAb-210-474 bound approximately two molecules of IL-18, two molecules of IL-13 and only one molecule of IL-4. The anti-IL4 dAb alone (DOM9-112-210) is known to be a dimer in solution state and is only able to bind one molecule of IL-4. It is therefore not unexpected that IL18mAb-210-474 would

bind only one molecule of IL-4. These data indicated that the molecules tested could be fully occupied by the expected number of IL-18, IL-13 and IL-4 molecules. The stoichiometry data also indicated that the order of capture of the cytokines appears to be independent of the order of addition of the cytokines.

## Example 10

### 10.1 Generation of a Dual Targeting anti-TNF/anti-EGFR mAbdAb

This dual targeting mAbdAb was constructed by fusion of a dAb to the C-terminus of the mAb heavy chain. The anti-TNF mAb heavy and light chain expression cassettes had been previously constructed. The restriction sites which were used for cloning are shown below (Figure 42). To introduce restriction sites for dAb insertion in the heavy chain, site directed mutagenesis was used to create SalI and HindIII cloning sites using the mAb heavy chain expression vector as a template. DNA coding an anti-EGFR dAb (DOM16-39-542) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID NO: 170 and 169) for light and heavy chains respectively) were selected and large scale were made using Qiagen Mega Prep Kit following the manufacturer's protocols. mAbdAbs were expressed in mammalian HEK293-6E cells using transient transfection techniques by co-transfection of light and heavy chains (SEQ ID NO: 73 and 74)

### 10.2 Purification and SEC analysis of the Dual Targeting anti-TNF/anti-EGFR mAbdAb

The anti-TNF/anti-EGFR mAbdAb was purified from clarified expression supernatant using Protein-A affinity chromatography according to established protocols. Concentrations of purified samples were determined by spectrophotometry from measurements of light absorbance at 280nm. SDS-PAGE analysis (Figure 43) of the purified sample shows non-reduced sample running at ~170kDa whilst reduced sample shows two bands running at ~25 and ~60kDa corresponding light chain and dAb-fused heavy chain respectively.

For size exclusion chromatography (SEC) analysis the anti-TNF/anti-EGFR mAbdAb was applied onto a Superdex-200 10/30 HR column (attached to an Akta Express FPLC system) pre-equilibrated and running in PBS at 0.5ml/min. The SEC profile shows a single species running as a symmetrical peak (figure 44).

### 10.3 Binding Affinities of the Dual Targeting anti-TNF/anti-EGFR mAbdAb

Binding affinities to EGFR and TNF were determined as described in methods 13 and 14 respectively. Assay data were analysed using GraphPad Prism. Potency values were determined using a sigmoidal dose response curve and the data fitted using the best fit model. Anti-EGFR potency (Figure 45) of this mAbdAb was calculated to be 39.1nM whilst the control, an anti-EGFR mAb gave an EC50 value of 3.4nM. In the anti-TNF bioassay (Figure 46) the potency was of the mAbdAb was 3pM (0.0028nM) whilst an anti-TNF control mAb produced an EC50 of 104pM. In conclusion, assay data shows that the construct of example 10, a dual targeting anti-TNF/anti-EGFR mAbdAb is potent against both antigens.

### 10.4 Rat PK of the Dual Targeting anti-TNF/anti-EGFR mAbdAb

This molecule was tested for its *in vivo* pharmacokinetic properties in the rat. The anti-TNF/anti-EGFR mAbdAb was administered i.v. to three rats, and serum samples collected over a period of 7 days (168 hours). The concentration of drug remaining at various time points post-dose was assessed by ELISA against both TNF & EGFR. Results are shown in Figure 125.

The PK parameters confirmed that this molecule had a long half life, in the same region as that previously observed for unmodified adalimumab (125 hours). Further details are shown in Table 17.1

Table 17.1

| Assay Antigen | Half Life (hr) | Cmax (ug/mL) | AUC (0-inf) (hr*ug/mL) | Clearance (mL/hr/kg) | % AUC Extrapolated |
|---|---|---|---|---|---|
| TNF | 157.2 | 149.8 | 10301.3 | 0.5 | 40.6 |
| EGFR | 140.8 | 123.6 | 7986.7 | 0.7 | 35 |

**Example 11**

**11.1 Generation of a Dual Targeting anti-TNF/anti-VEGF mAbdAb**

An anti-TNF/anti-VEGF mAbdAb was produced employing a similar strategy described for example 10. For construction of the heavy chain expression cassette, vector DNA encoding the heavy chain of example 10 was taken as a starting point. The anti-EGFR dAb was excised using the restriction enzymes SalI and HindIII. DOM15-26-593, an anti-VEGF dAb was amplified by PCR (using primers coding SalI and HindIII ends) and ligated into the vector backbone which previously had the anti-EGFR dAb excised using the same restriction sites, resulting in a linker of 'STG' (serine, threonine, glycine) between the mAb and the dAb.

Sequence verified clones (SEQ ID NO: 169 and 168 for light and heavy chains respectively) were selected and large scale DNA preparations were made and the anti-TNF/anti-VEGF mAbdAb was expressed in mammalian HEK293-6E cells using transient transfection techniques by co-transfection of light and heavy chains (SEQ ID NO: 73 and 75).

**11.2 Purification and SEC analysis of the Dual Targeting anti-TNF/anti-VEGF mAbdAb**

The anti-TNF/anti-VEGF mAbdAb (designated DMS4000) was purified from clarified expression supernatant using Protein-A affinity chromatography according to established protocols. Concentrations of purified samples were determined by spectrophotometry from measurements of light absorbance at 280nm. SDS-PAGE analysis (figure 47) of the purified sample shows non-reduced sample running at ~170kDa whilst reduced sample shows two bands running at ~25 and ~60kDa corresponding light chain and dAb-fused heavy chain respectively.

For size exclusion chromatography (SEC) analysis the anti-TNF/anti-VEGF mAbdAb was applied onto a Superdex-200 10/30 HR column (attached to an Akta Express FPLC system) pre-equilibrated and running in PBS at 0.5ml/min. The SEC profile shows a single species running as a symmetrical peak (figure 48).

**11.3 Binding Affinities of the Dual Targeting anti-TNF/anti-VEGF mAbdAb**

Binding affinities to VEGF and TNF were determined as described in methods 12 and 14 respectively. Assay data were analysed using GraphPad Prism. Potency values were determined using a sigmoidal dose response curve and the data fitted using the best fit model. Anti-VEGF potency (Figure 49) of this mAbdAb was calculated to be 57pM whilst the control, an anti-VEGF mAb gave an EC50 value of 366pM. In the anti-TNF bioassay (Figure 50) the potency was 10pM whilst an anti-TNF control mAb produced an EC50 of 22pM. In conclusion, assay data shows that the molecule of Example 11, a dual targeting anti-TNF/anti-VEGF mAbdAb is potent against both antigens.

**11.4 Rat PK of the Dual Targeting anti-TNF/anti-VEGF mAbdAb**

This molecule was tested for its *in vivo* pharmacokinetic properties in the rat. The anti-TNF/anti-VEGF mAbdAb was administered i.v. to three rats, and serum samples collected over a period of 10 days (240 hours). The concentration of drug remaining at various time points post-dose was assessed by ELISA against both TNF & VEGF. The results are shown in Figure 126

The PK parameters confirmed that this molecule had *in vivo* pharmacokinetic properties that compared with those of unmodified adalimumab. The shorter observed $t_{1/2}$□ for the VEGF component is not considered to be significant and may be an assay artefact. Further details are shown in Table 17.2

Table 17.2

| Antigen | Half Life (hr) | Cmax (μg/mL) | AUC (0-inf) (hr* μg/mL) | Clearance (mL/hr/kg) | % AUC Extrapolated |
|---|---|---|---|---|---|
| TNF | 180.1 | 89.9 | 7286.3 | 0.7 | 35.8 |
| VEGF | 94.2 | 102.8 | 4747.1 | 1.1 | 14.3 |

**11.5 Generation of an alternative anti-TNF/anti-VEGF mAbdAb**

An alternative anti-TNF/anti-VEGF mAbdAb was constructed in a similar way to that described above in Example 11.1, using the same anti-TNF mAb linked to a VEGF dAb on the C-terminus of the heavy chain using an STG linker. The anti-VEGF dAb used in this case was DOM15-10-11. This molecule was expressed in mammalian HEK293-6E cells using transient transfection techniques by co-transfection of light and heavy chains (SEQ ID NO: 73 and 185). This

molecule expressed to give a mAbdAb of similar expression levels to that described in Example 11.2, however when tested for potency in the same VEGF assay as described in Example 11.3 it was found to have undetectable levels of inhibition of VEGF binding to VEGF receptor in this assay.

## Example 12

### 12.1 Generation of a Dual Targeting anti-VEGF/anti-IL1R1 dAb-extended IgG

Two dual targeting dAb-extended IgG molecules were constructed using standard molecular biology techniques following a strategy of insertion of Dummy V domain coding regions in between dAb and constant regions of both chains.

For the light chain, the anti-IL1R1 dAb DOM4-130-54 was previously cloned into an expression cassette with SalI and BsiWI sites (Figure 51) to produce a dAb-Ck chain. To produce the dAb-extended IgG light chain, Dummy Vk region was amplified by PCR with primers coding BsiWI on both ends. Plasmid containing the dAb-Ck expression cassette was digested with BsiWI. BsiWI digested Dummy Vk domain was ligated into this to produce the dAb-extended IgG light chain, with a linker of 'TVAAPS' between the two variable domains.

An identical strategy was followed to produce the dAb-extended IgG heavy chain where the PCR amplified Dummy VH domain with NheI ends was ligated into an NheI digested dAb heavy chain in between the dAb DOM15-26 and CH1 (Figure 51), with a linker of 'ASTKGPS' between the two variable domains.

This is designated DMS2090 and has the sequence set out in SEQ ID NO: 163 (DNA sequence SEQ ID NO: 162). This was paired with the light chain set out in SEQ ID NO: 77 (DNA sequence SEQ ID NO: 171).

A second heavy chain was constructed in the same way but using the dAb DOM15-26-593. This is designated DMS2091, and has the sequence set out in SEQ ID NO: 76 (DNA sequence SEQ ID NO: 172). This was paired with the light chain set out in SEQ ID NO: 77 (DNA sequence SEQ ID NO: 171).

Sequence verified clones were selected and large scale DNA preparations were made using Qiagen Maxi or Mega Prep Kits following the manufacturer's protocols. The resulting construct was expressed in mammalian cells using transient transfection techniques by co-transfection of light and heavy chains.

### 12.2 Purification and SEC analysis of the Dual Targeting anti-VEGF/anti-IL1R1 mAbdAb

Both anti-IL1R1/anti-VEGF dAb-extended IgG molecules were purified from clarified expression supernatant using Protein-A affinity chromatography according to established protocols. Concentrations of purified samples were determined by spectrophotometry from measurements of light absorbance at 280nm. SDS-PAGE analysis for DMS2090 is shown in Figure 52, and for DMS2091 is shown in Figure 53. Both purified samples show non-reduced samples running at 190kDa whilst the reduced samples show two bands running at 35 and 60kDa corresponding to dAb-extended light chain and heavy chains respectively.

For size exclusion chromatography (SEC) analysis the anti-VEGF/anti-IL1R1 dAb-extended-IgG was applied onto a Superdex-200 10/30 HR column (attached to an Akta Express FPLC system) pre-equilibrated and running in PBS at 0.5ml/min. The SEC profiles for DMS2090 (Figure 54) and DMS2091 (Figure 55) both show a single species running as a symmetrical peak.

### 12.3 Binding Affinities of the Dual Targeting anti-VEGF/anti-IL1R1 mAbdAb

Binding affinities to VEGF and IL1R1 were determined as described in methods 12 and 15 respectively. Assay data were analysed using GraphPad Prism. Potency values were determined using a sigmoidal dose response curve and the data fitted using the best fit model. Anti-VEGF potency (Figure 57) of DMS2090 was calculated to be 158.4pM whilst the control, an anti-VEGF mAb gave an EC50 value of 689.2pM. Anti-VEGF potency (Figure 56) of DMS2091 was calculated to be 55pM whilst the control, an anti-VEGF mAb gave an EC50 value of 766pM.

In the anti-IL1R1 bioassay the potency of DMS2090 (Figure 58) was 32pM whilst an anti-IL1R1 control mAb produced an EC50 of 35pM. The potency of DMS2091 (Figure 59) was 17.47pM whilst an anti-IL1-R1 control mAb produced an EC50 of 35.02pM.

In conclusion, assay data shows that example 12, a dual targeting anti-IL1R1/anti-VEGF dAb-extended IgG is potent against both antigens.

### 12.4 Rat PK of the Dual Targeting anti-VEGF/anti-IL1R1 mAbdAb

This molecule was tested for its *in vivo* pharmacokinetic properties in the rat. The anti-IL1R1/anti-VEGF dAb-extended IgG A was administered i.v. to three rats, and serum samples collected over a period of 7 days (168 hours). The concentration of drug remaining at various time points post-dose was assessed by ELISA against both IL1R1 &

VEGF. The results are shown in Figure 127

The PK parameters are shown in Table 17.3

Table 17.3

| Molecule | Assay Antigen | Half Life | Cmax | AUC (0-inf) | Clearance | % AUC Extrapolated |
|---|---|---|---|---|---|---|
| | | (hr) | (ug/mL) | (hr*ug/mL) | (mL/hr/kg) | |
| DMS2090 | VEGF | 72.1 | 100.4 | 4811.6 | 1.1 | 19 |
| DMS2090 | IL-1R1 | 86.3 | 87.7 | 3467.4 | 1.6 | 23.7 |

## Example 13

### 13.1 Generation of a Triple Targeting anti-TNF/anti-VEGF/anti-EGFR mAbdAb

A triple targeting mAb was constructed using standard molecular biology techniques and following a strategy of insertion of mAb V domain coding regions in between dAb and constant regions of both chains.

For the light chain, the anti-EGFR dAb DOM16-39-542 was previously cloned into an expression cassette with SalI and BsiWI sites (Figure 15) to produce a dAb-Ck chain. To produce the mAbdAb light chain, the mAb VL region was amplified by PCR with primers coding BsiWI on both ends. Plasmid containing the dAb-Ck expression cassette was digested with BsiWI. BsiWI digested mAb VL domain was ligated into this to produce the mAbdAb light chain, resulting in a linker of 'TVAAPS' between the two variable domains.

An identical strategy was followed to produce the mAbdAb heavy chain where the PCR amplified mAb VH region with NheI ends was ligated into an NheI digested dAb heavy chain vector in between the dAb (DOM15-26) and CH1 (Figure 60), resulting in a linker of 'ASTKGPS' between the two variable domains.

Sequence verified clones (amino acid SEQ ID NO: 78 and 79 for heavy and light chains respectively) were selected and large scale DNA preparations were made using Qiagen Mega Prep Kits following the manufacturer's protocols. mAbdAbs were expressed in mammalian cells using transient transfection techniques by co-transfection of light and heavy chains.

### 13.2 Purification of the Triple Targeting anti-TNF/anti-VEGF/anti-EGFR mAbdAb

The anti-TNF/anti-VEGF/anti-EGFR mAbdAb was purified from clarified expression supernatant using Protein-A affinity chromatography according to established protocols. Concentrations of purified samples were determined by spectrophotometry from measurements of light absorbance at 280nm. SDS-PAGE analysis (figure 61) of the purified sample shows non-reduced sample running at 190kDa whilst reduced sample shows two bands running at 35 and 60kDa corresponding to dAb-extended light chain and heavy chains respectively.

### 13.3 Binding Affinities of the Triple Targeting anti-TNF/anti-VEGF/anti-EGFR mAbdAb

Binding affinities to VEGF, EGFR and TNF were determined as described in methods 12, 13 and 14 respectively. Assay data were analysed using GraphPad Prism. Potency values were determined using a sigmoidal dose response curve and the data fitted using the best fit model. Anti-VEGF potency (Figure 62) of this mAbdAb was calculated to be 1.885mM whilst the control, an anti-VEGF mAb gave an EC50 value of 0.145nM.

In the anti-TNF bioassay (Figure 63) the potency was 87pM whilst an anti-TNF control mAb produced an EC50 of 104pM. In the anti-EGFR assay (Figure 64) the triple targeting mAbdAb produced ~20% inhibition at ~300nM. Whilst EC50 values were calculated for VEGF and TNF binding, for EGFR binding a full curve was not produced to calculate an EC50 value.

## Example 14: IGF-1R/VEGF mAbdAb

### 14.1 Construction of IGF-1R/VEGF mAbdAb

Anti IGF-1R variable heavy and variable light gene sequences were originally built *de novo* from PCR of overlapping oligonucleotides. These regions were fused to human IgG1 or kappa constant regions in a mammalian expression vector using standard molecular biology techniques. The gene sequence for an anti VEGF domain antibody was likewise constructed by PCR using overlapping oligonucleotides and fused to the 3' end of either the heavy or light

chain genes of the anti IGF-1R components described above. The fusion incorporated either a two amino acid (GS) linker or an 8 amino acid (TVAAPSGS) linker between the antibody and the domain antibody components. Antibody heavy and light chains were also constructed without the domain antibody and linker sequences. Sequence verified clones were selected for large scale DNA preparations using Endofree Qiagen Maxiprep kit following the manufacturer's protocols.

In sequences SEQ ID NO: 108, 109, 111 and 112, the position of the linker sequence between the antibody and domain antibody is underlined. Alternative variants could be constructed by removing the linker entirely or by using different linkers. Examples of other suitable linkers are provided in SEQ ID NO: 6 and 8 to 11. Table 18 provides a list of the antibodies constructed and expressed.

**Table 18** - Antibodies constructed and tested

| **Identifier** | **Alternative names** | **Antibody** | **Domain antibody** | **Linker** | **SEQ ID NO for heavy chain** | **SEQ NO light chain** | **Site of fusion** |
|---|---|---|---|---|---|---|---|
| BPC1603 | 381H TVAAPSGS 593, DMS4019 | Anti-IGF-1R antibody H0L0 | Dom15-26-593 anti-VEGF | TVAAPSGS | 108 | 113 | Heavy chain C terminus |
| BPC1604 | 381H GS 593, DMS4020 | Anti-IGF-1R antibody H0L0 | Dom15-26-593 anti-VEGF | GS | 109 | 113 | Heavy chain C terminus |
| BPC1605 | 381L TVAAPSGS 593, DMS4021 | Anti-IGF-1R antibody H0L0 | Dom15-26-593 anti-VEGF | TVAAPSGS | 110 | 111 | Light chain C terminus |
| BPC1606 | 381L GS 593, DMS4022 | Anti-IGF-1R antibody H0L0 | Dom15-26-593 anti-VEGF | GS | 110 | 112 | Light chain C terminus |

**Example 14.2: Expression, purification and SEC profile of IGF-1R/VEGF mAbdAb**

Combinations of the heavy and light chain vectors expressed in transient transfections of HEK293-6E. Briefly, 50 ml of HEK293-6E cells at $1.5x10^6$ cells/ml were transfected with 25μg of heavy and 25μg of light chain plasmid previously incubated with 293fectin reagent (Invitrogen # 51-0031). Cells were placed in a shaking incubator at 37°C, 5% $CO_2$, and 95% releative humidity. After 24 hours, tryptone feeding media was added and the cells grown for a further 72 hours. Supernatant was harvested by centrifugation followed by filtration using a 0.22μm filter. The expressed protein was purified using a Protein A sepharose column and dialysed into PBS. Purified protein was analysed by size exclusion chromatography (SEC) and is shown in Figure 65.

An IGF-1R antibody (H0L0) was used as a comparator in the following assays. This molecule has the heavy chain sequence set out in SEQ ID NO: 110, and the light chain sequence set out in SEQ ID NO: 113.

Another mAbdAb with irrelevant specificity was used as a comparator in the following assays. This molecule has the heavy chain sequence set out in SEQ ID NO: 87, and the light chain sequence set out in SEQ ID NO: 13 and is designated BPC1601.

**Example 14.3: IGF-1R Binding ELISA**

A binding ELISA was carried out to test the binding of the purified anti-IGF-1R/VEGF mAbdAbs to IGF-1R. Briefly, ELISA plates coated with anti-polyhistidine (AbCam AB9108) at 1μg/ml and blocked with blocking solution (4% BSA in Tris buffered saline) were loaded with 400ng/ml recombinant human IGF-1R-his tag (R&D Systems 305-GR) in PBS. The plate was incubated for 1 hour at room temp before washing in TBS + 0.05% Tween 20 (TBST). Various concentrations of the purified mAbdAbs were added as well as an anti IGF-1R monoclonal antibody (H0L0) and an irrelevant mAbdAb (BPC1601), diluted in blocking solution. The plate was incubated for 1 hour at room temperature

before washing in TBST. Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results of the binding ELISA are presented in Figure 66 and confirm that all the IGF1R-VEGF mAbdAb variants tested (BPC1603-1606) show binding to IGF-1R at levels comparable to the anti-IGF-1R antibody H0L0. EC50 values were calculated using Cambridgesoft Bioassay software and are as follows: H0L0 (0.1797μg/ml)), BPC1603 (0.1602μg/ml), BPC1604 (0.1160μg/ml), BPC1605 (0.1975μg/ml), BPC1606 (0.1403μg/ml). The irrelevant control bispecific antibody BPC1601 showed now detectable binding to IGF-1R.

## Example 14.4: VEGF Binding ELISA

A binding ELISA was carried out to test the binding of the purified anti IGF-1R/VEGF bispecific antibodies to VEGF. ELISA plates were coated with recombinant human VEGF (GSK) at 400ng/ml in PBS and then blocked in blocking solution (4% BSA in TBS). Various concentrations of the purified mAbdAbs diluted in blocking solution were added and mAbdAb BPC1601 was included as a negative control. The plate was incubated for 1 hour at room temperature before washing in TBST. Binding was detected by the addition of a peroxidase labelled anti human kappa light chain antibody (Sigma A7164) at a dilution of 1/1000 in blocking solution. The plate was incubated for 40 minutes at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results of the binding ELISA are presented in Figure 67 and confirm that all four anti-IGF-1R/VEGF mAbdAbs (BPC1603-1606) can bind to immobilised VEGF. The apparent lower binding activity of BPC1605 and BPC1606 may be attributable to interference between the domain antibody (located at the C-terminus of the light chain) and the detection antibody. EC50 values were calculated using Cambridgesoft Bioassay software and are as follows: BPC1603 (0.044μg/ml), BPC1604 (0.059μg/ml), BPC1605 (0.571μg/ml). It was not possible to calculate an accurate EC50 value for BPC1606 due to the lower response values. The anti-IGF-1R antibody H0L0 and the irrelevant control mAbdAb BPC1601 showed now detectable binding to VEGF.

## Example 14.5: Kinetics of binding to VEGF

A mouse monoclonal against human IgG (Biacore BR-1008-39) was immobilised by primary amine coupling to a CM5 biosensor chip. The antibody constructs were captured using this surface. After capture VEGF was passed over the surface which was then regenerated using 3M $MgCl_2$. The concentrations of VEGF used to generate kinetics were 256, 64, 16, 4, 1 and 0.25nM, with a buffer only injection over the captured surface used for double referencing. The experiments were carried out on the Biacore T100 machine, using 1x HBS-EP buffer (BR-1006-69) at 25°C. The data were fitted to the 1:1 model inherent to the machine in its analysis software. The data shown in Table 19 is from two independent experiments.

**Table 19 - Kinetics of binding to human VEGF**

| | Experiment 1 | | | Experiment 2 | | |
|---|---|---|---|---|---|---|
| Construct | ka | kd | KD (nM) | ka | kd | KD (nM) |
| BPC1603 | 2.398E+6 | 2.762E-4 | 0.115 | 1.334E+6 | 3.497E-4 | 0.262 |
| BPC1604 | 9.933E+5 | 3.092E-4 | 0.311 | 5.806E+5 | 3.266E-4 | 0.563 |
| BPC1605 | 1.599E+6 | 2.161E-4 | 0.135 | 1.089E+6 | 2.727E-4 | 0.251 |
| BPC1606 | 4.343E+5 | 1.573E-4 | 0.362 | 2.717E+5 | 1.607E-4 | 0.591 |

## Example 14.6: Inhibition of VEGF binding to receptor

The activity of the mAbdAbs was measured using a VEGF receptor binding assay as described in Method 12. The IC50s obtained in this assay for inhibition of the binding of VEGF to VEGFR2 are:

BPC1603 (0.037nM)

BPC1604 (0.010nM)
BPC1605 (0.167nM)
BPC1606 (0.431nM)

These results confirm that all four antigen binding constructs inhibit ligand binding to receptor.

**Example 14.7: Inhibition of IGF-1R receptor phosphorylation**

3T3/LISN c4 cells were plated at a density of 10 000 cells/well into 96 well plates and incubated overnight in complete DMEM (DMEM-Hepes modification +10%FCS). Purified mAbdAbs were added to the cells and incubated for 1 hour. rhIGF-1 was added to the treated cells to achieve a final concentration of 50ng/ml and incubated for a further 30 mins to stimulate receptor phosphorylation. The media was aspirated and then the cells lysed by the addition of RIPA lysis buffer (150mM NaCl, 50mM TrisHCl, 6mM Na Deoxycholate, 1% Tween 20) plus protease inhibitor cocktail (Roche 11 697 498 001). The plate was frozen overnight. After thawing, lysate from each well was transferred to a 96 well ELISA plate pre-coated with an anti IGF-1R capture antibody 2B9 (GSK) at 2μg/ml and blocked with 4%BSA/TBS. The plate was washed with TBST (TBS+0.1%Tween 20) and a Europium labelled anti Phosphotyrosine antibody (PerkinElmer DELFIA Eu-N1 PT66) diluted 1/2500 in 4%BSA/TBS was added to each well. After 1 hour incubation the plate was washed and DELFIA Enhancement (PerkinElmer 1244-105) solution added. After 10 min incubation the level of receptor phosphorylation was determined using a plate reader set up to measure Europium time resolved fluorescence (TRF).
The results of the experiment are presented in Figures 68 and 69. The results confirm that the mAbdAbs BPC1603-1606 can inhibit IGF-I mediated receptor phosphorylation at levels comparable to the anti-IGF-1R monoclonal antibody H0L0. an irrelevant antibody (labelled as IgG1, Sigma I5154) showed no activity in this assay.

**Example 15 anti-CD20/IL-13 antigen binding protein**

**Example 15.1: Molecular biology**

The mammalian expression vectors encoding the heavy and light chain sequences of an anti-CD20 mAb set out in SEQ ID NO: 117 and 120 were constructed de novo using a PCR based approach and standard molecular biology techniques. Bispecific anti-CD20mAb-anti-IL13dAb heavy and light chains were constructed by cloning the sequences encoding anti-CD20 mAb heavy and light variable regions into mammalian expression vectors containing human antibody constant regions fused to an anti-human IL-13 domain antibody (DOM10-53-474).
The mAbdAb expression constructs were transfected into CHOE1a cells. The supernatant was harvested and then the antibody purified using immobilised Protein A and quantified by reading absorbance at 280nm. The mAbdAbs (and the anti-CD20 control mAb) constructed and tested are listed in Table 20.

Table 20

| **Identifier** | **Alternative names** | **Antibody Target** | **Domain antibody** | **Linker** | **Seq ID: for heavy chain** | **Seq ID: for light chain** | **Site of fusion** |
|---|---|---|---|---|---|---|---|
| BPC1401 | RituxanH-TVAAPSGS-DOM474 | CD20 | DOM10-53-474 | TVAAPS GS | 116 | 117 | Heavy chain C -term |
| BPC1402 | RituxanH-GS-DOM474 | CD20 | DOM10-53-474 | GS | 118 | 117 | Heavy chain C -term |
| BPC1403 | RituxanL-TVAAPSGS-DOM474 | CD20 | DOM10-53-474 | TVAAPS GS | 120 | 119 | Light chain C -term |
| BPC1404 | RituxanL-GS-DOM474 | CD20 | DOM10-53-474 | GS | 120 | 121 | Light chain C -term |
| anti-CD20 mAb | Rituxan | CD20 | - | - | 120 | 117 | - |

**Example 15.2: Kinetics of binding to human IL-13**

The binding affinity of mAbdAb constructs for human IL-13 were assessed by BIAcore™ analysis. Analyses were carried out anti-human IgG capture. Briefly, Anti-human IgG (Biacore BR-1008-39) was coupled onto a CM5 chip by primary amine coupling. MAbdAb constructs were then captured onto this surface and human IL-13 (made and purified at GSK) passed over at defined concentrations. The surface was regenerated back to the Anti-human IgG surface using 3M $MgCl_2$. This treatment did not significantly affect the ability to capture antibody for a subsequent IL-13 binding event. The runs were carried out at 25°C using HBS-EP buffer, on the BIAcore™ T100 machine. Data were analysed using the evaluation software in the machine and fitted to the 1:1 binding model. The results of the analysis are presented in Table 48, confirming that for all mAbdAb constructs, the kinetics of binding to IL-13 are comparable.

Table 48 - Surface plasmon resonance (BIAcore™) data

| Antibody name | Ka ($M^{-1}.s^{-1}$) | Kd ($s^{-1}$) | KD (nM) |
|---|---|---|---|
| BPC1401 | 5.81E+5 | 1.82E-4 | 0.313 |
| BPC1402 | 8.52E+5 | 3.05E-4 | 0.358 |
| BPC1403 | 1.07E+6 | 2.95E-4 | 0.277 |
| BPC1404 | 4.99E+5 | 5.08E-4 | 1.02 |
| PascoH-474 GS removed | 6.29E+5 | 2.66E-4 | 0.423 |
| anti-CD20 mAb | No binding detected | | |

Binding of the mAb-dAbs to CD20 was assessed by flow cytometry using a CD20 positive cell line (Wein133). All mAb-dAbs (BPC1401-BPC1404) and the anti-CD20 control antibody showed a dose dependent increase in mean fluorescence intensity (MFI) (data not shown).

**Example 15.3: ADCC assay with anti-CD20/IL-13 bispecific antibody**

The ADCC assay was based on the published method of Boyd et al. (1995) J. Imm. Meth. 184:29-38. Briefly, Raji cells (targets) were labelled with Europium as follows. Cells were harvested, counted and prepared to a final density of $1x10^7$ in a 15ml falcon tube, wash once with Hepes buffer (50mM HEPES, 83mM NaCl, 5mM KCl, 2mM $MgCl_2.H_20$, pH7.4). The cells were pelleted and 1ml of ice cold Europium labelling buffer (HEPES buffer plus 600μM $EuCl_3$, 3mM DTPA and 25mg/ml Dextran sulphate) was added to each tube. The cell suspension was flicked vigorously at the start of the labelling and then every 10 minutes during the 30 minute incubation period on ice. 10ml ice cold repair buffer (Hepes buffer containing 294mg/l $CaCl_2.2H_2O$, 1.8g/l D-Glucose, pH7.4) was added and the cells incubated on ice for a further 10 minutes. The cells were then centrifuged, the supernatant decanted and washed twice with repair buffer and then once with complete medium. The labelled cells were then counted and resuspended in serum free medium at $2x10^5$ cells/ml and stored on ice.

Human purified blood mononuclear cells (PBMCs or effector cells) were prepared as follows. 150mls of whole blood was centrifuged at 2000rpm for 10mins to remove the serum. The cells were the diluted to twice the original volume with PBS (Invitrogen/Gibco, #14190). Accuspin density gradient tubes (Sigma, #A2055-10EA) were prepared by adding 15ml lymphoprep (Axis shield, #NYC1114547) and centrifuged for 1min at 1500rpm. 25ml of blood suspension was added to the density gradient tubes and centrifuged for 20min at 2500rpm with the centrifuge brake off. The top 10ml of supernatant was discarded. The remainder (including the "buff" layer) was poured into a clean tube, topped up with PBS and centrifuged at 1500rpm for 5mins. The supernatant was discarded, the cell pellets pooled, wash once in RPMI medium, recentrifuged and counted. Effector cells at were prepared at $5x10^6$/ml in serum free RPMI medium.

The assay plates were set up in 96-well round bottom plates (Nunc 96 maxisorb plate, #735-0199) as follows. Antibody dilutions were made in serum free RPMI medium at a starting concentration of approximately 12μg/ml and eleven further 3-fold dilutions. Using the plate layout below, 50μl antibody sample was added to the appropriate wells (rows B-G only), allowing 6 replicates per dilution). 50μl RPMI medium was added to all wells in rows A and H. 50μl of RPMI medium was added to all wells in plates labelled medium. 50μl recombinant human IL-13 diluted in RPMI medium to 4μg/ml (1μg/ml final concentration, GSK in-house material) was added to all wells in plates labelled +IL13. All plates were incubated at 4°C for minimum 30 minutes. 50μl of Europium labelled target cells were added to all plates. 20μl of a 10X triton was added to all wells in row H on all plates. Plates were incubated 4°C for a minimum of 30 minutes. 50μl RPMI medium was added to all wells in columns labelled targets alone. 50μl PBMCs was added to all wells in columns labelled effector:targets to give a 25:1 ratio. The plates were centrifuged at 1500rpm for 3mins and incubated at 37°C

for 3-4hrs. 200μl of enhancement solution (Wallac/Perkin Elmer, Catalogue# 1244-105) was added to each well of a nunc immunosorbant ELISA plates (one ELISA plate for each assay plate). 20μl of supernatant was transferred from assay plate to ELISA plate. The ELISA plates were incubated at room temperature on plate shaker for a minimum 30 minutes or stored over night at 4°C. Europium release is measured using time-delayed fluorimetry (Wallac Victor plate reader). Spontaneous lysis = measurement of Europium released from cells and medium alone. Maximum lysis = non-specific lysis of target cells by addition of Triton-X100 (non-ionic detergent).

| | Effector:Targets | | | TargetsEffector:Targets | | | | | Targets | | | |
|---|---|---|---|---|---|---|---|---|---|---|---|---|
| | 1 | 2 | 3 | 4 | 5 | 6 | 7 | 8 | 9 | 10 | 11 | 12 |
| A | Spontaneous release | | | | | | Spontaneous release | | | | | |
| B | 3μg/ml | | | | | | 0.003μg/ml | | | | | |
| C | 1μg/ml | | | | | | 0.001μg/ml | | | | | |
| D | 0.3μg/ml | | | | | | 0.0003μg/ml | | | | | |
| E | 0.1μg/ml | | | | | | 0.0001μg/ml | | | | | |
| F | 0.03μg/ml | | | | | | 0.00003μg/ml | | | | | |
| G | 0.01μg/ml | | | | | | 0.00001μg/ml | | | | | |
| H | Maximum release | | | | | | Maximum release | | | | | |

The ADCC assay was performed on two separate occasions using two different donor PBMCs. The results from one representative assay are presented in Figures 70 and 71. In addition, a similar ADCC assay using a shorter dose range was performed on a separate occasion using different donor PBMCs. The results from this assay are presented in Figure 72 and 73.

**Example 15.4: CDC assay with anti-CD20/IL-13 bispecific antibody**

WEIN cells (targets) were labelled with Europium as follows. Briefly, cells were harvested, counted and prepared to a final density of $1x10^7$ in a 15ml falcon tube, wash once with Hepes buffer (50mM HEPES, 83mM NaCl, 5mM KCl, 2mM $MgCl_2.H_20$, pH7.4). The cells were pelleted and 1 ml of ice cold Europium labelling buffer (HEPES buffer plus 600μM $EuCl_3$, 3mM DTPA and 25mg/ml Dextran sulphate) was added to each tube. The cell suspension was flicked vigorously at the start of the labelling and then every 10 minutes during the 30 minute incubation period on ice. 10ml ice cold repair buffer (Hepes buffer containing 294mg/l $CaCl_2.2H_2O$, 1.8g/l D-Glucose, pH7.4) was added and the cells incubated on ice for a further 10 minutes. The cells were then centrifuged, the supernatant decanted and washed twice with repair buffer and then once with complete medium. The labelled cells were then counted and resuspended in serum free medium at $2x10^5$ cells/ml and stored on ice.

Serum was removed from whole blood collected from in house donors by centrifugation. Half of the sample was inactivated by heat treatment at 56°C for 30mins. Antibodies samples were diluted in serum free RPMI medium, starting a 12μg/ml with five further 3-fold dilutions. 50μl antibody sample was added to appropriate wells in rows B-G only (as per the plate layout below). 50μl RPMI medium was added to all wells in columns 1 to 6. Where indicated, 50μl recombinant human IL-13 (at 4μg/ml in RPMI medium) was added to all wells in columns 7 to 12. The plates were incubated at 4°C for a minimum 30 minutes. 50μl of Europium labelled target cells were added to all the plates and the plates incubated at 4°C for minimum of 30 minutes. 50μl of serum (active or heat-inactivated) was added to the appropriate wells (see plate layout below). The plates were incubated at 37°C incubator for 2-3hrs, after which time the plates were centrifuged at 1500rpm for 3mins. 200μl of enhancement solution (Wallac/Perkin Elmer, Catalogue# 1244-105) was added to each well of a Nunc immunosorbant ELISA plates (one ELISA plate for each assay plate). 20μl of supernatant was transferred from assay plate to ELISA plate. The ELISA plates were incubated at room temperature on plate shaker for a minimum 30 minutes or stored over night at 4°C. Europium release is measured using time-delayed fluorimetry (Wallac Victor plate reader). Spontaneous lysis = measurement of Europium released from cells and medium alone. Maximum lysis = non-specific lysis of target cells by addition of Triton-X100 (non-ionic detergent).

| | MEDIUM | | | | | | IL13 | | | | | |
|---|---|---|---|---|---|---|---|---|---|---|---|---|
| | Active complement | | | Heat treated | | | Active complement | | | Heat treated | | |
| | 1 | 2 | 3 | 4 | 5 | 6 | 7 | 8 | 9 | 10 | 11 | 12 |
| A | Spontaneous release | | | | | | | | | | | |
| B | 3μg/ml | | | | | | | | | | | |
| C | 1μg/ml | | | | | | | | | | | |
| D | 0.3μg/ml | | | | | | | | | | | |
| E | 0.1μg/ml | | | | | | | | | | | |
| F | 0.03μg/ml | | | | | | | | | | | |
| G | 0.01μg/ml | | | | | | | | | | | |
| H | Maximum release | | | | | | | | | | | |

The CDC assay was performed on three separate occasions using three different donor sera. The results from one representative assay are presented in Figures 74 and 75 and show that the CDC activity of the antibody samples is comparable in the absence of IL-13. In the presence of excess IL-13, the CDC activity of antibody samples BPC1401 and BPC1402 (domain antibody fused to the heavy chain) is reduced whilst the CDC activity of BPC1403 and BPC1404 (domain antibody fused to the light chain) is largely unaffected by the presence of IL-13.

**Example** 16

**16.1 Design and construction of antigen binding proteins comprising epitope binding domains composed of alternative scaffolds**

Five alternative scaffolds, listed below, were combined with monoclonal antibodies to provide mAb-alternative scaffold bispecific molecules:

- anti VEGF tear lipocalin (TLPC)
- anti HER2 Affibody (AFFI)
- anti HER2 DARPin (DRPN)
- anti hen egg white lysozyme (NARV)
- anti-RNaseA Camelid VHH

The protein sequences of TLPC (for further information see US2007/0224633), AFFI (for further information see WO2005003156A1), DRPN (for further information see Zahnd, C. et al. (2007), J. Mol. Biol., 369, 1015-1028) and NARV (for further information see US20050043519A) were reverse-translated to DNA and codon optimised. A BamHI site at the N-terminus and EcoR1 site at the C-terminus were included on each of these four alternative scaffolds to facilitate cloning. DNA fragments encoding the four final alternative scaffold DNA sequences were constructed de novo using a PCR-based strategy and overlapping oligonucleotides. The TLPC, AFFI and DRPN PCR products were cloned into mammalian expression vectors containing the heavy chain of H0L0, an anti-hIGF-1R antibody. The resulting DNA sequences encode the alternative scaffolds fused onto the C-terminus of the heavy chain via a TVAAPSGS linker or GS linker. The NAR V PCR product was cloned into mammalian expression vectors containing DNA encoding the heavy chain of Pascolizumab (an anti-IL-4 antibody). The resulting DNA sequence encodes the NAR V fused onto the C-terminus of the heavy chain via a GS linker.

An anti-RNAse A camelid VHH DNA sequence was modified by PCR to include a BamHI site at the 5' end and an EcoR1 site at the 3' end in order to facilitate cloning. The PCR product was cloned into mammalian expression vectors containing the heavy chain of Pascolizumab, an anti-IL4 antibody. The resulting DNA sequence encodes a camelid VHH fused onto the C-terminus of the heavy chain via a GS linker.

Table 21 below is a summary of the antigen binding proteins that have been constructed.

Table 21

| Antibody ID | Description | SEQ ID NO: amino acid sequence |
|---|---|---|
| BPC1803 | antiIGF1R Heavy Chain-GS-TLPC | 123 |
| | antiIGF1R Light Chain | 113 |
| BPC1804 | antiIGF1R Heavy Chain-TVAAPSGS-TLPC | 125 |
| | antiIGF1R Light Chain | 113 |
| BPC1805 | antiIGF1R Heavy Chain-GS-AFFI | 126 |
| | antiIGF1R Light Chain | 113 |
| BPC1806 | antiIGF1R Heavy Chain-TVAAPSGS-AFFI | 127 |
| | antiIGF1R Light Chain | 113 |
| BPC1807 | antiIGF1R Heavy Chain-GS-DRPN | 128 |
| | antiIGF1R Light Chain | 113 |
| BPC1808 | antiIGF1R Heavy Chain-TVAAPSGS-DRPN | 129 |
| | antiIGF1R Light Chain | 113 |
| BPC1809 | Anti IL-4 heavy Chain-GS-anti RNAse A camelidVHH | 130 |
| | Anti IL-4 Light Chain | 15 |
| BPC1816 | Anti IL-4 heavy Chain-GS-NARV | 131 |
| | Anti IL-4 Light Chain | 15 |

Expression plasmids encoding the heavy and light chains of the antigen binding proteins set out in Table 21 were transiently co-transfected into HEK 293-6E cells using 293fectin (Invitrogen, 12347019). A tryptone feed was added to each of the cell cultures the same day or the following day and the supernatant material was harvested after about 2 to 6 days from initial transfection. The antigen binding protein was purified from the supernatant using a Protein A column before being tested in binding assays.

**16.2: rhIGF-1R Binding ELISA**

96-well high binding plates were coated with 1μg/ml of anti-his-tag antibody (Abcam, ab9108) in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1hour at room temperature. Another wash step was then performed. 0.4μg/mL of rhIGF-1R (R&D systems) was added to each well at 50μL per well. The plates were incubated for an hour at room temperature and then washed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well.

The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figures 76, 78 and 80 show the ELISA results and confirm that antigen binding proteins BPC1803 - BPC1808 bind to recombinant human IGF-1R. The anti-IGF-1R monoclonal antibody H0L0 also showed binding to recombinant human IGF-1R whereas the negative control antibody (sigma I5154) showed no binding to IGF-1R.

**16.3: VEGF Binding ELISA**

96-well high binding plates were coated with 0.4μg/mL of hVEGF165 (R&D Systems) and incubated at +4°C overnight. The plates were washed twice with Tris-Buffered Saline with 0.05% of Tween-20. 200μL of blocking solution (5% BSA in DPBS buffer) was added to each well and the plates were incubated for at least 1 hour at room temperature. Another wash step was then performed. The purified antigen binding proteins/antibodies were successively diluted across the plates in blocking solution. After 1 hour incubation, the plates were washed. Goat anti-human kappa light

chain specific peroxidase conjugated antibody was diluted in blocking solution to 1μg/mL and 50μL was added to each well. The plates were incubated for one hour. After another wash step, 50μl of OPD (o-phenylenediaminedihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 77 shows the results of the VEGF binding ELISA and confirms that bispecific antibodies BPC1803 and BPC1804 bind to human VEGF. An anti VEGF bispecific antibody (BPC1603) was used as a positive control in this assay and showed binding to VEGF. In contrast the anti-IGF-1R monoclonal antibody H0L0 showed no binding to human VEGF.

### 16.4: HER2 Binding ELISA

96-well high binding plates were coated with 1μg/mL of HER2 (R&D Systems) and incubated at +4°C overnight. The plates were washed twice with Tris-Buffered Saline with 0.05% of Tween-20. 200μL of blocking solution (5% BSA in DPBS buffer) was added to each well and the plates were incubated for at least 1 hour at room temperature. Another wash step was then performed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediaminedihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figures 79 and 81 show the results of the HER2 binding ELISA and confirms that the antigen binding proteins BPC1805, BPC1806, BPC1807 and BPC1808 bind to recombinant human HER2. Herceptin was used as a positive control in this assay and showed binding to HER2. In contrast the anti-IGF-1R monoclonal antibody H0L0 showed no binding to human HER2.

### 16.5: IL-4 Binding ELISA

96-well high binding plates were coated with 5μg/ml of human IL-4 in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1hour at room temperature. Another wash step was then performed. The purified antigen binding proteins/antibodies were successively diluted across the plates in blocking solution. After 1 hour incubation, the plates were washed. Goat anti-human kappa light chain specific peroxidase conjugated antibody (Sigma, A7164) was diluted in blocking solution to 1μg/mL and 50μL was added to each well. The plates were incubated for one hour. After another wash step, 50μl of OPD (o-phenylenediaminedihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 82 shows the results of the ELISA and confirms that antigen binding protein BPC1809 binds to human IL-4 at levels comparable to the anti IL-4 monoclonal antibody, Pascolizumab. The negative control antibody (Sigma I5154)showed no binding to IL-4.

Figure 84 shows the results of the ELISA and confirms that antigen binding protein BPC1816 binds to human IL-4 at levels comparable to the anti IL-4 monoclonal antibody, Pascolizumab. The negative control antibody (Sigma I5154)showed no binding to IL-4.

### 16.6: RNAse A Binding ELISA

50uL of 1ug/mL RNAse A (Qiagen, 19101) that had been diluted in PBS was added to each well of a 96 well Costar plate. The plate was incubated for 2 hours at room temperature then washed with PBST before addition of 200uL of 4% BSA/PBS block to each well. The plate was incubated for an hour and washed before addition of the samples. Purified antibodies and antigen binding protein BPC1809 were added at a concentration of 2ug/mL in wells of column 1 then serially diluted 1 in 2 across the plate in block. The plate was incubated for an hour then washed. 50ul/well of Goat anti-human kappa light chain specific peroxidase conjugated antibody (Sigma, A7164) was added at a 1 in 1000 dilution. The plate was incubated for an hour then washed. 50uL of OPD was added to each well and the reaction was stopped with 3M sulphuric acid after 15-30 minutes. Absorbance was read at 490nm using the VersaMax Tunable Microplate Reader (Molecular Devices) using a basic endpoint protocol.

Figure 83 shows the results of the RNAse A binding ELISA and confirms that purified human monoclonal

antibody-camelid VHH bispecific antibody BPC1809 shows binding to RNAse A. In contrast both the IL-4 monoclonal antibody Pascolizumab and the negative control (sigma I5154) showed no binding to RNAse A.

**16.7: HEL Binding ELISA**

A 96-well high binding plate was coated with 5μg/ml of HEL (Hen Egg Lysozyme, Sigma L6876) in PBS and stored overnight at 4°C. The plate was washed twice with Tris-Buffered Saline with 0.05% of Tween-20. 200μL of blocking solution (5% BSA in DPBS buffer) was added in each well and the plate was incubated for at least 1hour at room temperature. Another wash step was then performed. The purified antibodies were successively diluted across the plate in blocking solution. After 1 hour incubation, the plate was washed. Goat anti-human kappa light chain specific peroxidase conjugated antibody (Sigma, A7164) was diluted in blocking solution to 1μg/mL and 50μL was added to each well. The plate was incubated for one hour. After another wash step, 50μl of OPD (o-phenylenediaminedihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 85 shows the results of the HEL binding ELISA and confirms that purified human monoclonal antibody - NAR V bispecific antibody BPC1816 binds to HEL. In contrast the IL-4 monoclonal antibody Pascolizumab showed no binding to HEL.

**Example 17**

**17.1 Design and construction of antigen-binding proteins comprising epitope binding domains composed of adnectin**

CT01 adnectin is specific for VEGFR2 (for further information see WO2005/056764). The CT01 adnectin protein sequence was reverse-translated to DNA and codon optimised. A BamHI site at the N-terminus and EcoR1 site at the C-terminus were included to facilitate cloning. DNA fragments encoding the final CT01 DNA sequence were constructed de novo using a PCR-based strategy and overlapping oligonucleotides. The PCR product was cloned into mammalian expression vectors containing the heavy chain of H0L0 (an anti-hIGF-1R antibody) allowing the adnectin to be fused onto the C-terminus of the heavy chain via either a GS linker or a TVAAPSGS linker. Protein sequences of the heavy and light chains of the IGF-1R - VEGFR2 bispecific are given in SEQ ID numbers 124, 113 and 133.

Another adnectin protein sequence coding for an anti-TNF-α adnectin (for further information see US20080139791) was reverse translated to DNA, codon optimised and modified to include terminal BamHI and EcoR1 sites before being constructed using the overlapping oligonucleotide PCR method described previously. The PCR product was cloned into mammalian expression vectors containing DNA encoding the heavy chain of Pascolizumab (an anti-IL-4 antibody) allowing DNA encoding the anti-TNF-a adnectin to be fused onto the C-terminus of the heavy chain via either a GS linker or a TVAAPSGS linker. Protein sequences of the heavy and light chains of the IL-4 - TNF-a bispecific are given in SEQ ID NO: 146, 147 and 15.

Antigen binding proteins using the TNF- a specific adnectin fused at the C-terminus of the heavy chain of an IL-13 monoclonal antibody have also been designed. Example protein sequences are given in SEQ ID's 134, 13 and 135. In addition, bispecific molecules based on the fusion of CT01 at either the C-terminus or the N-terminus of the heavy or light chain of anti-EGFR antibodies Erbitux and IMC-11F8 have been designed. Examples of protein sequences which have been designed are given in SEQ ID NO: 136-145.

Of these example sequences, a number were constructed. DNA sequences encoding SEQ ID NO 136 (CT01 fused onto the C-terminus of the Erbitux heavy chain), SEQ ID NO 144 (CT01 fused onto the N-terminus of Erbitux heavy chain) and SEQ ID NO 138 (CT01 fused onto the C-terminus of the Erbitux light chain) were constructed. All three sequences were constructed using PCR-based cloning methods and cloned into mammalian expression vectors. Table 22 below is a summary of the antigen binding proteins that have been designed and/or constructed.

Table 22

| Antibody ID | Description | SEQ ID NO: amino acid sequence |
|---|---|---|
| BPC1801 (constructed) | antiIGF1R Heavy Chain-GS-CT01 adnectin | 124 |
| | antiIGF1R Light Chain | 113 |
| BPC1802 (constructed) | antiIGF1R Heavy Chain-TVAAPSGS-CT01 adnectin | 133 |
| | antiIGF1R Light Chain | 113 |

(continued)

| Antibody ID | Description | SEQ ID NO: amino acid sequence |
|---|---|---|
| BPC1810 (designed) | antiIL13-Heavy Chain-GS-antiTNFa adnectin | 134 |
| | antiIL13-Light Chain | 13 |
| BPC1811 (designed) | antiIL13-Heavy Chain-TVAAPSGS-antiTNFa adnectin | 135 |
| | antiIL13-Light Chain | 13 |
| BPC1812 (constructed) | Erbitux Heavy chain-RS-CT01 adnectin | 136 |
| | Erbitux Light Chain | 137 |
| BPC1813 (constructed) | Erbitux Light chain-RS-CT01 adnectin | 138 |
| | Erbitux Heavy Chain | 139 |
| BPC1814 (designed) | 11F8 Heavy Chain-GS-CT01 adnectin | 140 |
| | 11F8 Light Chain | 141 |
| BPC1815 (designed) | 11F8 Light Chain-GS-CT01 adnectin | 142 |
| | 11F8 Heavy Chain | 143 |
| BPC1818 (constructed) | GS-CT01 adnectin-GSTG- Erbitux Heavy Chain | 144 |
| | Erbitux Light Chain | 137 |
| BPC1819 (designed) | CT01 adnectin-STG-Erbitux Light Chain | 145 |
| | Erbitux Heavy Chain | 139 |
| BPC1823 (constructed) | Anti IL-4 Heavy Chain-GS-anti TNF-a adnectin | 146 |
| | Anti IL-4 Light Chain | 15 |
| BPC1822 (constructed) | Anti IL-4 Heavy Chain-TVAAPSGS- anti TNF-a adnectin | 147 |
| | Anti IL-4 Light Chain | 15 |

Expression plasmids encoding the heavy and light chains of BPC1801, BPC1802, BPC1822, BPC1823, BPC1812, BPC1813 and BPC1818 were transiently co-transfected into HEK 293-6E cells using 293fectin (Invitrogen, 12347019). A tryptone feed was added to the cell culture the same day or the following day and the supernatant material was harvested after about 2 to 6 days from initial transfection. In some instances the supernatant material was used as the test article in binding assays. In other instances, the antigen binding protein was purified using a Protein A column before being tested in binding assays.

**17.2: rhIGF-1R Binding ELISA**

96-well high binding plates were coated with 1μg/ml of anti-his-tag antibody (Abcam, ab9108) in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1hour at room temperature. Another wash step was then performed. 0.4μg/mL of rhIGF-1R (R&D systems) was added to each well at 50μL per well. The plates were incubated for an hour at room temperature and then washed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol. Figure 86 shows the results of the IGF-1R binding ELISA and confirms that purified human monoclonal antibody-adnectin bispecific antibodies (BPC1801 and BPC 1802) bind to recombinant human IGF-1R at levels comparable to the anti-IGF-1R monoclonal antibody H0L0. The negative control antibody (Sigma I5154)showed no binding to IGF-1R.

## 17.3: VEGFR2 Binding ELISA

96-well high binding plates were coated with 0.4μg/mL of VEGFR2 (R&D Systems) and incubated at +4°C overnight. The plates were washed twice with Tris-Buffered Saline with 0.05% of Tween-20. 200μL of blocking solution (5% BSA in DPBS buffer) was added to each well and the plates were incubated for at least 1 hour at room temperature. Another wash step was then performed. The supernatants or purified antibodies were successively diluted across the plates in blocking solution. After 1 hour incubation, the plate was washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediaminedihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 87 shows the results of the VEGFR2 binding ELISA and confirms that purified human monoclonal antibody-adnectin bispecific antibodies (BPC1801 and BPC1802) bind to recombinant human VEGFR2. In contrast the anti-IGF-1R monoclonal antibody H0L0 showed no binding to human VEGFR2.

Figure 172 shows the results of the VEGFR2 binding ELISA and confirms that antigen binding proteins BPC1818 and BPC1813 bind to recombinant human VEGFR2. In contrast Erbitux showed no binding to human VEGFR2. For the antigen binding proteins BPC1813 and BPC1818, the amount of antibody in the supernatant was not quantified thus the data presented in Figure 172 is represented as a dilution factor of the neat supernatant material. For Erbitux, purified material was used in the assay aT the starting concentration of 2μg/ml, which is equivalent to dilution factor of 1 in Figure 172.

Figure 175 shows the results of the VEGFR2 binding ELISA and confirms that antigen binding protein BPC1812 binds to recombinant human VEGFR2. In contrast Erbitux and the negative control Sigma IgG I5154 antibody showed no binding to human VEGFR2. For the antigen binding protein BPC1812, the amount of antibody in the supernatant was not quantified thus the data presented in Figure 175 is represented as a dilution factor of the neat supernatant material. For Erbitux and Sigma IgG I5154 purified material was used in the assay at the starting concentration of 2μg/ml, which is equivalent to dilution factor of 1 in Figure 175.

## 17.4: IL-4 Binding ELISA

96-well high binding plates were coated with 5μg/ml of human IL-4 in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1hour at room temperature. Another wash step was then performed. The supernatant or purified antibodies/antigen binding proteins were successively diluted across the plate in blocking solution. After 1 hour incubation, the plates were washed. Goat anti-human kappa light chain specific peroxidase conjugated antibody (Sigma, A7164) was diluted in blocking solution to 1μg/mL and 50μL was added to each well. The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 88 shows the results of the IL-4 binding ELISA and confirms that human monoclonal antibody-adnectin bispecific antibodies (BPC1823 and BPC 1822) bind to recombinant human IL-4. The positive control anti-IL-4 monoclonal antibody Pascolizumab showed binding to IL-4 and the negative control antibody (Sigma I5154) showed no binding to IL-4.

The HEK transfection for this experiment was repeated to obtain supernatant material with a higher antibody concentration. Figure 88b shows binding of this higher concentration supernatant human monoclonal antibody-adnectin bispecific antibody (BPC1823) to human IL-4 as determined by ELISA

For the antigen binding proteins BPC1823 and BPC1822, the amount of antibody in the supernatant was not quantified thus the data presented in Figure 88 and 88b is represented as a dilution factor of the neat supernatant material. For Pascolizumab and the negative control antibody (Sigma I5154), purified material was used in the assay and the starting concentration of 1μg/ml, which is equivalent to dilution factor of 1 in Figure 88 and 88b.

## 17.5: TNF-a Binding ELISA

A 96-well high binding plate was coated with 0.4μg/ml of recombinant human TNFα (RnD Systems 210-TA-050/CF) in PBS and stored overnight at 4°C. The plate was washed twice with Tris-Buffered Saline with 0.05% of Tween-20. 200μL of blocking solution (5% BSA in DPBS buffer) was added in each well and the plate was incubated for at least 1hour at room temperature. Another wash step was then performed. The supernatant or purified antibodies were successively diluted across the plate in blocking solution. After 1 hour incubation, the plate was washed. Goat anti-human

kappa light chain specific peroxidase conjugated antibody (Sigma, A7164) was diluted in blocking solution to 1μg/mL and 50μL was added to each well. The plate was incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 89 shows the results of the TNF-a binding ELISA and confirms that human monoclonal antibody-adnectin bispecific antibodies (BPC1823 and BPC1822) bind to recombinant human TNF-a. In contrast the anti-IL-4 monoclonal antibody Pascolizumab showed no binding to recombinant human TNF-a.

The HEK transfection for this experiment was repeated to obtain supernatant material with a higher antibody concentration. Figure 89b shows binding of this higher concentration supernatant human monoclonal antibody-adnectin bispecific antibody (BPC1823) to recombinant human TNF-a as determined by ELISA. The IgG control showed no binding to recombinant human TNF-a.

For the antigen binding proteins BPC1822 and BPC1823, the amount of antibody in the supernatant was not quantified thus the data presented in Figure 89 and 89b is represented as a dilution factor of the neat supernatant material. For Pascolizumab, purified material was used in the assay at the starting concentration of 1μg/ml, which is equivalent to dilution factor of 1 in Figure 89 and 89b.

<u>**17.6: EGFR Binding ELISA**</u>

A 96-well high binding plate was coated with 0.67μg/ml of recombinant human EGFR protein in PBS and stored overnight at 4°C. The plate was washed twice with Tris-Buffered Saline with 0.05% of Tween-20. 200μL of blocking solution (5% BSA in DPBS buffer) was added in each well and the plate was incubated for at least 1hour at room temperature. Another wash step was then performed. The antigen binding proteins/antibodies were successively diluted across the plate in blocking solution. After 1 hour incubation, the plate was washed. Goat anti-human kappa light chain specific peroxidase conjugated antibody (Sigma, A7164) was diluted in blocking solution to 1μg/mL and 50μL was added to each well. The plate was incubated for one hour. After another wash step, 50μl of OPD (o-phenylenediamine dihydrochloride) SigmaFast substrate solution was added to each well and the reaction was stopped 25 minutes later by addition of 25μL of 3M sulphuric acid. Absorbance was read at 490nm using the VersaMax Tunable Microplate Reader (Molecular Devices) using a basic endpoint protocol.

Figure 171 shows the results of the EGFR binding ELISA and confirms that bispecific antibodies BPC1818 and BPC1813 bind to recombinant human EGFR. The positive control antibody, Erbitux, also showed binding to recombinant human EGFR. In contrast the Sigma IgG I5154 showed no binding to recombinant human EGFR. For the bispecific antibodies BPC1813 and BPC1818, the amount of antibody in the supernatant was not quantified thus the data presented in Figure 171 is represented as a dilution factor of the neat supernatant material. For Erbitux and the negative control antibody (Sigma I5154), purified material was used in the assay and the starting concentration of 2μg/ml and 1μg/ml respectively, which is equivalent to dilution factor of 1 in Figure 171.

Figure 176 shows the results of the EGFR binding ELISA and confirms that bispecific antibodies BPC1812 binds to recombinant human EGFR. The positive control antibody, Erbitux, also showed binding to recombinant human EGFR. In contrast the Sigma IgG I5154 showed no binding to recombinant human EGFR. For the bispecific antibodies BPC1812, the amount of antibody in the supernatant was not quantified thus the data presented in Figure 176 is represented as a dilution factor of the neat supernatant material. For Erbitux and the negative control antibody (Sigma I5154), purified material was used in the assay at the starting concentration of 2μg/ml and 1μg/ml respectively, which is equivalent to dilution factor of 1 in Figure 176.

<u>**Example 18**</u>

<u>**Binding activity data of IL-13/IL-4 mAbdAbs where the 'G and S' amino acid residues have been removed.**</u>

<u>18.1 Construction of mAbdAbs</u>

mAbdAbs were constructed in which the G and S amino acid residues (next to the linker sequence) were removed. Expression plasmids were constructed using standard molecular biology techniques. These mAbdAbs are described in Table 23. They were cloned, then expressed in one or more of HEK293-6E cells, CHOK1 cells, or CHOE1a cells, they were purified (as described in examples 1, 1.3 and 1.5 respectively) and analysed in a number of IL-13 and IL-4 activity assays.

Table 23

| Name | Description | Sequence ID No. |
|---|---|---|
| PascoH-474 GS removed | H chain = Pascolizumab heavy chain-DOM10-53-474 dAb<br>L chain = Pascolizumab light chain | 91 (=H chain)<br>15 (=L chain) |
| PascoH-TVAAPS-474 GS removed | H chain = Pascolizumab heavy chain-TVAAPS-DOM10-53-474 dAb<br>L chain = Pascolizumab light chain | 92 (=H chain)<br>15 (=L chain) |
| PascoH-GS-ASTKGPT-474 2nd GS removed | H chain = Pascolizumab heavy chain-GS-ASTKGPT-DOM10-53-474 dAb<br>L chain = Pascolizumab light chain | 96 (=H chain)<br>15 (=L chain) |
| 586H-210 GS removed | H chain = Anti-human IL-13 mAb heavy chain-DOM9-112-210 dAb<br>L chain = Anti-human IL-13 mAb light chain | 87 (=H chain)<br>13 (=L chain) |
| 586H-TVAAPS-210 GS removed | H chain = Anti-human IL-13 mAb heavy chain-TVAAPS-DOM9-112-210 dAb<br>L chain = Anti-human IL-13 mAb light chain | 88 (=H chain)<br>13 (=L chain) |

18.2 Expression and purification

These mAbdAbs were purified and analysed by SEC and SDS PAGE. A number of purified preparations were made and the SEC and SDS PAGE data shown in Figures 90 to 98 are representative of these preparations.

18.3 Binding to human IL-4 in a direct binding ELISA

Purified PascoH-474 GS removed and PascoH-TVAAPS-474 GS removed were tested for binding to human IL-4 in a direct binding ELISA as described in method 2 (PascoH-474, PascoH-TVAAPS-474, PascoH-ASTKG-474 and PascoH-ELQLE-474 were also tested for binding in this assay). A number of ELISA assays have been completed for these molecules, the data shown in Figure 99 is representative of these assays.

PascoH-474 GS removed and PascoH-TVAAPS-474 GS removed, both bound human IL-4. Purified anti-human IL4 mAb alone (Pascolizumab) was included in this assay as a positive control for binding to IL-4. Purified anti-human IL13 mAb was included as a negative control for IL-4 binding. The binding activity of PascoH-474 GS removed and PascoH-TVAAPS-474 GS removed was similar to purified anti-IL4 mAb alone (Pascolizumab), PascoH-474, PascoH-TVAAPS-474, PascoH-ASTKG-474 and PascoH-ELQLE-474.

18.4 Binding to human IL-13 in a direct binding ELISA

Purified PascoH-474 GS removed and PascoH-TVAAPS-474 GS removed were also tested for binding to human IL-13 in a direct binding ELISA as described in method 1 (PascoH-616 and PascoH-TVAAPS-616 were also tested for binding in this assay, the generation of these molecules is described in Example 19). A number of ELISA assays have been completed for these molecules, the data shown in Figure 100 is representative of all assays.

PascoH-474 GS removed, PascoH-TVAAPS-474 GS removed, PascoH-616 and PascoH-TVAAPS-616 all bound to human IL-13. Purified anti-human IL4 mAb alone (Pascolizumab) was included in this assay as a negative control for binding to IL-13. Purified anti-human IL13 mAb was included as a positive control for IL-13 binding. Note that the anti-IL-13 dAb alone (DOM10-53-474) was not tested in this assay as the dAb is not detected by the secondary detection antibody; instead, the anti-human IL13 mAb was used as a positive control to demonstrate IL-13 binding in this assay.

18.5 Binding to cynomolgus IL-13 in a direct binding ELISA

Purified PascoH-474 GS removed, PascoH-TVAAPS-474 GS removed, PascoH-616 and PascoH-TVAAPS-616 mAbdAbs were also tested for binding to cynomolgus IL-13 in a direct binding ELISA (as described in method 17). A number of ELISA assays have been completed for these molecules, the data shown in Figure 101 is representative of all assays.

PascoH-474 GS removed, PascoH-TVAAPS-474 GS removed, PascoH-616 and PascoH-TVAAPS-616 all bound cynomolgus IL-13. Purified anti-human IL4 mAb alone (Pascolizumab) was included in this assay as a negative control for binding to IL-13. Purified anti-human IL13 mAb was included as a positive control for cynomolgus IL-13 binding. Note that the anti-IL-13 dAbs alone (DOM10-53-474 and DOM10-53-616) were not tested in this assay as the dAb is not detected by the secondary detection antibody; instead, the anti-human IL13 mAb was used as a positive control to demonstrate IL-13 binding in this assay.

18.6 Biacore analysis for binding to human IL-4 and human IL-13

Purified mAbdAbs were tested for binding to human IL-4 and human IL-13 using the BIAcore™ T100 at 25°C (as described in methods 4 and 5). These data are shown in Table 24.

In experiment 1, a mAbdAb capture level of approximately 600 relative response units was achieved and six IL-13 and IL-4 concentration curves (256nM, 64nM, 16nM, 4nM, 1nM and 0.25nM) were assessed. Only one IL-13 (256nM) and IL-4 (256nM) concentration curve was assessed for the mAbs in experiment 1.

In experiment 2, a mAbdAb a capture level of approximately 400 relative response units was achieved and six IL-4 (64nM, 16nM, 4nM, 1nM, 0.25nM and 0.0625nM) and six IL-13 concentration curves (256nM, 64nM, 16nM, 4nM, 1nM and 0.25nM) were assessed. In experiment 2, only one IL-13 concentration curve (256nM) was assessed for the anti-IL13 mAb and five IL-4 concentration curves (64nM, 16nM, 4nM, 1nM and 0.25nM) were assessed for Pascolizumab.

In experiment 3, a mAbdAb or mAb capture level of approximately 700 relative response units was achieved and six IL-4 concentration curves (256nM, 64nM, 16nM, 4nM, 1nM and 0.25nM) and six IL-13 concentration curves (256nM, 64nM, 16nM, 4nM, 1nM and 0.25nM) were assessed.

Table 24

| **Molecule** (purified material) | **Binding affinity, KD (nM)** | | | | | |
|---|---|---|---|---|---|---|
| | Human IL-4 | | | Human IL-13 | | |
| | Experiment 1 | Experiment 2 | Experiment 3 | Experiment 1 | Experiment 2 | Experiment 3 |
| PascoH-474 GS removed | not done | 0.005 | not done | 0.3307 | 0.351 | not done |
| PascoH-TVAAPS-474 GS removed | not done | 0.011 | not done | 0.2677 | 0.305 | not done |
| 586H-210 GS removed | not done | not done | very tight binder * | not done | not done | 0.438 |
| 586H-TVAAPS-210 GS removed | not done | not done | very tight binder * | not done | not done | 0.501 |
| Anti-human IL-13 mAb | does not bind | does not bind | does not bind | 0.2799 | 0.31 | 0.547 |
| Pascolizumab | 0.0137 | 0.011 | 0.013 | does not bind | does not bind | does not bind |

In experiments 1 and 2, PascoH-474 GS removed and PascoH-TVAAPS-474 GS removed, both bound IL-4 with similar binding affinities and this was approximately equivalent to the binding affinity of the anti-human IL4 mAb alone (Pascolizumab). PascoH-474 GS removed and PascoH-TVAAPS-474 GS removed, also bound IL-13 with similar binding affinities. Note that the anti-IL-13 dAb alone (DOM10-53-474) was not tested in this assay as the dAb cannot be captured onto the Protein A or anti-human IgG coated CM5 chip; instead, the anti-human IL13 mAb was used as a positive control to demonstrate IL-13 binding in this assay.

In experiment 3, 586H-210 GS removed and 586H-TVAAPS-210 GS removed, both bound IL-13 with similar binding affinities and this was approximately equivalent to the binding affinity of the anti-human IL13 mAb. 586H-210 GS removed and 586H-TVAAPS-210 GS removed, also bound IL-4 very tightly, however this method was unable to determine the binding affinity due to positive dissociation effects and the sensitivity level of the BIAcore™ technique (*). Note that the anti-IL-4 dAb alone (DOM9-112-210) was not tested in this assay as the dAb cannot be captured onto the Protein A or anti-human IgG coated CM5 chip; instead, the anti-human IL4 mAb (Pascolizumab) was used as a positive control to demonstrate IL-4 binding in this assay.

18.7 Biacore analysis for binding to cynomolgus IL-4 and cynomolgus IL-13

Purified mAbdAbs were tested for binding to cynomolgus IL-4 and cynomolgus IL-13 using the BIAcore™ T100 at 25°C (as described in methods 24 and 23). These data are shown in Table 25. A mAbdAb capture level of approximately 600 relative response units was achieved and six IL-13 concentration curves (256, 64, 16, 4, 1, 0.25nM) and five IL-4 concentration curves (64, 16, 4, 1, 0.25nM) were assessed.

Table 25

| **Molecule** (purified material) | **Binding affinity, KD** | | | | | |
|---|---|---|---|---|---|---|
| | Cynomolgus IL-13 | | | Cynomolgus IL-4 | | |
| | on rate (ka, $Ms^{-1}$) | off rate (kd, $s^{-1}$) | KD (nM) | on rate (ka, $Ms^{-1}$) | off rate (kd, $s^{-1}$) | KD (pM) |
| PascoH-474 GS removed | 6.62E+5 | 1.10E-2 | 16.6 | 1.87E+6 | 6.38E-5 | 34.2 |
| PascoH-TVAAPS-474 GS removed | 4.83E+5 | 1.29E-2 | 26.7 | 1.83E+6 | 5.30E-5 | 29.0 |
| PascoH-ASTKGPT-474 2nd GS removed | 4.79E+5 | 1.14E-2 | 23.8 | 1.83E+6 | 5.30E-5 | 29.0 |
| PascoH-474 | 5.86E+5 | 1.09E-2 | 18.6 | 1.85E+6 | 8.14E-5 | 43.9 |
| PascoH-TVAAPS-474 | 4.33E+5 | 1.17E-2 | 27.1 | 1.80E+6 | 8.85E-5 | 49.1 |
| PascoH-ASTKG-474 | 3.64E+5 | 1.07E-2 | 29.5 | 1.78E+6 | 7.90E-5 | 44.5 |

PascoH-474 GS removed, PascoH-TVAAPS-474 GS removed, PascoH-ASTKGPT-474 2nd GS removed, PascoH-474, PascoH-TVAAPS-474 and PascoH-ASTKG-474 all bound cynomolgus IL-4 with similar binding affinities. PascoH-474 GS removed, PascoH-TVAAPS-474 GS removed, PascoH-ASTKGPT-474 2nd GS removed, PascoH-474, PascoH-TVAAPS-474 and PascoH-ASTKG-474, also all bound IL-13 with similar binding affinities.

Purified mAbdAbs were also tested for binding to cynomolgus IL-4 and cynomolgus IL-13 using the BIAcore™ T100 at 25°C (as described in methods 24 and 23). These data are shown in

Table 26. A mAbdAb capture level of approximately 600 relative response units was achieved and six IL13 and six IL-4 concentration curves (256, 64, 16, 4, 1 and 0.25nM) were assessed.

Table 26

| **Molecule** (purified material) | **Binding affinity, KD** | | | | | |
|---|---|---|---|---|---|---|
| | Cynomolgus IL-13 | | | Cynomolgus IL-4 | | |
| | on rate (ka, $Ms^{-1}$) | off rate (kd, $s^{-1}$) | KD (pM) | on rate (ka, $Ms^{-1}$) | off rate (kd, $s^{-1}$) | KD (pM) |
| 586H-TVAAPS-210 GS removed | 4.92E+5 | 2.86E-5 | 58 | very tight binder * | very tight binder * | very tight binder * |
| 586H-210 GS removed | 5.07E+5 | 2.24E-5 | 44 | very tight binder * | very tight binder * | very tight binder * |
| Anti-IL13 mAb | 4.74E+5 | 1.05E-4 | 222 | does not bind | does not bind | does not bind |
| Pascolizumab | does not bind | does not bind | does not bind | 2.34E+6 | 1.08E-4 | 46 |

586H-210 GS removed and 586H-TVAAPS-210 GS removed, both bound cynomolgus IL-13 with similar binding

affinities; these mAbdAbs appeared to bind IL-13 more potently than the anti-human IL13 mAb, however in the case of the mAb only one concentration curve was completed which is inherently less accurate than a full concentration range assessment. 586H-210 GS removed and 586H-TVAAPS-210 GS removed, also bound IL-4 very tightly, however this method was unable to determine the binding affinity due to positive dissociation effects and the sensitivity level of the BIAcore™ technique (*). Note that the anti-IL-4 dAb alone (DOM9-112-210) was not tested in this assay as the dAb cannot be captured onto the Protein A or anti-human IgG coated CM5 chip; instead, the anti-human IL4 mAb (Pascolizumab) was used as a positive control to demonstrate IL-4 binding in this assay.

18.8 Biacore analysis of effect of IL-4 binding to mAbdAbs on subsequent IL-13 binding kinetics and vice versa, and the effect of IL-13 binding to mAbdAbs on subsequent IL-4 binding kinetics and vice versa.

The IL-13 and IL-4 BIAcore™ binding assays were also used to investigate the effect of IL-4 binding to PascoH-474 GS removed on subsequent IL-13 binding kinetics and vice versa; and the effect of IL-13 binding to 586H-TVAAPS-210 on subsequent IL-4 binding kinetics and vice versa. Analyses were carried out on the BIAcore™ T100 machine at 25°C, using anti-human IgG capture of the mAbdAb (or positive control mAb). Briefly, anti-human IgG was coupled onto a CM5 chip by primary amine coupling in accordance with the manufactures recommendations. mAbdAb constructs (or positive control mAb) were then captured onto this surface (at approximately 250 to 750RUs) and the first analyte (either human IL-13 or human IL-4) was passed over at 256nM for 4 minutes. The second analyte (human IL-4 or human IL-13 respectively) was then passed over at concentrations of 256nM, 64nM, 16nM, 4nM, 1nM and 0.25nM, and for double referencing a buffer injection was passed over the capture antibody or mAbdAb surface. The data was analysed (fitted to the 1:1 model of binding) using the evaluation software in the machine. The surface was then regenerated using 3M magnesium chloride. The data from these experiments are shown in Tables 27 and 28.

Table 27

| Molecule | IL-13 Binding Kinetics | | | | | |
|---|---|---|---|---|---|---|
| | with human IL-4 bound to molecule | | | without human IL-4 bound to molecule | | |
| | on rate (ka, $Ms^{-1}$) | off rate (kd, $s^{-1}$) | KD (pM) | on rate (ka, $Ms^{-1}$) | off rate (kd, $s^{-1}$) | KD (pM) |
| PascoH-474 GS removed | 5.66E+5 | 2.31E-4 | 407.7 | 6.09E+5 | 2.59E-4 | 425.2 |
| 586H-TVAAPS-210 | 9.68E+5 | 3.15E-4 | 325.2 | 9.09E+5 | 3.47E-4 | 381.3 |
| Anti-IL13 mAb | not tested | | | 1.01E+6 | 3.68E-4 | 366.1 |

The binding affinity of PascoH-474 GS removed for human IL-13 was similar, irrespective of whether human IL-4 was bound to this molecule or not. In addition, the binding affinity of 586H-TVAAPS-210 for human IL-13 was similar, irrespective of whether human IL-4 was bound to this molecule or not and it was also similar to the binding affinity of the anti-IL13 mAb for human IL-13.

The off-rates (kd) for IL-4 binding obtained for PascoH-474 GS removed and 586H-TVAAPS-210, are very slow and out of the sensitivity range of the BIAcore™ T100 hence could not be used as an accurate determination of the binding affinity (data not shown). However, the data do indicate that all of the constructs tested bind very tightly to human IL-4. Thus the binding affinity of PascoH-474 GS removed for human IL-4 was very tight, irrespective of whether human IL-13 was bound to this molecule or not. In addition, the binding affinity of 586H-TVAAPS-210 for human IL-4 was very tight, irrespective of whether human IL-13 was bound to this molecule or not.

18.9 Potency of mAbdAbs

mAbdAbs were tested for inhibition of human IL-4 binding to human IL-4R$\alpha$ by ELISA, as described in Method 19. All of the molecules shown in Table 28 were tested in one experiment, however the data have been plotted on two graphs to distinguish between the curve plots (586H-TVAAPS-210 was run twice, this is labelled as sample 1 and sample 2 in table 25). These data are shown in Figures 102 and 103.

PascoH-474 GS removed, inhibited binding of human IL-4 to human IL4R$\alpha$ similarly to Pascolizumab. 586H-210 GS removed, 586H-TVAAPS-210 GS removed, 586H-TVAAPS-210, 586H-210, 586H-G4S-210 and 586H-ASTKG-210 all inhibited binding of human IL-4 to human IL4R$\alpha$ similarly to DOM9-112-210. Pascolizumab and DOM9-112-210

were included as positive controls for the inhibition of IL-4 binding to IL4Rα. DOM10-53-474 and an isotype-matched mAb (with specificity for an irrelevant antigen) were included as negative controls for the inhibition of IL-4 binding to IL4Rα.

These data were also used to determine $IC_{50}$ values for each molecule. The $IC_{50}$ value is the concentration of mAbdAb or mAb or dAb, which is able to inhibit binding of human IL-4 to human IL4Rα by 50%. The $IC_{50}$ values are shown in Table 28.

Table 28

| Molecule | $IC_{50}$ (nM) |
|---|---|
| PascoH-474 GS removed | 5.14 |
| Pascolizumab | 3.45 |
| DOM9-112-210 | 36.77 |
| 586H -210 | 26.79 |
| 586H-210 GS removed | 36.18 |
| 586H-TVAAPS-210 (sample 1) | 23.77 |
| 586H-TVAAPS-210 (sample 2) | 21.03 |
| 586H-TVAAPS-210 GS removed | 19.21 |
| 586H-ASTKG-210 | 27.32 |
| 586H-G4S-210 | 29.85 |
| DOM10-53-474 | No inhibition at concentration range tested |
| Negative control mAb | No inhibition at concentration range tested |

These data confirm that PascoH-474 GS removed behaves similarly to Pascolizumab and that all 586H-210 mAbdAb 'family members' behave similarly to the DOM9-112-210 dAb.

<u>18.10 Neutralisation of human and cynomolgus IL-13 in TF-1 cell bioassays by mAbdAbs</u>

A number of purified mAbdAbs were tested for neutralisation of human and cynomolgus IL-13 in TF-1 cell bioassays (as described in method 8 and method 20 respectively). Each molecule was tested between one and nine times in these assays, not all graphs are shown, but Figures 104 and 105 are representative graphs showing the neutralisation data for human IL-13 and cynomolgus IL-13 respectively. DOM10-53-474 was included as a positive control for neutralisation of human or cynomolgus IL-13 in the bioassays. A dAb with specificity for an irrelevant antigen (negative control dAb) was also included as a negative control for neutralisation of human or cynomolgus IL-13 in the bioassays.

PascoH-474 GS removed, PascoH-TVAAPS-474 GS removed and PascoH-ASTKG-474 2nd GS removed, as well as PascoH-616, PascoH-TVAAPS-616 and DOM10-53-616 (these are described in Example 19), fully neutralised the bioactivity of both human and cynomolgus IL-13 in TF-1 cell bioassays.

<u>18.11 Neutralisation of human and cynomolgus IL-4 in TF-1 cell bioassays by mAbdAbs</u>

A number of purified mAbdAbs were also tested for neutralisation of human and cynomolgus IL-4 in TF-1 cell bioassays (as described in method 9 and method 21 respectively). Each molecule was tested twice in these assays, not all graphs are shown, but Figures 106 and 107 are representative graphs (from the dataset) showing neutralisation data for human IL-4 and cynomolgus IL-4 respectively. The anti-IL13 mAb was included as a negative control and Pascolizumab was included as a positive control for neutralisation of human or cynomolgus IL-4 in the bioassays. In addition, PascoH-474, PascoH-TVAAPS-474, PascoH-ASTKG-474 and PascoH-G4S-474 were also tested for neutralisation of human and cynomolgus IL-4 in these bioassays.

PascoH-474 GS removed and PascoH-TVAAPS-474 GS removed, fully neutralised the bioactivity of both human and cynomolgus IL-4 in TF-1 cell bioassays. In addition PascoH-474, PascoH-TVAAPS-474, PascoH-ASTKG-474 and PascoH-G4S-474 also fully neutralised the bioactivity of human IL-4 in the TF-1 cell bioassay.

$ND_{50}$ values were derived based on data obtained from a number of different experiments. The $ND_{50}$ value is the concentration of mAbdAb or mAb or dAb, which is able to neutralise the bioactivity of IL-13 or IL-4 by 50%. The

mean $ND_{50}$ value, the standard deviation (SD) and the number of times tested (n) are shown in table 29.

Table 29

| Molecule | Mean $ND_{50}$ value & standard deviation (nM) | | | | | | | |
|---|---|---|---|---|---|---|---|---|
| | human IL-13 | | cyno IL-13 | | human IL-4 | | cyno IL-4 | |
| | mean (SD) | n | mean (SD) | n | mean (SD) | n | mean (SD) | n |
| **PascoH-474 GS removed** | 2.269 (0.763) | 9 | 39.834 (14.675) | 8 | 2.855 (1.464) | 2 | 1.89 (0.325) | 2 |
| **PascoH-TVAAPS-474 GS removed** | 2.114 (0.766) | 9 | 47.882 (13.181) | 9 | 3.015 (2.015) | 2 | 1.36 (0.41) | 2 |
| **PascoH-ASTKGPT-474 2nd GS removed** | 1.37 | 1 | 21.49 | 1 | not done | | not done | |
| **DOM10-53-474** | 1.035 (0.741) | 4 | 10.495 (6.958) | 4 | did not neutralise | | did not neutralise | |
| **Negative control dAb** | did not neutralise | | did not neutralise | | not done | | not done | |
| **Pascolizumab** | did not | | did not | | 1.36 | 2 | 2.615 | 2 |
| | neutralise | neutralise | (0.198) | | (2.242) | | | |

PascoH-474 GS removed, PascoH-TVAAPS-474 GS removed, and PascoH-ASTKGPT-474 2nd GS removed, all fully neutralised the bioactivity of human and cynomolgus IL-13 in TF-1 cell bioassays. In addition, the neutralisation potencies ($ND_{50}$ values) of PascoH-474 GS removed, PascoH-TVAAPS-474 GS removed, and PascoH-ASTKGPT-474 2nd GS removed, for human IL-13 were similar and within-fold of the $ND_{50}$ value for purified anti-IL13 dAb alone (DOM10-53-474).

PascoH-474 GS removed and PascoH-TVAAPS-474 GS removed, both fully neutralised the bioactivity of human and cynomolgus IL-4 in TF-1 cell bioassays. In addition, the neutralisation potencies ($ND_{50}$ values) of PascoH-474 GS removed and PascoH-TVAAPS-474 GS removed, for human IL-13 and cyno IL-13 were similar and withinfold of of the $ND_{50}$ value for Pascolizumab.

<u>18.12 Ability of mAbdAbs to inhibit binding of human IL-13 binding to human IL-13Rα2</u>

The molecules listed in Table 30 were tested for inhibition of human IL-13 binding to human IL-13Rα2 by ELISA, as described in Method 22. All molecules were tested in one experiment. The data are shown in Figure 108.

All mAbdAbs tested inhibited binding of human IL-13 to human IL13Rα2. The level of inhibition was similar to that of DOM10-53-474, DOM10-53-616 and the anti-IL13 mAb. Pascolizumab and a negative control dAb (with specificity for an irrelevant antigen), were included as negative controls for the inhibition of IL-13 binding to IL13Rα2.

These data were also used to determine $IC_{50}$ values for each molecule. The $IC_{50}$ value is the concentration of mAbdAb or mAb or dAb, which is able to inhibit binding of human IL-13 to human IL13Rα2 by 50%. The $IC_{50}$ values are shown in Table 30.

Table 30

| Molecule | $IC_{50}$ (nM) |
|---|---|
| PascoH-474 GS removed | 9.58 |
| PascoH-TVAAPS-474 GS removed | 7.41 |
| DOM10-53-474 | 7.61 |
| PascoH-616 | 6.41 |
| PascoH-TVAAPS-616 | 6.17 |

(continued)

| Molecule | $IC_{50}$ (nM) |
|---|---|
| DOM10-53-616 | 5.76 |
| Anti-IL13 mAb | 6.43 |
| Pascolizumab | No inhibition at concentration range tested |
| Negative control dAb | No inhibition at concentration range tested |

These data confirm that PascoH-474 GS removed, PascoH-TVAAPS-474 GS removed, PascoH-616 and PascoH-TVAAPS-616 behaved similarly to DOM10-53-474, DOM10-53-616 and the anti-IL13 mAb.

**Example 19**

**mAbdAbs containing the anti-IL13 DOM10-53-616 dAb**

19.1 Construction of mAbdAbs containing the anti-IL13 DOM10-53-616 dAb

Two anti-IL4mAb-anti-IL13dAbs as set out in Table 31 were cloned from exisiting vectors by site-directed mutagenesis as described in example 1.

Table 31

| Name | Description | Sequence ID No. |
|---|---|---|
| PascoH-616 | H chain = Pascolizumab heavy chain-DOM10-53-616 dAb<br>L chain = Pascolizumab light chain | 149 (=H chain)<br>15 (=L chain) |
| PascoH-TVAAPS-616 | H chain = Pascolizumab heavy chain-TVAAPS-DOM10-53-616 dAb<br>L chain = Pascolizumab light chain | 150 (=H chain)<br>15 (=L chain) |

19.2 Expression and purification of mAbdAbs containing the anti-IL13 DOM10-53-616 dAb

These mAbdAbs were expressed in HEK293-6E cells and CHOE1a cells as described in example 1.3.

The mAbdAbs were purified and analysed by SEC and SDS PAGE. A number of purified preparations of PascoH-616 and PascoH-TVAAPS-616 mAbdAbs were made, the SEC and SDS PAGE data shown in Figures 109 (SEC profile for PascoH-616), 110 (SEC profile for PascoH-TVAAPS_616), 111(SDS PAGE for PascoH-616) and 112 (SDS PAGE for PascoH-TVAAPS-616), are representative of these preparations.

19.3 Binding of mAbdAbs to human IL-13 in a direct binding ELISA

PascoH-616 and PascoH-TVAAPS-616 purified mAb dAbs were tested for binding to human IL-13 in a direct binding ELISA (as described in method 1). These data are shown in Figure 113.

Purified PascoH-616 and PascoH-TVAAPS-616 both bound human IL-13. Purified anti-human IL4 mAb alone (Pascolizumab) was included in this assay as a negative control for binding to IL-13. Purified anti-human IL13 mAb was included as a positive control for IL-13 binding. Note that the anti-IL-13 dAb alone (DOM10-53-616) was not tested in this assay as the dAb is not detected by the secondary detection antibody; instead, the anti-human IL13 mAb was used as a positive control to demonstrate IL-13 binding in this assay.

19.4 Biacore analysis for binding to human IL-4 and human IL-13

Purified mAbdAbs were tested for binding to human IL-4 and human IL-13 using the BIAcore™ T100 at 25°C (as described in methods 4 and 5). These data are shown in Table 32. In experiment 1, a mAbdAb capture level of approximately 600 relative response units was achieved and six IL-13 and IL-4 concentration curves (256nM, 64nM, 16nM, 4nM, 1nM and 0.25nM) were assessed. Only one IL-13 (256nM) and IL-4 (256nM) concentration curve was assessed for the mAbs in experiment 1.

In experiment 2, a mAbdAb a capture level of approximately 400 relative response units was achieved and six IL-4 (64nM, 16nM, 4nM, 1nM, 0.25nM and 0.0625nM) and six IL-13 concentration curves (256nM, 64nM, 16nM, 4nM,

1nM and 0.25nM) were assessed. In experiment 2, only one IL-13 concentration curve (256nM) was assessed for the anti-IL13 mAb and five IL-4 concentration curves (64nM, 16nM, 4nM, 1nM and 0.25nM) were assessed for Pascolizumab.

Table 32

| **Molecule** (purified material) | **Binding affinity, KD (nM)** | | | |
|---|---|---|---|---|
| | Human IL-4 | | Human IL-13 | |
| | Experiment 1 | Experiment 2 | Experiment 1 | Experiment 2 |
| PascoH-616 | 0.00172 | Very tight binder | 0.1137 | 0.15 |
| PascoH-TVAAPS-616 | 0.003 | 0.005 | 0.0497 | 0.056 |
| Anti-human IL-13 mAb | does not bind | does not bind | 0.2799 | 0.31 |
| Pascolizumab | 0.0137 | 0.011 | does not bind | does not bind |

PascoH-616 and PascoH-TVAAPS-616 both bound IL-4 with similar binding affinities and this was similar to the binding affinity of the anti-human IL4 mAb alone (Pascolizumab). PascoH-616 and PascoH-TVAAPS-616 both bound IL-13. Note that the anti-IL-13 dAb alone (DOM10-53-616) was not tested in this assay as the dAb cannot be captured onto the Protein A or anti-human IgG coated CM5 chip; instead, the anti-human IL13 mAb was used as a positive control to demonstrate IL-13 binding in this assay.

19.5 Biacore analysis for binding to cynomolgus IL-13

Purified mAbdAbs were also tested for binding to cynomolgus IL-13 using the BIAcore™ T100 at 25°C (as described in method and 30). These data are shown in Table 33. A mAbdAb capture level of approximately 400 relative response units was achieved and six IL-13 concentration curves (256, 64, 16, 4, 1 and 0.25nM) were assessed. There was only one IL-13 concentration curve (256nM) for the anti-IL13 mAb.

Table 33

| **Molecule** (purified material) | **Binding affinity for cynomolgus IL-13 (KD)** | | |
|---|---|---|---|
| | on rate (ka, $Ms^{-1}$) | off rate (kd, $s^{-1}$) | KD (nM) |
| PascoH-616 | 3.411E+5 | 1.842E-3 | 5.4 |
| PascoH-TVAAPS-616 | 4.597E+5 | 4.514E-3 | 9.8 |
| Anti-IL13 mAb | 5.498E+5 | 6.549E-5 | 0.119 |
| Pascolizumab | does not bind | does not bind | does not bind |

PascoH-616 and PascoH-TVAAPS-616 both bound cynomolgus IL-13 with similar binding affinities. Note that the anti-IL-13 dAb alone (DOM10-53-616) was not tested in this assay as the dAb cannot be captured onto the Protein A or anti-human IgG coated CM5 chip; instead, the anti-human IL13 mAb was used as a positive control to demonstrate IL-13 binding in this assay.

19.6 Neutralisation of human and cynomolgus IL-13 in TF-1 cell bioassays by mAbdAbs

Purified mAbdAbs were tested for neutralisation of human IL-13 and cynomolgus IL-13 in TF-1 cell bioassays (as described in method 8 and method 20 respectively). These molecules were tested 3 times in each assay, and Figure 114 is a representative graph showing the neutralisation data for human IL-13. Figure 114a is a representative graph showing the neutralisation data for cyno IL-13. DOM10-53-616 was included as a positive control for neutralisation of IL-13 in this bioassay. A dAb with specificity for an irrelevant antigen (negative control dAb) was also included as a negative control for neutralisation of IL-13 in this bioassay.

The mean $ND_{50}$ value, the standard deviation (SD) and the number of times tested (n) are shown in table 34.

Table 34

| Molecule | Mean $ND_{50}$ value & standard deviation (nM) | | | |
|---|---|---|---|---|
| | human IL-13 | | cyno IL-13 | |
| | mean (SD) | n | mean (SD) | n |
| **PascoH-616** | 0.7956 (0.129) | 3 | 9.23 (1.422) | 3 |
| **PascoH-TVAAPS-616** | 0.722 (0.245) | 3 | 14.477 (2.847) | 3 |
| **DOM10-53-616** | 0.416 (0.144) | 3 | 4.81 (3.266) | 3 |
| **Negative control dAb** | did not neutralise | | did not neutralise | |

Both PascoH-616 and PascoH-TVAAPS-616, as well as In addition mAbdAbs PascoH-TVAAPS-474 GS removed and PascoH-474 GS removed fully neutralised the bioactivity of human and cynomolgus IL-13 in TF-1 cell bioassays.

In addition, the neutralisation potencies ($ND_{50}$ values) of PascoH-616 and PascoH-TVAAPS-616 for human IL-13 were similar and within 2-fold of the $ND_{50}$ value for the purified anti-IL13 dAb alone (DOM10-53-616).

PascoH-616 and PascoH-TVAAPS-616 were also tested for inhibition of human IL-13 binding to human IL-13Rα2 by ELISA, as described in Method 22. These data are presented in Example 18.12

## Example 20

### Ability of mAbdAbs to neutralise human IL-13 or IL-4 in a human whole blood phospho STAT6 bioassay

The ability of mAbdAbs to neutralise human IL-13 or IL-4 in a human whole blood phospho STAT6 bioassay was carried out as described in Method 16.

The IL-4 or IL-13 neutralisation potencies (ie. inhibition of IL-4 or IL-13 bioactivity) of 2 mAbdAb constructs (the purified anti-IL13mAb-anti-IL4dAb, 586H-TVAAPS-210; and the purified anti-IL4mAb-anti-IL13dAb, PascoH-474 GS removed) were determined. Purified anti-human IL-4 mAb (Pascolizumab) and purified anti-IL4 dAb (DOM9-112-210) were included as positive controls for neutralisation of rhIL-4 in this assay. Purified anti-human IL-13 mAb and purified anti-IL13 dAb (DOM10-53-474) were included as positive controls for neutralisation of rhIL-13. An isotype matched mAb mixed with a dAb (both with specificities for irrelevant antigens), were included as a negative control for neutralisation of rhIL-4 or rhIL-13. Each molecule was tested at least twice, using blood from different donors. Figures 115 to 124 are graphs showing representative data.

The purified mAbdAbs fully neutralised the bioactivity of rhIL-13 and rhIL-4.

As described in method 16, the ability of the test molecules to neutralise rhIL-13 or rhIL-4 bioactivity was expressed as the concentration of the molecules (e.g. mAbdAbs) required to neutralise 2ng/mL of human IL-4 or human IL-13 by 50% ($IC_{50}$). These data are shown in Table 35. The combined mean $IC_{50}$ from all donors for each molecule is presented, along with the standard deviation.

Table 35

| Molecule | Target in assay | Mean $IC_{50}$ (standard deviation) nM | Number of donors |
|---|---|---|---|
| 586-TVAAPS-210 | IL-4 | 1.23 (0.6) | 3 |
| | IL-13 | 2.68 (1.2) | 3 |
| PascoH-474 GS removed | IL-4 | 7.95 (7.8) | 3 |
| | IL-13 | 2.78(0.7) | 3 |
| Anti-IL13 mAb | IL-13 | 1.47 (0.4) | 3 |
| Pascolizumab | IL-4 | 2.44(1.2) | 3 |
| DOM10-53-474 | IL-13 | 6.83 (2.2) | 2 |
| DOM9-112-210 | IL-4 | 3.51 (0.8) | 2 |
| Negative control mAb | --- | No inhibition shown | 4 |
| Negative control dAb | --- | No inhibition shown | 4 |

Comparison of $IC_{50}$ values indicated that 586-TVAAPS-210 inhibited IL-13 and IL-4 induced pSTAT-6 similarly to the anti-IL13 mAb and DOM9-112-210 (in the IL-13 and IL-4 whole blood assays respectively). Comparison of $IC_{50}$ data also indicated that PascoH-474 GS removed, inhibited IL-13 and IL-4 induced pSTAT-6 similarly to DOM10-53-474 and Pascolizumab (in the IL-13 and IL-4 whole blood assays respectively). The control mAb showed no inhibition up to the maximum concentration tested of 661nM in all donors, and the control dAb showed no neutralisation up to the maximum concentration tested of 2291nM in all donors.

**Example 21**

**Rat PK studies of the Dual Targeting anti-IL4/anti-IL13 mAbdAb**

PascoH-G4S-474, PascoL-G4S-474, 586H-TVAAPS-210 and 586H-TVAAPS-154 were assessed in rat PK studies (as summarised in Table 35.1). In brief, male Sprague-Dawley rats (approximately 200 grams to 220 grams in weight) were given a single intravenous (i/v) administration of mAbdAb at a target dose level of 2mg/kg. At allotted time points (0 hours through to 312 hours) 100□l blood samples were withdrawn and processed for plasma. The rat plasma samples were evaluated for the presence of the test molecule in a human IgG detection assay, and/or an IL-13 ligand binding assay, and/or an IL-4 ligand binding assay. In addition, the PK profile in plasma for Pascolizumab (in rat) was also evaluated: in this case the rat plasma samples were evaluated for the presence of Pascolizumab in a human IgG detection assay and an IL-4 ligand binding assay.

In a first study, pascolizumab was given to 4 rats. In a second study, there were four treatment groups (2mg/kg PascoH-G4S-474, 2mg/kg PascoL-G4S-474, 2mg/kg 586H-TVAAPS-210 and 2mg/kg 586H-TVAAPS-154), with 4 rats in each group.

The PK parameters (shown in Table 35.1) were derived from plasma concentration-time profile data (which are not shown). Note that, some plasma samples were analysed more than once in these assays and the PK parameters in Table 35.1 were derived from only one of these datasets. Also note that the PK parameters have not been normalised for individual animal doses, instead nominal doses of 2mg/kg have been assumed. Note that some technical difficulties were encountered with the IgG PK assay for PascoH-G4S-474 hence the concentrations may be overestimated. In addition, analysis of the IgG plasma concentration-time profiles was performed twice in some cases (at analysis 1 and analysis 2, as annotated in Table 35.1). Analysis of the plasma concentration-time profile data generated from the IL-13 and IL-4 ligand binding PK assays was only performed at analysis 2. The plasma concentration-time profile data generated from the IL-13 and IL-4 ligand binding assays for PascoL-G4S-474 and 586H-TVAAPS-154 have not been used to derive PK parameters for these molecules.

Table 35.1

| **Molecule** | **Assay** | **Analysis** | **T1/2** (hr) | **Cmax** (ng/mL) | **AUC (last)** (ng.h/mL) | **AUC (extrap)** (%) | **Clearance** (mL/hr/kg) | **Mean residence time** (hr) |
|---|---|---|---|---|---|---|---|---|
| PascoH-G4S-474 | IgG | 1 | 129 | 60950 | 3430345 | 18.7 | 0.49 | 83 |
| | IL-4 | 2 | 117 | 21975 | 1231243 | 17.2 | 1.42 | 83 |
| | IL-13 | 2 | 87 | 22050 | 1328651 | 13.2 | 1.36 | 85 |
| 586H-TVAAPS-210 | IgG | 1 | 134 | 42400 | 1932615 | 21.9 | 0.81 | 88 |
| | IgG | 2 | 92 | 42400 | 2215579 | 16.1 | 0.76 | 82 |
| | IL-4 | 2 | 60 | 41350 | 1674740 | 20.9 | 1.08 | 77 |
| | IL-13 | 2 | 101 | 31125 | 1515155 | 16.0 | 1.13 | 83 |
| Pascolizumab | IgG | 1 | 188 | 45150 | 3528174 | 31.9 | 0.39 | 110 |
| | IgG | 2 | 193 | 45150 | 3496503 | 34.9 | 0.38 | 109 |
| | IL-4 | 2 | 136 | 42825 | 2590114 | 30.6 | 0.54 | 110 |

(continued)

| Molecule | Assay | Analysis | T1/2 (hr) | Cmax (ng/mL) | AUC (last) (ng.h/mL) | AUC (extrap) (%) | Clearance (mL/hr/kg) | Mean residence time (hr) |
|---|---|---|---|---|---|---|---|---|
| PascoL-G4S-474 | IgG | 1 | 69 | 42550 | 2539978 | 6.8 | 0.75 | 87 |
| 586H-TVAAPS-154 | IgG | 1 | 166 | 43900 | 3315164 | 41.7 | 0.36 | 93 |

Plasma concentration-time profile data, generated in the IL-13 and IL-4 assays for PascoH-G4S-474 (and subsequent derived PK parameters), tended to be comparable; this was also the case for the plasma concentration-time profile data generated in the IL-13, IL-4 and IgG PK assays for 586H-TVAAPS-210 (and subsequent derived PK parameters). This suggests that these mAbdAbs were 'intact' in the rat plasma throughout the course of the study. The derived PK parameters for 586H-TVAAPS-154 appeared to be more similar to the derived PK parameters for Pascolizumab than any of the other mAbdAbs.

**Example 22**

**Cyno PK studies**

PK studies were carried out in cynomolgus monkeys. The animals were given a single intravenous administration at a target dose level of 1mg/kg. At allotted time points, 500□l blood samples were withdrawn and processed for plasma. The cynomolgus plasma samples were then evaluated for the presence of the test molecule in an IL-13 ligand binding assay, an IL-4 ligand binding assay and an IL-13/IL-4 bridging assay.

Preliminary data from these studies with the mAbdAbs 'PascoH-474 GS removed' and '586H-TVAAPS-210' are consistent with the rat PK data shown above and indicated that these molecules are cleared from the systemic circulation more rapidly than a mAb but less rapidly than a dAb.

**Example** 23

**23.1 Generation of a Dual targeting anti-EGFR/anti-VEGF mAbdAb**

This dual targeting mAbdAb was constructed by fusion of a dAb to the C-terminus of the mAb heavy chain. The anti-EGFR mAb heavy and light chain expression cassettes had been previously constructed. The restriction sites which were used for cloning are the same as those set out in Example 10 (SalI and HindIII).

DNA coding an anti-VEGF dAb (DOM15-26-593) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID NO: 164 and 243) for light and heavy chains respectively) were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 165 and 137).

**23.2 Purification and SEC analysis of the Dual targeting anti-EGFR/anti-VEGF mAbdAb**

This dual targeting mAbdAb was purified from clarified expression supernatant using Protein-A affinity chromatography according to established protocols. Concentrations of purified samples were determined by spectrophotometry from measurements of light absorbance at 280nm. SDS-PAGE analysis (Figure 128) of the purified sample (designated DMS4010) shows non-reduced sample running at ~170kDa whilst reduced sample shows two bands running at ~25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile shows a single species running as a symmetrical peak (figure 129).

**23.3 Potency of the Dual targeting anti-EGFR/anti-VEGF mAbdAb**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 130) of this mAbdAb (designated DMS4010) was calculated to be 4.784nM whilst the control, an anti-EGFR mAb gave an EC50 value of 4.214nM. In the anti-VEGF receptor binding assay (Figure 131) the EC50 of the mAbdAb (designated DMS4010) was 58pM (0.058nM) whilst an anti-VEGF control mAb produced an EC50 of 214.1pM (0.2141nM). In conclusion, assay data shows that the construct of example 23, a dual targeting anti-EGFR/anti-VEGF mAbdAb is potent against both antigens.

**23.4 PK of the dual targeting anti-EGFR/anti-VEGF mAbdAb**

The pharmacokinetic profile of the dual targeting anti-EGFR/anti-VEGF mAbdAb (designated DMS4010) was determined after administration to cynomolgus monkeys. The compound was administered at a dose of 5mg/kg i.v. and the serum levels of drug at multiple time points post-administration was determined by binding to both EGFR and VEGF in separate ELISA assays. Figure 132 shows the results for this assay in which the data was compared with historical data that had been generated for the mAbs cetuximab (anti-EGFR) and bevacizumab (anti-VEGF). Further details are shown in table 36.

Table 36

| | Antigen | Half Life | Cmax | AUC (0-inf) | Clearance | % AUC Extrapolated |
|---|---|---|---|---|---|---|
| | | (hr) | (ug/mL) | (hr*ug/mL) | (mL/hr/kg) | |
| cetuximab | EGFR | 43.6 | 151.4 | 5684.3 | 0.9 | 18.4 |
| bevacizumab | VEGF | 238.7 | 167.4 | 24201.9 | 0.2 | 13.4 |
| DMS4010 | EGFR | 7.5 | 89.7 | 623.2 | 8.1 | 5.2 |
| DMS4010 | VEGF | 6.7 | 125.5 | 733.8 | 7 | 7.2 |

**23.5 Generation of an alternative anti-EGFR/anti-VEGF mAbdAb**

An alternative anti-EGFR/anti-VEGF mAbdAb was constructed in a similar way to that described above in Example 11.1, using the same anti-EGFR mAb linked to a VEGF dAb on the C-terminus of the heavy chain using an STG linker. The anti-VEGF dAb used in this case was DOM15-10-11. This molecule was expressed in mammalian HEK293-6E cells using transient transfection techniques by co-transfection of light and heavy chains (SEQ ID NO: 165 and 186), however significantly reduced levels of expression were achieved in comparison to the expression of the molecule described in Example 23.2. When tested for potency in the same VEGF assay as described in Example 23.3 it was found to have undetectable levels of inhibition of VEGF binding to VEGF receptor in this assay.

**Example** 24

**24.1 Generation of a dual targeting anti-EGFR/anti-VEGF mAbdAb with no linker**

A derivative of the mAbdAb described above in Example 23 was made where the 'STG' linker between the dAb and the CH3 domain of the mAb was removed. SDM was used to delete the residues encoding the STG linker from the plasmid encoding the heavy chain. Sequence verified clones for light and heavy chains (SEQ ID NO: 243 and SEQ ID NO: 174) respectively were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 175 and 137).

**24.2 Purification and SEC analysis of the dual targeting anti-EGFR/anti-VEGF mAbdAb with no linker**

This dual targeting mAbdAb was purified from clarified expression supernatant using Protein-A affinity chromatography according to established protocols. Concentrations of purified samples were determined by spectrophotometry from measurements of light absorbance at 280nm. SDS-PAGE analysis (Figure 133) of the purified sample (designated DMS4011) shows non-reduced sample running at ~170kDa whilst reduced sample shows two bands running

at ~25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile shows a single species running as a symmetrical peak (figure 134).

**24.3 Potency of the dual targeting anti-EGFR/anti-VEGF mAbdAb with no linker**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 135) of this mAbdAb (designated DMS4011) was calculated to be 3.529nM whilst the control, an anti-EGFR mAb gave an EC50 value of 3.647nM. In the anti-VEGF receptor binding assay (Figure 136) the EC50 of the mAbdAb (designated DMS4011) was 342.9pM (0.3429nM) whilst an anti-VEGF control mAb produced an EC50 of 214.1pM (0.2141nM). In conclusion, assay data shows that the construct of example 24, a dual targeting anti-EGFR/anti-VEGF mAbdAb with no linker is potent against both antigens.

**Example 25**

**25.1 Generation of a dual targeting anti-EGFR/anti-VEGF mAbdAb with longer linkers**

Derivatives of the mAbdAb described above in Example 23 were made where the linker between the dAb and the CH3 domain of the mAb was lengthened by the insertion of one or two repeats of a flexible "GGGGS" motif into the plasmid encoding the heavy chain.

The first molecule with a heavy chain sequence as set out in SEQ ID NO: 175 has one repeat of this motif, hence having a linker of 'STGGGGGS'.

The second molecule with a heavy chain sequence as set out in SEQ ID NO: 177 has two repeats of this motif, hence having a linker of 'STGGGGGSGGGGS'.

These were both indepently paired with the same light chain as used in Example 23 (SEQ ID NO: 243)

Sequence verified clones for light and heavy chains were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 176 and 137 which is designated DMS4023; and SEQ ID NO: 178 and 137 which is designated DMS4024).

**25.2 Purification and SEC analysis of the dual targeting anti-EGFR/anti-VEGF mAbdAb with longer linkers**

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified samples DMS4023 and DMS4024 (Figure 137) shows non-reduced samples running at ~170kDa whilst reduced samples show two bands running at ~25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile for both DMS4023 (Figure 138) and DMS4024 (Figure 139) show a single species with a slightly trailing peak.

**25.3 Potency of the dual targeting anti-EGFR/anti-VEGF mAbdAb with longer linkers**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 140) of the mAbdAb DMS4023 was calculated to be 7.066nM and the potency of mAbdAb DMS4024 was calculated to be 6.420nM whilst the control, an anti-EGFR mAb gave an EC50 value of 7.291nM. In the anti-VEGF receptor binding assay (Figure 141) the EC50 of the mAbdAb DMS4023 was 91.79pM (0.091nM) and the EC50 of the mAbdAb DMS4024 was 90pM (0.0906nM) whilst an anti-VEGF control mAb produced an EC50 of 463.2pM (0.4632nM). In conclusion, assay data shows that the constructs of example 25, dual targeting anti-EGFR/anti-VEGF mAbdAbs with longer linkers are potent against both antigens.

**Example 26**

**26.1 Generation of a dual targeting anti-VEGF/anti-EGFR mAbdAb**

This dual targeting mAbdAb was constructed by fusion of a dAb to the C-terminus of the mAb heavy chain. The anti-VEGF mAb heavy and light chain expression cassettes had been previously constructed. The restriction sites which were used for cloning are the same as those set out in Example 10 (SalI and HindIII).

DNA coding an anti-EGFR dAb (DOM16-39-542) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID NO: 179 and 181) for light and heavy chains respectively) were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 180 and 182).

**26.2 Purification and SEC analysis of the dual targeting anti-VEGF/anti-EGFR mAbdAb**

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified sample (designated DMS4009) (Figure 142) shows non-reduced samples running at ~170kDa whilst reduced samples show two bands running at ~25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile for this molecule (Figure 143) shows a single species with a symmetrical peak.

**26.3 Potency of the dual targeting anti-VEGF/anti-EGFR mAbdAb**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 144) of the mAbdAb DMS4009 was calculated to be 132.4nM whilst the control, an anti-EGFR mAb gave an EC50 value of 6.585nM. In the anti-VEGF receptor binding assay (Figure 145) the EC50 of the mAbdAb was 539.7pM (0.5397nM) whilst an anti-VEGF control mAb produced an EC50 of 380.5pM (0.3805nM). In conclusion, assay data shows that the construct of example 26, a dual targeting anti-VEGF/anti-EGFR mAbdAb is potent against both antigens.

**Example 27**

**27.1 Generation of a dual targeting anti-EGFR/anti-IL-13 mAbdAb**

This dual targeting mAbdAb was constructed by fusion of a dAb to the C-terminus of the mAb heavy chain. The anti-EGFR mAb heavy and light chain expression cassettes had been previously constructed. The restriction sites which were used for cloning are the same as those set out in Example 10 (SalI and HindIII).

DNA coding an anti-IL-13 dAb (DOM10-53-474) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID NO: 243 and 183) for light and heavy chains respectively) were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 137 and 184).

**27.2 Purification and SEC analysis of the dual targeting anti-EGFR/anti-IL-13 mAbdAb**

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified sample (designated DMS4029) (Figure 146) shows non-reduced samples running at ~170kDa whilst reduced samples show two bands running at ~25 and ~60kDa corresponding light chain and dAb-fused heavy chain respectively.

For size exclusion chromatography (SEC) analysis the anti-EGFR/anti-IL-13 mAbdAb was applied onto a S-200 10/300 GL column (attached to an HPLC system) pre-equilibrated and running in PBS at 0.5ml/min. The SEC profile for this molecule (Figure 147) shows a single species with a symmetrical peak.

**27.3 Potency of the dual targeting anti-EGFR/anti-IL-13 mAbdAb**

The ability of the molecule to neutralise EGFR and IL-13 were determined as described in methods 13 and 25 respectively. Assay data were analysed using GraphPad Prism. Potency values were determined using a sigmoidal dose response curve and the data fitted using the best fit model. Anti-EGFR potency (Figure 148) of the mAbdAb DMS4029 was calculated to be 9.033nM whilst the control, an anti-EGFR mAb gave an EC50 value of 8.874nM. In the IL-13 cell-based neutralisation assay (Figure 149) the EC50 of the mAbdAb was 1.654nM whilst an anti-IL-13 control dAb produced an EC50 of 0.996nM. In conclusion, assay data shows that the construct of example 27, a dual targeting anti-EGFR/anti-IL-13 mAbdAb is potent against both antigens.

**Example 28**

**28.1 Generation of a dual targeting anti-EGFR/anti-VEGF mAbdAbs where the dAb is located on the light chain**

Dual targeting anti-EGFR/anti-VEGF mAbdAbs were constructed by fusion of a dAb to the C-terminus of the mAb light chain. The anti-EGFR mAb heavy and light chain expression cassettes had been previously constructed.

To introduce restriction sites for dAb insertion in the light chain, site directed mutagenesis was used to create BamHI and HindIII cloning sites using the mAb light chain expression vector as a template. DNA coding an anti-VEGF dAb (DOM15-26-593) was then amplified by PCR (using primers coding BamHI and HindIII ends) and inserted into the modified 3' coding region, resulting in a linker of either 'GSTG' or 'GSTVAAPS' between the mAb and the dAb.

The first molecule with a light chain sequence as set out in SEQ ID NO: 187 has a linker of 'GSTG'.

The second molecule with a light chain sequence as set out in SEQ ID NO: 189 has a linker of 'GSTVAAPS'.

These were both independently paired with the heavy chain of SEQ ID NO: 245.

Sequence verified clones for light and heavy chains were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 188 and 139 which is designated DMS4013; and SEQ ID NO: 190 and 139 which is designated DMS4027).

**28.2 Purification and SEC analysis of the dual targeting anti-EGFR/anti-VEGF mAbdAbs where the dAb is located on the light chain**

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified samples DMS4013 and DMS4027 (Figure 150) shows non-reduced samples running at ~170kDa whilst reduced samples show two bands running at ~38 and ~50kDa corresponding to dAb-fused light chain and heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile for both DMS4013 (Figure 151) and DMS4027 (Figure 152) show a single species with a symmetrical peak.

**28.3 Potency of the dual targeting anti-EGFR/anti-VEGF mAbdAbs where the dAb is located on the light chain**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 153) of the mAbdAb DMS4013 was calculated to be 7.384nM and the potency of mAbdAb DMS4027 was calculated to be 7.554nM whilst the control, an anti-EGFR mAb gave an EC50 value of 7.093nM. In the anti-VEGF receptor binding assay (Figure 154) the EC50 of the mAbdAb DMS4013 was 1.179nM and the EC50 of the mAbdAb DMS4027 was 0.1731nM whilst an anti-VEGF control mAb produced an EC50 of 0.130nM. In conclusion, assay data shows that the constructs of example 28, dual targeting anti-EGFR/anti-VEGF mAbdAbs where the dAb is located on the light chain are potent against both antigens.

**Example 29**

**Biacore analysis of dual targeting anti-EGFR/anti-VEGF and anti-TNF/anti-VEGF mAbdAbs**

The mAbdAbs described in example 11 (anti-TNF/anti-VEGF mAbdAb) and examples 23, 24, 25 and 28 (anti-EGFR/anti-VEGF mAbdAbs) were subjected to BIAcore analysis to determine kinetic association and dissociation constants for binding to their corresponding antigens. Analysis was performed on BIAcore™ 3000 instrument. The temperature of the instrument was set to 25°C. HBS-EP buffer was used as running buffer. Experimental data were collected at the highest possible rate for the instrument. One flow cell on a research grade CM5 chip was coated with protein A using standard amine coupling chemistry according to manufacturers instructions, and a second flow cells was treated equally but buffer was used instead of protein A to generate a reference surface. The flow cell coated with protein A was then used to capture mAbdAbs. Antigen was injected as a series 2x serial dilutions as detailed in table 37. Several dilutions were run in duplicate. Injections of buffer alone instead of ligand were used for background subtraction. Samples were injected in random order using the kinetics Wizard inherent to the instrument software. The surface was regenerated at the end of each cycle by injecting 10mM Glycine, pH 1.5. Both data processing and kinetic fitting were performed using BIAevaluation software 4.1. Data showing averages of duplicate results (from the same run) is shown in Table 37. The multiple values shown for DMS4010 represent two experiments run on separate occasions. The value of 787nM probably overestimates the affinity due to the concentrations of ligand analysed

Table 37

| **mAbdAb Example** | **Molecule number** | Antigen | Ka [1/Ms] | Kd [1/s] | KD [pM] | Top concentration (nM) | # dilutions |
|---|---|---|---|---|---|---|---|
| **11** | **4000** | TNF | 3.65E+05 | 4.16E-05 | 112 | 10 | 6 |
| **23** | **4010** | EGFR | 1.47E+06 | 1.16E-03 | 787 | 1.25 | 5 |
| **23** | **4010** | EGFR | 3.14E+05 | 1.16E-03 | 3700 | 10 | 6 |
| **24** | **4011** | EGFR | 1.81E+05 | 1.11E-03 | 6120 | 5 | 6 |
| **28** | **4013** | EGFR | 2.20E+05 | 1.17E-03 | 5310 | 20 | 5 |
| **28** | **4013** | EGFR | 3.01E+05 | 1.40E-03 | 4650 | 10 | 7 |
| **25** | **4023** | EGFR | 2.38E+05 | 1.10E-03 | 4630 | 5 | 7 |
| **25** | **4024** | EGFR | 2.34E+05 | 1.10E-03 | 4700 | 10 | 6 |
| **28** | **4027** | EGFR | 2.80E+05 | 1.14E-03 | 4060 | 20 | 7 |
| **11** | **4000** | VEGF | 9.19E+05 | 4.78E-04 | 520 | 2.5 | 5 |
| **23** | **4010** | VEGF | 9.85E+05 | 1.90E-04 | 193 | 10 | 8 |
| **24** | **4011** | VEGF | 6.17E+05 | 1.26E-04 | 204 | 10 | 8 |
| **28** | **4013** | VEGF | 7.62E+05 | 3.64E-04 | 478 | 2 | 5 |
| **25** | **4023** | VEGF | 1.60E+06 | 2.40E-04 | 150 | 2 | 6 |
| **25** | **4024** | VEGF | 1.01E+06 | 2.30E-04 | 224 | 2 | 4 |
| **28** | **4027** | VEGF | 7.47E+05 | 2.23E-04 | 229 | 2 | 5 |

**Example 30**

**Trispecific antibodies which comprise single domain antibodies fused onto a bispecific antibody scaffold**

30.1 Construction

Genes encoding variable heavy and light domains of a bispecific antibody molecule which has specificity for IL-18 and IL-12 antigens (for further information see WO 2007/024715) were constructed de-novo with appropriate restriction enzyme sites and signal sequence added. Using standard molecular biology techniques, the variable heavy domains were cloned into an expression vector containing the IgG1 heavy chain constant region fused to an anti-IL4

domain antibody DOM9-112-210 (SEQ ID NO: 4) via a TVAAPS linker at the c-terminus of the constant region. The light chain variable domain was similarly cloned into an expression vector containing the Ck constant region sequence. The antibodies constructed and expressed are listed in Table 38.

Table 38

| Antibody ID | Description | SED ID NO: of amino acid sequence |
|---|---|---|
| BPC1616 | IL-12/18 DVDH TVAAPS-210 heavy chain | 193 |
| | IL-12/18 DVD Kappa light chain | 194 |

30.2 Expression and purification

Briefly, 25 ml of HEK293 cells at 1.5x$10^6$ cells/ml were co-transfected with heavy and light chain expression plasmids previously incubated with 293fectin reagent (Invitrogen # 51-0031). These were placed in a shaking incubator at 37°C, 5% $CO_2$, and 95%RH. After 24 hours Tryptone feeding media was added and the cells grown for a further 48 hours. Supernatant was harvested by centrifugation and IgG levels quantified by ELISA. The resulting mAbdAb was designated BPC1616 (SEQ ID NO: 193 and 194)

30.3 IL-12 binding ELISA

The cell supernatant from the transfections were assessed for binding to recombinant human IL-12. Briefly, ELISA plates coated with anti-human IL-12 (R&D Systems AF219NA) at 2μg/ml and blocked with blocking solution (4% BSA in Tris buffered saline). The plates were then loaded with 25ng/ml recombinant human IL-12 (PeproTech #200-12) in blocking solution. The plate was incubated for 1 hour at room temp before washing in TBS + 0.05% Tween 20 (TBST). Various dilutions of the cell supernatant were added as well as irrelevant control antibodies (Pascolizumab and an isotyped matched control hIgG) diluted in blocking solution. The plate was incubated for 1 hour at room temperature before washing in TBST. Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results are presented in Figure 155 and show that BPC1616 binds to recombinant human IL-12 whereas the two control antibodies showed no binding.

30.4 IL-18 binding ELISA

The cell supernatants from the transfections were assessed for binding to recombinant human IL-18. Briefly, ELISA plates were coated with human IL-18 (made at GSK) at 1μg/ml and blocked with blocking solution (4% BSA in Tris buffered saline). Various dilutions of the cell supernatant were added as well as irrelevant antibodies (Pascolizumab and an isotype matched control human IgG), diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted. The results are presented in Figure 156 and show that BPC1616 binds to recombinant human IL-18 whereas the two control antibodies show no binding.

30.5 IL-4 Binding ELISA

The cell supernatants from the transfections were assessed for binding to recombinant human IL-4. Briefly, ELISA plates were coated with human IL-4 (made at GSK) at 1μg/ml and blocked with blocking solution (4% BSA in Tris buffered saline). Various dilutions of the cell supernatant were added as well as an anti IL-4 monoclonal antibody (Pascolizumab) and irrelevant antibody (Isotype matched control hIgG,), diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured

at 490nm with a plate reader and the mean absorbance plotted.

The results are presented in Figure 157 show that BPC1616 and Pascolizumab bind to recombinant human IL-4 whereas the control antibody shows no binding.

**Example** 31

**Trispecific mAbdAbs comprising two single domains antibodies fused in-line at the C-terminus of a monoclonal antibody**

31.1 Construction

Three trispecific antibodies (mAbdAb-dAb) were constructed where two single domain antibodies are fused in-line at the C-terminus of the heavy chain of a monoclonal antibody. Briefly, a BglII restriction site at the N-terminus and BamHI restriction site at the C-terminus were introduced by PCR to flank the DNA sequences encoding the domain antibodies into the DOM10-53-474 (SEQ ID NO: 5), DOM9-155-154 (SEQ ID NO: 3) and DOM9-112-210 (SEQ ID NO: 4).

The DNA fragment encoding the DOM10-53-474 domain antibody was then cloned into a BamHI site of mammalian expression vector encoding the heavy chain of an anti IL-5 monoclonal antibody fused with an anti IL-4 domain antibody DOM9-112-210 (SEQ ID NO: 71). The DNA fragments encoding the DOM9-155-154 and DOM9-112-210 domain antibodies were both independently cloned into the BamHI site of a mammalian expression vector encoding the heavy chain of an anti-CD20 monoclonal antibody fused with an anti IL-13 domain antibody DOM10-53-474 (SEQ ID NO: 116). The resulting expression vectors encode a heavy chain with two single domain antibodies fused onto the C-terminus. The protein sequences of the heavy chains are given in SEQ ID NO: 195, 196 and 197 as set out in Table 39.

Table 39 is a summary of the mAbdAbs that have been constructed.

Table 39

| Antibody ID | Description | SEQ ID NO: of amino acid sequence |
|---|---|---|
| BPC1008 | Anti IL-5 Heavy Chain-G4S-dAb474-TVAAPSGS-dAb210 | 195 |
| | Anti IL-5 Light Chain | 66 |
| BPC1009 | Anti CD-20 Heavy Chain-TVAAPSGS-dAb154-TVAAPSGS-dAb474 | 196 |
| | Anti CD-20 Light Chain | 117 |
| BPC1010 | Anti CD-20 Heavy Chain-TVAAPSGS-dAb210-TVAAPSGS-dAb474 | 197 |
| | Anti CD-20 Light Chain | 117 |

31.2 Expression and purification

Expression plasmids encoding the heavy and light chains of BPC1008, BPC1009 and BPC1010 were co-transfected into HEK 2936E cells using 293fectin (Invitrogen, 12347019). A tryptone feed was added to the cell culture the following day and the supernatant material was harvested after about 2 to 6 days from initial transfection. The antibodies were purified using a Protein A column before being tested in binding assays.

31.3: IL-4 Binding ELISA

96-well high binding plates were coated with 5μg/ml human IL-4 (GSK) in Coating buffer (0.05M bicarbonate pH9.6, Sigma C-3041) $_3$ and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least one hour at room temperature. The purified antibodies were successively diluted across the plates in blocking solution. After one hour incubation, the plates were washed three times. Goat anti-human kappa light chain specific peroxidase conjugated antibody (Sigma A7164) was diluted 1 in 2000 in blocking solution and 50μL was added to each well. The plates were incubated for one hour. The plates were washed three times, then OPD (o-phenylenediamine dihydrochloride) SigmaFast substrate solution was added to each well and the reaction was stopped 5 minutes later by addition of 25μL of 3M sulphuric acid. Absorbance was read at 490nm using the VersaMax Tunable Microplate Reader (Molecular Devices) using a basic endpoint protocol.

The results of the ELISA are shown in the Figure 158 and confirm that antibodies BPC1008, 1009 and BPC1010 bind to recombinant human IL-4. The positive control Pascolizumab also showed binding to recombinant IL-4 whereas the negative control anti IL-13 mAb and Mepolizumab showed no binding to IL-4. Antibodies BPC1009 and BPC1010 were also tested in a separate experiment which gave similar result to those shown in Figure 158.

31.4: IL-5 Binding ELISA

96-well high binding plates were coated with 5.9μg/ml human IL-5 (GSK) in coating buffer (0.05M bicarbonate pH9.6) and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least one hour at room temperature. The purified antibodies were successively diluted across the plates in blocking solution. After one hour incubation, the plates were washed three times. Goat anti-human kappa light chain specific peroxidase conjugated antibody (Sigma A7164) was diluted 1 in 2000 in blocking solution and 50μL was added to each well. The plates were incubated for one hour. This was washed three times,then OPD (o-phenylenediamine dihydrochloride) SigmaFast substrate solution was added to each well and the reaction was stopped 5 minutes later by addition of 25μL of 3M sulphuric acid. Absorbance was read at 490nm using the VersaMax Tunable Microplate Reader (Molecular Devices) using a basic endpoint protocol.

Figure 159 shows the results of the ELISA which confirms that antibodies BPC1008 bind to recombinant human IL-5 whereas BPC1009 and BPC1010 showed no binding to IL-5. The positive control Mepolizumab also showed binding to recombinant IL-5 whereas the negative control anti IL-13 mAb and Pascolizumab showed no binding to IL-5.

IL-13 Binding ELISA

96-well high binding plates were coated with 5μg/ml human IL-13 (GSK) in Coating buffer (0.05M bicarbonate pH9.6, Sigma C-3041) $_3$ and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least one hour at room temperature. The purified antibodies were successively diluted across the plates in blocking solution. After one hour incubation, the plates were washed three times. Goat anti-human kappa light chain specific peroxidase conjugated antibody (Sigma A7164) was diluted 1 in 2000 in blocking solution and 50μL was added to each well. The plates were incubated for one hour. This was washed three times, then OPD (o-phenylenediamine dihydrochloride) SigmaFast substrate solution was added to each well and the reaction was stopped 5 minutes later by addition of 25μL of 3M sulphuric acid. Absorbance was read at 490nm using the VersaMax Tunable Microplate Reader (Molecular Devices) using a basic endpoint protocol.

The results of the ELISA are shown in the Figure 160 and confirm that antibodies BPC1008, 1009 and BPC1010 bind to recombinant human IL-13. The positive control anti IL-13 mAb also showed binding to recombinant IL-13 whereas the negative control Pascolizumab and Mepolizumab showed no binding to IL-13. Antibodies BPC1009 and BPC1010 were also tested in a separate experiment which gave similar result to those shown in Figure 160.

## Example 32

### mAbdAbs with single domain antibodies fused onto monovalent scaffold

### 32.1 Construction of mAbdAbs

Bispecific antibodies comprising a fusion of a monovalent antibody (for further information see WO2006015371 and WO2007059782) and a domain antibody DOM-15-26-293 were constructed as follows. DNA sequences encoding the anti-c-Met Knob-into-hole heavy chain (SEQ ID NO: 202 and 203) was constructed using a PCR-based strategy followed by site directed mutagenesis. The DNA sequence encoding the anti-c-Met Unibody heavy chain (SEQ ID NO: 204) was constructed using a PCR-based strategy followed by removal of the hinge region by a PCR-based approach. Additionally, for fusion constructs, BamHI and EcoRI restriction sites were included at the C-terminus of the heavy chain expression cassette to facilitate the subsequent cloning of the anti VEGF-A domain antibody (DOM-15-26-593) DNA sequence (encoding amino acids 455-570 of SEQ ID NO: 75) as a BamHI-EcoRI fragment from an existing vector. The resulting expression vectors encode an anti-VEGFA domain antibody fused onto the C-terminus of the heavy chain via a GS linker (SEQ ID NO: 198, 199 and 201). The DNA sequence encoding the anti-c-Met light chain (SEQ ID NO: 200) was constructed by a PCR-based strategy.

The construction of BPC1604 is described in Example 14. Table 40 below is a summary of the monovalent scaffold mAbdAbs and antibodies that have been generated and expressed.

Table 40

| Antibody ID | Description | SED ID NO: of amino acid sequence |
|---|---|---|
| BPC1017 | anti cMET 5D5v2 Heavy Chain (hole)-GS-dAb593 | 198 |
| | anti cMET 5D5v2 Heavy Chain (knob)-GS-dAb593 | 199 |
| | anti cMET 5D5v2 Light Chain | 200 |
| BPC1018 | anti cMET 5D5v2 IgG4 Heavy Chain (UNIBODY)-GS-dAb593 | 201 |
| | anti cMET 5D5v2 Light Chain | 200 |
| BPC1019 | anti cMET 5D5v2 Heavy Chain (hole) | 202 |
| | anti cMET 5D5v2 Heavy Chain (knob) | 203 |
| | anti cMET 5D5v2 Light Chain | 200 |
| BPC1020 | anti cMET 5D5v2 IgG4 Heavy Chain (UNIBODY) | 204 |
| | anti cMET 5D5v2 Light Chain | 200 |

32.2 Expression and purification

Expression plasmids encoding the heavy chain of BPC1017, BPC1018, BPC1019 and BPC1020 were co-transfected into HEK 2936E cells using 293fectin (Invitrogen, 12347019). A tryptone feed was added to the cell culture the following day and the supernatant material was harvested after about 2 to 6 days from initial transfection. The antibodies were purified using a Protein A column before being tested in binding assays.

32.3 HGF Receptor Binding ELISA

96-well high binding plates were coated with 5μg/ml Recombinant Human HGF R (c-MET)/Fc Chimera (R&D system, Catalog Number: 358-MT/CF) in Coating buffer (0.05M bicarbonate pH9.6, Sigma C-3041) $_3$ and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least 30 minutes at room temperature. The plates were washed three times. Then the purified antibodies were successively diluted across the plates in blocking solution. After one hour incubation at room temp, the plates were washed three times. Goat anti-human kappa light chain specific peroxidase conjugated antibody (Sigma A7164) was diluted in blocking solution to 1 in 2000 and was added to each well. The plates were incubated for one hour. This was washed three times and then OPD (o-phenylenediamine dihydrochloride) SigmaFast substrate solution was added to each well and the reaction was stopped 5 minutes later by the addition of 25μL of 3M sulphuric acid. Absorbance was read at 490nm using the VersaMax Tunable Microplate Reader (Molecular Devices) using a basic endpoint protocol. The results of the ELISA are shown in the Figure 161 and confirm that mAbdAbs BPC1017 and BPC1018 bind to recombinant human c-MET with comparable activity to the antibodies BPC1019 and BPC1020. The negative control Pascolizumab and BPC1604 (an IGF-1R/VEGF mAbdAb) showed no binding to c-MET.

32.4 VEGF Binding ELISA

96-well high binding plates were coated with 0.4μg/mL of human VEGF (GSK) in PBS and incubated at 4°C overnight. The plates were washed twice with Tris-Buffered Saline with 0.05% of Tween-20 (TBST). 100μL of blocking solution (4% BSA in TBST buffer) was added to each well and the plates were incubated for at least one hour at room temperature. Another wash step was then performed. The purified antibodies were successively diluted across the plates in blocking solution. After one hour incubation at room temp, the plates were washed. Goat anti-human kappa light chain specific peroxidase conjugated antibody was diluted in blocking solution to 1 in 2000 and was added to each well. The plates were incubated for one hour at room temp. After another wash step, OPD (o-phenylenediamine dihydrochloride) SigmaFast substrate solution was added to each well and the reaction was stopped 5 minutes later by the addition of 25μL of 3M sulphuric acid. Absorbance was read at 490nm using the VersaMax Tunable Microplate Reader (Molecular Devices) using a basic endpoint protocol.

Figure 162 shows the results of the ELISA which confirms that mAbdAbs BPC1017 and BPC1018 bind to recombinant human VEGF. The positive control BPC1604 also showed binding to recombinant human VEGF whereas

Pascolizumab, BPC1019 and BPC1020 showed no binding to VEGF.

**Example** 33

**mAbdAbs containing the anti-IL13 dAbs DOM10-53-546 dAb and DOM10-53-567**

33.1 Construction, expression and purification

The anti-IL4mAb-anti-IL13dAbs shown in Table 41 were cloned and expressed transiently in HEK2936E cells, purified (as described in examples 1, 1.3 and 1.5 respectively).

Table 41

| Name | Description | Sequence ID No. |
|---|---|---|
| PascoH-TVAAPS-546 | H chain = Pascolizumab heavy chain-TVAAPS-DOM10-53-546 dAb<br>L chain = Pascolizumab light chain | 157 (=H chain)<br>15 (=L chain) |
| PascoH-TVAAPS-567 | H chain = Pascolizumab heavy chain-TVAAPS-DOM10-53-567 dAb<br>L chain = Pascolizumab light chain | 159 (=H chain)<br>15 (=L chain) |

Purified PascoH-TVAAPS-546 and PascoH-TVAAPS-567 mAbdAbs were analysed by size exclusion chromatography (SEC) and sodium dodecyl sulphate poly acrylamide gel electrophoresis (SDS PAGE), under reducing conditions. The SEC and SDS PAGE data are shown in Figures 163, 164, 165 and 166.

**33.2 Biacore analysis of binding to IL-13 and IL-4**

Purified PascoH-TVAAPS-546 and PascoH-TVAAPS-567 were tested for binding to human IL-13 and human IL-4 using the BIAcore™ T100 at 25°C (as described in methods 4 and 5). These data are shown in Table 42.

Table 42

| **Molecule** (purified material) | **Binding affinity, KD (nM)** | | | | | |
|---|---|---|---|---|---|---|
| | Human IL-4 | | | Human IL-13 | | |
| | on rate (ka, $Ms^{-1}$) | off rate (kd, $s^{-1}$) | KD (nM) | on rate (ka, $Ms^{-1}$) | off rate (kd, $s^{-1}$) | KD (nM) |
| PascoH-TVAAPS-546 | 4.92E+6 | 2.37E-5 | 0.00482 | 2.26E+5 | 1.69E-4 | 0.747 |
| PascoH-TVAAPS-567 | - | - | Tight binding observed | 4.46E+5 | 1.70E-5 | 0.038 |
| Anti-human IL-13 mAb | - | - | Does not bind | 1.00E+6 | 3.78E-4 | 0.377 |
| Pascolizumab | 4.25E+6 | 2.43E-5 | 0.00572 | - | - | Does not bind |

The mAbdAbs tested in this assay both bound IL-4 with very high affinity (NB, for PascoH-TVAAPS-567 this was beyond the sensitivity of the machine) and with similar binding affinity to that of the anti-human IL4 mAb alone (Pascolizumab). PascoH-TVAAPS-546 and PascoH-TVAAPS-567 both bound IL-13. Note that the anti-IL-13 dAbs alone (DOM10-53-546 and DOM10-53-567) were not tested in this assay as the dAb cannot be captured onto the Protein A or anti-human IgG coated CM5 chip; instead, the anti-human IL13 mAb was used as a positive control to demonstrate IL-13 binding in this assay.

These mAbdAbs were also tested for binding to cynomolgus IL-13 using the BIAcore™ T100 at 25°C (as described in method 23). These data are shown in Table 43. A mAbdAb capture level between 500 and 750 relative response units was achieved, six IL-13 concentration curves (256, 64, 16, 4, 1 and 0.25nM) were assessed for both the mAbdAbs and the anti-IL13 mAb.

Table 43

| Molecule (purified material) | Binding affinity for cynomolgus IL-13 (KD) | | |
|---|---|---|---|
| | on rate (ka, $Ms^{-1}$) | off rate (kd, $s^{-1}$) | KD (nM) |
| PascoH-TVAAPS-546 | 1.67E+6 | 2.09E-2 | 12.5 |
| PascoH-TVAAPS-567 | 5.92E+5 | 5.22E-3 | 8.8 |
| Anti-IL13 mAb | 4.79E+5 | 8.22E-5 | 0.171 |

PascoH-TVAAPS-546 and PascoH-TVAAPS-567 both bound cynomolgus IL-13 with similar binding affinities. Note that the anti-IL-13 dAbs alone (DOM10-53-546 and DOM10-53-567) were not tested in this assay as the dAb cannot be captured onto the Protein A or anti-human IgG coated CM5 chip; instead, the anti-human IL13 mAb was used as a positive control to demonstrate IL-13 binding in this assay.

33.3 Neutralisation of human IL-13 and cynomolgus IL-13 in TF-1 cell bioassays

Purified PascoH-TVAAPS-546 and PascoH-TVAAPS-567 were tested for neutralisation of human IL-13 and cynomolgus IL-13 in TF-1 cell bioassays (as described in method 8 and method 20 respectively). Figures 167 and 168 show the neutralisation data for human IL-13 and cynomolgus IL-13 (in the TF-1 cell bioassays) respectively. DOM10-53-616 was included as a positive control for neutralisation of IL-13 in these bioassays. A dAb with specificity for an irrelevant antigen (negative control dAb) was also included as a negative control for neutralisation of IL-13. In addition, PascoH-616 and PascoH-TVAAPS-616 were also tested in these assays.

Both PascoH-TVAAPS-546 and PascoH-TVAAPS-567 fully neutralised the bioactivity of human and cynomolgus IL-13 in the TF-1 cell bioassays.

$ND_{50}$ values were calculated from the dataset. The $ND_{50}$ value is the concentration of mAbdAb or mAb or dAb, which is able to neutralise the bioactivity of IL-13 by 50%. The mean $ND_{50}$ value, the standard deviation (SD) and the number of times tested (n) are shown in table 44.

Table 44

| Molecule | Mean $ND_{50}$ value & standard deviation (nM) | | | |
|---|---|---|---|---|
| | human IL-13 | | cyno IL-13 | |
| | mean (SD) | n | mean (SD) | n |
| **PascoH-TVAAPS-546** | 1.522 | 1 | 33.88 | 1 |
| **PascoH-TVAAPS-567** | 2.06 | 1 | 38.25 | 1 |
| **DOM10-53-616** | 0.536 | 1 | 3.57 | 1 |
| **Negative control dAb** | did not neutralise | | did not neutralise | |

Example 34

mAbdAbs with IaG2, IaG4 and IgG4PE heavy chain constant regions

34.1 Construction of mAbdAbs

The heavy chain constant regions of human antibody isotypes IgG2, IgG4 and a variant IgG4 (IgG4PE) genes were amplified from existing constructs by PCR and cloned using standard molecular biology techniques into an expression vector encoding the PascoH-GS-474 heavy chain (SEQ ID NO: 48). The mAbdAbs antibodies designed and tested are listed in Table 45.

Table 45

| Antibody ID | Description | SED ID NO: of amino acid sequence |
|---|---|---|
| BPC1617 | PascoH IgG2-GS-474 | 207 |

(continued)

| Antibody ID | Description | SED ID NO: of amino acid sequence |
|---|---|---|
| | Pasco Kappa | 15 |
| BPC1618 | PascoH IgG4-GS-474 | 208 |
| | Pasco Kappa | 15 |
| BPC1619 | PascoH IgG4PE-GS-474 | 209 |
| | Pasco Kappa | 15 |

34.2 Expression

The mAbdAbs set out in table 45 were expressed, along with PascoH-GS-474 (SEQ ID NO: 48 and 15) which is designated BPC1000. Briefly, 750μl of HEK293 cells at $1.5 \times 10^6$ cells/ml were co-transfected with heavy and light chain expression plasmids previously incubated with 293fectin reagent (Invitrogen # 51-0031). These were placed in a shaking incubator at 37°C, 5% $CO_2$, and 95%RH. After 1 hour, Tryptone feeding media was added and the cells grown for a further 72 hours. Supernatant was harvested by centrifugation.

34.3 IL-4 Binding ELISA

The supernatants containing these mAbdAbs were assessed for binding to recombinant human IL-4. Briefly, ELISA plates were coated with human IL-4 (made at GSK) at 1μg/ml and blocked with blocking solution (4% BSA in Tris buffered saline). Various dilutions of the cell supernatant, an anti IL-4 monoclonal antibody (Pascolizumab) and an antibody of irrelevant specificity (586 anti IL-13) were added. All samples were diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results presented in Figure 169 show that the mAbdAbs containing the alternative isotypes all bind to human IL-4. For the mAbdAbs BPC1000, BPC1617, BPC1618 and BPC1619, the amount of antibody in the supernatant was not quantified thus the data presented in Figure 169 is represented as a dilution factor of the neat supernatant material. For the anti-IL4 and IL-13 control antibodies, purified material was used in the assay and the starting concentration of 1μg/ml and 1μg/ml was used respectively (which is equivalent to dilution factor of 1 in Figure 169).

34.4 IL-13 Binding ELISA

The supernatants containing these mAbdAbs were assessed for binding to recombinant human IL-13. Briefly, ELISA plates were coated with human IL-13 (made at GSK) at 5μg/ml and blocked with blocking solution (4% BSA in Tris buffered saline). Various dilutions of the cell supernatant, an anti IL-13 monoclonal antibody (586) and an antibody of irrelevant specificity (Pascolizumab anti IL-4) were added. All samples were diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results presented in Figure 170 show that the bispecific antibodies containing the alternative isotypes all bind to human IL-13. For the bispecific antibodies BPC1000, BPC1617, BPC1618 and BPC1619, the amount of antibody in the supernatant was not quantified thus the data presented in Figure 170 is represented as a dilution factor of the neat supernatant material. For the anti-IL4 and IL-13 control antibodies, purified material was used in the assay at a starting concentration of 1μg/ml and 1μg/ml respectively (which is equivalent to dilution factor of 1 in Figure 170).

## Example 35

Alternative anti-IL-13/IL-4 mAbdAbs

35.1 Construction of anti-IL-13/IL-4 mAbdAbs with alternative variable region sequences

Using standard molecular biology techniques, DNA sequences encoding alternative heavy chain variable region anti-IL-13 mAb designated 'C1' and 'D1' were transferred from existing constructs to an expression vector containing DNA encoding the hIgG1 constant region fused to an anti IL-4 domain antibody (DOM9-112-210) via a TVAAPSGS linker at the c-terminus of the constant region. DNA sequences encoding alternative light chain variable region of IL-13mAbs designated 'M0' and 'N0' were assembled *de novo* and cloned into expression vectors containing the human Ck constant region. These alternative heavy and light chain antibody variable regions comprise the same CDR regions as the anti-IL-13 antibody described in SEQ ID NO: 12 and 13 but with an alternative humanised variable framework region.

35.2 Construction of mAbdAbs using the variable regions of the anti IL-13 mAb '656'

Using standard molecular biology techniques, a DNA sequence encoding the heavy chain variable region of the humanised anti IL-13 mAb '656', were transferred from an existing construct and cloned into an expression vector containing DNA encoding the hIgG1 constant region fused to an anti IL-4 domain antibody (DOM9-112-210) via a TVAAPS linker at the c-terminus of the constant region. A DNA sequence encoding the variable light region was transferred from an existing construct and cloned into an expression vector containing the human Ck constant region.

35.3 Expression of mAbdAbs

Briefly, 25 ml of HEK293 cells at $1.5x10^6$ cells/ml were co-transfected with heavy and light chain expression plasmids previously incubated with 293fectin reagent (Invitrogen # 51-0031). These were placed in a shaking incubator at 37°C, 5% $CO_2$, and 95%RH. After 24 hours Tryptone feeding media was added and the cells grown for a further 72 hours. Supernatant was harvested by centrifugation and IgG levels quantified by ELISA. The antibodies constructed and expressed are listed in Table 46.

Table 46

| Antibody ID /Name | Description | SED ID NO: of amino acid sequence |
|---|---|---|
| BPC1607 | H chain = C1-TVAAPSGS-210 | 151 |
| | L chain = M0 Kappa | 154 |
| BPC1608 | H chain = C1-TVAAPSGS-210 | 151 |
| | L chain = N0 Kappa | 153 |
| BPC1609 | H chain = C1-TVAAPSGS-210 | 151 |
| | L chain = 586 Kappa | 13 |
| | (Anti-human IL-13 mAb light chain) | |
| BPC1610 | H chain = D1-TVAAPSGS-210 | 152 |
| | L chain = M0 Kappa | 154 |
| BPC1611 | H chain = D1-TVAAPSGS-210 | 152 |
| | L chain = N0 Kappa | 153 |
| BPC1612 | H chain = D1-TVAAPSGS-210 | 152 |
| | L chain = 586 Kappa | 13 |
| | (Anti-human IL-13 mAb light chain) | |
| BPC1613 | H chain = 586H-TVAAPS-210 | 88 |
| | (Anti-human IL-13 mAb heavy chain-TVAAPS-DOM9-112-210 dAb) | |

(continued)

| Antibody ID /Name | Description | SED ID NO: of amino acid sequence |
|---|---|---|
| | L chain = M0 Kappa | 154 |
| BPC1614 | H chain = 586H-TVAAPS-210 | 88 |
| | (Anti-human IL-13 mAb heavy chain-TVAAPS-DOM9-112-210 dAb) | |
| | L chain = N0 Kappa | 153 |
| BPC1615 | H chain = 656H-TVAAPS-210 | 155 |
| | (Anti-human IL-13 mAb 2 heavy chain-TVAAPS-DOM9-112-210 dAb) | |
| | L chain = 656 Kappa (Anti-human IL-13 mAb 2 light chain) | 156 |
| BPC1602 (586H-TVAAPS-210 GS removed) | H chain = 586H-TVAAPS-210 | 88 |
| | (Anti-human IL-13 mAb heavy chain-TVAAPS-DOM9-112-210 dAb) | |
| | L chain = 586 Kappa | 13 |
| | (Anti-human IL-13 mAb light chain) | |

35.4 Binding of the mAbdAbs to IL-13

The binding activity of the mAbdAbs to IL-13 was assessed by ELISA. In brief, 5μg/ml recombinant *E.coli*-expressed human IL-13 (made and purified at GSK) was coated to a 96-well ELISA plate. The wells were blocked for 2 hours at room temperature, mAbdAb constructs were then titrated out down the plate. Binding was detected using a 1 in 1000 dilution of anti-human kappa light chain peroxidase conjugated antibody (catalogue number A7164, Sigma-Aldrich).

Figure 177 shows that all of the tested molecules were able to bind to human IL-13. Although BPC1615 showed binding in this ELISA it was not possible to accurately quantify the concentration of this molecule and therefore the IL-13 binding ELISA data for this molecule is not plotted in Figure 177. BPC1615 has also been shown to have high affinity binding to IL-13 in an independent Biacore assay (Table 47).

35.5 Binding of anti-IL13mAb-anti-IL4dAbs to IL-13 by BIAcore™

Cell supernatants from the HEK cell transfections were also tested for binding to recombinant E.Coli-expressed human IL-13 using BIAcore™ at 25°C (as described in method 4). BPC1601 was tested as a purified protein. Binding affinities, presented in Table 47, confirm that all antibodies show high affinity binding to human IL-13.

Table 47

| | ka | kd | KD (nM) |
|---|---|---|---|
| **C1-TVAAPSGS-210 & M0Kappa** BPC1607 | 5.15E+5 | 8.89E-4 | 1.73 |
| **C1-TVAAPSGS-210 & N0Kappa** BPC1608 | 4.90E+5 | 8.83E-4 | 1.80 |
| **C1-TVAAPSGS-210 & 586kappa** BPC1609 | 7.55E+5 | 7.61E-4 | 1.01 |
| **D1-TVAAPSGS-210 & M0kappa** BPC1610 | 3.31E+5 | 4.66E-4 | 1.41 |
| **D1-TVAAPSGS-210 & N0kappa** BPC1611 | 2.59E+5 | 3.31E-4 | 1.28 |
| **D1-TVAAPSGS-210 & 586kappa** BPC1612 | 4.85E+5 | 2.74E-4 | 0.565 |
| **586H TVAAPS-210 & M0kappa** BPC1613 | 5.54E+5 | 4.45E-4 | 0.804 |
| **586H TVAAPS-210 & N0kappa** BPC1614 | 5.49E+5 | 4.42E-4 | 0.805 |
| **656H TVAAPS-210 & 656kappa** BPC1615 | 4.89E+6 | 4.17E-4 | 0.085 |

(continued)

| | ka | kd | KD (nM) |
|---|---|---|---|
| **586H-TVAAPS-210noGS** BPC1602 | 8.21 E+5 | 4.62E-4 | 0.562 |
| **586H-nolinker-210noGS** BPC1601 purified | 8.93E+5 | 3.93E-4 | 0.440 |

**Example 36**

**Generation of mAbdAb with specificity for human IL-5 and human IL-13**

36.1 Construction and expression of mAbdAb

A mAbdAb molecule having the heavy chain set out in SEQ ID NO: 65 and the light chain set out in SEQ ID NO: 72 was expressed in HEK2936E cells. This was designated MepolizumabL-G4S-474 or BPC1021.

36.2 Binding of anti-IL5mAb-anti-IL13dAb to IL-5 and IL-13

This mAbdAb (in cell supernatants) was tested for binding to human IL-13 in a direct binding ELISA (as described in method 1). These data are shown in Figure 173. The sample was transfected and tested in duplicate and this has been annotated as sample A and sample B.
This mAbdAb bound IL-13. Purified anti-human IL13 mAb alone was included in this assay as a positive control for IL-13 binding. Purified anti-human IL-4 mAb (Pascolizumab) and anti-human IL-5 mAb (Mepolizumab) were included as negative controls for IL-13 binding.
This mAbdAb was also tested for binding to human IL-5 in a direct binding ELISA (as described in Example 31.4) These data are illustrated in Figure 174.
MepolizumabL-G4S-474 bound IL-5. Purified anti-human IL4 mAb (Pascolizumab) and purified anti human 13 mAb were included as negative controls for binding to IL-5. Purified anti-human IL5 mAb (Mepolizumab) was used as a positive control to demonstrate IL-5 binding in this assay.

**Sequences**

Table 49:

| Protein or polynucleotide description | DNA Sequence (SEQ ID NO:) | Protein Sequence (SEQ ID NO:) |
|---|---|---|
| DOM9-155-25 | - | 1 |
| DOM9-155-147 | 60 | 2 |
| DOM9-155-154 | 61 | 3 |
| DOM9-112-210 | - | 4 |
| DOM10-53-474 | - | 5 |
| G4S Linker | - | 6 |
| Linker | - | 7 |
| Linker | - | 8 |
| Linker | - | 9 |
| Linker | - | 10 |
| Linker | - | 11 |
| Anti-human IL13 mAb Heavy chain & alternative polynucleotide sequence | 205<br>206 | 12 |
| Anti-human IL13 mAb Light chain | 210 | 13 |
| Pascolizumab Heavy chain | 211 | 14 |

(continued)

| Protein or polynucleotide description | DNA Sequence (SEQ ID NO:) | Protein Sequence (SEQ ID NO:) |
|---|---|---|
| Pascolizumab Light chain | 212 | 15 |
| Mepolizumab Heavy chain | 213 | 65 |
| Mepolizumab Light chain | 214 | 66 |
| Anti-human IL18 mAb Heavy chain | - | 67 |
| Anti-human IL18 mAb Light chain | - | 68 |
| 586H-25 Heavy chain | - | 16 |
| 586H-147 Heavy chain | - | 17 |
| 586H-154 Heavy chain | - | 18 |
| 586H-210 Heavy chain | - | 19 |
| 586H-G4S-25 Heavy chain | - | 20 |
| 586H-G4S-147 Heavy chain | - | 21 |
| 586H-G4S-154 Heavy chain | - | 22 |
| 586H-G4S-210 Heavy chain | - | 23 |
| 586H-TVAAPS-25 Heavy chain | - | 24 |
| 586H-TVAAPS-147 Heavy chain | - | 25 |
| 586H-TVAAPS-154 Heavy chain | 122 | 26 |
| 586H-TVAAPS-210 Heavy chain | 167 | 27 |
| 586H-ASTKG-25 Heavy chain | - | 28 |
| 586H-ASTKG-147 Heavy chain | - | 29 |
| 586H-ASTKG-154 Heavy chain | - | 30 |
| 586H-ASTKG-210 Heavy chain | 192 | 31 |
| 586H-EPKSC-25 Heavy chain | - | 32 |
| 586H-EPKSC-147 Heavy chain | - | 33 |
| 586H-EPKSC-154 Heavy chain | - | 34 |
| 586H-EPKSC-210 Heavy chain | - | 35 |
| 586H-ELQLE-25 Heavy chain | - | 36 |
| 586H-ELQLE-147 Heavy chain | - | 37 |
| 586H-ELQLE-154 Heavy chain | - | 38 |
| 586H-ELQLE-210 Heavy chain | - | 39 |
| 586H Heavy chain-GS | - | 40 |
| 586H-ASTKG Heavy chain | - | 41 |
| 586H-EPKSC Heavy chain | - | 42 |
| 586H-ELQLE Heavy chain | - | 43 |
| 586L-G4S-25 Light chain | - | 44 |
| 586L-G4S-147 Light chain | - | 45 |
| 586L-G4S-154 Light chain | - | 46 |
| 586L-G4S-210 Light chain | - | 47 |
| PascoH-474 Heavy chain | 215 | 48 |

(continued)

| Protein or polynucleotide description | DNA Sequence (SEQ ID NO:) | Protein Sequence (SEQ ID NO:) |
|---|---|---|
| PascoH-G4S-474 Heavy chain | - | 49 |
| PascoH-TVAAPS-474 Heavy chain | 216 | 50 |
| PascoH-ASTKG-474 Heavy chain | - | 51 |
| PascoH-EPKSC-474 Heavy chain | - | 52 |
| PascoH-ELQLE-474 Heavy chain | - | 53 |
| PascoL-474 Light chain | 217 | 54 |
| PascoL-G4S-474 Light chain | - | 55 |
| PascoL-TVAAPS-474 Light chain | 218 | 56 |
| PascoL-ASTKG-474 Light chain | - | 57 |
| PascoL-EPKSC-474 Light chain | - | 58 |
| PascoL-ELQLE-474 Light chain | - | 59 |
| Interleukin-4 | - | 62 |
| Interleukin-13 | - | 63 |
| Mammalian signal sequence | - | 64 |
| IGF1R binding VH CDR3 | - | 80 |
| IGF1R binding VH CDR2 | - | 81 |
| IGF1R binding VH CDR1 | - | 82 |
| IGF1R binding VL CDR1 | - | 83 |
| IGF1R alternative VL CDR2 | - | 84 |
| IGF1R binding VL CDR3 | - | 85 |
| IGF1R binding VL CDR2 | - | 86 |
| IL-18mAb-G4S-DOM9-112-210 heavy chain | - | 69 |
| IL-18mAb-G4S-DOM10-53-474 Light Chain | - | 70 |
| IL-5 mAb-G4S-DOM9-112-210 heavy chain | 219 | 71 |
| IL-5 mAb-G4S-DOM10-53-474 light chain | 220 | 72 |
| Anti-TNF mAb Light Chain | 169 | 73 |
| Anti-TNF mAb-DOM16-39-542 Heavy Chain | 170 | 74 |
| Anti-TNF mAb-DOM15-26-593 Heavy Chain | 168 | 75 |
| DOM 15-26-593-VHdUMMY Heavy Chain | 172 | 76 |
| DOM 4-130-54-VκdUMMY Light Chain | 171 | 77 |
| DOM 15-26-anti-TNFmAb Heavy Chain | - | 78 |
| DOM 16-39-542-anti-TNFmAb Light Chain | - | 79 |
| 586H-210 Heavy chain (GS removed) | 221 | 87 |
| 586H-TVAAPS-210 Heavy chain (GS removed) | 222 | 88 |
| 586H-ASTKGPT-210 Heavy chain (both GS removed) | - | 89 |
| 586H-ASTKGPS-210 Heavy chain (both GS removed, linker ASTKGPS) | - | 90 |

(continued)

| Protein or polynucleotide description | DNA Sequence (SEQ ID NO:) | Protein Sequence (SEQ ID NO:) |
|---|---|---|
| PascoH-474 Heavy Chain (GS removed) | 223 | 91 |
| PascoH-TVAAPS-474 Heavy Chain (GS removed) | 224 | 92 |
| PascoH-ASTKGPT-474 Heavy Chain (both GS removed) | - | 93 |
| PascoH-ASTKGPS-474 Heavy Chain (both GS removed, linker ASTKGPS) | - | 94 |
| PascoH-ASTKGPS-474 Heavy Chain (second GS removed, linker ASTKGPS) | - | 95 |
| PascoH-ASTKGPT-474 Heavy Chain (second GS removed) | 225 | 96 |
| EGFR binding VH CDR1 | - | 97 |
| EGFR binding VH CDR2 | - | 98 |
| EGFR binding VH CDR3 | - | 99 |
| EGFR binding VL CDR1 | - | 100 |
| EGFR binding VL CDR2 | - | 101 |
| EGFR binding VL CDR3 | - | 102 |
| EGFR epitope | - | 103 |
| EGFR binding VH alternative CDR1 | - | 104 |
| EGFR binding VH alternative CDR2 | - | 105 |
| EGFR binding VH alternative CDR3 | - | 106 |
| EGFR binding VH alternative CDR2 | - | 107 |
| Heavy chain of anti-IGF-1R antibody H0L0 with DOM15-26-593 fused at C-terminus with TVAAPSGS linker | 226 | 108 |
| IGF1RmAb-GS-DOM15-26-593 Heavy chain | 227 | 109 |
| anti-IGF-1R antibody Heavy chain | 228 | 110 |
| Light chain of anti-IGF-1R antibody H0L0 with DOM15-26-593 fused at C-terminus with TVAAPSGS linker | 229 | 111 |
| Light chain of anti-IGF-1R antibody H0L0 with DOM15-26-593 fused at C-terminus with GS linker | 230 | 112 |
| anti-IGF-1R antibody Light chain | 231 | 113 |
| Variable heavy domain of antibody 2B9 | - | 114 |
| Variable light domain of antibody 2B9 | - | 115 |
| Anti-CD20 mAb heavy chain with TVAAPSGS linker and DOM10-53-474 domain antibody fused at C-terminus | - | 116 |
| Anti-CD20 mAb Light Chain | - | 117 |

(continued)

| Protein or polynucleotide description | DNA Sequence (SEQ ID NO:) | Protein Sequence (SEQ ID NO:) |
|---|---|---|
| Anti-CD20 mAb heavy chain with GS linker and DOM10-53-474 domain antibody fused at C-terminus | - | 118 |
| Anti-CD20 light chain with TVAAPSGS linker and DOM10-53-474 domain antibody fused at C-terminus | - | 119 |
| Anti-CD20 mAb heavy chain | - | 120 |
| Anti-CD20 mAb light chain with GS linker and DOM10-53-474 domain antibody fused at C-terminus | - | 121 |
| antiIGF1R Heavy Chain-GS-TLPC | 232 | 123 |
| antiIGF1R Heavy Chain-GS-CT01 adnectin | 233 | 124 |
| antiIGF1R Heavy Chain-TVAAPSGS-TLPC | 234 | 125 |
| antiIGF1R Heavy Chain-GS-AFFI | 235 | 126 |
| antiIGF1R Heavy Chain-TVAAPSGS-AFFI | 236 | 127 |
| antiIGF1R Heavy Chain-GS-DRPN | 237 | 128 |
| antiIGF1R Heavy Chain-TVAAPSGS-DRPN | 238 | 129 |
| Anti IL-4 heavy Chain-GS-anti RNAse A camelidVHH | 239 | 130 |
| Anti IL-4 heavy Chain-GS-NARV | 240 | 131 |
| antiIGF1R Heavy Chain-TVAAPSGS-CT01 adnectin | 241 | 133 |
| antiIL13-Heavy Chain-GS-antiTNFa adnectin | - | 134 |
| antiIL13-Heavy Chain-TVAAPSGS-antiTNFa adnectin | - | 135 |
| Erbitux Heavy chain-RS-CT01 adnectin | 242 | 136 |
| Anti-EGFR mAb Light chain (RS12) | 243 | 137 |
| Erbitux Light chain-RS-CT01 adnectin | 244 | 138 |
| Anti-EGFR mAb Heavy chain | 245 | 139 |
| 11F8 Heavy Chain-GS-CT01 adnectin | - | 140 |
| 11F8 Light Chain | - | 141 |
| 11F8 Light Chain-GS-CT01 adnectin | - | 142 |
| 11F8 Heavy Chain | - | 143 |
| CT01 adnectin-GSTG- Erbitux Heavy Chain | 246 | 144 |
| CT01 adnectin-STG-Erbitux Light Chain | 247 | 145 |
| Anti IL-4 Heavy Chain-GS-anti TNF-a adnectin | 270 | 146 |
| Anti IL-4 Heavy Chain-TVAAPSGS- anti TNF-a adnectin | 132 | 147 |
| DOM10-53-616 | - | 148 |
| PascoH-616 Heavy chain | 248 | 149 |
| PascoH-TVAAPS-616 Heavy chain | 249 | 150 |

(continued)

| Protein or polynucleotide description | DNA Sequence (SEQ ID NO:) | Protein Sequence (SEQ ID NO:) |
|---|---|---|
| C1-TVAAPSGS-210 Heavy chain | - | 151 |
| D1-TVAAPSGS-210 Heavy chain | - | 152 |
| N0 Light chain | - | 153 |
| M0 Light chain | - | 154 |
| 656H-TVAAPS-210 Heavy chain | 250 | 155 |
| 656 Light chain | 251 | 156 |
| PascoH-TVAAPS-546_Heavy chain | 252 | 157 |
| PascoH-546_Heavy chain | 253 | 158 |
| PascoH-TVAAPS-567_Heavy chain | 254 | 159 |
| PascoH-567_Heavy chain | 255 | 160 |
| 656 Heavy chain | 256 | 161 |
| DOM15-26-VHdUMMY Heavy Chain | 162 | 163 |
| Cetuximab-DOM15-26-593 Heavy Chain | 164 | 165 |
| Pascolizumab alternative Heavy chain | | 166 |
| Cetuximab-DOM15-26-593 Heavy Chain no linker | 173 | 174 |
| Cetuximab-DOM15-26-593 Heavy Chain STGGGGGS linker | 175 | 176 |
| Cetuximab-DOM15-26-593 Heavy Chain STGGGGGSGGGGS linker | 177 | 178 |
| Avastin-DOM16-39-542 Heavy Chain | 179 | 180 |
| Avastin Light chain | 181 | 182 |
| Cetuximab-DOM10-53-474 Heavy chain | 183 | 184 |
| Anti-TNF mAb-DOM15-10-11 Heavy Chain | - | 185 |
| Anti-EGFR mAb-DOM15-10-11 Heavy Chain | - | 186 |
| Anti-EGFR mAb-DOM15-26-593 Light Chain GSTG | 187 | 188 |
| Anti-EGFR mAb-DOM15-26-593 Light Chain GSTVAAPS | 189 | 190 |
| Mepolizumab alternative Heavy chain | | 191 |
| IL-12/18 DVDH TVAAPS-210 heavy chain | - | 193 |
| IL-12/18 DVD Kappa light chain | - | 194 |
| Anti IL-5 Heavy Chain-G4S-dAb474-TVAAPSGS-dAb210 | 257 | 195 |
| Anti CD-20 Heavy Chain-TVAAPSGS-dAb154-TVAAPSGS-dAb474 | 258 | 196 |
| Anti CD-20 Heavy Chain-TVAAPSGS-dAb210-TVAAPSGS-dAb474 | 259 | 197 |
| anti cMET 5D5v2 Heavy Chain (hole)-GS-dAb593 | 260 | 198 |

(continued)

| Protein or polynucleotide description | DNA Sequence (SEQ ID NO:) | Protein Sequence (SEQ ID NO:) |
|---|---|---|
| anti cMET 5D5v2 Heavy Chain (knob)-GS-dAb593 | 261 | 199 |
| anti cMET 5D5v2 Light Chain | 262 | 200 |
| anti cMET 5D5v2 IgG4 Heavy Chain (UNIBODY)-GS-dAb593 | 263 | 201 |
| anti cMET 5D5v2 Heavy Chain (hole) | 264 | 202 |
| anti cMET 5D5v2 Heavy Chain (knob) | 265 | 203 |
| anti cMET 5D5v2 IgG4 Heavy Chain (UNIBODY) | 266 | 204 |
| PascoH IgG2-GS-474 heavy chain | 267 | 207 |
| PascoH IgG4-GS-474 heavy chain | 268 | 208 |
| PascoH IgG4PE-GS-474 heavy chain | 269 | 209 |

**Brief Description of Figures**

Figures 1 to 7: Examples of antigen-binding constructs
Figure 8: Schematic diagram of mAbdAb constructs.
Figures 9: SEC and SDS Page analysis of PascoH-G4S-474
Figure 10: SEC and SDS Page analysis of PascoL-G4S-474
Figure 11: SEC and SDS Page analysis of PascoH-474
Figure 12: SEC and SDS Page analysis of PascoHL-G4S-474
Figure 13: mAbdAb supernatants binding to human IL-13 in a direct binding ELISA
Figure 14: mAbdAb supernatants binding to human IL-4 in a direct binding ELISA
Figure 15: Purified mAbdAbs binding to human IL-13 in a direct binding ELISA
Figure 16: purified mAbdAbs binding to human IL-4 in a direct binding ELISA
Figure 17: mAbdAb supernatants binding to human IL-4 in a direct binding ELISA
Figure 18: mAbdAb supernatants binding to human IL-13 in a direct binding ELISA
Figure 19: purified mAbdAb binding to human IL-4 in a direct binding ELISA
Figure 20A: purified mAbdAb binding to human IL-13 in a direct binding ELISA
Figure 20B: purified mAbdAb binding to cynomolgus IL-13 in a direct binding ELISA
Figure 21: mAbdAb binding kinetics for IL-4 using BIAcore™
Figure 22: mAbdAb binding kinetics for IL-4 using BIAcore™
Figure 23: mAbdAbs binding kinetics for IL-13 using BIAcore™
Figure 24: Purified anti-IL13mAb-anti-IL4dAbs ability to neutralise human IL-13 in a TF-1 cell bioassay
Figure 25: Purified anti-IL13mAb-anti-IL4dAbs ability to neutralise human IL-4 in a TF-1 cell bioassay
Figure 26: purified anti-IL4mAb-anti-IL13dAbs PascoH-G4S-474, PascoH-474, PascoL-G4S-474 and PascoHL-G4S-474 ability to neutralise human IL-4 in a TF-1 cell bioassay
Figure 27: purified anti-IL4mAb-anti-IL13dAbs, PascoH-G4S-474, PascoH-474, PascoL-G4S-474 and PascoHL-G4S-474 ability to neutralise human IL-13 in a TF-1 cell bioassay
Figure 28: purified anti-IL4mAb-anti-IL13dAbs, PascoH-G4S-474, PascoH-474, PascoL-G4S-474 and PascoHL-G4S-474 ability to simultaneously neutralise human IL-4 and human IL-13 in a dual neutralisation TF-1 cell bioassay
Figure 29: DOM10-53-474 SEC-MALLS
Figure 30: DOM9-112-210 SEC-MALLS
Figure 31: DOM9-155-25 SEC-MALLS
Figure 32: DOM9-155-25 SEC-MALLS Overlay of all three signals
Figure 33: DOM9-155-147 SEC-MALLS
Figure 34: DOM9-155-159 SEC-MALLS
Figure 35: Control for MW assignment by SEC-MALLS: BSA
Figure 36: schematic diagram of a trispecific mAbdAb molecule
Figure 37: Trispecific mAbdAb IL18mAb-210-474 (supernatants) binding to human IL-18 in direct binding ELISA

Figure 38: Trispecific mAbdAb IL18mAb-210-474 (supernatants) binding to human IL-13 in direct binding ELISA
Figure 39: Trispecific mAbdAb IL18mAb-210-474 (supernatants) binding to human IL-4 in direct binding ELISA
Figure 40: Trispecific mAbdAb Mepo-210-474 (supernatant) binding to human IL-13 in direct binding ELISA
Figure 41: Trispecific mAbdAb Mepo-210-474 (supernatant) binding to human IL-4 in direct binding ELISA
Figure 42: Cloning of the anti-TNF/anti-EGFR mAb-dAb
Figure 43. SDS-PAGE analysis of the anti-TNF/anti-EGFR mAb-dAb
Figure 44. SEC profile of the anti-TNF/anti-EGFR mAb-dAb (Example 10)
Figure 45: Anti-EGFR activity of Example 10
Figure 46. Anti-TNF activity of Example 10
Figure 47. SDS-PAGE analysis of the anti-TNF/anti-VEGF mAb-dAb (Example 11)
Figure 48. SEC profile of the anti-TNF/anti-VEGF mAb-dAb (Example 11)
Figure 49. Anti-VEGF activity of Example 11
Figure 50. Anti-TNF activity of example 11
Figure 51. Cloning of the anti-VEGF/anti-IL1R1 dAb-extended-IgG (Example 12)
Figure 52. SDS-PAGE analysis of the anti-TNF/anti-VEGF dAb-extended IgG A (Example 12)
Figure 53: SDS-PAGE analysis of the anti-TNF/anti-VEGF dAb-extended IgG B (Example 12)
Figure 54. SEC profile of the anti-TNF/anti-VEGF dAb-extended IgG A (Example 12)
Figure 55: SEC profile of the anti-TNF/anti-VEGF dAb-extended IgG B (Example 12)
Figure 56. Anti-VEGF activity of Example 12 (DMS2091)
Figure 57 Anti-VEGF activity of Example 12 (DMS2090)
Figure 58. Anti-IL1R1 activity of Example 12 (DMS2090)
Figure 59: Anti-IL1R1 activity of Example 12 (DMS2091)
Figure 60: Cloning of the anti-TNF/anti-VEGF/anti-EGFR mAb-dAb (Example 13)
Figure 61. SDS-PAGE analysis of the anti-TNF/anti-VEGF/anti-EGFR mAb-dAb (Example 13)
Figure 62: Anti-VEGF activity of Example 13
Figure 63: Anti-TNF activity of Example 13
Figure 64: Anti-EGFR activity of Example 13
Figure 65: SEC analysis of purified Bispecific antibodies, BPC1603 (A), BPC1604 (B), BPC1605 (C), BPC1606 (D)
Figure 66. Binding of bispecific antibodies to immobilised IGF-1R
Figure 67. Binding of Bispecific antibodies to immobilised VEGF
Figure 68. Inhibition of ligand mediated receptor phosphorylation by various bispecific antibodies
Figure 69: Inhibition of ligand mediated receptor phosphorylation by various bispecific antibodies
Figure 70 ADCC assay with anti-CD20/IL-13 bispecific antibody
Figure 71: ADCC assay with anti-CD20/IL-13 bispecific antibody
Figure 72: ADCC assay with anti-CD20/IL-13 bispecific antibody using a shorter dose range
Figure 73: ADCC assay with anti-CD20/IL-13 bispecific antibody using a shorter dose range
Figure 74: CDC assay with anti-CD20/IL-13 bispecific antibody
Figure 75: CDC assay with anti-CD20/IL-13 bispecific antibody
Figure 76: BPC1803 and BPC1804 binding in recombinant human IGF-1R ELISA
Figure 77: BPC1803 and BPC1804 binding in recombinant VEGF binding ELISA
Figure 78: BPC1805 and BPC1806 binding in recombinant human IGF-1R ELISA
Figure 79: BPC1805 and BPC1806 binding in recombinant human HER2 ELISA
Figure 80: BPC1807 and BPC1808 binding in recombinant human IGF-1R ELISA
Figure 81: BPC1807 and BPC1808 binding in recombinant human HER2 ELISA
Figure 82: BPC1809 binding in recombinant human IL-4 ELISA
Figure 83: BPC1809 binding in RNAse A ELISA.
Figure 84: BPC1816 binding in recombinant human IL-4 ELISA
Figure 85: BPC1816 binding in HEL ELISA
Figure 86: BPC1801 and BPC 1802 binding in recombinant human IGF-1R ELISA
Figure 87: BPC1801 and BPC1802 binding in recombinant human VEGFR2 ELISA
Figure 88 BPC1823 and BPC 1822 binding in recombinant human IL-4 ELISA
Figure 88b BPC1823 (higher concentration supernatant) binding in recombinant human IL-4 ELISA
Figure 89: BPC1823 and BPC1822 binding in recombinant human TNF-a ELISA
Figure 89b: BPC1823 (higher concentration supernatant) binding in recombinant human TNF-a ELISA
Figure 90: SEC profile for PascoH-474 GS removed
Figure 91:SEC profile for PascoH-TVAAPS-474 GS removed
Figure 92: SEC profile for PascoH-GS-ASTKGPT-474 2nd GS removed
Figure 93: SEC profile for 586H-210 GS removed

Figure 94: SEC profile for 586H-TVAAPS-210 GS removed
Figure 95: SDS PAGE for PascoH-474 GS removed (lane B) and PascoH-TVAAPS-474 GS removed (lane A)
Figure 96: SDS PAGE for PascoH-GS-ASTKGPT-474 2nd GS removed [A = non-reducing conditions, B = reducing conditions]
Figure 97: SDS PAGE for 586H-210 GS removed (lane A)
Figure 98: SDS PAGE for 586H-TVAAPS-210 GS removed (lane A)
Figure 99: Purified PascoH-474 GS removed and PascoH-TVAAPS-474 GS removed binding in human IL-4 ELISA
Figure 100: Purified PascoH-474 GS removed and PascoH-TVAAPS-474 GS removed binding in human IL-13 ELISA
Figure 101: Purified PascoH-474 GS removed, PascoH-TVAAPS-474 GS removed, PascoH-616 and PascoH-TVAAPS-616 binding in cynomolgus IL-13 ELISA
Figure 102: mAbdAbs inhibition of human IL-4 binding to human IL-4Rα by ELISA
Figure 103: mAbdAbs inhibition of human IL-4 binding to human IL-4Rα by ELISA
Figure 104 Neutralisation of human IL-13 in TF-1 cell bioassays by mAbdAbs
Figure 105: Neutralisation of cynomolgus IL-13 in TF-1 cell bioassays by mAbdAbs
Figure 106: Neutralisation of human IL-4 in TF-1 cell bioassays by mAbdAbs
Figure 107: Neutralisation of cynomolgus IL-4 in TF-1 cell bioassays by mAbdAbs
Figure 108: Ability of mAbdAbs to inhibit binding of human IL-13 binding to human IL-13Rα2
Figure 109: SEC profile for PascoH-616
Figure 110: SEC profile for PascoH-TVAAPS_616
Figure 111:SDS PAGE for PascoH-616 [E1 = non-reducing conditions, E2 = reducing conditions]
Figure 112: SDS PAGE for PascoH-TVAAPS-616 [A = non-reducing conditions, B = reducing conditions]
Figure 113: purified PascoH-616 and PascoH-TVAAPS-616 binding in human IL-13 ELISA
Figure 114: Neutralisation of human IL-13 in TF-1 cell bioassays by mAbdAbs
Figure 114a: Neutralisation of cynomolgus IL-13 in TF-1 cell bioassays by mAbdAbs
Figure 115: Inhibition of IL-4 activity by PascoH-474 GS removed
Figure 116: Inhibition of IL-13 activity by PascoH-474 GS removed
Figure 117:Inhibition of IL-4 activity by 586-TVAAPS-210
Figure 118: Inhibition of IL-13 activity by 586-TVAAPS-210
Figure 119: Inhibition of IL-4 activity by Pascolizumab
Figure 120: Inhibition of IL-4 activity by DOM9-112-210
Figure 121: Inhibition of IL-13 activity by anti-IL13 mAb
Figure 122: Inhibition of IL-13 activity by DOM10-53-474
Figure 123: Activity of control mAb and dAb in IL-4 whole blood assay
Figure 124: Activity of control mAb and dAb in IL-13 whole blood assay
Figure 125: The concentration of drug remaining at various time points post-dose assessed by ELISA against both TNF & EGFR.
Figure 126: The concentration of drug remaining at various time points post-dose assessed by ELISA against both TNF & VEGF.
Figure 127: The concentration of drug remaining at various time points post-dose assessed by ELISA against both IL1R1 & VEGF.
Figure 128: SDS-PAGE of the purified DMS4010
Figure 129: SEC profile of the purified DMS4010
Figure 130: Anti-EGFR potency of DMS4010
Figure 131: anti-VEGF receptor binding assay
Figure 132: pharmacokinetic profile of the dual targeting anti-EGFR/anti-VEGF mAbdAb
Figure 133: SDS-PAGE analysis purified DMS4011
Figure 134: SEC profile of the purified DMS4011
Figure 135: Anti-EGFR potency of DMS4011
Figure 136: DMS4011 in anti-VEGF receptor binding assay
Figure 137: SDS-PAGE analysis of the purified samples DMS4023 and DMS4024
Figure 138: The SEC profile for DMS4023
Figure 139: The SEC profile for DMS4024
Figure 140: Anti-EGFR potency of the mAbdAb DMS4023
Figure 141: DMS4023 and DMS4024 in anti-VEGF receptor binding assay
Figure 142: SDS-PAGE analysis of the purified DMS4009
Figure 143: The SEC profile for DMS4009
Figure 144: Anti-EGFR potency of the mAbdAb DMS4009
Figure 145: DMS4009 in anti-VEGF receptor binding assay

Figure 146: SDS-PAGE analysis of the purified DMS4029
Figure 147: The SEC profile for DMS4029
Figure 148: Anti-EGFR potency of the mAbdAb DMS4029
Figure 149: DMS4029 in the IL-13 cell-based neutralisation assay
Figure 150: SDS-PAGE analysis of the purified samples DMS4013 and DMS4027
Figure 151: The SEC profile for DMS4013
Figure 152: The SEC profile for DMS4027
Figure 153: Anti-EGFR potency of the mAbdAb DMS4013
Figure 154: DMS4013 in anti-VEGF receptor binding assay
Figure 155: ;BPC1616 binding in recombinant human IL-12 ELISA
Figure 156: BPC1616 binding in recombinant human IL-18 ELISA
Figure 157: BPC1616 binding in recombinant human IL-4 ELISA
Figure 158: BPC1008, 1009 and BPC1010 binding in recombinant human IL-4 ELISA
Figure 159: BPC1008 binding in recombinant human IL-5 ELISA
Figure 160: BPC1008, 1009 and BPC1010 binding in recombinant human IL-13 ELISA
Figure 161: BPC1017 and BPC1018 binding in recombinant human c-MET ELISA
Figure 162: BPC1017 and BPC1018 binding in recombinant human VEGF ELISA
Figure 163: SEC profile for PascoH-TVAAPS-546
Figure 164: SEC profile for PascoH-TVAAPS-567
Figure 165: SDS PAGE for PascoH-TVAAPS-546 [A = non-reducing conditions, B = reducing conditions]
Figure 166: SDS PAGE for PascoH-TVAAPS-567 [A = non-reducing conditions, B = reducing conditions]
Figure 167: neutralisation data for human IL-13 in the TF-1 cell bioassay
Figure 168: neutralisation data for cynomolgus IL-13 in the TF-1 cell bioassay
Figure 169: mAbdAbs containing alternative isotypes binding in human IL-4 ELISA
Figure 170: mAbdAbs containing alternative isotypes binding in human IL-13 ELISA
Figure 171: BPC1818 and BPC1813 binding in recombinant human EGFR ELISA
Figure 172: BPC1818 and BPC1813 binding in recombinant human VEGFR2 ELISA
Figure 173: anti-IL5mAb-anti-IL13dAb binding in IL-13 ELISA
Figure 174: anti-IL5mAb-anti-IL13dAb binding in IL-5 ELISA
Figure 175: BPC1812 binding in recombinant human VEGFR2 ELISA
Figure 176: BPC1812 binding in recombinant human EGFR ELISA
Figure 177: mAbdAb binding in human IL-13 ELISA

**1. Domain antibodies**

**SEQ ID NO: 1 = DOM9-155- 25**

DIQMTQSPSSLSASVGDRVTITCRASRPISDWLHWYQQKPGKAPKLLIAWASTLDSGVPSRFSGSGSGTDFTLT
ISSLQPEDFATYYCLQEGWGPPTFGQGTKVEIKR

**SEQ ID NO: 2 = DOM9-155-147**

DIQMTQSPSSLSASVGDRVTITCRASRPISDWLHWYQQKPGKAPKLLIAWASSLYEGVPSRFSGSGSGTDFTLTI
SSLQPEDFATYYCLQEGWGPPTFGQGTKVEIKR

**SEQ ID NO: 3 = DOM9-155-154**

DIQMTQSPSSLSASVGDRVTITCRASRPISDWLHWYQQKPGKAPKLLIAWASSLQGGVPSRFSGSGSGTDFTLT
ISSLQPEDFATYYCLQEGWGPPTFGQGTKVEIKR

**SEQ ID NO: 4 = DOM9-112-210**

EVQLLESGGGLVQPGGSLRLSCAASGFTFRNFGMGWVRQAPGKGLEWVSWIISSGTETYYADSVKGRFTISRD
NSKNTLYLQMNSLRAEDTAVYYCAKSLGRFDYWGQGTLVTVSS

**SEQ ID NO: 5 = DOM10-53-474**

GVQLLESGGGLVQPGGSLRLSCAASGFTFAWYDMGWVRQAPGKGLEWVSSIDWHGEVTYYADSVKGRFTISR
DNSKNTLYLQMNSLRAEDTAVYYCATAEDEPGYDYWGQGTLVTVSS

**SEQ ID NO: 60 = DNA sequence of DOM9-155-147 (protein = SEQ ID NO:2)**

GACATCCAGATGACCCAATCACCATCCTCCCTGTCTGCATCTGTAGGAGACCGTGTCACCATCACTtGCCGGG
CAAGTCGCCCCATtAGCGACTGGTTACATtGGTATCAGCAGAAACCAGGGAAAGCCCCCAAGCTCCTGATCGC

CTGGGCGtCCTCGTTGTACGAGGGGGtCCCATCACGtTTCAGTGGCAGTGGGTCGGGGACAGATTTCACTCTC
ACCATCAGCAGTCTGCAACCCGAAGATTTCGCTACGTACTACTGTTTGCAGGAGGGGTGGGGTCCTCCGACG
TTCGGCCAAGGGACCAAGGTGGAAATCAAACGG

**SEQ ID NO: 61 = DNA sequence of DOM9-155-154 (protein = SEQ ID NO:3)**

GACATCCAGATGACCCAATCACCATCCTCCCTGTCTGCATCTGTAGGAGACCGTGTCACCATCACTTGCCGG
GCAAGTCGCCCCATTAGCGACTGGTTACATTGGTATCAGCAGAAACCAGGGAAAGCCCCCAAGCTCCTGATC
GCCTGGGCGTCCAGCTTGCAGGGGGGGGTCCCATCACGTTTCAGTGGCAGTGGGTCGGGGACAGATTTCAC
TCTCACCATCAGCAGTCTGCAACCCGAAGATTTCGCTACGTACTACTGTTTGCAGGAGGGGTGGGGTCCTCC
GACGTTCGGCCAAGGGACCAAGGTGGAAATCAAACGG

**2. Linkers**

**SEQ ID NO: 6 = G4S linker**
GGGGS

**SEQ ID NO: 7 = linker**
TVAAPS

**SEQ ID NO: 8 = linker**
ASTKGPT

**SEQ ID NO: 9 = linker**
ASTKGPS

**SEQ ID NO: 10 = linker**
EPKSCDKTHTCPPCP

**SEQ ID NO: 11 = linker**
ELQLEESCAEAQDGELDG

**3. Monoclonal** antibodies

**SEQ ID NO: 12 = Anti-human IL13 mAb (H chain)**

QVQLVQSGAEVKKPGSSVKVSCKASGFYIKDTYMHWVRQAPGQGLEWMGTIDPANGNTKYVPKFQGRVTITA DESTSTAYMELSSLRSEDTAVYYCARSIYDDYHYDDYYAMDYWGQGTLVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQGNVFSCSVMHEALHNHYT QKSLSLSPGK

**SEQ ID NO: 13 = Anti-human IL13 mAb (L chain)**

DIVMTQSPLSLPVTPGEPASISCRSSQNIVHINGNTYLEWYLQKPGQSPRLLIYKISDRFSGVPDRFSGSGSGTDF TLKISRVEADDVGIYYCFQGSHVPWTFGQGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEC

**SEQ ID NO: 14 = Pascolizumab (H chain)**

QVTLRESGPALVKPTQTLTLTCTFSGFSLSTSGMGVSWIRQPPGKGLEWLAHIYWDDDKRYNPSLKSRLTISKD TSRNQVVLTMTNMDPVDTATYYCARRETVFYWYFDVWGRGTLVTVSSASTKGPSVFPLAPSSKSTSGGTAALG CLVKDYFPEPVTVSWNSGALTSGVHTFPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPPSRDELTKNQVSLTCL VKGFYPSDIAVEWESNGQPENNYKTTPPVLDSDGSFFLYSKLTVDKSRWQQGNVFSCSVMHEALHNHYTQKSL SLSPGK

**SEQ ID NO: 15 = Pascolizumab (L chain)**

DIVLTQSPSSLSASVGDRVTITCKASQSVDYDGDSYMNWYQQKPGKAPKLLIYAASNLESGIPSRFSGSGSGTDF TFTISSLQPEDIATYYCQQSNEDPPTFGQGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEC

**SEQ ID NO: 65 = Mepolizumab (H chain)**

QVTLRESGPALVKPTQTLTLTCTVSGFSLTSYSVHWVRQPPGKGLEWLGVIWASGGTDYNSALMSRLSISKDTS RNQVVLTMTNMDPVDTATYYCARDPPSSLLRLDYWGRGTLVTVSSASTKGPSVFPLAPSSKSTSGGTAALGCLV KDYFPEPVTVSWNSGALTSGVHTFPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHYTQKSLSLSP GK

**SEQ ID NO: 66 = Mepolizumab (L chain)**

DIVMTQSPDSLAVSLGERATINCKSSQSLLNSGNQKNYLAWYQQKPGQPPKLLIYGASTRESGVPDRFSGSGSG TDFTLTISSLQAEDVAVYYCQNVHSFPFTFGGGTKLEIKRTVAAPSVFIFPPSDEQLKSGTASVVCLLNNFYPREA KVQWKVDNALQSGNSQESVTEQDSKDSTYSLSSTLTLSKADYEKHKVYACEVTHQGLSSPVTKSFNRGEC

**SEQ ID NO: 67 = Anti-human IL-18 mAb (H chain)**

QVQLVQSGAEVKKPGASVKVSCKVSGEISTGYYFHWVRQAPGKGLEWMGRIDPEDDSTKYAERFKDRVTMTED
TSTDTAYMELSSLRSEDTAVYYCTTWRIYRDSSGRPFYVMDAWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK

**SEQ ID NO: 68 = Anti-human IL-18 mAb (L chain)**

DIQMTQSPSSVSASVGDRVTITCLASEDIYTYLTWYQQKPGKAPKLLIYGANKLQDGVPSRFSGSGSGTDYTLTI
SSLQPEDFATYYCLQGSKFPLTFGQGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC

**4. Bispecific mAbdAbs**

NB, the underlined portion of the sequence in "bold" text corresponds to the linker. 'GS'amino acid residues) the DNA coding sequence of which was used as the BamHI cloning site) are not in 'bold' text, but are underlined.

**SEQ ID NO: 16 = 586H-25 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK<u>GS</u>DIQMTQSPSSLSASVGDRVTITCRASRPISDWLHWYQQKPGKAPKLLIAWASTLDSGVPSRF
SGSGSGTDFTLTISSLQPEDFATYYCLQEGWGPPTFGQGTKVEIKR

**SEQ ID NO: 17 = 586H-147 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK<u>GS</u>DIQMTQSPSSLSASVGDRVTITCRASRPISDWLHWYQQKPGKAPKLLIAWASSLYEGVPSRFS
GSGSGTDFTLTISSLQPEDFATYYCLQEGWGPPTFGQGTKVEIKR

**SEQ ID NO: 18 = 586H-154 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK<u>GS</u>DIQMTQSPSSLSASVGDRVTITCRASRPISDWLHWYQQKPGKAPKLLIAWASSLQGGVPSRF
SGSGSGTDFTLTISSLQPEDFATYYCLQEGWGPPTFGQGTKVEIKR

**SEQ ID NO: 19 = 586H-210 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGSEVQLLESGGGLVQPGGSLRLSCAASGFTFRNFGMGWVRQAPGKGLEWVSWIISSGTETYYA
DSVKGRFTISRDNSKNTLYLQMNSLRAEDTAVYYCAKSLGRFDYWGQGTLVTVSS

**SEQ ID NO: 20 = 586H-G4S-25 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**GGGGS**DIQMTQSPSSLSASVGDRVTITCRASRPISDWLHWYQQKPGKAPKLLIAWASTLDSGV
PSRFSGSGSGTDFTLTISSLQPEDFATYYCLQEGWGPPTFGQGTKVEIKR

**SEQ ID NO: 21 = 586H-G4S-147 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**GGGGS**DIQMTQSPSSLSASVGDRVTITCRASRPISDWLHWYQQKPGKAPKLLIAWASSLYEGV
PSRFSGSGSGTDFTLTISSLQPEDFATYYCLQEGWGPPTFGQGTKVEIKR

**SEQ ID NO: 22 = 586H-G4S-154 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**GGGGS**DIQMTQSPSSLSASVGDRVTITCRASRPISDWLHWYQQKPGKAPKLLIAWASSLQGGV
PSRFSGSGSGTDFTLTISSLQPEDFATYYCLQEGWGPPTFGQGTKVEIKR

**SEQ ID NO: 23 = 586H-G4S-210 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK

TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**GGGGS**EVQLLESGGGLVQPGGSLRLSCAASGFTFRNFGMGWVRQAPGKGLEWVSWIISSGTET
YYADSVKGRFTISRDNSKNTLYLQMNSLRAEDTAVYYCAKSLGRFDYWGQGTLVTVSS

**SEQ ID NO: 24 = 586H-TVAAPS-25 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**TVAAPS**GSDIQMTQSPSSLSASVGDRVTITCRASRPISDWLHWYQQKPGKAPKLLIAWASTLDS
GVPSRFSGSGSGTDFTLTISSLQPEDFATYYCLQEGWGPPTFGQGTKVEIKR

**SEQ ID NO: 25 = 586H-TVAAPS-147 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**TVAAPS**GSDIQMTQSPSSLSASVGDRVTITCRASRPISDWLHWYQQKPGKAPKLLIAWASSLYE
GVPSRFSGSGSGTDFTLTISSLQPEDFATYYCLQEGWGPPTFGQGTKVEIKR

**SEQ ID NO: 26 = 586H-TVAAPS-154 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**TVAAPS**GSDIQMTQSPSSLSASVGDRVTITCRASRPISDWLHWYQQKPGKAPKLLIAWASSLQ
GGVPSRFSGSGSGTDFTLTISSLQPEDFATYYCLQEGWGPPTFGQGTKVEIKR

**SEQ ID NO: 27 = 586H-TVAAPS-210 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**TVAAPS**GSEVQLLESGGGLVQPGGSLRLSCAASGFTFRNFGMGWVRQAPGKGLEWVSWIISSG
TETYYADSVKGRFTISRDNSKNTLYLQMNSLRAEDTAVYYCAKSLGRFDYWGQGTLVTVSS

**SEQ ID NO: 28 = 586H-ASTKG-25 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ASTKGPT**GSDIQMTQSPSSLSASVGDRVTITCRASRPISDWLHWYQQKPGKAPKLLIAWAS
TLDSGVPSRFSGSGSGTDFTLTISSLQPEDFATYYCLQEGWGPPTFGQGTKVEIKR

**SEQ ID NO: 29 = 586H-ASTKG-147 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ASTKGPT**GSDIQMTQSPSSLSASVGDRVTITCRASRPISDWLHWYQQKPGKAPKLLIAWAS
SLYEGVPSRFSGSGSGTDFTLTISSLQPEDFATYYCLQEGWGPPTFGQGTKVEIKR

**SEQ ID NO: 30 = 586H-ASTKG-154 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ASTKGPT**GSDIQMTQSPSSLSASVGDRVTITCRASRPISDWLHWYQQKPGKAPKLLIAWAS
SLQGGVPSRFSGSGSGTDFTLTISSLQPEDFATYYCLQEGWGPPTFGQGTKVEIKR

**SEQ ID NO: 31 = 586H-ASTKG-210 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ASTKGPT**GSEVQLLESGGGLVQPGGSLRLSCAASGFTFRNFGMGWVRQAPGKGLEWVSWII
SSGTETYYADSVKGRFTISRDNSKNTLYLQMNSLRAEDTAVYYCAKSLGRFDYWGQGTLVTVSS

**SEQ ID NO: 32 = 586H-EPKSC-25 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**EPKSCDKTHTCPPCP**GSDIQMTQSPSSLSASVGDRVTITCRASRPISDWLHWYQQKPGKA
PKLLIAWASTLDSGVPSRFSGSGSGTDFTLTISSLQPEDFATYYCLQEGWGPPTFGQGTKVEIKR

**SEQ ID NO: 33 = 586H-EPKSC-147 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**EPKSCDKTHTCPPCP**GSDIQMTQSPSSLSASVGDRVTITCRASRPISDWLHWYQQKPGKA
PKLLIAWASSLYEGVPSRFSGSGSGTDFTLTISSLQPEDFATYYCLQEGWGPPTFGQGTKVEIKR

**SEQ ID NO: 34 = 586H-EPKSC-154 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK

KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**EPKSCDKTHTCPPCP**GSDIQMTQSPSSLSASVGDRVTITCRASRPISDWLHWYQQKPGKA
PKLLIAWASSLQGGVPSRFSGSGSGTDFTLTISSLQPEDFATYYCLQEGWGPPTFGQGTKVEIKR

**SEQ ID NO: 35 = 586H-EPKSC-210 (H chain)**

QVQLVQSGAEVKKPGSSVKVSCKASGFYIKDTYMHWVRQAPGQGLEWMGTIDPANGNTKYVPKFQGRVTITADESTSTAYMELSSLRSEDTAVYYCARSIYDDYHYDDYYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGS**EPKSCDKTHTCPPCP**GSEVQLLESGGGLVQPGGSLRLSCAASGFTFRNFGMGWVRQAPGKGLEWVSWIISSGTETYYADSVKGRFTISRDNSKNTLYLQMNSLRAEDTAVYYCAKSLGRFDYWGQGTLVTVSS

**SEQ ID NO: 36 = 586H-ELQLE-25 (H chain)**

QVQLVQSGAEVKKPGSSVKVSCKASGFYIKDTYMHWVRQAPGQGLEWMGTIDPANGNTKYVPKFQGRVTITADESTSTAYMELSSLRSEDTAVYYCARSIYDDYHYDDYYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGS**ELQLEESCAEAQDGELDG**GSDIQMTQSPSSLSASVGDRVTITCRASRPISDWLHWYQQKPGKAPKLLIAWASTLDSGVPSRFSGSGSGTDFTLTISSLQPEDFATYYCLQEGWGPPTFGQGTKVEIKR

**SEQ ID NO: 37 = 586H-ELQLE-147 (H chain)**

QVQLVQSGAEVKKPGSSVKVSCKASGFYIKDTYMHWVRQAPGQGLEWMGTIDPANGNTKYVPKFQGRVTITADESTSTAYMELSSLRSEDTAVYYCARSIYDDYHYDDYYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGS**ELQLEESCAEAQDGELDG**GSDIQMTQSPSSLSASVGDRVTITCRASRPISDWLHWYQQKPGKAPKLLIAWASSLYEGVPSRFSGSGSGTDFTLTISSLQPEDFATYYCLQEGWGPPTFGQGTKVEIKR

**SEQ ID NO: 38 = 586H-ELQLE-154 (H chain)**

QVQLVQSGAEVKKPGSSVKVSCKASGFYIKDTYMHWVRQAPGQGLEWMGTIDPANGNTKYVPKFQGRVTITADESTSTAYMELSSLRSEDTAVYYCARSIYDDYHYDDYYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGS**ELQLEESCAEAQDGELDG**GSDIQMTQSPSSLSASVGDRVTITCRASRPISDWLHWYQQKPGKAPKLLIAWASSLQGGVPSRFSGSGSGTDFTLTISSLQPEDFATYYCLQEGWGPPTFGQGTKVEIKR

**SEQ ID NO: 39 = 586H-ELQLE-210 (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ELQLEESCAEAQDGELDG**GSEVQLLESGGGLVQPGGSLRLSCAASGFTFRNFGMGWVRQ
APGKGLEWVSWIISSGTETYYADSVKGRFTISRDNSKNTLYLQMNSLRAEDTAVYYCAKSLGRFDYWGQGTLVT
VSS

**SEQ ID NO: 40 = 586H-GS (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS

**SEQ ID NO: 41 = 586H-ASTKG (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ASTKGPT**GS

**SEQ ID NO: 42 = 586H-EPKSC (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**EPKSCDKTHTCPPCP**GS

**SEQ ID NO: 43 = 586H-ELQLE (H chain)**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGS**ELQLEESCAEAQDGELDG**GS

**SEQ ID NO: 44 = 586L-G4S-25 (L chain)**

DIVMTQSPLSLPVTPGEPASISCRSSQNIVHINGNTYLEWYLQKPGQSPRLLIYKISDRFSGVPDRFSGSGSGTDF
TLKISRVEADDVGIYYCFQGSHVPWTFGQGTKLEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**GGGGS**D
IQMTQSPSSLSASVGDRVTITCRASRPISDWLHWYQQKPGKAPKLLIAWASTLDSGVPSRFSGSGSGTDFTLTIS
SLQPEDFATYYCLQEGWGPPTFGQGTKVEIKR

**SEQ ID NO: 45 = 586L-G4S-147 (L chain)**

DIVMTQSPLSLPVTPGEPASISCRSSQNIVHINGNTYLEWYLQKPGQSPRLLIYKISDRFSGVPDRFSGSGSGTDF
TLKISRVEADDVGIYYCFQGSHVPWTFGQGTKLEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**GGGGS**D
IQMTQSPSSLSASVGDRVTITCRASRPISDWLHWYQQKPGKAPKLLIAWASSLYEGVPSRFSGSGSGTDFTLTIS
SLQPEDFATYYCLQEGWGPPTFGQGTKVEIKR

**SEQ ID NO: 46 = 586L-G4S-154 (L chain)**

DIVMTQSPLSLPVTPGEPASISCRSSQNIVHINGNTYLEWYLQKPGQSPRLLIYKISDRFSGVPDRFSGSGSGTDF
TLKISRVEADDVGIYYCFQGSHVPWTFGQGTKLEIKRTVAAPSVFIFPPSDEQLKSGTASVVCLLNNFYPREAKVQ

WKVDNALQSGNSQESVTEQDSKDSTYSLSSTLTLSKADYEKHKVYACEVTHQGLSSPVTKSFNRGEC**GGGGS**D
IQMTQSPSSLSASVGDRVTITCRASRPISDWLHWYQQKPGKAPKLLIAWASSLQGGVPSRFSGSGSGTDFTLTI
SSLQPEDFATYYCLQEGWGPPTFGQGTKVEIKR

**SEQ ID NO: 47 = 586L-G4S-210 (L chain)**

DIVMTQSPLSLPVTPGEPASISCRSSQNIVHINGNTYLEWYLQKPGQSPRLLIYKISDRFSGVPDRFSGSGSGTDF
TLKISRVEADDVGIYYCFQGSHVPWTFGQGTKLEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**GGGGS**E
VQLLESGGGLVQPGGSLRLSCAASGFTFRNFGMGWVRQAPGKGLEWVSWIISSGTETYYADSVKGRFTISRDN
SKNTLYLQMNSLRAEDTAVYYCAKSLGRFDYWGQGTLVTVSS

**SEQ ID NO: 48 = PascoH-474 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGSGVQLLESGGGLVQPGGSLRLSCAASGFTFAWYDMGWVRQAPGKGLEWVSSIDWHGEVTYYADSV
KGRFTISRDNSKNTLYLQMNSLRAEDTAVYYCATAEDEPGYDYWGQGTLVTVSS

**SEQ ID NO: 49 = PascoH-G4S-474 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGK**GGGGS**GVQLLESGGGLVQPGGSLRLSCAASGFTFAWYDMGWVRQAPGKGLEWVSSIDWHGEVTYY
ADSVKGRFTISRDNSKNTLYLQMNSLRAEDTAVYYCATAEDEPGYDYWGQGTLVTVSS

**SEQ ID NO: 50 = PascoH-TVAAPS-474 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGK**TVAAPS**GSGVQLLESGGGLVQPGGSLRLSCAASGFTFAWYDMGWVRQAPGKGLEWVSSIDWHGEV
TYYADSVKGRFTISRDNSKNTLYLQMNSLRAEDTAVYYCATAEDEPGYDYWGQGTLVTVSS

**SEQ ID NO: 51 = PascoH-ASTKG-474 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGS**ASTKGPT**GSGVQLLESGGGLVQPGGSLRLSCAASGFTFAWYDMGWVRQAPGKGLEWVSSIDWH
GEVTYYADSVKGRFTISRDNSKNTLYLQMNSLRAEDTAVYYCATAEDEPGYDYWGQGTLVTVSS

**SEQ ID NO: 52 = PascoH-EPKSC-474 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK

SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGS**EPKSCDKTHTCPPCP**GSGVQLLESGGGLVQPGGSLRLSCAASGFTFAWYDMGWVRQAPGKGLE
WVSSIDWHGEVTYYADSVKGRFTISRDNSKNTLYLQMNSLRAEDTAVYYCATAEDEPGYDYWGQGTLVTVSS

**SEQ ID NO: 53 = PascoH-ELQLE-474 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGS**ELQLEESCAEAQDGELDG**GSGVQLLESGGGLVQPGGSLRLSCAASGFTFAWYDMGWVRQAPGK
GLEWVSSIDWHGEVTYYADSVKGRFTISRDNSKNTLYLQMNSLRAEDTAVYYCATAEDEPGYDYWGQGTLVTV
SS

**SEQ ID NO: 54 = PascoL-474 (L chain)**

DIVLTQSPSSLSASVGDRVTITCKASQSVDYDGDSYMNWYQQKPGKAPKLLIYAASNLESGIPSRFSGSGSGTDF
TFTISSLQPEDIATYYCQQSNEDPPTFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGECGSGVQLL
ESGGGLVQPGGSLRLSCAASGFTFAWYDMGWVRQAPGKGLEWVSSIDWHGEVTYYADSVKGRFTISRDNSKN
TLYLQMNSLRAEDTAVYYCATAEDEPGYDYWGQGTLVTVSS

**SEQ ID NO: 55 = PascoL-G4S-474 (L chain)**

DIVLTQSPSSLSASVGDRVTITCKASQSVDYDGDSYMNWYQQKPGKAPKLLIYAASNLESGIPSRFSGSGSGTDF
TFTISSLQPEDIATYYCQQSNEDPPTFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**GGGGS**G
VQLLESGGGLVQPGGSLRLSCAASGFTFAWYDMGWVRQAPGKGLEWVSSIDWHGEVTYYADSVKGRFTISRD
NSKNTLYLQMNSLRAEDTAVYYCATAEDEPGYDYWGQGTLVTVSS

**SEQ ID NO: 56 = PascoL-TVAAPS-474 (L chain)**

DIVLTQSPSSLSASVGDRVTITCKASQSVDYDGDSYMNWYQQKPGKAPKLLIYAASNLESGIPSRFSGSGSGTDF
TFTISSLQPEDIATYYCQQSNEDPPTFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**TVAAPS**
GSGVQLLESGGGLVQPGGSLRLSCAASGFTFAWYDMGWVRQAPGKGLEWVSSIDWHGEVTYYADSVKGRFTI
SRDNSKNTLYLQMNSLRAEDTAVYYCATAEDEPGYDYWGQGTLVTVSS

**SEQ ID NO: 57 = PascoL-ASTKG-474 (L chain)**

DIVLTQSPSSLSASVGDRVTITCKASQSVDYDGDSYMNWYQQKPGKAPKLLIYAASNLESGIPSRFSGSGSGTDF
TFTISSLQPEDIATYYCQQSNEDPPTFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**ASTKGP**
**T**GSGVQLLESGGGLVQPGGSLRLSCAASGFTFAWYDMGWVRQAPGKGLEWVSSIDWHGEVTYYADSVKGRFT
ISRDNSKNTLYLQMNSLRAEDTAVYYCATAEDEPGYDYWGQGTLVTVSS

**SEQ ID NO: 58 = PascoL-EPKSC-474 (L chain)**

DIVLTQSPSSLSASVGDRVTITCKASQSVDYDGDSYMNWYQQKPGKAPKLLIYAASNLESGIPSRFSGSGSGTDF
TFTISSLQPEDIATYYCQQSNEDPPTFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**EPKSCD**
**KTHTCPPCP**GSGVQLLESGGGLVQPGGSLRLSCAASGFTFAWYDMGWVRQAPGKGLEWVSSIDWHGEVTYY
ADSVKGRFTISRDNSKNTLYLQMNSLRAEDTAVYYCATAEDEPGYDYWGQGTLVTVSS

**SEQ ID NO: 59 = PascoL-ELQLE-474 (L chain)**

DIVLTQSPSSLSASVGDRVTITCKASQSVDYDGDSYMNWYQQKPGKAPKLLIYAASNLESGIPSRFSGSGSGTDF
TFTISSLQPEDIATYYCQQSNEDPPTFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEVTHQGLSSPVTKSFNRGEC**ELQLEE**

**SCAEAQDGELDG**GSGVQLLESGGGLVQPGGSLRLSCAASGFTFAWYDMGWVRQAPGKGLEWVSSIDWHGE
VTYYADSVKGRFTISRDNSKNTLYLQMNSLRAEDTAVYYCATAEDEPGYDYWGQGTLVTVSS

**5. Cytokines**

**SEQ ID NO: 62 = IL-4 (Interleukin-4)**

HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSS

**SEQ ID NO: 63 = IL-13 (Interleukin-13)**

GPVPPSTALRELIEELVNITQNQKAPLCNGSMVWSINLTAGMYCAALESLINVSGCSAIEKTQRMLSGFCPHKVS
AGQFSSLHVRDTKIEVAQFVKDLLLHLKKLFREGRFN

**6. Signal sequence**

**SEQ ID NO: 64 = Mammalian amino acid signal sequence**
MGWSCIILFLVATATGVHS

**7. IGF-1R binding CDRs**

**SEQ ID 80 = VH CDR3**

WILYYGRSKWYFDV

**SEQ ID 81 = VH CDR2**

NINPNNGGTNYNQKFKD

**SEQ ID 82 = VH CDR1**

DYYMN

**SEQ ID 83 = VL CDR1**

RSSQSIVQSNGDTYLE

**SEQ ID 84 = alternative VL CDR2**

RISNRFS

**SEQ ID 85 = VL CDR3**

FQGSHVPYT

**SEQ ID 86 = VL CDR2**

RVSNRFS

**8. Trispecific mAbdAbs**

**SEQ ID NO: 69 = IL18mAb-G4S-DOM9-112-210 (H chain)**

QVQLVQSGAEVKKPGASVKVSCKVSGEISTGYYFHWVRQAPGKGLEWMGRIDPEDDSTKYAERFKDRVTMTED
TSTDTAYMELSSLRSEDTAVYYCTTWRIYRDSSGRPFYVMDAWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**GGGGS**EVQLLESGGGLVQPGGSLRLSCAASGFTFRNFGMGWVRQAPGKGLEWVSWIISSGTET
YYADSVKGRFTISRDNSKNTLYLQMNSLRAEDTAVYYCAKSLGRFDYWGQGTLVTVSS

**SEQ ID NO: 70 = IL18mAb-G4S-DOM10-53-474 (L chain)**

DIQMTQSPSSVSASVGDRVTITCLASEDIYTYLTWYQQKPGKAPKLLIYGANKLQDGVPSRFSGSGSGTDYTLTI
SSLQPEDFATYYCLQGSKFPLTFGQGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC**GGGGS**GVQLL
ESGGGLVQPGGSLRLSCAASGFTFAWYDMGWVRQAPGKGLEWVSSIDWHGEVTYYADSVKGRFTISRDNSKN
TLYLQMNSLRAEDTAVYYCATAEDEPGYDYWGQGTLVTVSS

**SEQ ID NO: 71 = IL-5 mAb-G4S-DOM9-112-210 heavy chain**

QVTLRESGPALVKPTQTLTLTCTVSGFSLTSYSVHWVRQPPGKGLEWLGVIWASGGTDYNSALMSRLSISKDTS
RNQVVLTMTNMDPVDTATYYCARDPPSSLLRLDYWGRGTLVTVSSASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALHNHYTQKSLSLSP
GK**GGGGS**EVQLLESGGGLVQPGGSLRLSCAASGFTFRNFGMGWVRQAPGKGLEWVSWIISSGTETYYADSVK
GRFTISRDNSKNTLYLQMNSLRAEDTAVYYCAKSLGRFDYWGQGTLVTVSS

**SEQ ID NO: 72 = IL-5 mAb-G4S-DOM10-53-474 light chain**

DIVMTQSPDSLAVSLGERATINCKSSQSLLNSGNQKNYLAWYQQKPGQPPKLLIYGASTRESGVPDRFSGSGSGTDFTLTISSLQAEDVAVYYCQNVHSFPFTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC**GGGGS**GVQLLESGGGLVQPGGSLRLSCAASGFTFAWYDMGWVRQAPGKGLEWVSSIDWHGEVTYYADSVKGRFTISRDNSKNTLYLQMNSLRAEDTAVYYCATAEDEPGYDYWGQGTLVTVSS

**9. Dual Targeting anti-TNF/anti-EGFR mAbdAb**

**SEQ ID NO: 73 = anti-TNFmAb (L chain)**

DIQMTQSPSSLSASVGDRVTITCRASQGIRNYLAWYQQKPGKAPKLLIYAASTLQSGVPSRFSGSGSGTDFTLTISSLQPEDVATYYCQRYNRAPYTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

**SEQ ID NO: 74 = anti-TNFmAb-DOM16-39-542 (H chain)**

EVQLVESGGGLVQPGRSLRLSCAASGFTFDDYAMHWVRQAPGKGLEWVSAITWNSGHIDYADSVEGRFTISRDNAKNSLYLQMNSLRAEDTAVYYCAKVSYLSTASSLDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKSTGDIQMTQSPSSLSASVGDRVTITCRASQWIGNLLDWYQQKPGKAPKLLIYYASFLQSGVPSRFSGSGYGTDFTLTISSLQPEDFATYYCQQANPAPLTFGQGTKVEIKR

**10. Dual Targeting anti-TNF/anti-VEGF mAbdAb**

**SEQ ID NO: 75 = anti-TNFmAb-DOM15-26-593 (H chain)**

EVQLVESGGGLVQPGRSLRLSCAASGFTFDDYAMHWVRQAPGKGLEWVSAITWNSGHIDYADSVEGRFTISRDNAKNSLYLQMNSLRAEDTAVYYCAKVSYLSTASSLDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKSTGEVQLLVSGGGLVQPGGSLRLSCAASGFTFKAYPMMWVRQAPGKGLEWVSEISPSGSYTYYADSVKGRFTISRDNSKNTLYLQMNSLRAEDTAVYYCAKDPRKLDYWGQGTLVTVSS

**11. Dual Targeting anti-IL1R1/anti-VEGF dAb-extended IgG**

**SEQ ID NO: 76 = DOM 15-26-593-VHdUMMY (H chain)**

EVQLLVSGGGLVQPGGSLRLSCAASGFTFKAYPMMWVRQAPGKGLEWVSEISPSGSYTYYADSVKGRFTISRDN
SKNTLYLQMNSLRAEDTAVYYCAKDPRKLDYWGQGTLVTVSSASTKGPSEVQLLESGGGLVQPGGSLRLSCAAS
GFTFSSYAMSWVRQAPGKGLEWVSAISGSGGSTYYADSVKGRFTISRDNSKNTLYLQMNSLRAEDTAVYYCAKS
YGAFDYWGQGTLVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQDWLNGKEYKCKV

SNKALPAPIEKTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

**SEQ ID NO: 77 = DOM4-130-54-VkdUMMY (L chain)**

DIQMTQSPSSLSASVGDRVTITCRASQDIYLNLDWYQQKPGKAPKLLINFGSELQSGVPSRFSGSGYGTDFTLTI
SSLQPEDFATYYCQPSFYFPYTFGQGTKVEIKRTVAAPSDIQMTQSPSSLSASVGDRVTITCRASQSISSYLNWY
QQKPGKAPKLLIYAASSLQSGVPSRFSGSGSGTDFTLTISSLQPEDFATYYCQQSYSTPNTFGQGTKVEIKRTVAA
PSVFIFPPSDEQLKSGTASVVCLLNNFYPREAKVQWKVDNALQSGNSQESVTEQDSKDSTYSLSSTLTLSKADYE
KHKVYACEVTHQGLSSPVTKSFNRGEC

**12. Triple Targeting anti-TNF/anti-EGFR/anti-VEGF mAbdAb**

**SEQ ID NO: 78 = DOM 15-26-anti-TNFmAb (H chain)**

EVQLLESGGGLVQPGGSLRLSCAASGFTFGAYPMMWVRQAPGKGLEWVSEISPSGSYTYYADSVKGRFTISRDN
SKNTLYLQMNSLRAEDTAVYYCAKDPRKFDYWGQGTLVTVSSASTKGPSEVQLVESGGGLVQPGRSLRLSCAAS
GFTFDDYAMHWVRQAPGKGLEWVSAITWNSGHIDYADSVEGRFTISRDNAKNSLYLQMNSLRAEDTAVYYCA
KVSYLSTASSLDYWGQGTLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK

**SEQ ID NO: 79 = DOM 16-39-542-anti-TNFmAb (L chain)**

DIQMTQSPSSLSASVGDRVTITCRASQWIGNLLDWYQQKPGKAPKLLIYYASFLQSGVPSRFSGSGYGTDFTLTI
SSLQPEDFATYYCQQANPAPLTFGQGTKVEIKRTVAAPSDIQMTQSPSSLSASVGDRVTITCRASQGIRNYLAWY
QQKPGKAPKLLIYAASTLQSGVPSRFSGSGSGTDFTLTISSLQPEDVATYYCQRYNRAPYTFGQGTKVEIKRTVA
APSVFIFPPSDEQLKSGTASVVCLLNNFYPREAKVQWKVDNALQSGNSQESVTEQDSKDSTYSLSSTLTLSKADY
EKHKVYACEVTHQGLSSPVTKSFNRGEC

**SEQ ID NO: 87 = 586H-210 (H chain) GS removed**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKEVQLLESGGGLVQPGGSLRLSCAASGFTFRNFGMGWVRQAPGKGLEWVSWIISSGTETYYADSV
KGRFTISRDNSKNTLYLQMNSLRAEDTAVYYCAKSLGRFDYWGQGTLVTVSS

**SEQ ID NO: 88 = 586H-TVAAPS-210 (H chain) GS removed**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**TVAAPS**EVQLLESGGGLVQPGGSLRLSCAASGFTFRNFGMGWVRQAPGKGLEWVSWIISSGTE
TYYADSVKGRFTISRDNSKNTLYLQMNSLRAEDTAVYYCAKSLGRFDYWGQGTLVTVSS

**SEQ ID NO: 89 = 586H-ASTKGPT-210 (H chain) both GS removed**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV

SLTCLVKGFYPSDIAVEWESNGQPENNYKTTPPVLDSDGSFFLYSKLTVDKSRWQQGNVFSCSVMHEALHNHYT
QKSLSLSPGK**ASTKGPT**EVQLLESGGGLVQPGGSLRLSCAASGFTFRNFGMGWVRQAPGKGLEWVSWIISSGT
ETYYADSVKGRFTISRDNSKNTLYLQMNSLRAEDTAVYYCAKSLGRFDYWGQGTLVTVSS

**SEQ ID NO: 90 = 586H-ASTKGPS-210 (H chain) both GS removed**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGK**ASTKGPS**EVQLLESGGGLVQPGGSLRLSCAASGFTFRNFGMGWVRQAPGKGLEWVSWIISSGT
ETYYADSVKGRFTISRDNSKNTLYLQMNSLRAEDTAVYYCAKSLGRFDYWGQGTLVTVSS

**SEQ ID NO: 91 = PascoH-474 (H chain) GS removed**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGVQLLESGGGLVQPGGSLRLSCAASGFTFAWYDMGWVRQAPGKGLEWVSSIDWHGEVTYYADSVKGRFTISRDNSKNTLYLQMNSLRAEDTAVYYCATAEDEPGYDYWGQGTLVTVSS

**SEQ ID NO: 92 = PascoH-TVAAPS-474 (H chain) GS removed**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK**TVAAPS**GVQLLESGGGLVQPGGSLRLSCAASGFTFAWYDMGWVRQAPGKGLEWVSSIDWHGEVTYYADSVKGRFTISRDNSKNTLYLQMNSLRAEDTAVYYCATAEDEPGYDYWGQGTLVTVSS

**SEQ ID NO: 93 = PascoH-ASTKGPT-474 (H chain) Both GS removed**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK**ASTKGPT**GVQLLESGGGLVQPGGSLRLSCAASGFTFAWYDMGWVRQAPGKGLEWVSSIDWHGEVTYYADSVKGRFTISRDNSKNTLYLQMNSLRAEDTAVYYCATAEDEPGYDYWGQGTLVTVSS

**SEQ ID NO: 94 = PascoH-ASTKGPS-474 (H chain) Both GS removed**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK**ASTKGPS**GVQLLESGGGLVQPGGSLRLSCAASGFTFAWYDMGWVRQAPGKGLEWVSSIDWHGEVTYYADSVKGRFTISRDNSKNTLYLQMNSLRAEDTAVYYCATAEDEPGYDYWGQGTLVTVSS

**SEQ ID NO: 95 = PascoH-ASTKGPS-474 (H chain) Second GS removed**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGS**ASTKGPS**GVQLLESGGGLVQPGGSLRLSCAASGFTFAWYDMGWVRQAPGKGLEWVSSIDWHGE
VTYYADSVKGRFTISRDNSKNTLYLQMNSLRAEDTAVYYCATAEDEPGYDYWGQGTLVTVSS

**SEQ ID NO: 96 = PascoH-ASTKGPT-474 (H chain) Second GS removed**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGS**ASTKGPT**GVQLLESGGGLVQPGGSLRLSCAASGFTFAWYDMGWVRQAPGKGLEWVSSIDWHGE
VTYYADSVKGRFTISRDNSKNTLYLQMNSLRAEDTAVYYCATAEDEPGYDYWGQGTLVTVSS

**SEQ ID NO: 97 = CDRH1**
GYSITSDFAWN
**SEQ ID NO: 98 = CDRH2**
GYISYSGNYNPSLK
**SEQ ID NO: 99 = CDRH3**
VTAGRGFPY
**SEQ ID NO: 100 = CDRL1**
HSSQDINSNIG
**SEQ ID NO: 101 = CDRL2**
HGINLDD
**SEQ ID NO: 102 = CDRL3**
VQYAQFPWT
**SEQ ID NO: 103 = EGFR epitope**
CGADSYEMEEDGVRKC
**SEQ ID NO: 104 = CDRH1**
SDFAWN
**SEQ ID NO: 105 = CDRH2**
YISYSGNYNPSLK
**SEQ ID NO: 106 = CDRH3**
AGRGFPY
**SEQ ID NO: 107 = CDRH2**
YISYSGNYNPSLKS
**SEQ ID NO: 108 = Heavy chain of anti-IGF-1R antibody H0L0 with DOM15-26-593 fused at C-terminus with TVAAPSGS linker**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKTVAAPSGSEVQLLVSGGGLVQPGGSLRLSCAASGFTFKAYPMMWVRQAPGKGLEWVSEISPSGS
YTYYADSVKGRFTISRDNSKNTLYLQMNSLRAEDTAVYYCAKDPRKLDYWGQGTLVTVSS

**SEQ ID NO: 109 = Heavy chain of anti-IGF-1R antibody H0L0 with DOM15-26-593 fused at C-terminus with GS linker**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKGSEVQLLVSGGGLVQPGGSLRLSCAASGFTFKAYPMMWVRQAPGKGLEWVSEISPSGSYTYYAD
SVKGRFTISRDNSKNTLYLQMNSLRAEDTAVYYCAKDPRKLDYWGQGTLVTVSS

**SEQ ID NO: 110 = Heavy chain of anti-IGF-1R antibody H0L0**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGK

**SEQ ID NO: 111 = Light chain of anti-IGF-1R antibody H0L0 with DOM15-26-593 fused at C-terminus with TVAAPSGS linker**

DIVMTQSPLSLPVTPGEPASISCRSSQSIVQSNGDTYLEWYLQKPGQSPQLLIYRVSNRFSGVPDRFSGSGSGTD
FTLKISRVEAEDVGVYYCFQGSHVPYTFGQGTKLEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGECTVAAPS
GSEVQLLVSGGGLVQPGGSLRLSCAASGFTFKAYPMMWVRQAPGKGLEWVSEISPSGSYTYYADSVKGRFTISR
DNSKNTLYLQMNSLRAEDTAVYYCAKDPRKLDYWGQGTLVTVSS

**SEQ ID NO: 112 = Light chain of anti-IGF-1R antibody H0L0 with DOM15-26-593 fused at C-terminus with GS linker**

DIVMTQSPLSLPVTPGEPASISCRSSQSIVQSNGDTYLEWYLQKPGQSPQLLIYRVSNRFSGVPDRFSGSGSGTD
FTLKISRVEAEDVGVYYCFQGSHVPYTFGQGTKLEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGECGSEVQL
LVSGGGLVQPGGSLRLSCAASGFTFKAYPMMWVRQAPGKGLEWVSEISPSGSYTYYADSVKGRFTISRDNSKNT
LYLQMNSLRAEDTAVYYCAKDPRKLDYWGQGTLVTVSS

**SEQ ID NO: 113 = Light chain of anti-IGF-1R antibody H0L0**

DIVMTQSPLSLPVTPGEPASISCRSSQSIVQSNGDTYLEWYLQKPGQSPQLLIYRVSNRFSGVPDRFSGSGSGTD
FTLKISRVEAEDVGVYYCFQGSHVPYTFGQGTKLEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSSPVTKSFNRGEC

**SEQ ID NO: 114 = Variable heavy domain of antibody 2B9**

QVQLKQSGPGLVQSSQSLSITCTISGFSLTSHGIYWLRQSPGKGLEWLGVIWSGGSADYNAAFISRLSISKDNSK
SQVFFKMNSLQADDTAIYYCARSPYYYRSSLYAMDYWGQGTSVTVSS

**SEQ ID NO: 115 = Variable light domain of antibody 2B9**

NIVLTQSPKSMSMSIGERVTLSCKASENVGTYVSWYQQKAEQSPKLLIYGASNRHTGVPDRFTGSGSSTDFTLTI
SSVQAEDLADYHCGQSYSDPLTFGAGTKLELKRA

**SEQ ID NO: 116 = Protein sequence of anti-CD20 mAb heavy chain with TVAAPSGS linker and DOM10-53-474 domain antibody fused at C-terminus**

QVQLQQPGAELVKPGASVKMSCKASGYTFTSYNMHWVKQTPGRGLEWIGAIYPGNGDTSYNQKFKGKATLTA
DKSSSTAYMQLSSLTSEDSAVYYCARSTYYGGDWYFNVWGAGTLVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSLTC

LVKGFYPSDIAVEWESNGQPENNYKTTPPVLDSDGSFFLYSKLTVDKSRWQQGNVFSCSVMHEALHNHYTQKS
LSLSPGK<u>TVAAPSGS</u>GVQLLESGGGLVQPGGSLRLSCAASGFTFAWYDMGWVRQAPGKGLEWVSSIDWHGEV
TYYADSVKGRFTISRDNSKNTLYLQMNSLRAEDTAVYYCATAEDEPGYDYWGQGTLVTVSS

**SEQ ID NO: 117 = Protein sequence anti-CD20 mAb VL-human C light chain**

QIVLSQSPAILSASPGEKVTMTCRASSSVSYIHWFQQKPGSSPKPWIYATSNLASGVPVRFSGSGSGTSYSLTISR
VEAEDAATYYCQQWTSNPPTFGGGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC

**SEQ ID NO: 118 = Protein sequence of anti-CD20 mAb heavy chain with GS linker and DOM10-53-474 domain antibody fused at C-terminus**

QVQLQQPGAELVKPGASVKMSCKASGYTFTSYNMHWVKQTPGRGLEWIGAIYPGNGDTSYNQKFKGKATLTA
DKSSSTAYMQLSSLTSEDSAVYYCARSTYYGGDWYFNVWGAGTLVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKS
LSLSPGK<u>GS</u>GVQLLESGGGLVQPGGSLRLSCAASGFTFAWYDMGWVRQAPGKGLEWVSSIDWHGEVTYYADS
VKGRFTISRDNSKNTLYLQMNSLRAEDTAVYYCATAEDEPGYDYWGQGTLVTVSS

**SEQ ID NO: 119 = Protein sequence of anti-CD20 mAb light chain with TVAAPSGS linker and DOM10-53-474 domain antibody fused at C-terminus**

QIVLSQSPAILSASPGEKVTMTCRASSSVSYIHWFQQKPGSSPKPWIYATSNLASGVPVRFSGSGSGTSYSLTISR
VEAEDAATYYCQQWTSNPPTFGGGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGECTVAAPSGSGVQ
LLESGGGLVQPGGSLRLSCAASGFTFAWYDMGWVRQAPGKGLEWVSSIDWHGEVTYYADSVKGRFTISRDNS
KNTLYLQMNSLRAEDTAVYYCATAEDEPGYDYWGQGTLVTVSS

**SEQ ID NO: 120 = Protein sequence of anti-CD20 mAb VH-human IgG1 heavy chain**

QVQLQQPGAELVKPGASVKMSCKASGYTFTSYNMHWVKQTPGRGLEWIGAIYPGNGDTSYNQKFKGKATLTA
DKSSSTAYMQLSSLTSEDSAVYYCARSTYYGGDWYFNVWGAGTLVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKS
LSLSPGK

**SEQ ID NO: 121 = Protein sequence of anti-CD20 mAb light chain with GS linker and DOM10-53-474 domain antibody fused at C-terminus**

QIVLSQSPAILSASPGEKVTMTCRASSSVSYIHWFQQKPGSSPKPWIYATSNLASGVPVRFSGSGSGTSYSLTISR
VEAEDAATYYCQQWTSNPPTFGGGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGECGSGVQLLESGG
GLVQPGGSLRLSCAASGFTFAWYDMGWVRQAPGKGLEWVSSIDWHGEVTYYADSVKGRFTISRDNSKNTLYL
QMNSLRAEDTAVYYCATAEDEPGYDYWGQGTLVTVSS

**SEQ ID NO: 122: 586H-TVAAPS-154 Heavy chain**

CAGGTGCAGCTCGTGCAGAGCGGCGCCGAAGTGAAAAAGCCCGGCAGCAGCGTGAAGGTGAGCTGCAAGGC
CTCCGGCTTCTACATCAAGGACACCTACATGCACTGGGTCAGGCAGGCTCCTGGCCAGGGCCTGGAGTGGAT
GGGCACTATCGACCCCGCCAACGGCAACACCAAGTACGTGCCCAAGTTCCAGGGCAGGGTGACCATCACCGC
CGATGAGAGCACCAGCACCGCCTACATGGAACTGAGCAGCCTGAGGTCTGAGGACACCGCCGTGTACTATTG
CGCCAGGAGCATCTACGACGACTACCACTACGACGACTACTACGCCATGGACTACTGGGGACAGGGCACACT
AGTGACCGTGTCCAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTACAGCCTGAGC
AGCGTGGTGACCGTGCCCAGCAGCAGCCTGGGCACCCAGACCTACATCTGTAACGTGAACCACAAGCCCAGC

AACACCAAGGTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATGATCAGCAGA
ACCCCCGAGGTGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCACCTACCGGGTGG
TGTCCGTGCTGACCGTGCTGCACCAGGATTGGCTGAACGGCAAGGAGTACAAGTGTAAGGTGTCCAACAAG
GCCCTGCCTGCCCCTATCGAGAAAACCATCAGCAAGGCCAAGGGCCAGCCCAGAGAGCCCCAGGTGTACACC
CTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTGTCCCCTGG
CAAGACCGTGGCCGCCCCCTCGGGATCCGACATCCAGATGACCCAGAGCCCCAGCAGCCTGAGCGCCAGCGT
GGGCGACAGGGTGACCATTACCTGCAGGGCCAGCAGGCCCATCAGCGACTGGCTGCACTGGTACCAACAGA
AGCCCGGCAAGGCTCCCAAGCTGCTGATCGCCTGGGCCAGCAGCCTGCAGGGAGGCGTGCCCAGCAGGTTT
AGCGGCAGCGGCAGCGGCACCGACTTCACCCTCACCATCTCTTCCCTGCAGCCCGAGGACTTCGCCACCTAC
TACTGCCTGCAGGAGGGCTGGGGGCCCCCTACTTTCGGCCAGGGCACCAAGGTGGAGATCAAGAGG

**SEQ ID NO: 123**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKGSDGGGIRRSMSGTWYLKAMTVDREFPEMNLESVTPMTLTLLKGHNLEAKVTMLISGRCQEVKA
VLGRTKERKKYTADGGKHVAYIIPSAVRDHVIFYSEGQLHGKPVRGVKLVGRDPKNNLEALEDFEKAAGARGLST
ESILIPRQSETCSPG

**SEQ ID NO: 124**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKGSEVVAATPTSLLISWRHPHFPTRYYRITYGETGGNSPVQEFTVPLQPPTATISGLKPGVDYTITV
YAVTDGRNGRLLSIPISINYRT

**SEQ ID NO:125**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKTVAAPSGSDGGGIRRSMSGTWYLKAMTVDREFPEMNLESVTPMTLTLLKGHNLEAKVTMLISGR
CQEVKAVLGRTKERKKYTADGGKHVAYIIPSAVRDHVIFYSEGQLHGKPVRGVKLVGRDPKNNLEALEDFEKAAG
ARGLSTESILIPRQSETCSPG

**SEQ ID NO: 126**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT

KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKGSVDNKFNKELRQAYWEIQALPNLNWTQSRAFIRSLYDDPSQSANLLAEAKKLNDAQAPK

**SEQ ID NO: 127**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKTVAAPSGSVDNKFNKELRQAYWEIQALPNLNWTQSRAFIRSLYDDPSQSANLLAEAKKLNDAQA
PK

**SEQ ID NO: 128**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKGSDLGKKLLEAARAGQDDEVRILMANGADVNAKDEYGLTPLYLATAHGHLEIVEVLLKNGADVNA
VDAIGFTPLHLAAFIGHLEIAEVLLKHGADVNAQDKFGKTAFDISIGNGNEDLAEILQKL

**SEQ ID NO: 129**

QVQLVQSGAEVKKPGASVKVSCKASGYTFTDYYMNWVRQAPGQGLEWMGNINPNNGGTNYNQKFKDRVTMTTDTSTSTAYMELRSLRSDDTAVYYCARWILYYGRSKWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKTVAAPSGSDLGKKLLEAARAGQDDEVRILMANGADVNAKDEYGLTPLYLATAHGHLEIVEVLLKNGADVNAVDAIGFTPLHLAAFIGHLEIAEVLLKHGADVNAQDKFGKTAFDISIGNGNEDLAEILQKL

**SEQ ID NO: 130**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGSQVQLVESGGGLVQAGGSLRLSCAASGYAYTYIYMGWFRQAPGKEREGVAAMDSGGGGTLYADSVKGRFTISRDKGKNTVYLQMDSLKPEDTATYYCAAGGYELRDRTYGQWGQGTQVTVSS

**SEQ ID NO: 131**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGSARVDQTPRSVTKETGESLTINCVLRDASYALGSTCWYRKKSGEGNEESISKGGRYVETVNSGSKSFSLRINDLTVEDGGTYRCGLGVAGGYCDYALCSSRYAECGDGTAVTVN

**SEQ ID NO: 132: Anti IL-4 Heavy Chain-TVAAPSGS- anti TNF-a adnectin**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGACCGT
GGCCGCCCCCTCGGGATCCGTGAGCGACGTGCCAAGGGACCTCGAGGTGGTGGCAGCCACTCCCACCTCTC
TGCTGATCAGCTGGGACACACACAACGCCTACAACGGCTACTACAGGATCACCTACGGAGAGACCGGCGGCA
ATAGCCCCGTGAGGGAGTTCACCGTGCCCCACCCCGAGGTGACCGCCACCATTAGCGGCCTGAAGCCCGGC
GTGGACGATACCATCACCGTCTACGCCGTGACCAACCACCACATGCCCCTGAGGATCTTCGGCCCCATCAGC
ATCAACCATAGGACC

**SEQ ID NO: 133**

QVQLVQSGAEVKKPGASVKVSCKASGYTFTDYYMNWVRQAPGQGLEWMGNINPNNGGTNYNQKFKDRVTMT
TDTSTSTAYMELRSLRSDDTAVYYCARWILYYGRSKWYFDVWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYT
QKSLSLSPGKTVAAPSGSEVVAATPTSLLISWRHPHFPTRYYRITYGETGGNSPVQEFTVPLQPPTATISGLKPGV
DYTITVYAVTDGRNGRLLSIPISINYRT

**SEQ ID NO: 134**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT
QKSLSLSPGKGSVSDVPRDLEVVAATPTSLLISWDTHNAYNGYYRITYGETGGNSPVREFTVPHPEVTATISGLK
PGVDDTITVYAVTNHHMPLRIFGPISINHRT

**SEQ ID NO: 135**

QVQLVQSGAEVKKPGSSVKVSCKASGFYIKDTYMHWVRQAPGQGLEWMGTIDPANGNTKYVPKFQGRVTITA
DESTSTAYMELSSLRSEDTAVYYCARSIYDDYHYDDYYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYT

QKSLSLSPGKTVAAPSGSVSDVPRDLEVVAATPTSLLISWDTHNAYNGYYRITYGETGGNSPVREFTVPHPEVTA
TISGLKPGVDDTITVYAVTNHHMPLRIFGPISINHRT

**SEQ ID NO: 136**

QVQLKQSGPGLVQPSQSLSITCTVSGFSLTNYGVHWVRQSPGKGLEWLGVIWSGGNTDYNTPFTSRLSINKDN
SKSQVFFKMNSLQSNDTAIYYCARALTYYDYEFAYWGQGTLVTVSAASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALHNHYTQKSLSLSP
GKRSEVVAATPTSLLISWRHPHFPTRYYRITYGETGGNSPVQEFTVPLQPPTATISGLKPGVDYTITVYAVTDGR
NGRLLSIPISINYRT

**SEQ ID NO: 137**

DILLTQSPVILSVSPGERVSFSCRASQSIGTNIHWYQQRTNGSPRLLIKYASESISGIPSRFSGSGSGTDFTLSINS
VESEDIADYYCQQNNNWPTTFGAGTKLELKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC

**SEQ ID NO: 138**

DILLTQSPVILSVSPGERVSFSCRASQSIGTNIHWYQQRTNGSPRLLIKYASESISGIPSRFSGSGSGTDFTLSINS
VESEDIADYYCQQNNNWPTTFGAGTKLELKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGECRSEVVAATPTSL
LISWRHPHFPTRYYRITYGETGGNSPVQEFTVPLQPPTATISGLKPGVDYTITVYAVTDGRNGRLLSIPISINYRT

**SEQ ID NO: 139**

QVQLKQSGPGLVQPSQSLSITCTVSGFSLTNYGVHWVRQSPGKGLEWLGVIWSGGNTDYNTPFTSRLSINKDN
SKSQVFFKMNSLQSNDTAIYYCARALTYYDYEFAYWGQGTLVTVSAASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALHNHYTQKSLSLSP
GK

**SEQ ID NO: 140**

QVQLQESGPGLVKPSQTLSLTCTVSGGSISSGDYYWSWIRQPPGKGLEWIGYIYYSGSTDYNPSLKSRVTMSVD
TSKNQFSLKVNSVTAADTAVYYCARVSIFGVGTFDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQKSLSL
SPGKGSEVVAATPTSLLISWRHPHFPTRYYRITYGETGGNSPVQEFTVPLQPPTATISGLKPGVDYTITVYAVTDG
RNGRLLSIPISINYRT

**SEQ ID NO: 141**

EIVMTQSPATLSLSPGERATLSCRASQSVSSYLAWYQQKPGQAPRLLIYDASNRATGIPARFSGSGSGTDFTLTIS
SLEPEDFAVYYCHQYGSTPLTFGGGTKAEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC

**SEQ ID NO: 142**

EIVMTQSPATLSLSPGERATLSCRASQSVSSYLAWYQQKPGQAPRLLIYDASNRATGIPARFSGSGSGTDFTLTIS
SLEPEDFAVYYCHQYGSTPLTFGGGTKAEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGECGSEVVAATPTSL
LISWRHPHFPTRYYRITYGETGGNSPVQEFTVPLQPPTATISGLKPGVDYTITVYAVTDGRNGRLLSIPISINYRT

**SEQ ID NO: 143**

QVQLQESGPGLVKPSQTLSLTCTVSGGSISSGDYYWSWIRQPPGKGLEWIGYIYYSGSTDYNPSLKSRVTMSVD
TSKNQFSLKVNSVTAADTAVYYCARVSIFGVGTFDYWGQGTLVTVSSASTKGPSVFPLAPSSKSTSGGTAALGCL

VKDYFPEPVTVSWNSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQKSLSL
SPGK

**SEQ ID NO: 144**

EVVAATPTSLLISWRHPHFPTRYYRITYGETGGNSPVQEFTVPLQPPTATISGLKPGVDYTITVYAVTDGRNGRLL
SIPISINYRTGGSTGQVQLKQSGPGLVQPSQSLSITCTVSGFSLTNYGVHWVRQSPGKGLEWLGVIWSGGNTDYN
TPFTSRLSINKDNSKSQVFFKMNSLQSNDTAIYYCARALTYYDYEFAYWGQGTLVTVSAASTKGPSVFPLAPSSK
STSGGTAALGCLVKDYFPEPVTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQGNVFSCSVMHEAL
HNHYTQKSLSLSPGK

**SEQ ID NO: 145**

EVVAATPTSLLISWRHPHFPTRYYRITYGETGGNSPVQEFTVPLQPPTATISGLKPGVDYTITVYAVTDGRNGRLL
SIPISINYRTSTGDILLTQSPVILSVSPGERVSFSCRASQSIGTNIHWYQQRTNGSPRLLIKYASESISGIPSRFSGS
GSGTDFTLSINSVESEDIADYYCQQNNNWPTTFGAGTKLELKRTVAAPSVFIFPPSDEQLKSGTASVVCLLNNFY
PREAKVQWKVDNALQSGNSQESVTEQDSKDSTYSLSSTLTLSKADYEKHKVYACEVTHQGLSSPVTKSFNRGEC

**SEQ ID NO: 146**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGSVSDVPRDLEVVAATPTSLLISWDTHNAYNGYYRITYGETGGNSPVREFTVPHPEVTATISGLKPGVD
DTITVYAVTNHHMPLRIFGPISINHRT

**SEQ ID NO: 147**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKTVAAPSGSVSDVPRDLEVVAATPTSLLISWDTHNAYNGYYRITYGETGGNSPVREFTVPHPEVTATISG
LKPGVDDTITVYAVTNHHMPLRIFGPISINHRT

**SEQ ID NO: 148 = DOM10-53-616 (dAb)**

GVQLLESGGGLVQPGGSLRLSCAASGFVFPWYDMGWVRQAPGKGLEWVSSIDWHGKITYYADSVKGRFTISRD
NSKNTLYLQMNSLRAEDTAVYYCATAEDEPGYDYWGQGTLVTVSS

**SEQ ID NO: 149 = PascoH-616 (H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGVQLLESGGGLVQPGGSLRLSCAASGFVFPWYDMGWVRQAPGKGLEWVSSIDWHGKITYYADSVKG
RFTISRDNSKNTLYLQMNSLRAEDTAVYYCATAEDEPGYDYWGQGTLVTVSS

**SEQ ID NO: 150 = PascoH-TVAAPS-616 (H chain)**

QVTLRESGPALVKPTQTLTLTCTFSGFSLSTSGMGVSWIRQPPGKGLEWLAHIYWDDDKRYNPSLKSRLTISKDTSRNQVVLTMTNMDPVDTATYYCARRETVFYWYFDVWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK**TVAAPS**GVQLLESGGGLVQPGGSLRLSCAASGFVFPWYDMGWVRQAPGKGLEWVSSIDWHGKITYYADSVKGRFTISRDNSKNTLYLQMNSLRAEDTAVYYCATAEDEPGYDYWGQGTLVTVSS

**SEQ ID NO: 151 = C1-TVAAPSGS-210 (H chain)**

QVQLVQSGAEVKKPGASVKVSCKASGFYIKDTYMHWVRQAPGQGLEWMGTIDPANGNTKYVPKFQGRVTMTTDTSTSTAYMELRSLRSDDTAVYYCARSIYDDYHYDDYYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK**TVAAPS**GSEVQLLESGGGLVQPGGSLRLSCAASGFTFRNFGMGWVRQAPGKGLEWVSWIISSGTETYYADSVKGRFTISRDNSKNTLYLQMNSLRAEDTAVYYCAKSLGRFDYWGQGTLVTVSS

**SEQ ID NO: 152 = D1-TVAAPSGS-210 (H chain)**

EVQLVQSGAEVKKPGESLKISCKGSGFYIKDTYMHWVRQMPGKGLEWMGTIDPANGNTKYVPKFQGQVTISADKSISTAYLQWSSLKASDTAMYYCARSIYDDYHYDDYYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK**TVAAPS**GSEVQLLESGGGLVQPGGSLRLSCAASGFTFRNFGMGWVRQAPGKGLEWVSWIISSGTETYYADSVKGRFTISRDNSKNTLYLQMNSLRAEDTAVYYCAKSLGRFDYWGQGTLVTVSS

**SEQ ID NO: 153 = N0 (L chain)**

EIVLTQSPATLSLSPGERATLSCRSSQNIVHINGNTYLEWYQQKPGQAPRLLIYKISDRFSGIPARFSGSGSGTDFTLTISSLEPEDFAVYYCFQGSHVPWTFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

**SEQ ID NO: 154 = M0 (L chain)**

EIVLTQSPATLSLSPGERATLSCRSSQNIVHINGNTYLEWYQQKPGQAPRLLIYKISDRFSGIPARFSGSGSGTDFTLTISSLEPEDFAVYYCFQGSHVPWTFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

**SEQ ID NO: 155 = 656H-TVAAPS-210_(H chain)**

QVQLVQSGAEVKKPGASVKVSCKASGYTFIDYEIHWVRQAPGQGLEWMGAIDPETGGTAYNQKFKGRVTMTT
DTSTSTAYMELRSLRSDDTAVYYCTRILLYYYPMDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQKSLSL
SPGK**TVAAPS**EVQLLESGGGLVQPGGSLRLSCAASGFTFRNFGMGWVRQAPGKGLEWVSWIISSGTETYYADS
VKGRFTISRDNSKNTLYLQMNSLRAEDTAVYYCAKSLGRFDYWGQGTLVTVSS

**SEQ ID NO: 156 = 656_(L chain)**

EIVLTQSPATLSLSPGERATLSCRASQNISDYLHWYQQKPGQAPRLLIYYASQSISGIPARFSGSGSGTDFTLTISS
LEPEDFAVYYCQNGHSFPLTFGGGTKVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVTKSFNRGEC

**SEQ ID NO: 157 = PascoH-TVAAPS-546_(H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGK**TVAAPS**GVQLLESGGGLVQPGGSLRLSCAASGFVFPWYDMGWVRQAPGKGLEWVSSIDWKGGKTYY
ADSVKGRFTISRDNSKNTLYLQMNSLRAEDTAVYYCATAEDEPGYDYWGQGTLVTVSS

**SEQ ID NO: 158 = PascoH-546_(H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGVQLLESGGGLVQPGGSLRLSCAASGFVFPWYDMGWVRQAPGKGLEWVSSIDWKGGKTYYADSVKG
RFTISRDNSKNTLYLQMNSLRAEDTAVYYCATAEDEPGYDYWGQGTLVTVSS

**SEQ ID NO: 159 = PascoH-TVAAPS-567_(H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGK**TVAAPS**GVQLLESGGGLVQPGGSLRLSCAASGFVFAWYDMGWVRQAPGKGLEWVSSIDWHGEVTYY
ADSVKGRFTISRDNSKNTLYLQMNSLRAEDTAVYYCATAEDEPGYDYWGQGTLVTVSS

**SEQ ID NO: 160 = PascoH-567_(H chain)**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSL
SLSPGKGVQLLESGGGLVQPGGSLRLSCAASGFVFAWYDMGWVRQAPGKGLEWVSSIDWHGEVTYYADSVKG
RFTISRDNSKNTLYLQMNSLRAEDTAVYYCATAEDEPGYDYWGQGTLVTVSS

**SEQ ID NO: 161 = 656_(H chain)**

QVQLVQSGAEVKKPGASVKVSCKASGYTFIDYEIHWVRQAPGQGLEWMGAIDPETGGTAYNQKFKGRVTMTT
DTSTSTAYMELRSLRSDDTAVYYCTRILLYYYPMDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQKSLSL
SPGK

**SEQ ID NO: 162**

GAGGTGCAGCTGTTGGAGTCTGGGGGAGGCTTGGTACAGCCTGGGGGGTCCCTGCGTCTCTCCTGTGCAGC
CTCCGGATTCACCTTTGGGGCTTATCCGATGATGTGGGTCCGCCAGGCTCCAGGGAAGGGTCTAGAGTGGG
TCTCAGAGATTTCGCCTTCGGGTTCTTATACATACTACGCAGACTCCGTGAAGGGCCGGTTCACCATCTCCC
GCGACAATTCCAAGAACACGCTGTATCTGCAAATGAACAGCCTGCGTGCCGAGGACACCGCGGTATATTACT
GTGCGAAAGATCCTCGGAAGTTTGACTACTGGGGTCAGGGAACCCTGGTCACCGTCTCGAGCGCTAGCACCA

AGGGCCCCAGCGAGGTGCAGCTGTTGGAGTCTGGGGGAGGCTTGGTACAGCCTGGGGGGTCCCTGCGTCTC
TCCTGTGCAGCCTCCGGATTCACCTTTAGCAGCTATGCCATGAGCTGGGTCCGCCAGGCTCCAGGGAAGGGT
CTAGAGTGGGTCTCAGCTATTAGTGGTAGTGGTGGTAGCACATACTACGCAGACTCCGTGAAGGGCCGGTTC
ACCATCTCCCGCGACAATTCCAAGAACACGCTGTATCTGCAAATGAACAGCCTGCGTGCCGAGGACACCGCG
GTATATTACTGTGCGAAAAGTTATGGTGCTTTTGACTACTGGGGCCAGGGAACCCTGGTCACCGTCTCGAGC
GCTAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAGCCTGTGACCGTGTCCTGGAATAGCGGAGCCCTGACCTC
CGGCGTGCACACCTTCCCCGCCGTGCTGCAGAGCAGCGGCCTGTACTCCCTGAGCAGCGTGGTGACCGTGC
CCAGCAGCAGCCTGGGCACCCAGACCTACATCTGCAACGTGAACCACAAGCCCAGCAACACCAAAGTGGACA
AGAAAGTGGAGCCCAAGAGCTGCGATAAGACCCACACCTGCCCCCCCTGCCCTGCCCCCGAGCTGCTGGGCG
GACCTAGCGTGTTCCTGTTCCCCCCCAAGCCTAAGGACACCCTGATGATCAGCAGGACCCCCGAAGTGACCT
GCGTGGTGGTGGATGTGAGCCACGAGGACCCTGAAGTGAAGTTCAACTGGTACGTGGACGGCGTGGAAGTG
CACAACGCCAAGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCCTGCCCCTATC
GAGAAAACCATCAGCAAGGCCAAGGGCCAGCCTAGAGAGCCCCAGGTCTACACCCTGCCTCCCTCCAGAGAT
GAGCTGACCAAGAACCAGGTGTCCCTGACCTGTCTGGTGAAGGGCTTCTACCCCAGCGACATCGCCGTGGAG
TGGGAGAGCAACGGCCAGCCCGAGAACAACTACAAGACCACCCCCCCTGTGCTGGACAGCGATGGCAGCTTC
TTCCTGTACTCCAAGCTGACCGTGGACAAGAGCAGATGGCAGCAGGGCAACGTGTTCAGCTGCAGCGTGATG
CACGAGGCCCTGCACAATCACTACACCCAGAAGAGTCTGAGCCTGTCCCCTGGCAAG

**SEQ ID NO: 163**

EVQLLESGGGLVQPGGSLRLSCAASGFTFGAYPMMWVRQAPGKGLEWVSEISPSGSYTYYADSVKGRFTISRDN
SKNTLYLQMNSLRAEDTAVYYCAKDPRKFDYWGQGTLVTVSSASTKGPSEVQLLESGGGLVQPGGSLRLSCAAS
GFTFSSYAMSWVRQAPGKGLEWVSAISGSGGSTYYADSVKGRFTISRDNSKNTLYLQMNSLRAEDTAVYYCAKS
YGAFDYWGQGTLVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQDWLNGKEYKCKV
SNKALPAPIEKTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

**SEQ ID NO: 164**

CAGGTGCAGCTGAAGCAGAGCGGCCCTGGCCTGGTGCAGCCCTCTCAGAGCCTGAGCATCACCTGTACCGTGAGCGGCTTCAGCCTGA
CCAATTACGGCGTGCATTGGGTGCGGCAGTCTCCAGGCAAGGGCCTGGAATGGCTGGGAGTGATCTGGTCCGGCGGCAACACCGACTA
CAACACCCCCTTCACCAGCAGACTGAGCATCAACAAGGACAACAGCAAGAGCCAGGTGTTCTTCAAGATGAACAGCCTGCAGAGCAACG
ACACCGCCATCTACTATTGTGCCAGGGCCCTGACCTACTACGACTACGAGTTCGCCTACTGGGGCCAGGGCACCCTGGTGACCGTGAGC
GCCGCTAGCACCAAGGGCCCCAGCGTGTTCCCCCTGGCCCCCAGCAGCAAGAGCACCAGCGGCGGCACAGCCGCCCTGGGCTGCCTGG
TGAAGGACTACTTCCCCGAGCCTGTGACCGTGTCCTGGAATAGCGGAGCCCTGACCTCCGGCGTGCACACCTTCCCCGCCGTGCTGCAG
AGCAGCGGCCTGTACTCCCTGAGCAGCGTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCTGCCCCCCCTGCCCTGCCCCCGAGCTGC
TGGGCGGACCTAGCGTGTTCCTGTTCCCCCCCAAGCCTAAGGACACCCTGATGATCAGCAGGACCCCCGAAGTGACCTGCGTGGTGGTG
GATGTGAGCCACGAGGACCCTGAAGTGAAGTTCAACTGGTACGTGGACGGCGTGGAAGTGCACAACGCCAAGACCAAGCCCAGAGAGG
AGCAGTACAACAGCACCTACCGCGTGGTGTCTGTGCTGACCGTGCTGCACCAGGATTGGCTGAACGGCAAGGAGTACAAGTGCAAAGT
GAGCAACAAGGCCCTGCCTGCCCCTATCGAGAAAACCATCAGCAAGGCCAAGGGCCAGCCTAGAGAGCCCCAGGTCTACACCCTGCCTC
CCTCCAGAGATGAGCTGACCAAGAACCAGGTGTCCCTGACCTGTCTGGTGAAGGGCTTCTACCCCAGCGACATCGCCGTGGAGTGGGA
GAGCAACGGCCAGCCCGAGAACAACTACAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTGCACAATCACTACACCCAGAAGAGTCTG
AGCCTGTCCCCTGGCAAGTCGACCGGTGAGGTGCAGCTGTTGGTGTCTGGGGGAGGCTTGGTACAGCCTGGGGGGTCCCTGCGTCTCT
CCTGTGCAGCCTCCGGATTCACCTTTAAGGCTTATCCGATGATGTGGGTCCGCCAGGCTCCAGGGAAGGGTCTAGAGTGGGTTTCAGAG
ATTTCGCCTTCGGGTTCTTATACATACTACGCAGACTCCGTGAAGGGCCGGTTCACCATCTCCCGCGACAATTCCAAGAACACGCTGTAT
CTGCAAATGAACAGCCTGCGTGCCGAGGACACCGCGGTATATTACTGTGCGAAAGATCCTCGGAAGTTAGACTACTGGGGTCAGGGAAC
CCTGGTCACCGTCTCGAGC

**SEQ ID NO: 165:**

QVQLKQSGPGLVQPSQSLSITCTVSGFSLTNYGVHWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLSLSPGKSTGEVQLLVSG
GGLVQPGGSLRLSCAASGFTFKAYPMMWVRQAPGKGLEWVSEISPSGSYTYYADSVKGRFTISRDNSKNTLYLQMNSLRAEDTAVYYCAKD
PRKLDYWGQGTLVTVSS

**SEQ ID NO: 166**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTPVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKRVEPK
SCDKTHTCPPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMHEALHNHYTQKSL
SLSPGK

**SEQ ID NO: 167 586H-TVAAPS-210 Heavy chain**

CAGGTGCAGCTCGTGCAGAGCGGCGCCGAAGTGAAAAAGCCCGGCAGCAGCGTGAAGGTGAGCTGCAAGGC
CTCCGGCTTCTACATCAAGGACACCTACATGCACTGGGTCAGGCAGGCTCCTGGCCAGGGCCTGGAGTGGAT
GGGCACTATCGACCCCGCCAACGGCAACACCAAGTACGTGCCCAAGTTCCAGGGCAGGGTGACCATCACCGC
CGATGAGAGCACCAGCACCGCCTACATGGAACTGAGCAGCCTGAGGTCTGAGGACACCGCCGTGTACTATTG
CGCCAGGAGCATCTACGACGACTACCACTACGACGACTACTACGCCATGGACTACTGGGGACAGGGCACACT
AGTGACCGTGTCCAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTACAGCCTGAGC
AGCGTGGTGACCGTGCCCAGCAGCAGCCTGGGCACCCAGACCTACATCTGTAACGTGAACCACAAGCCCAGC
AACACCAAGGTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATGATCAGCAGA
ACCCCCGAGGTGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCACCTACCGGGTGG
TGTCCGTGCTGACCGTGCTGCACCAGGATTGGCTGAACGGCAAGGAGTACAAGTGTAAGGTGTCCAACAAG
GCCCTGCCTGCCCCTATCGAGAAAACCATCAGCAAGGCCAAGGGCCAGCCCAGAGAGCCCCAGGTGTACACC
CTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTGTCCCCTGG
CAAGACCGTGGCCGCCCCCTCGGGATCCGAAGTGCAGCTCCTGGAGAGCGGCGGCGGCCTGGTGCAGCCCG
GCGGCAGCCTGAGGCTGAGCTGCGCCGCTAGCGGCTTCACCTTCAGGAACTTCGGCATGGGCTGGGTCAGG
CAGGCCCCCGGCAAGGGCCTGGAGTGGGTCAGCTGGATCATCAGCTCCGGCACCGAGACCTACTACGCCGA
CAGCGTGAAGGGCAGGTTCACCATCAGCCGCGACAACAGCAAGAACACCCTGTACCTGCAGATGAACAGCCT
GAGGGCCGAGGACACCGCCGTCTACTACTGCGCCAAGAGCCTGGGCAGGTTCGACTACTGGGGACAGGGGA
CCCTGGTGACTGTGAGCAGC

**SEQ ID NO: 168**

GAGGTGCAGCTGGTGGAGTCTGGCGGCGGACTGGTGCAGCCCGGCAGAAGCCTGAGACTGAGCTGTGCCGC
CAGCGGCTTCACCTTCGACGACTACGCCATGCACTGGGTGAGGCAGGCCCCTGGCAAGGGCCTGGAGTGGG
TGTCCGCCATCACCTGGAATAGCGGCCACATCGACTACGCCGACAGCGTGGAGGGCAGATTCACCATCAGCC
GGGACAACGCCAAGAACAGCCTGTACCTGCAGATGAACAGCCTGAGAGCCGAGGACACCGCCGTGTACTACT
GTGCCAAGGTGTCCTACCTGAGCACCGCCAGCAGCCTGGACTACTGGGGCCAGGGCACCCTGGTGACAGTC
TCGAGCGCTAGCACCAAGGGCCCCAGCGTGTTCCCCCTGGCCCCCAGCAGCAAGAGCACCAGCGGCGGCACA

GCCGCCCTGGGCTGCCTGGTGAAGGACTACTTCCCCGAGCCTGTGACCGTGTCCTGGAATAGCGGAGCCCT
GACCTCCGGCGTGCACACCTTCCCCGCCGTGCTGCAGAGCAGCGGCCTGTACTCCCTGAGCAGCGTGGTGAC
CGTGCCCAGCAGCAGCCTGGGCACCCAGACCTACATCTGCAACGTGAACCACAAGCCCAGCAACACCAAAGT
GGACAAGAAAGTGGAGCCCAAGAGCTGCGATAAGACCCACACCTGCCCCCCCTGCCCTGCCCCCGAGCTGCT
GGGCGGACCTAGCGTGTTCCTGTTCCCCCCCAAGCCTAAGGACACCCTGATGATCAGCAGGACCCCCGAAGT
GACCTGCGTGGTGGTGGATGTGAGCCACGAGGACCCTGAAGTGAAGTTCAACTGGTACGTGGACGGCGTGG
AAGTGCACAACGCCAAGACCAAGCCCAGAGAGGAGCAGTACAACAGCACCTACCGCGTGGTGTCTGTGCTGA
CCGTGCTGCACCAGGATTGGCTGAACGGCAAGGAGTACAAGTGCAAAGTGAGCAACAAGGCCCTGCCTGCCC
CTATCGAGAAAACCATCAGCAAGGCCAAGGGCCAGCCTAGAGAGCCCCAGGTCTACACCCTGCCTCCCTCCA
GAGATGAGCTGACCAAGAACCAGGTGTCCCTGACCTGTCTGGTGAAGGGCTTCTACCCCAGCGACATCGCCG
TGGAGTGGGAGAGCAACGGCCAGCCCGAGAACAACTACAAGACCACCCCCCCTGTGCTGGACAGCGATGGC
AGCTTCTTCCTGTACTCCAAGCTGACCGTGGACAAGAGCAGATGGCAGCAGGGCAACGTGTTCAGCTGCAGC
GTGATGCACGAGGCCCTGCACAATCACTACACCCAGAAGAGTCTGAGCCTGTCCCCTGGCAAGTCGACCGGT
GAGGTGCAGCTGTTGGTGTCTGGGGGAGGCTTGGTACAGCCTGGGGGGTCCCTGCGTCTCTCCTGTGCAGC
CTCCGGATTCACCTTTAAGGCTTATCCGATGATGTGGGTCCGCCAGGCTCCAGGGAAGGGTCTAGAGTGGGT
TTCAGAGATTTCGCCTTCGGGTTCTTATACATACTACGCAGACTCCGTGAAGGGCCGGTTCACCATCTCCCG
CGACAATTCCAAGAACACGCTGTATCTGCAAATGAACAGCCTGCGTGCCGAGGACACCGCGGTATATTACTG
TGCGAAAGATCCTCGGAAGTTAGACTACTGGGGTCAGGGAACCCTGGTCACCGTCTCGAGC

**SEQ ID NO: 169**

GATATCCAGATGACCCAGAGCCCCAGCAGCCTGAGCGCCTCTGTGGGCGATAGAGTGACCATCACCTGCCGGGCCAGCCAGGGCATCA
GAAACTACCTGGCCTGGTATCAGCAGAAGCCTGGCAAGGCCCCTAAGCTGCTGATCTACGCCGCCAGCACCCTGCAGAGCGGCGTGCCC
AGCAGATTCAGCGGCAGCGGCTCCGGCACCGACTTCACCCTGACCATCAGCAGCCTGCAGCCCGAGGACGTGGCCACCTACTACTGCCA
GCGGTACAACAGAGCCCCTTACACCTTCGGCCAGGGCACCAAGGTGGAGATCAAGCGTACGGTGGCCGCCCCCAGCGTGTTCATCTTCC
CCCCCAGCGATGAGCAGCTCAAGAGCGGCACCGCCAGCGTGGTGTGTCTGCTGAACAACTTCTACCCCCGGGAGGCCAAAGTGCAGTG
GAAAGTGGACAACGCCCTGCAGAGCGGCAACAGCCAGGAGAGCGTGACCGAGCAGGACAGCAAGGACTCCACCTACAGCCTGAGCAGC
ACCCTGACCCTGAGCAAGGCCGACTACGAGAAGCACAAAGTGTACGCCTGCGAAGTGACCCACCAGGGCCTGTCCAGCCCCGTGACCAA
GAGCTTCAACCGGGGCGAGTGC

**SEQ ID NO: 170**

GAGGTGCAGCTGGTGGAGTCTGGCGGCGGACTGGTGCAGCCCGGCAGAAGCCTGAGACTGAGCTGTGCCGC CAGCGGCTTCACCTTCGACGACTACGCCATGCACTGGGTGAGGCAGGCCCCTGGCAAGGGCCTGGAGTGGG TGTCCGCCATCACCTGGAATAGCGGCCACATCGACTACGCCGACAGCGTGGAGGGCAGATTCACCATCAGCC GGGACAACGCCAAGAACAGCCTGTACCTGCAGATGAACAGCCTGAGAGCCGAGGACACCGCCGTGTACTACT GTGCCAAGGTGTCCTACCTGAGCACCGCCAGCAGCCTGGACTACTGGGGCCAGGGCACCCTGGTGACAGTC TCGAGCGCTAGCACCAAGGGCCCCAGCGTGTTCCCCCTGGCCCCCAGCAGCAAGAGCACCAGCGGCGGCACA GCCGCCCTGGGCTGCCTGGTGAAGGACTACTTCCCCGAGCCTGTGACCGTGTCCTGGAATAGCGGAGCCCT GACCTCCGGCGTGCACACCTTCCCCGCCGTGCTGCAGAGCAGCGGCCTGTACTCCCTGAGCAGCGTGGTGAC CGTGCCCAGCAGCAGCCTGGGCACCCAGACCTACATCTGCAACGTGAACCACAAGCCCAGCAACACCAAAGT GGACAAGAAAGTGGAGCCCAAGAGCTGCGATAAGACCCACACCTGCCCCCCCTGCCCTGCCCCCGAGCTGCT GGGCGGACCTAGCGTGTTCCTGTTCCCCCCCAAGCCTAAGGACACCCTGATGATCAGCAGGACCCCCGAAGT GACCTGCGTGGTGGTGGATGTGAGCCACGAGGACCCTGAAGTGAAGTTCAACTGGTACGTGGACGGCGTGG AAGTGCACAACGCCAAGACCAAGCCCAGAGAGGAGCAGTACAACAGCACCTACCGCGTGGTGTCTGTGCTGA CCGTGCTGCACCAGGATTGGCTGAACGGCAAGGAGTACAAGTGCAAAGTGAGCAACAAGGCCCTGCCTGCCC CTATCGAGAAAACCATCAGCAAGGCCAAGGGCCAGCCTAGAGAGCCCCAGGTCTACACCCTGCCTCCCTCCA GAGATGAGCTGACCAAGAACCAGGTGTCCCTGACCTGTCTGGTGAAGGGCTTCTACCCCAGCGACATCGCCG TGGAGTGGGAGAGCAACGGCCAGCCCGAGAACAACTACAAGACCACCCCCCCTGTGCTGGACAGCGATGGC AGCTTCTTCCTGTACTCCAAGCTGACCGTGGACAAGAGCAGATGGCAGCAGGGCAACGTGTTCAGCTGCAGC GTGATGCACGAGGCCCTGCACAATCACTACACCCAGAAGAGTCTGAGCCTGTCCCCTGGCAAGTCGACCGGT GACATCCAGATGACCCAGAGCCCTTCAAGCCTGAGCGCCAGCGTGGGCGACAGAGTGACCATCACCTGCCGG GCCAGCCAGTGGATCGGCAACCTGCTGGACTGGTATCAGCAGAAGCCCGGCAAGGCCCCCAAGCTGCTGATC

TACTACGCCAGCTTCCTGCAGAGCGGCGTGCCCAGCCGGTTTAGCGGCAGCGGCTACGGCACCGACTTCACC CTGACCATCAGCAGCCTGCAGCCCGAGGACTTCGCCACCTACTACTGCCAGCAGGCCAACCCTGCCCCCCTG ACCTTCGGCCAGGGTACCAAGGTGGAAATCAAACGG

**SEQ ID NO: 171**

GACATCCAGATGACCCAGTCTCCATCCTCCCTGTCTGCATCTGTAGGAGACCGTGTCACCATCACTTGCCGG GCAAGTCAGGATATTTACCTGAATTTAGACTGGTATCAGCAGAAACCAGGGAAAGCCCCTAAGCTCCTGATC AATTTTGGTTCCGAGTTGCAAAGTGGTGTCCCATCACGTTTCAGTGGCAGTGGATATGGGACAGATTTCACT CTCACCATCAGCAGTCTGCAACCTGAAGATTTCGCTACGTACTACTGTCAACCGTCTTTTTACTTCCCTTATA CGTTCGGCCAAGGGACCAAGGTGGAAATCAAACGTACGGTGGCCGCCCCCAGCGACATCCAGATGACCCAGT CTCCATCCTCCCTGTCTGCATCTGTAGGAGACCGTGTCACCATCACTTGCCGGGCAAGTCAGAGCATTAGCA GCTATTTAAATTGGTACCAGCAGAAACCAGGGAAAGCCCCTAAGCTCCTGATCTATGCTGCATCCAGTTTGCA AAGTGGGGTCCCATCACGTTTCAGTGGCAGTGGATCTGGGACAGATTTCACTCTCACCATCAGCAGTCTGCA ACCTGAAGATTTTGCTACGTACTACTGTCAACAGAGTTACAGTACCCCTAATACGTTCGGCCAAGGGACCAAG GTGGAAATCAAACGTACGGTGGCCGCCCCCAGCGTGTTCATCTTCCCCCCCAGCGATGAGCAGCTCAAGAGC GGCACCGCCAGCGTGGTGTGTCTGCTGAACAACTTCTACCCCCGGGAGGCCAAAGTGCAGTGGAAAGTGGA CAACGCCCTGCAGAGCGGCAACAGCCAGGAGAGCGTGACCGAGCAGGACAGCAAGGACTCCACCTACAGCC TGAGCAGCACCCTGACCCTGAGCAAGGCCGACTACGAGAAGCACAAAGTGTACGCCTGCGAAGTGACCCACC AGGGCCTGTCCAGCCCCGTGACCAAGAGCTTCAACCGGGGCGAGTGC

**SEQ ID NO: 172**

GAGGTGCAGCTGTTGGTGTCTGGGGGAGGCTTGGTACAGCCTGGGGGGTCCCTGCGTCTCTCCTGTGCAGC
CTCCGGATTCACCTTTAAGGCTTATCCGATGATGTGGGTCCGCCAGGCTCCAGGGAAGGGTCTAGAGTGGGT
TTCAGAGATTTCGCCTTCGGGTTCTTATACATACTACGCAGACTCCGTGAAGGGCCGGTTCACCATCTCCCG
CGACAATTCCAAGAACACGCTGTATCTGCAAATGAACAGCCTGCGTGCCGAGGACACCGCGGTATATTACTG
TGCGAAAGATCCTCGGAAGTTAGACTACTGGGGTCAGGGAACCCTGGTCACCGTCTCGAGCGCTAGCACCAA
GGGCCCCAGCGAGGTGCAGCTGTTGGAGTCTGGGGGAGGCTTGGTACAGCCTGGGGGGTCCCTGCGTCTCT
CCTGTGCAGCCTCCGGATTCACCTTTAGCAGCTATGCCATGAGCTGGGTCCGCCAGGCTCCAGGGAAGGGTC
TAGAGTGGGTCTCAGCTATTAGTGGTAGTGGTGGTAGCACATACTACGCAGACTCCGTGAAGGGCCGGTTCA
CCATCTCCCGCGACAATTCCAAGAACACGCTGTATCTGCAAATGAACAGCCTGCGTGCCGAGGACACCGCGG
TATATTACTGTGCGAAAAGTTATGGTGCTTTTGACTACTGGGGCCAGGGAACCCTGGTCACCGTCTCGAGCG
CTAGCACCAAGGGCCCCAGCGTGTTCCCCCTGGCCCCCAGCAGCAAGAGCACCAGCGGCGGCACAGCCGCCC
TGGGCTGCCTGGTGAAGGACTACTTCCCCGAGCCTGTGACCGTGTCCTGGAATAGCGGAGCCCTGACCTCCG
GCGTGCACACCTTCCCCGCCGTGCTGCAGAGCAGCGGCCTGTACTCCCTGAGCAGCGTGGTGACCGTGCCCA
GCAGCAGCCTGGGCACCCAGACCTACATCTGCAACGTGAACCACAAGCCCAGCAACACCAAAGTGGACAAGA
AAGTGGAGCCCAAGAGCTGCGATAAGACCCACACCTGCCCCCCCTGCCCTGCCCCCGAGCTGCTGGGCGGAC
CTAGCGTGTTCCTGTTCCCCCCCAAGCCTAAGGACACCCTGATGATCAGCAGGACCCCCGAAGTGACCTGCG
TGGTGGTGGATGTGAGCCACGAGGACCCTGAAGTGAAGTTCAACTGGTACGTGGACGGCGTGGAAGTGCAC
AACGCCAAGACCAAGCCCAGAGAGGAGCAGTACAACAGCACCTACCGCGTGGTGTCTGTGCTGACCGTGCTG
CACCAGGATTGGCTGAACGGCAAGGAGTACAAGTGCAAAGTGAGCAACAAGGCCCTGCCTGCCCCTATCGAG
AAAACCATCAGCAAGGCCAAGGGCCAGCCTAGAGAGCCCCAGGTCTACACCCTGCCTCCCTCCAGAGATGAG
CTGACCAAGAACCAGGTGTCCCTGACCTGTCTGGTGAAGGGCTTCTACCCCAGCGACATCGCCGTGGAGTGG
GAGAGCAACGGCCAGCCCGAGAACAACTACAAGACCACCCCCCCTGTGCTGGACAGCGATGGCAGCTTCTTC
CTGTACTCCAAGCTGACCGTGGACAAGAGCAGATGGCAGCAGGGCAACGTGTTCAGCTGCAGCGTGATGCA
CGAGGCCCTGCACAATCACTACACCCAGAAGAGTCTGAGCCTGTCCCCTGGCAAG

**SEQ ID NO: 173**

CAGGTGCAGCTGAAGCAGAGCGGCCCTGGCCTGGTGCAGCCCTCTCAGAGCCTGAGCATCACCTGTACCGT
GAGCGGCTTCAGCCTGACCAATTACGGCGTGCATTGGGTGCGGCAGTCTCCAGGCAAGGGCCTGGAATGGC
TGGGAGTGATCTGGTCCGGCGGCAACACCGACTACAACACCCCCTTCACCAGCAGACTGAGCATCAACAAGG
ACAACAGCAAGAGCCAGGTGTTCTTCAAGATGAACAGCCTGCAGAGCAACGACACCGCCATCTACTATTGTG
CCAGGGCCCTGACCTACTACGACTACGAGTTCGCCTACTGGGGCCAGGGCACCCTGGTGACCGTGAGCGCC

GCTAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAGCCTGTGACCGTGTCCTGGAATAGCGGAGCCCTGACCTC
CGGCGTGCACACCTTCCCCGCCGTGCTGCAGAGCAGCGGCCTGTACTCCCTGAGCAGCGTGGTGACCGTGC
CCAGCAGCAGCCTGGGCACCCAGACCTACATCTGCAACGTGAACCACAAGCCCAGCAACACCAAAGTGGACA
AGAAAGTGGAGCCCAAGAGCTGCGATAAGACCCACACCTGCCCCCCCTGCCCTGCCCCCGAGCTGCTGGGCG
GACCTAGCGTGTTCCTGTTCCCCCCCAAGCCTAAGGACACCCTGATGATCAGCAGGACCCCCGAAGTGACCT
GCGTGGTGGTGGATGTGAGCCACGAGGACCCTGAAGTGAAGTTCAACTGGTACGTGGACGGCGTGGAAGTG
CACAACGCCAAGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCCTGCCCCTATC
GAGAAAACCATCAGCAAGGCCAAGGGCCAGCCTAGAGAGCCCCAGGTCTACACCCTGCCTCCCTCCAGAGAT
GAGCTGACCAAGAACCAGGTGTCCCTGACCTGTCTGGTGAAGGGCTTCTACCCCAGCGACATCGCCGTGGAG
TGGGAGAGCAACGGCCAGCCCGAGAACAACTACAAGACCACCCCCCCTGTGCTGGACAGCGATGGCAGCTTC
TTCCTGTACTCCAAGCTGACCGTGGACAAGAGCAGATGGCAGCAGGGCAACGTGTTCAGCTGCAGCGTGATG
CACGAGGCCCTGCACAATCACTACACCCAGAAGAGTCTGAGCCTGTCCCCTGGCAAGGAGGTGCAGCTGTTG
GTGTCTGGGGGAGGCTTGGTACAGCCTGGGGGGTCCCTGCGTCTCTCCTGTGCAGCCTCCGGATTCACCTT
TAAGGCTTATCCGATGATGTGGGTCCGCCAGGCTCCAGGGAAGGGTCTAGAGTGGGTTTCAGAGATTTCGC
CTTCGGGTTCTTATACATACTACGCAGACTCCGTGAAGGGCCGGTTCACCATCTCCCGCGACAATTCCAAGA
ACACGCTGTATCTGCAAATGAACAGCCTGCGTGCCGAGGACACCGCGGTATATTACTGTGCGAAAGATCCTC
GGAAGTTAGACTACTGGGGTCAGGGAACCCTGGTCACCGTCTCGAGC

**SEQ ID NO: 174**

QVQLKQSGPGLVQPSQSLSITCTVSGFSLTNYGVHWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLSLSPGKEVQLLVSGGGL
VQPGGSLRLSCAASGFTFKAYPMMWVRQAPGKGLEWVSEISPSGSYTYYADSVKGRFTISRDNSKNTLYLQMNSLRAEDTAVYYCAKDPRK
LDYWGQGTLVTVSS

**SEQ ID NO: 175**

CAGGTGCAGCTGAAGCAGAGCGGCCCTGGCCTGGTGCAGCCCTCTCAGAGCCTGAGCATCACCTGTACCGTGAGCGGCTTCAGCCTGA
CCAATTACGGCGTGCATTGGGTGCGGCAGTCTCCAGGCAAGGGCCTGGAATGGCTGGGAGTGATCTGGTCCGGCGGCAACACCGACTA
CAACACCCCCTTCACCAGCAGACTGAGCATCAACAAGGACAACAGCAAGAGCCAGGTGTTCTTCAAGATGAACAGCCTGCAGAGCAACG
ACACCGCCATCTACTATTGTGCCAGGGCCCTGACCTACTACGACTACGAGTTCGCCTACTGGGGCCAGGGCACCCTGGTGACCGTGAGC
GCCGCTAGCACCAAGGGCCCCAGCGTGTTCCCCCTGGCCCCCAGCAGCAAGAGCACCAGCGGCGGCACAGCCGCCCTGGGCTGCCTGG
TGAAGGACTACTTCCCCGAGCCTGTGACCGTGTCCTGGAATAGCGGAGCCCTGACCTCCGGCGTGCACACCTTCCCCGCCGTGCTGCAG
AGCAGCGGCCTGTACTCCCTGAGCAGCGTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCTGCCCCCCCTGCCCTGCCCCCGAGCTGC
TGGGCGGACCTAGCGTGTTCCTGTTCCCCCCCAAGCCTAAGGACACCCTGATGATCAGCAGGACCCCCGAAGTGACCTGCGTGGTGGTG
GATGTGAGCCACGAGGACCCTGAAGTGAAGTTCAACTGGTACGTGGACGGCGTGGAAGTGCACAACGCCAAGACCAAGCCCAGAGAGG
AGCAGTACAACAGCACCTACCGCGTGGTGTCTGTGCTGACCGTGCTGCACCAGGATTGGCTGAACGGCAAGGAGTACAAGTGCAAAGT
GAGCAACAAGGCCCTGCCTGCCCCTATCGAGAAAACCATCAGCAAGGCCAAGGGCCAGCCTAGAGAGCCCCAGGTCTACACCCTGCCTC
CCTCCAGAGATGAGCTGACCAAGAACCAGGTGTCCCTGACCTGTCTGGTGAAGGGCTTCTACCCCAGCGACATCGCCGTGGAGTGGGA
GAGCAACGGCCAGCCCGAGAACAACTACAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTGCACAATCACTACACCCAGAAGAGTCTG
AGCCTGTCCCCTGGCAAGTCGACCGGTGGTGGAGGTGGATCAGAGGTGCAGCTGTTGGTGTCTGGGGGAGGCTTGGTACAGCCTGGG
GGGTCCCTGCGTCTCTCCTGTGCAGCCTCCGGATTCACCTTTAAGGCTTATCCGATGATGTGGGTCCGCCAGGCTCCAGGGAAGGGTCT
AGAGTGGGTTTCAGAGATTTCGCCTTCGGGTTCTTATACATACTACGCAGACTCCGTGAAGGGCCGGTTCACCATCTCCCGCGACAATT
CCAAGAACACGCTGTATCTGCAAATGAACAGCCTGCGTGCCGAGGACACCGCGGTATATTACTGTGCGAAAGATCCTCGGAAGTTAGAC
TACTGGGGTCAGGGAACCCTGGTCACCGTCTCGAGC

**SEQ ID NO: 176**

QVQLKQSGPGLVQPSQSLSITCTVSGFSLTNYGVHWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLSLSPGKSTGGGGSEVQ
LLVSGGGLVQPGGSLRLSCAASGFTFKAYPMMWVRQAPGKGLEWVSEISPSGSYTYYADSVKGRFTISRDNSKNTLYLQMNSLRAEDTAVY
YCAKDPRKLDYWGQGTLVTVSS

**SEQ ID NO: 177**

CAGGTGCAGCTGAAGCAGAGCGGCCCTGGCCTGGTGCAGCCCTCTCAGAGCCTGAGCATCACCTGTACCGTGAGCGGCTTCAGCCTGA
CCAATTACGGCGTGCATTGGGTGCGGCAGTCTCCAGGCAAGGGCCTGGAATGGCTGGGAGTGATCTGGTCCGGCGGCAACACCGACTA
CAACACCCCCTTCACCAGCAGACTGAGCATCAACAAGGACAACAGCAAGAGCCAGGTGTTCTTCAAGATGAACAGCCTGCAGAGCAACG
ACACCGCCATCTACTATTGTGCCAGGGCCCTGACCTACTACGACTACGAGTTCGCCTACTGGGGCCAGGGCACCCTGGTGACCGTGAGC
GCCGCTAGCACCAAGGGCCCCAGCGTGTTCCCCCTGGCCCCCAGCAGCAAGAGCACCAGCGGCGGCACAGCCGCCCTGGGCTGCCTGG
TGAAGGACTACTTCCCCGAGCCTGTGACCGTGTCCTGGAATAGCGGAGCCCTGACCTCCGGCGTGCACACCTTCCCCGCCGTGCTGCAG
AGCAGCGGCCTGTACTCCCTGAGCAGCGTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCTGCCCCCCCTGCCCTGCCCCCGAGCTGC
TGGGCGGACCTAGCGTGTTCCTGTTCCCCCCCAAGCCTAAGGACACCCTGATGATCAGCAGGACCCCCGAAGTGACCTGCGTGGTGGTG
GATGTGAGCCACGAGGACCCTGAAGTGAAGTTCAACTGGTACGTGGACGGCGTGGAAGTGCACAACGCCAAGACCAAGCCCAGAGAGG
AGCAGTACAACAGCACCTACCGCGTGGTGTCTGTGCTGACCGTGCTGCACCAGGATTGGCTGAACGGCAAGGAGTACAAGTGCAAAGT
GAGCAACAAGGCCCTGCCTGCCCCTATCGAGAAAACCATCAGCAAGGCCAAGGGCCAGCCTAGAGAGCCCCAGGTCTACACCCTGCCTC
CCTCCAGAGATGAGCTGACCAAGAACCAGGTGTCCCTGACCTGTCTGGTGAAGGGCTTCTACCCCAGCGACATCGCCGTGGAGTGGGA
GAGCAACGGCCAGCCCGAGAACAACTACAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTGCACAATCACTACACCCAGAAGAGTCTG
AGCCTGTCCCCTGGCAAGTCGACCGGTGGTGGAGGTGGATCAGGTGGAGGTGGATCAGAGGTGCAGCTGTTGGTGTCTGGGGGAGGC
TTGGTACAGCCTGGGGGGTCCCTGCGTCTCTCCTGTGCAGCCTCCGGATTCACCTTTAAGGCTTATCCGATGATGTGGGTCCGCCAGGC
TCCAGGGAAGGGTCTAGAGTGGGTTTCAGAGATTTCGCCTTCGGGTTCTTATACATACTACGCAGACTCCGTGAAGGGCCGGTTCACCA
TCTCCCGCGACAATTCCAAGAACACGCTGTATCTGCAAATGAACAGCCTGCGTGCCGAGGACACCGCGGTATATTACTGTGCGAAAGAT
CCTCGGAAGTTAGACTACTGGGGTCAGGGAACCCTGGTCACCGTCTCGAGC

**SEQ ID NO: 178**

QVQLKQSGPGLVQPSQSLSITCTVSGFSLTNYGVHWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLSLSPGKSTGGGGGSGG
GGSEVQLLVSGGGLVQPGGSLRLSCAASGFTFKAYPMMWVRQAPGKGLEWVSEISPSGSYTYYADSVKGRFTISRDNSKNTLYLQMNSLRA
EDTAVYYCAKDPRKLDYWGQGTLVTVSS

**SEQ ID NO: 179**

GAGGTGCAGCTGGTCGAGTCTGGCGGCGGACTGGTGCAGCCTGGCGGCAGCCTGAGACTGAGCTGCGCCGC
CAGCGGCTACACCTTCACCAACTACGGCATGAACTGGGTGCGGCAGGCCCCTGGCAAGGGCCTGGAATGGG
TGGGCTGGATCAACACCTACACCGGCGAGCCCACCTACGCCGCCGACTTCAAGCGGCGGTTCACCTTCAGCC
TGGACACCAGCAAGAGCACCGCCTACCTGCAGATGAACAGCCTGCGGGCCGAGGACACCGCCGTGTACTACT
GCGCCAAGTACCCCCACTACTACGGCAGCAGCCACTGGTACTTCGACTACTGGGGGCAGGGTACCCTGGTCA
CCGTCTCGAGCGCTAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAGCCTGTGACCGTGTCCTGGAATAGCGGA
GCCCTGACCTCCGGCGTGCACACCTTCCCCGCCGTGCTGCAGAGCAGCGGCCTGTACTCCCTGAGCAGCGTG
GTGACCGTGCCCAGCAGCAGCCTGGGCACCCAGACCTACATCTGCAACGTGAACCACAAGCCCAGCAACACC
AAAGTGGACAAGAAAGTGGAGCCCAAGAGCTGCGATAAGACCCACACCTGCCCCCCCTGCCCTGCCCCCGAG
CTGCTGGGCGGACCTAGCGTGTTCCTGTTCCCCCCCAAGCCTAAGGACACCCTGATGATCAGCAGGACCCCC
GAAGTGACCTGCGTGGTGGTGGATGTGAGCCACGAGGACCCTGAAGTGAAGTTCAACTGGTACGTGGACGG

CGTGGAAGTGCACAACGCCAAGACCAAGCCCAGAGAGGAGCAGTACAACAGCACCTACCGCGTGGTGTCTGTGCTGACCGTGCTGCACCAGGATTGGCTGAACGGCAAGGAGTACAAGTGCAAAGTGAGCAACAAGGCCCTGCCTGCCCCTATCGAGAAAACCATCAGCAAGGCCAAGGGCCAGCCTAGAGAGCCCCAGGTCTACACCCTGCCTCCCTCCAGAGATGAGCTGACCAAGAACCAGGTGTCCCTGACCTGTCTGGTGAAGGGCTTCTACCCCAGCGACATCGCCGTGGAGTGGGAGAGCAACGGCCAGCCCGAGAACAACTACAAGACCACCCCCCCTGTGCTGGACAGCGATGGCAGCTTCTTCCTGTACTCCAAGCTGACCGTGGACAAGAGCAGATGGCAGCAGGGCAACGTGTTCAGCTGCAGCGTGATGCACGAGGCCCTGCACAATCACTACACCCAGAAGAGTCTGAGCCTGTCCCCTGGCAAGTCGACCGGTGACATCCAGATGACCCAGAGCCCTTCAAGCCTGAGCGCCAGCGTGGGCGACAGAGTGACCATCACCTGCCGGGCCAGCCAGTGGATCGGCAACCTGCTGGACTGGTATCAGCAGAAGCCCGGCAAGGCCCCCAAGCTGCTGATCTACTACGCCAGCTTCCTGCAGAGCGGCGTGCCCAGCCGGTTTAGCGGCAGCGGCTACGGCACCGACTTCACCCTGACCATCAGCAGCCTGCAGCCCGAGGACTTCGCCACCTACTACTGCCAGCAGGCCAACCCTGCCCCCCTGACCTTCGGCCAGGGTACCAAGGTGGAAATCAAACGG

**SEQ ID NO: 180**

EVQLVESGGGLVQPGGSLRLSCAASGYTFTNYGMNWVRQAPGKGLEWVGWINTYTGEPTYAADFKRRFTFSLDTSKSTAYLQMNSLRAEDTAVYYCAKYPHYYGSSHWYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKSTGDIQMTQSPSSLSASVGDRVTITCRASQWIGNLLDWYQQKPGKAPKLLIYYASFLQSGVPSRFSGSGYGTDFTLTISSLQPEDFATYYCQQANPAPLTFGQGTKVEIKR

**SEQ ID NO: 181**

GACATCCAGATGACCCAGAGCCCCAGCAGCCTGAGCGCCAGCGTGGGCGACAGAGTGACCATCACCTGCAGCGCCAGCCAGGACATCAGCAACTACCTGAACTGGTATCAGCAGAAGCCCGGCAAGGCCCCCAAGGTGCTGATCTACTTCACCAGCTCCCTGCACAGCGGCGTGCCCAGCCGGTTTAGCGGCAGCGGCTCCGGCACCGACTTCACCCTGACCATCAGCAGCCTGCAGCCCGAGGACTTCGCCACCTACTACTGCCAGCAGTACAGCACCGTGCCCTGGACCTTCGGCCAGGGTACCAAGGTGGAGATCAAGCGTACGGTGGCCGCTCCCAGCGTGTTCATCTTCCCCCCCAGCGACGAGCAGCTGAAGAGCGGCACCGCCTCCGTGGTGTGCCTGCTGAACAACTTCTACCCCCGGGAGGCCAAGGTGCAGTGGAAGGTGGACAACGCCCTGCAGTCCGGCAACAGCCAGGAAAGCGTCACCGAGCAGGACTCCAAGGACTCCACCTACAGCCTGAGCAGCACCCTGACCCTGAGCAAGGCCGACTACGAGAAGCACAAGGTGTACGCCTGCGAAGTGACCCACCAGGGCCTGTCCAGCCCCGTGACCAAGAGCTTCAACCGGGGCGAGTGC

**SEQ ID NO: 182**

DIQMTQSPSSLSASVGDRVTITCSASQDISNYLNWYQQKPGKAPKVLIYFTSSLHSGVPSRFSGSGSGTDFTLTISSLQPEDFATYYCQQYSTVPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

**SEQ ID NO: 183**

CAGGTGCAGCTGAAGCAGAGCGGCCCTGGCCTGGTGCAGCCCTCTCAGAGCCTGAGCATCACCTGTACCGT
GAGCGGCTTCAGCCTGACCAATTACGGCGTGCATTGGGTGCGGCAGTCTCCAGGCAAGGGCCTGGAATGGC
TGGGAGTGATCTGGTCCGGCGGCAACACCGACTACAACACCCCCTTCACCAGCAGACTGAGCATCAACAAGG
ACAACAGCAAGAGCCAGGTGTTCTTCAAGATGAACAGCCTGCAGAGCAACGACACCGCCATCTACTATTGTG
CCAGGGCCCTGACCTACTACGACTACGAGTTCGCCTACTGGGGCCAGGGCACCCTGGTGACCGTGAGCGCC
GCTAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAGCCTGTGACCGTGTCCTGGAATAGCGGAGCCCTGACCTC
CGGCGTGCACACCTTCCCCGCCGTGCTGCAGAGCAGCGGCCTGTACTCCCTGAGCAGCGTGGTGACCGTGC
CCAGCAGCAGCCTGGGCACCCAGACCTACATCTGCAACGTGAACCACAAGCCCAGCAACACCAAAGTGGACA
AGAAAGTGGAGCCCAAGAGCTGCGATAAGACCCACACCTGCCCCCCCTGCCCTGCCCCCGAGCTGCTGGGCG
GACCTAGCGTGTTCCTGTTCCCCCCCAAGCCTAAGGACACCCTGATGATCAGCAGGACCCCCGAAGTGACCT
GCGTGGTGGTGGATGTGAGCCACGAGGACCCTGAAGTGAAGTTCAACTGGTACGTGGACGGCGTGGAAGTG
CACAACGCCAAGACCAAGCCCAGAGAGGAGCAGTACAACAGCACCTACCGCGTGGTGTCTGTGCTGACCGTG

CTGCACCAGGATTGGCTGAACGGCAAGGAGTACAAGTGCAAAGTGAGCAACAAGGCCCTGCCTGCCCCTATC
GAGAAAACCATCAGCAAGGCCAAGGGCCAGCCTAGAGAGCCCCAGGTCTACACCCTGCCTCCCTCCAGAGAT
GAGCTGACCAAGAACCAGGTGTCCCTGACCTGTCTGGTGAAGGGCTTCTACCCCAGCGACATCGCCGTGGAG
TGGGAGAGCAACGGCCAGCCCGAGAACAACTACAAGACCACCCCCCCTGTGCTGGACAGCGATGGCAGCTTC
TTCCTGTACTCCAAGCTGACCGTGGACAAGAGCAGATGGCAGCAGGGCAACGTGTTCAGCTGCAGCGTGATG
CACGAGGCCCTGCACAATCACTACACCCAGAAGAGTCTGAGCCTGTCCCCTGGCAAGTCGACCGGTGGGGTG
CAGCTGTTGGAGTCTGGGGGAGGCTTGGTACAGCCTGGGGGGTCCCTGCGTCTCTCCTGTGCAGCCTCCGG
ATTCACCTTCGCTTGGTATGATATGGGGTGGGTCCGCCAGGCTCCAGGGAAGGGTCTAGAGTGGGTCTCAA
GTATTGATTGGCATGGTGAGGTTACATACTACGCAGACTCCGTGAAGGGCCGGTTCACCATCTCCCGCGACA
ATTCCAAGAACACGCTGTATCTGCAAATGAACAGCCTGCGTGCCGAGGACACCGCGGTATATTACTGTGCGA
CAGCGGAGGACGAGCCGGGGTATGACTACTGGGGCCAGGGAACCCTGGTCACCGTCTCGAGC

**SEQ ID NO:184**

QVQLKQSGPGLVQPSQSLSITCTVSGFSLTNYGVHWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLSLSPGKSTGGVQLLESG
GGLVQPGGSLRLSCAASGFTFAWYDMGWVRQAPGKGLEWVSSIDWHGEVTYYADSVKGRFTISRDNSKNTLYLQMNSLRAEDTAVYYCAT
AEDEPGYDYWGQGTLVTVSS

**SEQ ID NO: 185**

EVQLVESGGGLVQPGRSLRLSCAASGFTFDDYAMHWVRQAPGKGLEWVSAITWNSGHIDYADSVEGRFTISRDNAKNSLYLQMNSLRAED
TAVYYCAKVSYLSTASSLDYWGQGTLVTVSSASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQKSLSLSPGKSTGDIQMTQ
SPSSLSASVGDRVTITCRASQWIGPELRWYQQKPGKAPKLLIYHTSILQSGVPSRFSGSGSGTDFTLTISSLQPEDFATYYCQQYMFQPMTFG
QGTKVEIKR

**SEQ ID NO: 186**

QVQLKQSGPGLVQPSQSLSITCTVSGFSLTNYGVHWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLSLSPGKSTGDIQMTQSP
SSLSASVGDRVTITCRASQWIGPELRWYQQKPGKAPKLLIYHTSILQSGVPSRFSGSGSGTDFTLTISSLQPEDFATYYCQQYMFQPMTFGQ
GTKVEIKR

**SEQ ID NO: 187**

GACATCCTGCTGACCCAGAGCCCCGTGATCCTGAGCGTGAGCCCTGGCGAGAGAGTGAGCTTCAGCTGCCGGGCCAGCCAGAGCATCG
GCACCAACATCCACTGGTATCAGCAGCGGACCAACGGCAGCCCCAGGCTGCTGATCAAGTACGCCAGCGAGTCCATCAGCGGCATCCCC
AGCCGGTTCAGCGGCAGCGGCTCCGGCACCGACTTCACCCTGAGCATCAACAGCGTGGAGAGCGAGGATATCGCCGACTACTACTGCCA
GCAGAACAACAACTGGCCCACCACCTTCGGAGCCGGCACCAAGCTGGAACTGAAGCGTACGGTGGCCGCCCCCAGCGTGTTCATCTTCC
CCCCCAGCGATGAGCAGCTCAAGAGCGGCACCGCCAGCGTGGTGTGTCTGCTGAACAACTTCTACCCCCGGGAGGCCAAAGTGCAGTG
GAAAGTGGACAACGCCCTGCAGAGCGGCAACAGCCAGGAGAGCGTGACCGAGCAGGACAGCAAGGACTCCACCTACAGCCTGAGCAGC
ACCCTGACCCTGAGCAAGGCCGACTACGAGAAGCACAAAGTGTACGCCTGCGAAGTGACCCACCAGGGCCTGTCCAGCCCCGTGACCAA
GAGCTTCAACCGGGGCGAGTGCGGATCCACCGGCGAGGTGCAGCTGTTGGTGTCTGGGGGAGGCTTGGTACAGCCTGGGGGGTCCCT
GCGTCTCTCCTGTGCAGCCTCCGGATTCACCTTTAAGGCTTATCCGATGATGTGGGTCCGCCAGGCTCCAGGGAAGGGTCTAGAGTGGG
TTTCAGAGATTTCGCCTTCGGGTTCTTATACATACTACGCAGACTCCGTGAAGGGCCGGTTCACCATCTCCCGCGACAATTCCAAGAACA
CGCTGTATCTGCAAATGAACAGCCTGCGTGCCGAGGACACCGCGGTATATTACTGTGCGAAAGATCCTCGGAAGTTAGACTACTGGGGT
CAGGGAACCCTGGTCACCGTCTCGAGC

**SEQ ID NO: 188**

DILLTQSPVILSVSPGERVSFSCRASQSIGTNIHWYQQRTNGSPRLLIKYASESISGIPSRFSGSGSGTDFTLSINSVESEDIADYYCQQNNNW
PTTFGAGTKLELKRTVAAPSVFIFPPSDEQLKSGTASVVCLLNNFYPREAKVQWKVDNALQSGNSQESVTEQDSKDSTYSLSSTLTLSKADYE
KHKVYACEVTHQGLSSPVTKSFNRGECGSTGEVQLLVSGGGLVQPGGSLRLSCAASGFTFKAYPMMWVRQAPGKGLEWVSEISPSGSYTYY
ADSVKGRFTISRDNSKNTLYLQMNSLRAEDTAVYYCAKDPRKLDYWGQGTLVTVSS

**SEQ ID NO: 189**

GACATCCTGCTGACCCAGAGCCCCGTGATCCTGAGCGTGAGCCCTGGCGAGAGAGTGAGCTTCAGCTGCCGGGCCAGCCAGAGCATCG
GCACCAACATCCACTGGTATCAGCAGCGGACCAACGGCAGCCCCAGGCTGCTGATCAAGTACGCCAGCGAGTCCATCAGCGGCATCCCC
AGCCGGTTCAGCGGCAGCGGCTCCGGCACCGACTTCACCCTGAGCATCAACAGCGTGGAGAGCGAGGATATCGCCGACTACTACTGCCA
GCAGAACAACAACTGGCCCACCACCTTCGGAGCCGGCACCAAGCTGGAACTGAAGCGTACGGTGGCCGCCCCCAGCGTGTTCATCTTCC
CCCCCAGCGATGAGCAGCTCAAGAGCGGCACCGCCAGCGTGGTGTGTCTGCTGAACAACTTCTACCCCCGGGAGGCCAAAGTGCAGTG
GAAAGTGGACAACGCCCTGCAGAGCGGCAACAGCCAGGAGAGCGTGACCGAGCAGGACAGCAAGGACTCCACCTACAGCCTGAGCAGC
ACCCTGACCCTGAGCAAGGCCGACTACGAGAAGCACAAAGTGTACGCCTGCGAAGTGACCCACCAGGGCCTGTCCAGCCCCGTGACCAA
GAGCTTCAACCGGGGCGAGTGCGGATCCACGGTGGCCGCCCCCAGCGAGGTGCAGCTGTTGGTGTCTGGGGGAGGCTTGGTACAGCCT
GGGGGGTCCCTGCGTCTCTCCTGTGCAGCCTCCGGATTCACCTTTAAGGCTTATCCGATGATGTGGGTCCGCCAGGCTCCAGGGAAGG
GTCTAGAGTGGGTTTCAGAGATTTCGCCTTCGGGTTCTTATACATACTACGCAGACTCCGTGAAGGGCCGGTTCACCATCTCCCGCGAC
AATTCCAAGAACACGCTGTATCTGCAAATGAACAGCCTGCGTGCCGAGGACACCGCGGTATATTACTGTGCGAAAGATCCTCGGAAGTT
AGACTACTGGGGTCAGGGAACCCTGGTCACCGTCTCGAGC

**SEQ ID NO: 190**

DILLTQSPVILSVSPGERVSFSCRASQSIGTNIHWYQQRTNGSPRLLIKYASESISGIPSRFSGSGSGTDFTLSINSVESEDIADYYCQQNNNW
PTTFGAGTKLELKRTVAAPSVFIFPPSDEQLKSGTASVVCLLNNFYPREAKVQWKVDNALQSGNSQESVTEQDSKDSTYSLSSTLTLSKADYE
KHKVYACEVTHQGLSSPVTKSFNRGECGSTVAAPSEVQLLVSGGGLVQPGGSLRLSCAASGFTFKAYPMMWVRQAPGKGLEWVSEISPSGS
YTYYADSVKGRFTISRDNSKNTLYLQMNSLRAEDTAVYYCAKDPRKLDYWGQGTLVTVSS

**SEQ ID NO: 191**

QVTLRESGPALVKPTQTLTLTCTVSGFSLTSYSVHWVRQPPGKGLEWLGVIWASGGTDYNSALMSRLSISKDTS
RNQVVLTMTNMDPVDTATYYCARDPPSSLLRLDYWGRGTPVTVSSASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEALHNHYTQKSLSLSP
GK

**SEQ ID NO: 192:** 586H-ASTKG-210 Heavy chain

CAGGTGCAGCTCGTGCAGAGCGGCGCCGAAGTGAAAAAGCCCGGCAGCAGCGTGAAGGTGAGCTGCAAGGC
CTCCGGCTTCTACATCAAGGACACCTACATGCACTGGGTCAGGCAGGCTCCTGGCCAGGGCCTGGAGTGGAT
GGGCACTATCGACCCCGCCAACGGCAACACCAAGTACGTGCCCAAGTTCCAGGGCAGGGTGACCATCACCGC
CGATGAGAGCACCAGCACCGCCTACATGGAACTGAGCAGCCTGAGGTCTGAGGACACCGCCGTGTACTATTG
CGCCAGGAGCATCTACGACGACTACCACTACGACGACTACTACGCCATGGACTACTGGGGACAGGGCACACT
AGTGACCGTGTCCAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTACAGCCTGAGC
AGCGTGGTGACCGTGCCCAGCAGCAGCCTGGGCACCCAGACCTACATCTGTAACGTGAACCACAAGCCCAGC
AACACCAAGGTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATGATCAGCAGA
ACCCCCGAGGTGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCACCTACCGGGTGG
TGTCCGTGCTGACCGTGCTGCACCAGGATTGGCTGAACGGCAAGGAGTACAAGTGTAAGGTGTCCAACAAG
GCCCTGCCTGCCCCTATCGAGAAAACCATCAGCAAGGCCAAGGGCCAGCCCAGAGAGCCCCAGGTGTACACC
CTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAGGGCAACGT

GTTCAGCTGCTCCGTGATGCACGAGGCCCTGCACAATCACTACACCCAGAAGAGCCTGAGCCTGTCCCCTGG
CAAGGGATCAGCCAGCACCAAGGGCCCCACGGGATCCGAAGTGCAGCTCCTGGAGAGCGGCGGCGGCCTGG
TGCAGCCCGGCGGCAGCCTGAGGCTGAGCTGCGCCGCTAGCGGCTTCACCTTCAGGAACTTCGGCATGGGC
TGGGTCAGGCAGGCCCCCGGCAAGGGCCTGGAGTGGGTCAGCTGGATCATCAGCTCCGGCACCGAGACCTA
CTACGCCGACAGCGTGAAGGGCAGGTTCACCATCAGCCGCGACAACAGCAAGAACACCCTGTACCTGCAGAT
GAACAGCCTGAGGGCCGAGGACACCGCCGTCTACTACTGCGCCAAGAGCCTGGGCAGGTTCGACTACTGGG
GACAGGGGACCCTGGTGACTGTGAGCAGC

**SEQ ID NO: 193**

EVTLRESGPALVKPTQTLTLTCTFSGFSLSKSVMGVSWIRQPPGKALEWLAHIYWDDDKYYNPSLKSRLTISKDT
SKNQVVLTMTNMDPVDTATYYCARRGIRSAMDYWGQGTTVTVSSASTKGPEVQLVQSGTEVKKPGESLKISCK
GSGYTVTSYWIGWVRQMPGKGLEWMGFIYPGDSETRYSPTFQGQVTISADKSFNTAFLQWSSLKASDTAMYY
CARVGSGWYPYTFDIWGQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGL
VQPGGSLRLSCAASGFTFRNFGMGWVRQAPGKGLEWVSWIISSGTETYYADSVKGRFTISRDNSKNTLYLQMN
SLRAEDTAVYYCAKSLGRFDYWGQGTLVTVSS

**SEQ ID NO: 194**

DIVMTQSPDSLAVSLGERATINCKASQSVSNDVAWYQQKPGQPPKLLIYYASNRYTGVPDRFSGSGSGTDFTLTI
SSLQAEDVAVYYCQQDYNSPWTFGGGTKVEIKRTVAAPEIVMTQSPATLSVSPGERATLSCRASESASSNLAWY
QQKPGQAPRLFIYTASTRATDIPARFSGSGSGTEFTLTISSLQSEDFAVYYCQQYNNWPSITFGQGTRLEIKRTV
AAPSVFIFPPSDEQLKSGTASVVCLLNNFYPREAKVQWKVDNALQSGNSQESVTEQDSKDSTYSLSSTLTLSKAD
YEKHKVYACEVTHQGLSSPVTKSFNRGEC

**SEQ ID NO: 195: Anti IL-5 Heavy Chain-G4S-dAb474-TVAAPSGS-dAb210**

QVTLRESGPALVKPTQTLTLTCTVSGFSLTSYSVHWVRQPPGKGLEWLGVIWASGGTDYNSALMSRLSISKDTS
RNQVVLTMTNMDPVDTATYYCARDPPSSLLRLDYWGRGTLVTVSSASTKGPSVFPLAPSSKSTSGGTAALGCLV
KDYFPEPVTVSWNSGALTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALHNHYTQKSLSLSP
GKGGGGSGVQLLESGGGLVQPGGSLRLSCAASGFTFAWYDMGWVRQAPGKGLEWVSSIDWHGEVTYYADSV
KGRFTISRDNSKNTLYLQMNSLRAEDTAVYYCATAEDEPGYDYWGQGTLVTVSSTVAAPSGSEVQLLESGGGLV
QPGGSLRLSCAASGFTFRNFGMGWVRQAPGKGLEWVSWIISSGTETYYADSVKGRFTISRDNSKNTLYLQMNS
LRAEDTAVYYCAKSLGRFDYWGQGTLVTVSS

**SEQ ID NO: 196: Anti CD-20 Heavy Chain-TVAAPSGS-dAb154-TVAAPSGS-dAb474**

QVQLQQPGAELVKPGASVKMSCKASGYTFTSYNMHWVKQTPGRGLEWIGAIYPGNGDTSYNQKFKGKATLTA
DKSSSTAYMQLSSLTSEDSAVYYCARSTYYGGDWYFNVWGAGTLVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKS
LSLSPGKTVAAPSGSDIQMTQSPSSLSASVGDRVTITCRASRPISDWLHWYQQKPGKAPKLLIAWASSLQGGVP
SRFSGSGSGTDFTLTISSLQPEDFATYYCLQEGWGPPTFGQGTKVEIKRTVAAPSGSGVQLLESGGGLVQPGGSL
RLSCAASGFTFAWYDMGWVRQAPGKGLEWVSSIDWHGEVTYYADSVKGRFTISRDNSKNTLYLQMNSLRAED
TAVYYCATAEDEPGYDYWGQGTLVTVSS

**SEQ ID NO: 197: Anti CD-20 Heavy Chain-TVAAPSGS-dAb210-TVAAPSGS-dAb474**

QVQLQQPGAELVKPGASVKMSCKASGYTFTSYNMHWVKQTPGRGLEWIGAIYPGNGDTSYNQKFKGKATLTA
DKSSSTAYMQLSSLTSEDSAVYYCARSTYYGGDWYFNVWGAGTLVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKS
LSLSPGKTVAAPSGSEVQLLESGGGLVQPGGSLRLSCAASGFTFRNFGMGWVRQAPGKGLEWVSWIISSGTETY
YADSVKGRFTISRDNSKNTLYLQMNSLRAEDTAVYYCAKSLGRFDYWGQGTLVTVSSTVAAPSGSGVQLLESGG
GLVQPGGSLRLSCAASGFTFAWYDMGWVRQAPGKGLEWVSSIDWHGEVTYYADSVKGRFTISRDNSKNTLYL
QMNSLRAEDTAVYYCATAEDEPGYDYWGQGTLVTVSS

**SEQ ID NO: 198: anti cMET 5D5v2 Heavy Chain (hole)-GS-dAb593**

EVQLVESGGGLVQPGGSLRLSCAASGYTFTSYWLHWVRQAPGKGLEWVGMIDPSNSDTRFNPNFKDRFTISAD
TSKNTAYLQMNSLRAEDTAVYYCATYRSYVTPLDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLSCAVK
GFYPSDIAVEWESNGQPENNYKTTPPVLDSDGSFFLVSKLTVDKSRWQQGNVFSCSVMHEALHNHYTQKSLSL
SPGKGSEVQLLVSGGGLVQPGGSLRLSCAASGFTFKAYPMMWVRQAPGKGLEWVSEISPSGSYTYYADSVKGRF
TISRDNSKNTLYLQMNSLRAEDTAVYYCAKDPRKLDYWGQGTLVTVSS

**SEQ ID NO: 199: anti cMET 5D5v2 Heavy Chain (knob)-GS-dAb593**

CPPCPAPELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLWCLVKGFYPS
DIAVEWESNGQPENNYKTTPPVLDSDGSFFLYSKLTVDKSRWQQGNVFSCSVMHEALHNHYTQKSLSLSPGKG
SEVQLLVSGGGLVQPGGSLRLSCAASGFTFKAYPMMWVRQAPGKGLEWVSEISPSGSYTYYADSVKGRFTISRD
NSKNTLYLQMNSLRAEDTAVYYCAKDPRKLDYWGQGTLVTVSS

**SEQ ID NO: 200: anti cMET 5D5v2 Light Chain**

DIQMTQSPSSLSASVGDRVTITCKSSQSLLYTSSQKNYLAWYQQKPGKAPKLLIYWASTRESGVPSRFSGSGSG
TDFTLTISSLQPEDFATYYCQQYYAYPWTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGEC

**SEQ ID NO: 201: anti cMET 5D5v2 IgG4 Heavy Chain (UNIBODY)-GS-dAb593**

EVQLVESGGGLVQPGGSLRLSCAASGYTFTSYWLHWVRQAPGKGLEWVGMIDPSNSDTRFNPNFKDRFTISAD
TSKNTAYLQMNSLRAEDTAVYYCATYRSYVTPLDYWGQGTLVTVSSASTKGPSVFPLAPCSRSTSESTAALGCLV
KDYFPEPVTVSWNSGALTSGVHTFPAVLQSSGLYSLSSVVTVPSSSLGTKTYTCNVDHKPSNTKVDKRVAPEFLG
GPSVFLFPPKPKDTLMISRTPEVTCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQVSLTCLVKGFYPSDIAVEWESN
GQPENNYKTTPPVLDSDGSFFLYSRLTVDKSRWQEGNVFSCSVMHEALHNHYTQKSLSLSLGKGSEVQLLVSGG
GLVQPGGSLRLSCAASGFTFKAYPMMWVRQAPGKGLEWVSEISPSGSYTYYADSVKGRFTISRDNSKNTLYLQM
NSLRAEDTAVYYCAKDPRKLDYWGQGTLVTVSS

**SEQ ID NO: 202: anti cMET 5D5v2 Heavy Chain (hole)**

EVQLVESGGGLVQPGGSLRLSCAASGYTFTSYWLHWVRQAPGKGLEWVGMIDPSNSDTRFNPNFKDRFTISAD
TSKNTAYLQMNSLRAEDTAVYYCATYRSYVTPLDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLSCAVK
GFYPSDIAVEWESNGQPENNYKTTPPVLDSDGSFFLVSKLTVDKSRWQQGNVFSCSVMHEALHNHYTQKSLSL
SPGK

**SEQ ID NO: 203: anti cMET 5D5v2 Heavy Chain (knob)**

CPPCPAPELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLWCLVKGFYPS
DIAVEWESNGQPENNYKTTPPVLDSDGSFFLYSKLTVDKSRWQQGNVFSCSVMHEALHNHYTQKSLSLSPGK

**SEQ ID NO: 204: anti cMET 5D5v2 IgG4 Heavy Chain (UNIBODY)**

EVQLVESGGGLVQPGGSLRLSCAASGYTFTSYWLHWVRQAPGKGLEWVGMIDPSNSDTRFNPNFKDRFTISAD
TSKNTAYLQMNSLRAEDTAVYYCATYRSYVTPLDYWGQGTLVTVSSASTKGPSVFPLAPCSRSTSESTAALGCLV
KDYFPEPVTVSWNSGALTSGVHTFPAVLQSSGLYSLSSVVTVPSSSLGTKTYTCNVDHKPSNTKVDKRVAPEFLG
GPSVFLFPPKPKDTLMISRTPEVTCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQVSLTCLVKGFYPSDIAVEWESN
GQPENNYKTTPPVLDSDGSFFLYSRLTVDKSRWQEGNVFSCSVMHEALHNHYTQKSLSLSLGK

**SEQ ID NO: 205: Anti-human IL13 mAb Heavy chain**

CAGGTGCAGCTCGTGCAGAGCGGCGCCGAAGTGAAAAAGCCCGGCAGCAGCGTGAAGGTGAGCTGCAAGGC
CTCCGGCTTCTACATCAAGGACACCTACATGCACTGGGTCAGGCAGGCTCCTGGCCAGGGCCTGGAGTGGAT
GGGCACTATCGACCCCGCCAACGGCAACACCAAGTACGTGCCCAAGTTCCAGGGCAGGGTGACCATCACCGC
CGATGAGAGCACCAGCACCGCCTACATGGAACTGAGCAGCCTGAGGTCTGAGGACACCGCCGTGTACTATTG
CGCCAGGAGCATCTACGACGACTACCACTACGACGACTACTACGCCATGGACTACTGGGGACAGGGCACACT
AGTGACCGTGTCCAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTACAGCCTGAGC
AGCGTGGTGACCGTGCCCAGCAGCAGCCTGGGCACCCAGACCTACATCTGTAACGTGAACCACAAGCCCAGC
AACACCAAGGTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATGATCAGCAGA
ACCCCCGAGGTGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCACCTACCGGGTGG
TGTCCGTGCTGACCGTGCTGCACCAGGATTGGCTGAACGGCAAGGAGTACAAGTGTAAGGTGTCCAACAAG
GCCCTGCCTGCCCCTATCGAGAAAACCATCAGCAAGGCCAAGGGCCAGCCCAGAGAGCCCCAGGTGTACACC
CTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTGTCCCCTGG
CAAG

**SEQ ID NO: 206: Alternative Anti-human IL13 mAb Heavy chain**

CAGGTGCAGCTGGTGCAGTCTGGGGCTGAGGTGAAGAAGCCTGGGTCCTCGGTGAAGGTCTCCTGCAAGGC
TTCTGGATTCTACATTAAAGACACCTATATGCACTGGGTGCGACAGGCCCCTGGACAAGGGCTTGAGTGGAT
GGGAACGATTGATCCTGCGAATGGTAATACTAAATATGTCCCGAAGTTCCAGGGCAGAGTCACGATTACCGC
GGACGAATCCACGAGCACAGCCTACATGGAGCTGAGCAGCCTGAGATCTGAGGACACGGCCGTGTATTACTG
TGCGAGAAGCATCTATGATGATTACCACTACGACGATTACTATGCTATGGACTACTGGGGCCAAGGGACACT
AGTCACAGTCTCCTCAGCCTCCACCAAGGGCCCATCGGTCTTCCCCCTGGCACCCTCCTCCAAGAGCACCTCT
GGGGGCACAGCGGCCCTGGGCTGCCTGGTCAAGGACTACTTCCCCGAACCGGTGACGGTGTCGTGGAACTC
AGGCGCCCTGACCAGCGGCGTGCACACCTTCCCGGCTGTCCTACAGTCCTCAGGACTCTACTCCCTCAGCAG
CGTGGTGACCGTGCCCTCCAGCAGCTTGGGCACCCAGACCTACATCTGCAACGTGAATCACAAGCCCAGCAA
CACCAAGGTGGACAAGAAAGTTGAGCCCAAATCTTGTGACAAAACTCACACATGCCCACCGTGCCCAGCACC
TGAACTCCTGGGGGGACCGTCAGTCTTCCTCTTCCCCCCAAAACCCAAGGACACCCTCATGATCTCCCGGACC
CCTGAGGTCACATGCGTGGTGGTGGACGTGAGCCACGAAGACCCTGAGGTCAAGTTCAACTGGTACGTGGA
CGGCGTGGAGGTGCATAATGCCAAGACAAAGCCGCGGGAGGAGCAGTACAACAGCACGTACCGTGTGGTCA
GCGTCCTCACCGTCCTGCACCAGGACTGGCTGAATGGCAAGGAGTACAAGTGCAAGGTCTCCAACAAAGCCC
TCCCAGCCCCCATCGAGAAAACCATCTCCAAAGCCAAAGGGCAGCCCCGAGAACCACAGGTGTACACCCTGC
CCCCATCCCGGGATGAGCTGACCAAGAACCAGGTCAGCCTGACCTGCCTGGTCAAAGGCTTCTATCCCAGCG
ACATCGCCGTGGAGTGGGAGAGCAATGGGCAGCCGGAGAACAACTACAAGACCACGCCTCCCGTGCTGGAC
TCCGACGGCTCCTTCTTCCTCTACAGCAAGCTCACCGTGGACAAGAGCAGGTGGCAGCAGGGGAACGTCTTC
TCATGCTCCGTGATGCATGAGGCTCTGCACAACCACTACACGCAGAAGAGCCTCTCCCTGTCTCCGGGTAAA

**SEQ ID NO 207: PascoH IgG2-GS-474 heavy**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPCSRSTSESTAALG
CLVKDYFPEPVTVSWNSGALTSGVHTFPAVLQSSGLYSLSSVVTVPSSNFGTQTYTCNVDHKPSNTKVDKTVER
KCCVECPPCPAPPVAGPSVFLFPPKPKDTLMISRTPEVTCVVVDVSHEDPEVQFNWYVDGVEVHNAKTKPREEQ
FNSTFRVVSVLTVVHQDWLNGKEYKCKVSNKGLPAPIEKTISKTKGQPREPQVYTLPPSREEMTKNQVSLTCLVK
GFYPSDIAVEWESNGQPENNYKTTPPMLDSDGSFFLYSKLTVDKSRWQQGNVFSCSVMHEALHNHYTQKSLSL
SPGKGSGVQLLESGGGLVQPGGSLRLSCAASGFTFAWYDMGWVRQAPGKGLEWVSSIDWHGEVTYYADSVKG
RFTISRDNSKNTLYLQMNSLRAEDTAVYYCATAEDEPGYDYWGQGTLVTVSS

**SEQ ID NO 208: PascoH IgG4-GS-474 heavy chain**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPCSRSTSESTAALG
CLVKDYFPEPVTVSWNSGALTSGVHTFPAVLQSSGLYSLSSVVTVPSSSLGTKTYTCNVDHKPSNTKVDKRVESK
YGPPCPSCPAPEFLGGPSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVMHEALHNHYTQKSLSLSL
GKGSGVQLLESGGGLVQPGGSLRLSCAASGFTFAWYDMGWVRQAPGKGLEWVSSIDWHGEVTYYADSVKGRF
TISRDNSKNTLYLQMNSLRAEDTAVYYCATAEDEPGYDYWGQGTLVTVSS

**SEQ ID NO 209: PascoH IgG4PE-GS-474 heavy chain**

QVTLRESGPALVKPTQTLTLTCTFSGFSLSTSGMGVSWIRQPPGKGLEWLAHIYWDDDKRYNPSLKSRLTISKD
TSRNQVVLTMTNMDPVDTATYYCARRETVFYWYFDVWGRGTLVTVSSASTKGPSVFPLAPCSRSTSESTAALG
CLVKDYFPEPVTVSWNSGALTSGVHTFPAVLQSSGLYSLSSVVTVPSSSLGTKTYTCNVDHKPSNTKVDKRVESK
YGPPCPPCPAPEFEGGPSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVMHEALHNHYTQKSLSLSL
GKGSGVQLLESGGGLVQPGGSLRLSCAASGFTFAWYDMGWVRQAPGKGLEWVSSIDWHGEVTYYADSVKGRF
TISRDNSKNTLYLQMNSLRAEDTAVYYCATAEDEPGYDYWGQGTLVTVSS

**SEQ ID NO: 210: Anti-human IL13 mAb Light chain**

GACATCGTGATGACCCAGTCTCCTCTGAGCCTCCCCGTGACCCCCGGCGAACCAGCCAGCATCTCCTGCAGA
AGCAGCCAGAACATCGTGCACATCAACGGCAACACCTACCTGGAGTGGTACCTGCAAAAGCCCGGCCAGAGC
CCCAGGCTGCTGATCTACAAGATCAGCGACAGGTTCAGCGGCGTGCCCGATAGGTTCAGCGGCAGCGGCAG
CGGCACCGACTTCACCCTGAAGATCAGCAGGGTGGAGGCCGACGACGTGGGCATCTACTACTGCTTCCAGG
GCAGCCACGTCCCCTGGACTTTCGGACAGGGCACCAAGCTGGAGATTAAGCGTACGGTGGCCGCCCCCAGC
GTGTTCATCTTCCCCCCCAGCGATGAGCAGCTGAAGAGCGGCACCGCCAGCGTGGTGTGTCTGCTGAACAAC
TTCTACCCCCGGGAGGCCAAGGTGCAGTGGAAGGTGGACAATGCCCTGCAGAGCGGCAACAGCCAGGAGAG
CGTGACCGAGCAGGACAGCAAGGACTCCACCTACAGCCTGAGCAGCACCCTGACCCTGAGCAAGGCCGACTA
CGAGAAGCACAAGGTGTACGCCTGTGAGGTGACCCACCAGGGCCTGTCCAGCCCCGTGACCAAGAGCTTCAA
CCGGGGCGAGTGC

**SEQ ID NO: 211: Pascolizumab Heavy chain**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG

TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAG

**SEQ ID NO: 212:** Pascolizumab Light chain

GACATCGTGCTGACCCAGAGCCCCTCTTCCCTGAGCGCAAGCGTGGGCGATAGGGTGACCATCACCTGCAAG
GCCAGCCAGAGCGTGGACTACGACGGCGACAGCTACATGAACTGGTACCAGCAGAAGCCCGGCAAGGCCCC
CAAACTGCTGATCTACGCCGCCAGCAACCTCGAGTCAGGCATTCCCAGCAGGTTTAGCGGCAGCGGCAGCGG
CACCGACTTCACCTTCACAATCAGCAGCCTGCAGCCCGAGGACATCGCCACCTACTACTGCCAGCAGAGCAAC
GAGGACCCTCCCACCTTCGGACAGGGCACCAAGGTCGAGATCAAGCGTACGGTGGCCGCCCCCAGCGTGTTC
ATCTTCCCCCCCAGCGATGAGCAGCTGAAGAGCGGCACCGCCAGCGTGGTGTGTCTGCTGAACAACTTCTAC
CCCCGGGAGGCCAAGGTGCAGTGGAAGGTGGACAATGCCCTGCAGAGCGGCAACAGCCAGGAGAGCGTGAC
CGAGCAGGACAGCAAGGACTCCACCTACAGCCTGAGCAGCACCCTGACCCTGAGCAAGGCCGACTACGAGAA
GCACAAGGTGTACGCCTGTGAGGTGACCCACCAGGGCCTGTCCAGCCCCGTGACCAAGAGCTTCAACCGGG
GCGAGTGC

**SEQ ID NO: 213:** Mepolizumab Heavy chain

CAGGTGACCCTGAGGGAGAGCGGCCCCGCCCTGGTGAAGCCCACACAGACCCTCACTCTGACCTGCACCGTG
AGCGGCTTCAGCCTGACCTCCTACAGCGTCCACTGGGTGAGGCAGCCCCCCGGCAAGGGCCTGGAGTGGCT
GGGCGTGATCTGGGCAAGCGGCGGCACCGACTACAACAGCGCCCTGATGAGCAGGCTCTCCATCAGCAAGG
ACACCAGCCGGAACCAGGTGGTGCTGACCATGACCAACATGGACCCCGTGGACACCGCCACCTATTACTGCG
CCAGGGACCCTCCCTCTAGCCTGCTGAGGCTGGACTACTGGGGCAGGGGAACACTAGTGACCGTGTCCAGC
GCCAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAGCCCTGACCA
GCGGCGTGCACACCTTCCCCGCCGTGCTGCAGAGCAGCGGCCTGTACAGCCTGAGCAGCGTGGTGACCGTG
CCCAGCAGCAGCCTGGGCACCCAGACCTACATCTGTAACGTGAACCACAAGCCCAGCAACACCAAGGTGGAC
AAGAAGGTGGAGCCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCG
TGCTGCACCAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCCCTAGCAGAG
ATGAGCTGACCAAGAACCAGGTGTCCCTGACCTGCCTGGTGAAGGGCTTCTACCCCAGCGACATCGCCGTGG
AGTGGGAGAGCAACGGCCAGCCCGAGAACAACTACAAGACCACCCCCCCTGTGCTGGACAGCGATGGCAGCT
TCTTCCTGTACAGCAAGCTGACCGTGGACAAGAGCAGATGGCAGCAGGGCAACGTGTTCAGCTGCTCCGTGA
TGCACGAGGCCCTGCACAATCACTACACCCAGAAGAGCCTGAGCCTGTCCCCTGGCAAG

**SEQ ID NO: 214:** Mepolizumab Light chain

GACATCGTGATGACCCAGTCTCCCGATTCACTGGCCGTGAGCCTGGGCGAGAGGGCCACCATCAACTGCAAG
AGCAGCCAGAGCCTCCTGAACAGCGGCAACCAGAAGAACTACCTGGCCTGGTACCAGCAGAAACCCGGCCAG
CCCCCCAAGCTGCTGATCTATGGCGCCTCCACCAGGGAGAGCGGCGTGCCAGACAGGTTTAGCGGCAGCGG
CAGCGGCACCGACTTCACCCTGACAATCAGCAGCCTGCAGGCCGAGGACGTGGCCGTGTACTACTGCCAGAA
CGTCCACAGCTTCCCCTTCACCTTCGGCGGGGGAACCAAGCTGGAGATCAAGCGTACGGTGGCCGCCCCCAG
CGTGTTCATCTTCCCCCCCAGCGATGAGCAGCTGAAGAGCGGCACCGCCAGCGTGGTGTGTCTGCTGAACAA
CTTCTACCCCCGGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGAGCAAGGCCGACT

ACGAGAAGCACAAGGTGTACGCCTGTGAGGTGACCCACCAGGGCCTGTCCAGCCCCGTGACCAAGAGCTTCA
ACCGGGGCGAGTGC

**SEQ ID NO: 215:** PascoH-474 Heavy chain

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGATC
CGGCGTGCAGCTCCTGGAGAGCGGCGGAGGCCTGGTCCAGCCCGGCGGCAGCCTGAGGCTGAGCTGCGCC
GCCAGCGGCTTCACCTTCGCCTGGTATGATATGGGCTGGGTGAGGCAGGCCCCCGGCAAGGGCCTGGAGTG
GGTGTCCAGCATCGACTGGCACGGGGAGGTGACCTACTACGCCGACAGCGTGAAGGGCAGGTTCACCATCA
GCAGGGACAACAGCAAGAACACCCTGTACCTGCAGATGAACAGCCTGAGGGCCGAGGACACCGCAGTGTACT
ACTGCGCCACCGCCGAGGACGAACCCGGCTACGACTACTGGGGCCAGGGCACCCTGGTGACTGTGAGCAGC

**SEQ ID NO: 216:** PascoH-TVAAPS-474 Heavy chain

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGACCGT
GGCCGCCCCCTCGGGATCCGGCGTGCAGCTCCTGGAGAGCGGCGGAGGCCTGGTCCAGCCCGGCGGCAGCC
TGAGGCTGAGCTGCGCCGCCAGCGGCTTCACCTTCGCCTGGTATGATATGGGCTGGGTGAGGCAGGCCCCC

GGCAAGGGCCTGGAGTGGGTGTCCAGCATCGACTGGCACGGGGAGGTGACCTACTACGCCGACAGCGTGAA
GGGCAGGTTCACCATCAGCAGGGACAACAGCAAGAACACCCTGTACCTGCAGATGAACAGCCTGAGGGCCGA
GGACACCGCAGTGTACTACTGCGCCACCGCCGAGGACGAACCCGGCTACGACTACTGGGGCCAGGGCACCC
TGGTGACTGTGAGCAGC

**SEQ ID NO: 217:** PascoL-474 Light chain

GACATCGTGCTGACCCAGAGCCCCTCTTCCCTGAGCGCAAGCGTGGGCGATAGGGTGACC
ATCACCTGCAAGGCCAGCCAGAGCGTGGACTACGACGGCGACAGCTACATGAACTGGTAC
CAGCAGAAGCCCGGCAAGGCCCCCAAACTGCTGATCTACGCCGCCAGCAACCTCGAGTCA
GGCATTCCCAGCAGGTTTAGCGGCAGCGGCAGCGGCACCGACTTCACCTTCACAATCAGC
AGCCTGCAGCCCGAGGACATCGCCACCTACTACTGCCAGCAGAGCAACGAGGACCCTCCC
ACCTTCGGACAGGGCACCAAGGTCGAGATCAAGCGTACGGTGGCCGCCCCCAGCGTGTTC
ATCTTCCCCCCCAGCGATGAGCAGCTGAAGAGCGGCACCGCCAGCGTGGTGTGTCTGCTG
AACAACTTCTACCCCCGGGAGGCCAAGGTGCAGTGGAAGGTGGACAATGCCCTGCAGAGC
GGCAACAGCCAGGAGAGCGTGACCGAGCAGGACAGCAAGGACTCCACCTACAGCCTGAGC
AGCACCCTGACCCTGAGCAAGGCCGACTACGAGAAGCACAAGGTGTACGCCTGTGAGGTG
ACCCACCAGGGCCTGTCCAGCCCCGTGACCAAGAGCTTCAACCGGGGCGAGTGTGGATCC
GGCGTGCAGCTCCTGGAGAGCGGCGGAGGCCTGGTCCAGCCCGGCGGCAGCCTGAGGCTG
AGCTGCGCCGCCAGCGGCTTCACCTTCGCCTGGTATGATATGGGCTGGGTGAGGCAGGCC
CCCGGCAAGGGCCTGGAGTGGGTGTCCAGCATCGACTGGCACGGGGAGGTGACCTACTAC
GCCGACAGCGTGAAGGGCAGGTTCACCATCAGCAGGGACAACAGCAAGAACACCCTGTAC
CTGCAGATGAACAGCCTGAGGGCCGAGGACACCGCAGTGTACTACTGCGCCACCGCCGAG
GACGAACCCGGCTACGACTACTGGGGCCAGGGCACCCTGGTGACTGTGAGCAGC

**SEQ ID NO: 218:** PascoL-TVAAPS-474 Light chain

GACATCGTGCTGACCCAGAGCCCCTCTTCCCTGAGCGCAAGCGTGGGCGATAGGGTGACC
ATCACCTGCAAGGCCAGCCAGAGCGTGGACTACGACGGCGACAGCTACATGAACTGGTAC
CAGCAGAAGCCCGGCAAGGCCCCCAAACTGCTGATCTACGCCGCCAGCAACCTCGAGTCA
GGCATTCCCAGCAGGTTTAGCGGCAGCGGCAGCGGCACCGACTTCACCTTCACAATCAGC
AGCCTGCAGCCCGAGGACATCGCCACCTACTACTGCCAGCAGAGCAACGAGGACCCTCCC
ACCTTCGGACAGGGCACCAAGGTCGAGATCAAGCGTACGGTGGCCGCCCCCAGCGTGTTC
ATCTTCCCCCCCAGCGATGAGCAGCTGAAGAGCGGCACCGCCAGCGTGGTGTGTCTGCTG
AACAACTTCTACCCCCGGGAGGCCAAGGTGCAGTGGAAGGTGGACAATGCCCTGCAGAGC
GGCAACAGCCAGGAGAGCGTGACCGAGCAGGACAGCAAGGACTCCACCTACAGCCTGAGC
AGCACCCTGACCCTGAGCAAGGCCGACTACGAGAAGCACAAGGTGTACGCCTGTGAGGTG
ACCCACCAGGGCCTGTCCAGCCCCGTGACCAAGAGCTTCAACCGGGGCGAGTGCACCGTG
GCCGCCCCCTCGGGATCCGGCGTGCAGCTCCTGGAGAGCGGCGGAGGCCTGGTCCAGCCC
GGCGGCAGCCTGAGGCTGAGCTGCGCCGCCAGCGGCTTCACCTTCGCCTGGTATGATATG
GGCTGGGTGAGGCAGGCCCCCGGCAAGGGCCTGGAGTGGGTGTCCAGCATCGACTGGCAC
GGGGAGGTGACCTACTACGCCGACAGCGTGAAGGGCAGGTTCACCATCAGCAGGGACAAC
AGCAAGAACACCCTGTACCTGCAGATGAACAGCCTGAGGGCCGAGGACACCGCAGTGTAC
TACTGCGCCACCGCCGAGGACGAACCCGGCTACGACTACTGGGGCCAGGGCACCCTGGTG
ACTGTGAGCAGC

**SEQ ID NO: 219:** IL-5 mAb-G4S-DOM9-112-210 heavy chain

CAGGTGACCCTGAGGGAGAGCGGCCCCGCCCTGGTGAAGCCCACACAGACCCTCACTCTGACCTGCACCGTG
AGCGGCTTCAGCCTGACCTCCTACAGCGTCCACTGGGTGAGGCAGCCCCCCGGCAAGGGCCTGGAGTGGCT
GGGCGTGATCTGGGCAAGCGGCGGCACCGACTACAACAGCGCCCTGATGAGCAGGCTCTCCATCAGCAAGG
ACACCAGCCGGAACCAGGTGGTGCTGACCATGACCAACATGGACCCCGTGGACACCGCCACCTATTACTGCG
CCAGGGACCCTCCCTCTAGCCTGCTGAGGCTGGACTACTGGGGCAGGGGAACACTAGTGACCGTGTCCAGC
GCCAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAGCCCTGACCA

GCGGCGTGCACACCTTCCCCGCCGTGCTGCAGAGCAGCGGCCTGTACAGCCTGAGCAGCGTGGTGACCGTG
CCCAGCAGCAGCCTGGGCACCCAGACCTACATCTGTAACGTGAACCACAAGCCCAGCAACACCAAGGTGGAC
AAGAAGGTGGAGCCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCG
TGCTGCACCAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCCCTAGCAGAG
ATGAGCTGACCAAGAACCAGGTGTCCCTGACCTGCCTGGTGAAGGGCTTCTACCCCAGCGACATCGCCGTGG
AGTGGGAGAGCAACGGCCAGCCCGAGAACAACTACAAGACCACCCCCCCTGTGCTGGACAGCGATGGCAGCT
TCTTCCTGTACAGCAAGCTGACCGTGGACAAGAGCAGATGGCAGCAGGGCAACGTGTTCAGCTGCTCCGTGA
TGCACGAGGCCCTGCACAATCACTACACCCAGAAGAGCCTGAGCCTGTCCCCTGGCAAGGGCGGCGGCGGA
TCCGAAGTGCAGCTCCTGGAGAGCGGCGGCGGCCTGGTGCAGCCCGGCGGCAGCCTGAGGCTGAGCTGCGC
CGCTAGCGGCTTCACCTTCAGGAACTTCGGCATGGGCTGGGTCAGGCAGGCCCCCGGCAAGGGCCTGGAGT
GGGTCAGCTGGATCATCAGCTCCGGCACCGAGACCTACTACGCCGACAGCGTGAAGGGCAGGTTCACCATCA
GCCGCGACAACAGCAAGAACACCCTGTACCTGCAGATGAACAGCCTGAGGGCCGAGGACACCGCCGTCTACT
ACTGCGCCAAGAGCCTGGGCAGGTTCGACTACTGGGGACAGGGGACCCTGGTGACTGTGAGCAGC

**SEQ ID NO: 220:** IL-5 mAb-G4S-DOM10-53-474 light chain

GACATCGTGATGACCCAGTCTCCCGATTCACTGGCCGTGAGCCTGGGCGAGAGGGCCACCATCAACTGCAAG
AGCAGCCAGAGCCTCCTGAACAGCGGCAACCAGAAGAACTACCTGGCCTGGTACCAGCAGAAACCCGGCCAG
CCCCCCAAGCTGCTGATCTATGGCGCCTCCACCAGGGAGAGCGGCGTGCCAGACAGGTTTAGCGGCAGCGG
CAGCGGCACCGACTTCACCCTGACAATCAGCAGCCTGCAGGCCGAGGACGTGGCCGTGTACTACTGCCAGAA
CGTCCACAGCTTCCCCTTCACCTTCGGCGGGGGAACCAAGCTGGAGATCAAGCGTACGGTGGCCGCCCCCAG
CGTGTTCATCTTCCCCCCCAGCGATGAGCAGCTGAAGAGCGGCACCGCCAGCGTGGTGTGTCTGCTGAACAA
CTTCTACCCCCGGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGAGCAAGGCCGACT
ACGAGAAGCACAAGGTGTACGCCTGTGAGGTGACCCACCAGGGCCTGTCCAGCCCCGTGACCAAGAGCTTCA
ACCGGGGCGAGTGCGGCGGCGGCGGATCCGGCGTGCAGCTCCTGGAGAGCGGCGGAGGCCTGGTCCAGCC
CGGCGGCAGCCTGAGGCTGAGCTGCGCCGCCAGCGGCTTCACCTTCGCCTGGTATGATATGGGCTGGGTGA
GGCAGGCCCCCGGCAAGGGCCTGGAGTGGGTGTCCAGCATCGACTGGCACGGGGAGGTGACCTACTACGCC
GACAGCGTGAAGGGCAGGTTCACCATCAGCAGGGACAACAGCAAGAACACCCTGTACCTGCAGATGAACAGC
CTGAGGGCCGAGGACACCGCAGTGTACTACTGCGCCACCGCCGAGGACGAACCCGGCTACGACTACTGGGG
CCAGGGCACCCTGGTGACTGTGAGCAGC

**SEQ ID NO: 221: 586H-210 Heavy chain (GS removed)**

CAGGTGCAGCTCGTGCAGAGCGGCGCCGAAGTGAAAAAGCCCGGCAGCAGCGTGAAGGTGAGCTGCAAGGC
CTCCGGCTTCTACATCAAGGACACCTACATGCACTGGGTCAGGCAGGCTCCTGGCCAGGGCCTGGAGTGGAT
GGGCACTATCGACCCCGCCAACGGCAACACCAAGTACGTGCCCAAGTTCCAGGGCAGGGTGACCATCACCGC
CGATGAGAGCACCAGCACCGCCTACATGGAACTGAGCAGCCTGAGGTCTGAGGACACCGCCGTGTACTATTG
CGCCAGGAGCATCTACGACGACTACCACTACGACGACTACTACGCCATGGACTACTGGGGACAGGGCACACT
AGTGACCGTGTCCAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTACAGCCTGAGC
AGCGTGGTGACCGTGCCCAGCAGCAGCCTGGGCACCCAGACCTACATCTGTAACGTGAACCACAAGCCCAGC
AACACCAAGGTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATGATCAGCAGA
ACCCCCGAGGTGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCACCTACCGGGTGG
TGTCCGTGCTGACCGTGCTGCACCAGGATTGGCTGAACGGCAAGGAGTACAAGTGTAAGGTGTCCAACAAG
GCCCTGCCTGCCCCTATCGAGAAAACCATCAGCAAGGCCAAGGGCCAGCCCAGAGAGCCCCAGGTGTACACC

CTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTGTCCCCTGG
CAAGGAAGTGCAGCTCCTGGAGAGCGGCGGCGGCCTGGTGCAGCCCGGCGGCAGCCTGAGGCTGAGCTGC
GCCGCTAGCGGCTTCACCTTCAGGAACTTCGGCATGGGCTGGGTCAGGCAGGCCCCCGGCAAGGGCCTGGA
GTGGGTCAGCTGGATCATCAGCTCCGGCACCGAGACCTACTACGCCGACAGCGTGAAGGGCAGGTTCACCAT
CAGCCGCGACAACAGCAAGAACACCCTGTACCTGCAGATGAACAGCCTGAGGGCCGAGGACACCGCCGTCTA
CTACTGCGCCAAGAGCCTGGGCAGGTTCGACTACTGGGGACAGGGGACCCTGGTGACTGTGAGCAGC

**SEQ ID NO: 222: 586H-TVAAPS-210 Heavy chain (GS removed)**

CAGGTGCAGCTCGTGCAGAGCGGCGCCGAAGTGAAAAAGCCCGGCAGCAGCGTGAAGGTGAGCTGCAAGGC
CTCCGGCTTCTACATCAAGGACACCTACATGCACTGGGTCAGGCAGGCTCCTGGCCAGGGCCTGGAGTGGAT
GGGCACTATCGACCCCGCCAACGGCAACACCAAGTACGTGCCCAAGTTCCAGGGCAGGGTGACCATCACCGC
CGATGAGAGCACCAGCACCGCCTACATGGAACTGAGCAGCCTGAGGTCTGAGGACACCGCCGTGTACTATTG
CGCCAGGAGCATCTACGACGACTACCACTACGACGACTACTACGCCATGGACTACTGGGGACAGGGCACACT
AGTGACCGTGTCTAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTACAGCCTGAGC
AGCGTGGTGACCGTGCCCAGCAGCAGCCTGGGCACCCAGACCTACATCTGTAACGTGAACCACAAGCCCAGC
AACACCAAGGTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATGATCAGCAGA
ACCCCCGAGGTGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCACCTACCGGGTGG
TGTCCGTGCTGACCGTGCTGCACCAGGATTGGCTGAACGGCAAGGAGTACAAGTGTAAGGTGTCCAACAAG
GCCCTGCCTGCCCCTATCGAGAAAACCATCAGCAAGGCCAAGGGCCAGCCCAGAGAGCCCCAGGTGTACACC
CTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTGTCCCCTGG
CAAGACCGTGGCCGCCCCCTCGGAAGTGCAGCTCCTGGAGAGCGGCGGCGGCCTGGTGCAGCCCGGCGGCA
GCCTGAGGCTGAGCTGCGCCGCTAGCGGCTTCACCTTCAGGAACTTCGGCATGGGCTGGGTCAGGCAGGCC
CCCGGCAAGGGCCTGGAGTGGGTCAGCTGGATCATCAGCTCCGGCACCGAGACCTACTACGCCGACAGCGT
GAAGGGCAGGTTCACCATCAGCCGCGACAACAGCAAGAACACCCTGTACCTGCAGATGAACAGCCTGAGGGC
CGAGGACACCGCCGTCTACTACTGCGCCAAGAGCCTGGGCAGGTTCGACTACTGGGGACAGGGGACCCTGG
TGACTGTGAGCAGC

**SEQ ID NO: 223:** PascoH-474 Heavy Chain (GS removed)

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT

GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGCGT
GCAGCTCCTGGAGAGCGGCGGAGGCCTGGTCCAGCCCGGCGGCAGCCTGAGGCTGAGCTGCGCCGCCAGCG
GCTTCACCTTCGCCTGGTATGATATGGGCTGGGTGAGGCAGGCCCCCGGCAAGGGCCTGGAGTGGGTGTCC
AGCATCGACTGGCACGGGGAGGTGACCTACTACGCCGACAGCGTGAAGGGCAGGTTCACCATCAGCAGGGA
CAACAGCAAGAACACCCTGTACCTGCAGATGAACAGCCTGAGGGCCGAGGACACCGCAGTGTACTACTGCGC
CACCGCCGAGGACGAACCCGGCTACGACTACTGGGGC
CAGGGCACCCTGGTGACTGTGAGCAGC

**SEQ ID NO: 224:** PascoH-TVAAPS-474 Heavy Chain (GS removed)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGCCCAAGAGCTGT
GACAAGACCCACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACCAAGCCCAG
GGAGGAGCAGTACAACAGCACCTACCGGGTGGTGTCCGTGCTGACCGTGCTGCACCAGGATTGGCTGAACG
GCAAGGAGTACAAGTGTAAGGTGTCCAACAAGGCCCTGCCTGCCCCTATCGAGAAAACCATCAGCAAGGCCA
AGGGCCAGCCCAGAGAGCCCCAGGTGTACACCCTGCCCCCTAGCAGAGATGAGCTGACCAAGAACCAGGTGT
CCCTGACCTGCCTGGTGAAGGGCTTCTACCCCAGCGACATCGCCGTGGAGTGGGAGAGCAACGGCCAGCCC
GAGAACAACTACAAGACCACCCCCCCTGTGCTGGACAGCGATGGCAGCTTCTTCCTGTACAGCAAGCTGACC
GTGGACAAGAGCAGATGGCAGCAGGGCAACGTGTTCAGCTGCTCCGTGATGCACGAGGCCCTGCACAATCA
CTACACCCAGAAGAGCCTGAGCCTGTCCCCTGGCAAGACCGTGGCCGCCCCCTCGGGCGTGCAGCTCCTGGA
GAGCGGCGGAGGCCTGGTCCAGCCCGGCGGCAGCCTGAGGCTGAGCTGCGCCGCCAGCGGCTTCACCTTCG
CCTGGTATGATATGGGCTGGGTGAGGCAGGCCCCCGGCAAGGGCCTGGAGTGGGTGTCCAGCATCGACTGG
CACGGGGAGGTGACCTACTACGCCGACAGCGTGAAGGGCAGGTTCACCATCAGCAGGGACAACAGCAAGAA
CACCCTGTACCTGCAGATGAACAGCCTGAGGGCCGAGGACACCGCAGTGTACTACTGCGCCACCGCCGAGGA
CGAACCCGGCTACGACTACTGGGGCCAGGGCACCCTGGTGACTGTGAGCAGC

**SEQ ID NO: 225:** PascoH-ASTKGPT-474 Heavy Chain (second GS removed)

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA

CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCTGTGCTGGACAGC
GATGGCAGCTTCTTCCTGTACAGCAAGCTGACCGTGGACAAGAGCAGATGGCAGCAGGGCAACGTGTTCAG
CTGCTCCGTGATGCACGAGGCCCTGCACAATCACTACACCCAGAAGAGCCTGAGCCTGTCCCCTGGCAAGGG
ATCAGCCAGCACCAAGGGCCCCACGGGCGTGCAGCTCCTGGAGAGCGGCGGAGGCCTGGTCCAGCCCGGCG
GCAGCCTGAGGCTGAGCTGCGCCGCCAGCGGCTTCACCTTCGCCTGGTATGATATGGGCTGGGTGAGGCAG
GCCCCCGGCAAGGGCCTGGAGTGGGTGTCCAGCATCGACTGGCACGGGGAGGTGACCTACTACGCCGACAG
CGTGAAGGGCAGGTTCACCATCAGCAGGGACAACAGCAAGAACACCCTGTACCTGCAGATGAACAGCCTGAG
GGCCGAGGACACCGCAGTGTACTACTGCGCCACCGCCGAGGACGAACCCGGCTACGACTACTGGGGCCAGG
GCACCCTGGTGACTGTGAGCAGC

**SEQ ID NO: 226: Heavy chain of anti-IGF-1R antibody H0L0 with DOM15-26-593 fused at C-terminus with TVAAPSGS linker**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTgTAaCgTgAACcACAAGCCCAGCAACACC
AAGGTGGACAAGAAGGTGGAGCCCAAGAGCTGTGACAAGACCCACACCTGCCCCCCCTGCCCTGCCCCCGAG
CTGCTGGGAGGCCCCAGCGTGTTCCTGTTCCCCCCCAAGCCTAAGGACACCCTGATGATCAGCAGAACCCCC
GAGGTGACCTGTGTGGTGGTGGATGTGAGCCACGAGGACCCTGAGGTGAAGTTCAACTGGTACGTGGACGG
CGTGGAGGTGCACAATGCCAAGACCAAGCCCAGGGAGGAGCAGTACAACAGCACCTACCGGGTGGTGTCCG
TGCTGACCGTGCTGCACCAGGATTGGCTGAACGGCAAGGAGTACAAGTGTAAGGTGTCCAACAAGGCCCTG
CCTGCCCCTATCGAGAAAACCATCAGCAAGGCCAAGGGCCAGCCCAGAGAGCCCCAGGTGTACACCCTGCCC
CCTAGCAGAGATGAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCTGTGCTGGACAGC
GATGGCAGCTTCTTCCTGTACAGCAAGCTGACCGTGGACAAGAGCAGATGGCAGCAGGGCAACGTGTTCAG
CTGCTCCGTGATGCACGAGGCCCTGCACAATCACTACACCCAGAAGAGCCTGAGCCTGTCCCCTGGCAAGAC
CGTGGCCGCCCCCTCGGGATCCGAGGTGCAGCTCCTGGTCAGCGGCGGCGGCCTGGTCCAGCCCGGAGGCT
CACTGAGGCTGAGCTGCGCCGCTAGCGGCTTCACCTTCAAGGCCTACCCCATGATGTGGGTCAGGCAGGCCC
CCGGCAAAGGCCTGGAGTGGGTGTCTGAGATCAGCCCCAGCGGCAGCTACACCTACTACGCCGACAGCGTG
AAGGGCAGGTTCACCATCAGCAGGGACAACAGCAAGAACACCCTGTACCTGCAGATGAACTCTCTGAGGGCC
GAGGACACCGCCGTGTACTACTGCGCCAAGGACCCCAGGAAGCTGGACTATTGGGGCCAGGGCACTCTGGT
GACCGTGAGCAGC

**SEQ ID NO: 227: IGF1RmAb-GS-DOM15-26-593 Heavy chain**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC

CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTgTAaCgTgAACCACAAGCCCAGCAACACC
AAGGTGGACAAGAAGGTGGAGCCCAAGAGCTGTGACAAGACCCACACCTGCCCCCCCTGCCCTGCCCCCGAG
CTGCTGGGAGGCCCCAGCGTGTTCCTGTTCCCCCCCAAGCCTAAGGACACCCTGATGATCAGCAGAACCCCC
GAGGTGACCTGTGTGGTGGTGGATGTGAGCCACGAGGACCCTGAGGTGAAGTTCAACTGGTACGTGGACGG
CGTGGAGGTGCACAATGCCAAGACCAAGCCCAGGGAGGAGCAGTACAACAGCACCTACCGGGTGGTGTCCG
TGCTGACCGTGCTGCACCAGGATTGGCTGAACGGCAAGGAGTACAAGTGTAAGGTGTCCAACAAGGCCCTG
CCTGCCCCTATCGAGAAAACCATCAGCAAGGCCAAGGGCCAGCCCAGAGAGCCCCAGGTGTACACCCTGCCC
CCTAGCAGAGATGAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCTGTGCTGGACAGC
GATGGCAGCTTCTTCCTGTACAGCAAGCTGACCGTGGACAAGAGCAGATGGCAGCAGGGCAACGTGTTCAG
CTGCTCCGTGATGCACGAGGCCCTGCACAATCACTACACCCAGAAGAGCCTGAGCCTGTCCCCTGGCAAGGG
ATCCGAGGTGCAGCTCCTGGTCAGCGGCGGCGGCCTGGTCCAGCCCGGAGGCTCACTGAGGCTGAGCTGCG
CCGCTAGCGGCTTCACCTTCAAGGCCTACCCCATGATGTGGGTCAGGCAGGCCCCCGGCAAAGGCCTGGAGT
GGGTGTCTGAGATCAGCCCCAGCGGCAGCTACACCTACTACGCCGACAGCGTGAAGGGCAGGTTCACCATCA
GCAGGGACAACAGCAAGAACACCCTGTACCTGCAGATGAACTCTCTGAGGGCCGAGGACACCGCCGTGTACT
ACTGCGCCAAGGACCCCAGGAAGCTGGACTATTGGGGCCAGGGCACTCTGGTGACCGTGAGCAGC

**SEQ ID NO: 228: anti-IGF-1R antibody Heavy chain**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAG

**SEQ ID NO: 229: Light chain of anti-IGF-1R antibody H0L0 with DOM15-26-593 fused at C-terminus with TVAAPSGS linker**

GACATCGTGATGACCCAGAGCCCCCTGAGCCTGCCCGTGACCCCTGGCGAGCCCGCCAGCATCAGCTGCAGA
AGCAGCCAGAGCATCGTCCAGAGCAACGGCGACACCTACCTGGAATGGTATCTGCAGAAGCCCGGCCAGTCC
CCCCAGCTGCTGATCTACAGAGTGAGCAACCGGTTCAGCGGCGTGCCCGACAGATTCAGCGGCAGCGGCTCC
GGCACCGACTTCACCCTGAAGATCAGCCGGGTGGAGGCCGAGGACGTGGGCGTGTACTACTGCTTTCAAGG
CAGCCACGTGCCCTACACCTTCGGCCAGGGCACCAAGCTGGAAATCAAGCGTACGGTGGCCGCCCCCAGCGT
GTTCATCTTCCCCCCCAGCGATGAGCAGCTGAAGAGCGGCACCGCCAGCGTGGTGTGTCTGCTGAACAACTT

CTACCCCCGGGAGGCCAAGGTGCAGTGGAAGGTGGACAATGCCCTGCAGAGCGGCAACAGCCAGGAGAGCG
TGACCGAGCAGGACAGCAAGGACTCCACCTACAGCCTGAGCAGCACCCTGACCCTGAGCAAGGCCGACTACG
AGAAGCACAAGGTGTACGCCTGTGAGGTGACCCACCAGGGCCTGTCCAGCCCCGTGACCAAGAGCTTCAACC
GGGGCGAGTGCACCGTGGCCGCCCCCTCGGGATCCGAGGTGCAGCTCCTGGTCAGCGGCGGCGGCCTGGTC
CAGCCCGGAGGCTCACTGAGGCTGAGCTGCGCCGCTAGCGGCTTCACCTTCAAGGCCTACCCCATGATGTGG
GTCAGGCAGGCCCCCGGCAAAGGCCTGGAGTGGGTGTCTGAGATCAGCCCCAGCGGCAGCTACACCTACTA
CGCCGACAGCGTGAAGGGCAGGTTCACCATCAGCAGGGACAACAGCAAGAACACCCTGTACCTGCAGATGAA
CTCTCTGAGGGCCGAGGACACCGCCGTGTACTACTGCGCCAAGGACCCCAGGAAGCTGGACTATTGGGGCC
AGGGCACTCTGGTGACCGTGAGCAGC

**SEQ ID NO: 230: Light chain of anti-IGF-1R antibody H0L0 with DOM15-26-593 fused at C-terminus with GS linker**

GACATCGTGATGACCCAGAGCCCCCTGAGCCTGCCCGTGACCCCTGGCGAGCCCGCCAGCATCAGCTGCAGA
AGCAGCCAGAGCATCGTCCAGAGCAACGGCGACACCTACCTGGAATGGTATCTGCAGAAGCCCGGCCAGTCC
CCCCAGCTGCTGATCTACAGAGTGAGCAACCGGTTCAGCGGCGTGCCCGACAGATTCAGCGGCAGCGGCTCC
GGCACCGACTTCACCCTGAAGATCAGCCGGGTGGAGGCCGAGGACGTGGGCGTGTACTACTGCTTTCAAGG
CAGCCACGTGCCCTACACCTTCGGCCAGGGCACCAAGCTGGAAATCAAGCGTACGGTGGCCGCCCCCAGCGT
GTTCATCTTCCCCCCCAGCGATGAGCAGCTGAAGAGCGGCACCGCCAGCGTGGTGTGTCTGCTGAACAACTT
CTACCCCCGGGAGGCCAAGGTGCAGTGGAAGGTGGACAATGCCCTGCAGAGCGGCAACAGCCAGGAGAGCG
TGACCGAGCAGGACAGCAAGGACTCCACCTACAGCCTGAGCAGCACCCTGACCCTGAGCAAGGCCGACTACG
AGAAGCACAAGGTGTACGCCTGTGAGGTGACCCACCAGGGCCTGTCCAGCCCCGTGACCAAGAGCTTCAACC
GGGGCGAGTGTGGATCCGAGGTGCAGCTCCTGGTCAGCGGCGGCGGCCTGGTCCAGCCCGGAGGCTCACTG
AGGCTGAGCTGCGCCGCTAGCGGCTTCACCTTCAAGGCCTACCCCATGATGTGGGTCAGGCAGGCCCCCGG
CAAAGGCCTGGAGTGGGTGTCTGAGATCAGCCCCAGCGGCAGCTACACCTACTACGCCGACAGCGTGAAGG
GCAGGTTCACCATCAGCAGGGACAACAGCAAGAACACCCTGTACCTGCAGATGAACTCTCTGAGGGCCGAGG
ACACCGCCGTGTACTACTGCGCCAAGGACCCCAGGAAGCTGGACTATTGGGGCCAGGGCACTCTGGTGACC
GTGAGCAGC

**SEQ ID NO: 231: anti-IGF-1R antibody Light chain**

GACATCGTGATGACCCAGAGCCCCCTGAGCCTGCCCGTGACCCCTGGCGAGCCCGCCAGCATCAGCTGCAGA
AGCAGCCAGAGCATCGTCCAGAGCAACGGCGACACCTACCTGGAATGGTATCTGCAGAAGCCCGGCCAGTCC
CCCCAGCTGCTGATCTACAGAGTGAGCAACCGGTTCAGCGGCGTGCCCGACAGATTCAGCGGCAGCGGCTCC
GGCACCGACTTCACCCTGAAGATCAGCCGGGTGGAGGCCGAGGACGTGGGCGTGTACTACTGCTTTCAAGG
CAGCCACGTGCCCTACACCTTCGGCCAGGGCACCAAGCTGGAAATCAAGCGTACGGTGGCCGCCCCCAGCGT
GTTCATCTTCCCCCCCAGCGATGAGCAGCTGAAGAGCGGCACCGCCAGCGTGGTGTGTCTGCTGAACAACTT
CTACCCCCGGGAGGCCAAGGTGCAGTGGAAGGTGGACAATGCCCTGCAGAGCGGCAACAGCCAGGAGAGCG
TGACCGAGCAGGACAGCAAGGACTCCACCTACAGCCTGAGCAGCACCCTGACCCTGAGCAAGGCCGACTACG
AGAAGCACAAGGTGTACGCCTGTGAGGTGACCCACCAGGGCCTGTCCAGCCCCGTGACCAAGAGCTTCAACC
GGGGCGAGTGC

**SEQ ID NO: 232: anti-IGF1R Heavy Chain-GS-TLPC**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC

CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGG
GATCCGACGGCGGCGGCATTAGGAGGAGCATGAGCGGCACCTGGTACCTGAAGGCCATGACCGTGGATAGG
GAGTTCCCCGAGATGAACCTGGAGAGCGTGACCCCCATGACACTGACCCTGCTCAAGGGCCACAACCTGGAG
GCCAAGGTCACCATGCTGATCTCAGGCAGGTGCCAGGAGGTGAAGGCAGTGCTGGGCAGGACCAAGGAGAG
GAAGAAGTACACCGCCGACGGGGGCAAGCACGTGGCCTATATCATCCCCAGCGCCGTGAGGGACCACGTGA
TCTTCTACAGCGAGGGCCAGCTCCACGGAAAGCCCGTGAGAGGCGTGAAGCTGGTGGGCAGGGACCCCAAG
AACAACCTGGAGGCCCTGGAGGACTTCGAAAAAGCCGCAGGCGCCAGGGGCCTGTCCACTGAGAGCATCCT
GATCCCTAGGCAGAGCGAGACCTGCAGCCCCGGC

**SEQ ID NO: 233: anti-IGF1R Heavy Chain-GS-CT01 adnectin**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGG
GATCCGAGGTGGTGGCCGCCACCCCCACCAGCCTGCTGATTTCCTGGAGGCACCCCCACTTCCCCACACGCT
ACTACAGGATCACCTACGGCGAGACCGGCGGCAACAGCCCCGTGCAGGAGTTCACCGTGCCCCTGCAGCCTC
CCACTGCCACCATCAGCGGCCTCAAGCCCGGCGTGGACTACACCATCACCGTGTACGCCGTCACCGACGGAA
GGAACGGCAGGCTGCTGAGCATCCCCATCAGCATCAACTACAGGACC

**SEQ ID NO: 234: anti-IGF1R Heavy Chain-TVAAPSGS-TLPC**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC

CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGA
CCGTGGCCGCCCCCTCGGGATCCGACGGCGGCGGCATTAGGAGGAGCATGAGCGGCACCTGGTACCTGAAG
GCCATGACCGTGGATAGGGAGTTCCCCGAGATGAACCTGGAGAGCGTGACCCCCATGACACTGACCCTGCTC
AAGGGCCACAACCTGGAGGCCAAGGTCACCATGCTGATCTCAGGCAGGTGCCAGGAGGTGAAGGCAGTGCT
GGGCAGGACCAAGGAGAGGAAGAAGTACACCGCCGACGGGGGCAAGCACGTGGCCTATATCATCCCCAGCG
CCGTGAGGGACCACGTGATCTTCTACAGCGAGGGCCAGCTCCACGGAAAGCCCGTGAGAGGCGTGAAGCTG
GTGGGCAGGGACCCCAAGAACAACCTGGAGGCCCTGGAGGACTTCGAAAAAGCCGCAGGCGCCAGGGGCCT
GTCCACTGAGAGCATCCTGATCCCTAGGCAGAGCGAGACCTGCAGCCCCGGC

**SEQ ID NO: 235: anti-IGF1R Heavy Chain-GS-AFFI**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGG
GATCCGTGGACAACAAGTTCAACAAGGAGCTGAGGCAGGCCTACTGGGAGATCCAGGCCCTGCCCAATCTGA
ACTGGACCCAGAGCAGGGCCTTCATCAGGAGCCTGTACGACGACCCCAGCCAGAGCGCTAACCTCCTGGCCG
AGGCCAAAAAGCTGAACGACGCCCAGGCCCCCAAG

**SEQ ID NO: 236 anti-IGF1R Heavy Chain-TVAAPSGS-AFFI**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT

GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGA
CCGTGGCCGCCCCCTCGGGATCCGTGGACAACAAGTTCAACAAGGAGCTGAGGCAGGCCTACTGGGAGATC
CAGGCCCTGCCCAATCTGAACTGGACCCAGAGCAGGGCCTTCATCAGGAGCCTGTACGACGACCCCAGCCAG
AGCGCTAACCTCCTGGCCGAGGCCAAAAAGCTGAACGACGCCCAGGCCCCCAAG

**SEQ ID NO: 237: anti-IGF1R Heavy Chain-GS-DRPN**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGG
GATCCGACCTGGGGAAAAAGCTGCTTGAAGCCGCTAGGGCAGGACAGGATGACGAGGTGAGGATTCTGATG
GCAAATGGCGCCGACGTCAATGCCAAAGACGAGTACGGCCTCACCCCTCTTTATCTGGCCACTGCACACGGA
CACTTGGAGATCGTGGAGGTGCTGCTCAAGAACGGAGCTGATGTGAACGCTGTGGACGCTATTGGGTTCAC
ACCCCTTCACCTCGCAGCCTTTATTGGCCACCTGGAGATCGCCGAAGTTCTCCTGAAACACGGCGCAGACGT
CAACGCACAGGATAAGTTCGGGAAGACCGCCTTCGACATCAGCATCGGCAATGGGAACGAGGATCTGGCCGA
GATCCTGCAGAAGCTG

**SEQ ID NO: 238: anti-IGF1R Heavy Chain-TVAAPSGS-DRPN**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT

GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGA
CCGTGGCCGCCCCCTCGGGATCCGACCTGGGGAAAAAGCTGCTTGAAGCCGCTAGGGCAGGACAGGATGAC
GAGGTGAGGATTCTGATGGCAAATGGCGCCGACGTCAATGCCAAAGACGAGTACGGCCTCACCCCTCTTTAT
CTGGCCACTGCACACGGACACTTGGAGATCGTGGAGGTGCTGCTCAAGAACGGAGCTGATGTGAACGCTGT
GGACGCTATTGGGTTCACACCCCTTCACCTCGCAGCCTTTATTGGCCACCTGGAGATCGCCGAAGTTCTCCT
GAAACACGGCGCAGACGTCAACGCACAGGATAAGTTCGGGAAGACCGCCTTCGACATCAGCATCGGCAATGG
GAACGAGGATCTGGCCGAGATCCTGCAGAAGCTG

**SEQ ID NO: 239: Anti IL-4 heavy Chain-GS-anti RNAse A camelid$V_{HH}$**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGATC
CCAGGTGCAGCTGGTGGAGTCTGGGGGAGGCTTGGTGCAGGCTGGGGGGTCTCTGAGACTCTCCTGTGCAG
CCTCTGGATACGCATACACTTACATCTACATGGGCTGGTTCCGCCAGGCTCCAGGGAAAGAGCGTGAGGGGG
TCGCAGCTATGGATAGTGGTGGTGGTGGCACACTCTACGCCGACTCCGTGAAGGGCCGATTCACCATCTCCC
GCGACAAAGGCAAGAACACGGTGTATCTGCAAATGGACAGCCTGAAACCTGAGGACACGGCCACGTATTACT
GTGCTGCAGGTGGCTACGAGCTGCGTGACCGGACATATGGGCAGTGGGGCCAGGGGACCCAGGTCACCGTC
TCCTCA

**SEQ ID NO: 240: Anti IL-4 heavy Chain-GS-NARV**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT

CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGATC
CGCGCGCGTCGACCAGACGCCGCGCAGCGTCACGAAGGAAACCGGCGAGTCCCTCACCATCAACTGCGTGCT
GCGGGATGCCTCCTACGCCCTGGGCAGCACATGTTGGTACAGAAAGAAGAGCGGGGAAGGCAACGAGGAGT
CCATCTCCAAGGGGGGAAGATACGTCGAGACCGTGAACAGCGGAAGCAAGAGCTTCAGCCTGCGGATCAAC
GACCTCACCGTCGAGGACGGGGGCACCTACCGTTGCGGTCTGGGCGTGGCCGGCGGCTATTGCGATTACGC
CCTGTGCAGTAGCCGGTATGCTGAGTGCGGCGACGGCACCGCTGTGACCGTGAAC

**SEQ ID NO: 241 anti-IGF1R Heavy Chain-TVAAPSGS-CT01 adnectin**

CAGGTGCAGCTGGTGCAGAGCGGAGCCGAGGTGAAGAAGCCTGGCGCCAGCGTCAAGGTGTCCTGCAAGGC
CAGCGGCTACACCTTCACCGACTACTACATGAACTGGGTGCGGCAGGCCCCAGGCCAGGGACTGGAATGGAT
GGGCAACATCAACCCCAACAACGGCGGCACCAACTACAACCAGAAGTTCAAGGACCGGGTCACCATGACCAC
CGACACCAGCACCAGCACCGCCTACATGGAACTGCGGAGCCTGAGAAGCGACGACACCGCCGTGTACTACTG
CGCCCGGTGGATCCTGTACTACGGCCGGTCCAAGTGGTACTTCGACGTGTGGGGCAGGGGCACACTAGTGA
CCGTGTCCAGCGCCAGCACCAAGGGCCCCAGCGTGTTCCCCCTGGCCCCCAGCAGCAAGAGCACCAGCGGCG
GCACAGCCGCCCTGGGCTGCCTGGTGAAGGACTACTTCCCCGAACCGGTGACCGTGTCCTGGAACAGCGGA
GCCCTGACCAGCGGCGTGCACACCTTCCCCGCCGTGCTGCAGAGCAGCGGCCTGTACAGCCTGAGCAGCGT
GGTGACCGTGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCCTGCCCCCGA
GCTGCTGGGAGGCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTGGTACGTGGACG
GCGTGGAGGTGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAACAAGGCCCT
GCCTGCCCCTATCGAGAAAACCATCAGCAAGGCCAAGGGCCAGCCCAGAGAGCCCCAGGTGTACACCCTGCC
CCCTAGCAGAGATGAGCTGACCAAGAACCAGGTGTCCCTGACCTGCCTGGTGAAGGGCTTCTACCCCAGCGA
CATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAGA
CCGTGGCCGCCCCCTCGGGATCCGAGGTGGTGGCCGCCACCCCCACCAGCCTGCTGATTTCCTGGAGGCACC
CCCACTTCCCCACACGCTACTACAGGATCACCTACGGCGAGACCGGCGGCAACAGCCCCGTGCAGGAGTTCA
CCGTGCCCCTGCAGCCTCCCACTGCCACCATCAGCGGCCTCAAGCCCGGCGTGGACTACACCATCACCGTGT
ACGCCGTCACCGACGGAAGGAACGGCAGGCTGCTGAGCATCCCCATCAGCATCAACTACAGGACC

**SEQ ID NO: 242: Erbitux Heavy chain-RS-CT01 adnectin**

CAGGTGCAGCTGAAGCAGAGCGGCCCTGGCCTGGTGCAGCCCTCTCAGAGCCTGAGCATCACCTGTACCGT
GAGCGGCTTCAGCCTGACCAATTACGGCGTGCATTGGGTGCGGCAGTCTCCAGGCAAGGGCCTGGAATGGC
TGGGAGTGATCTGGTCCGGCGGCAACACCGACTACAACACCCCCTTCACCAGCAGACTGAGCATCAACAAGG
ACAACAGCAAGAGCCAGGTGTTCTTCAAGATGAACAGCCTGCAGAGCAACGACACCGCCATCTACTATTGTG
CCAGGGCCCTGACCTACTACGACTACGAGTTCGCCTACTGGGGCCAGGGCACCCTGGTGACCGTGAGCGCC

GCTAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAGCCTGTGACCGTGTCCTGGAATAGCGGAGCCCTGACCTC
CGGCGTGCACACCTTCCCCGCCGTGCTGCAGAGCAGCGGCCTGTACTCCCTGAGCAGCGTGGTGACCGTGC
CCAGCAGCAGCCTGGGCACCCAGACCTACATCTGCAACGTGAACCACAAGCCCAGCAACACCAAAGTGGACA
AGAAAGTGGAGCCCAAGAGCTGCGATAAGACCCACACCTGCCCCCCCTGCCCTGCCCCCGAGCTGCTGGGCG
GACCTAGCGTGTTCCTGTTCCCCCCCAAGCCTAAGGACACCCTGATGATCAGCAGGACCCCCGAAGTGACCT
GCGTGGTGGTGGATGTGAGCCACGAGGACCCTGAAGTGAAGTTCAACTGGTACGTGGACGGCGTGGAAGTG
CACAACGCCAAGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCCTGCCCCTATC
GAGAAAACCATCAGCAAGGCCAAGGGCCAGCCTAGAGAGCCCCAGGTCTACACCCTGCCTCCCTCCAGAGAT
GAGCTGACCAAGAACCAGGTGTCCCTGACCTGTCTGGTGAAGGGCTTCTACCCCAGCGACATCGCCGTGGAG
TGGGAGAGCAACGGCCAGCCCGAGAACAACTACAAGACCACCCCCCCTGTGCTGGACAGCGATGGCAGCTTC
TTCCTGTACTCCAAGCTGACCGTGGACAAGAGCAGATGGCAGCAGGGCAACGTGTTCAGCTGCAGCGTGATG
CACGAGGCCCTGCACAATCACTACACCCAGAAGAGTCTGAGCCTGTCTCCTGGCAAGAGATCCGAGGTGGTG
GCCGCCACCCCCACCAGCCTGCTGATTTCCTGGAGGCACCCCCACTTCCCCACACGCTACTACAGGATCACCT
ACGGCGAGACCGGCGGCAACAGCCCCGTGCAGGAGTTCACCGTGCCCCTGCAGCCTCCCACTGCCACCATCA
GCGGCCTCAAGCCCGGCGTGGACTACACCATCACCGTGTACGCCGTCACCGACGGAAGGAACGGCAGGCTG
CTGAGCATCCCCATCAGCATCAACTACAGGACC

**SEQ ID NO: 243: Erbitux Light Chain**

GACATCCTGCTGACCCAGAGCCCCGTGATCCTGAGCGTGAGCCCTGGCGAGAGAGTGAGCTTCAGCTGCCG
GGCCAGCCAGAGCATCGGCACCAACATCCACTGGTATCAGCAGCGGACCAACGGCAGCCCCAGGCTGCTGAT
CAAGTACGCCAGCGAGTCCATCAGCGGCATCCCCAGCCGGTTCAGCGGCAGCGGCTCCGGCACCGACTTCAC
CCTGAGCATCAACAGCGTGGAGAGCGAGGATATCGCCGACTACTACTGCCAGCAGAACAACAACTGGCCCAC
CACCTTCGGAGCCGGCACCAAGCTGGAACTGAAGCGTACGGTGGCCGCCCCCAGCGTGTTCATCTTCCCCCC
CAGCGATGAGCAGCTCAAGAGCGGCACCGCCAGCGTGGTGTGTCTGCTGAACAACTTCTACCCCCGGGAGG
CCAAAGTGCAGTGGAAAGTGGACAACGCCCTGCAGAGCGGCAACAGCCAGGAGAGCGTGACCGAGCAGGAC
AGCAAGGACTCCACCTACAGCCTGAGCAGCACCCTGACCCTGAGCAAGGCCGACTACGAGAAGCACAAAGTG
TACGCCTGCGAAGTGACCCACCAGGGCCTGTCCAGCCCCGTGACCAAGAGCTTCAACCGGGGCGAGTGC

**SEQ ID NO: 244: Erbitux Light chain-RS-CT01 adnectin**

GACATCCTGCTGACCCAGAGCCCCGTGATCCTGAGCGTGAGCCCTGGCGAGAGAGTGAGCTTCAGCTGCCG
GGCCAGCCAGAGCATCGGCACCAACATCCACTGGTATCAGCAGCGGACCAACGGCAGCCCCAGGCTGCTGAT
CAAGTACGCCAGCGAGTCCATCAGCGGCATCCCCAGCCGGTTCAGCGGCAGCGGCTCCGGCACCGACTTCAC
CCTGAGCATCAACAGCGTGGAGAGCGAGGATATCGCCGACTACTACTGCCAGCAGAACAACAACTGGCCCAC
CACCTTCGGAGCCGGCACCAAGCTGGAACTGAAGCGTACGGTGGCCGCCCCCAGCGTGTTCATCTTCCCCCC
CAGCGATGAGCAGCTCAAGAGCGGCACCGCCAGCGTGGTGTGTCTGCTGAACAACTTCTACCCCCGGGAGG
CCAAAGTGCAGTGGAAAGTGGACAACGCCCTGCAGAGCGGCAACAGCCAGGAGAGCGTGACCGAGCAGGAC
AGCAAGGACTCCACCTACAGCCTGAGCAGCACCCTGACCCTGAGCAAGGCCGACTACGAGAAGCACAAAGTG
TACGCCTGCGAAGTGACCCACCAGGGCCTGTCCAGCCCCGTGACCAAGAGCTTCAACCGAGGCGAGTGCAGA
TCCGAGGTGGTGGCCGCCACCCCCACCAGCCTGCTGATTTCCTGGAGGCACCCCCACTTCCCCACACGCTAC
TACAGGATCACCTACGGCGAGACCGGCGGCAACAGCCCCGTGCAGGAGTTCACCGTGCCCCTGCAGCCTCCC
ACTGCCACCATCAGCGGCCTCAAGCCCGGCGTGGACTACACCATCACCGTGTACGCCGTCACCGACGGAAGG
AACGGCAGGCTGCTGAGCATCCCCATCAGCATCAACTACAGGACC

**SEQ ID NO: 245: Erbitux Heavy Chain**

CAGGTGCAGCTGAAGCAGAGCGGCCCTGGCCTGGTGCAGCCCTCTCAGAGCCTGAGCATCACCTGTACCGT
GAGCGGCTTCAGCCTGACCAATTACGGCGTGCATTGGGTGCGGCAGTCTCCAGGCAAGGGCCTGGAATGGC
TGGGAGTGATCTGGTCCGGCGGCAACACCGACTACAACACCCCCTTCACCAGCAGACTGAGCATCAACAAGG
ACAACAGCAAGAGCCAGGTGTTCTTCAAGATGAACAGCCTGCAGAGCAACGACACCGCCATCTACTATTGTG
CCAGGGCCCTGACCTACTACGACTACGAGTTCGCCTACTGGGGCCAGGGCACCCTGGTGACCGTGAGCGCC
GCTAGCACCAAGGGCCCCAGCGTGTTCCCCCTGGCCCCCAGCAGCAAGAGCACCAGCGGCGGCACAGCCGC

CCTGGGCTGCCTGGTGAAGGACTACTTCCCCGAGCCTGTGACCGTGTCCTGGAATAGCGGAGCCCTGACCTC
CGGCGTGCACACCTTCCCCGCCGTGCTGCAGAGCAGCGGCCTGTACTCCCTGAGCAGCGTGGTGACCGTGC
CCAGCAGCAGCCTGGGCACCCAGACCTACATCTGCAACGTGAACCACAAGCCCAGCAACACCAAAGTGGACA
AGAAAGTGGAGCCCAAGAGCTGCGATAAGACCCACACCTGCCCCCCCTGCCCTGCCCCCGAGCTGCTGGGCG
GACCTAGCGTGTTCCTGTTCCCCCCCAAGCCTAAGGACACCCTGATGATCAGCAGGACCCCCGAAGTGACCT
GCGTGGTGGTGGATGTGAGCCACGAGGACCCTGAAGTGAAGTTCAACTGGTACGTGGACGGCGTGGAAGTG
CACAACGCCAAGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCCTGCCCCTATC
GAGAAAACCATCAGCAAGGCCAAGGGCCAGCCTAGAGAGCCCCAGGTCTACACCCTGCCTCCCTCCAGAGAT
GAGCTGACCAAGAACCAGGTGTCCCTGACCTGTCTGGTGAAGGGCTTCTACCCCAGCGACATCGCCGTGGAG
TGGGAGAGCAACGGCCAGCCCGAGAACAACTACAAGACCACCCCCCCTGTGCTGGACAGCGATGGCAGCTTC
TTCCTGTACTCCAAGCTGACCGTGGACAAGAGCAGATGGCAGCAGGGCAACGTGTTCAGCTGCAGCGTGATG
CACGAGGCCCTGCACAATCACTACACCCAGAAGAGTCTGAGCCTGTCCCCTGGCAAG

**SEQ ID NO: 246: CT01 adnectin-GSTG- Erbitux Heavy Chain**

GAGGTGGTGGCCGCCACCCCCACCAGCCTGCTGATTTCCTGGAGGCACCCCCACTTCCCCACACGCTACTAC
AGGATCACCTACGGCGAGACCGGCGGCAACAGCCCCGTGCAGGAGTTCACCGTGCCCCTGCAGCCTCCCACT
GCCACCATCAGCGGCCTCAAGCCCGGCGTGGACTACACCATCACCGTGTACGCCGTCACCGACGGAAGGAAC
GGCAGGCTGCTGAGCATCCCCATCAGCATCAACTACAGGACCGGATCCACCGGCCAGGTGCAGCTGAAGCAG
AGCGGCCCTGGCCTGGTGCAGCCCTCTCAGAGCCTGAGCATCACCTGTACCGTGAGCGGCTTCAGCCTGACC
AATTACGGCGTGCATTGGGTGCGGCAGTCTCCAGGCAAGGGCCTGGAATGGCTGGGAGTGATCTGGTCCGG
CGGCAACACCGACTACAACACCCCCTTCACCAGCAGACTGAGCATCAACAAGGACAACAGCAAGAGCCAGGT
GTTCTTCAAGATGAACAGCCTGCAGAGCAACGACACCGCCATCTACTATTGTGCCAGGGCCCTGACCTACTA
CGACTACGAGTTCGCCTACTGGGGCCAGGGCACCCTGGTGACCGTGAGCGCCGCTAGCACCAAGGGCCCCA
GCGTGTTCCCCCTGGCCCCCAGCAGCAAGAGCACCAGCGGCGGCACAGCCGCCCTGGGCTGCCTGGTGAAG
GACTACTTCCCCGAGCCTGTGACCGTGTCCTGGAATAGCGGAGCCCTGACCTCCGGCGTGCACACCTTCCCC
GCCGTGCTGCAGAGCAGCGGCCTGTACTCCCTGAGCAGCGTGGTGACCGTGCCCAGCAGCAGCCTGGGCAC
CCAGACCTACATCTGCAACGTGAACCACAAGCCCAGCAACACCAAAGTGGACAAGAAAGTGGAGCCCAAGAG
CTGCGATAAGACCCACACCTGCCCCCCCTGCCCTGCCCCCGAGCTGCTGGGCGGACCTAGCGTGTTCCTGTT
CCCCCCCAAGCCTAAGGACACCCTGATGATCAGCAGGACCCCCGAAGTGACCTGCGTGGTGGTGGATGTGAG
CCACGAGGACCCTGAAGTGAAGTTCAACTGGTACGTGGACGGCGTGGAAGTGCACAACGCCAAGACCAAGC
CCAGAGAGGAGCAGTACAACAGCACCTACCGCGTGGTGTCTGTGCTGACCGTGCTGCACCAGGATTGGCTG
AACGGCAAGGAGTACAAGTGCAAAGTGAGCAACAAGGCCCTGCCTGCCCCTATCGAGAAAACCATCAGCAAG
GCCAAGGGCCAGCCTAGAGAGCCCCAGGTCTACACCCTGCCTCCCTCCAGAGATGAGCTGACCAAGAACCAG
GTGTCCCTGACCTGTCTGGTGAAGGGCTTCTACCCCAGCGACATCGCCGTGGAGTGGGAGAGCAACGGCCA
GCCCGAGAACAACTACAAGACCACCCCCCCTGTGCTGGACAGCGATGGCAGCTTCTTCCTGTACTCCAAGCT
GACCGTGGACAAGAGCAGATGGCAGCAGGGCAACGTGTTCAGCTGCAGCGTGATGCACGAGGCCCTGCACA
ATCACTACACCCAGAAGAGTCTGAGCCTGTCCCCTGGCAAG

**SEQ ID NO: 247: CT01 adnectin-STG-Erbitux Light Chain**

GAGGTGGTGGCCGCCACCCCCACCAGCCTGCTGATTTCCTGGAGGCACCCCCACTTCCCCACACGCTACTAC
AGGATCACCTACGGCGAGACCGGCGGCAACAGCCCCGTGCAGGAGTTCACCGTGCCCCTGCAGCCTCCCACT
GCCACCATCAGCGGCCTCAAGCCCGGCGTGGACTACACCATCACCGTGTACGCCGTCACCGACGGAAGGAAC
GGCAGGCTGCTGAGCATCCCCATCAGCATCAACTACAGGACGTCGACCGGTGACATCCTGCTGACCCAGAGC
CCCGTGATCCTGAGCGTGAGCCCTGGCGAGAGAGTGAGCTTCAGCTGCCGGGCCAGCCAGAGCATCGGCAC
CAACATCCACTGGTATCAGCAGCGGACCAACGGCAGCCCCAGGCTGCTGATCAAGTACGCCAGCGAGTCCAT
CAGCGGCATCCCCAGCCGGTTCAGCGGCAGCGGCTCCGGCACCGACTTCACCCTGAGCATCAACAGCGTGGA
GAGCGAGGATATCGCCGACTACTACTGCCAGCAGAACAACAACTGGCCCACCACCTTCGGAGCCGGCACCAA
GCTGGAACTGAAGCGTACGGTGGCCGCCCCCAGCGTGTTCATCTTCCCCCCCAGCGATGAGCAGCTCAAGAG
CGGCACCGCCAGCGTGGTGTGTCTGCTGAACAACTTCTACCCCCGGGAGGCCAAAGTGCAGTGGAAAGTGG
ACAACGCCCTGCAGAGCGGCAACAGCCAGGAGAGCGTGACCGAGCAGGACAGCAAGGACTCCACCTACAGC

CTGAGCAGCACCCTGACCCTGAGCAAGGCCGACTACGAGAAGCACAAAGTGTACGCCTGCGAAGTGACCCAC
CAGGGCCTGTCCAGCCCCGTGACCAAGAGCTTCAACCGGGGCGAGTGC

**SEQ ID NO: 248** (PascoH-616 Heavy chain)

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGCGT
GCAGCTCCTGGAGAGCGGCGGAGGCCTGGTCCAGCCCGGCGGCAGCCTGAGGCTGAGCTGCGCCGCCAGCG
GCTTCGTGTTCCCCTGGTATGATATGGGCTGGGTGAGGCAGGCCCCCGGCAAGGGCCTGGAGTGGGTGTCC
AGCATCGACTGGCACGGGAAGATCACCTACTACGCCGACAGCGTGAAGGGCAGGTTCACCATCAGCAGGGA
CAACAGCAAGAACACCCTGTACCTGCAGATGAACAGCCTGAGGGCCGAGGACACCGCAGTGTACTACTGCGC
CACCGCCGAGGACGAACCCGGCTACGACTACTGGGGCCAGGGCACCCTGGTGACTGTGAGCAGC

**SEQ ID NO: 249** (PascoH-TVAAPS-616 Heavy chain)

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGACCGT
GGCCGCCCCCTCGGGCGTGCAG
CTCCTGGAGAGCGGCGGAGGCCTGGTCCAGCCCGGCGGCAGCCTGAGGCTGAGCTGCGCC

GCCAGCGGCTTCGTGTTCCCCTGGTATGATATGGGCTGGGTGAGGCAGGCCCCCGGCAAG
GGCCTGGAGTGGGTGTCCAGCATCGACTGGCACGGGAAGATCACCTACTACGCCGACAGC
GTGAAGGGCAGGTTCACCATCAGCAGGGACAACAGCAAGAACACCCTGTACCTGCAGATG
AACAGCCTGAGGGCCGAGGACACCGCAGTGTACTACTGCGCCACCGCCGAGGACGAACCC
GGCTACGACTACTGGGGCCAGGGCACCCTGGTGACTGTGAGCAGC

**SEQ ID NO: 250: 656H-TVAAPS-210 Heavy chain**

CAGGTGCAGCTCGTCCAGTCTGGGGCCGAGGTGAAGAAGCCCGGAGCTTCTGTGAAGGTGTCCTGCAAGGC
CAGCGGCTATACCTTCATCGACTACGAGATCCATTGGGTGAGGCAGGCTCCCGGGCAGGGCCTGGAGTGGA
TGGGCGCCATCGACCCAGAGACCGGAGGCACGGCGTACAACCAGAAGTTCAAGGGACGGGTCACCATGACA
ACCGATACCAGCACCTCCACCGCTTACATGGAGCTGCGCAGCCTGAGAAGCGACGACACCGCGGTGTACTAC
TGTACGCGCATCCTGCTCTACTACTACCCCATGGATTACTGGGGCCAGGGCACACTAGTGACCGTGTCTAGC
GCCAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAGCCCTGACCA
GCGGCGTGCACACCTTCCCCGCCGTGCTGCAGAGCAGCGGCCTGTACAGCCTGAGCAGCGTGGTGACCGTG
CCCAGCAGCAGCCTGGGCAcCCAGACCTACATCTgTAACgTgAACCACAaGCCCAGCAACACCAAGGTGGACA
AGAAGGTGGAGCCCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCCCGAGGTGACCT
GTGTGGTGGTGGATGTGAGCCACGAGGACCCTGAGGTGAAGTTCAACTGGTACGTGGACGGCGTGGAGGTG
CACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAGACCGTGGCCGCCCC
CTCGGAAGTGCAGCTCCTGGAGAGCGGCGGCGGCCTGGTGCAGCCCGGCGGCAGCCTGAGGCTGAGCTGC
GCCGCTAGCGGCTTCACCTTCAGGAACTTCGGCATGGGCTGGGTCAGGCAGGCCCCCGGCAAGGGCCTGGA
GTGGGTCAGCTGGATCATCAGCTCCGGCACCGAGACCTACTACGCCGACAGCGTGAAGGGCAGGTTCACCAT
CAGCCGCGACAACAGCAAGAACACCCTGTACCTGCAGATGAACAGCCTGAGGGCCGAGGACACCGCCGTCTA
CTACTGCGCCAAGAGCCTGGGCAGGTTCGACTACTGGGGACAGGGGACCCTGGTGACTGTGAGCAGC

**SEQ ID NO: 251: 656 Light chain**

GAGATCGTGCTGACCCAGAGTCCAGCCACCCTCAGCCTGAGCCCTGGGGAACGCGCCACCCTGTCCTGCCGG
GCGAGTCAGAACATCTCCGACTACCTGCATTGGTACCAGCAGAAGCCCGGCCAGGCCCCTCGCCTGCTGATC
TACTACGCCTCCCAGAGCATCAGCGGAATCCCCGCCCGGTTCTCCGGAAGTGGGTCCGGAACCGACTTTACC
CTGACCATCAGCTCTCTCGAGCCAGAGGACTTCGCGGTGTACTACTGCCAGAACGGGCATAGTTTCCCACTG
ACCTTCGGAGGGGGCACAAAGGTGGAGATCAAGCGTACGGTGGCCGCCCCCAGCGTGTTCATCTTCCCCCCC
AGCGATGAGCAGCTGAAGAGCGGCACCGCCAGCGTGGTGTGTCTGCTGAACAACTTCTACCCCCGGGAGGC
CAAGGTGCAGTGGAAGGTGGACAATGCCCTGCAGAGCGGCAACAGCCAGGAGAGCGTGACCGAGCAGGACA
GCAAGGACTCCACCTACAGCCTGAGCAGCACCCTGACCCTGAGCAAGGCCGACTACGAGAAGCACAAGGTGT
ACGCCTGTGAGGTGACCCACCAGGGCCTGTCCAGCCCCGTGACCAAGAGCTTCAACCGGGGCGAGTGC

**SEQ ID NO: 252:** PascoH-TVAAPS-546_Heavy chain

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT

GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGACCGT
GGCCGCCCCCTCGGGCGTGCAGCTCCTGGAGAGCGGCGGAGGCCTGGTCCAGCCCGGCGGCAGCCTGAGGC
TGAGCTGCGCCGCCAGCGGCTTCGTGTTCCCCTGGTATGATATGGGCTGGGTGAGGCAGGCCCCCGGCAAG
GGCCTGGAGTGGGTGTCCAGCATCGACTGGAAGGGGGGCAAGACCTACTACGCCGACAGCGTGAAGGGCAG
GTTCACCATCAGCAGGGACAACAGCAAGAACACCCTGTACCTGCAGATGAACAGCCTGAGGGCCGAGGACAC
CGCAGTGTACTACTGCGCCACCGCCGAGGACGAACCCGGCTACGACTACTGGGGCCAGGGCACCCTGGTGA
CTGTGAGCAGC

**SEQ ID NO: 253:** PascoH-546_Heavy chain

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGCGT
GCAGCTCCTGGAGAGCGGCGGAGGCCTGGTCCAGCCCGGCGGCAGCCTGAGGCTGAGCTGCGCCGCCAGCG
GCTTCGTGTTCCCCTGGTATGATATGGGCTGGGTGAGGCAGGCCCCCGGCAAGGGCCTGGAGTGGGTGTCC
AGCATCGACTGGAAGGGGGGCAAGACCTACTACGCCGACAGCGTGAAGGGCAGGTTCACCATCAGCAGGGA
CAACAGCAAGAACACCCTGTACCTGCAGATGAACAGCCTGAGGGCCGAGGACACCGCAGTGTACTACTGCGC
CACCGCCGAGGACGAACCCGGCTACGACTACTGGGGCCAGGGCACCCTGGTGACTGTGAGCAGC

**SEQ ID NO: 254:** PascoH-TVAAPS-567_Heavy chain

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT

CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGACCGT
GGCCGCCCCCTCGGGCGTGCAGCTCCTGGAGAGCGGCGGAGGCCTGGTCCAGCCCGGCGGCAGCCTGAGGC
TGAGCTGCGCCGCCAGCGGCTTCGTGTTCGCCTGGTATGATATGGGCTGGGTGAGGCAGGCCCCCGGCAAG
GGCCTGGAGTGGGTGTCCAGCATCGACTGGCACGGGGAGGTGACCTACTACGCCGACAGCGTGAAGGGCAG
GTTCACCATCAGCAGGGACAACAGCAAGAACACCCTGTACCTGCAGATGAACAGCCTGAGGGCCGAGGACAC
CGCAGTGTACTACTGCGCCACCGCCGAGGACGAACCCGGCTACGACTACTGGGGCCAGGGCACCCTGGTGA
CTGTGAGCAGC

**SEQ ID NO: 255:** (PascoH-567_Heavy chain)

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGCGT
GCAGCTCCTGGAGAGCGGCGGAGGCCTGGTCCAGCCCGGCGGCAGCCTGAGGCTGAGCTGCGCCGCCAGCG
GCTTCGTGTTCGCCTGGTATGATATGGGCTGGGTGAGGCAGGCCCCCGGCAAGGGCCTGGAGTGGGTGTCC
AGCATCGACTGGCACGGGGAGGTGACCTACTACGCCGACAGCGTGAAGGGCAGGTTCACC
ATCAGCAGGGACAACAGCAAGAACACCCTGTACCTGCAGATGAACAGCCTGAGGGCCGAG
GACACCGCAGTGTACTACTGCGCCACCGCCGAGGACGAACCCGGCTACGACTACTGGGGC
CAGGGCACCCTGGTGACTGTGAGCAGC

**SEQ ID NO: 256: 656 Heavy chain**

CAGGTGCAGCTCGTCCAGTCTGGGGCCGAGGTGAAGAAGCCCGGAGCTTCTGTGAAGGTGTCCTGCAAGGC
CAGCGGCTATACCTTCATCGACTACGAGATCCATTGGGTGAGGCAGGCTCCCGGGCAGGGCCTGGAGTGGA
TGGGCGCCATCGACCCAGAGACCGGAGGCACGGCGTACAACCAGAAGTTCAAGGGACGGGTCACCATGACA
ACCGATACCAGCACCTCCACCGCTTACATGGAGCTGCGCAGCCTGAGAAGCGACGACACCGCGGTGTACTAC
TGTACGCGCATCCTGCTCTACTACTACCCCATGGATTACTGGGGCCAGGGCACACTAGTCACAGTCTCCTCA
GCCTCCACCAAGGGCCCATCGGTCTTCCCCCTGGCACCCTCCTCCAAGAGCACCTCTGGGGGCACAGCGGCC
CTGGGCTGCCTGGTCAAGGACTACTTCCCCGAACCGGTGACGGTGTCGTGGAACTCAGGCGCCCTGACCAG
CGGCGTGCACACCTTCCCGGCTGTCCTACAGTCCTCAGGACTCTACTCCCTCAGCAGCGTGGTGACCGTGCC
CTCCAGCAGCTTGGGCACCCAGACCTACATCTGCAACGTGAATCACAAGCCCAGCAACACCAAGGTGGACAA
GAAAGTTGAGCCCAAATCTTGTGACAAAACTCACACATGCCCACCGTGCCCAGCACCTGAACTCCTGGGGGG
ACCGTCAGTCTTCCTCTTCCCCCCAAAACCCAAGGACACCCTCATGATCTCCCGGACCCCTGAGGTCACATGC
GTGGTGGTGGACGTGAGCCACGAAGACCCTGAGGTCAAGTTCAACTGGTACGTGGACGGCGTGGAGGTGCA
TAATGCCAAGACAAAGCCGCGGGAGGAGCAGTACAACAGCACGTACCGTGTGGTCAGCGTCCTCACCGTCCT
GCACCAGGACTGGCTGAATGGCAAGGAGTACAAGTGCAAGGTCTCCAACAAAGCCCTCCCAGCCCCCATCGA
GAAAACCATCTCCAAAGCCAAAGGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCCCAGCGACATCGCCGTGGAGTG
GGAGAGCAATGGGCAGCCGGAGAACAACTACAAGACCACGCCTCCCGTGCTGGACTCCGACGGCTCCTTCTT
CCTCTACAGCAAGCTCACCGTGGACAAGAGCAGGTGGCAGCAGGGGAACGTCTTCTCATGCTCCGTGATGCA
TGAGGCTCTGCACAACCACTACACGCAGAAGAGCCTCTCCCTGTCTCCGGGTAAA

**SEQ ID NO: 257:** Anti IL-5 Heavy Chain-G4S-dAb474-TVAAPSGS-dAb210

CAGGTGACCCTGAGGGAGAGCGGCCCCGCCCTGGTGAAGCCCACACAGACCCTCACTCTG
ACCTGCACCGTGAGCGGCTTCAGCCTGACCTCCTACAGCGTCCACTGGGTGAGGCAGCCC
CCCGGCAAGGGCCTGGAGTGGCTGGGCGTGATCTGGGCAAGCGGCGGCACCGACTACAACAGCGCCCTGAT
GAGCAGGCTCTCCATCAGCAAGGACACCAGCCGGAACCAGGTGGTGCTGACCATGACCAACATGGACCCCGT
GGACACCGCCACCTATTACTGCGCCAGGGACCCTCCCTCTAGCCTGCTGAGGCTGGACTACTGGGGCAGGG
GAACACTAGTGACCGTGTCCAGCGCCAGCACCAAGGGCCCCAGCGTGTTCCCCCTGGCCCCCAGCAGCAAGA
GCACCAGCGGCGGCACAGCCGCCCTGGGCTGCCTGGTGAAGGACTACTTCCCCGAACCGGTGACCGTGTCC
TGGAACAGCGGAGCCCTGACCAGCGGCGTGCACACCTTCCCCGCCGTGCTGCAGAGCAGCGGCCTGTACAG
CCTGAGCAGCGTGGTGACCGTGCCCAGCAGCAGCCTGGGCACCCAGACCTACATCTGTAACGTGAACCACAA
GCCCAGCAACACCAAGGTGGACAAGAAGGTGGAGCCCAAGAGCTGTGACAAGACCCACACCTGCCCCCCCTG
CCCTGCCCCCGAGCTGCTGGGAGGCCCCAGCGTGTTCCTGTTCCCCCCCAAGCCTAAGGACACCCTGATGAT
CAGCAGAACCCCCGAGGTGACCTGTGTGGTGGTGGATGTGAGCCACGAGGACCCTGAGGTGAAGTTCAACT
GGTACGTGGACGGCGTGGAGGTGCACAATGCCAAGACCAAGCCCAGGGAGGAGCAGTACAACAGCACCTAC
CGGGTGGTGTCCGTGCTGACCGTGCTGCACCAGGATTGGCTGAACGGCAAGGAGTACAAGTGTAAGGTGTC
CAACAAGGCCCTGCCTGCCCCTATCGAGAAAACCATCAGCAAGGCCAAGGGCCAGCCCAGAGAGCCCCAGGT
GTACACCCTGCCCCCTAGCAGAGATGAGCTGACCAAGAACCAGGTGTCCCTGACCTGCCTGGTGAAGGGCTT
CTACCCCAGCGACATCGCCGTGGAGTGGGAGAGCAACGGCCAGCCCGAGAACAACTACAAGACCACCCCCCC
TGTGCTGGACAGCGATGGCAGCTTCTTCCTGTACAGCAAGCTGACCGTGGACAAGAGCAGATGGCAGCAGG
GCAACGTGTTCAGCTGCTCCGTGATGCACGAGGCCCTGCACAATCACTACACCCAGAAGAGCCTGAGCCTGT
CCCCTGGCAAGGGCGGCGGCGGATCTGGCGTGCAGCTCCTGGAGAGCGGCGGAGGCCTGGTCCAGCCCGG
CGGCAGCCTGAGGCTGAGCTGCGCCGCCAGCGGCTTCACCTTCGCCTGGTATGATATGGGCTGGGTGAGGC
AGGCCCCCGGCAAGGGCCTGGAGTGGGTGTCCAGCATCGACTGGCACGGGGAGGTGACCTACTACGCCGAC
AGCGTGAAGGGCAGGTTCACCATCAGCAGGGACAACAGCAAGAACACCCTGTACCTGCAGATGAACAGCCTG
AGGGCCGAGGACACCGCAGTGTACTACTGCGCCACCGCCGAGGACGAACCCGGCTACGACTACTGGGGCCA
GGGCACCCTGGTGACTGTGAGCAGCACCGTGGCCGCCCCCTCGGGATCCGAAGTGCAGCTCCTGGAGAGCG
GCGGCGGCCTGGTGCAGCCCGGCGGCAGCCTGAGGCTGAGCTGCGCCGCTAGCGGCTTCACCTTCAGGAAC
TTCGGCATGGGCTGGGTCAGGCAGGCCCCCGGCAAGGGCCTGGAGTGGGTCAGCTGGATCATCAGCTCCGG
CACCGAGACCTACTACGCCGACAGCGTGAAGGGCAGGTTCACCATCAGCCGCGACAACAGCAAGAACACCCT

GTACCTGCAGATGAACAGCCTGAGGGCCGAGGACACCGCCGTCTACTACTGCGCCAAGAGCCTGGGCAGGT
TCGACTACTGGGGACAGGGGACCCTGGTGACTGTGAGCAGC

**SEQ ID NO: 258:** Anti CD-20 Heavy Chain-TVAAPSGS-dAb154-TVAAPSGS-dAb474

CAGGTGCAGCTGCAGCAGCCTGGAGCCGAGCTGGTGAAGCCCGGCGCCAGCGTGAAAATGTCCTGCAAGGC
CAGCGGCTACACCTTCACCAGCTACAACATGCACTGGGTGAAGCAGACCCCCGGCAGGGGCCTCGAGTGGAT
CGGAGCTATCTACCCCGGCAACGGCGACACTAGCTACAACCAGAAGTTCAAGGGCAAGGCCACCCTGACCGC
CGACAAGAGCAGCAGCACCGCCTACATGCAGCTGAGCAGCCTGACCAGCGAGGACAGCGCCGTGTATTACTG
CGCCAGGAGCACCTACTACGGCGGCGACTGGTACTTCAACGTCTGGGGCGCCGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT
GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGACCGT
GGCCGCCCCCTCGGGATCTGACATCCAGATGACCCAGAGCCCCAGCAGCCTGAGCGCCAGCGTGGGCGACA
GGGTGACCATTACCTGCAGGGCCAGCAGGCCCATCAGCGACTGGCTGCACTGGTACCAACAGAAGCCCGGC
AAGGCTCCCAAGCTGCTGATCGCCTGGGCCAGCAGCCTGCAGGGAGGCGTGCCCAGCAGG
TTTAGCGGCAGCGGCAGCGGCACCGACTTCACCCTCACCATCTCTTCCCTGCAGCCCGAG
GACTTCGCCACCTACTACTGCCTGCAGGAGGGCTGGGGGCCCCCTACTTTCGGCCAGGGC
ACCAAGGTGGAGATCAAGAGGACCGTGGCCGCCCCCTCGGGATCCGGCGTGCAGCTCCTG
GAGAGCGGCGGAGGCCTGGTCCAGCCCGGCGGCAGCCTGAGGCTGAGCTGCGCCGCCAGC
GGCTTCACCTTCGCCTGGTATGATATGGGCTGGGTGAGGCAGGCCCCCGGCAAGGGCCTG
GAGTGGGTGTCCAGCATCGACTGGCACGGGGAGGTGACCTACTACGCCGACAGCGTGAAG
GGCAGGTTCACCATCAGCAGGGACAACAGCAAGAACACCCTGTACCTGCAGATGAACAGC
CTGAGGGCCGAGGACACCGCAGTGTACTACTGCGCCACCGCCGAGGACGAACCCGGCTAC
GACTACTGGGGCCAGGGCACCCTGGTGACTGTGAGCAGC

**SEQ ID NO: 259: Anti CD-20 Heavy Chain-TVAAPSGS-dAb210-TVAAPSGS-dAb474**

CAGGTGCAGCTGCAGCAGCCTGGAGCCGAGCTGGTGAAGCCCGGCGCCAGCGTGAAAATG
TCCTGCAAGGCCAGCGGCTACACCTTCACCAGCTACAACATGCACTGGGTGAAGCAGACC
CCCGGCAGGGGCCTCGAGTGGATCGGAGCTATCTACCCCGGCAACGGCGACACTAGCTAC
AACCAGAAGTTCAAGGGCAAGGCCACCCTGACCGCCGACAAGAGCAGCAGCACCGCCTAC
ATGCAGCTGAGCAGCCTGACCAGCGAGGACAGCGCCGTGTATTACTGCGCCAGGAGCACC
TACTACGGCGGCGACTGGTACTTCAACGTCTGGGGCGCCGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAACGTGAACCAC
AAGCCCAGCAACACCAAGGTGGACAAGAAGGTGGAGCCCAAGAGCTGTGACAAGACCCACACCTGCCCCCCC
TGCCCTGCCCCCGAGCTGCTGGGAGGCCCCAGCGTGTTCCTGTTCCCCCCCAAGCCTAAGGACACCCTGATG
ATCAGCAGAACCCCCGAGGTGACCTGTGTGGTGGTGGATGTGAGCCACGAGGACCCTGAGGTGAAGTTCAA

CTGGTACGTGGACGGCGTGGAGGTGCACAATGCCAAGACCAAGCCCAGGGAGGAGCAGTACAACAGCACCT
ACCGGGTGGTGTCCGTGCTGACCGTGCTGCACCAGGATTGGCTGAACGGCAAGGAGTACAAGTGTAAGGTG
TCCAACAAGGCCCTGCCTGCCCCTATCGAGAAAACCATCAGCAAGGCCAAGGGCCAGCCCAGAGAGCCCCAG
GTGTACACCCTGCCCCCTAGCAGAGATGAGCTGACCAAGAACCAGGTGTCCCTGACCTGCCTGGTGAAGGGC
TTCTACCCCAGCGACATCGCCGTGGAGTGGGAGAGCAACGGCCAGCCCGAGAACAACTACAAGACCACCCCC
CCTGTGCTGGACAGCGATGGCAGCTTCTTCCTGTACAGCAAGCTGACCGTGGACAAGAGCAGATGGCAGCA
GGGCAACGTGTTCAGCTGCTCCGTGATGCACGAGGCCCTGCACAATCACTACACCCAGAAGAGCCTGAGCCT
GTCCCCTGGCAAGACCGTGGCCGCCCCCTCGGGATCTGAAGTGCAGCTCCTGGAGAGCGGCGGCGGCCTGG
TGCAGCCCGGCGGCAGCCTGAGGCTGAGCTGCGCCGCTAGCGGCTTCACCTTCAGGAACTTCGGCATGGGC
TGGGTCAGGCAGGCCCCCGGCAAGGGCCTGGAGTGGGTCAGCTGGATCATCAGCTCCGGCACCGAGACCTA
CTACGCCGACAGCGTGAAGGGCAGGTTCACCATCAGCCGCGACAACAGCAAGAACACCCTGTACCTGCAGAT
GAACAGCCTGAGGGCCGAGGACACCGCCGTCTACTACTGCGCCAAGAGCCTGGGCAGGTTCGACTACTGGG
GACAGGGGACCCTGGTGACTGTGAGCAGCACCGTGGCCGCCCCCTCGGGATCCGGCGTGCAGCTCCTGGAG
AGCGGCGGAGGCCTGGTCCAGCCCGGCGGCAGCCTGAGGCTGAGCTGCGCCGCCAGCGGCTTCACCTTCGC
CTGGTATGATATGGGCTGGGTGAGGCAGGCCCCCGGCAAGGGCCTGGAGTGGGTGTCCAGCATCGACTGGC
ACGGGGAGGTGACCTACTACGCCGACAGCGTGAAGGGCAGGTTCACCATCAGCAGGGACAACAGCAAGAAC
ACCCTGTACCTGCAGATGAACAGCCTGAGGGCCGAGGACACCGCAGTGTACTACTGCGCCACCGCCGAGGAC
GAACCCGGCTACGACTACTGGGGCCAGGGCACCCTGGTGACTGTGAGCAGC

**SEQ ID NO: 260:** anti cMET 5D5v2 Heavy Chain (hole)-GS-dAb593

GAGGTGCAGCTGGTGGAAAGCGGCGGCGGCCTGGTGCAGCCCGGCGGCTCCCTGAGGCTGAGCTGCGCCG
CTAGCGGCTACACCTTCACCAGCTACTGGCTCCACTGGGTCAGGCAGGCCCCAGGCAAGGGACTGGAGTGG
GTGGGCATGATCGACCCCAGCAACAGCGACACCAGGTTCAACCCCAACTTCAAGGACAGGTTCACCATCAGC
GCCGACACTAGCAAGAACACCGCCTACCTGCAGATGAACAGCCTGAGGGCCGAGGACACCGCCGTGTATTAC
TGCGCCACCTACAGGAGCTACGTCACCCCCCTGGATTACTGGGGCCAGGGCACACTAGTGACCGTGTCCAGC
GCCAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAGCCCTGACCA
GCGGCGTGCACACCTTCCCCGCCGTGCTGCAGAGCAGCGGCCTGTACAGCCTGAGCAGCGTGGTGACCGTG
CCCAGCAGCAGCCTGGGCACCCAGACCTACATCTGTAACGTGAACCACAAGCCCAGCAACACCAAGGTGGAC
AAGAAGGTGGAGCCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCG
TGCTGCACCAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCCCTAGCAGAG
ATGAGCTGACCAAGAACCAGGTGTCCCTGAGCTGCGCCGTGAAGGGCTTCTACCCCAGCGACATCGCCGTGG
AGTGGGAGAGCAACGGCCAGCCCGAGAACAACTACAAGACCACCCCCCCTGTGCTGGACAGCGATGGCAGCT
TCTTCCTGGTGAGCAAGCTGACCGTGGACAAGAGCAGATGGCAGCAGGGCAACGTGTTCAGCTGCTCCGTG
ATGCACGAGGCCCTGCACAATCACTACACCCAGAAGAGCCTGAGCCTGTCCCCTGGCAAGGGATCCGAGGTG
CAGCTCCTGGTCAGCGGCGGCGGCCTGGTCCAGCCCGGAGGCTCACTGAGGCTGAGCTGCGCCGCTAGCGG
CTTCACCTTCAAGGCCTACCCCATGATGTGGGTCAGGCAGGCCCCCGGCAAAGGCCTGGAGTGGGTGTCTGA
GATCAGCCCCAGCGGCAGCTACACCTACTACGCCGACAGCGTGAAGGGCAGGTTCACCATCAGCAGGGACAA
CAGCAAGAACACCCTGTACCTGCAGATGAACTCTCTGAGGGCCGAGGACACCGCCGTGTACTACTGCGCCAA
GGACCCCAGGAAGCTGGACTATTGGGGCCAGGGCACTCTGGTGACCGTGAGCAGC

**SEQ ID NO: 261:** anti cMET 5D5v2 Heavy Chain (knob)-GS-dAb593

TGCCCCCCCTGCCCTGCCCCCGAGCTGCTGGGAGGCCCCAGCGTGTTCCTGTTCCCCCCCAAGCCTAAGGAC
ACCCTGATGATCAGCAGAACCCCCGAGGTGACCTGTGTGGTGGTGGATGTGAGCCACGAGGACCCTGAGGT
GAAGTTCAACTGGTACGTGGACGGCGTGGAGGTGCACAATGCCAAGACCAAGCCCAGGGAGGAGCAGTACA
ACAGCACCTACCGGGTGGTGTCCGTGCTGACCGTGCTGCACCAGGATTGGCTGAACGGCAAGGAGTACAAG
TGTAAGGTGTCCAACAAGGCCCTGCCTGCCCCTATCGAGAAAACCATCAGCAAGGCCAAGGGCCAGCCCAGA

GAGCCCCAGGTGTACACCCTGCCCCCTAGCAGAGATGAGCTGACCAAGAACCAGGTGTCCCTGTGGTGCCTG
GTGAAGGGCTTCTACCCCAGCGACATCGCCGTGGAGTGGGAGAGCAACGGCCAGCCCGAGAACAACTACAA
GACCACCCCCCCTGTGCTGGACAGCGATGGCAGCTTCTTCCTGTACAGCAAGCTGACCGTGGACAAGAGCAG
ATGGCAGCAGGGCAACGTGTTCAGCTGCTCCGTGATGCACGAGGCCCTGCACAATCACTACACCCAGAAGAG
CCTGAGCCTGTCCCCTGGCAAGGGATCCGAGGTGCAGCTCCTGGTCAGCGGCGGCGGCCTGGTCCAGCCCG
GAGGCTCACTGAGGCTGAGCTGCGCCGCTAGCGGCTTCACCTTCAAGGCCTACCCCATGATGTGGGTCAGGC
AGGCCCCCGGCAAAGGCCTGGAGTGGGTGTCTGAGATCAGCCCCAGCGGCAGCTACACCTACTACGCCGAC
AGCGTGAAGGGCAGGTTCACCATCAGCAGGGACAACAGCAAGAACACCCTGTACCTGCAGATGAACTCTCTG
AGGGCCGAGGACACCGCCGTGTACTACTGCGCCAAGGACCCCAGGAAGCTGGACTATTGGGGCCAGGGCAC
TCTGGTGACCGTGAGCAGC

**SEQ ID NO: 262:** anti cMET 5D5v2 Light Chain

GACATCCAGATGACCCAGAGCCCCAGCAGCCTGAGCGCCTCAGTGGGAGACAGGGTGACCATCACCTGCAAG
AGCAGCCAGAGCCTCCTGTACACCAGCAGCCAGAAGAACTACCTGGCCTGGTACCAGCAGAAACCCGGCAAG
GCCCCCAAGCTGCTGATCTACTGGGCTAGCACCAGGGAGTCAGGCGTGCCCAGCAGGTTCAGCGGCAGCGG
CAGCGGCACCGACTTCACTCTGACCATCAGCAGCCTGCAGCCCGAGGACTTCGCCACCTACTACTGCCAGCA
GTACTACGCCTATCCCTGGACCTTCGGCCAGGGCACCAAGGTGGAGATCAAGCGTACGGTGGCCGCCCCCAG
CGTGTTCATCTTCCCCCCCAGCGATGAGCAGCTGAAGAGCGGCACCGCCAGCGTGGTGTGTCTGCTGAACAA
CTTCTACCCCCGGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGAGCAAGGCCGACT
ACGAGAAGCACAAGGTGTACGCCTGTGAGGTGACCCACCAGGGCCTGTCCAGCCCCGTGACCAAGAGCTTCA
ACCGGGGCGAGTGC

**SEQ ID NO: 263:** anti cMET 5D5v2 IgG4 Heavy Chain (UNIBODY)-GS-dAb593

GAGGTGCAGCTGGTGGAAAGCGGCGGCGGCCTGGTGCAGCCCGGCGGCTCCCTGAGGCTGAGCTGCGCCG
CTAGCGGCTACACCTTCACCAGCTACTGGCTCCACTGGGTCAGGCAGGCCCCAGGCAAGGGACTGGAGTGG
GTGGGCATGATCGACCCCAGCAACAGCGACACCAGGTTCAACCCCAACTTCAAGGACAGGTTCACCATCAGC
GCCGACACTAGCAAGAACACCGCCTACCTGCAGATGAACAGCCTGAGGGCCGAGGACACCGCCGTGTATTAC
TGCGCCACCTACAGGAGCTACGTCACCCCCCTGGATTACTGGGGCCAGGGCACACTAGTCACCGTGAGCAGC
GCCAGCACCAAGGGCCCCAGCGTGTTCCCCCTGGCCCCCTGCAGCAGAAGCACCAGCGAGAGCACAGCCGCC
CTGGGCTGCCTGGTGAAGGACTACTTCCCCGAGCCCGTGACCGTGAGCTGGAACAGCGGAGCCCTGACCAG
CGGCGTGCACACCTTCCCCGCCGTGCTGCAGAGCAGCGGCCTGTACAGCCTGAGCAGCGTGGTGACCGTGC
CCAGCAGCAGCCTGGGCACCAAGACCTACACCTGCAACGTGGACCACAAGCCCAGCAACACCAAGGTGGACA
AGCGGGTGGCCCCCGAGTTCCTGGGCGGACCCTCCGTGTTCCTGTTCCCCCCCAAGCCCAAGGACACCCTGA
TGATCAGCCGGACCCCCGAGGTGACCTGCGTGGTGGTGGACGTGAGCCAGGAAGATCCCGAGGTCCAGTTC
AATTGGTACGTGGACGGCGTGGAGGTGCACAACGCCAAGACCAAGCCCCGGGAGGAACAGTTCAACAGCAC
CTACCGGGTGGTGTCCGTGCTGACCGTGCTGCACCAGGACTGGCTGAACGGCAAAGAATACAAGTGCAAGG
TGTCCAACAAGGGCCTGCCCAGCTCCATCGAGAAAACCATCAGCAAGGCCAAGGGCCAGCCTCGGGAGCCCC
AGGTGTACACCCTGCCCCCATCCCAGGAAGAGATGACCAAGAACCAGGTGTCCCTGACCTGTCTGGTGAAGG
GCTTCTACCCCAGCGACATCGCCGTGGAGTGGGAGAGCAACGGCCAGCCCGAGAACAACTACAAGACCACCC
CCCCTGTGCTGGACAGCGACGGCAGCTTCTTCCTGTACAGCAGGCTGACCGTGGACAAGAGCCGGTGGCAG
GAAGGCAACGTCTTTAGCTGCAGCGTGATGCACGAGGCCCTGCACAACCACTACACCCAGAAGAGCCTGAGC
CTGTCCCTGGGCAAGGGATCCGAGGTGCAGCTCCTGGTCAGCGGCGGCGGCCTGGTCCAGCCCGGAGGCTC
ACTGAGGCTGAGCTGCGCCGCTAGCGGCTTCACCTTCAAGGCCTACCCCATGATGTGGGTCAGGCAGGCCCC
CGGCAAAGGCCTGGAGTGGGTGTCTGAGATCAGCCCCAGCGGCAGCTACACCTACTACGCCGACAGCGTG
AAGGGCAGGTTCACCATCAGCAGGGACAACAGCAAGAACACCCTGTACCTGCAGATGAACTCTCTGAGGGCC
GAGGACACCGCCGTGTACTACTGCGCCAAGGACCCCAGGAAGCTGGACTATTGGGGCCAGGGCACTCTGGT
GACCGTGAGCAGC

**SEQ ID NO: 264:** anti cMET 5D5v2 Heavy Chain (hole)

GAGGTGCAGCTGGTGGAAAGCGGCGGCGGCCTGGTGCAGCCCGGCGGCTCCCTGAGGCTGAGCTGCGCCG
CTAGCGGCTACACCTTCACCAGCTACTGGCTCCACTGGGTCAGGCAGGCCCCAGGCAAGGGACTGGAGTGG

GTGGGCATGATCGACCCCAGCAACAGCGACACCAGGTTCAACCCCAACTTCAAGGACAGGTTCACCATCAGC
GCCGACACTAGCAAGAACACCGCCTACCTGCAGATGAACAGCCTGAGGGCCGAGGACACCGCCGTGTATTAC
TGCGCCACCTACAGGAGCTACGTCACCCCCCTGGATTACTGGGGCCAGGGCACACTAGTGACCGTGTCCAGC
GCCAGCACCAAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAGCCCTGACCA
GCGGCGTGCACACCTTCCCCGCCGTGCTGCAGAGCAGCGGCCTGTACAGCCTGAGCAGCGTGGTGACCGTG
CCCAGCAGCAGCCTGGGCACCCAGACCTACATCTGTAACGTGAACCACAAGCCCAGCAACACCAAGGTGGAC
AAGAAGGTGGAGCCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCG
TGCTGCACCAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCCCTAGCAGAG
ATGAGCTGACCAAGAACCAGGTGTCCCTGAGCTGCGCCGTGAAGGGCTTCTACCCCAGCGACATCGCCGTGG
AGTGGGAGAGCAACGGCCAGCCCGAGAACAACTACAAGACCACCCCCCCTGTGCTGGACAGCGATGGCAGCT
TCTTCCTGGTGAGCAAGCTGACCGTGGACAAGAGCAGATGGCAG
CAGGGCAACGTGTTCAGCTGCTCCGTGATGCACGAGGCCCTGCACAATCACTACACCCAG
AAGAGCCTGAGCCTGTCCCCTGGCAAG

**SEQ ID NO: 265:** anti cMET 5D5v2 Heavy Chain (knob)

TGCCCCCCCTGCCCTGCCCCCGAGCTGCTGGGAGGCCCCAGCGTGTTCCTGTTCCCCCCC
AAGCCTAAGGACACCCTGATGATCAGCAGAACCCCCGAGGTGACCTGTGTGGTGGTGGAT
GTGAGCCACGAGGACCCTGAGGTGAAGTTCAACTGGTACGTGGACGGCGTGGAGGTGCAC
AATGCCAAGACCAAGCCCAGGGAGGAGCAGTACAACAGCACCTACCGGGTGGTGTCCGTG
CTGACCGTGCTGCACCAGGATTGGCTGAACGGCAAGGAGTACAAGTGTAAGGTGTCCAAC
AAGGCCCTGCCTGCCCCTATCGAGAAAACCATCAGCAAGGCCAAGGGCCAGCCCAGAGAG
CCCCAGGTGTACACCCTGCCCCCTAGCAGAGATGAGCTGACCAAGAACCAGGTGTCCCTG
TGGTGCCTGGTGAAGGGCTTCTACCCCAGCGACATCGCCGTGGAGTGGGAGAGCAACGGC
CAGCCCGAGAACAACTACAAGACCACCCCCCCTGTGCTGGACAGCGATGGCAGCTTCTTC
CTGTACAGCAAGCTGACCGTGGACAAGAGCAGATGGCAGCAGGGCAACGTGTTCAGCTGC
TCCGTGATGCACGAGGCCCTGCACAATCACTACACCCAGAAGAGCCTGAGCCTGTCCCCT
GGCAAG

**SEQ ID NO: 266:** anti cMET 5D5v2 IgG4 Heavy Chain (UNIBODY)

GAGGTGCAGCTGGTGGAAAGCGGCGGCGGCCTGGTGCAGCCCGGCGGCTCCCTGAGGCTG
AGCTGCGCCGCTAGCGGCTACACCTTCACCAGCTACTGGCTCCACTGGGTCAGGCAGGCC
CCAGGCAAGGGACTGGAGTGGGTGGGCATGATCGACCCCAGCAACAGCGACACCAGGTTC
AACCCCAACTTCAAGGACAGGTTCACCATCAGCGCCGACACTAGCAAGAACACCGCCTAC
CTGCAGATGAACAGCCTGAGGGCCGAGGACACCGCCGTGTATTACTGCGCCACCTACAGG
AGCTACGTCACCCCCCTGGATTACTGGGGCCAGGGCACACTAGTCACCGTGAGCAGCGCC
AGCACCAAGGGCCCCAGCGTGTTCCCCCTGGCCCCCTGCAGCAGAAGCACCAGCGAGAGC
ACAGCCGCCCTGGGCTGCCTGGTGAAGGACTACTTCCCCGAGCCCGTGACCGTGAGCTGGAACAGCGGAGC
CCTGACCAGCGGCGTGCACACCTTCCCCGCCGTGCTGCAGAGCAGCGGCCTGTACAGCCTGAGCAGCGTGG
TGACCGTGCCCAGCAGCAGCCTGGGCACCAAGACCTACACCTGCAACGTGGACCACAAGCCCAGCAACACCA
AGGTGGACAAGCGGGTGGCCCCCGAGTTCCTGGGCGGACCCTCCGTGTTCCTGTTCCCCCCCAAGCCCAAG
GACACCCTGATGATCAGCCGGACCCCCGAGGTGACCTGCGTGGTGGTGGACGTGAGCCAGGAAGATCCCGA
GGTCCAGTTCAATTGGTACGTGGACGGCGTGGAGGTGCACAACGCCAAGACCAAGCCCCGGGAGGAACAGT
TCAACAGCACCTACCGGGTGGTGTCCGTGCTGACCGTGCTGCACCAGGACTGGCTGAACGGCAAAGAATACA
AGTGCAAGGTGTCCAACAAGGGCCTGCCCAGCTCCATCGAGAAAACCATCAGCAAGGCCAAGGGCCAGCCTC

GGGAGCCCCAGGTGTACACCCTGCCCCCATCCCAGGAAGAGATGACCAAGAACCAGGTGTCCCTGACCTGTC
TGGTGAAGGGCTTCTACCCCAGCGACATCGCCGTGGAGTGGGAGAGCAACGGCCAGCCCGAGAACAACTAC
AAGACCACCCCCCCTGTGCTGGACAGCGACGGCAGCTTCTTCCTGTACAGCAGGCTGACCGTGGACAAGAGC
CGGTGGCAGGAAGGCAACGTCTTTAGCTGCAGCGTGATGCACGAGGCCCTGCACAACCACTACACCCAGAAG
AGCCTGAGCCTGTCCCTGGGCAAG

**SEQ ID NO: 267: PascoH IgG2-GS-474 heavy chain**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTCACCGTGA
GCAGCGCCAGCACCAAGGGCCCCAGCGTGTTCCCCCTGGCCCCCTGCAGCAGAAGCACCAGCGAGAGCACA
GCCGCCCTGGGCTGCCTGGTGAAGGACTACTTCCCCGAGCCCGTGACCGTGAGCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAACTTCGGCACCCAGACCTACACCTGCAACGTGGACCACAAGCCCAGCAACACCAAGG
TGGACAAGACCGTGGAGCGGAAGTGCTGCGTGGAGTGCCCCCCCTGCCCTGCCCCTCCTGTGGCCGGACCC
TCCGTGTTCCTGTTCCCCCCCAAGCCCAAGGACACCCTGATGATCAGCCGGACCCCCGAGGTGACCTGCGTG
GTGGTGGACGTGAGCCACGAGGACCCCGAGGTGCAGTTCAATTGGTACGTGGACGGCGTGGAGGTGCACAA
CGCCAAGACCAAGCCCCGGGAGGAACAGTTCAACAGCACCTTCCGGGTGGTGTCCGTGCTGACCGTGGTGC
ACCAGGACTGGCTGAACGGCAAAGAATACAAGTGCAAGGTGTCCAACAAGGGCCTGCCTGCCCCCATCGAGA
AAACCATCAGCAAGACCAAGGGCCAGCCCAGGGAACCCCAGGTGTACACCCTGCCCCCCAGCCGGGAGGAAA
TGACCAAGAACCAGGTGTCCCTGACCTGTCTGGTGAAGGGCTTCTACCCCAGCGACATCGCCGTGGAGTGGG
AGAGCAACGGCCAGCCCGAGAACAACTACAAGACCACCCCCCCCATGCTGGACAGCGACGGCAGCTTCTTCC
TGTACAGCAAGCTGACAGTGGACAAGAGCCGGTGGCAGCAGGGCAACGTGTTCAGCTGCAGCGTGATGCAC
GAGGCCCTGCACAACCACTACACCCAGAAGAGCCTGAGCCTGTCCCCCGGCAAGGGATCCGGCGTGCAGCTC
CTGGAGAGCGGCGGAGGCCTGGTCCAGCCCGGCGGCAGCCTGAGGCTGAGCTGCGCCGCCAGCGGCTTCAC
CTTCGCCTGGTATGATATGGGCTGGGTGAGGCAGGCCCCCGGCAAGGGCCTGGAGTGGGTGTCCAGCATCG
ACTGGCACGGGGAGGTGACCTACTACGCCGACAGCGTGAAGGGCAGGTTCACCATCAGCAGGGACAACAGC
AAGAACACCCTGTACCTGCAGATGAACAGCCTGAGGGCCGAGGACACCGCAGTGTACTACTGCGCCACCGCC
GAGGACGAACCCGGCTACGACTACTGGGGCCAGGGCACCCTGGTGACTGTGAGCAGC

**SEQ ID NO: 268: PascoH IgG4-GS-474 heavy chain**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTCACCGTGA
GCAGCGCCAGCACCAAGGGCCCCAGCGTGTTCCCCCTGGCCCCCTGCAGCAGAAGCACCAGCGAGAGCACA
GCCGCCCTGGGCTGCCTGGTGAAGGACTACTTCCCCGAGCCCGTGACCGTGAGCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCAAGACCTACACCTGCAACGTGGACCACAAGCCCAGCAACACCAAGG
TGGACAAGCGGGTGGAGAGCAAGTACGGCCCTCCCTGCCCCAGCTGCCCTGCCCCCGAGTTCCTGGGCGGA
CCCTCCGTGTTCCTGTTCCCCCCCAAGCCCAAGGACACCCTGATGATCAGCCGGACCCCCGAGGTGACCTGC
GTGGTGGTGGACGTGAGCCAGGAAGATCCCGAGGTCCAGTTCAATTGGTACGTGGACGGCGTGGAGGTGCA
CAACGCCAAGACCAAGCCCCGGGAGGAACAGTTCAACAGCACCTACCGGGTGGTGTCCGTGCTGACCGTGCT
GCACCAGGACTGGCTGAACGGCAAAGAATACAAGTGCAAGGTGTCCAACAAGGGCCTGCCCAGCTCCATCGA
GAAAACCATCAGCAAGGCCAAGGGCCAGCCTCGGGAGCCCCAGGTGTACACCCTGCCCCCATCCCAGGAAGA
GATGACCAAGAACCAGGTGTCCCTGACCTGTCTGGTGAAGGGCTTCTACCCCAGCGACATCGCCGTGGAGTG
GGAGAGCAACGGCCAGCCCGAGAACAACTACAAGACCACCCCCCCTGTGCTGGACAGCGACGGCAGCTTCTT
CCTGTACAGCAGGCTGACCGTGGACAAGAGCCGGTGGCAGGAAGGCAACGTCTTTAGCTGCAGCGTGATGC

ACGAGGCCCTGCACAACCACTACACCCAGAAGAGCCTGAGCCTGTCCCTGGGCAAGGGATCCGGCGTGCAGC
TCCTGGAGAGCGGCGGAGGCCTGGTCCAGCCCGGCGGCAGCCTGAGGCTGAGCTGCGCCGCCAGCGGCTTC
ACCTTCGCCTGGTATGATATGGGCTGGGTGAGGCAGGCCCCCGGCAAGGGCCTGGAGTGGGTGTCCAGCAT
CGACTGGCACGGGGAGGTGACCTACTACGCCGACAGCGTGAAGGGCAGGTTCACCATCAGCAGGGACAACA
GCAAGAACACCCTGTACCTGCAGATGAACAGCCTGAGGGCCGAGGACACCGCAGTGTACTACTGCGCCACCG
CCGAGGACGAACCCGGCTACGACTACTGGGGCCAGGGCACCCTGGTGACTGTGAGCAGC

**SEQ ID NO: 269: PascoH IgG4PE-GS-474 heavy chain**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTCACCGTGA
GCAGCGCCAGCACCAAGGGCCCCAGCGTGTTCCCCCTGGCCCCCTGCAGCAGAAGCACCAGCGAGAGCACA
GCCGCCCTGGGCTGCCTGGTGAAGGACTACTTCCCCGAGCCCGTGACCGTGAGCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCAAGACCTACACCTGCAACGTGGACCACAAGCCCAGCAACACCAAGG
TGGACAAGCGGGTGGAGAGCAAGTACGGCCCTCCCTGCCCCCCCTGCCCTGCCCCCGAGTTCGAGGGCGGA
CCCTCCGTGTTCCTGTTCCCCCCCAAGCCCAAGGACACCCTGATGATCAGCCGGACCCCCGAGGTGACCTGC
GTGGTGGTGGACGTGAGCCAGGAAGATCCCGAGGTCCAGTTCAATTGGTACGTGGACGGCGTGGAGGTGCA
CAACGCCAAGACCAAGCCCCGGGAGGAACAGTTCAACAGCACCTACCGGGTGGTGTCCGTGCTGACCGTGCT
GCACCAGGACTGGCTGAACGGCAAAGAATACAAGTGCAAGGTGTCCAACAAGGGCCTGCCCAGCTCCATCGA
GAAAACCATCAGCAAGGCCAAGGGCCAGCCTCGGGAGCCCCAGGTGTACACCCTGCCCCCATCCCAGGAAGA
GATGACCAAGAACCAGGTGTCCCTGACCTGTCTGGTGAAGGGCTTCTACCCCAGCGACATCGCCGTGGAGTG
GGAGAGCAACGGCCAGCCCGAGAACAACTACAAGACCACCCCCCCTGTGCTGGACAGCGACGGCAGCTTCTT
CCTGTACAGCAGGCTGACCGTGGACAAGAGCCGGTGGCAGGAAGGCAACGTCTTTAGCTGCAGCGTGATGC
ACGAGGCCCTGCACAACCACTACACCCAGAAGAGCCTGAGCCTGTCCCTGGGCAAGGGATCCGGCGTGCAGC
TCCTGGAGAGCGGCGGAGGCCTGGTCCAGCCCGGCGGCAGCCTGAGGCTGAGCTGCGCCGCCAGCGGCTTC
ACCTTCGCCTGGTATGATATGGGCTGGGTGAGGCAGGCCCCCGGCAAGGGCCTGGAGTGGGTGTCCAGCAT
CGACTGGCACGGGGAGGTGACCTACTACGCCGACAGCGTGAAGGGCAGGTTCACCATCAGCAGGGACAACA
GCAAGAACACCCTGTACCTGCAGATGAACAGCCTGAGGGCCGAGGACACCGCAGTGTACTACTGCGCCACCG
CCGAGGACGAACCCGGCTACGACTACTGGGGCCAGGGCACCCTGGTGACTGTGAGCAGC

**SEQ ID NO: 270: Anti IL-4 Heavy Chain-GS-anti TNF-a adnectin**

CAGGTGACCCTGAGGGAGAGCGGCCCCGCCCTGGTGAAGCCCACCCAGACCCTGACCCTGACCTGCACCTTC
AGCGGCTTTAGCCTCAGCACCTCCGGCATGGGCGTGAGCTGGATCAGGCAGCCACCCGGCAAAGGCCTGGA
GTGGCTGGCCCACATCTACTGGGACGACGACAAGAGGTACAACCCCAGCCTGAAGAGCCGGCTGACCATCAG
CAAGGATACCAGCAGGAACCAGGTGGTGCTGACCATGACCAACATGGACCCCGTGGACACCGCTACCTACTA
CTGCGCCAGGAGGGAGACCGTCTTCTACTGGTACTTCGACGTGTGGGGAAGGGGCACACTAGTGACCGTGT
CCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCT
GACCAGCGGCGTGCACACCTTCCCCGCCGTGCTGCAGAGCAGCGGCCTGTACAGCCTGAGCAGCGTGGTGA
CCGTGCCCAGCAGCAGCCTGGGCACCCAGACCTACATCTGTAACGTGAACCACAAGCCCAGCAACACCAAGG
TGGACAAGAAGGTGGAGCCCAAGAGCTGTGACAAGACCCACACCTGCCCCCCCTGCCCTGCCCCCGAGCTGC
TGGGAGGCCCCAGCGTGTTCCTGTTCCCCCCCAAGCCTAAGGACACCCTGATGATCAGCAGAACCCCCGAGG
TGACCTGTGTGGTGGTGGATGTGAGCCACGAGGACCCTGAGGTGAAGTTCAACTGGTACGTGGACGGCGTG
GAGGTGCACAATGCCAAGACCAAGCCCAGGGAGGAGCAGTACAACAGCACCTACCGGGTGGTGTCCGTGCT
GACCGTGCTGCACCAGGATTGGCTGAACGGCAAGGAGTACAAGTGTAAGGTGTCCAACAAGGCCCTGCCTG
CCCCTATCGAGAAAACCATCAGCAAGGCCAAGGGCCAGCCCAGAGAGCCCCAGGTGTACACCCTGCCCCCTA
GCAGAGATGAGCTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGCTGGACAGCGAT

GGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAGGGATC
CGTGAGCGACGTGCCAAGGGACCTCGAGGTGGTGGCAGCCACTCCCACCTCTCTGCTGATCAGCTGGGACA
CACACAACGCCTACAACGGCTACTACAGGATCACCTACGGAGAGACCGGCGGCAATAGCCCCGTGAGGGAGT
TCACCGTGCCCCACCCCGAGGTGACCGCCACCATTAGCGGCCTGAAGCCCGGCGTGGACGATACCATCACCG
TCTACGCCGTGACCAACCACCACATGCCCCTGAGGATCTTCGGCCCCATCAGCATCAACCATAGGACC

## Claims

1. An antigen-binding construct comprising a protein scaffold which is linked to one or more epitope-binding domains wherein the antigen-binding construct has specificity for more than one antigen and is capable of binding IL-13 and IL-5; and has at least two antigen binding sites, at least one of which is from an epitope binding domain which is a dAb, and at least one of which is from a paired VH/VL domain; and wherein the protein scaffold is an Ig scaffold.

2. An antigen-binding construct according to claim 1, where the at least one epitope-binding domain is capable of binding to IL-13

3. An antigen-binding construct according to claim 1 or claim 2 wherein the dAb is a human dAb.

4. An antigen-binding construct according to any previous claim, wherein the Igscaffold is an IgG scaffold.

5. An antigen-binding construct according to any previous claim, wherein the IgG scaffold comprises all the domains of an antibody

6. An antigen-binding construct according to any preceding claim, wherein at least one of the epitope binding domains is directly attached to the Ig scaffold with a linker comprising from 1 to 20 amino acids.

7. An antigen-binding construct according to claim 6 wherein at least one of the epitope binding domains is directly attached to the Ig scaffold with a linker Ig scaffold with a linker selected from any one of those set out in SEQ 10 NO: 6 to 11 or 'GS', or any combination thereof.

8. An antigen-binding construct according to any previous claim comprising an epitope binding domain attached to the Ig scaffold at the C terminus of the heavy chain.

**9.** An antigen-binding construct as defined in any preceding claim for use in medicine.

**10.** An antigen-binding construct as defined in any one of claims 1 to 8 for use in the manufacture of a medicament for treating cancer or inflammatory diseases such as asthma, rheumatoid arthritis or osteoarthritis.

**11.** An antigen-binding construct as defined in any one of claims 1 to 8 for the treatment of cancer or inflammatory diseases such as asthma, rheumatoid arthritis or osteoarthritis.

**12.** A polynucleotide sequence encoding a heavy chain of an antigen binding construct as defined in any one of claims 1 to 8.

**13.** A polynucleotide encoding a light chain of an antigen binding construct as defined in any one of claims 1 to 8.

**14.** A recombinant transformed or transfected host cell comprising one or more polynucleotide sequences encoding a heavy chain and a light chain of an antigen-binding construct as defined in any one of claims 1 to 8.

**15.** A pharmaceutical composition comprising an antigen-binding construct as defined in any one of claims 1 to 8 and a pharmaceutically acceptable carrier.

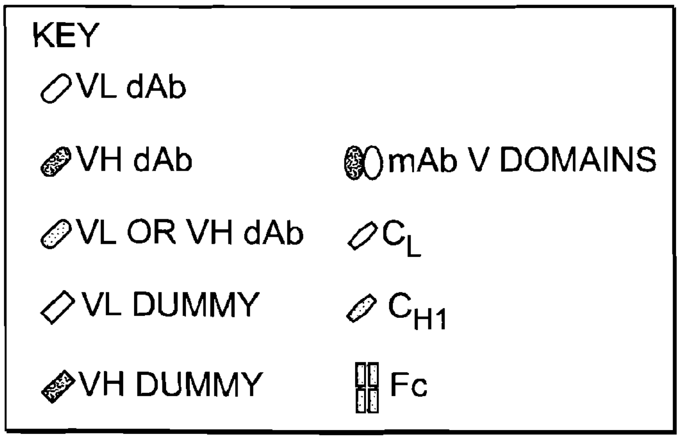
KEY
VL dAb
VH dAb
mAb V DOMAINS
VL OR VH dAb
$C_L$
VL DUMMY
$C_{H1}$
VH DUMMY
Fc

*FIG. 1*

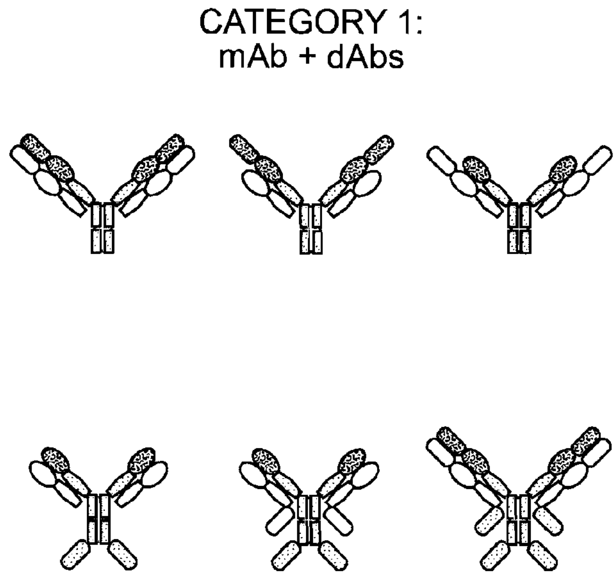
CATEGORY 1:
mAb + dAbs

*FIG. 2*

CATEGORY 2:
DUMMY IgG + VARIABLE DOMAINS (Fv AND/OR dAbs)

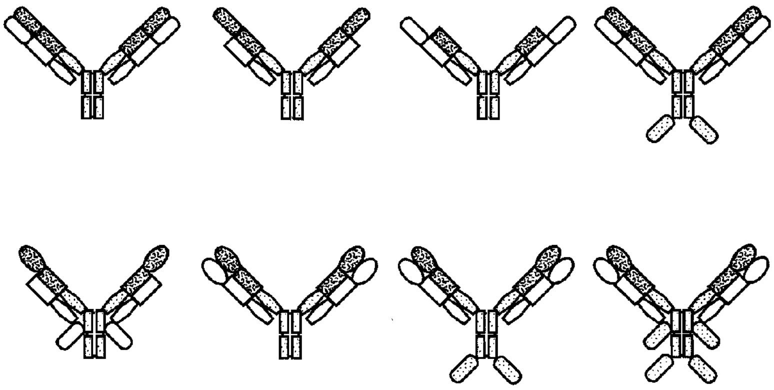

*FIG. 3*

CATEGORY 3:
PORTION OF DUMMY IgG + VARIABLE DOMAINS (Fv AND/OR dAbs)

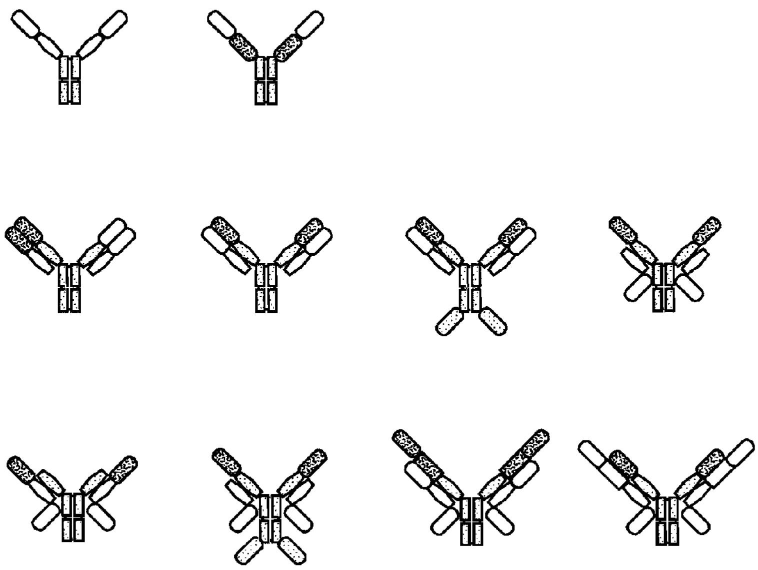

*FIG. 4*

CATEGORY 4:
VARIABLE AND/OR CONSTANT DOMAINS SWAPPED

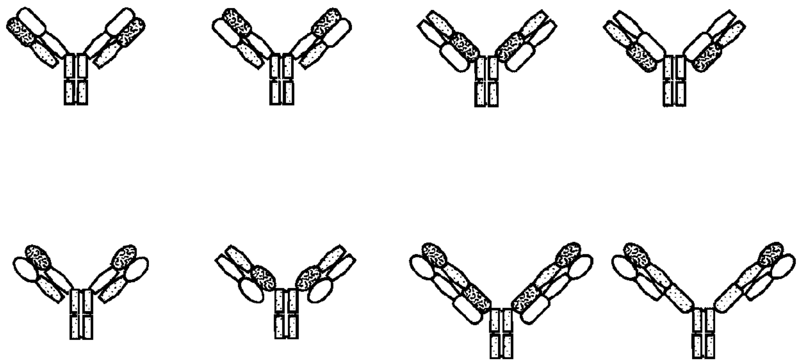

*FIG. 5*

CATEGORY 5:
CONSTANT DOMAINS DUPLICATED

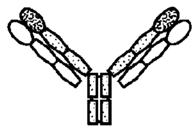

*FIG. 6*

CATEGORY 6:
AlbudAbs

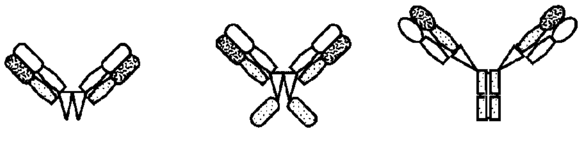

*FIG. 7*

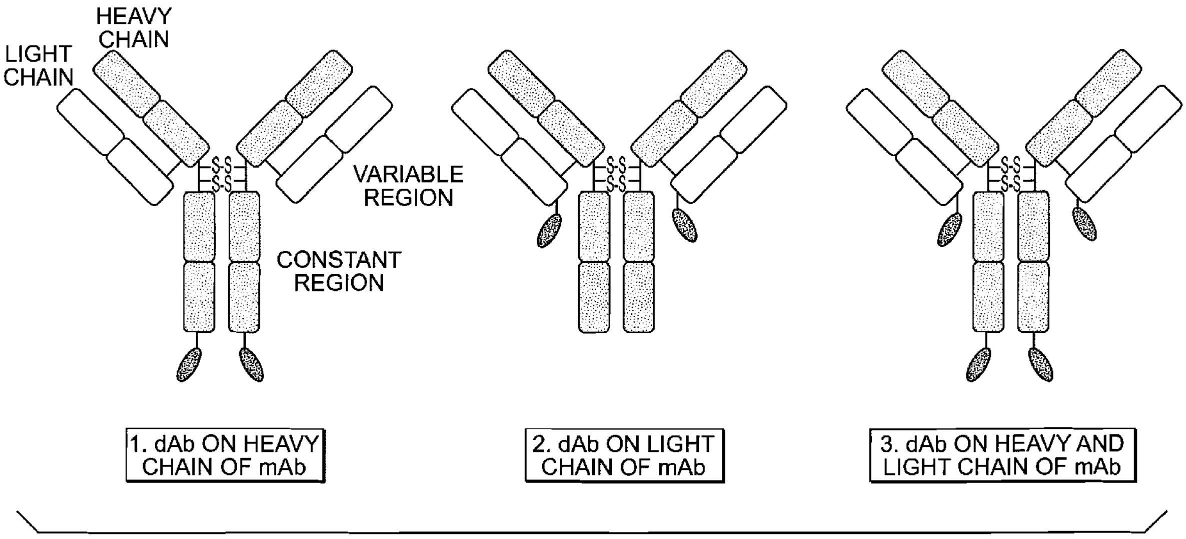
HEAVY
CHAIN
LIGHT
CHAIN
S-S
S-S
VARIABLE
REGION
CONSTANT
REGION
1. dAb ON HEAVY
CHAIN OF mAb
2. dAb ON LIGHT
CHAIN OF mAb
3. dAb ON HEAVY AND
LIGHT CHAIN OF mAb

*FIG. 8*

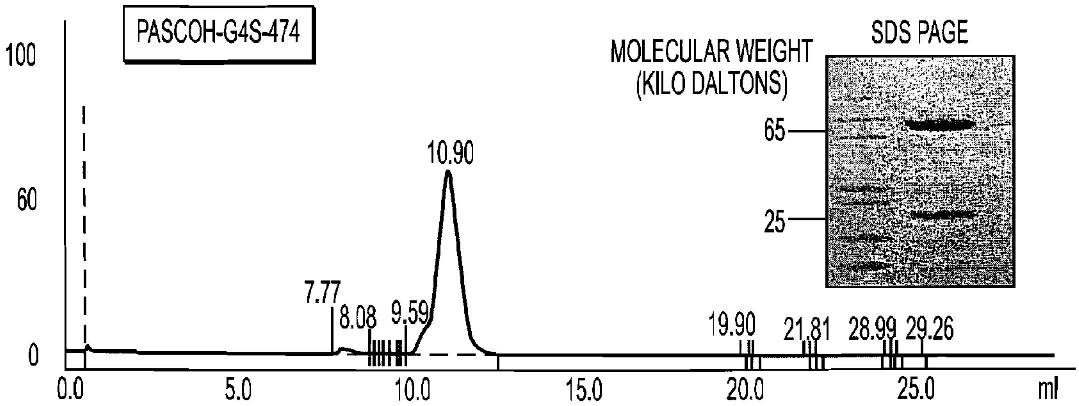


FIG. 9

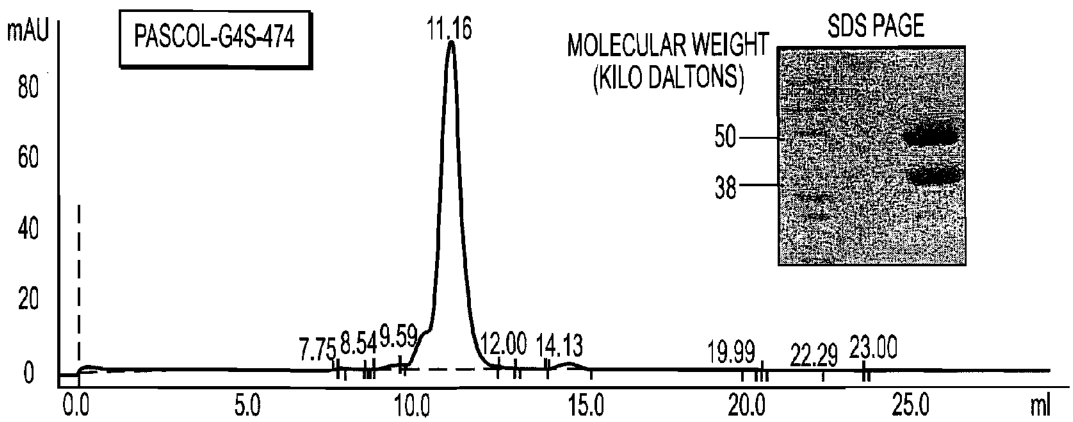


FIG. 10

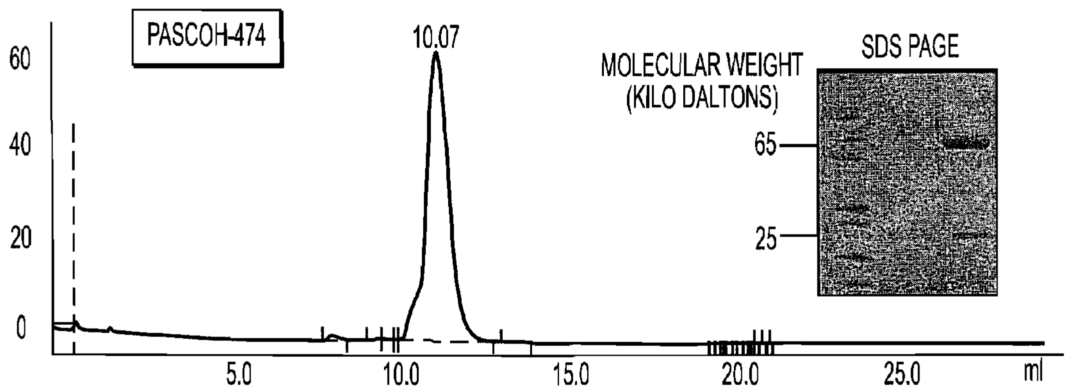


FIG. 11

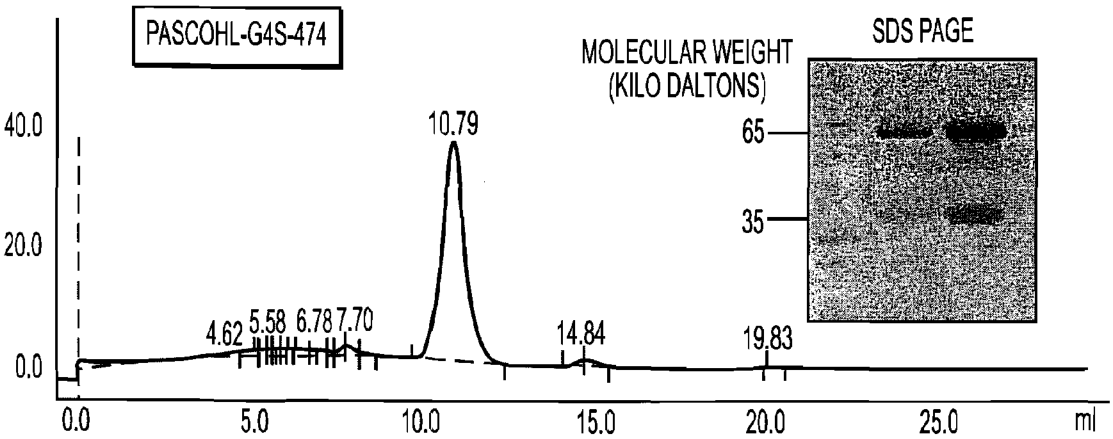
PASCOHL-G4S-474
MOLECULAR WEIGHT (KILO DALTONS)
SDS PAGE
65
35
40.0
20.0
0.0
10.79
4.62 5.58 6.78 7.70
14.84
19.83
0.0
5.0
10.0
15.0
20.0
25.0
ml

*FIG. 12*

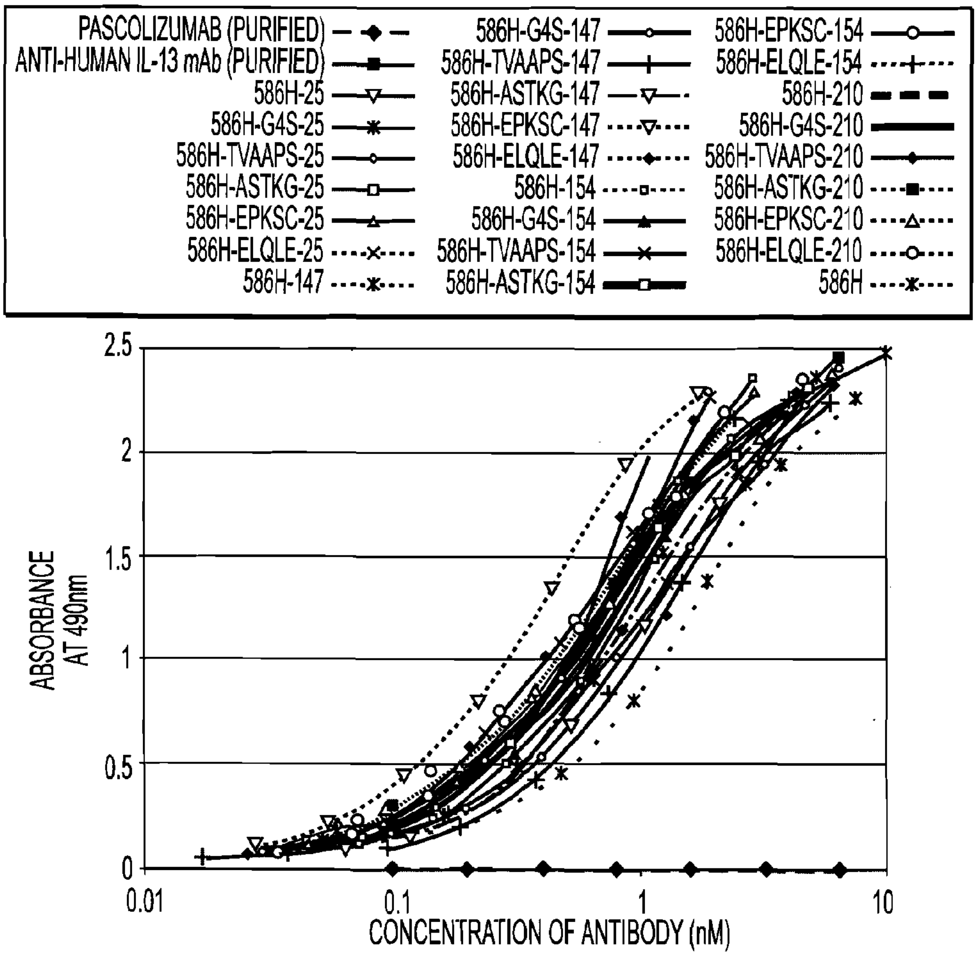
PASCOLIZUMAB (PURIFIED)
ANTI-HUMAN IL-13 mAb (PURIFIED)
586H-25
586H-G4S-25
586H-TVAAPS-25
586H-ASTKG-25
586H-EPKSC-25
586H-ELQLE-25
586H-147
586H-G4S-147
586H-TVAAPS-147
586H-ASTKG-147
586H-EPKSC-147
586H-ELQLE-147
586H-154
586H-G4S-154
586H-TVAAPS-154
586H-ASTKG-154
586H-EPKSC-154
586H-ELQLE-154
586H-210
586H-G4S-210
586H-TVAAPS-210
586H-ASTKG-210
586H-EPKSC-210
586H-ELQLE-210
586H
ABSORBANCE AT 490nm
2.5
2
1.5
1
0.5
0
0.01
0.1
1
10
CONCENTRATION OF ANTIBODY (nM)

*FIG. 13*

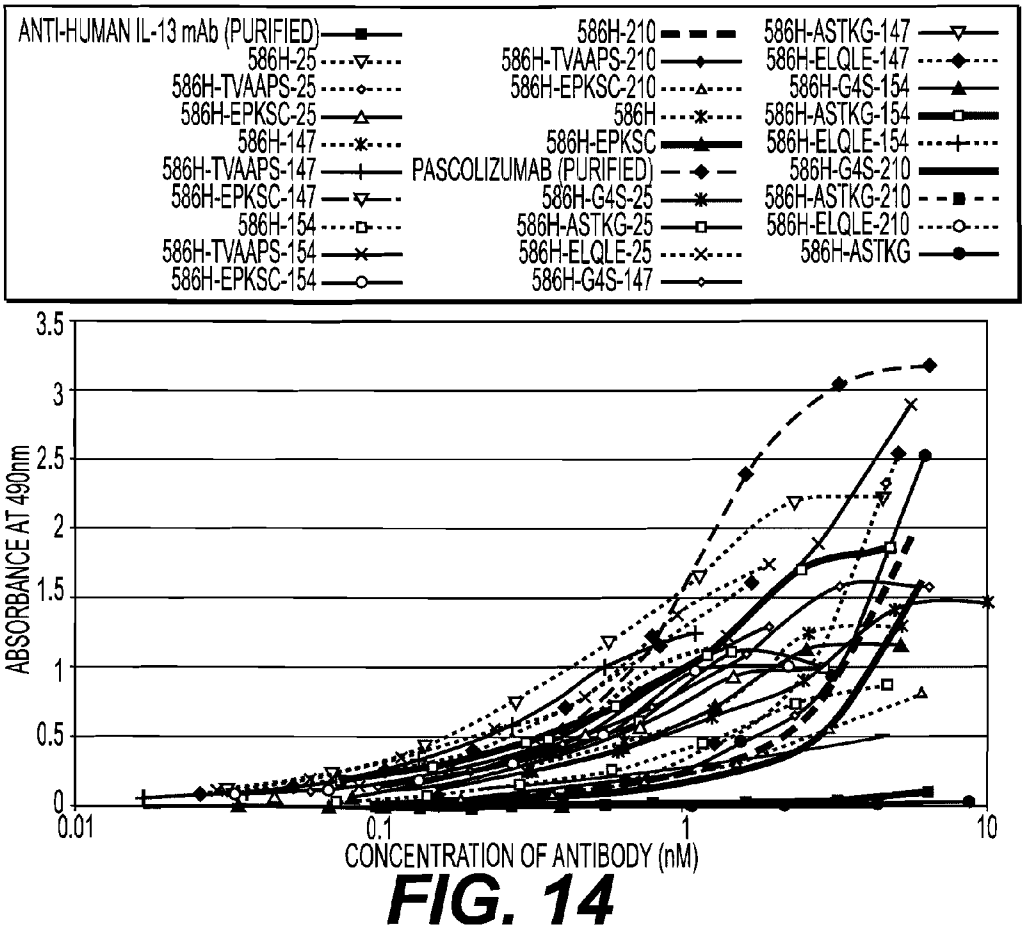
ANTI-HUMAN IL-13 mAb (PURIFIED)
586H-25
586H-TVAAPS-25
586H-EPKSC-25
586H-147
586H-TVAAPS-147
586H-EPKSC-147
586H-154
586H-TVAAPS-154
586H-EPKSC-154
586H-210
586H-TVAAPS-210
586H-EPKSC-210
586H
586H-EPKSC
PASCOLIZUMAB (PURIFIED)
586H-G4S-25
586H-ASTKG-25
586H-ELQLE-25
586H-G4S-147
586H-ASTKG-147
586H-ELQLE-147
586H-G4S-154
586H-ASTKG-154
586H-ELQLE-154
586H-G4S-210
586H-ASTKG-210
586H-ELQLE-210
586H-ASTKG
ABSORBANCE AT 490nm
3.5
3
2.5
2
1.5
1
0.5
0
0.01
0.1
1
10
CONCENTRATION OF ANTIBODY (nM)

*FIG. 14*

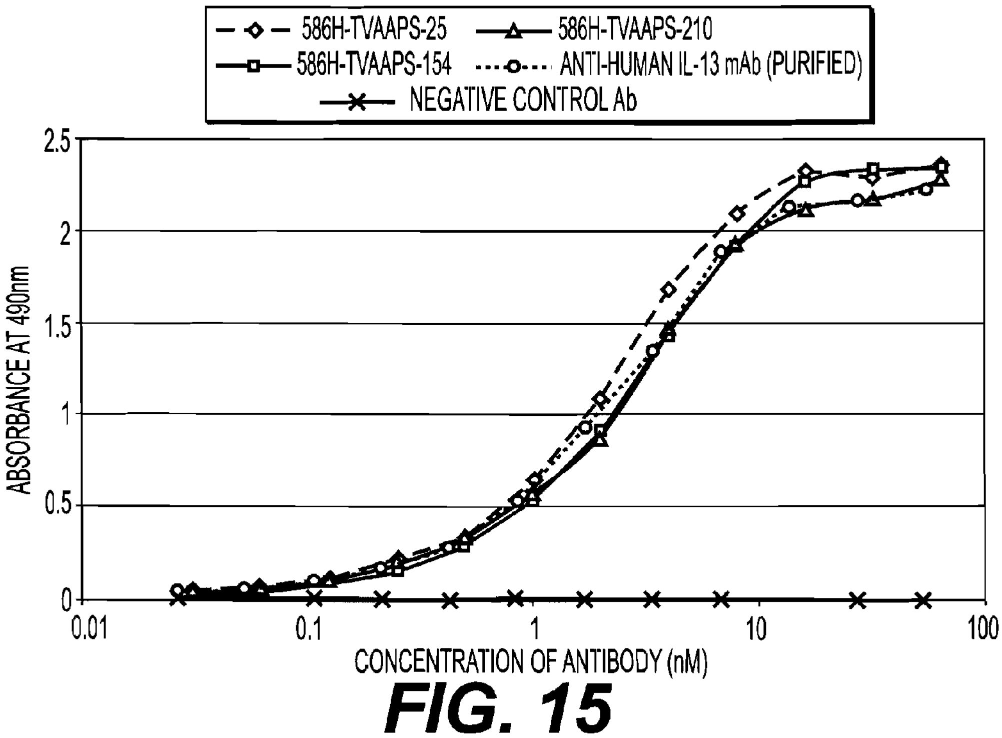
586H-TVAAPS-25
586H-TVAAPS-210
586H-TVAAPS-154
ANTI-HUMAN IL-13 mAb (PURIFIED)
NEGATIVE CONTROL Ab
ABSORBANCE AT 490nm
2.5
2
1.5
1
0.5
0
0.01
0.1
1
10
100
CONCENTRATION OF ANTIBODY (nM)

*FIG. 15*

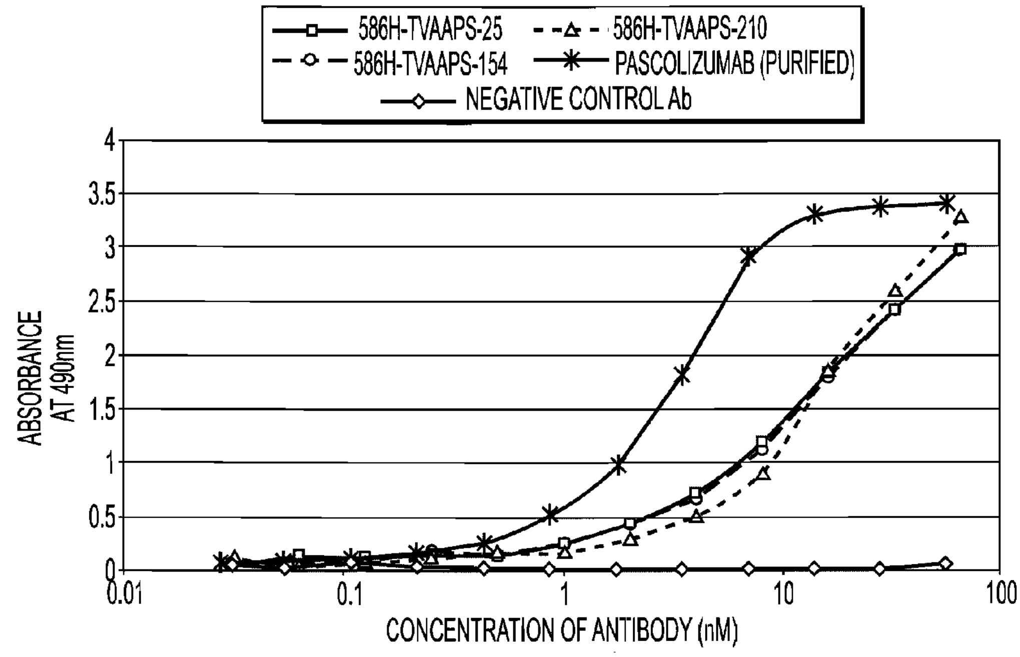
586H-TVAAPS-25
586H-TVAAPS-210
586H-TVAAPS-154
PASCOLIZUMAB (PURIFIED)
NEGATIVE CONTROL Ab
ABSORBANCE AT 490nm
CONCENTRATION OF ANTIBODY (nM)
0
0.5
1
1.5
2
2.5
3
3.5
4
0.01
0.1
1
10
100

*FIG. 16*

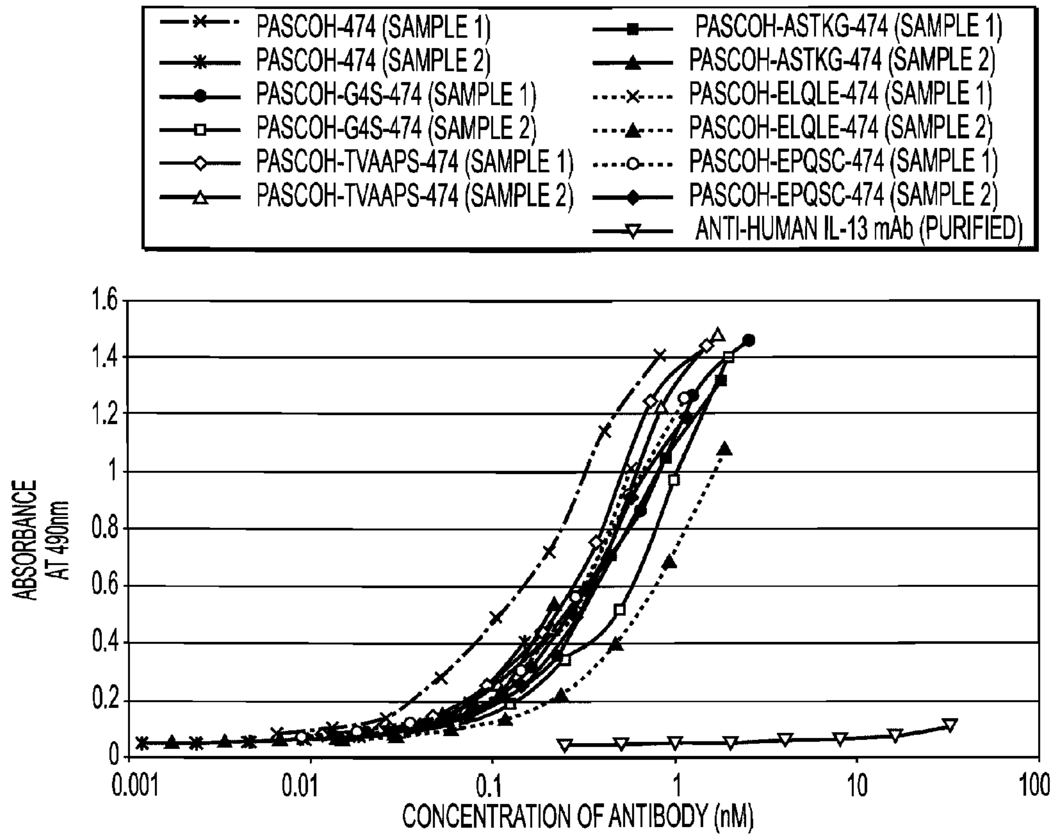
PASCOH-474 (SAMPLE 1)
PASCOH-474 (SAMPLE 2)
PASCOH-G4S-474 (SAMPLE 1)
PASCOH-G4S-474 (SAMPLE 2)
PASCOH-TVAAPS-474 (SAMPLE 1)
PASCOH-TVAAPS-474 (SAMPLE 2)
PASCOH-ASTKG-474 (SAMPLE 1)
PASCOH-ASTKG-474 (SAMPLE 2)
PASCOH-ELQLE-474 (SAMPLE 1)
PASCOH-ELQLE-474 (SAMPLE 2)
PASCOH-EPQSC-474 (SAMPLE 1)
PASCOH-EPQSC-474 (SAMPLE 2)
ANTI-HUMAN IL-13 mAb (PURIFIED)
ABSORBANCE AT 490nm
CONCENTRATION OF ANTIBODY (nM)
0
0.2
0.4
0.6
0.8
1
1.2
1.4
1.6
0.001
0.01
0.1
1
10
100

*FIG. 17*

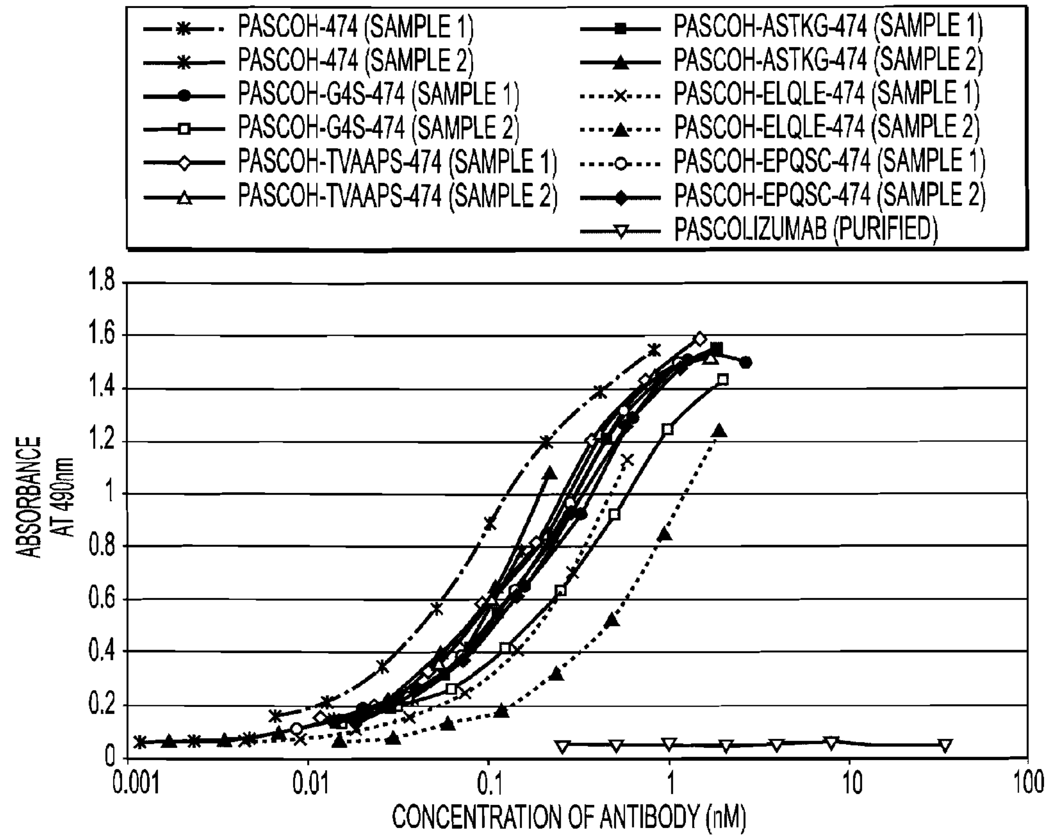
PASCOH-474 (SAMPLE 1)
PASCOH-474 (SAMPLE 2)
PASCOH-G4S-474 (SAMPLE 1)
PASCOH-G4S-474 (SAMPLE 2)
PASCOH-TVAAPS-474 (SAMPLE 1)
PASCOH-TVAAPS-474 (SAMPLE 2)
PASCOH-ASTKG-474 (SAMPLE 1)
PASCOH-ASTKG-474 (SAMPLE 2)
PASCOH-ELQLE-474 (SAMPLE 1)
PASCOH-ELQLE-474 (SAMPLE 2)
PASCOH-EPQSC-474 (SAMPLE 1)
PASCOH-EPQSC-474 (SAMPLE 2)
PASCOLIZUMAB (PURIFIED)
ABSORBANCE AT 490nm
CONCENTRATION OF ANTIBODY (nM)
0
0.2
0.4
0.6
0.8
1
1.2
1.4
1.6
1.8
0.001
0.01
0.1
1
10
100

*FIG. 18*

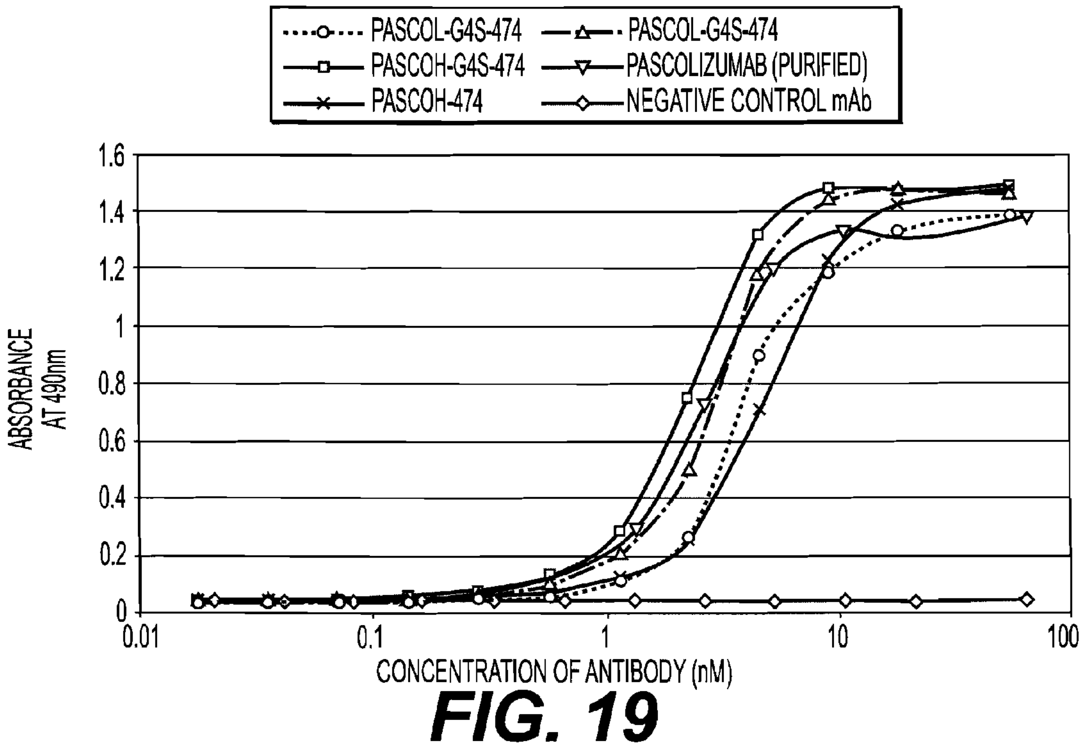
PASCOL-G4S-474
PASCOL-G4S-474
PASCOH-G4S-474
PASCOLIZUMAB (PURIFIED)
PASCOH-474
NEGATIVE CONTROL mAb
ABSORBANCE AT 490nm
CONCENTRATION OF ANTIBODY (nM)
0
0.2
0.4
0.6
0.8
1
1.2
1.4
1.6
0.01
0.1
1
10
100

*FIG. 19*

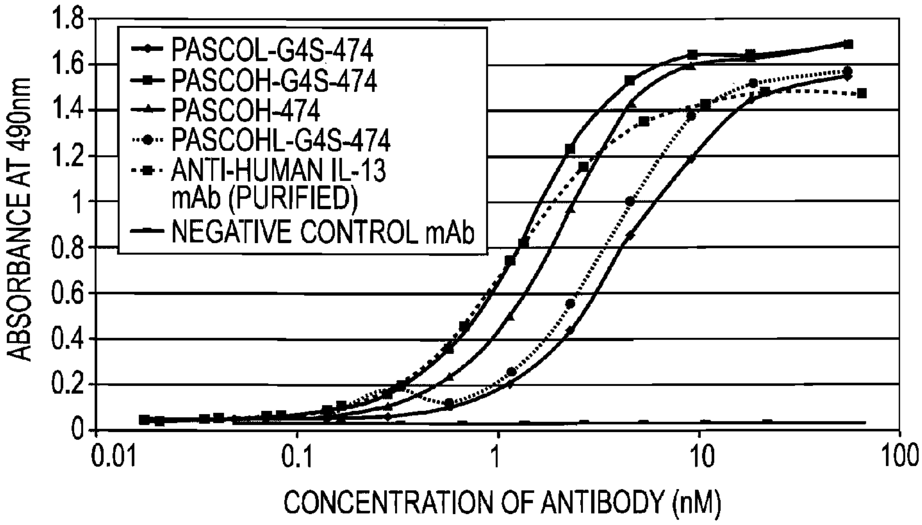


*FIG. 20A*

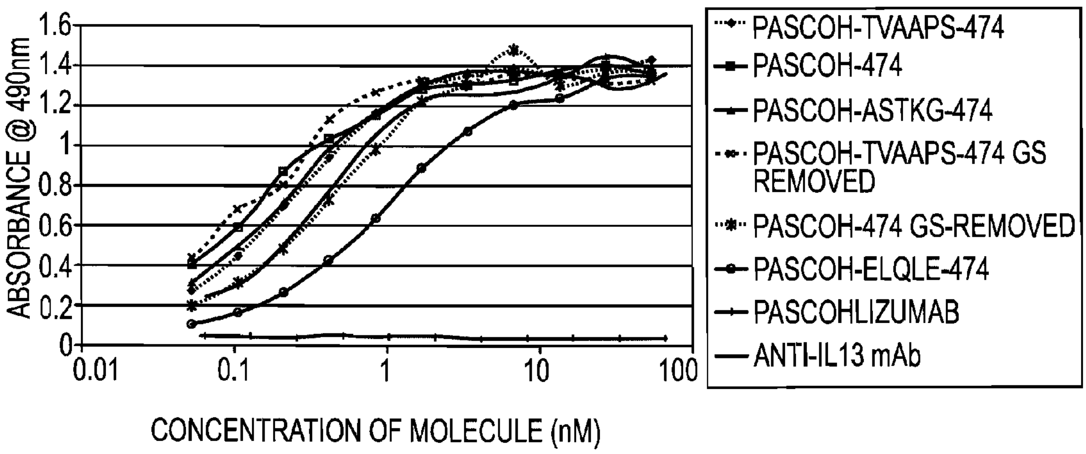


*FIG. 20B*

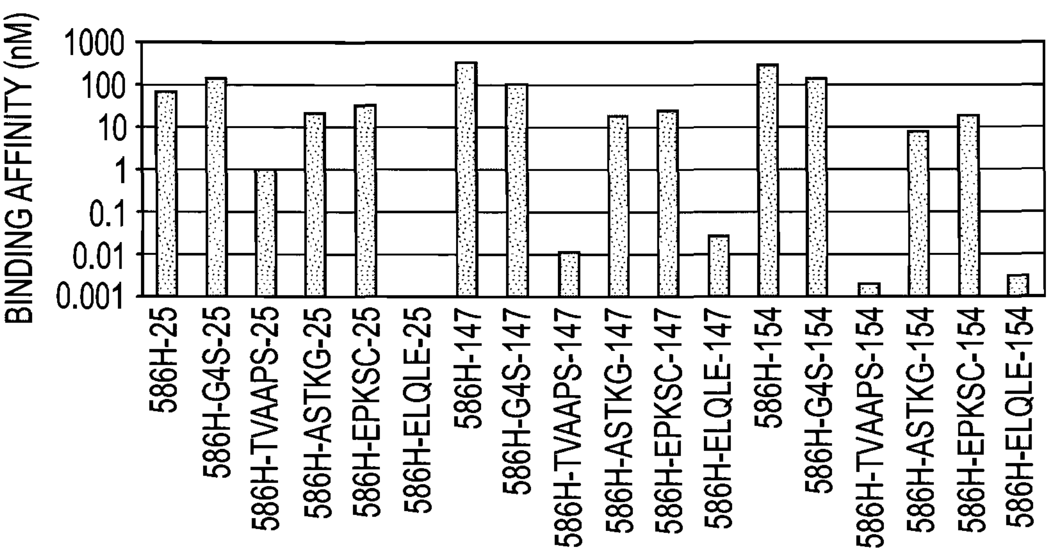
BINDING AFFINITY (nM)
1000
100
10
1
0.1
0.01
0.001
586H-25
586H-G4S-25
586H-TVAAPS-25
586H-ASTKG-25
586H-EPKSC-25
586H-ELQLE-25
586H-147
586H-G4S-147
586H-TVAAPS-147
586H-ASTKG-147
586H-EPKSC-147
586H-ELQLE-147
586H-154
586H-G4S-154
586H-TVAAPS-154
586H-ASTKG-154
586H-EPKSC-154
586H-ELQLE-154

*FIG. 21*

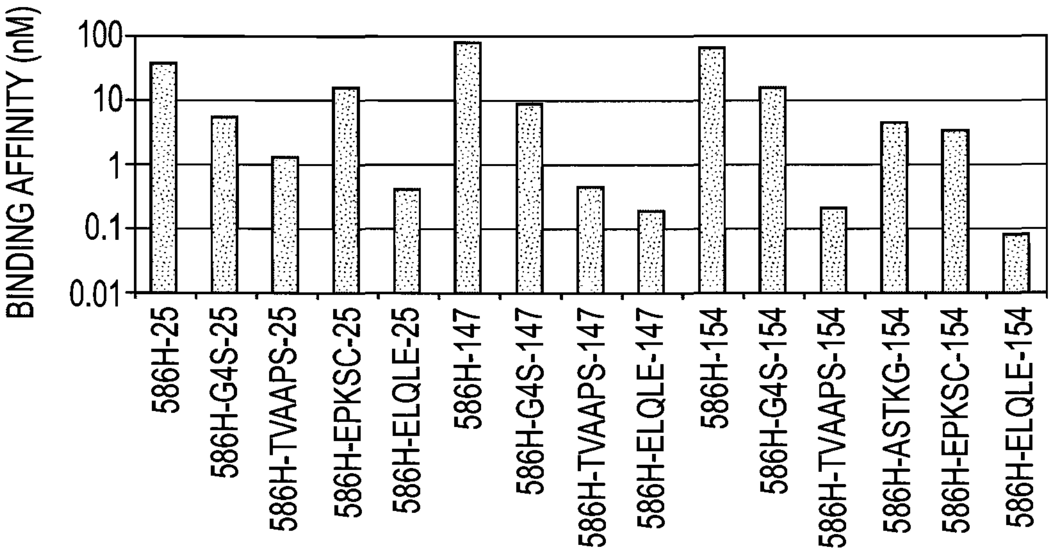
BINDING AFFINITY (nM)
100
10
1
0.1
0.01
586H-25
586H-G4S-25
586H-TVAAPS-25
586H-EPKSC-25
586H-ELQLE-25
586H-147
586H-G4S-147
586H-TVAAPS-147
586H-ELQLE-147
586H-154
586H-G4S-154
586H-TVAAPS-154
586H-ASTKG-154
586H-EPKSC-154
586H-ELQLE-154

*FIG. 22*

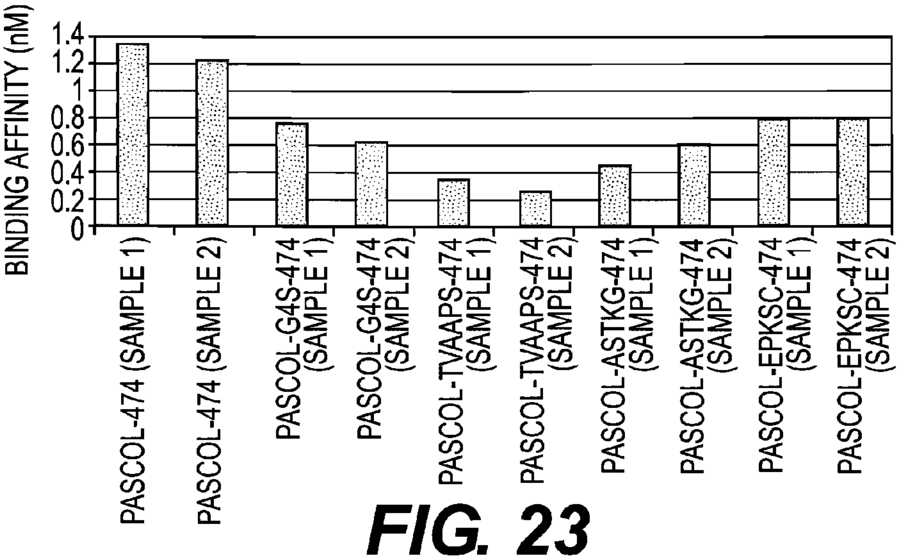


*FIG. 23*

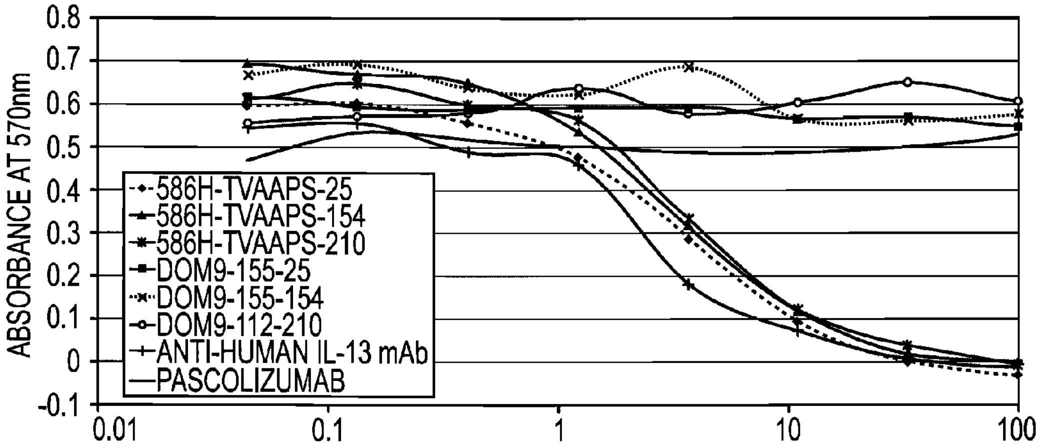


*FIG. 24*

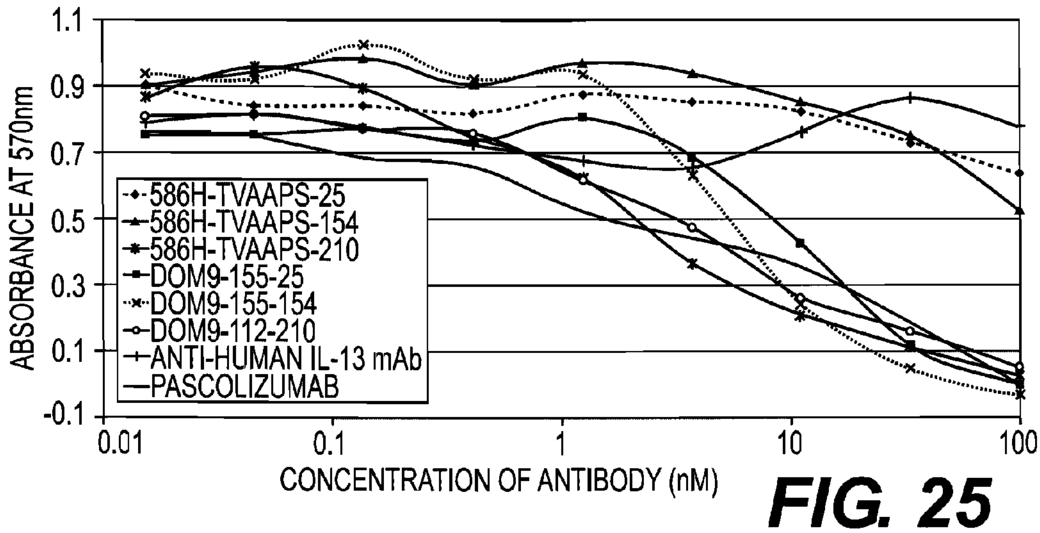


*FIG. 25*

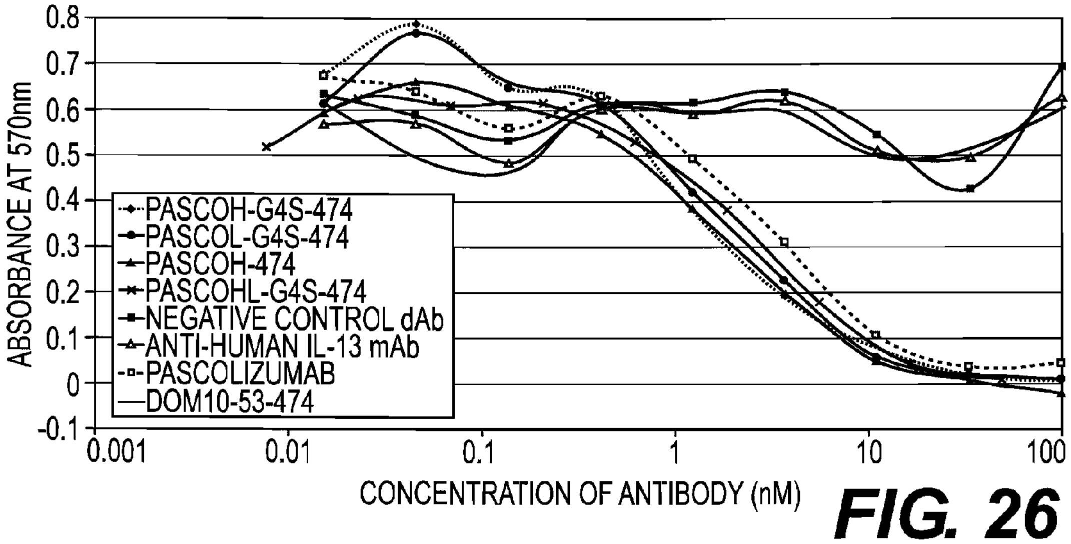


FIG. 26

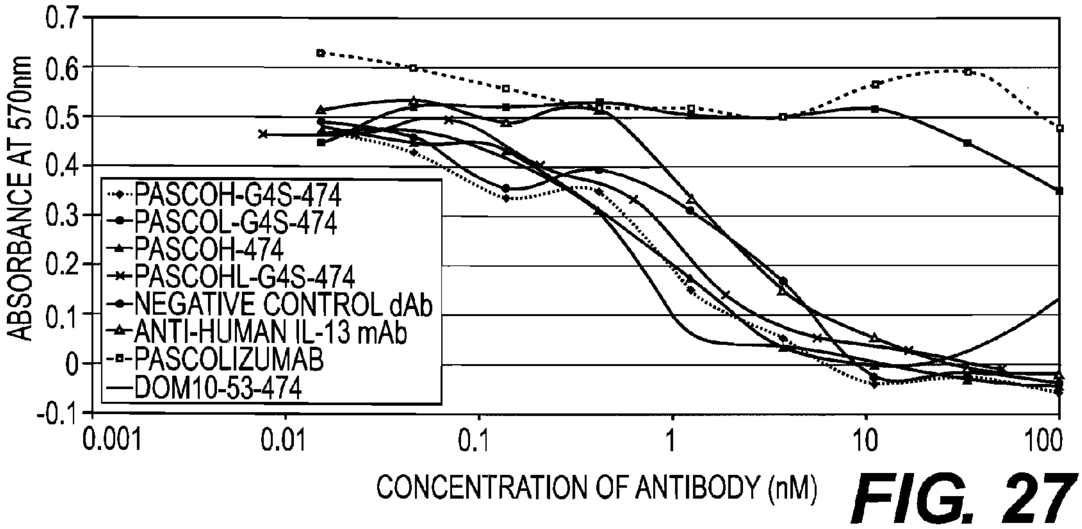


FIG. 27

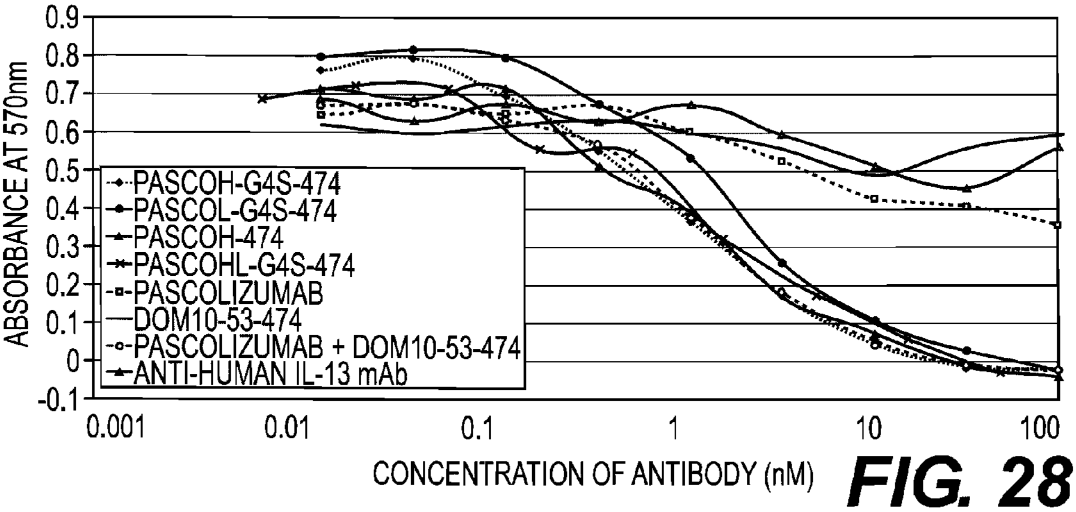


FIG. 28

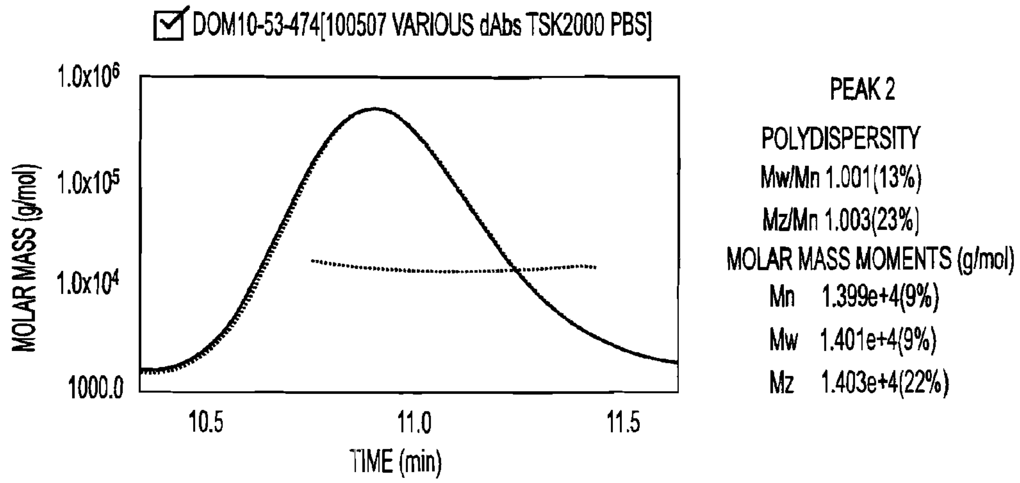
DOM10-53-474[100507 VARIOUS dAbs TSK2000 PBS]
1.0x10^6
1.0x10^5
1.0x10^4
1000.0
MOLAR MASS (g/mol)
10.5
11.0
11.5
TIME (min)
PEAK 2
POLYDISPERSITY
Mw/Mn 1.001(13%)
Mz/Mn 1.003(23%)
MOLAR MASS MOMENTS (g/mol)
Mn 1.399e+4(9%)
Mw 1.401e+4(9%)
Mz 1.403e+4(22%)

*FIG. 29*

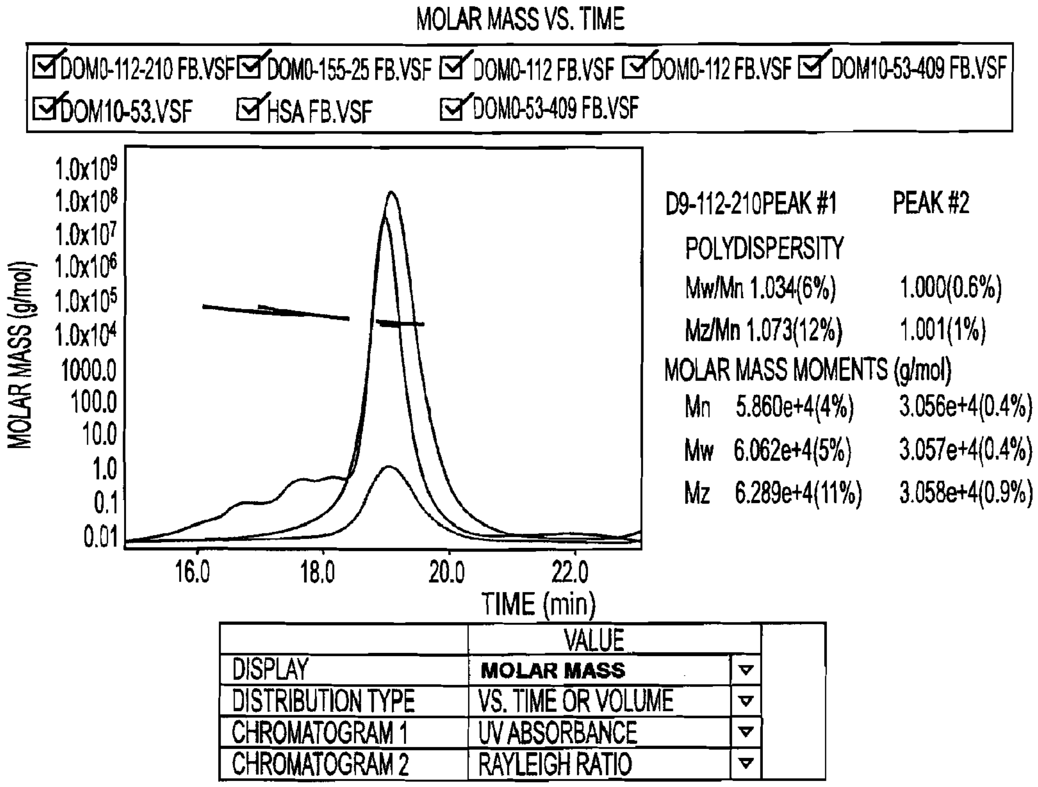
MOLAR MASS VS. TIME
DOM0-112-210 FB.VSF
DOM0-155-25 FB.VSF
DOM0-112 FB.VSF
DOM0-112 FB.VSF
DOM10-53-409 FB.VSF
DOM10-53.VSF
HSA FB.VSF
DOM0-53-409 FB.VSF
1.0x10^9
1.0x10^8
1.0x10^7
1.0x10^6
1.0x10^5
1.0x10^4
1000.0
100.0
10.0
1.0
0.1
0.01
MOLAR MASS (g/mol)
16.0
18.0
20.0
22.0
TIME (min)
D9-112-210PEAK #1 PEAK #2
POLYDISPERSITY
Mw/Mn 1.034(6%) 1.000(0.6%)
Mz/Mn 1.073(12%) 1.001(1%)
MOLAR MASS MOMENTS (g/mol)
Mn 5.860e+4(4%) 3.056e+4(0.4%)
Mw 6.062e+4(5%) 3.057e+4(0.4%)
Mz 6.289e+4(11%) 3.058e+4(0.9%)
VALUE
DISPLAY MOLAR MASS
DISTRIBUTION TYPE VS. TIME OR VOLUME
CHROMATOGRAM 1 UV ABSORBANCE
CHROMATOGRAM 2 RAYLEIGH RATIO

*FIG. 30*

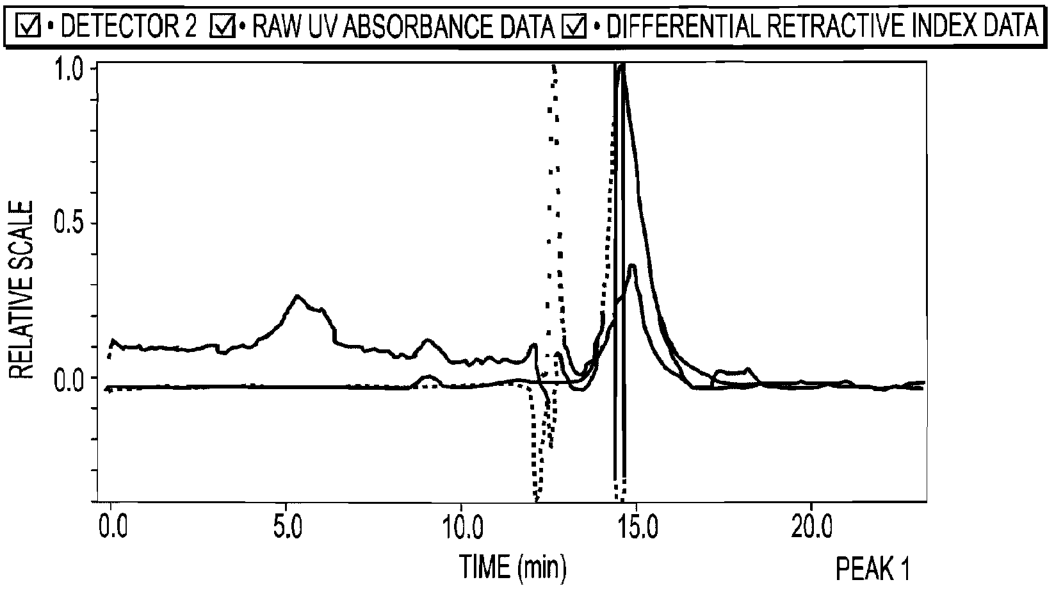


POLY DISPERSITY
Mw/Mn 1.000 (20%)
Mz/Mn 1.000 (34%)
MOLAR MASS MOMENTS (g/mol)
Mn 2.800e+4 (14%)
Mw 2.801e+4 (14%)
Mz 2.802e+4 (31%)

***FIG. 31***

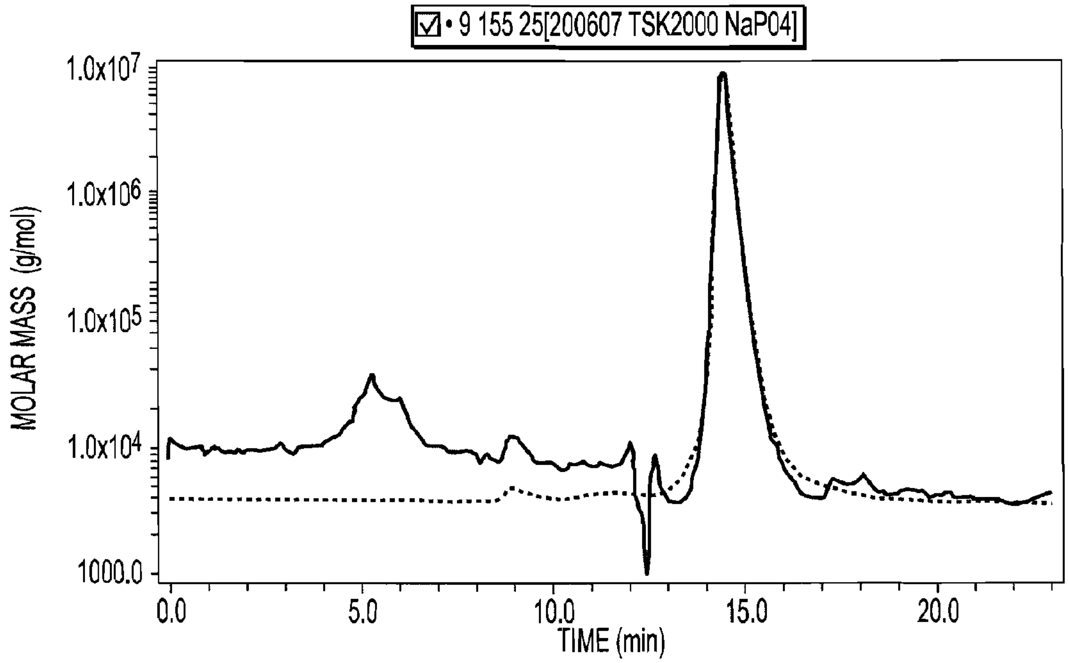


***FIG. 32***

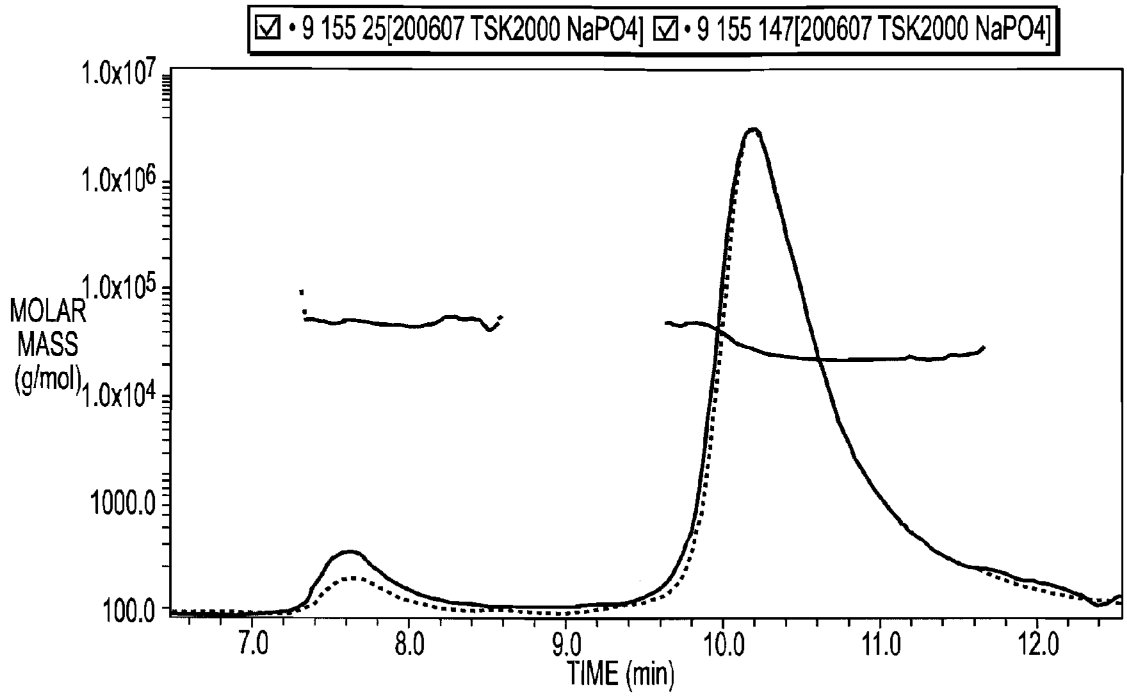


| | PEAK 1 | PEAK 2 (ALL) |
|---|---|---|
| POLYDISPERSITY | | |
| Mw/Mn | 1.002 (6%) | 1.039 (9%) |
| Mz/Mn | 1.003 (10%) | 1.092 (19%) |
| MOLAR MASS MOMENTS (g/mol) | | |
| Mn | 5.041e+4 (4%) | 2.942e+4 (5%) |
| Mw | 5.049e+4 (4%) | 3.058e+4 (7%) |
| Mz | 5.057e+4 (9%) | 3.214e+4 (18%) |

| PEAK 2 (RIGHT PART) | |
|---|---|
| POLYDISPERSITY | |
| Mw/Mn | 1.001 (4%) |
| Mz/Mn | 1.002 (7%) |
| MOLAR MASS MOMENTS (g/mol) | |
| Mn | 2.589e+4 (3%) |
| Mw | 2.591e+4 (3%) |
| Mz | 2.593e+4 (6%) |

***FIG. 33***

PEAK 3

POLYDISPERSITY

Mw/Mn 1.011 (10%)
Mz/Mn 1.022 (19%)

MOLAR MASS MOMENTS (g/mol)

| | | |
|---|---|---|
| Mn | 2.740e+4 (7%) |
| Mw | 2.769e+4 (7%) |
| Mz | 2.801e+4 (17%) |

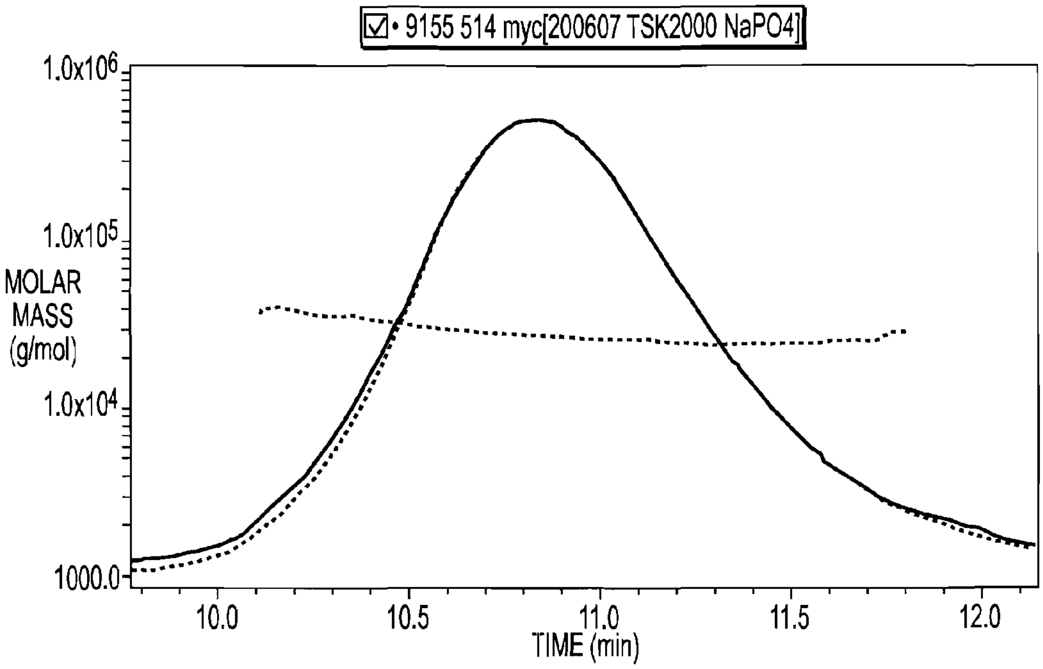


*FIG. 34*

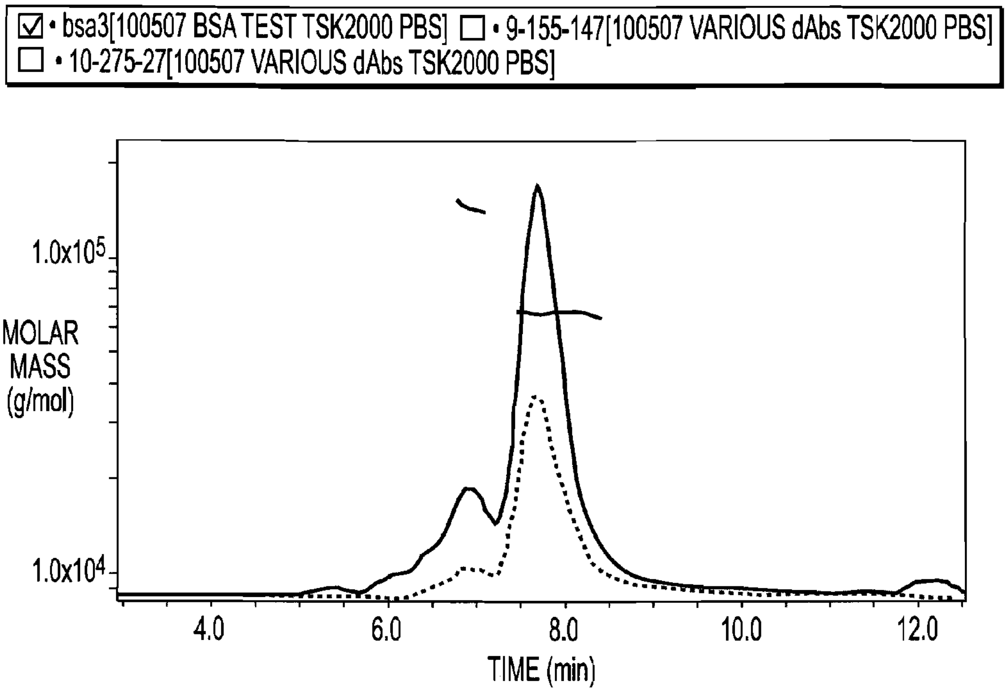


| | PEAK 2 | PEAK 3 |
|---|---|---|
| POLYDISPERSITY | | |
| Mw/Mn | 1.000 (2%) | 1.001 (3%) |
| Mz/Mn | 1.000 (3%) | 1.001 (6%) |
| MOLAR MASS MOMENTS (g/mol) | | |
| Mn | 6.735e+4 (1%) | 1.449e+5 (2%) |
| Mw | 6.736e+4 (1%) | 1.450e+5 (2%) |
| Mz | 6.737e+4 (3%) | 1.451e+5 (5%) |

*FIG. 35*

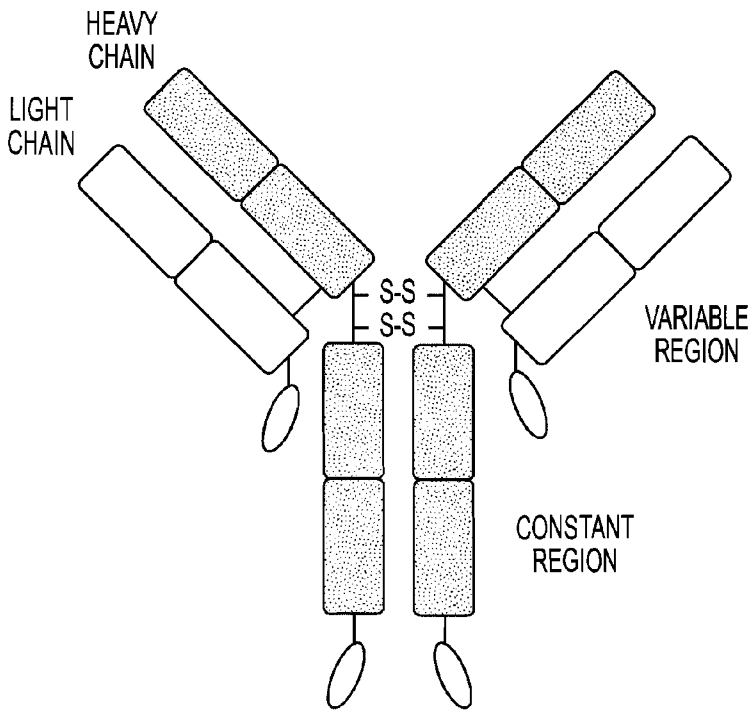


ONE dAb ON HEAVY
CHAIN AND DIFFERENT dAb
ON LIGHT CHAIN OF mAb

*FIG. 36*

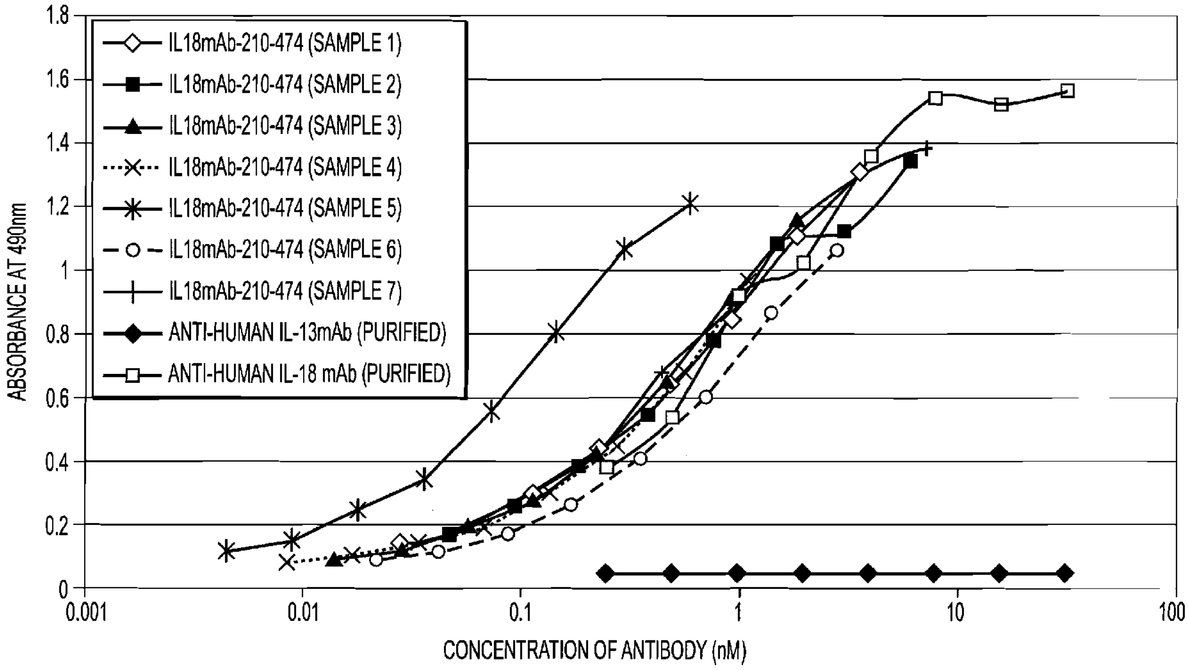
ABSORBANCE AT 490nm
1.8
1.6
1.4
1.2
1
0.8
0.6
0.4
0.2
0
IL18mAb-210-474 (SAMPLE 1)
IL18mAb-210-474 (SAMPLE 2)
IL18mAb-210-474 (SAMPLE 3)
IL18mAb-210-474 (SAMPLE 4)
IL18mAb-210-474 (SAMPLE 5)
IL18mAb-210-474 (SAMPLE 6)
IL18mAb-210-474 (SAMPLE 7)
ANTI-HUMAN IL-13mAb (PURIFIED)
ANTI-HUMAN IL-18 mAb (PURIFIED)
0.001
0.01
0.1
1
10
100
CONCENTRATION OF ANTIBODY (nM)

*FIG. 37*

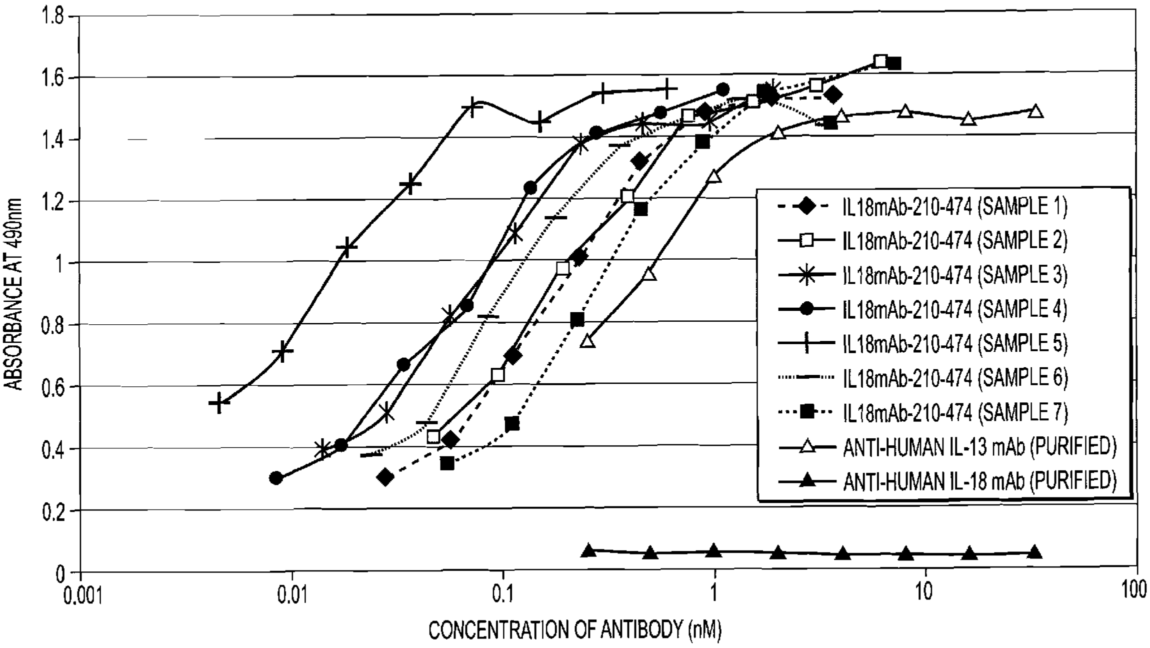
1.8
1.6
1.4
1.2
1
0.8
0.6
0.4
0.2
0
ABSORBANCE AT 490nm
0.001
0.01
0.1
1
10
100
CONCENTRATION OF ANTIBODY (nM)
IL18mAb-210-474 (SAMPLE 1)
IL18mAb-210-474 (SAMPLE 2)
IL18mAb-210-474 (SAMPLE 3)
IL18mAb-210-474 (SAMPLE 4)
IL18mAb-210-474 (SAMPLE 5)
IL18mAb-210-474 (SAMPLE 6)
IL18mAb-210-474 (SAMPLE 7)
ANTI-HUMAN IL-13 mAb (PURIFIED)
ANTI-HUMAN IL-18 mAb (PURIFIED)

*FIG. 38*

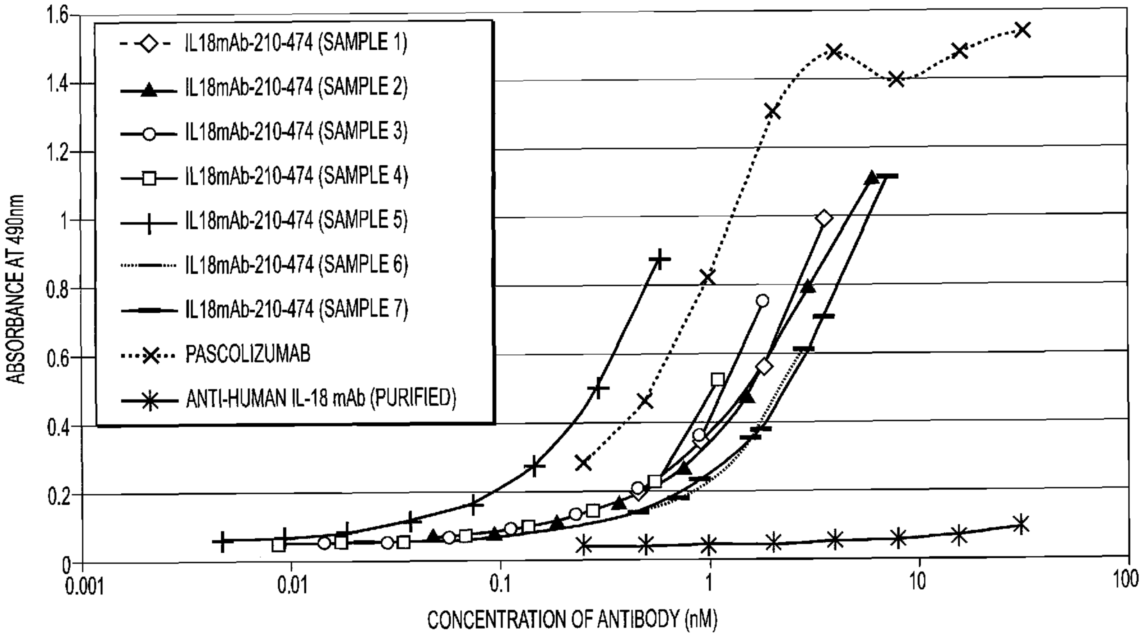
ABSORBANCE AT 490nm
1.6
1.4
1.2
1
0.8
0.6
0.4
0.2
0
0.001
0.01
0.1
1
10
100
CONCENTRATION OF ANTIBODY (nM)
IL18mAb-210-474 (SAMPLE 1)
IL18mAb-210-474 (SAMPLE 2)
IL18mAb-210-474 (SAMPLE 3)
IL18mAb-210-474 (SAMPLE 4)
IL18mAb-210-474 (SAMPLE 5)
IL18mAb-210-474 (SAMPLE 6)
IL18mAb-210-474 (SAMPLE 7)
PASCOLIZUMAB
ANTI-HUMAN IL-18 mAb (PURIFIED)

*FIG. 39*

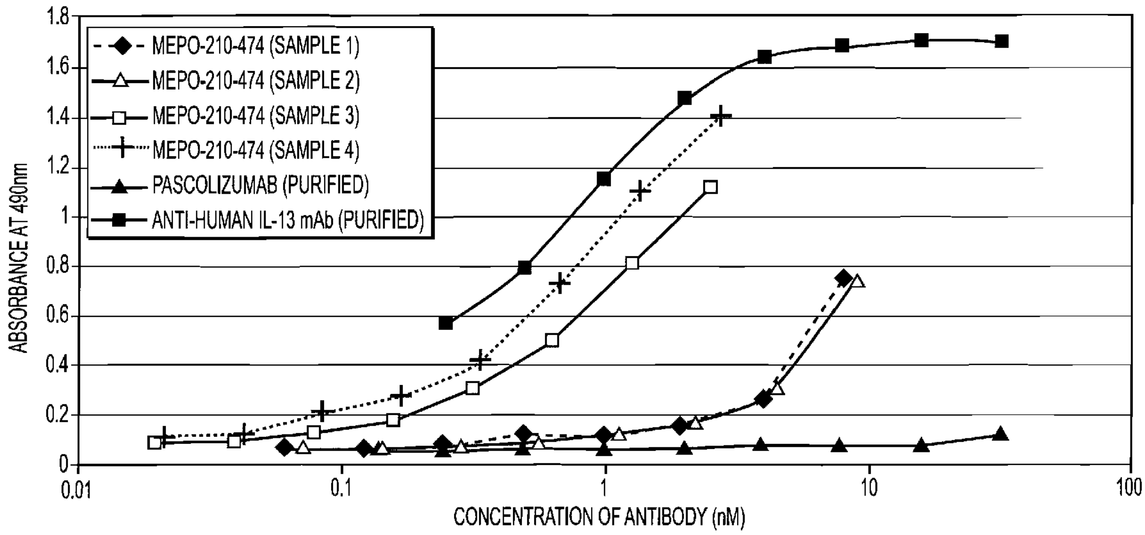
MEPO-210-474 (SAMPLE 1)
MEPO-210-474 (SAMPLE 2)
MEPO-210-474 (SAMPLE 3)
MEPO-210-474 (SAMPLE 4)
PASCOLIZUMAB (PURIFIED)
ANTI-HUMAN IL-13 mAb (PURIFIED)
ABSORBANCE AT 490nm
1.8
1.6
1.4
1.2
1
0.8
0.6
0.4
0.2
0
0.01
0.1
1
10
100
CONCENTRATION OF ANTIBODY (nM)

*FIG. 40*

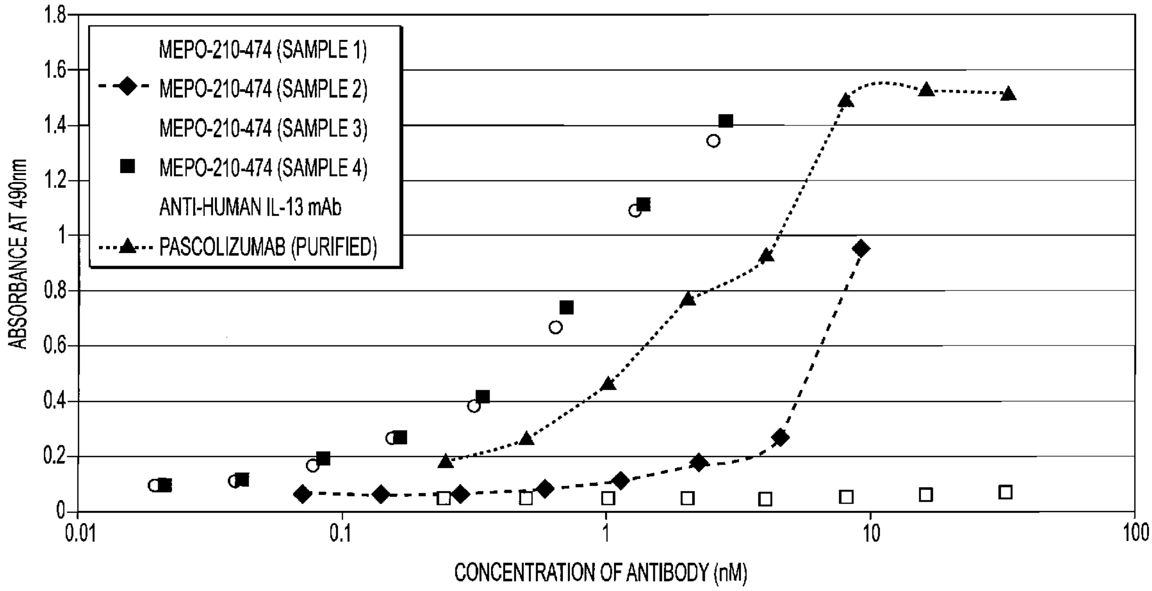
MEPO-210-474 (SAMPLE 1)
MEPO-210-474 (SAMPLE 2)
MEPO-210-474 (SAMPLE 3)
MEPO-210-474 (SAMPLE 4)
ANTI-HUMAN IL-13 mAb
PASCOLIZUMAB (PURIFIED)
ABSORBANCE AT 490nm
1.8
1.6
1.4
1.2
1
0.8
0.6
0.4
0.2
0
0.01
0.1
1
10
100
CONCENTRATION OF ANTIBODY (nM)

*FIG. 41*

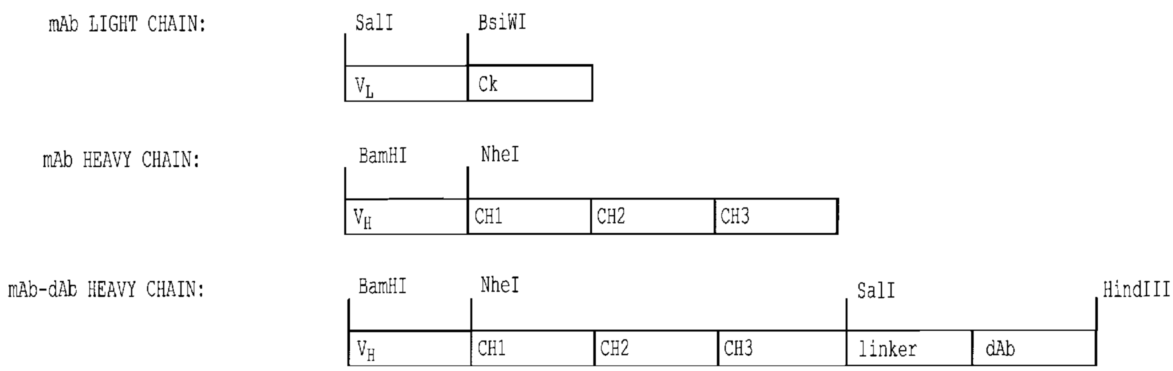


CLONING OF THE ANTI-TNF/ANTI-EGFR mAb-dAb (EXAMPLE 10)

*FIG. 42*

SDS-PAGE ANALYSIS OF THE ANTI-TNF/ANTI-EGFR mAb-dAb (EXAMPLE 10)

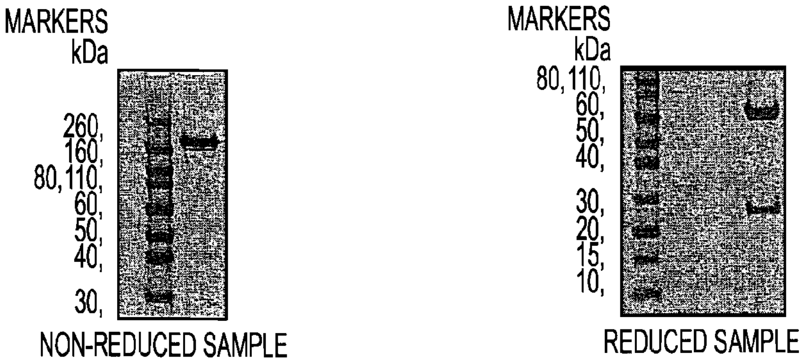


*FIG. 43*

SEC PROFILE OF THE ANTI-TNF/ANTI-EGFR mAb-dAb (EXAMPLE 10)

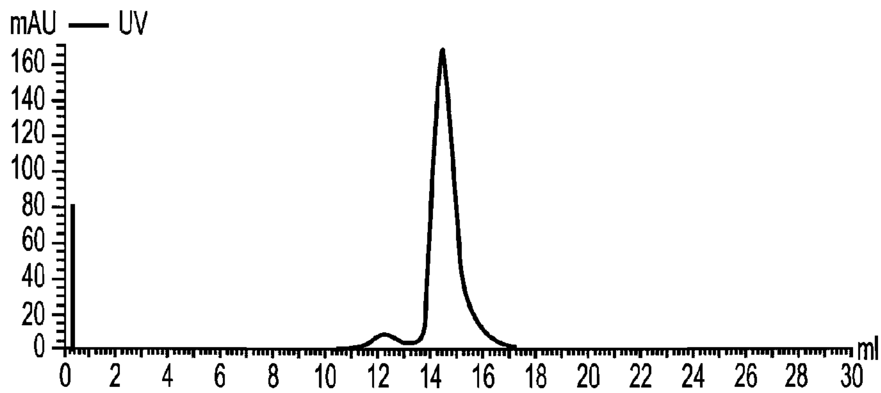


*FIG. 44*

ANTI-EGFR ACTIVITY OF EXAMPLE 10

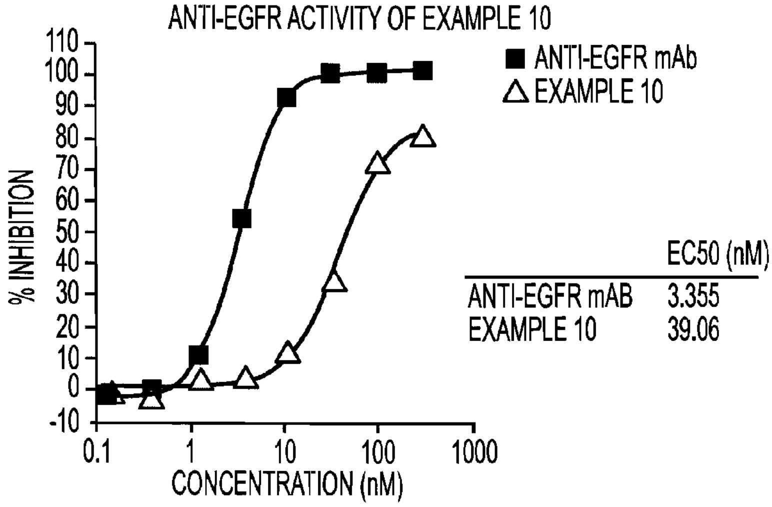


*FIG. 45*

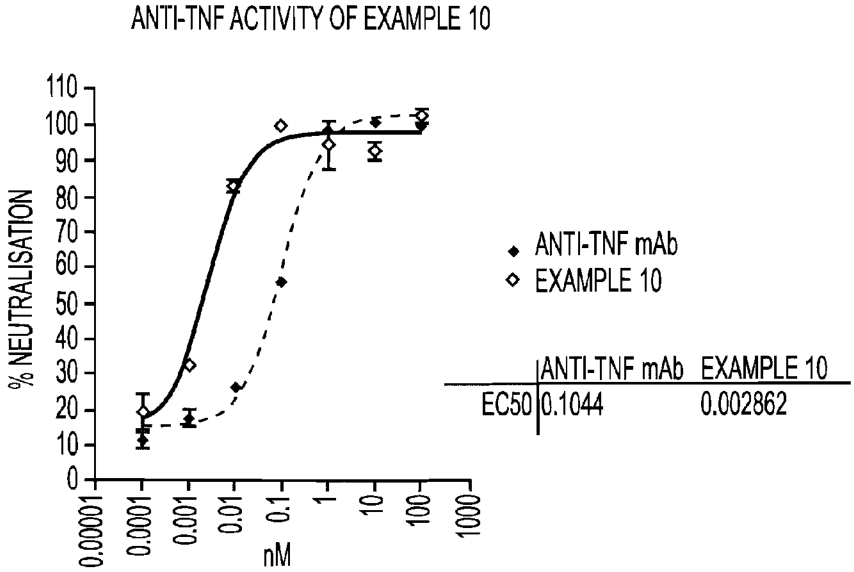


*FIG. 46*

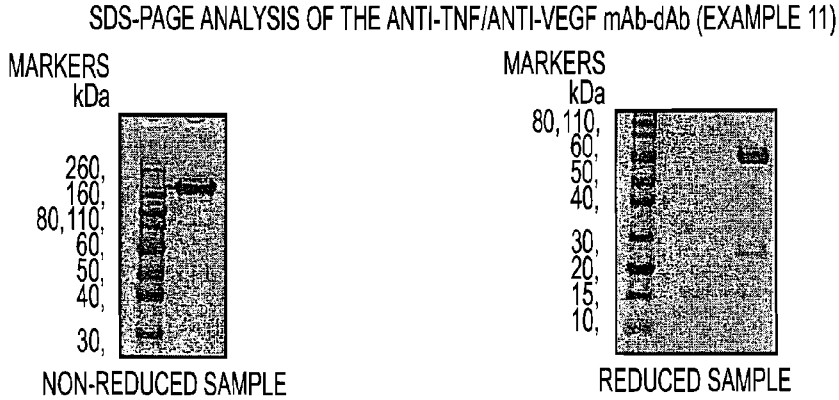


*FIG. 47*

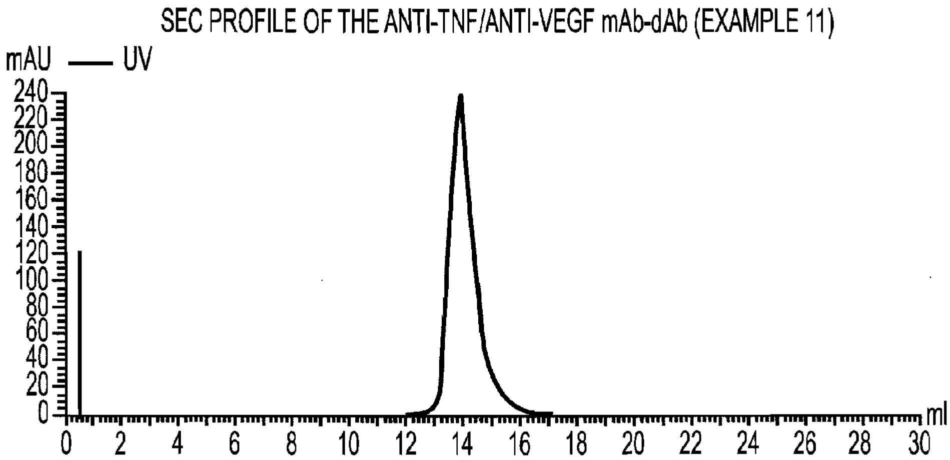


*FIG. 48*

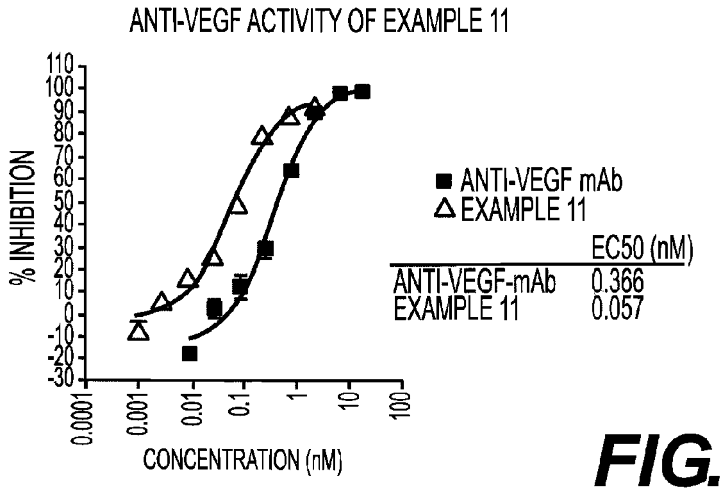


*FIG. 49*

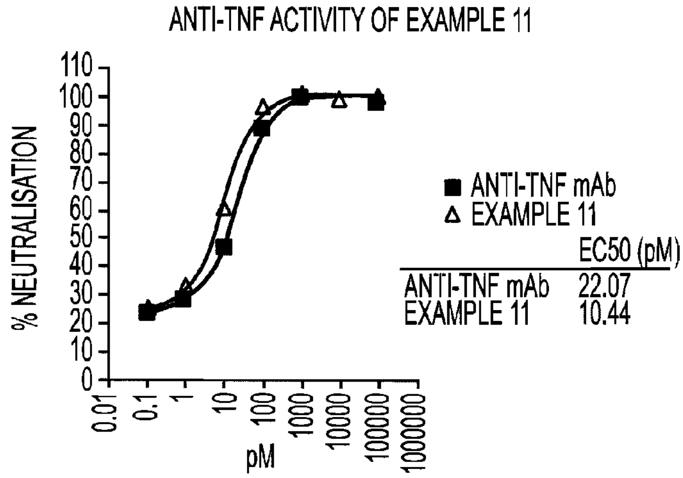


*FIG. 50*

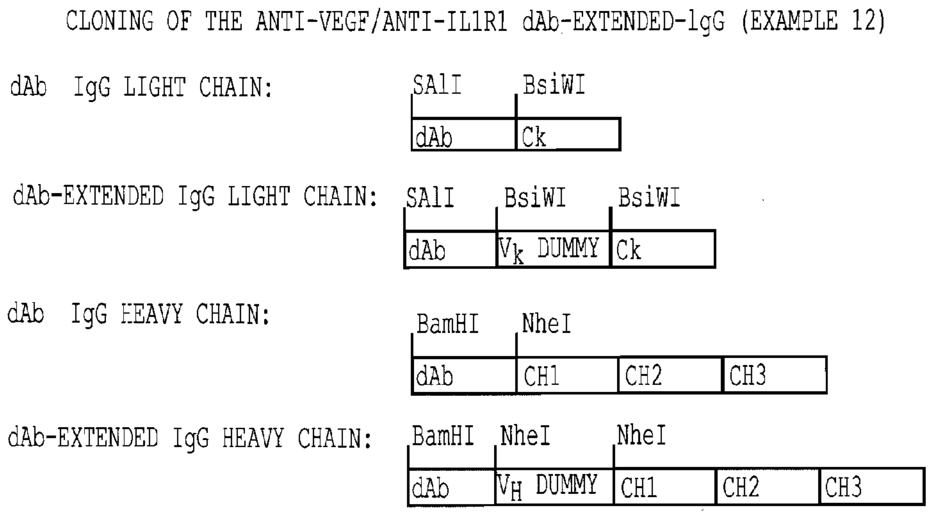


*FIG. 51*

SDS-PAGE ANALYSIS OF THE ANTI-TNF/ANTI-VEGF dAb-EXTENDED IgG A (EXAMPLE 12)

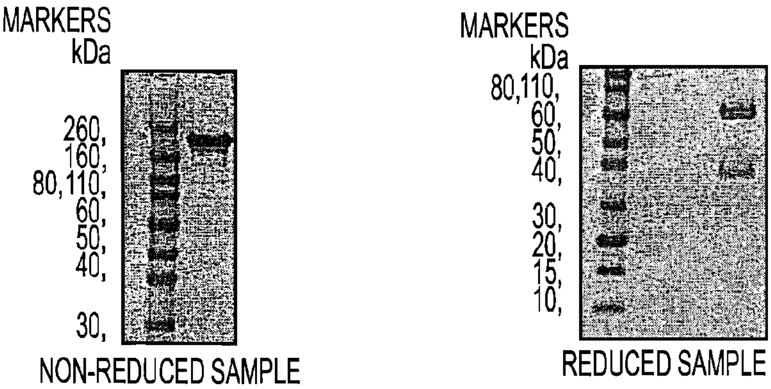


*FIG. 52*

SDS-PAGE ANALYSIS OF THE ANTI-TNF/ANTI-VEGF dAb-EXTENDED IgG B (EXAMPLE 12)

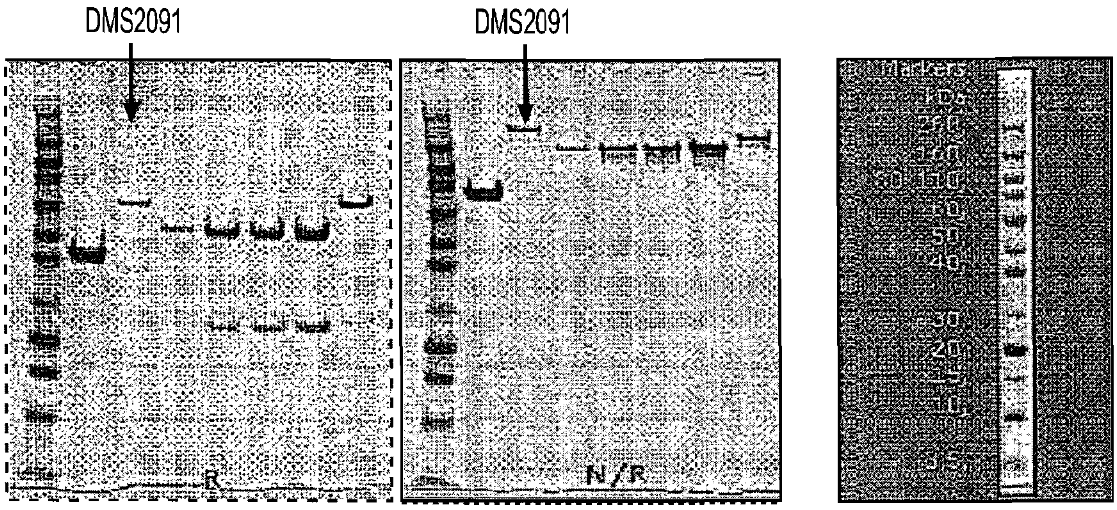


N/R=NON-REDUCED; R=REDUCED

*FIG. 53*

SEC PROFILE OF THE ANTI-TNF/ANTI-VEGF dAb-EXTENDED IgG A (EXAMPLE 12)

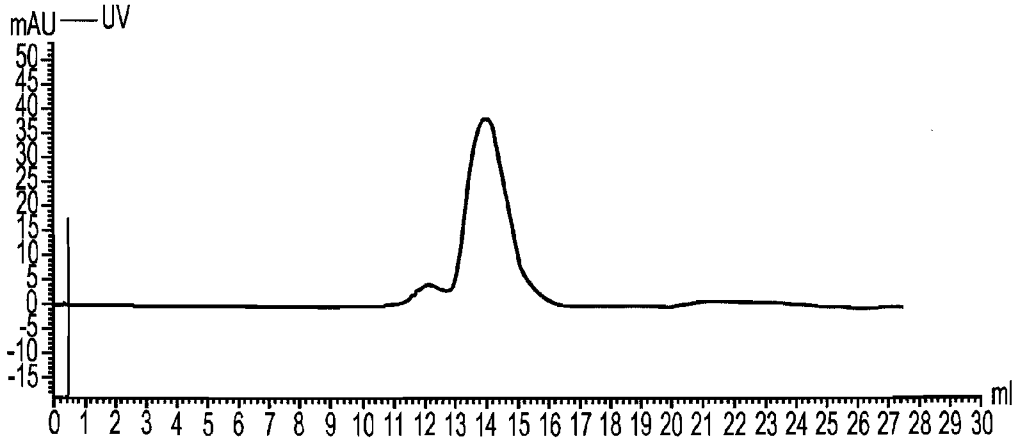


*FIG. 54*

SEC PROFILE OF THE ANTI-TNF/ANTI-VEGF dAb-EXTENDED IgG B (EXAMPLE 12)

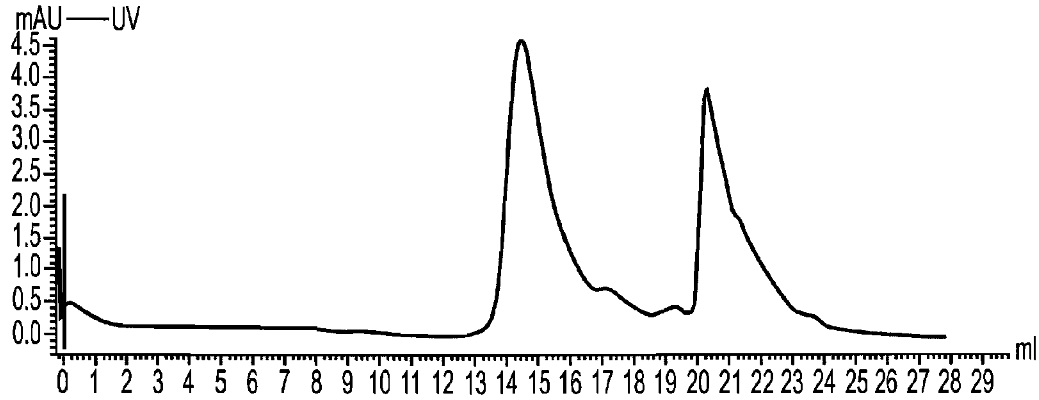


*FIG. 55*

ANTI-VEGF ACTIVITY OF EXAMPLE 12 (DMS2091)

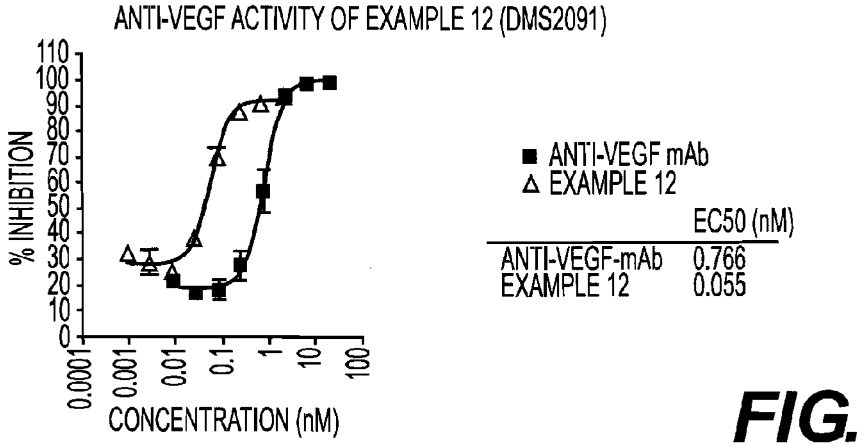


*FIG. 56*

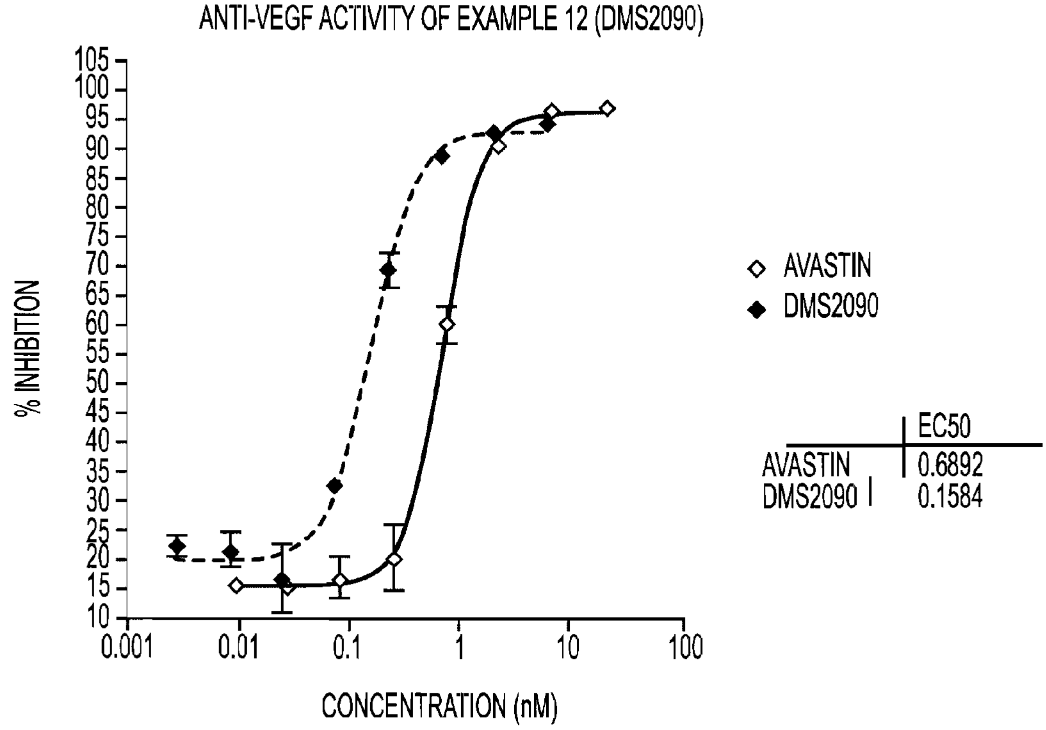
ANTI-VEGF ACTIVITY OF EXAMPLE 12 (DMS2090)
% INHIBITION
105
100
95
90
85
80
75
70
65
60
55
50
45
40
35
30
25
20
15
10
0.001
0.01
0.1
1
10
100
CONCENTRATION (nM)
AVASTIN
DMS2090
EC50
AVASTIN 0.6892
DMS2090 0.1584

FIG. 57

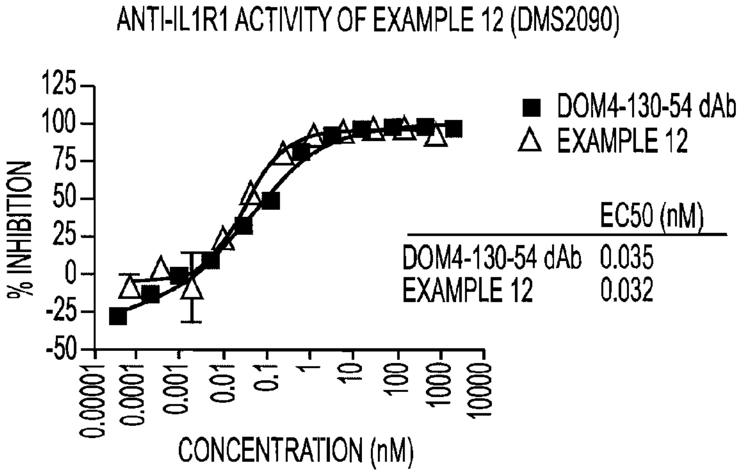
ANTI-IL1R1 ACTIVITY OF EXAMPLE 12 (DMS2090)
% INHIBITION
125
100
75
50
25
0
-25
-50
0.00001
0.0001
0.001
0.01
0.1
1
10
100
1000
10000
CONCENTRATION (nM)
DOM4-130-54 dAb
EXAMPLE 12
EC50 (nM)
DOM4-130-54 dAb 0.035
EXAMPLE 12 0.032

FIG. 58

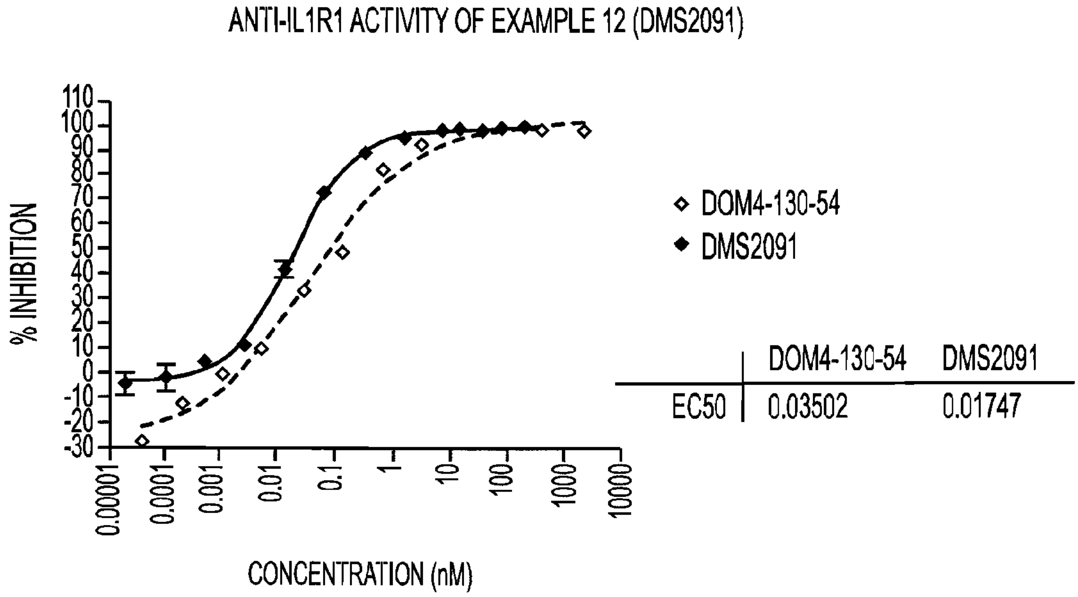
ANTI-IL1R1 ACTIVITY OF EXAMPLE 12 (DMS2091)
% INHIBITION
110
100
90
80
70
60
50
40
30
20
10
0
-10
-20
-30
0.00001
0.0001
0.001
0.01
0.1
1
10
100
1000
10000
CONCENTRATION (nM)
DOM4-130-54
DMS2091
DOM4-130-54 DMS2091
EC50 0.03502 0.01747

FIG. 59

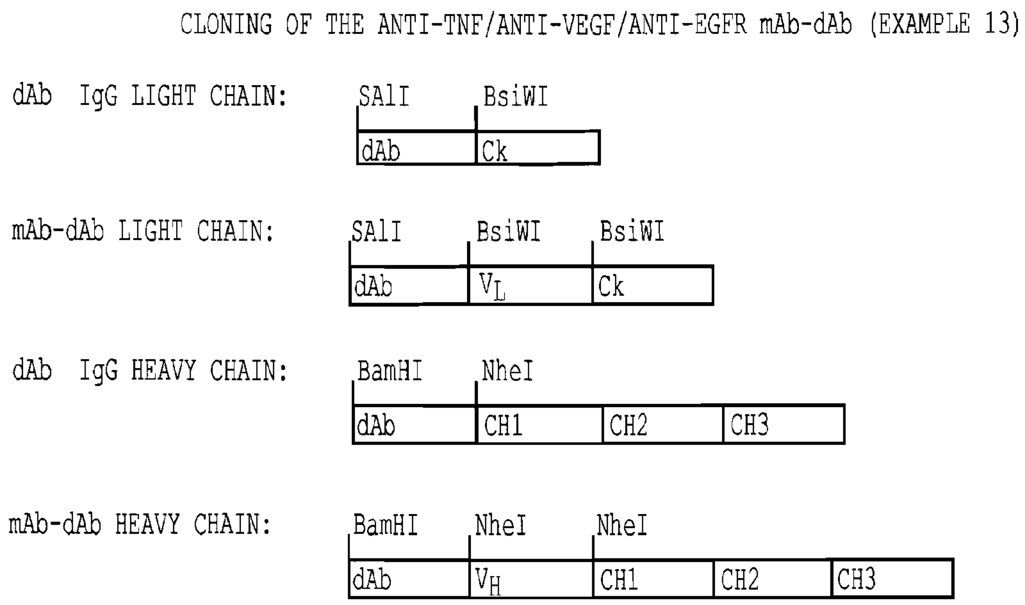
CLONING OF THE ANTI-TNF/ANTI-VEGF/ANTI-EGFR mAb-dAb (EXAMPLE 13)
dAb IgG LIGHT CHAIN:
SAlI
BsiWI
dAb
Ck
mAb-dAb LIGHT CHAIN:
SAlI
BsiWI
BsiWI
dAb
VL
Ck
dAb IgG HEAVY CHAIN:
BamHI
NheI
dAb
CH1
CH2
CH3
mAb-dAb HEAVY CHAIN:
BamHI
NheI
NheI
dAb
VH
CH1
CH2
CH3

FIG. 60

SDS-PAGE ANALYSIS OF THE ANTI-TNF/ANTI-VEGF/ANTI-EGFR mAb-dAb (EXAMPLE 13)

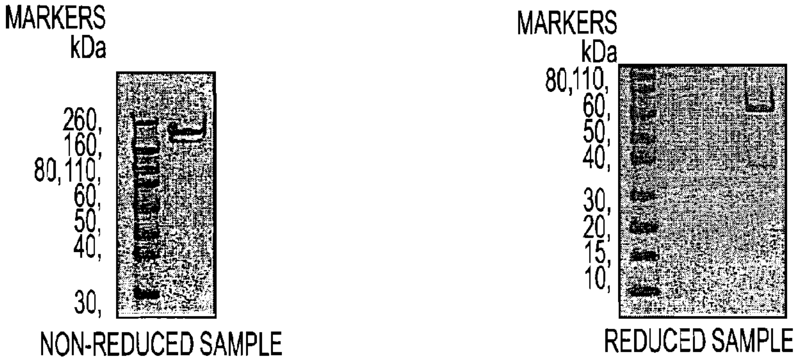


*FIG. 61*

ANTI-VEGF ACTIVITY OF EXAMPLE 13

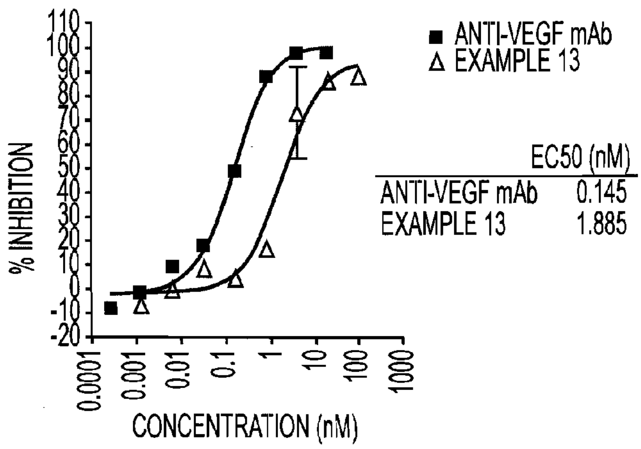


*FIG. 62*

ANTI-TNF ACTIVITY OF EXAMPLE 13

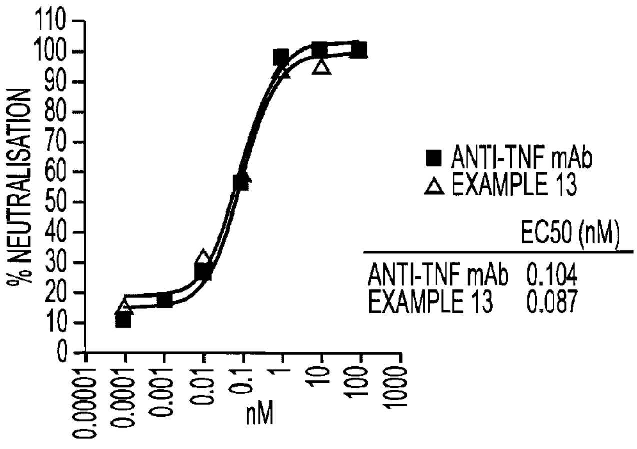


*FIG. 63*

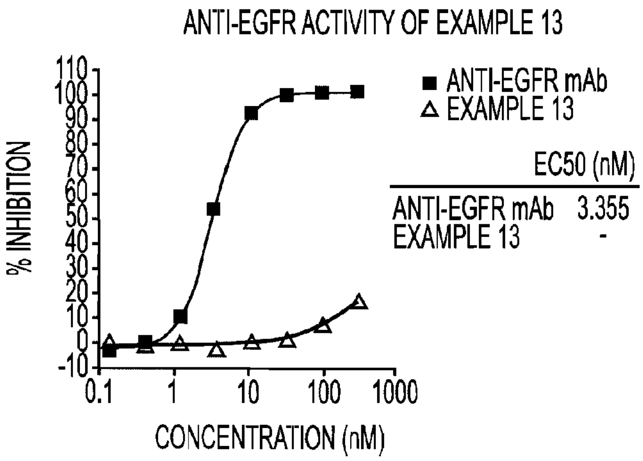


FIG. 64

SEC ANALYSIS OF PURIFIED BISPECIFIC ANTIBODIES, BPC1603 (A), BPC1604 (B), BPC1605 (C), BPC1606 (D)

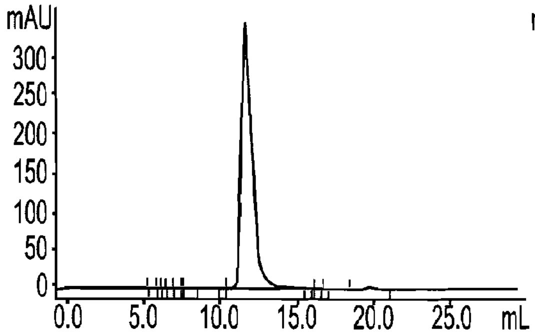


FIG. 65A

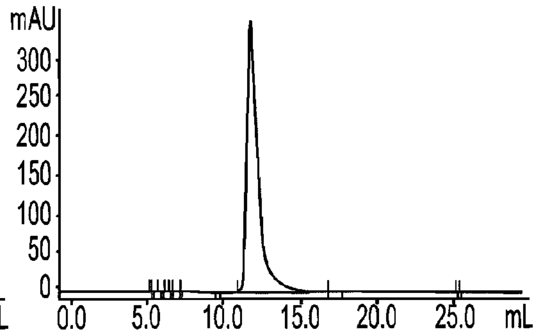


FIG. 65B

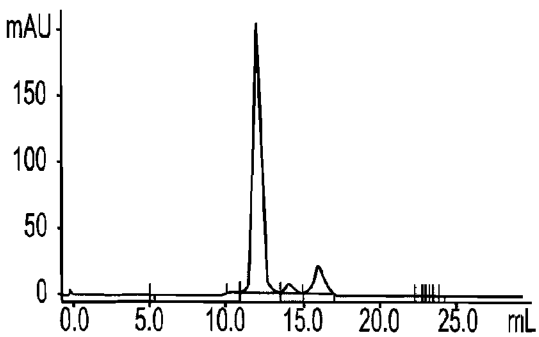


FIG. 65C

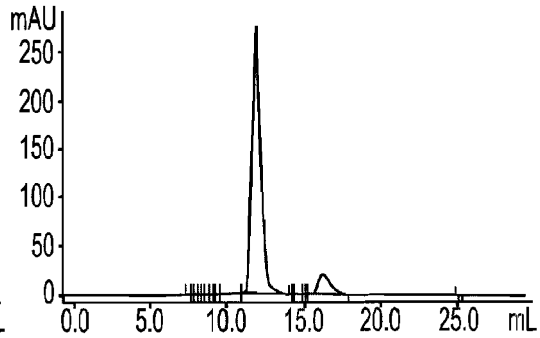


FIG. 65D

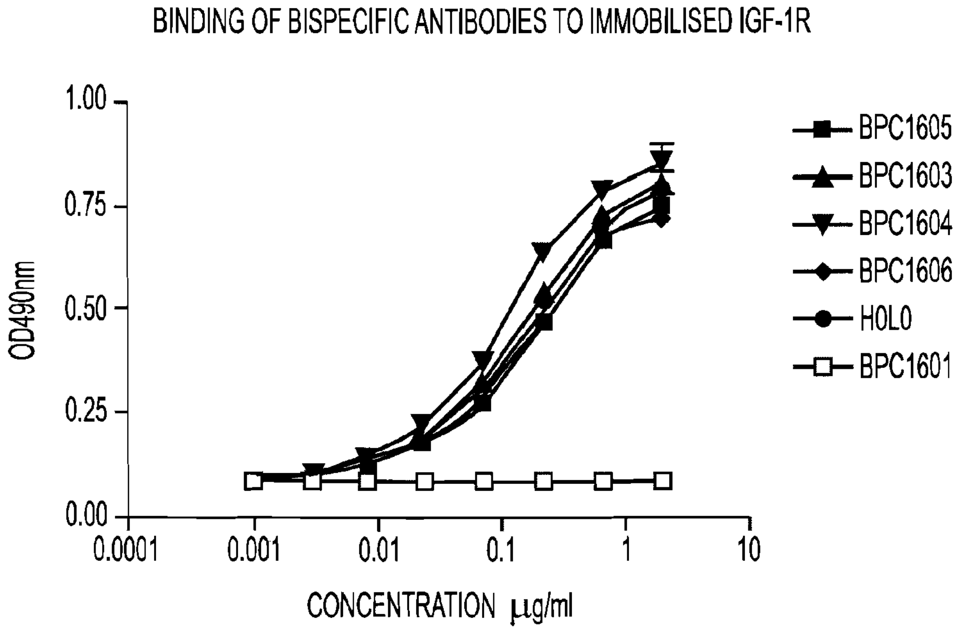
BINDING OF BISPECIFIC ANTIBODIES TO IMMOBILISED IGF-1R
1.00
0.75
0.50
0.25
0.00
OD490nm
0.0001
0.001
0.01
0.1
1
10
CONCENTRATION µg/ml
BPC1605
BPC1603
BPC1604
BPC1606
H0L0
BPC1601

*FIG. 66*

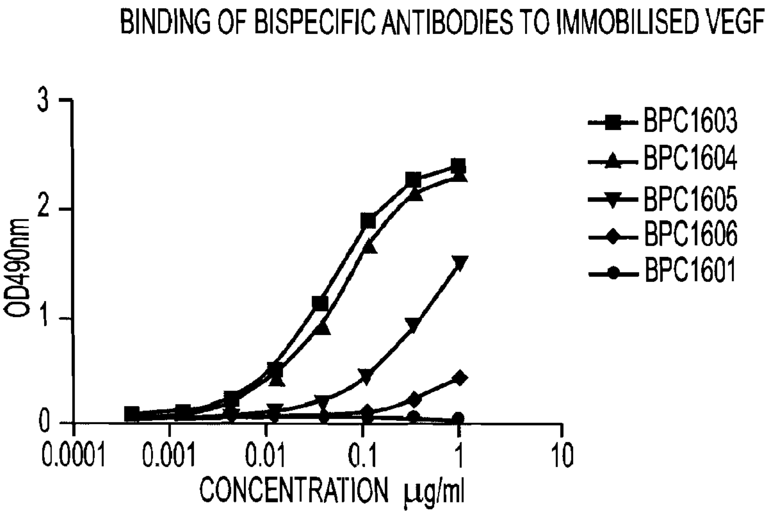
BINDING OF BISPECIFIC ANTIBODIES TO IMMOBILISED VEGF
3
2
1
0
OD490nm
0.0001
0.001
0.01
0.1
1
10
CONCENTRATION µg/ml
BPC1603
BPC1604
BPC1605
BPC1606
BPC1601

*FIG. 67*

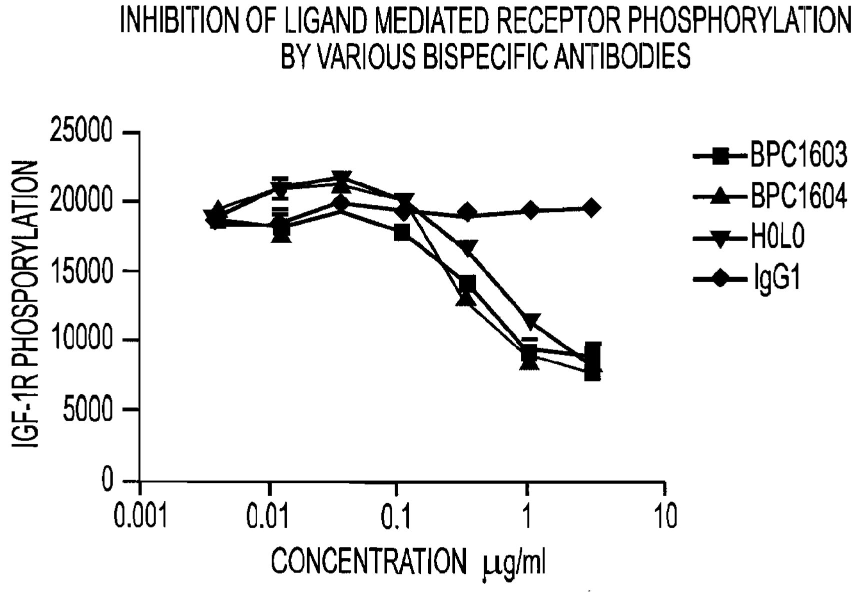
INHIBITION OF LIGAND MEDIATED RECEPTOR PHOSPHORYLATION
BY VARIOUS BISPECIFIC ANTIBODIES
IGF-1R PHOSPORYLATION
25000
20000
15000
10000
5000
0
0.001
0.01
0.1
1
10
CONCENTRATION μg/ml
BPC1603
BPC1604
H0L0
IgG1

*FIG. 68*

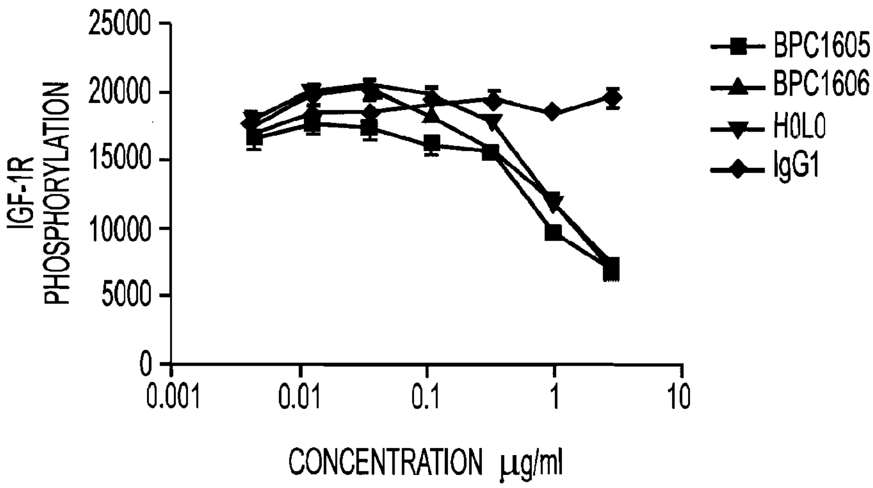
IGF-1R PHOSPHORYLATION
25000
20000
15000
10000
5000
0
0.001
0.01
0.1
1
10
CONCENTRATION μg/ml
BPC1605
BPC1606
H0L0
IgG1

*FIG. 69*

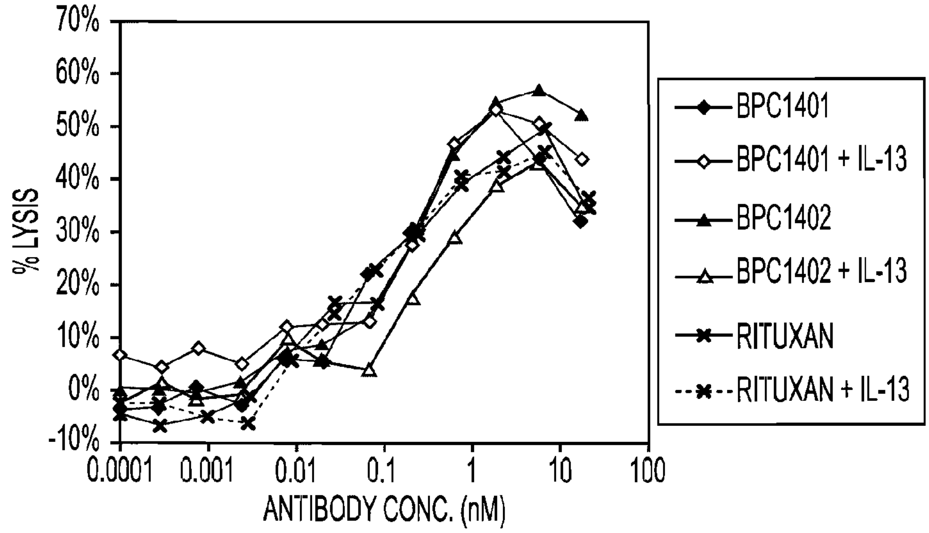
70%
60%
50%
40%
30%
20%
10%
0%
-10%
% LYSIS
0.0001
0.001
0.01
0.1
1
10
100
ANTIBODY CONC. (nM)
BPC1401
BPC1401 + IL-13
BPC1402
BPC1402 + IL-13
RITUXAN
RITUXAN + IL-13

FIG. 70

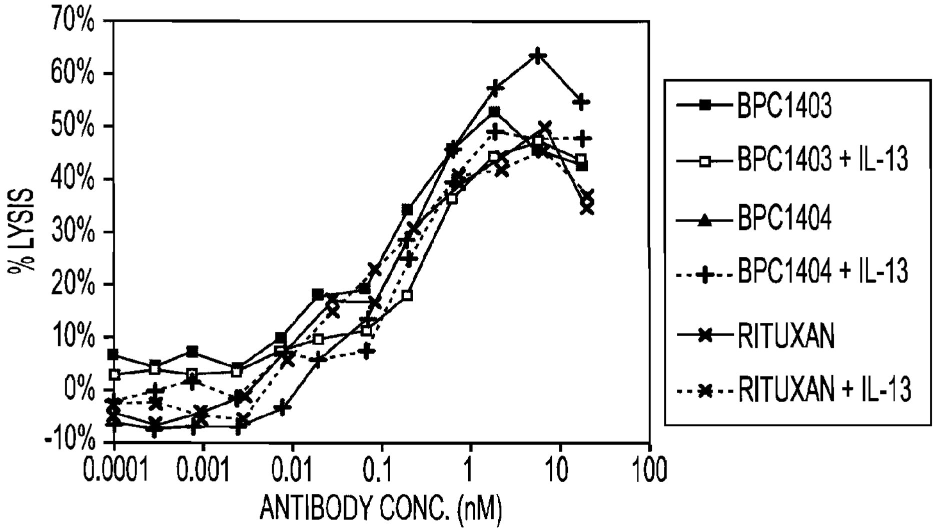
70%
60%
50%
40%
30%
20%
10%
0%
-10%
% LYSIS
0.0001
0.001
0.01
0.1
1
10
100
ANTIBODY CONC. (nM)
BPC1403
BPC1403 + IL-13
BPC1404
BPC1404 + IL-13
RITUXAN
RITUXAN + IL-13

FIG. 71

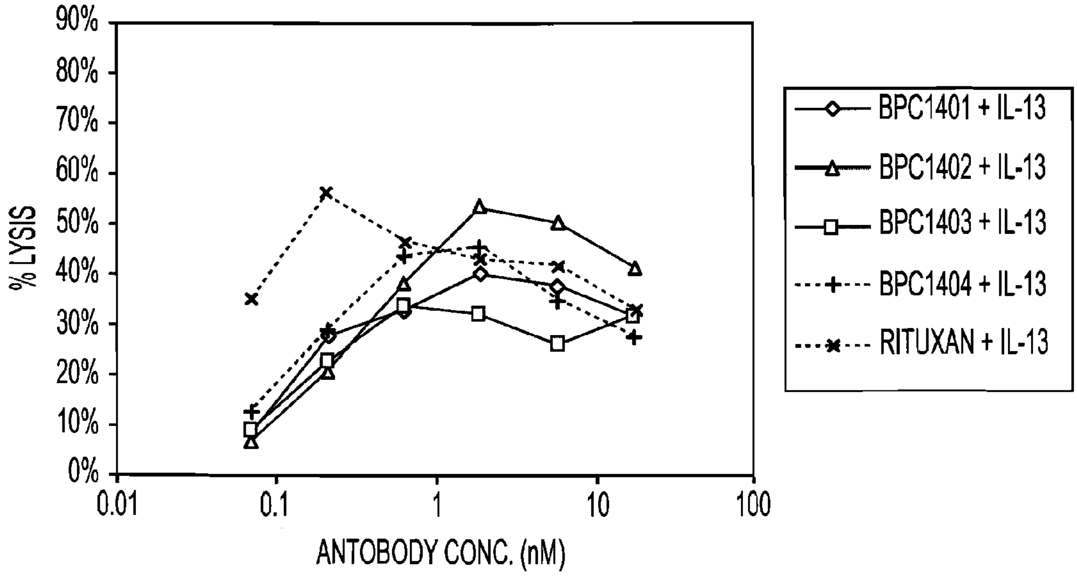
90%
80%
70%
60%
50%
40%
30%
20%
10%
0%
% LYSIS
0.01
0.1
1
10
100
ANTOBODY CONC. (nM)
BPC1401 + IL-13
BPC1402 + IL-13
BPC1403 + IL-13
BPC1404 + IL-13
RITUXAN + IL-13

*FIG. 72*

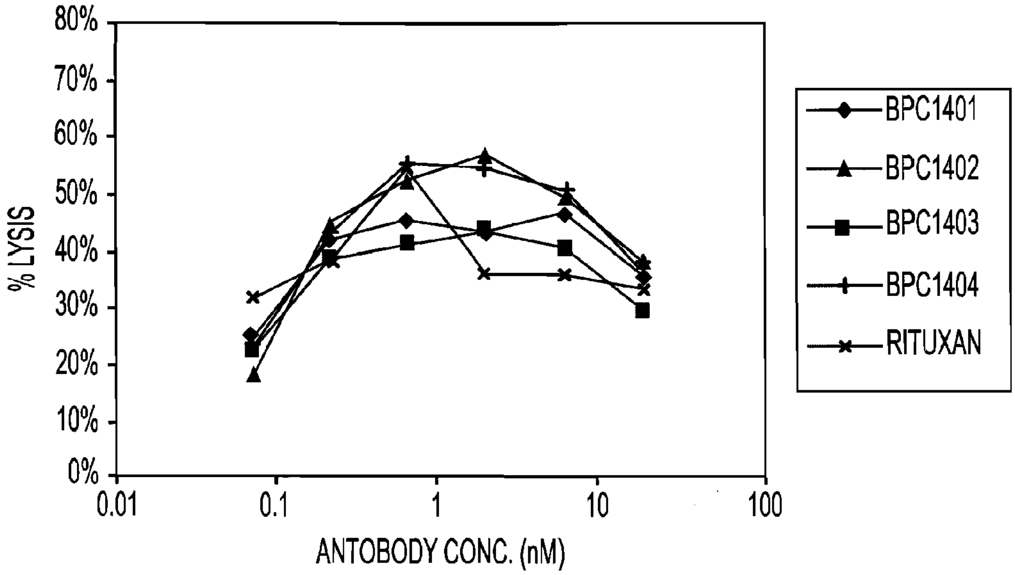
80%
70%
60%
50%
40%
30%
20%
10%
0%
% LYSIS
0.01
0.1
1
10
100
ANTOBODY CONC. (nM)
BPC1401
BPC1402
BPC1403
BPC1404
RITUXAN

*FIG. 73*

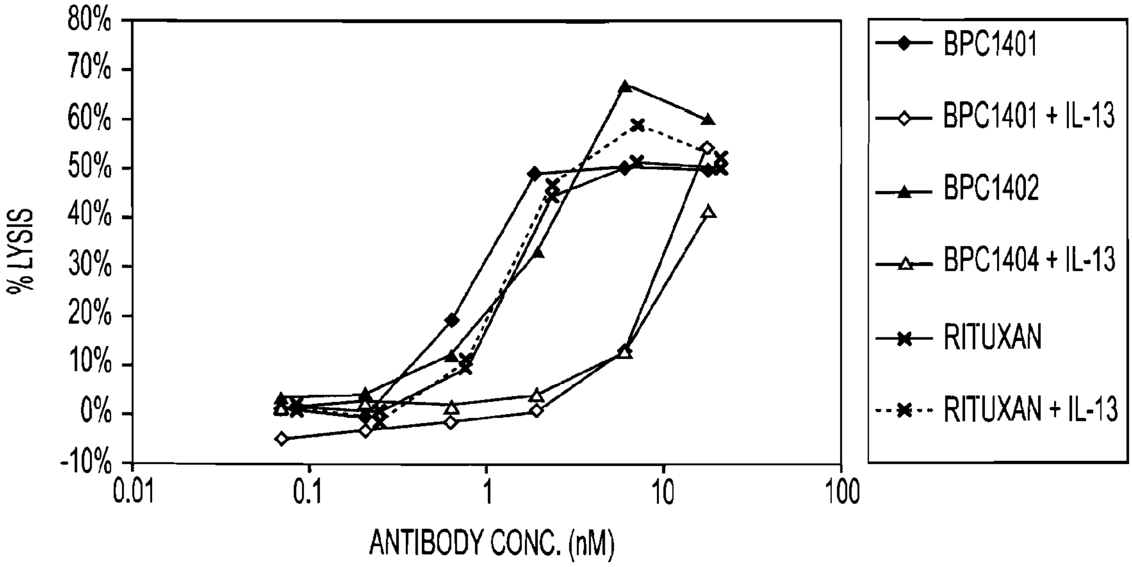
80%
70%
60%
50%
40%
30%
20%
10%
0%
-10%
% LYSIS
0.01
0.1
1
10
100
ANTIBODY CONC. (nM)
BPC1401
BPC1401 + IL-13
BPC1402
BPC1404 + IL-13
RITUXAN
RITUXAN + IL-13

FIG. 74

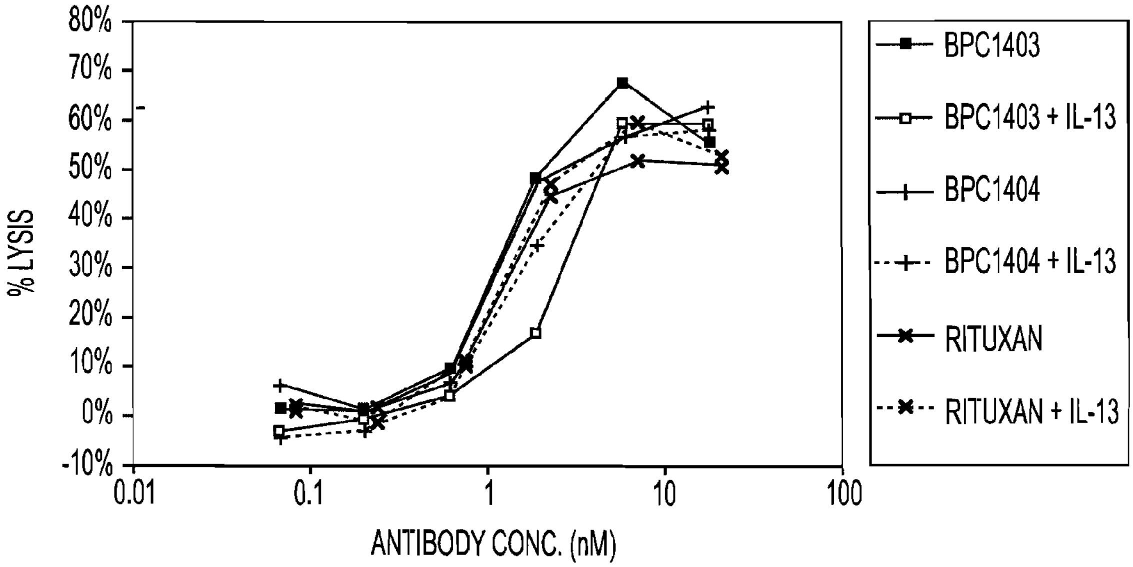
80%
70%
60%
50%
40%
30%
20%
10%
0%
-10%
% LYSIS
0.01
0.1
1
10
100
ANTIBODY CONC. (nM)
BPC1403
BPC1403 + IL-13
BPC1404
BPC1404 + IL-13
RITUXAN
RITUXAN + IL-13

FIG. 75

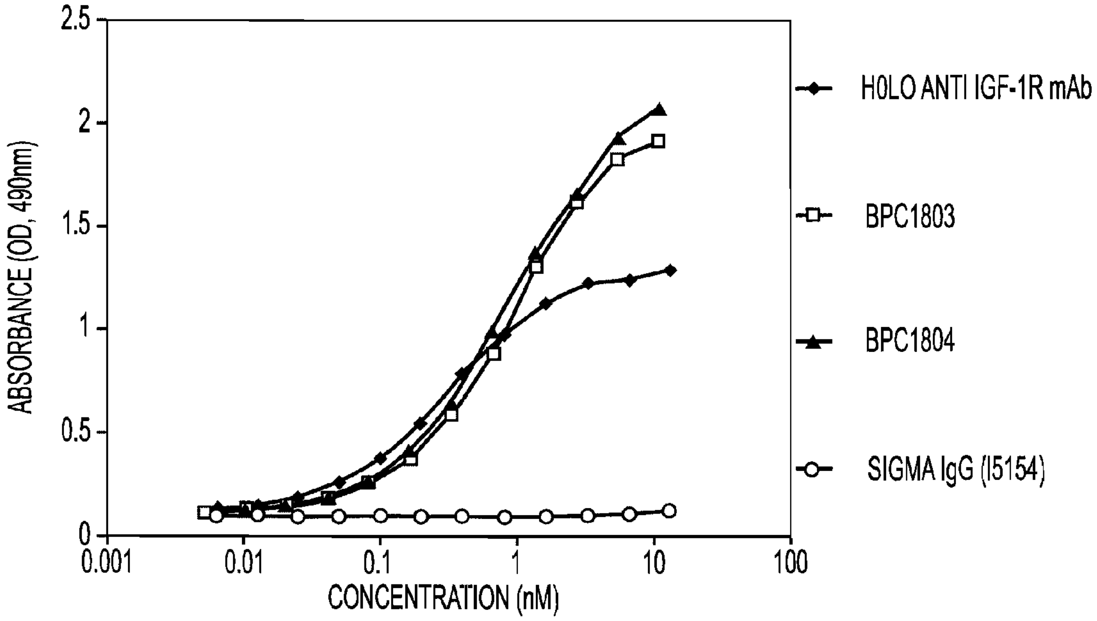
2.5
2
1.5
1
0.5
0
ABSORBANCE (OD, 490nm)
0.001
0.01
0.1
1
10
100
CONCENTRATION (nM)
H0LO ANTI IGF-1R mAb
BPC1803
BPC1804
SIGMA IgG (I5154)

*FIG. 76*

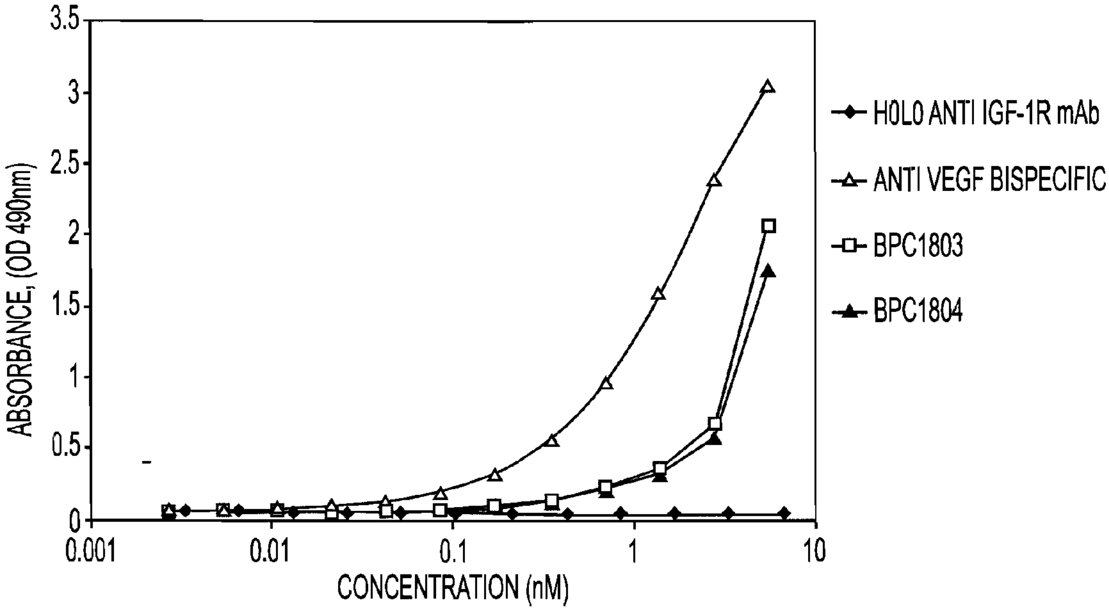
3.5
3
2.5
2
1.5
1
0.5
0
ABSORBANCE, (OD 490nm)
0.001
0.01
0.1
1
10
CONCENTRATION (nM)
H0L0 ANTI IGF-1R mAb
ANTI VEGF BISPECIFIC
BPC1803
BPC1804

*FIG. 77*

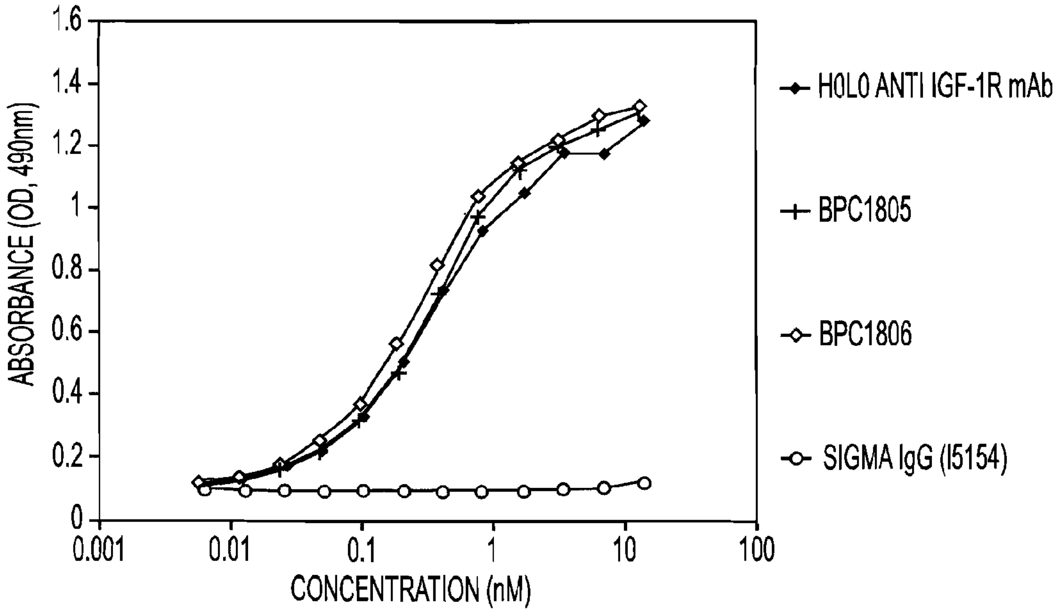
1.6
1.4
1.2
1
0.8
0.6
0.4
0.2
0
ABSORBANCE (OD, 490nm)
0.001
0.01
0.1
1
10
100
CONCENTRATION (nM)
H0L0 ANTI IGF-1R mAb
BPC1805
BPC1806
SIGMA IgG (I5154)

*FIG. 78*

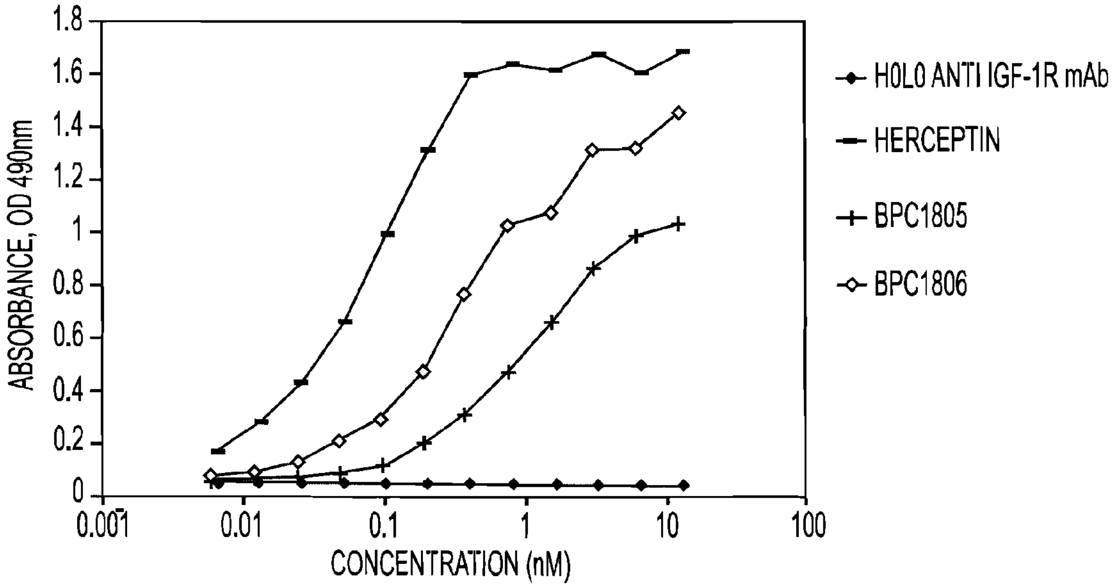
1.8
1.6
1.4
1.2
1
0.8
0.6
0.4
0.2
0
ABSORBANCE, OD 490nm
0.001
0.01
0.1
1
10
100
CONCENTRATION (nM)
H0L0 ANTI IGF-1R mAb
HERCEPTIN
BPC1805
BPC1806

*FIG. 79*

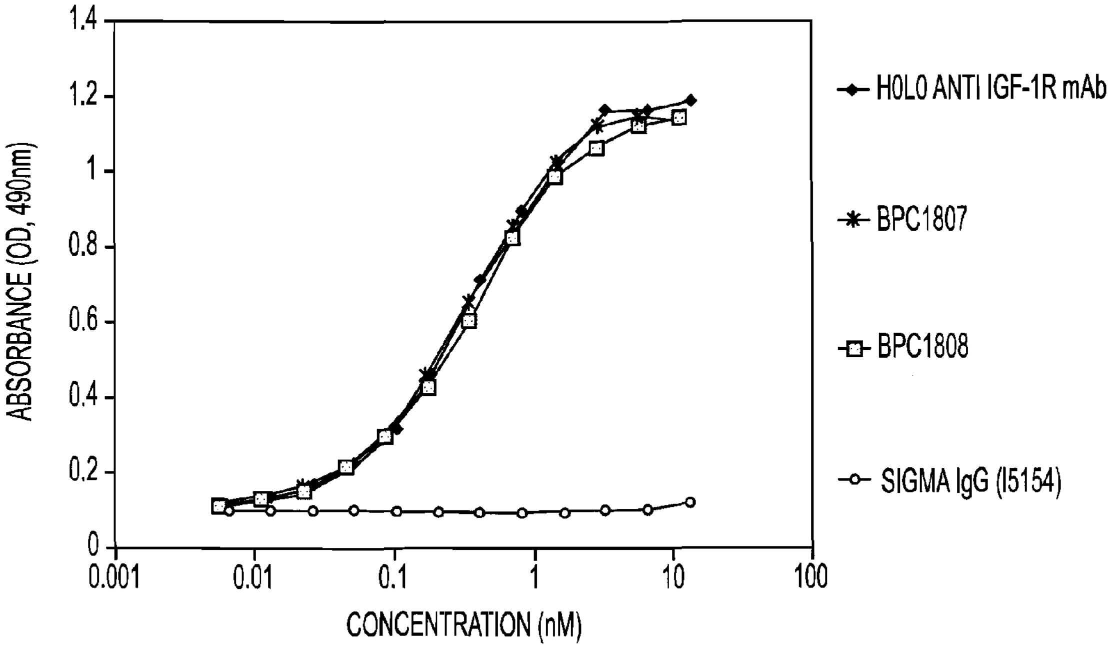
1.4
1.2
1
0.8
0.6
0.4
0.2
0
ABSORBANCE (OD, 490nm)
0.001
0.01
0.1
1
10
100
CONCENTRATION (nM)
H0L0 ANTI IGF-1R mAb
BPC1807
BPC1808
SIGMA IgG (I5154)

*FIG. 80*

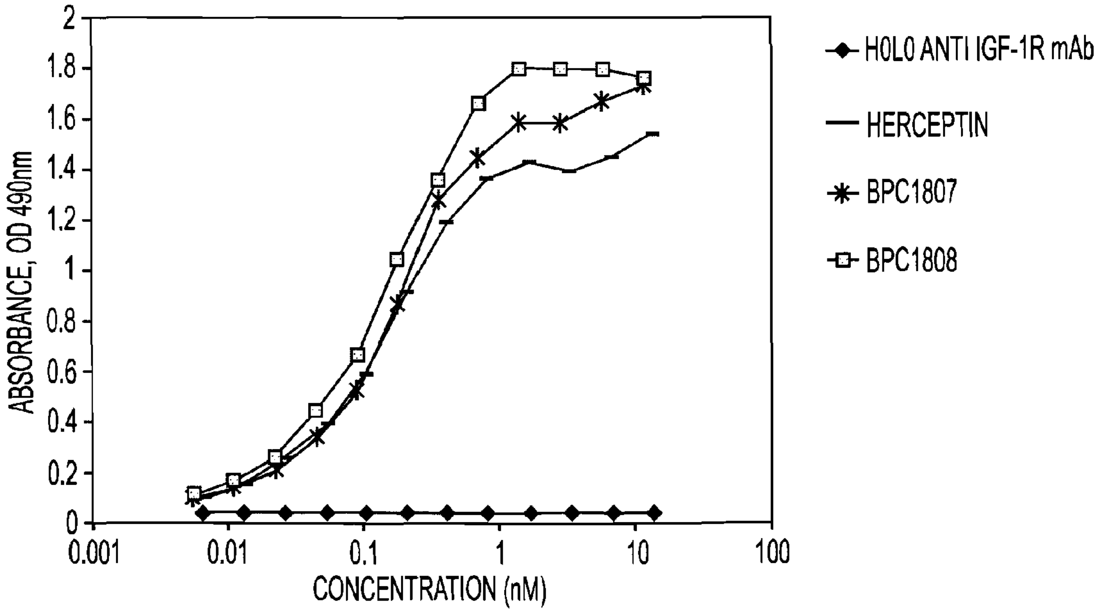
2
1.8
1.6
1.4
1.2
1
0.8
0.6
0.4
0.2
0
ABSORBANCE, OD 490nm
0.001
0.01
0.1
1
10
100
CONCENTRATION (nM)
H0L0 ANTI IGF-1R mAb
HERCEPTIN
BPC1807
BPC1808

*FIG. 81*

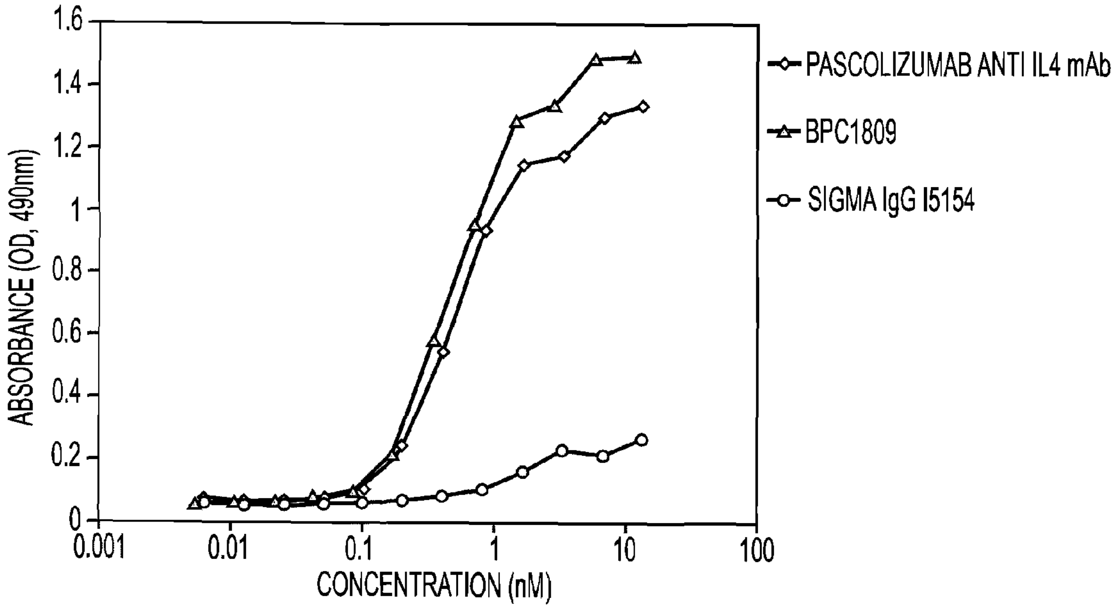
1.6
1.4
1.2
1
0.8
0.6
0.4
0.2
0
ABSORBANCE (OD, 490nm)
0.001
0.01
0.1
1
10
100
CONCENTRATION (nM)
PASCOLIZUMAB ANTI IL4 mAb
BPC1809
SIGMA IgG I5154

*FIG. 82*

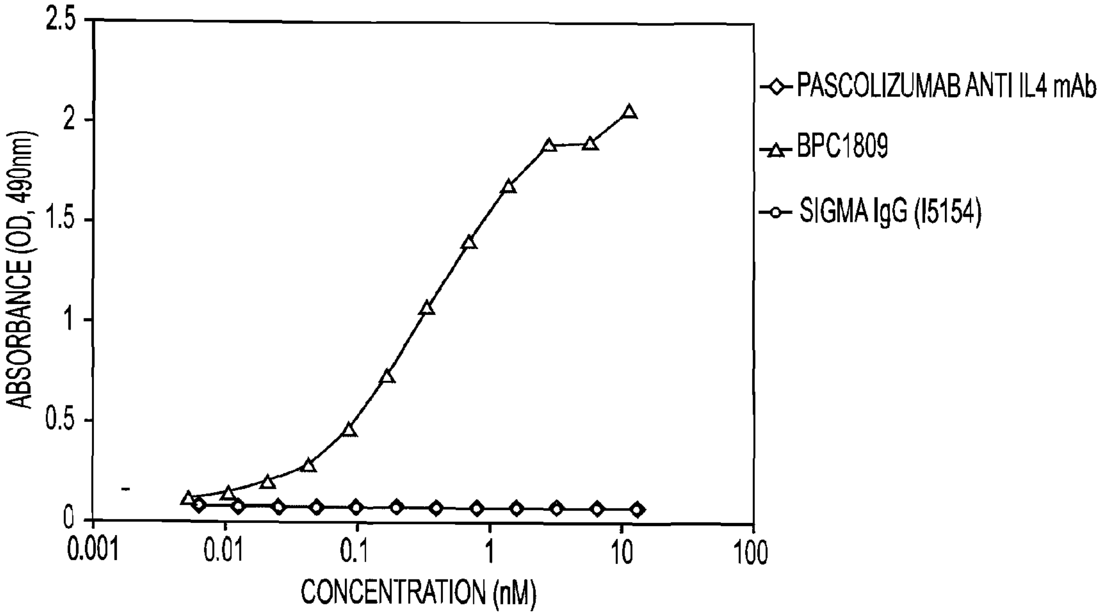
2.5
2
1.5
1
0.5
0
ABSORBANCE (OD, 490nm)
0.001
0.01
0.1
1
10
100
CONCENTRATION (nM)
PASCOLIZUMAB ANTI IL4 mAb
BPC1809
SIGMA IgG (I5154)

*FIG. 83*

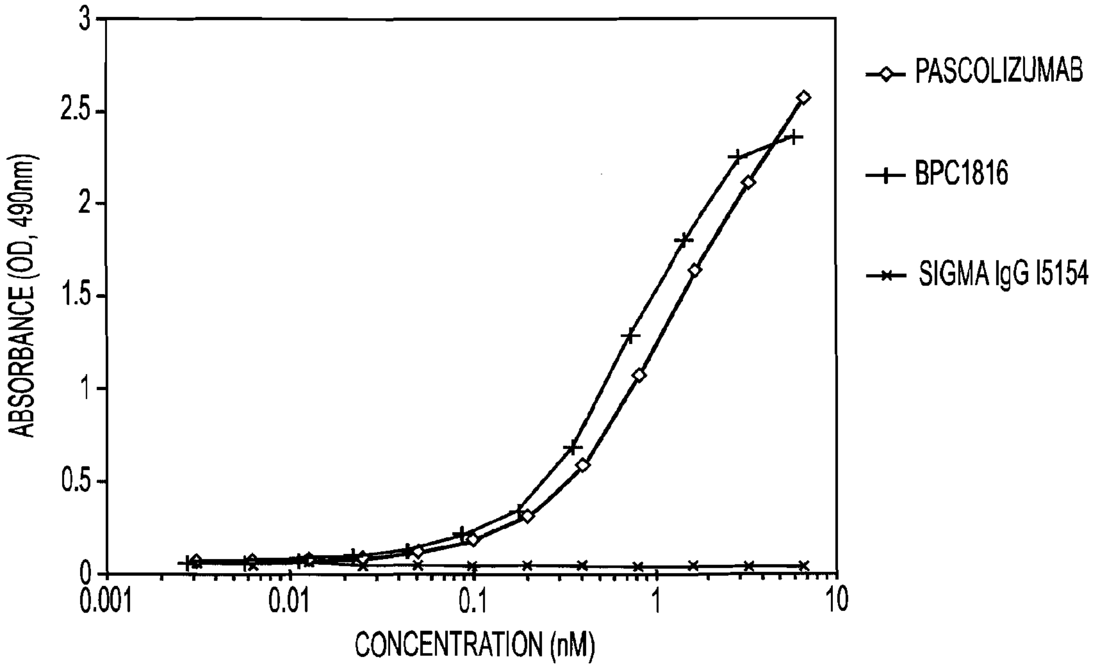

3
2.5
2
1.5
1
0.5
0
ABSORBANCE (OD, 490nm)
0.001
0.01
0.1
1
10
CONCENTRATION (nM)
PASCOLIZUMAB
BPC1816
SIGMA IgG I5154


FIG. 84

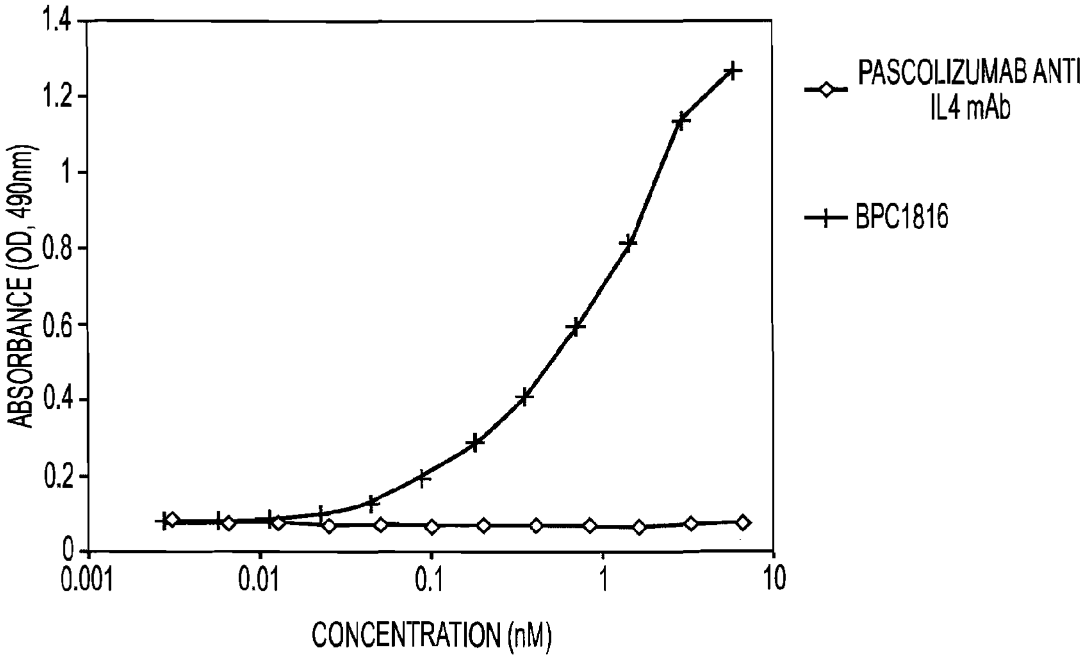

1.4
1.2
1
0.8
0.6
0.4
0.2
0
ABSORBANCE (OD, 490nm)
0.001
0.01
0.1
1
10
CONCENTRATION (nM)
PASCOLIZUMAB ANTI IL4 mAb
BPC1816


FIG. 85

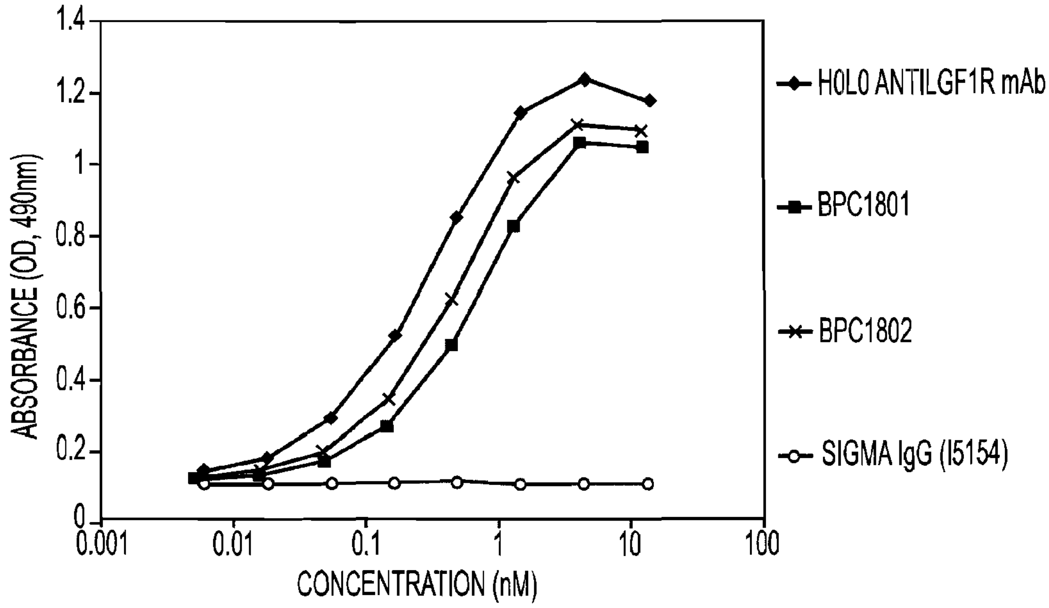
1.4
1.2
1
0.8
0.6
0.4
0.2
0
ABSORBANCE (OD, 490nm)
0.001
0.01
0.1
1
10
100
CONCENTRATION (nM)
H0L0 ANTILGF1R mAb
BPC1801
BPC1802
SIGMA IgG (I5154)

*FIG. 86*

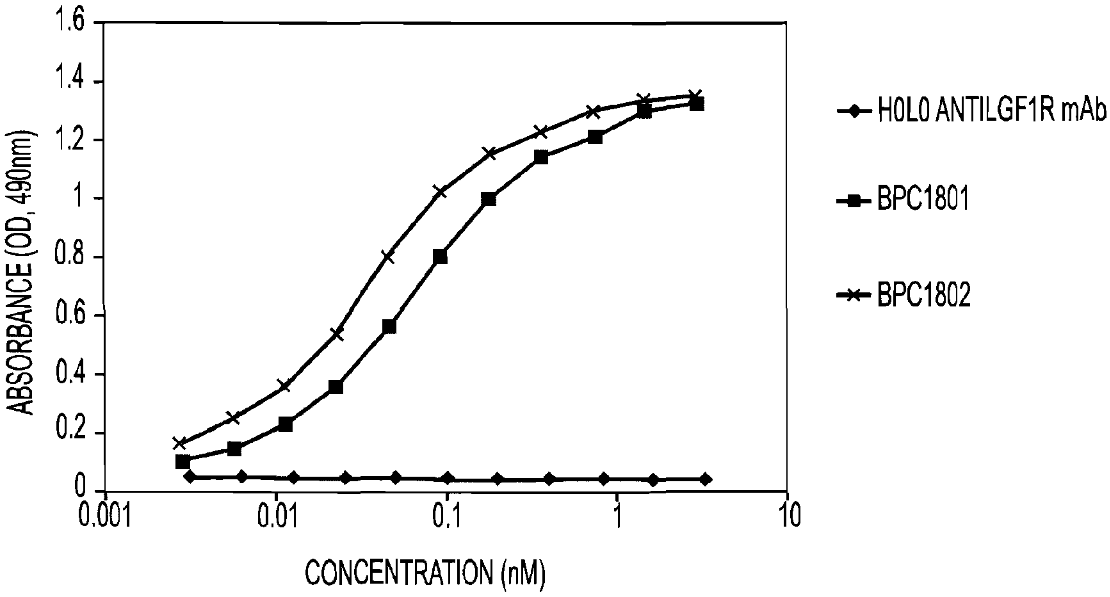
1.6
1.4
1.2
1
0.8
0.6
0.4
0.2
0
ABSORBANCE (OD, 490nm)
0.001
0.01
0.1
1
10
CONCENTRATION (nM)
H0L0 ANTILGF1R mAb
BPC1801
BPC1802

*FIG. 87*

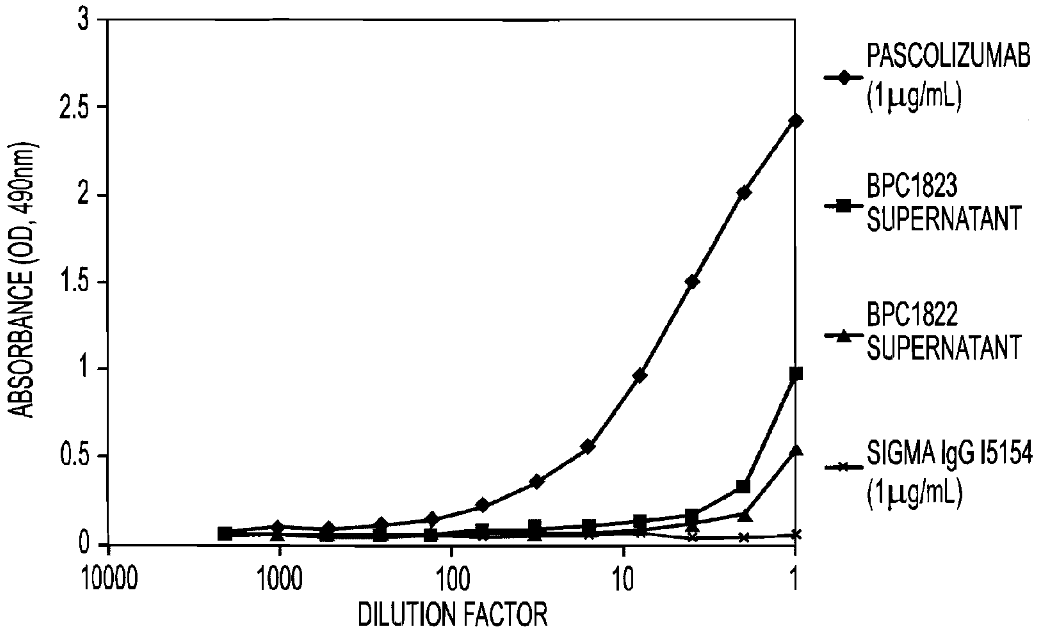


*FIG. 88a*

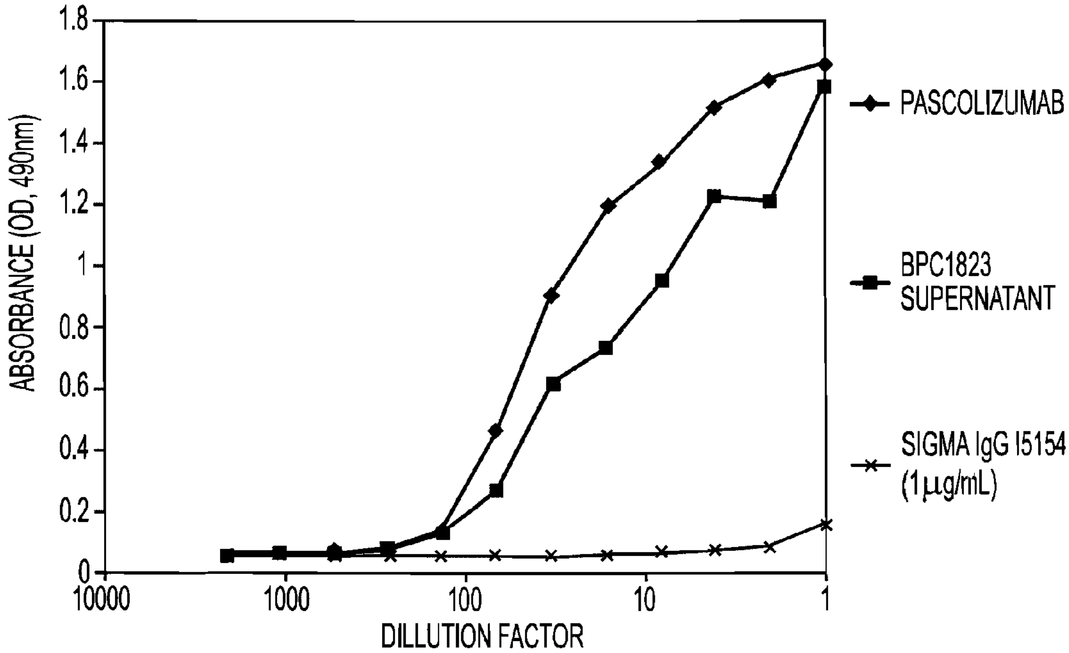


*FIG. 88b*

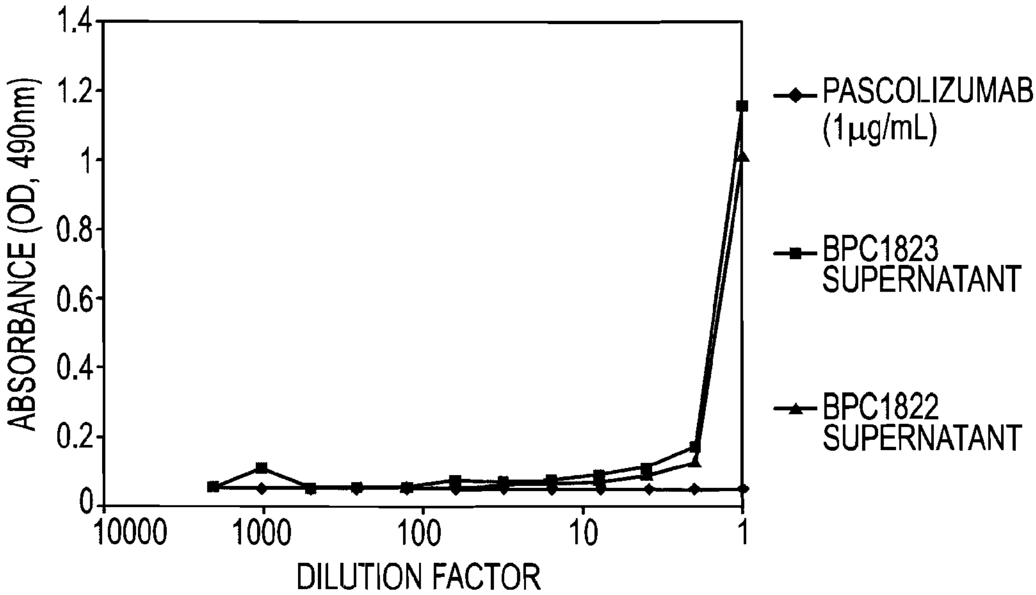


*FIG. 89a*

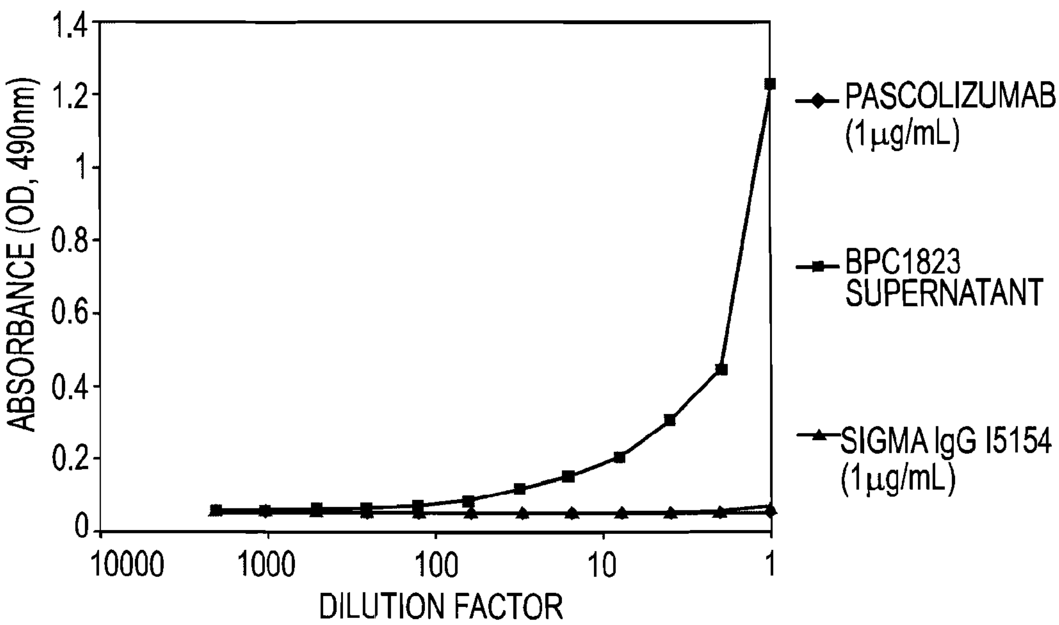


*FIG. 89b*

SEC PROFILE FOR PASCOH-474 GS REMOVED

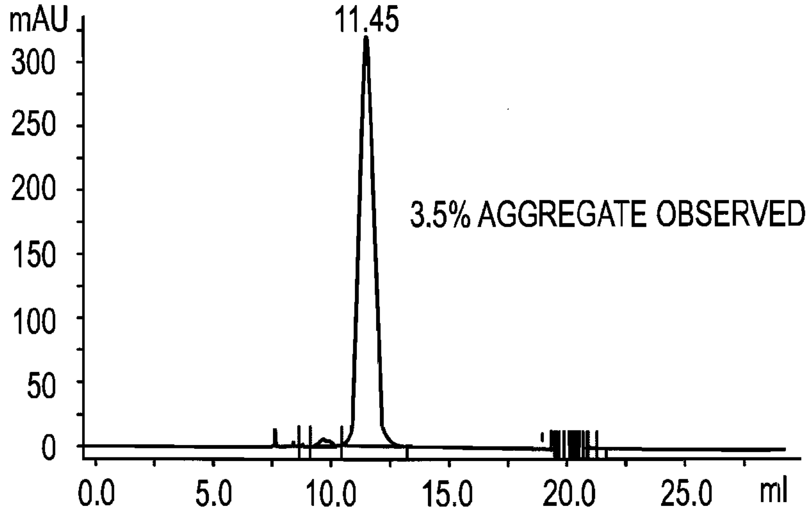


*FIG. 90*

SEC PROFILE FOR PASCOH-TVAAPS-474 GS REMOVED

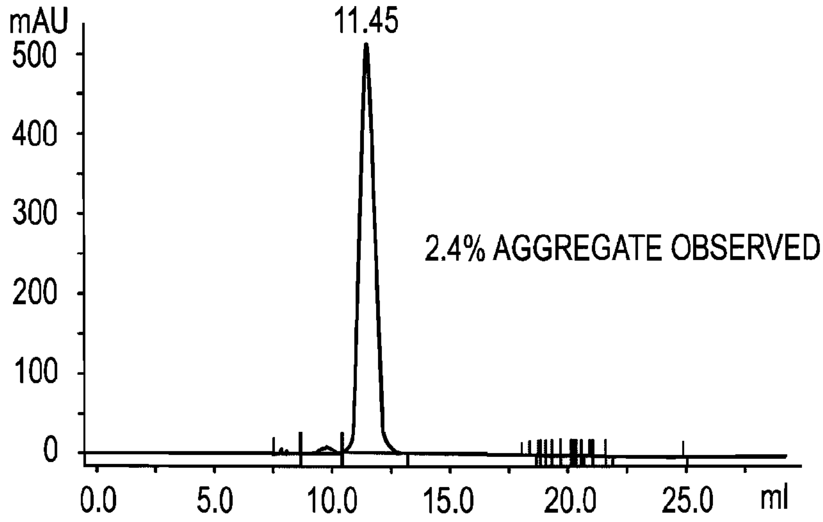


*FIG. 91*

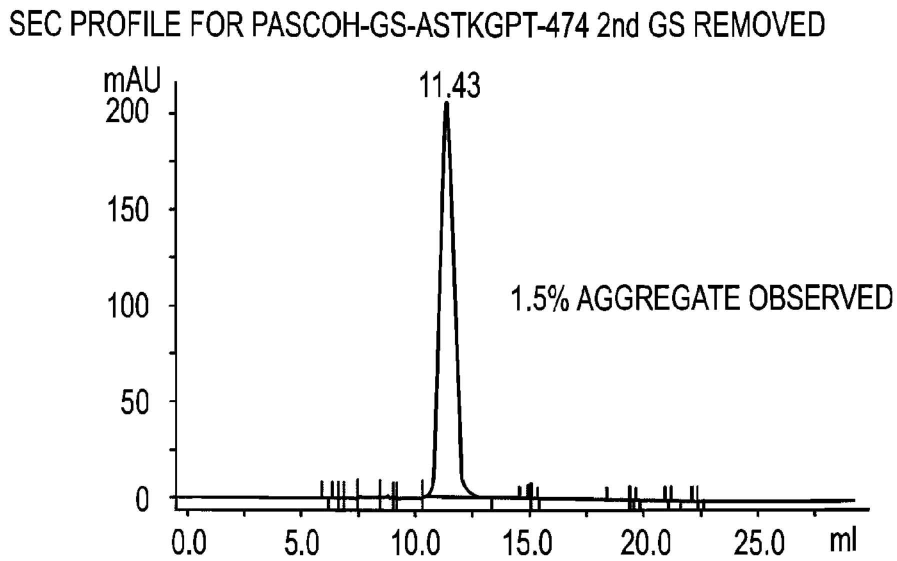
SEC PROFILE FOR PASCOH-GS-ASTKGPT-474 2nd GS REMOVED
mAU
200
150
100
50
0
11.43
1.5% AGGREGATE OBSERVED
0.0
5.0
10.0
15.0
20.0
25.0
ml

*FIG. 92*

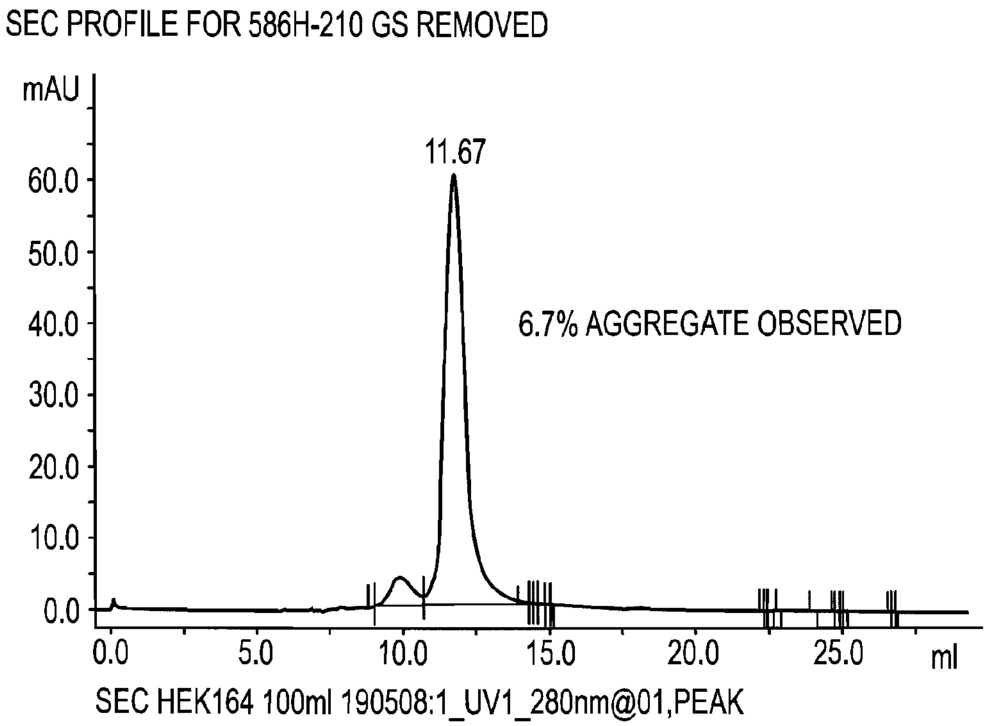
SEC PROFILE FOR 586H-210 GS REMOVED
mAU
60.0
50.0
40.0
30.0
20.0
10.0
0.0
11.67
6.7% AGGREGATE OBSERVED
0.0
5.0
10.0
15.0
20.0
25.0
ml
SEC HEK164 100ml 190508:1_UV1_280nm@01,PEAK

*FIG. 93*

SEC PROFILE FOR 586H-TVAAPS-210 GS REMOVED

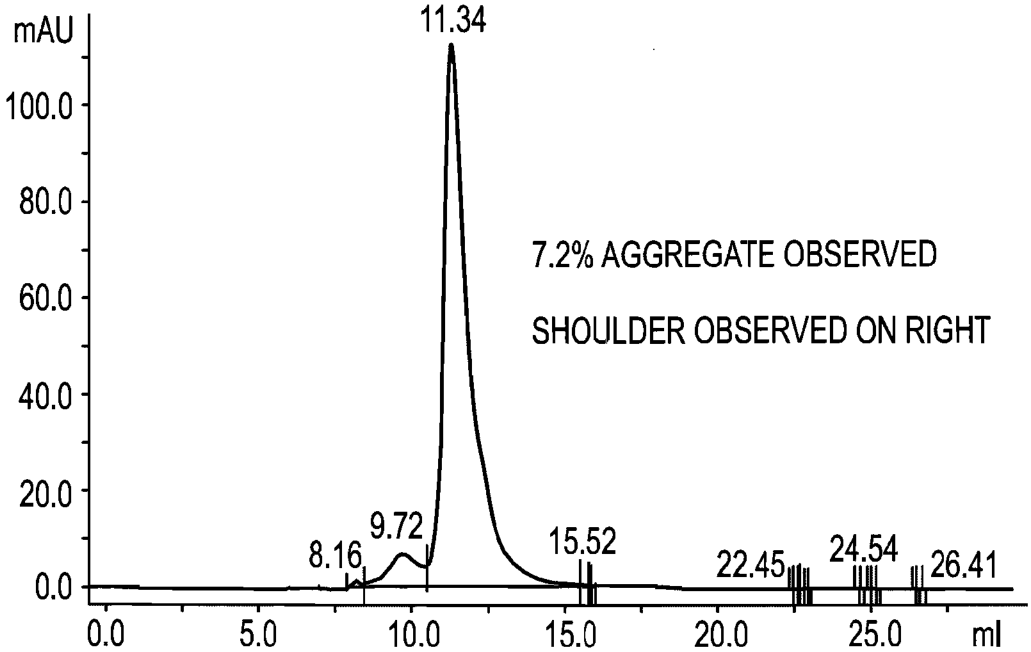


SEC HEK173 100μl 050608:1_UV1_280nm@01,PEAK

*FIG. 94*

SDS PAGE FOR PASCOH-474 GS REMOVED (LANE B) AND
PASCOH-TVAAPS-474 GS REMOVED (LANE A)

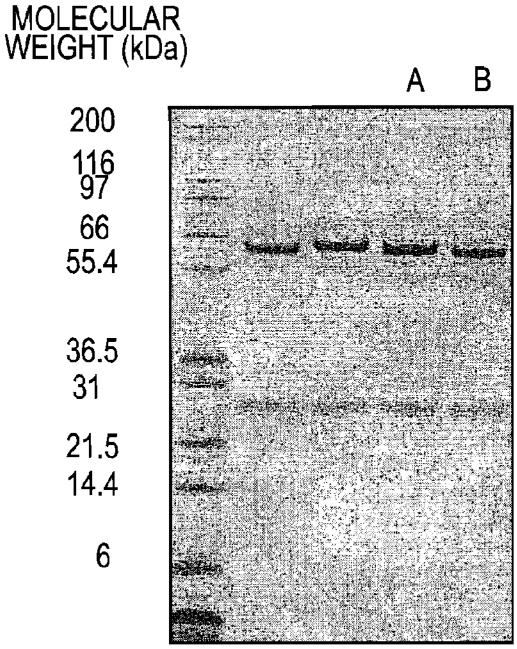


FIG. 95

SDS PAGE FOR PASCOH-GS-ASTKGPT-474 2nd GS REMOVED
[A = NON-REDUCING CONDITIONS, B = REDUCING CONDITIONS]

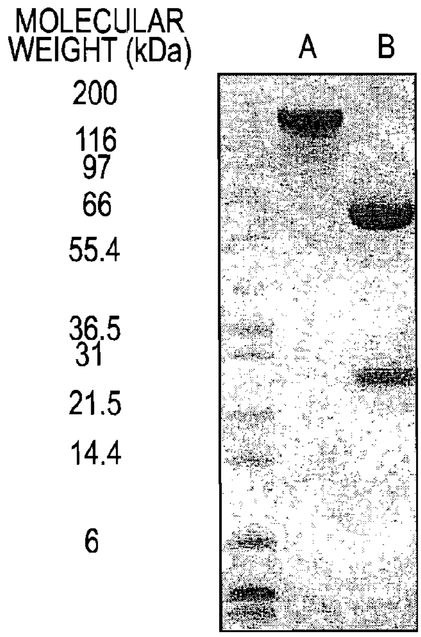


FIG. 96

SDS PAGE FOR 586H-210 GS REMOVED (LANE A)

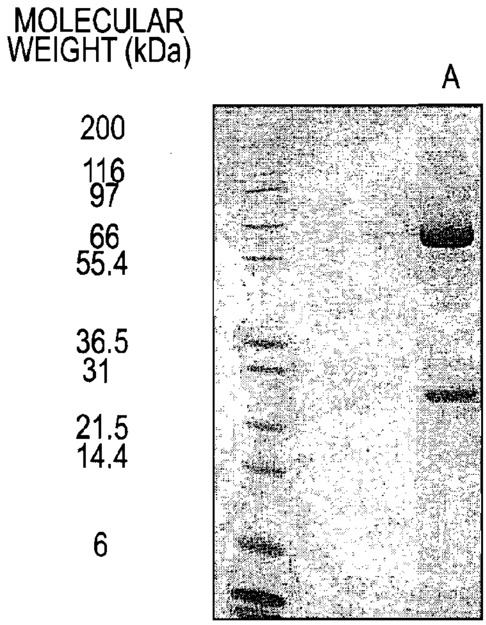


*FIG. 97*

SDS PAGE FOR 586H-TVAAPS-210 GS REMOVED (LANE A)

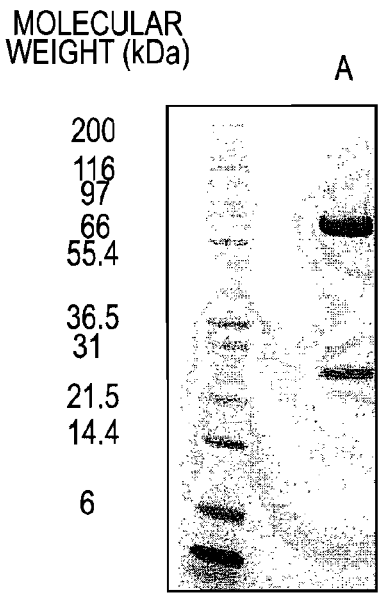


*FIG. 98*

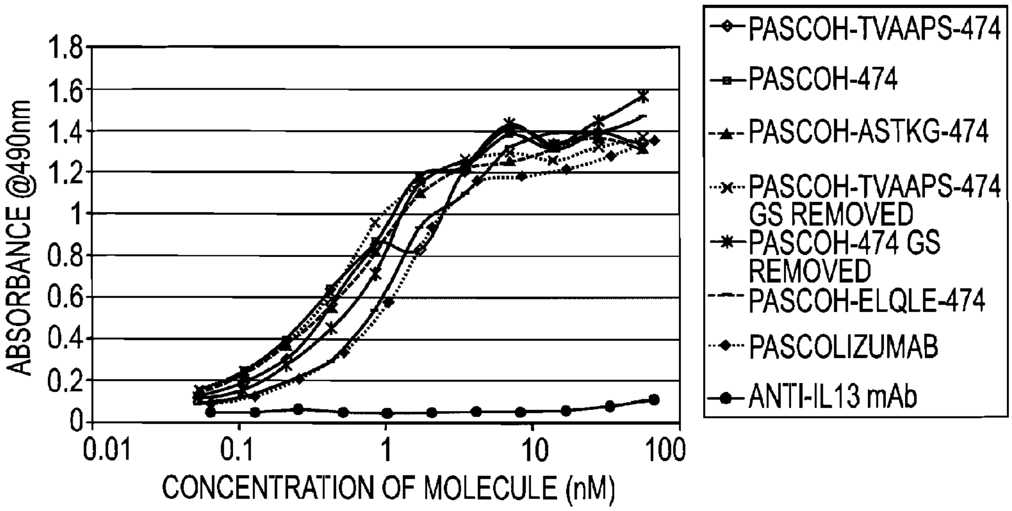


*FIG. 99*

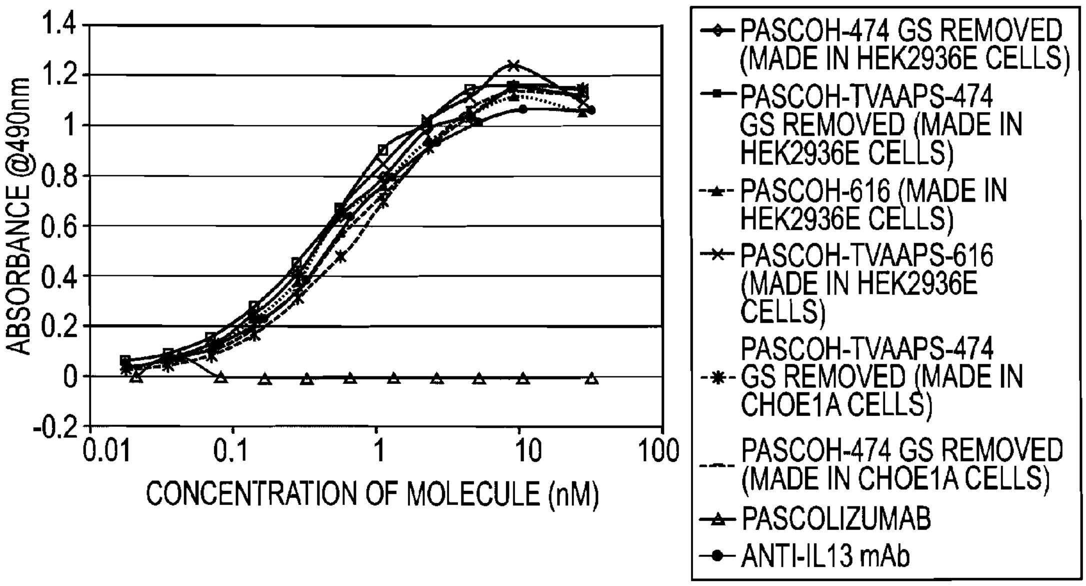


*FIG. 100*

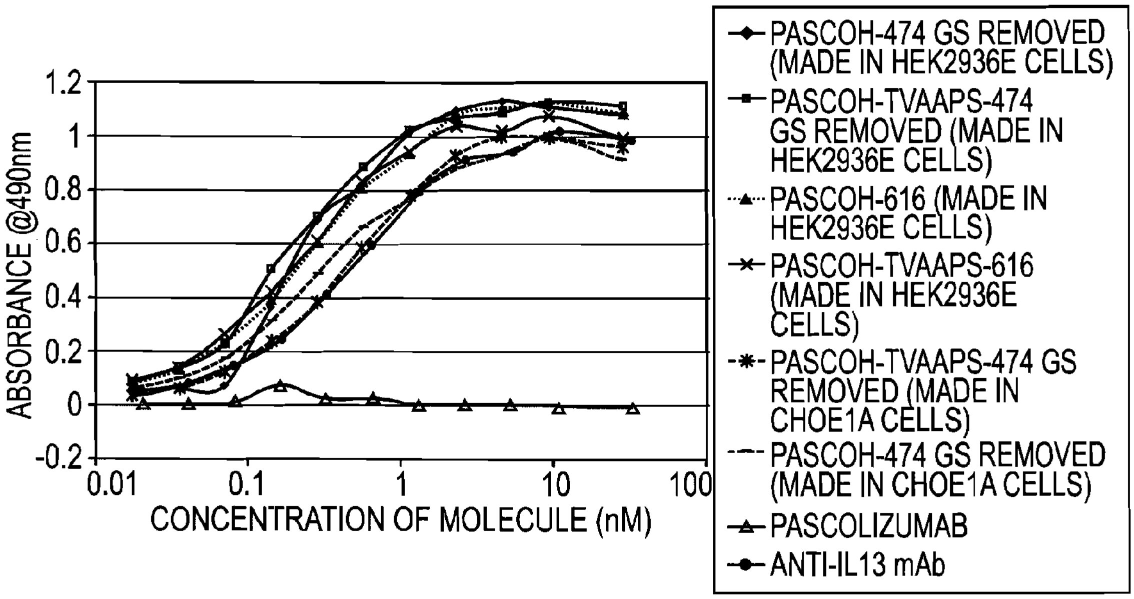


*FIG. 101*

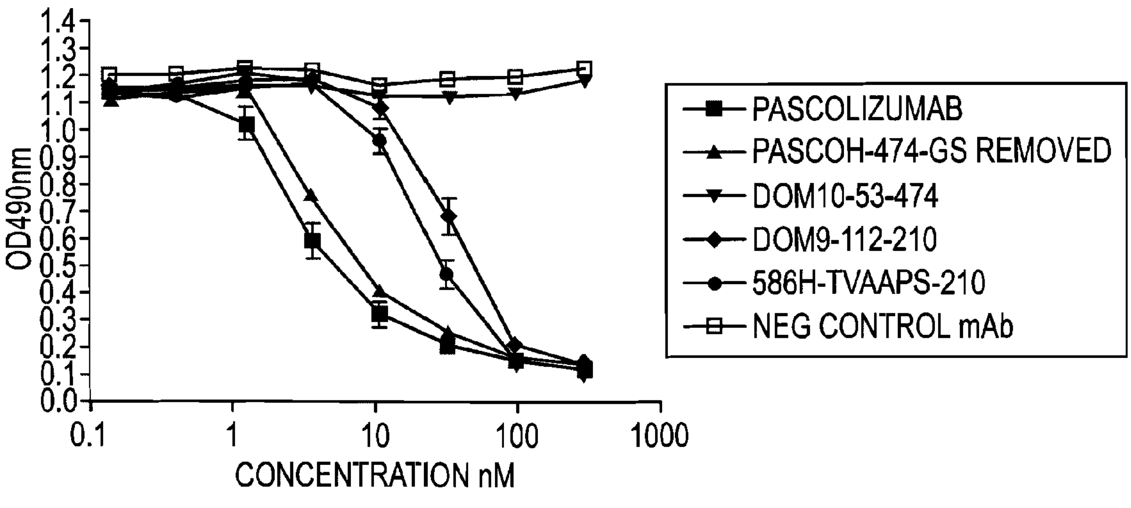


*FIG. 102*

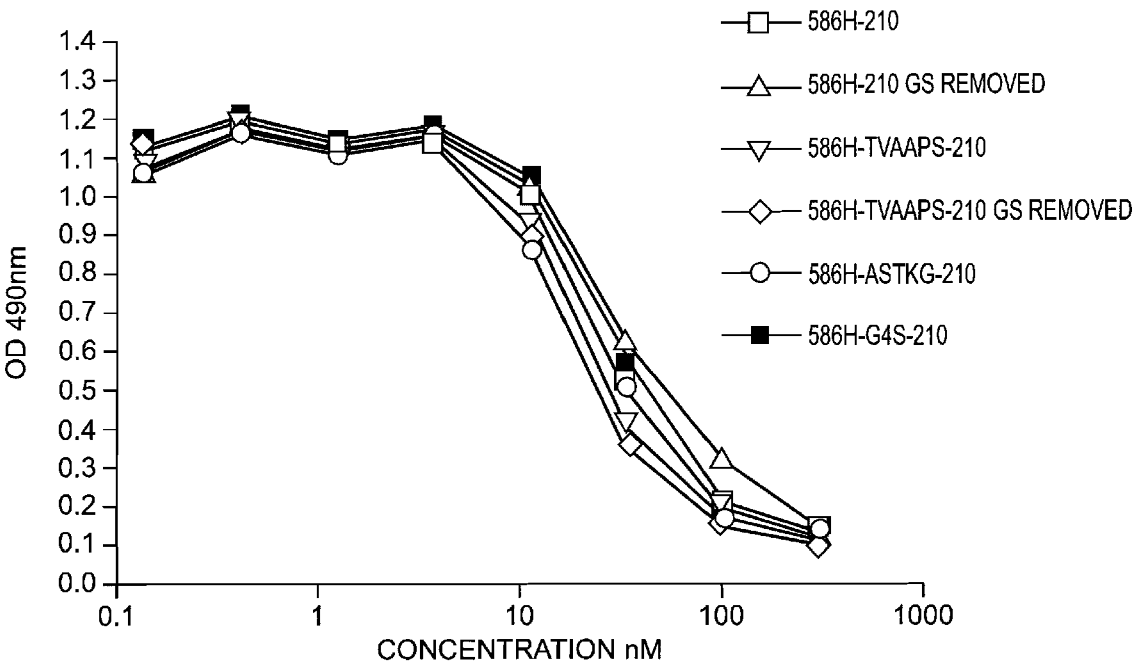

586H-210
586H-210 GS REMOVED
586H-TVAAPS-210
586H-TVAAPS-210 GS REMOVED
586H-ASTKG-210
586H-G4S-210
OD 490nm
1.4
1.3
1.2
1.1
1.0
0.9
0.8
0.7
0.6
0.5
0.4
0.3
0.2
0.1
0.0
0.1
1
10
100
1000
CONCENTRATION nM


*FIG. 103*

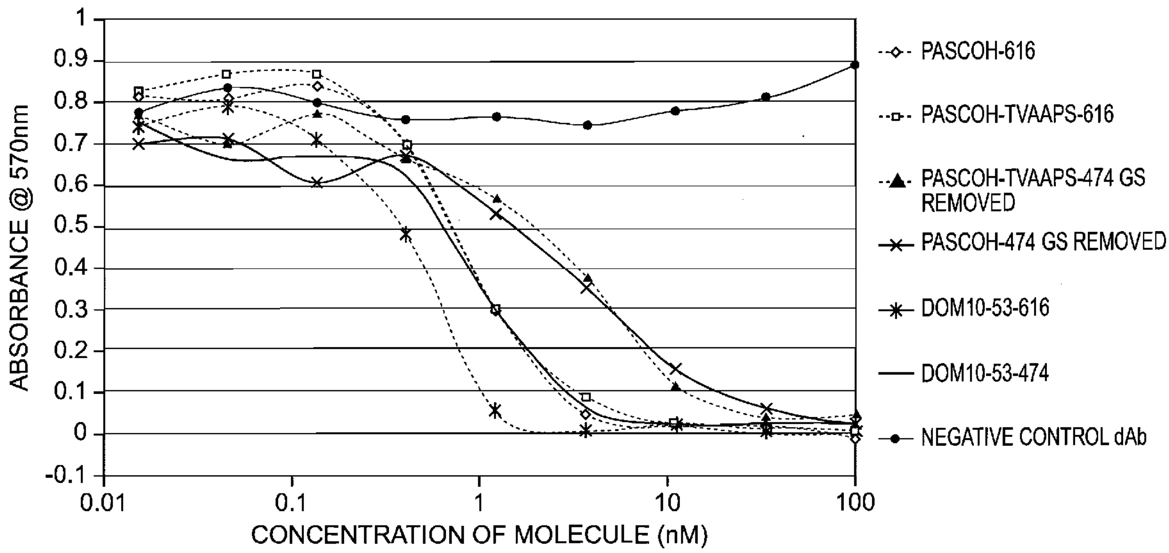
ABSORBANCE @ 570nm
1
0.9
0.8
0.7
0.6
0.5
0.4
0.3
0.2
0.1
0
-0.1
0.01
0.1
1
10
100
CONCENTRATION OF MOLECULE (nM)
PASCOH-616
PASCOH-TVAAPS-616
PASCOH-TVAAPS-474 GS REMOVED
PASCOH-474 GS REMOVED
DOM10-53-616
DOM10-53-474
NEGATIVE CONTROL dAb

*FIG. 104*

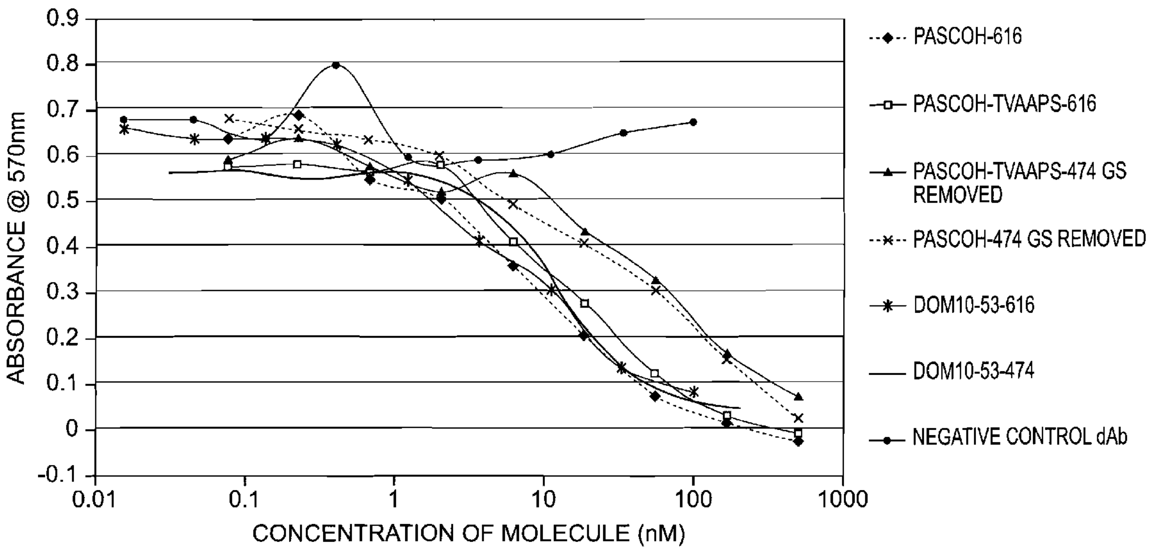
0.9
0.8
0.7
0.6
0.5
0.4
0.3
0.2
0.1
0
-0.1
ABSORBANCE @ 570nm
0.01
0.1
1
10
100
1000
CONCENTRATION OF MOLECULE (nM)
PASCOH-616
PASCOH-TVAAPS-616
PASCOH-TVAAPS-474 GS REMOVED
PASCOH-474 GS REMOVED
DOM10-53-616
DOM10-53-474
NEGATIVE CONTROL dAb

*FIG. 105*

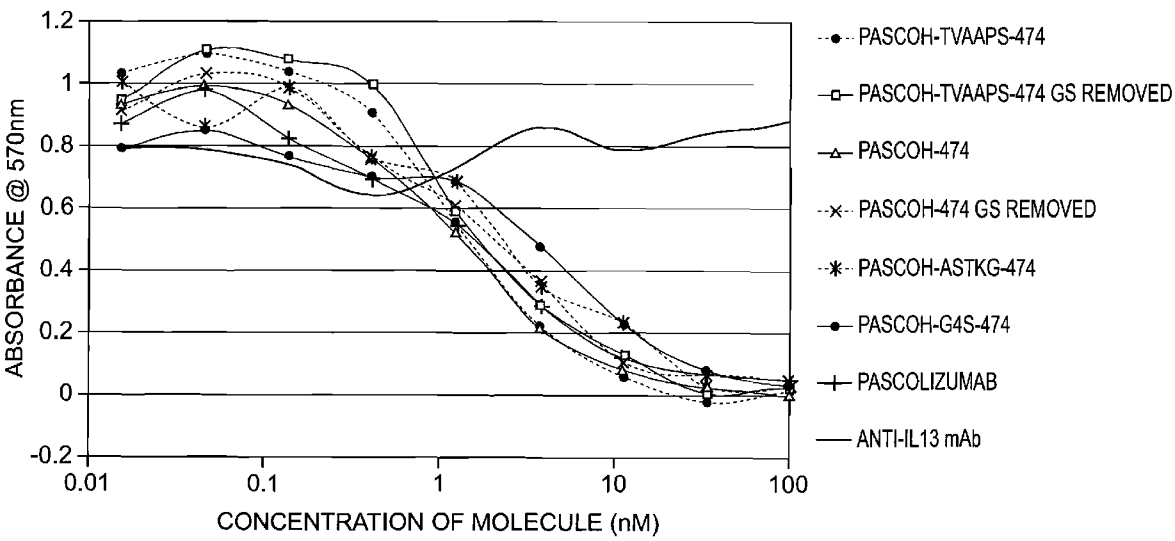
1.2
1
0.8
0.6
0.4
0.2
0
-0.2
ABSORBANCE @ 570nm
0.01
0.1
1
10
100
CONCENTRATION OF MOLECULE (nM)
PASCOH-TVAAPS-474
PASCOH-TVAAPS-474 GS REMOVED
PASCOH-474
PASCOH-474 GS REMOVED
PASCOH-ASTKG-474
PASCOH-G4S-474
PASCOLIZUMAB
ANTI-IL13 mAb

*FIG. 106*

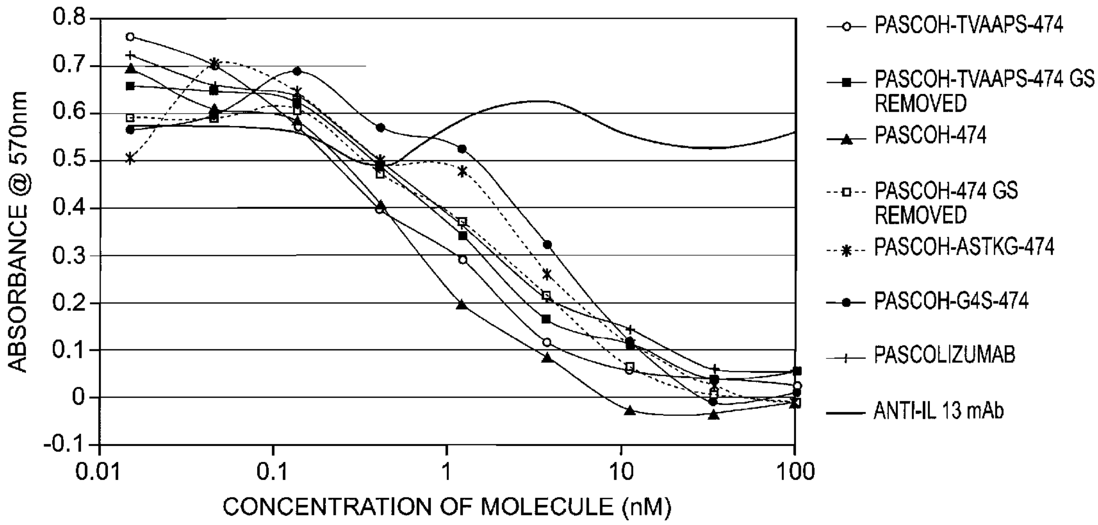
0.8
0.7
0.6
0.5
0.4
0.3
0.2
0.1
0
-0.1
ABSORBANCE @ 570nm
0.01
0.1
1
10
100
CONCENTRATION OF MOLECULE (nM)
PASCOH-TVAAPS-474
PASCOH-TVAAPS-474 GS REMOVED
PASCOH-474
PASCOH-474 GS REMOVED
PASCOH-ASTKG-474
PASCOH-G4S-474
PASCOLIZUMAB
ANTI-IL 13 mAb

FIG. 107

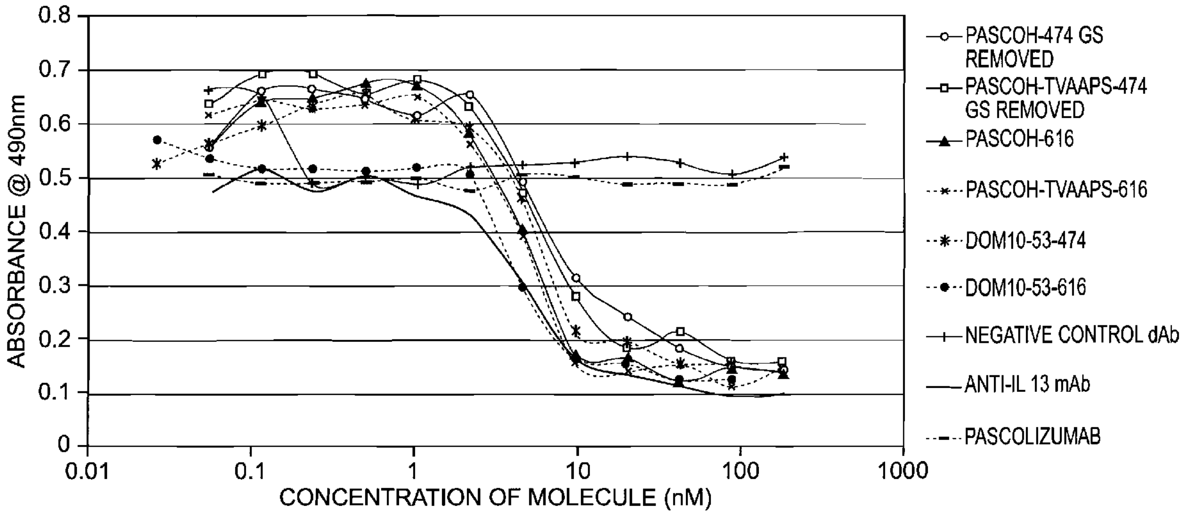
0.8
0.7
0.6
0.5
0.4
0.3
0.2
0.1
0
ABSORBANCE @ 490nm
0.01
0.1
1
10
100
1000
CONCENTRATION OF MOLECULE (nM)
PASCOH-474 GS REMOVED
PASCOH-TVAAPS-474 GS REMOVED
PASCOH-616
PASCOH-TVAAPS-616
DOM10-53-474
DOM10-53-616
NEGATIVE CONTROL dAb
ANTI-IL 13 mAb
PASCOLIZUMAB

*FIG. 108*

SEC PROFILE FOR PASCOH-616

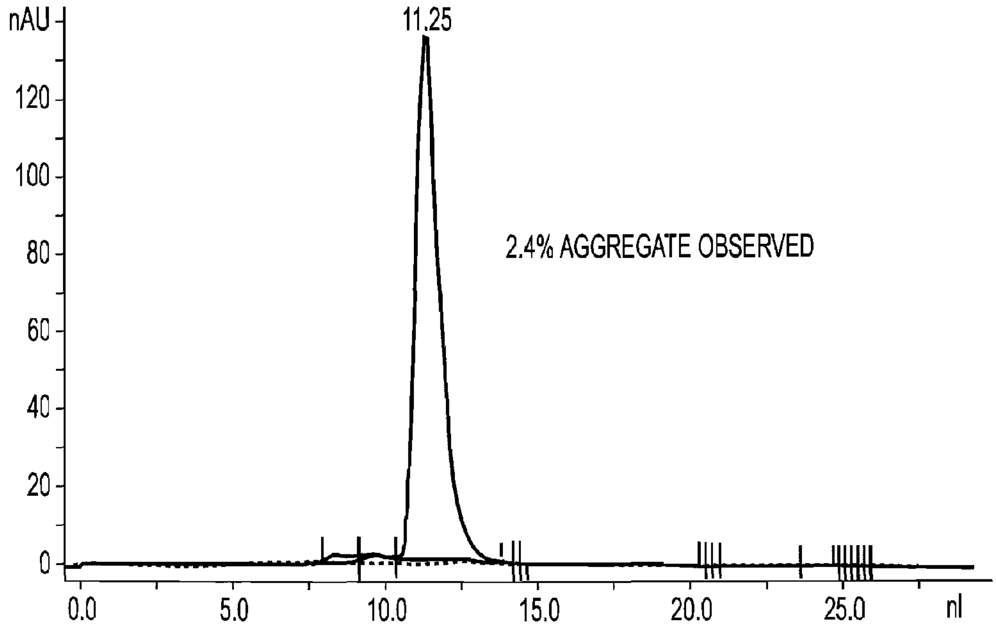


*FIG. 109*

SEC PROFILE FOR PASCOH-TVAAPS_616

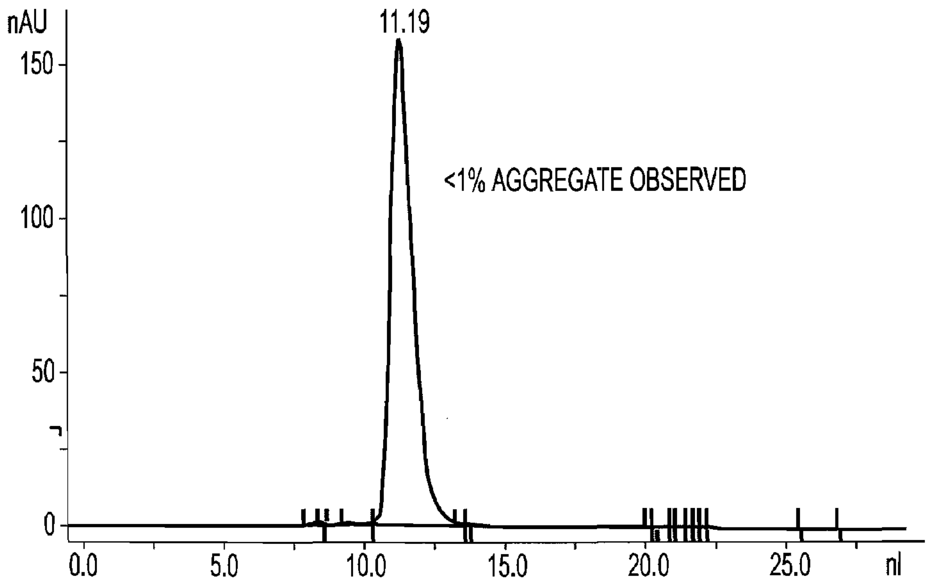


*FIG. 110*

SDS PAGE FOR PASCOH-616
[E1 = NON-REDUCING CONDITIONS, E2 = REDUCING CONDITIONS]

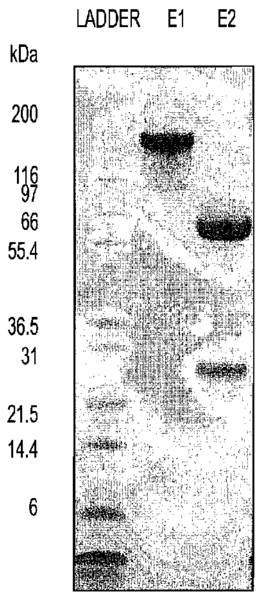


FIG. 111

SDS PAGE FOR PASCOH-TVAAPS-616
[A = NON-REDUCING CONDITIONS, B = REDUCING CONDITIONS]

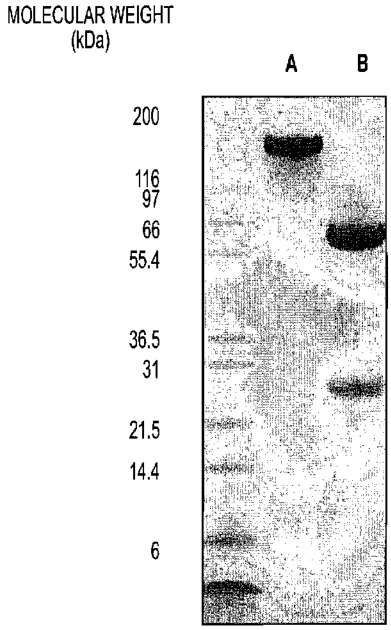


FIG. 112

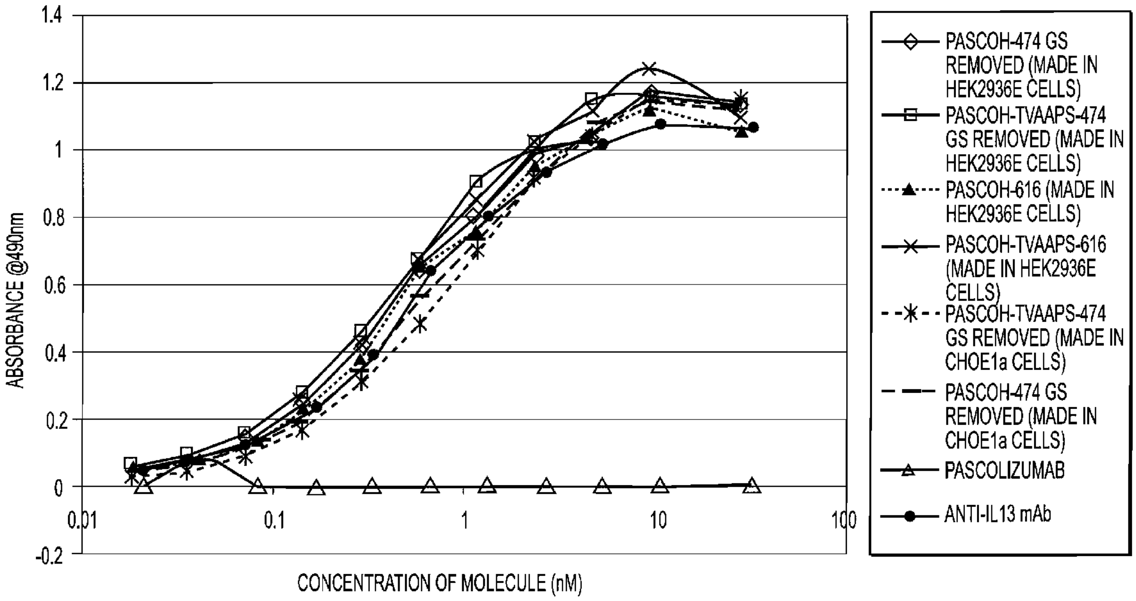
1.4
1.2
1
0.8
0.6
0.4
0.2
0
-0.2
ABSORBANCE @490nm
0.01
0.1
1
10
100
CONCENTRATION OF MOLECULE (nM)
PASCOH-474 GS REMOVED (MADE IN HEK2936E CELLS)
PASCOH-TVAAPS-474 GS REMOVED (MADE IN HEK2936E CELLS)
PASCOH-616 (MADE IN HEK2936E CELLS)
PASCOH-TVAAPS-616 (MADE IN HEK2936E CELLS)
PASCOH-TVAAPS-474 GS REMOVED (MADE IN CHOE1a CELLS)
PASCOH-474 GS REMOVED (MADE IN CHOE1a CELLS)
PASCOLIZUMAB
ANTI-IL13 mAb

*FIG. 113*

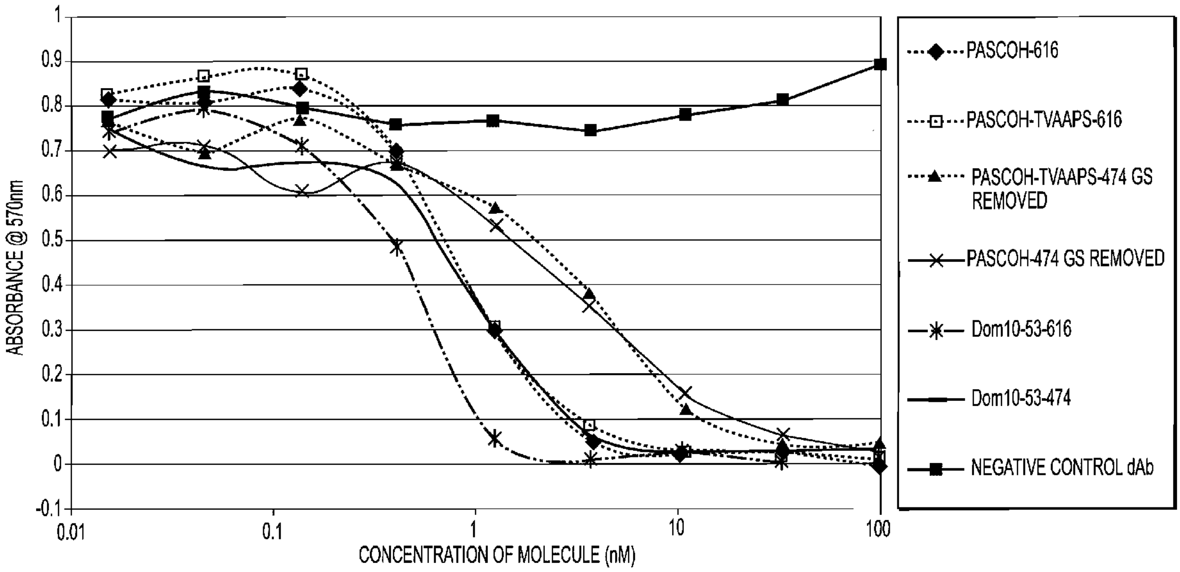
1
0.9
0.8
0.7
0.6
0.5
0.4
0.3
0.2
0.1
0
-0.1
ABSORBANCE @ 570nm
0.01
0.1
1
10
100
CONCENTRATION OF MOLECULE (nM)
PASCOH-616
PASCOH-TVAAPS-616
PASCOH-TVAAPS-474 GS REMOVED
PASCOH-474 GS REMOVED
Dom10-53-616
Dom10-53-474
NEGATIVE CONTROL dAb

*FIG. 114*

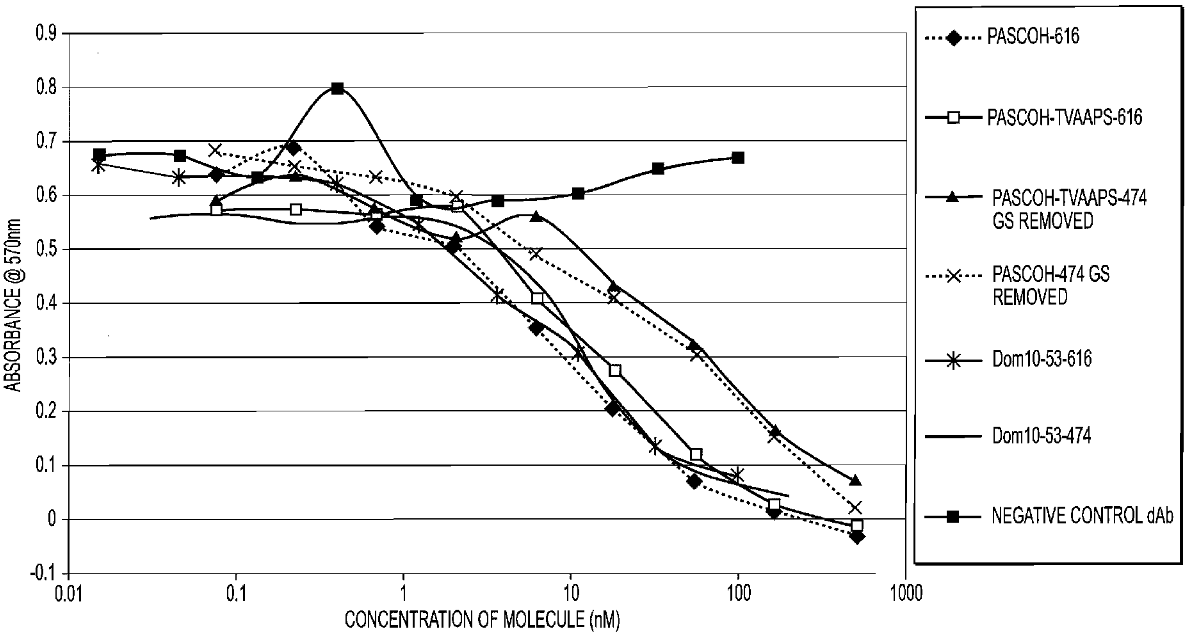
0.9
0.8
0.7
0.6
0.5
0.4
0.3
0.2
0.1
0
-0.1
ABSORBANCE @ 570nm
0.01
0.1
1
10
100
1000
CONCENTRATION OF MOLECULE (nM)
PASCOH-616
PASCOH-TVAAPS-616
PASCOH-TVAAPS-474 GS REMOVED
PASCOH-474 GS REMOVED
Dom10-53-616
Dom10-53-474
NEGATIVE CONTROL dAb

*FIG. 114a*

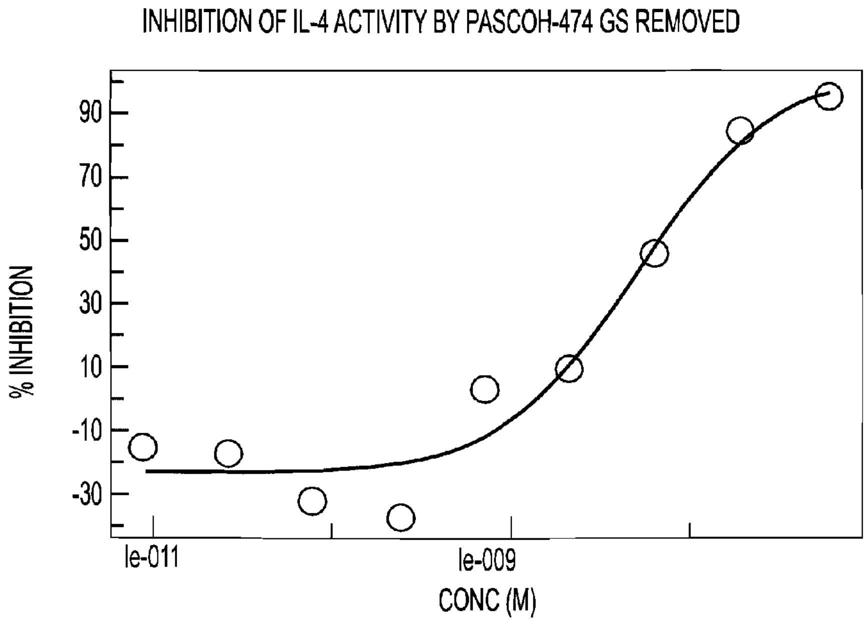


FIG. 115

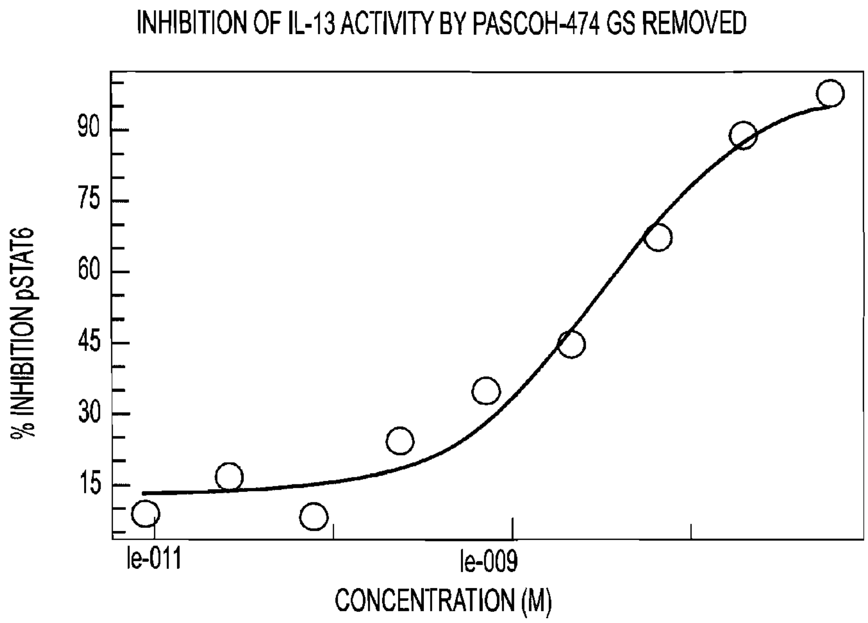


FIG. 116

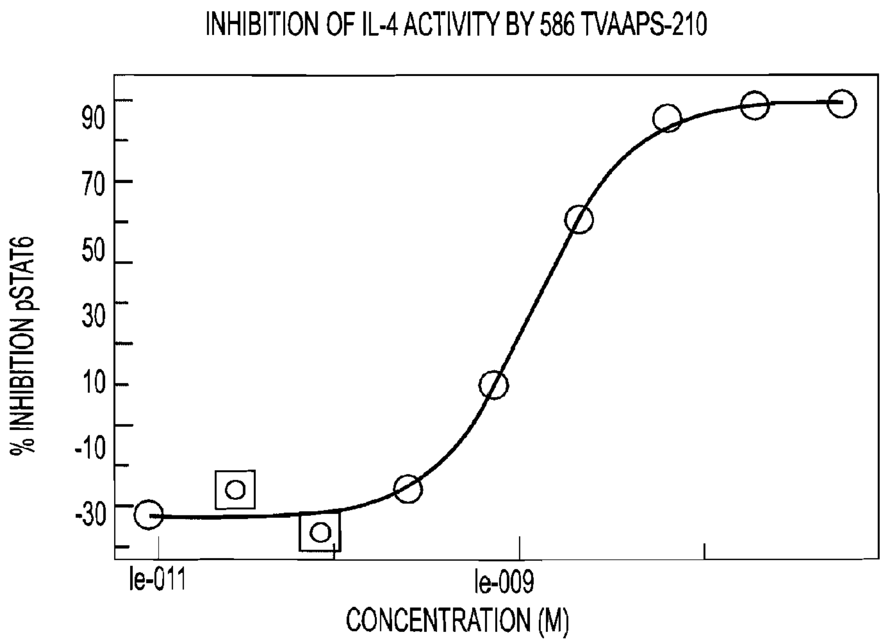


*FIG. 117*

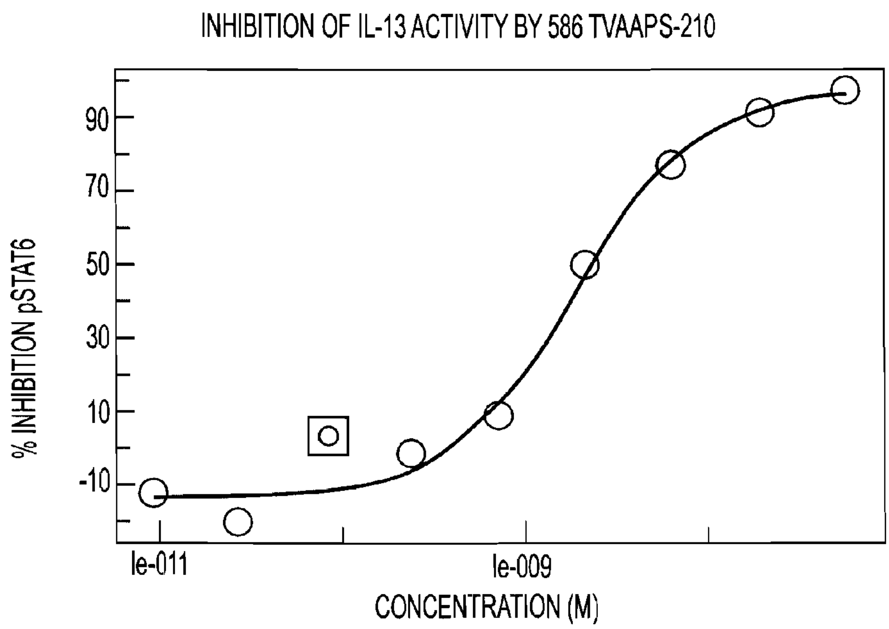


*FIG. 118*

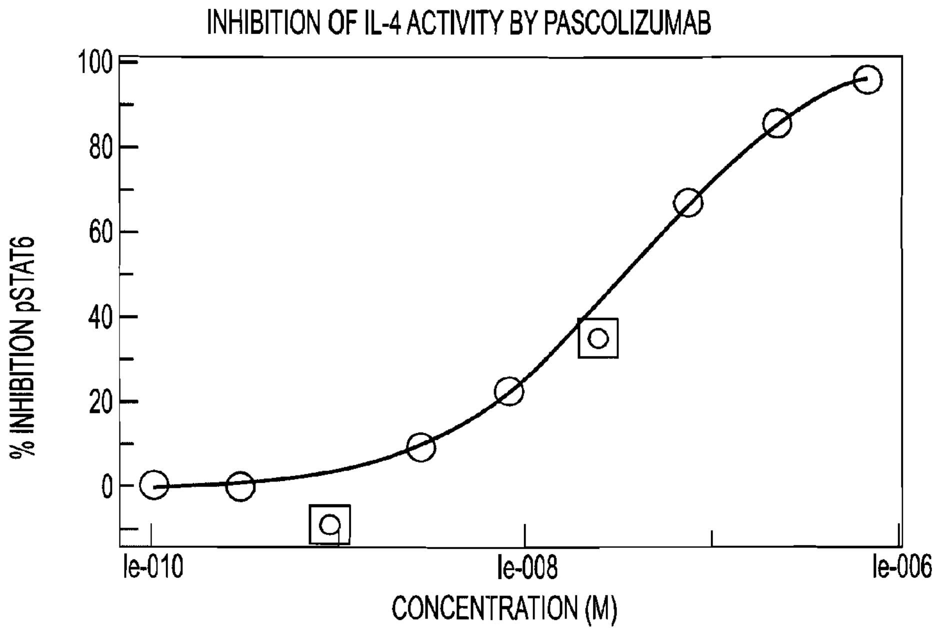


*FIG. 119*

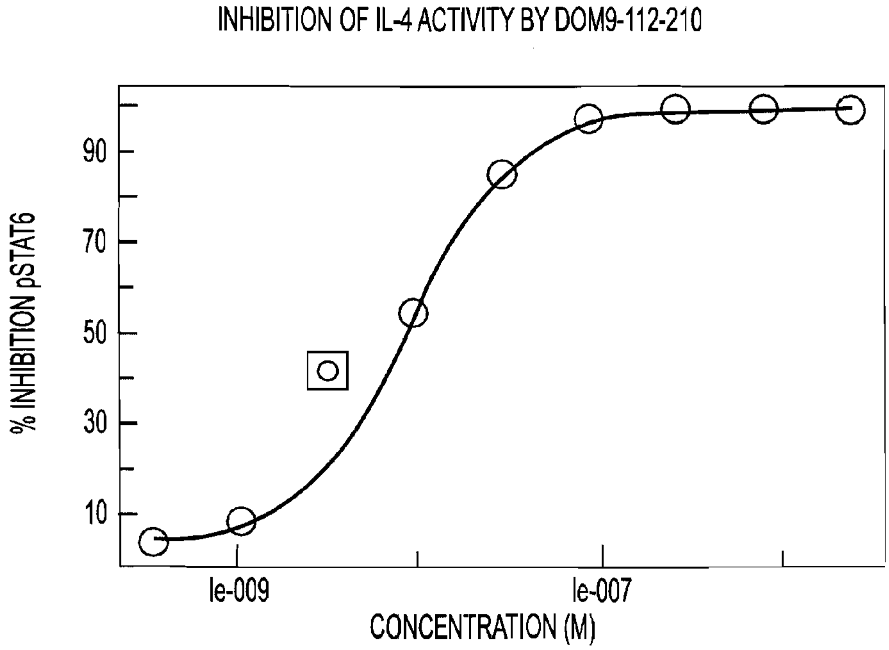


*FIG. 120*

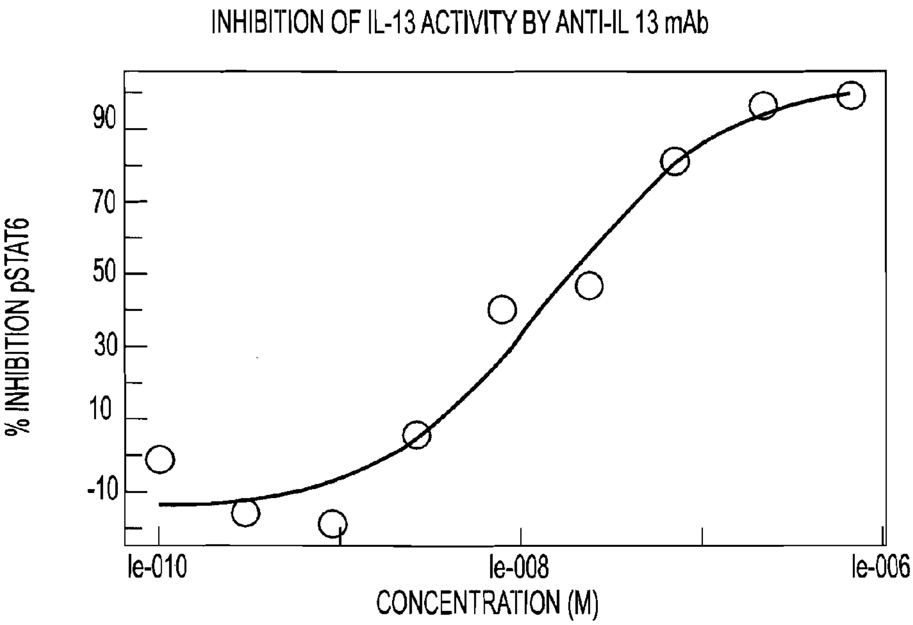


*FIG. 121*

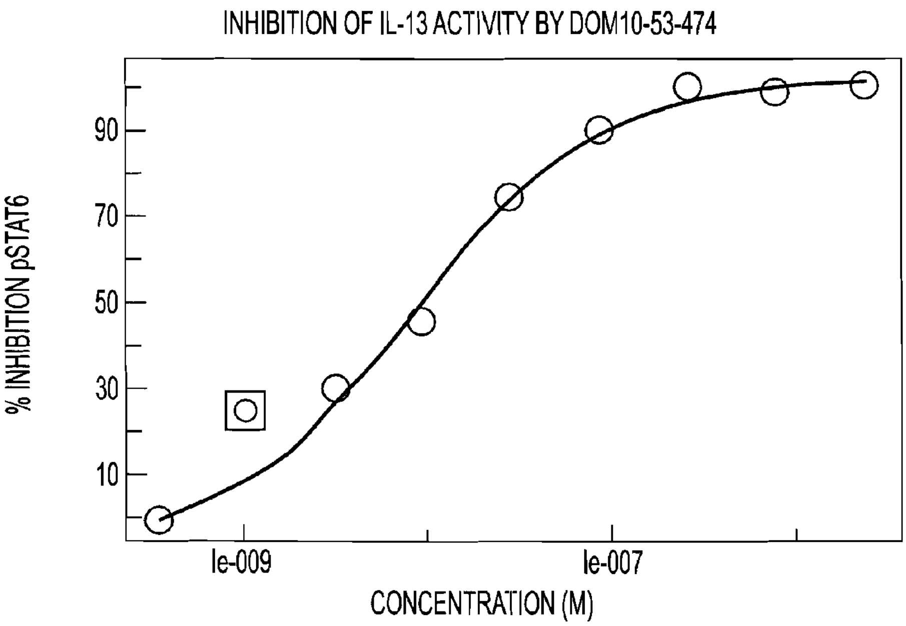


*FIG. 122*

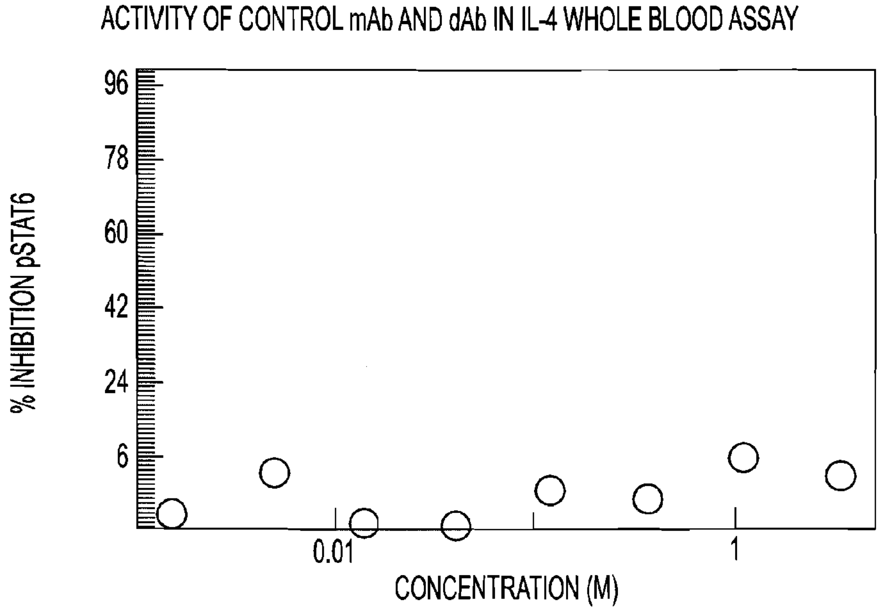


FIG. 123

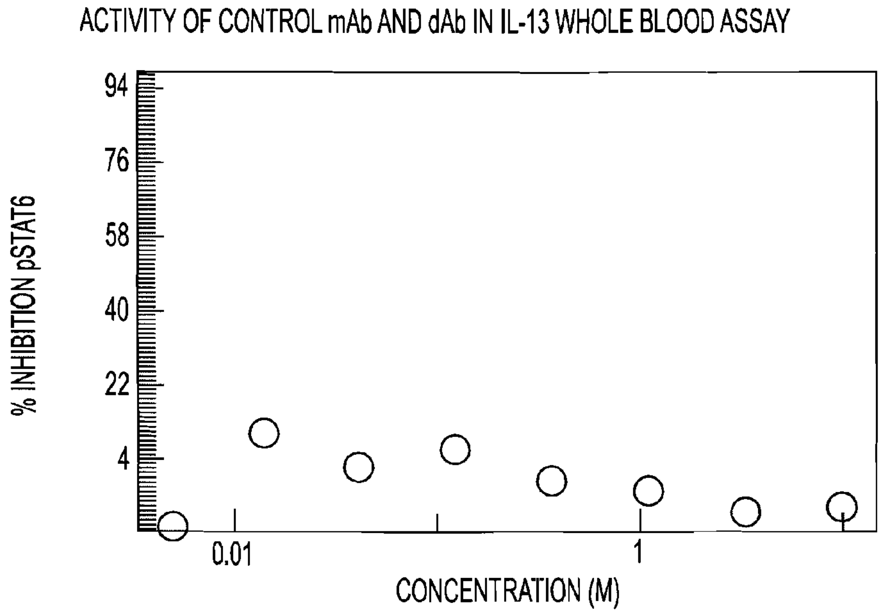


FIG. 124

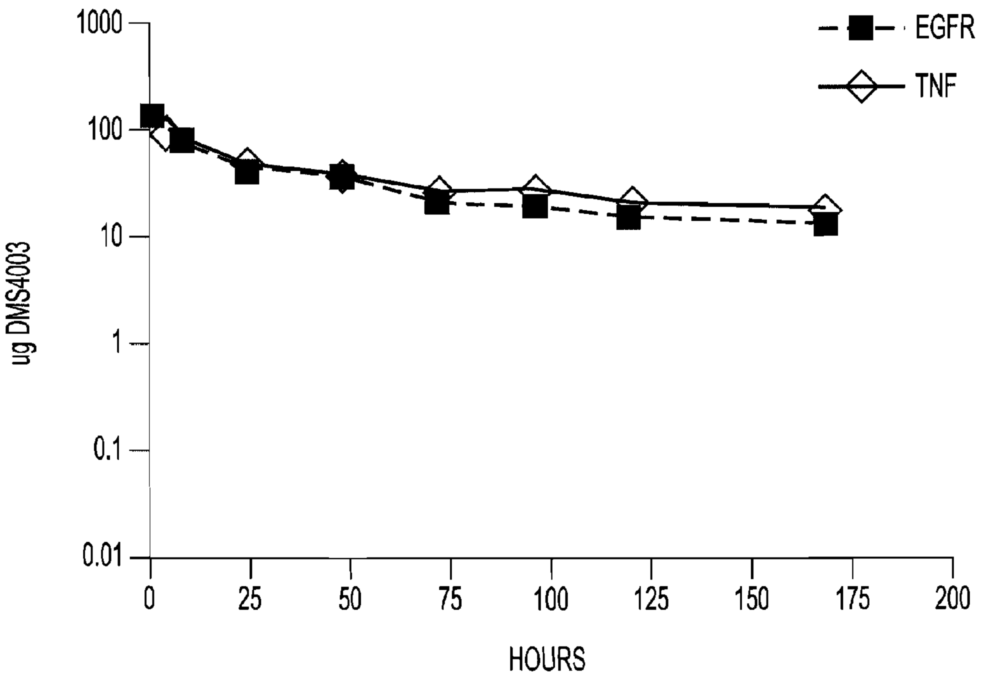


*FIG. 125*

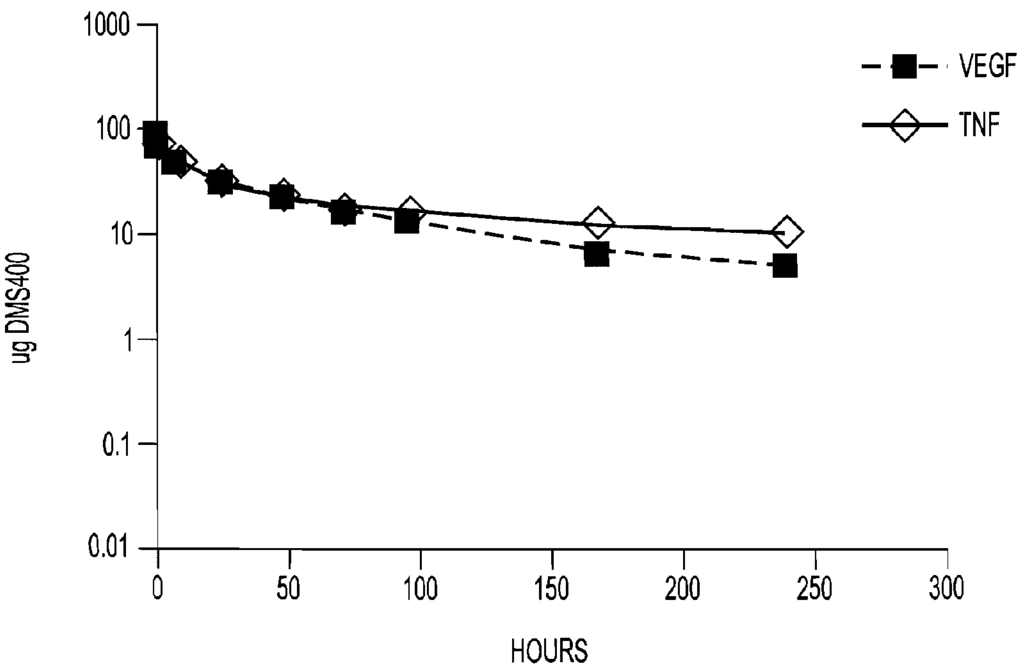


*FIG. 126*

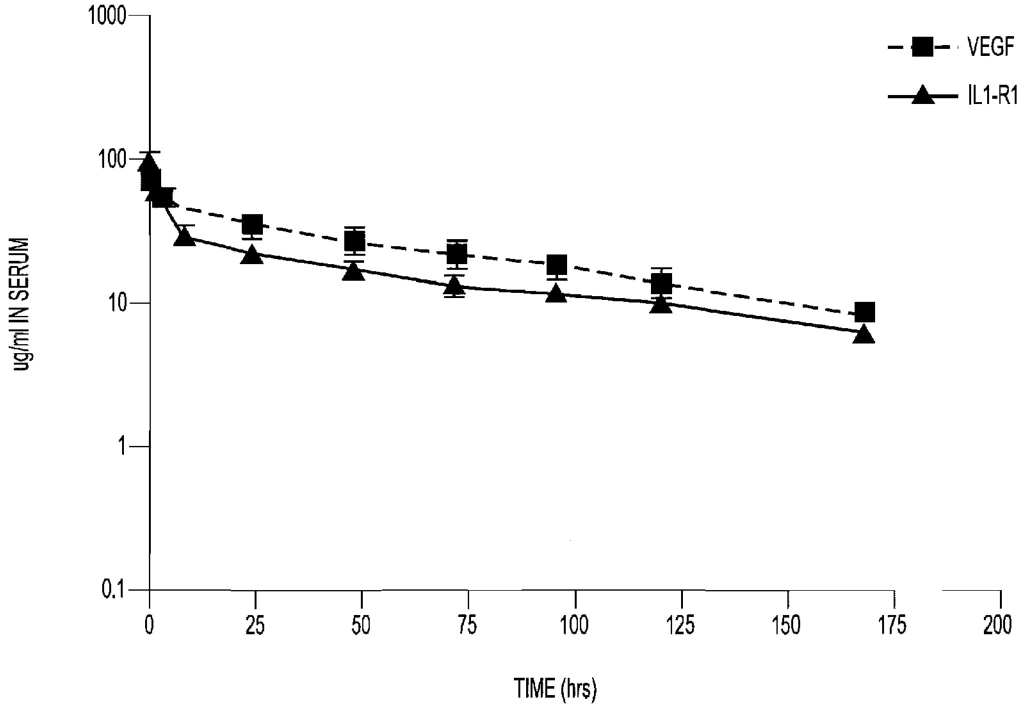
1000
100
10
1
0.1
ug/ml IN SERUM
0
25
50
75
100
125
150
175
200
TIME (hrs)
VEGF
IL1-R1

*FIG. 127*

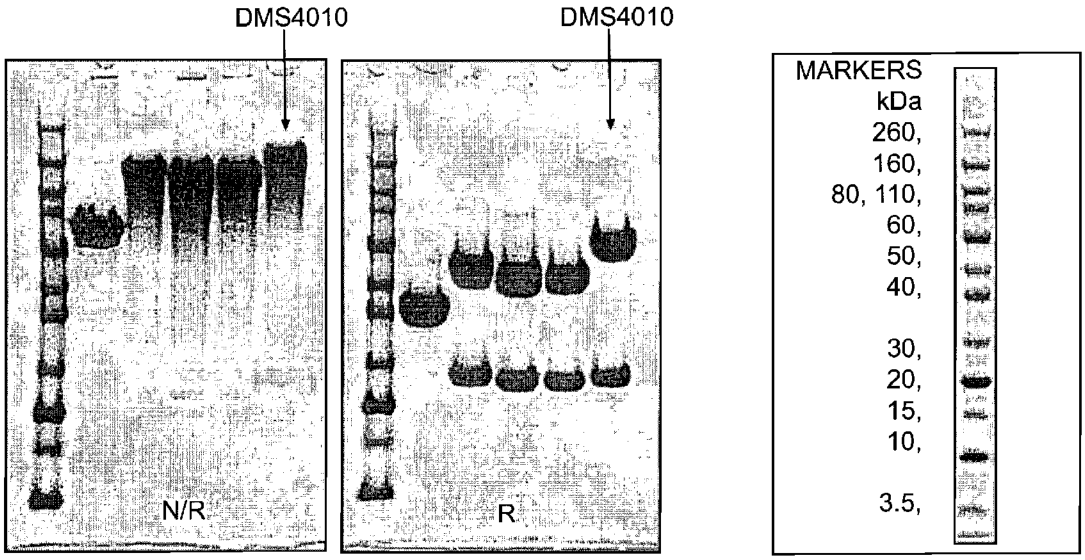


N/R=NON-REDUCED; R=REDUCED

*FIG. 128*

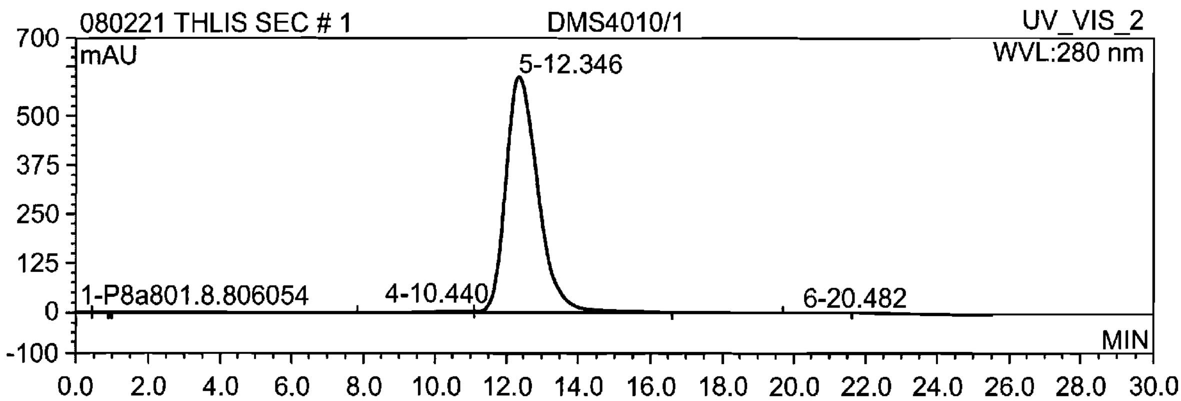
080221 THLIS SEC # 1
DMS4010/1
UV_VIS_2
WVL:280 nm
mAU
700
500
375
250
125
0
-100
5-12.346
1-P8a801.8.806054
4-10.440
6-20.482
MIN
0.0 2.0 4.0 6.0 8.0 10.0 12.0 14.0 16.0 18.0 20.0 22.0 24.0 26.0 28.0 30.0

*FIG. 129*

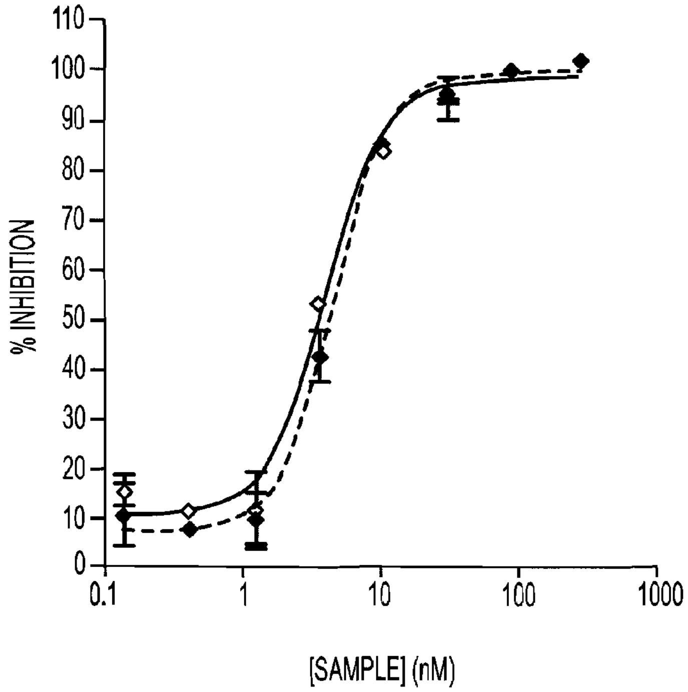


| | EC50 |
|---|---|
| ERBITUX | 4.214 |
| DMS4010 | 4.784 |

*FIG. 130*

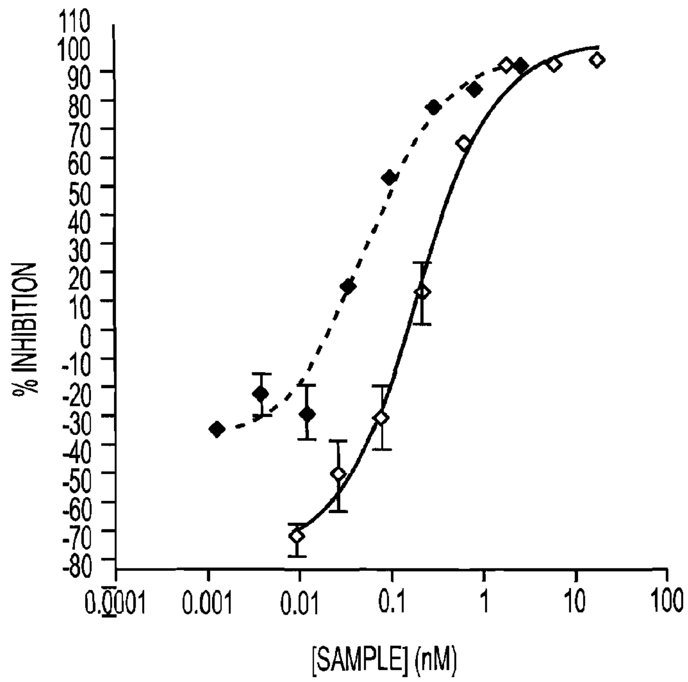


| | EC50 |
|---|---|
| AVASTIN | 0.2141 |
| DMS4010 | 0.05828 |

*FIG. 131*

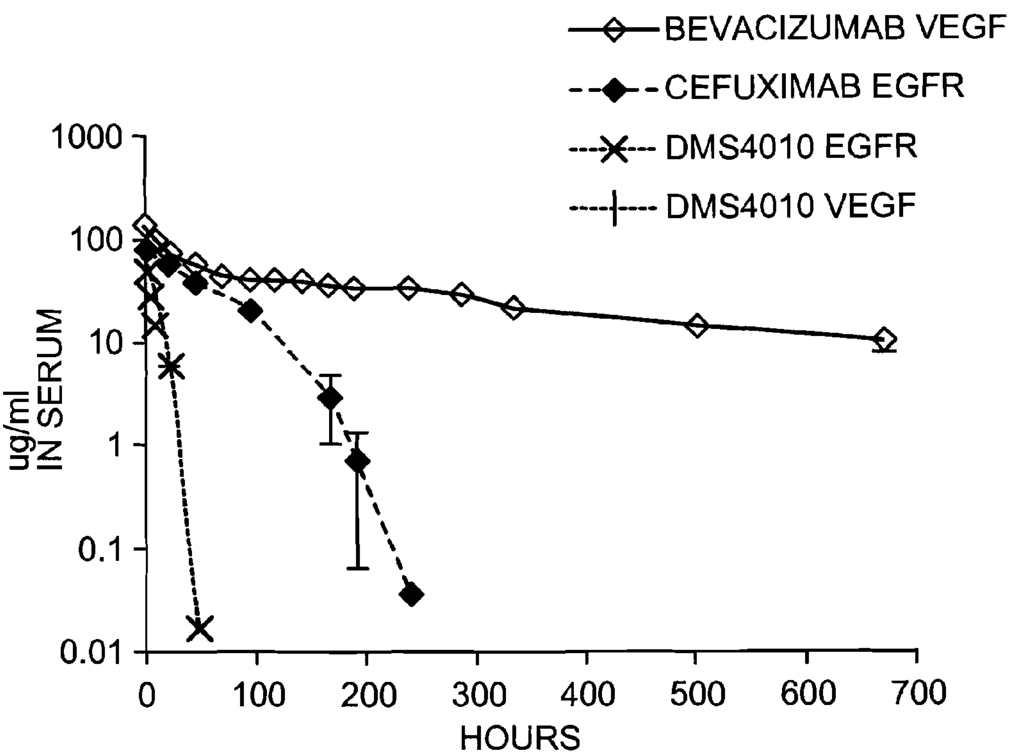


FIG. 132

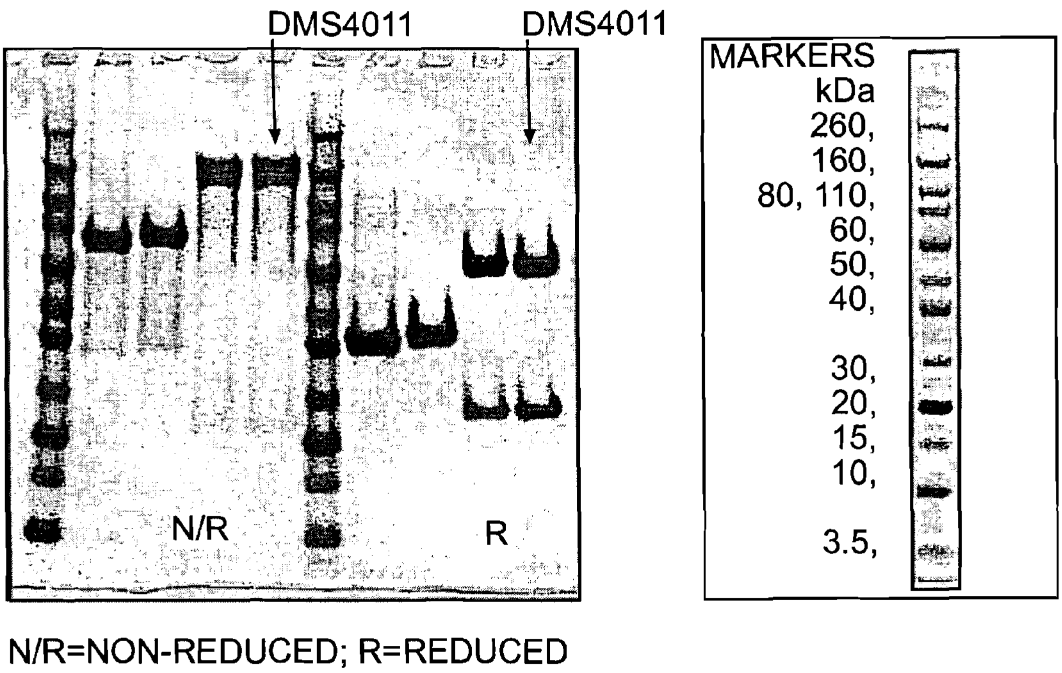


FIG. 133

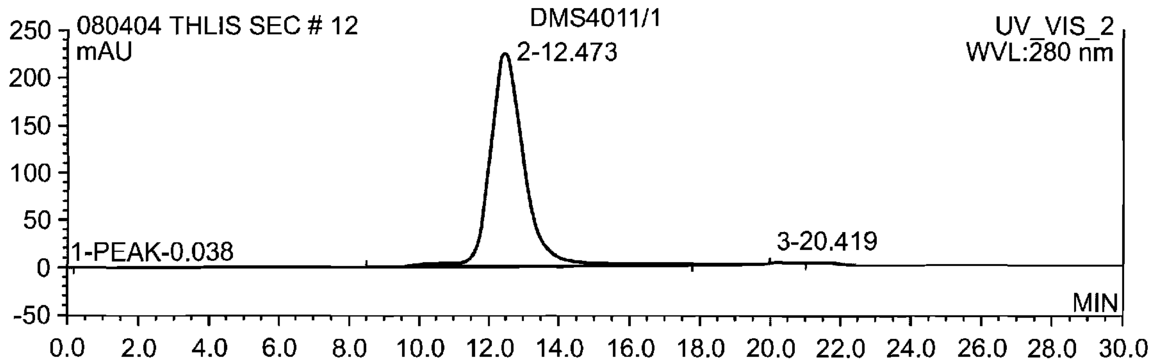
080404 THLIS SEC # 12
mAU
DMS4011/1
UV_VIS_2
WVL:280 nm
2-12.473
1-PEAK-0.038
3-20.419
MIN
250
200
150
100
50
0
-50
0.0 2.0 4.0 6.0 8.0 10.0 12.0 14.0 16.0 18.0 20.0 22.0 24.0 26.0 28.0 30.0

*FIG. 134*

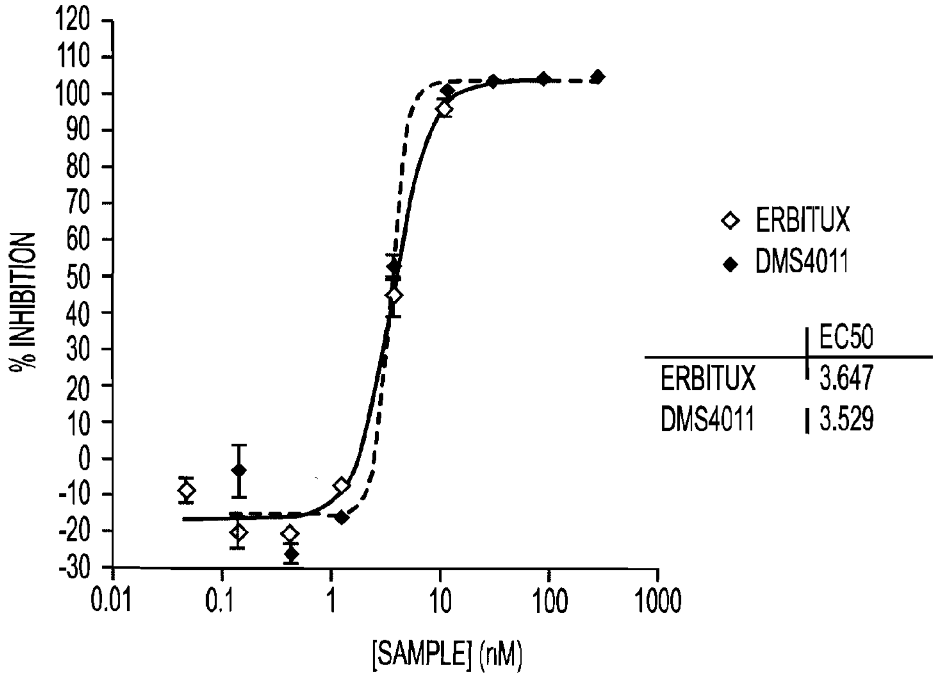


*FIG. 135*

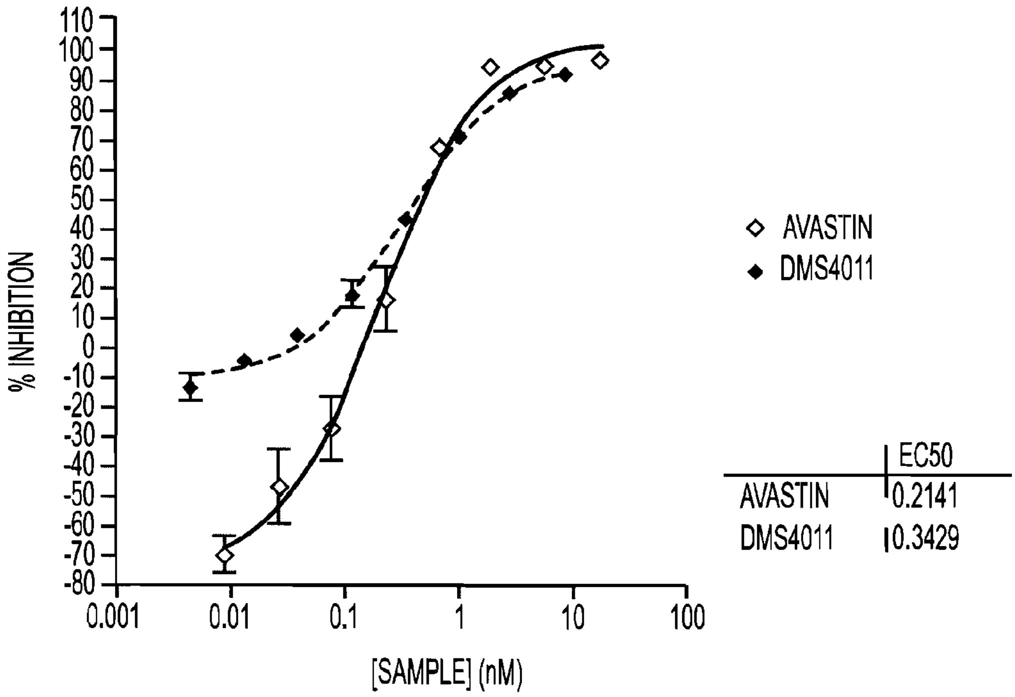


*FIG. 136*

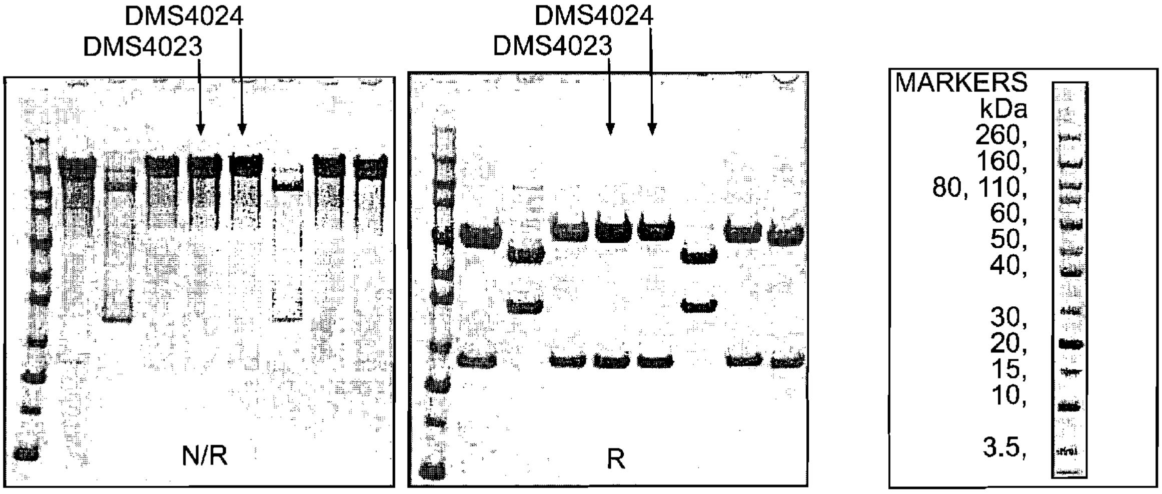


N/R=NON-REDUCED; R=REDUCED

FIG. 137

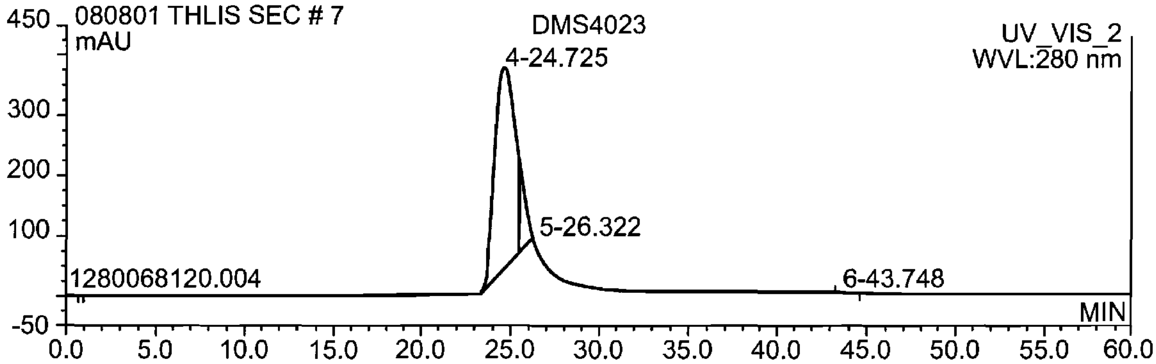
080801 THLIS SEC # 7
mAU
DMS4023
4-24.725
UV_VIS_2
WVL:280 nm
5-26.322
1280068120.004
6-43.748
MIN
450
300
200
100
-50
0.0
5.0
10.0
15.0
20.0
25.0
30.0
35.0
40.0
45.0
50.0
55.0
60.0

*FIG. 138*

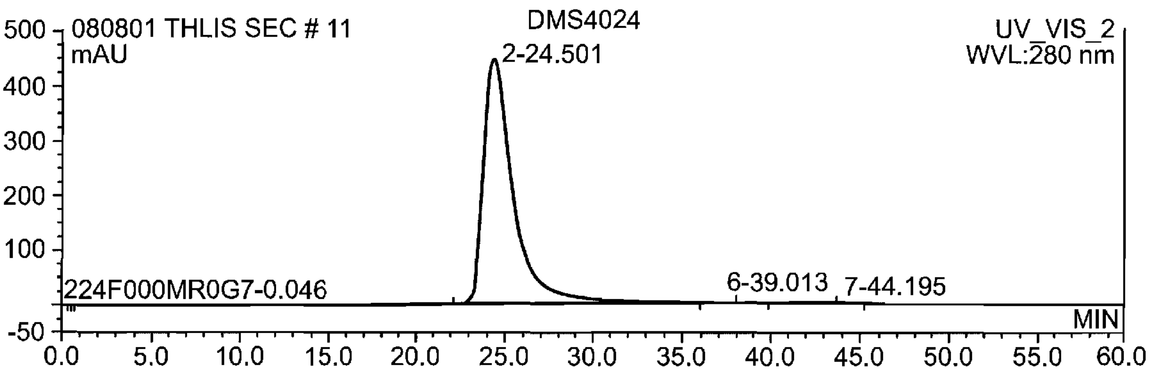
080801 THLIS SEC # 11
mAU
DMS4024
UV_VIS_2
WVL:280 nm
2-24.501
224F000MR0G7-0.046
6-39.013
7-44.195
MIN
500
400
300
200
100
-50
0.0
5.0
10.0
15.0
20.0
25.0
30.0
35.0
40.0
45.0
50.0
55.0
60.0

*FIG. 139*

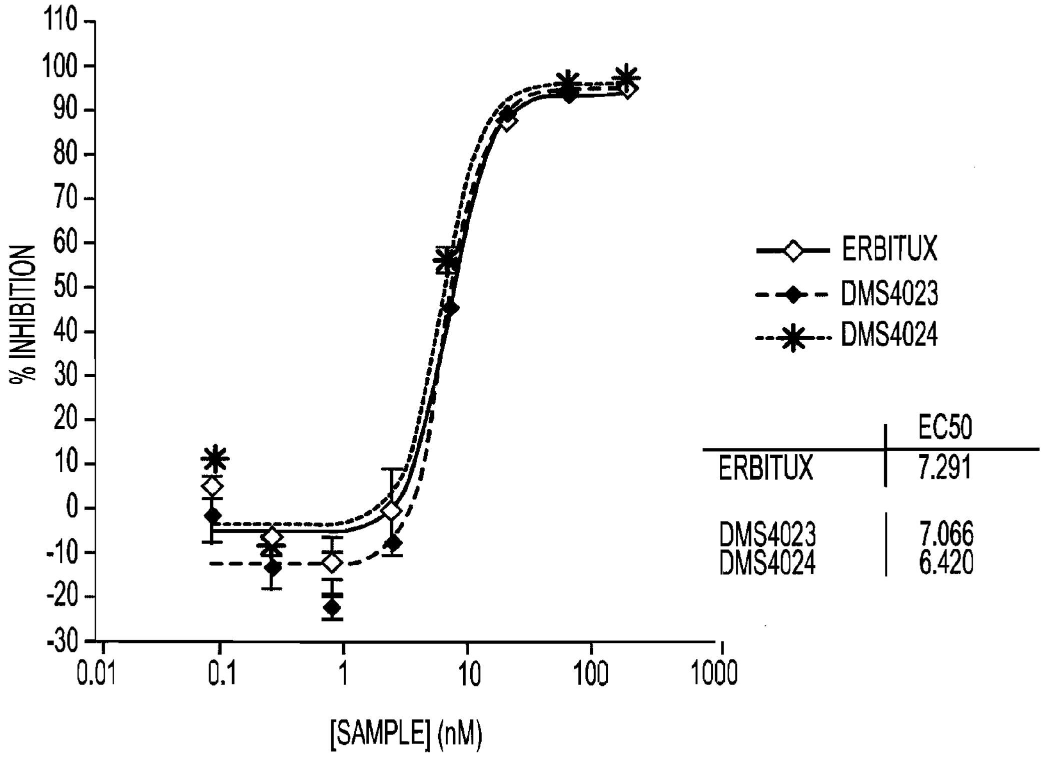


*FIG. 140*

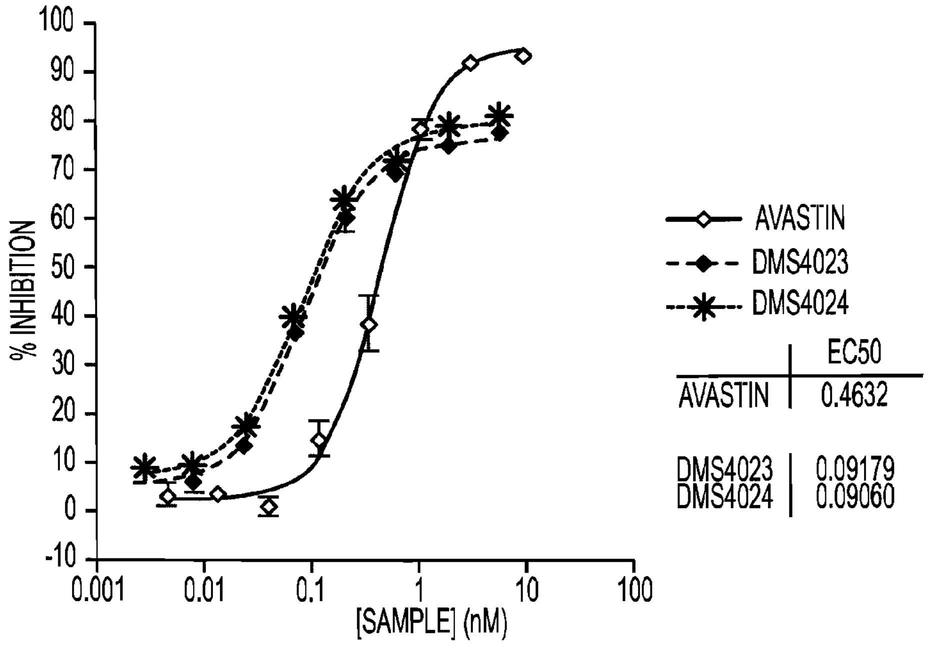


*FIG. 141*

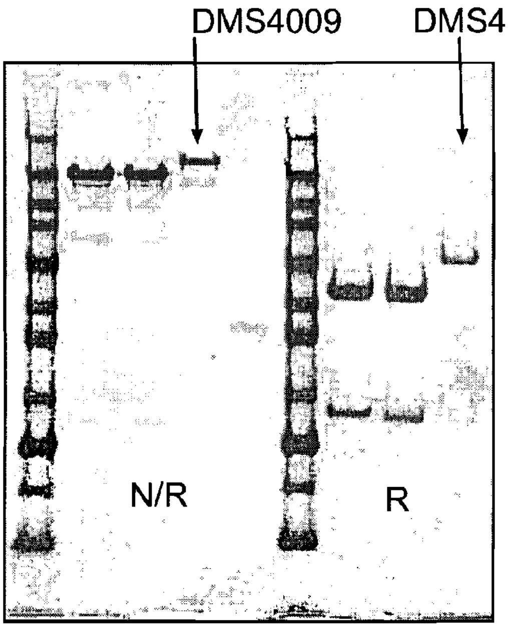


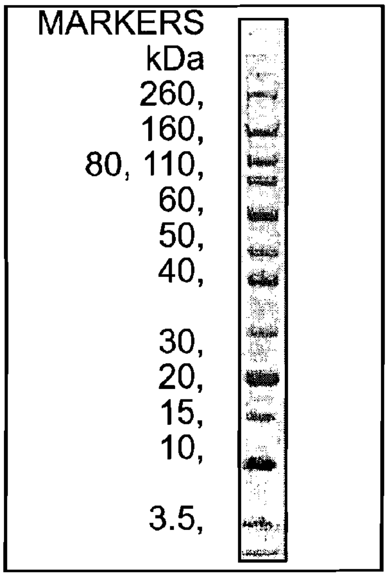


N/R=NON-REDUCED; R=REDUCED

FIG. 142

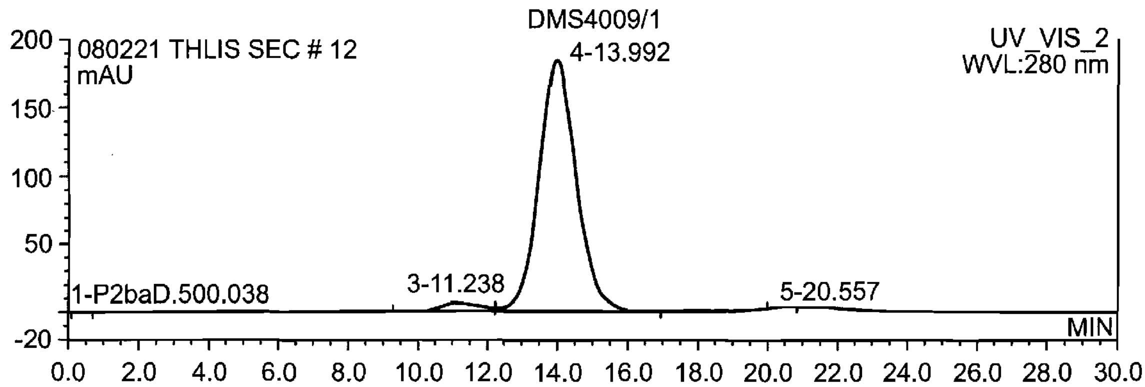
DMS4009/1
080221 THLIS SEC # 12
mAU
4-13.992
UV_VIS_2
WVL:280 nm
200
150
100
50
-20
1-P2baD.500.038
3-11.238
5-20.557
MIN
0.0 2.0 4.0 6.0 8.0 10.0 12.0 14.0 16.0 18.0 20.0 22.0 24.0 26.0 28.0 30.0

*FIG. 143*

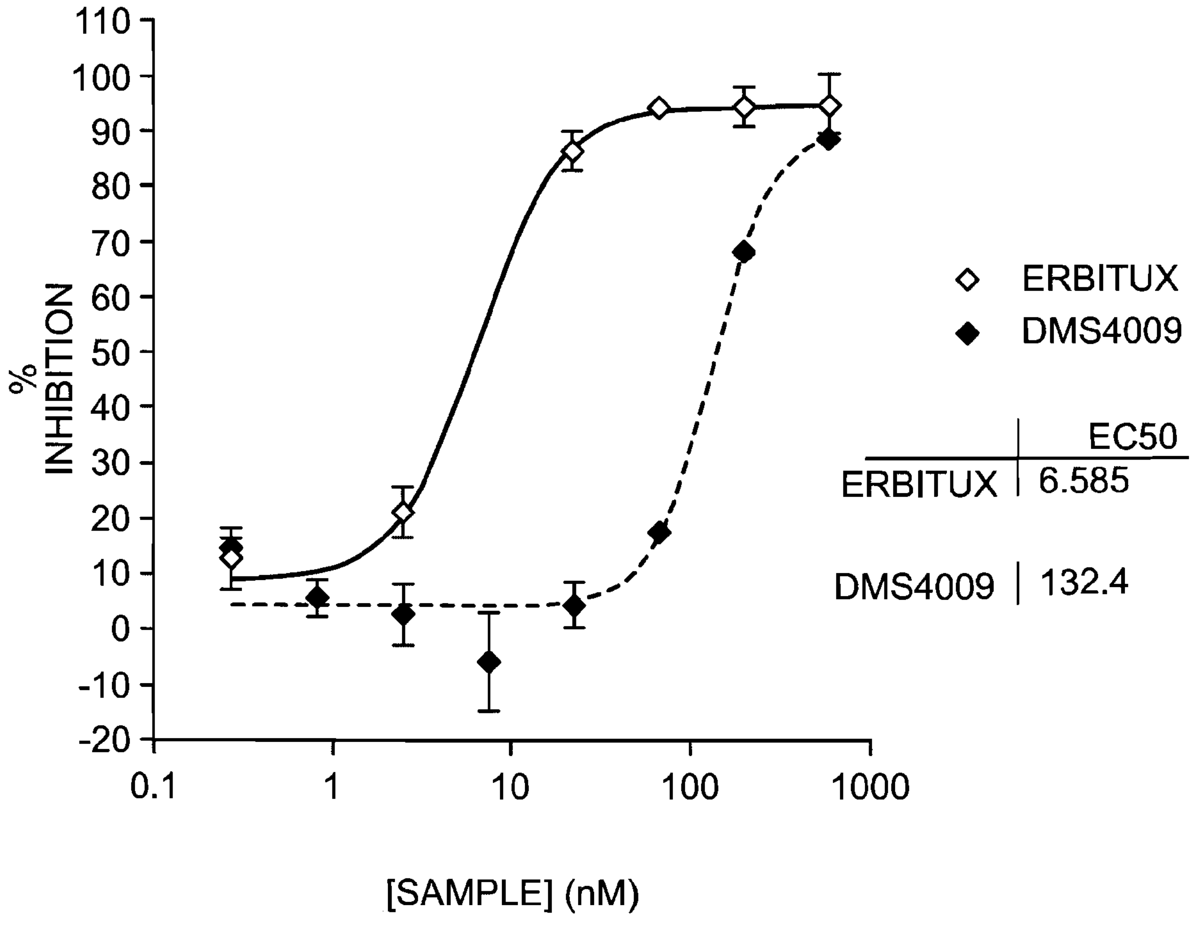
110
100
90
80
70
60
50
40
30
20
10
0
-10
-20
% INHIBITION
0.1
1
10
100
1000
[SAMPLE] (nM)
ERBITUX
DMS4009
EC50
ERBITUX 6.585
DMS4009 132.4

*FIG. 144*

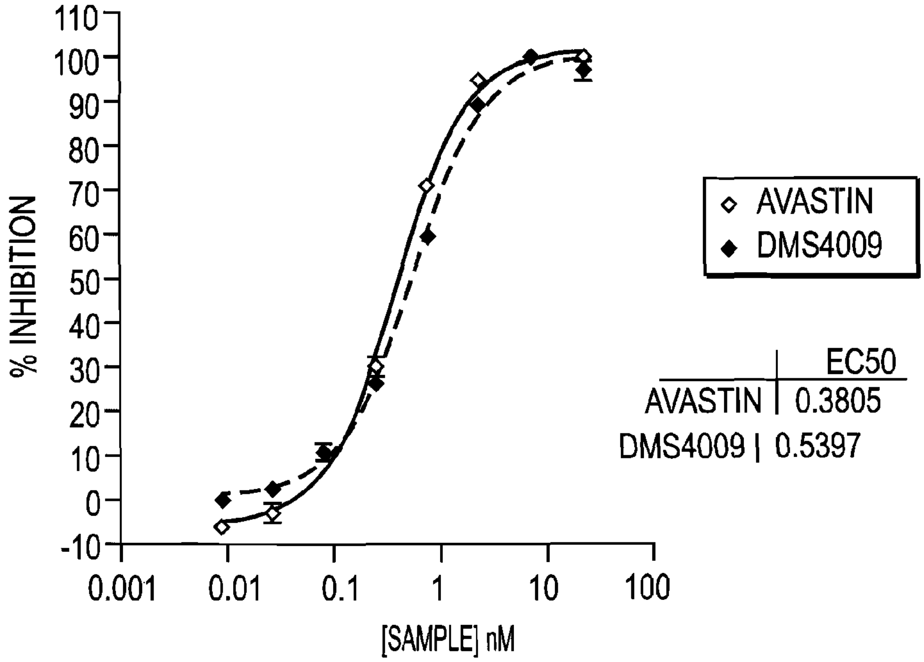


FIG. 145

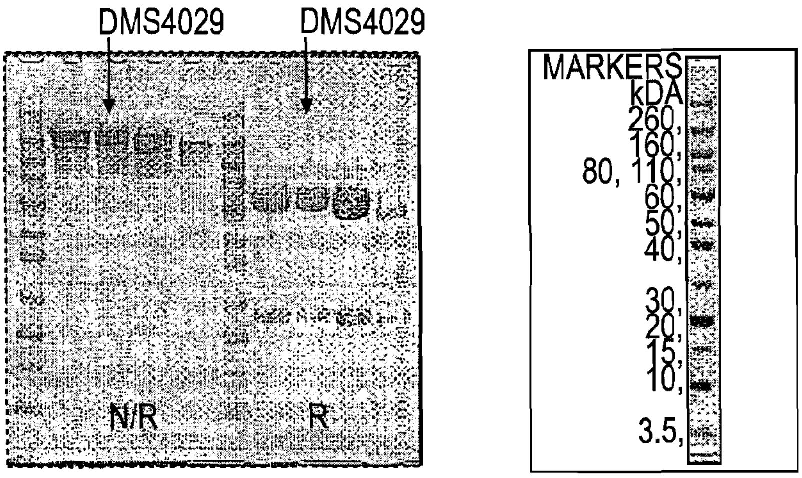


N/R=NON-REDUCED; R=REDUCED

FIG. 146

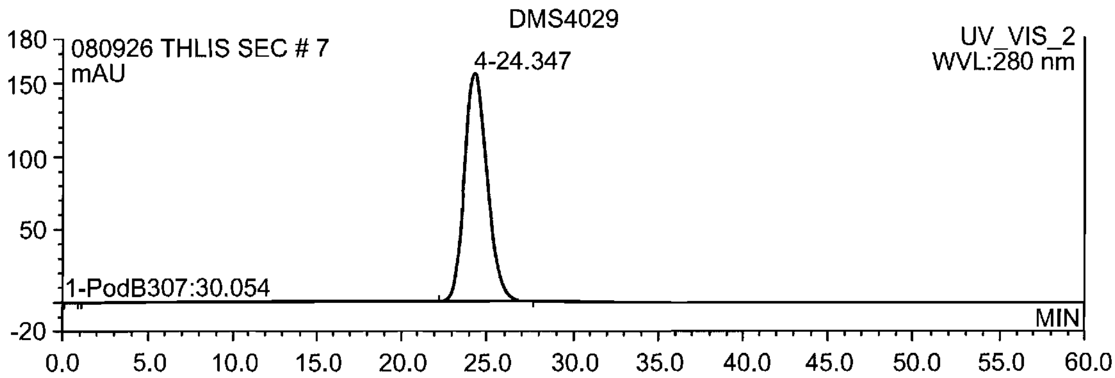
DMS4029
080926 THLIS SEC # 7
mAU
UV_VIS_2
WVL:280 nm
4-24.347
1-PodB307:30.054
MIN
180
150
100
50
-20
0.0
5.0
10.0
15.0
20.0
25.0
30.0
35.0
40.0
45.0
50.0
55.0
60.0

*FIG. 147*

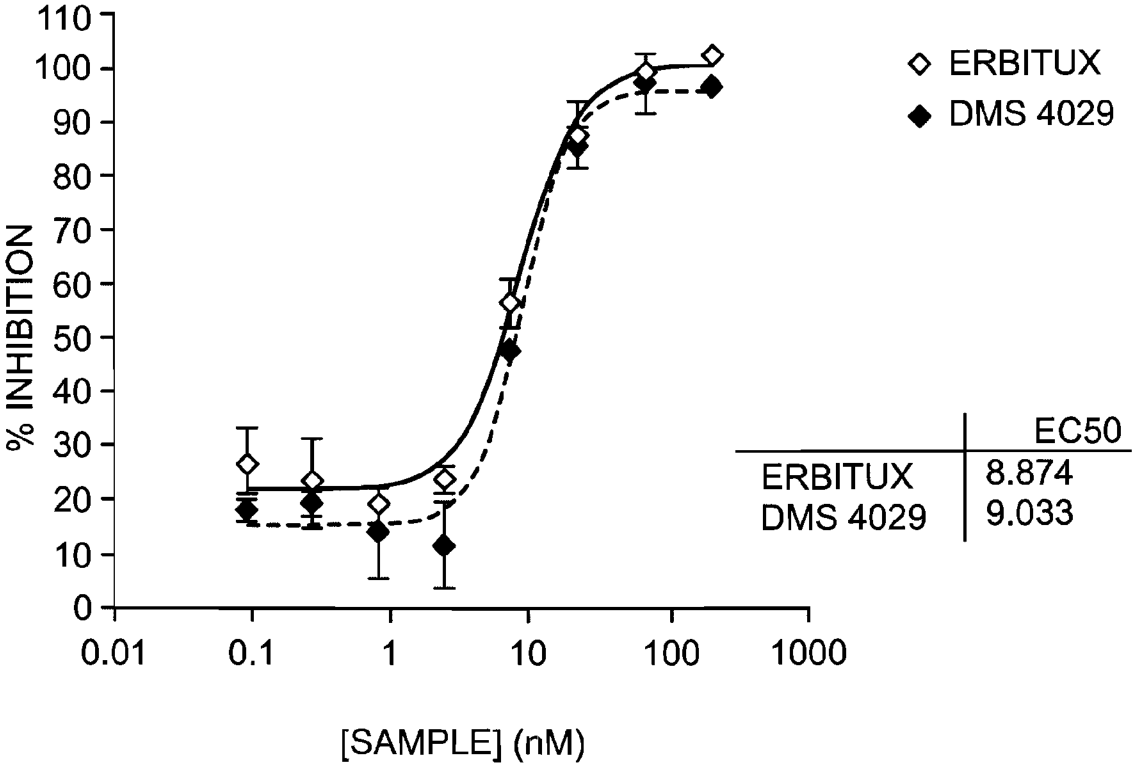
110
100
90
80
70
60
50
40
30
20
10
0
% INHIBITION
0.01
0.1
1
10
100
1000
[SAMPLE] (nM)
ERBITUX
DMS 4029
EC50
ERBITUX 8.874
DMS 4029 9.033

*FIG. 148*

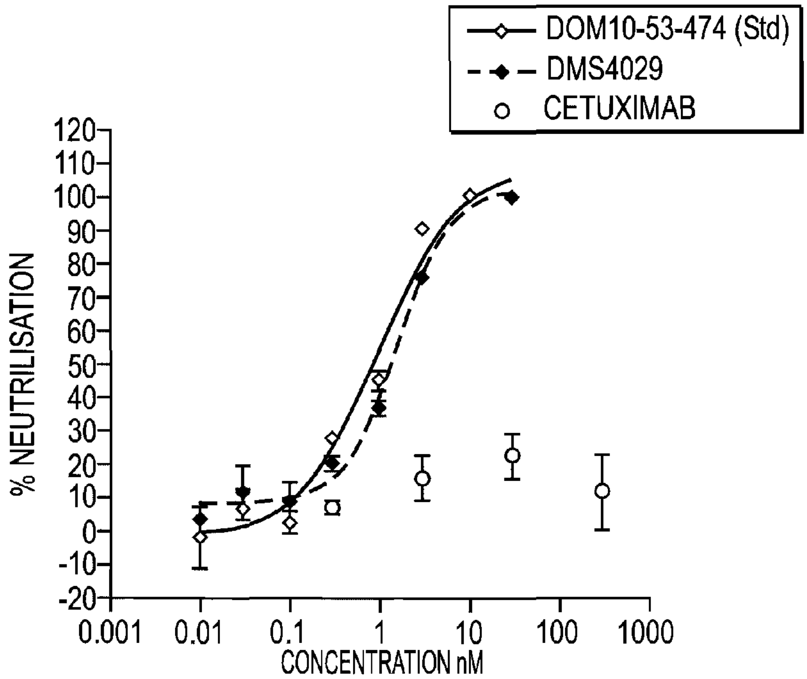


| | DOM10-53-474 (Std) | DMS4029 |
|---|---|---|
| EC50 | 0.9960 | 1.654 |

*FIG. 149*

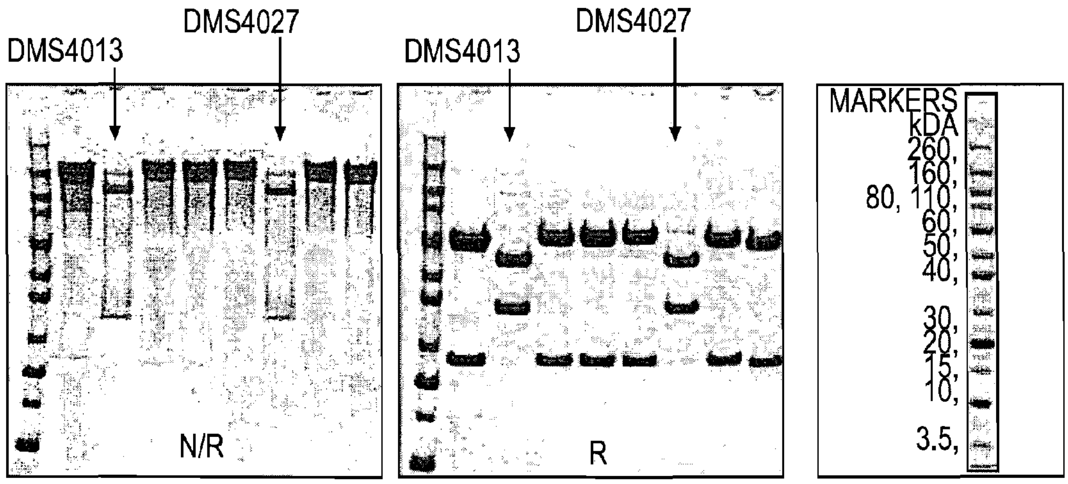


N/R=NON-REDUCED; R=REDUCED

*FIG. 150*

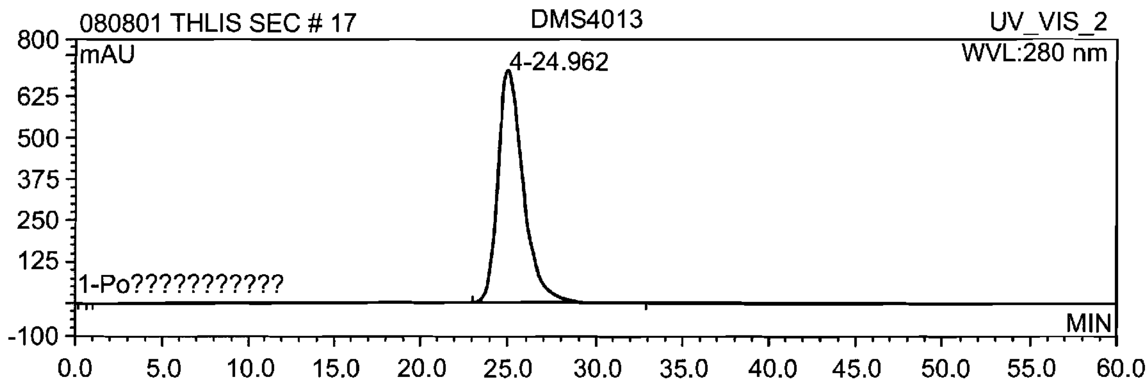

080801 THLIS SEC # 17
DMS4013
UV_VIS_2
WVL:280 nm
mAU
4-24.962
1-Po???????????
MIN
800
625
500
375
250
125
-100
0.0
5.0
10.0
15.0
20.0
25.0
30.0
35.0
40.0
45.0
50.0
55.0
60.0


FIG. 151

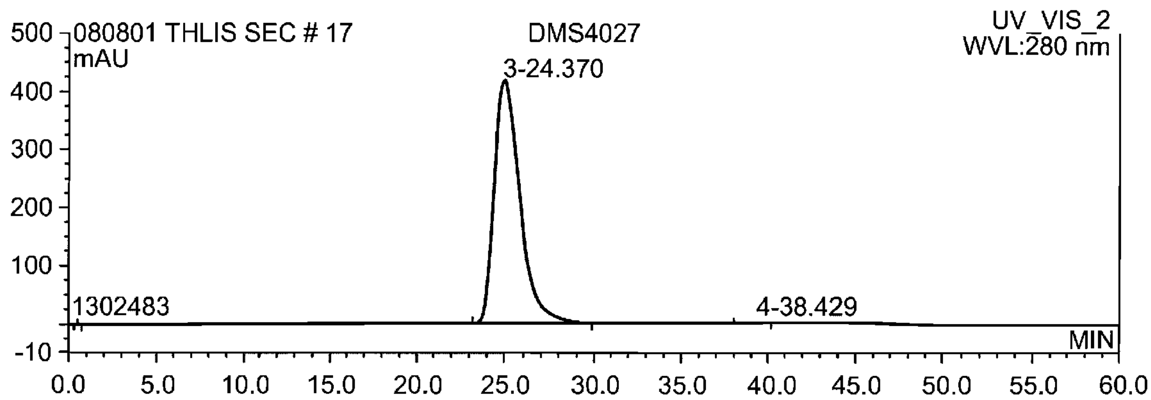

080801 THLIS SEC # 17
DMS4027
UV_VIS_2
WVL:280 nm
mAU
3-24.370
1302483
4-38.429
MIN
500
400
300
200
100
-10
0.0
5.0
10.0
15.0
20.0
25.0
30.0
35.0
40.0
45.0
50.0
55.0
60.0


*FIG. 152*

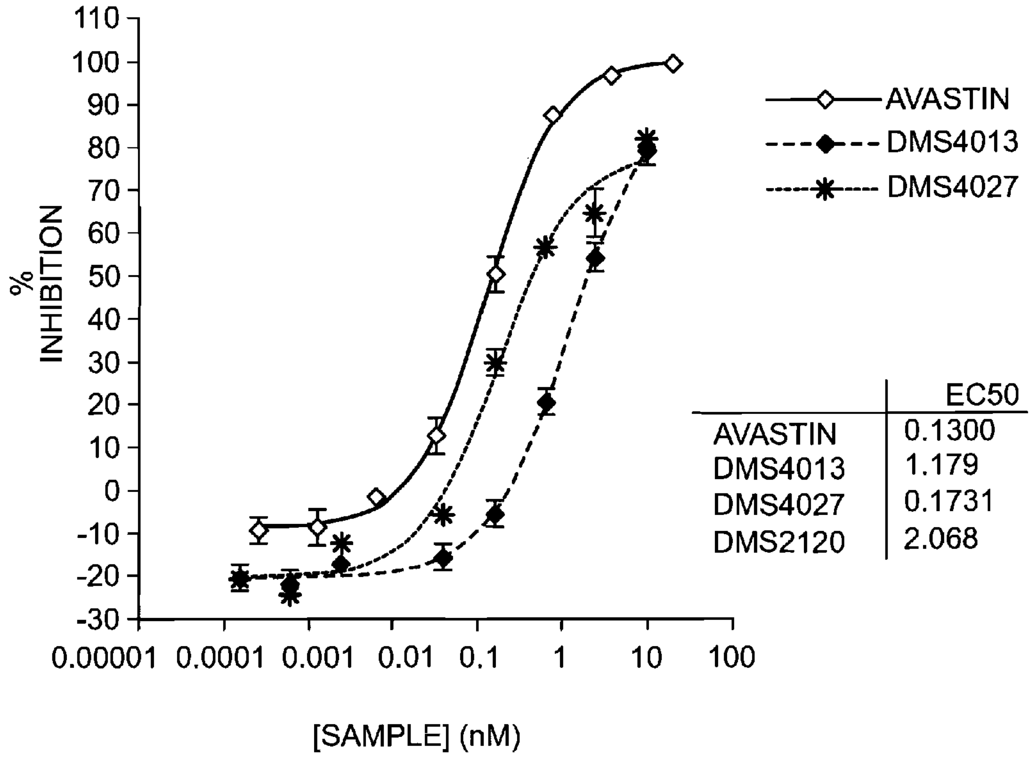
% INHIBITION
110
100
90
80
70
60
50
40
30
20
10
0
-10
-20
-30
0.00001
0.0001
0.001
0.01
0.1
1
10
100
[SAMPLE] (nM)
AVASTIN
DMS4013
DMS4027
EC50
AVASTIN 0.1300
DMS4013 1.179
DMS4027 0.1731
DMS2120 2.068

*FIG. 153*

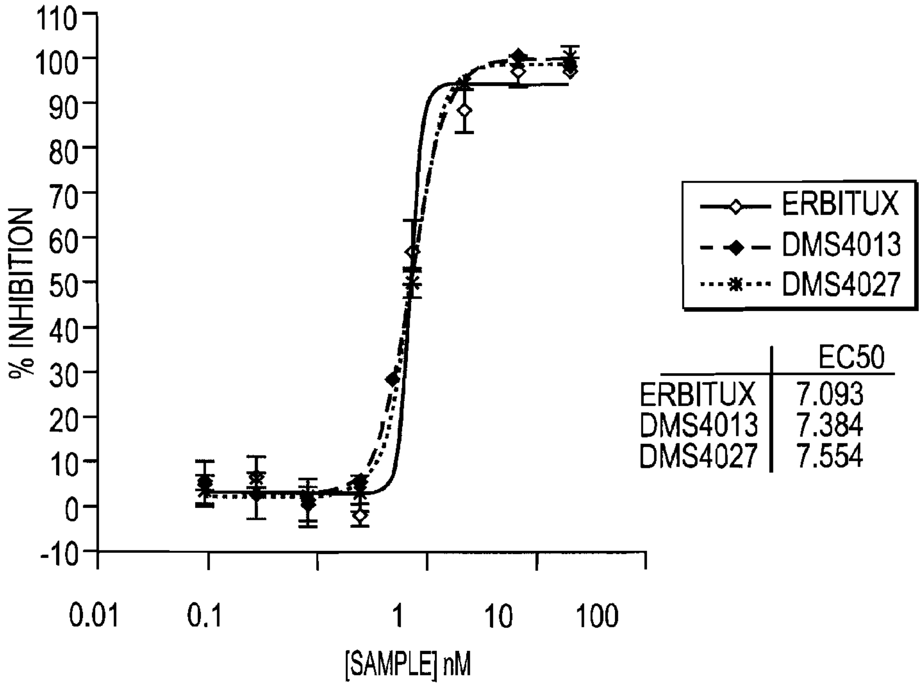


*FIG. 154*

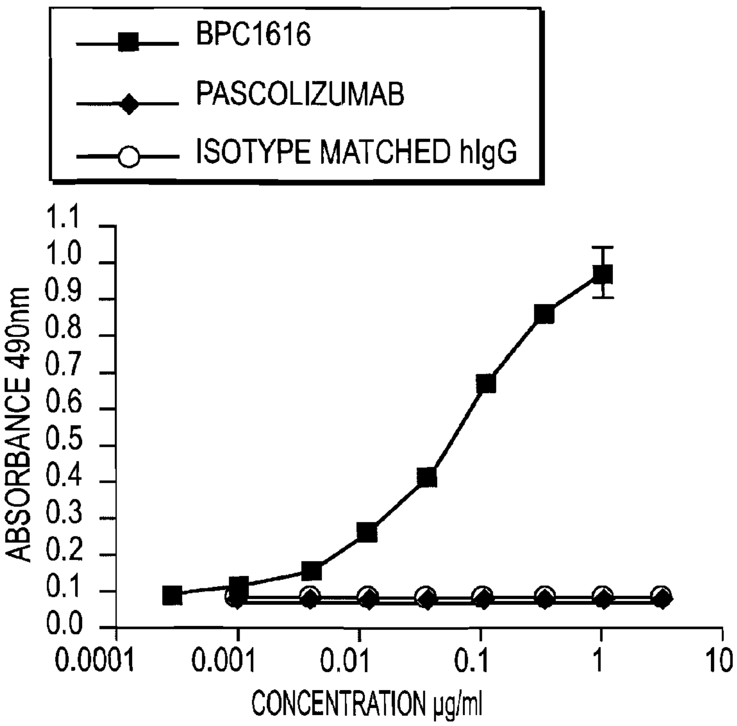


*FIG. 155*

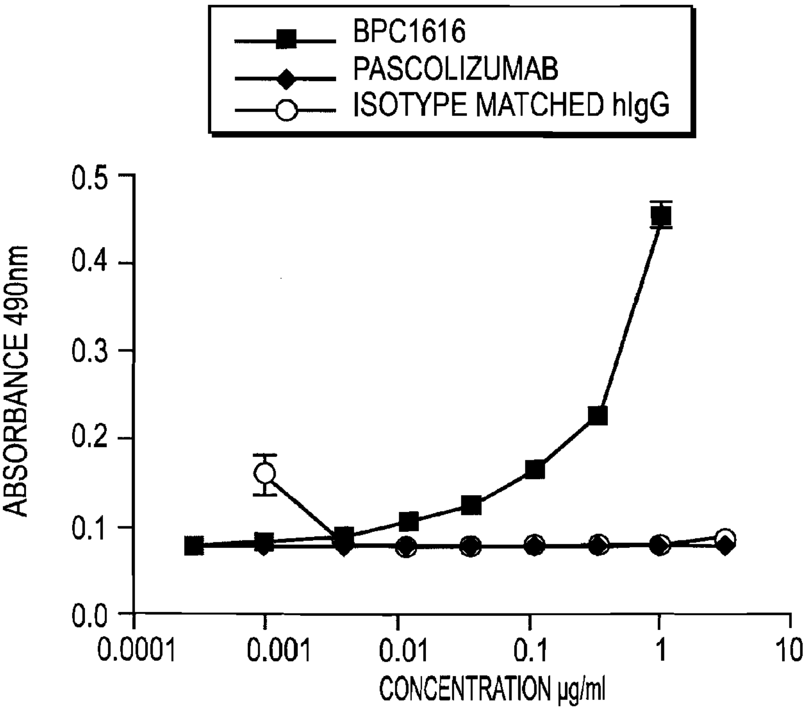


*FIG. 156*

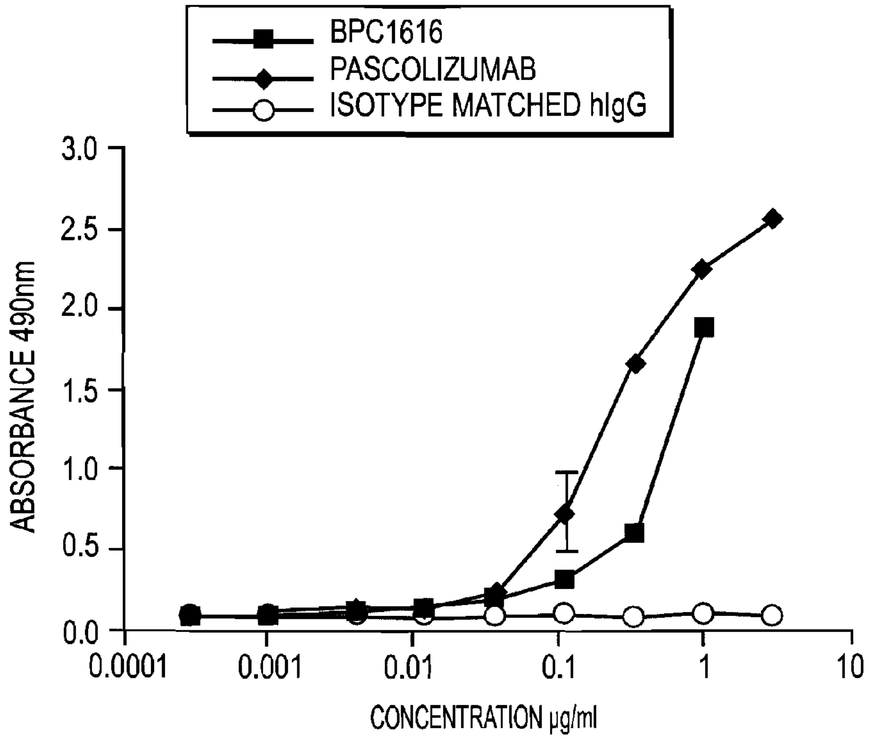


*FIG. 157*

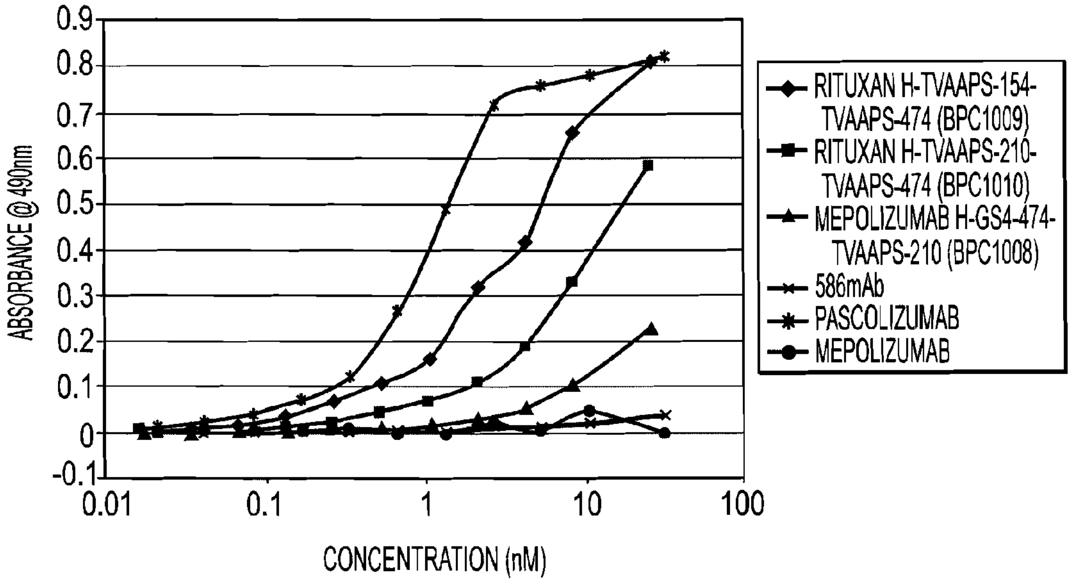


*FIG. 158*

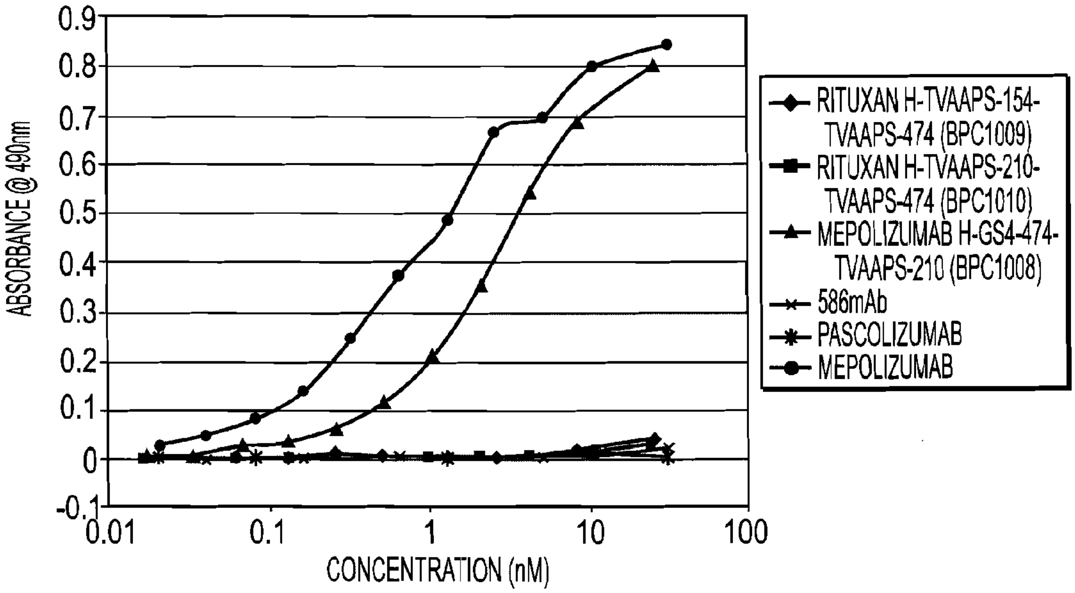


*FIG. 159*

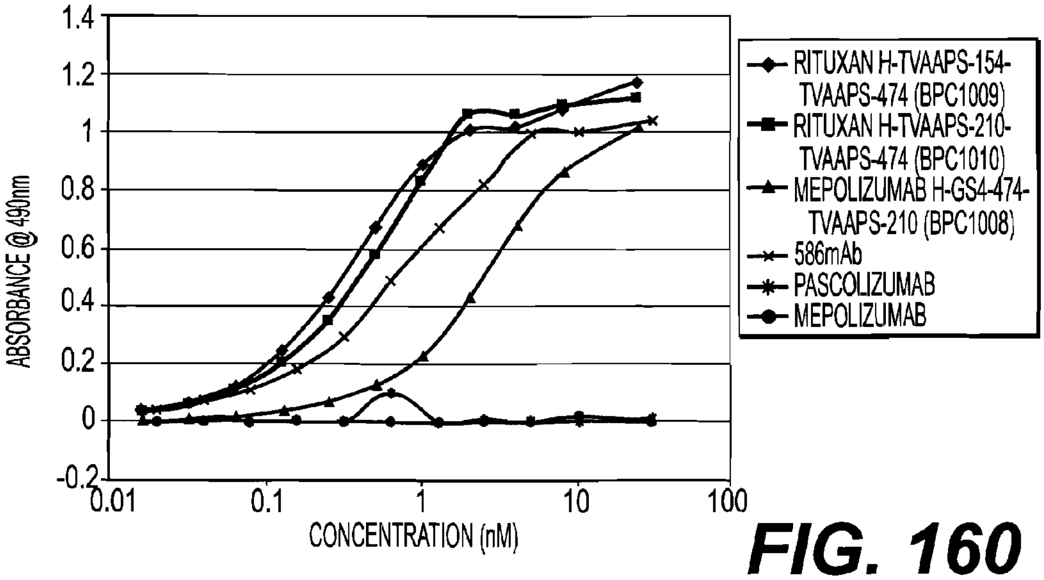


FIG. 160

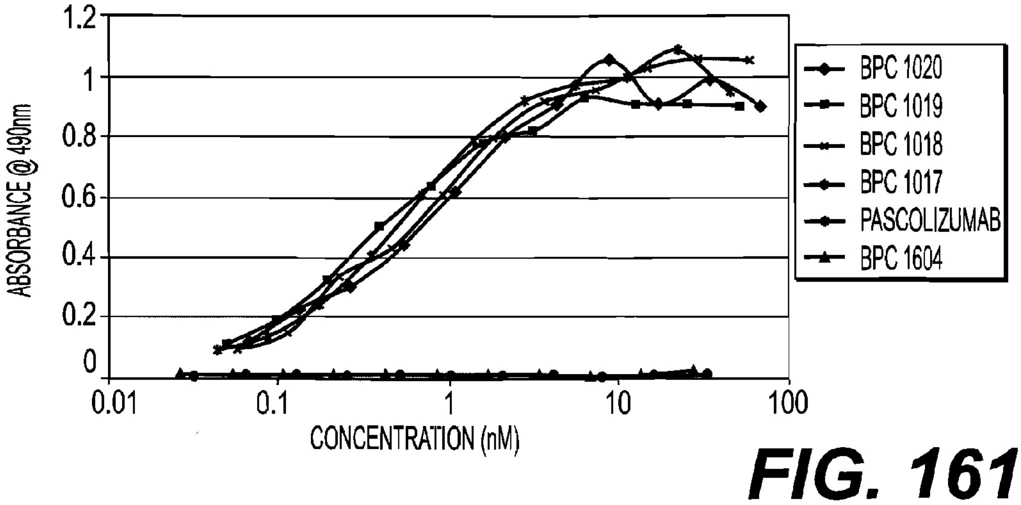


FIG. 161

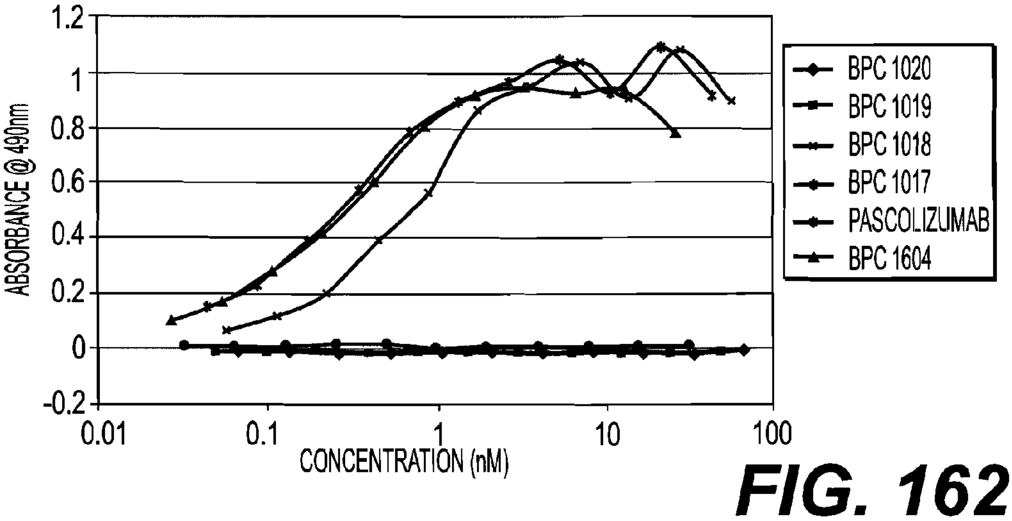


FIG. 162

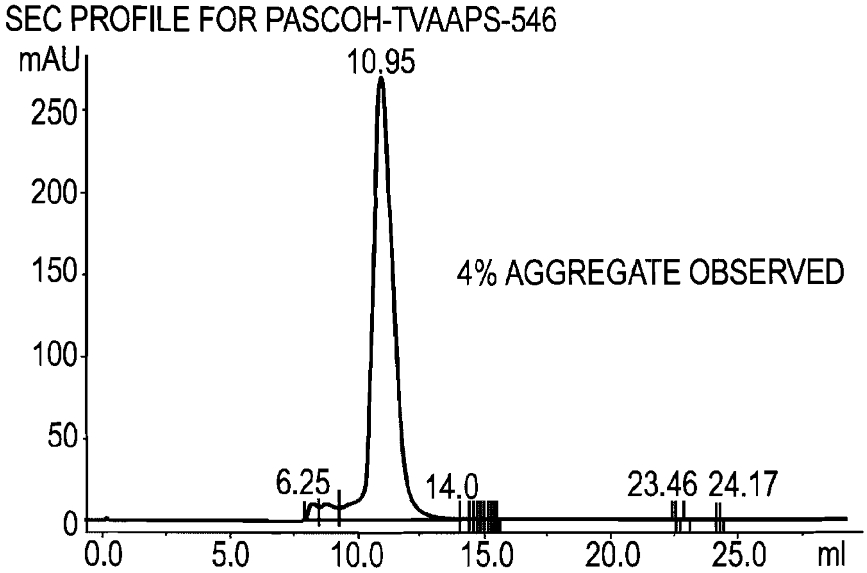


*FIG. 163*

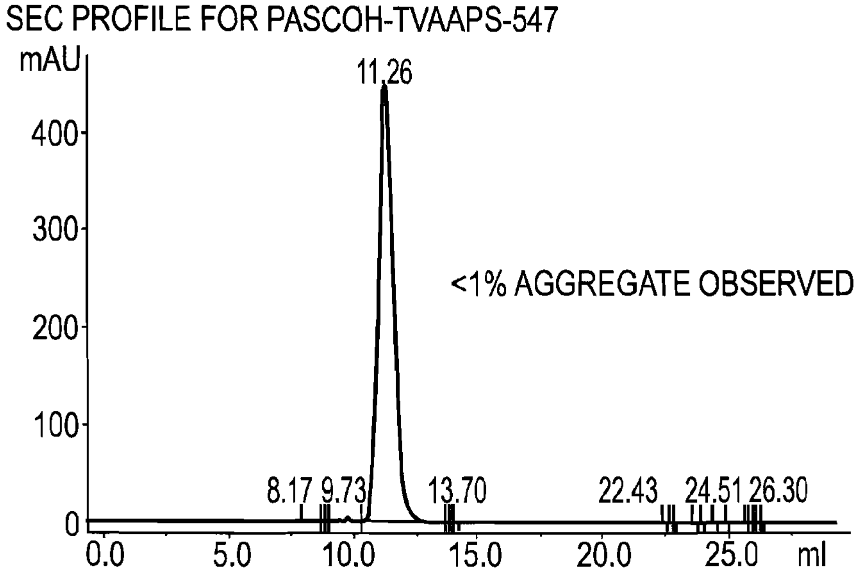


*FIG. 164*

SDS PAGE FOR PASCOH-TVAAPS-546 [A= NON-REDUCING CONDITIONS, B = REDUCING CONDITIONS]

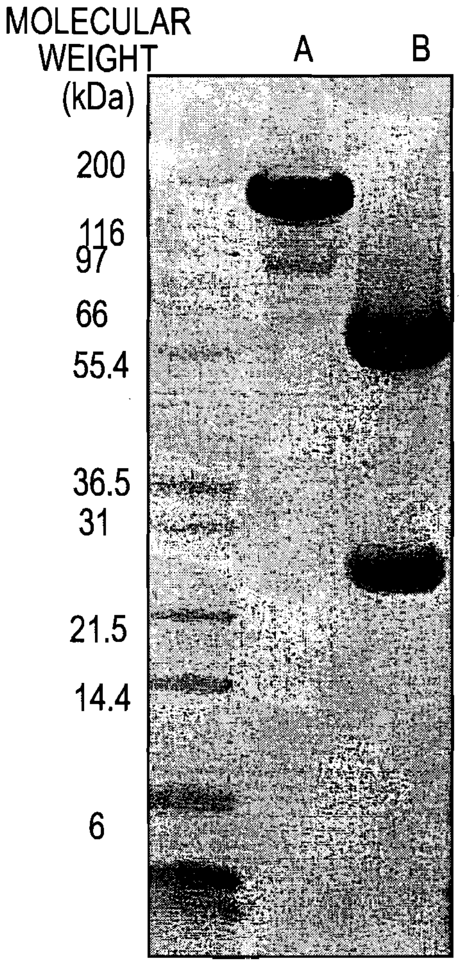


FIG. 165

SDS PAGE FRO PASCOH-TVAAPS-567 [A= NON-REDUCING CONDITIONS, B = REDUCING CONDITIONS]

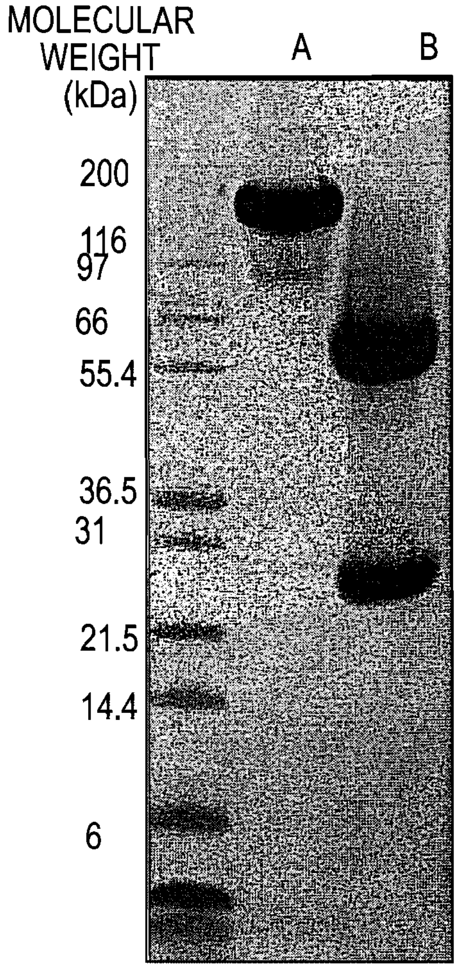


*FIG. 166*

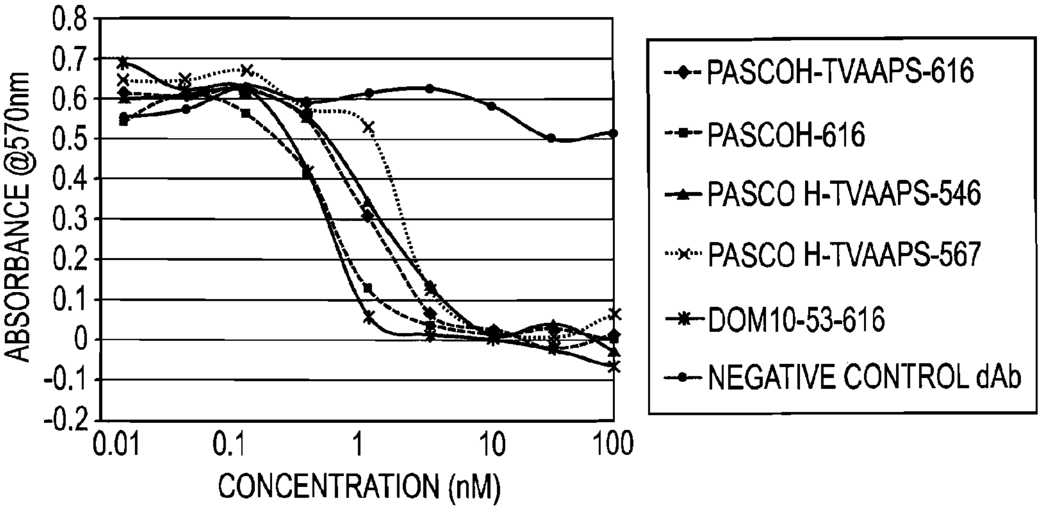


*FIG. 167*

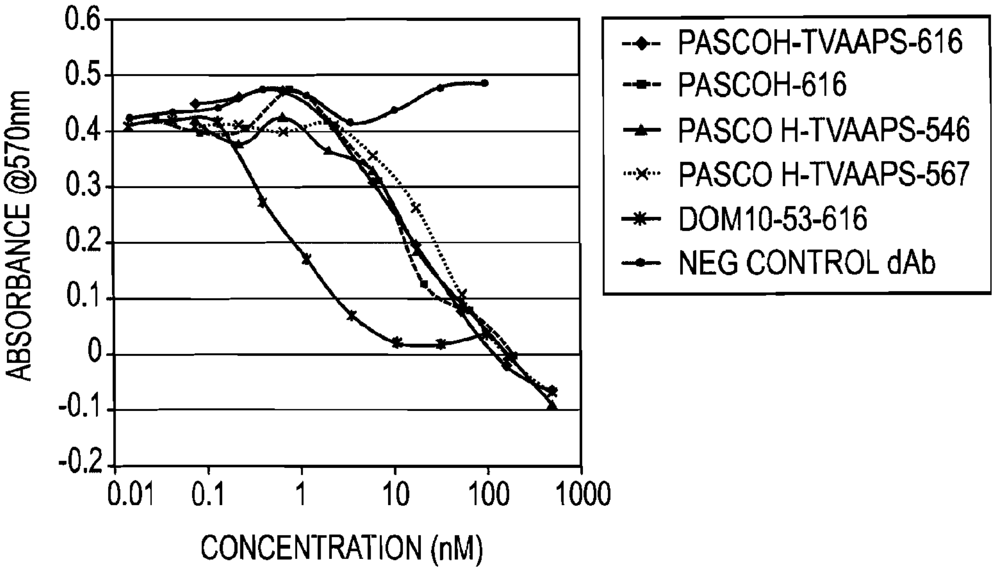


*FIG. 168*

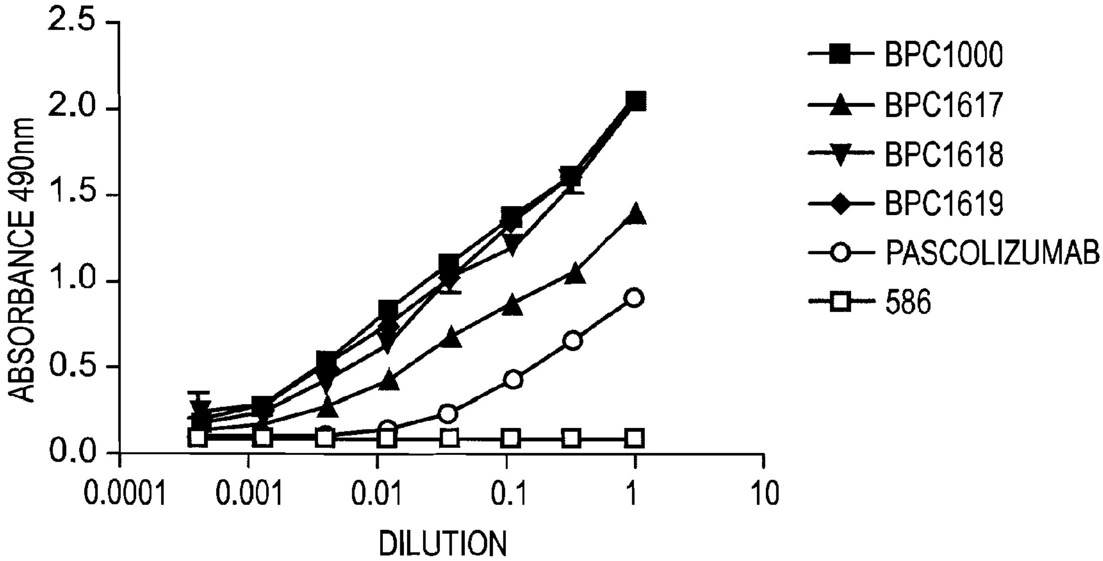


FIG. 169

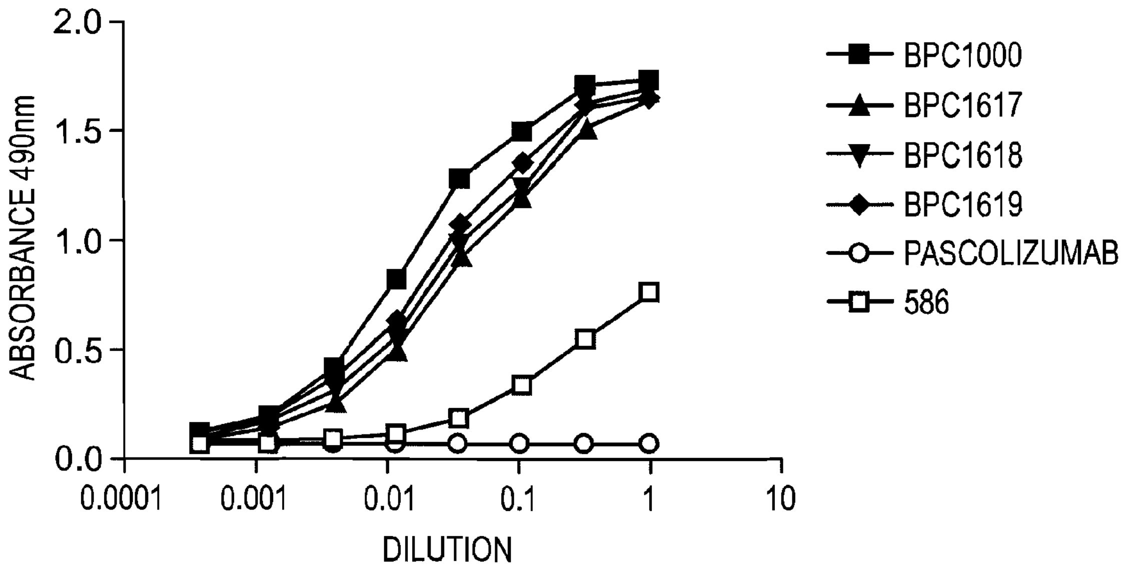


FIG. 170

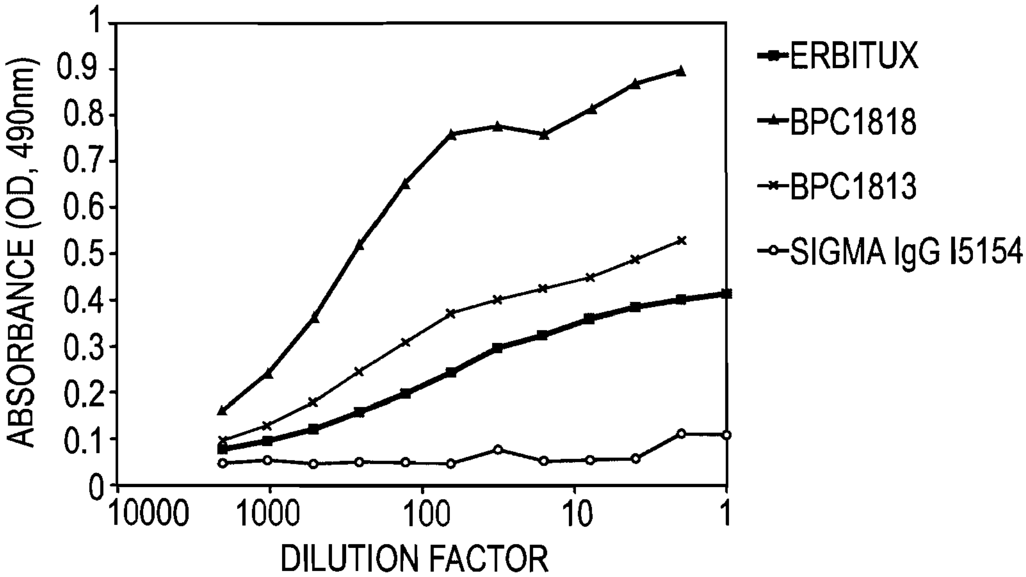


*FIG. 171*

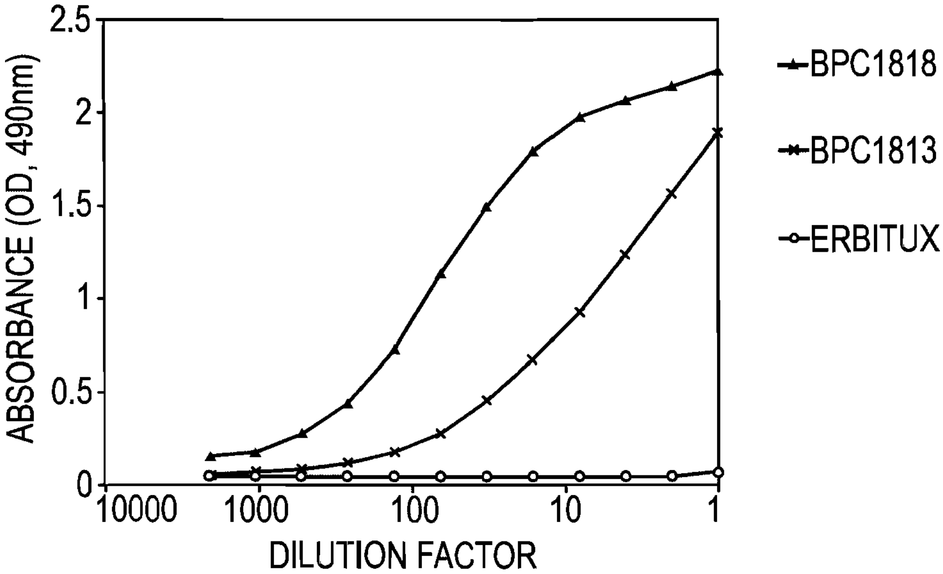


*FIG. 172*

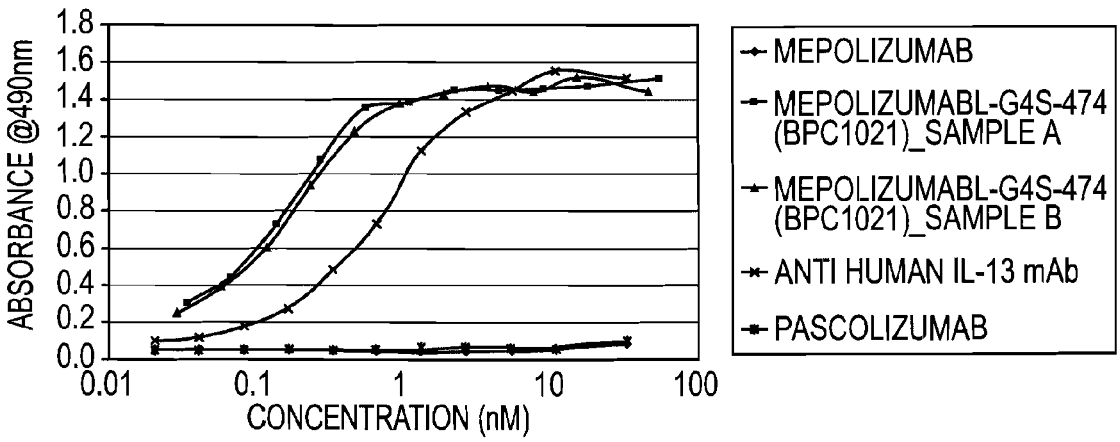


*FIG. 173*

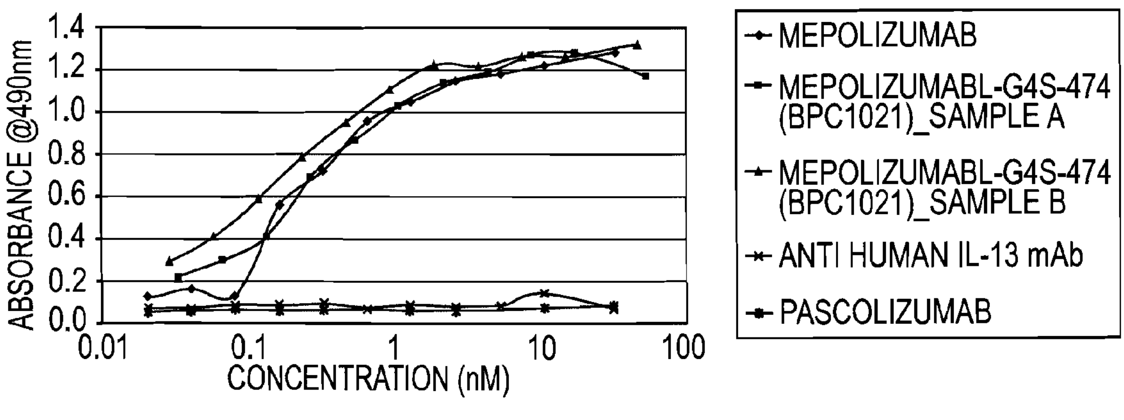


*FIG. 174*

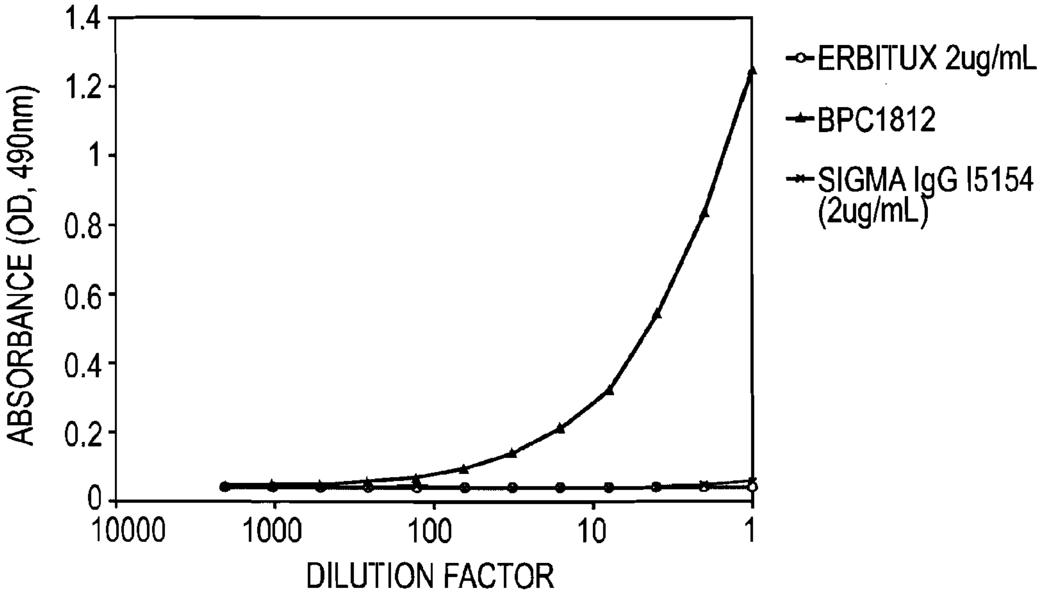


FIG. 175

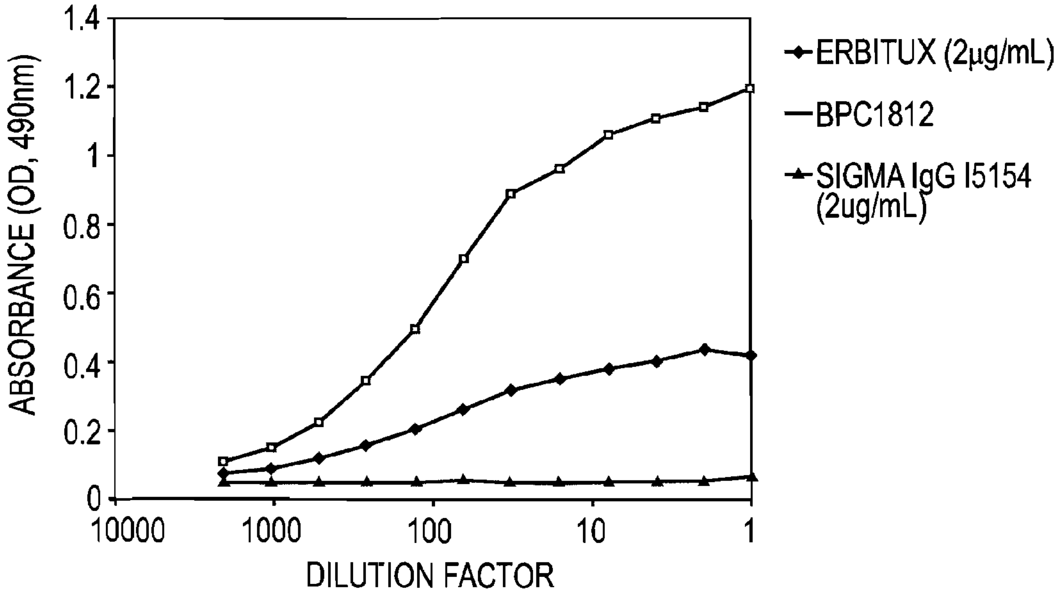


FIG. 176

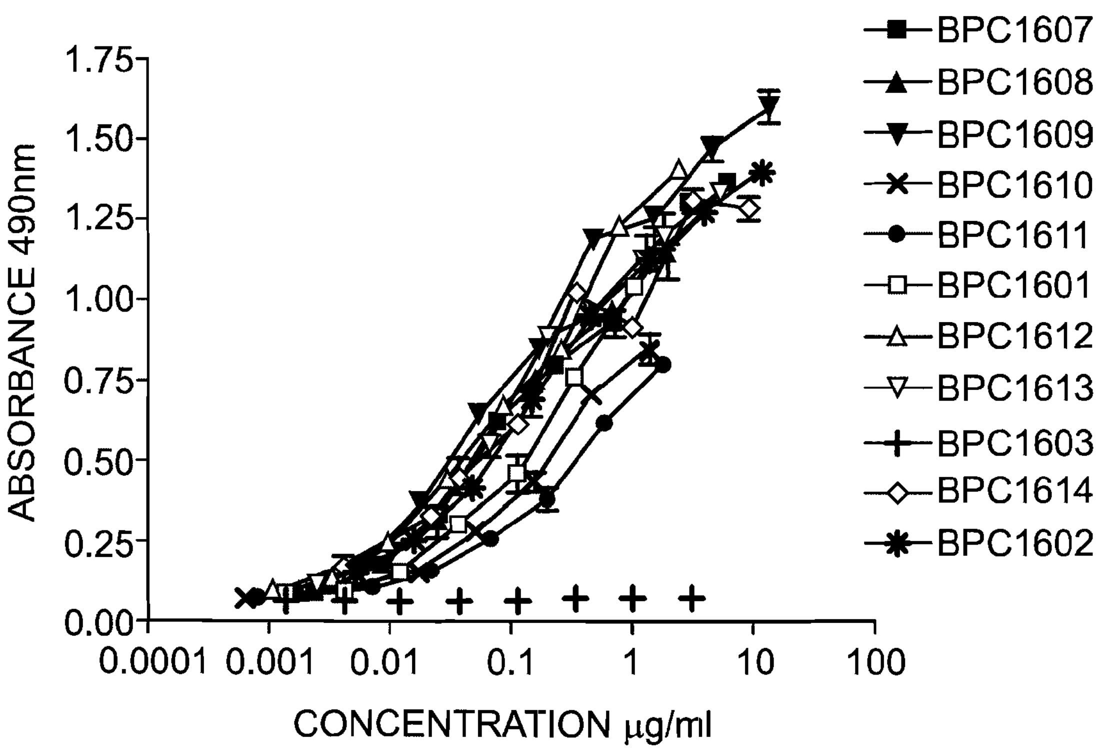
BPC1607
BPC1608
BPC1609
BPC1610
BPC1611
BPC1601
BPC1612
BPC1613
BPC1603
BPC1614
BPC1602
ABSORBANCE 490nm
1.75
1.50
1.25
1.00
0.75
0.50
0.25
0.00
0.0001
0.001
0.01
0.1
1
10
100
CONCENTRATION μg/ml

*FIG. 177*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9308829 A
- WO 9404690 A
- WO 2007095338 A
- WO 0029004 A
- US 20050043519 A
- US 7250297 B1
- US 20070224633 A
- EP 1641818 A1
- US 20040132028 A1
- US 20080139791 A
- WO 2005056764 A
- US 6818418 B1
- WO 2008098796 A
- EP 0307434 A
- WO 02092771 A
- WO 2005081854 A
- WO 2007059782 A
- WO 2006015371 A
- WO 9958679 A
- WO 9616990 A
- US 4873316 A
- US 6555313 B1, Griffiths
- US 5733743 A, Johnson
- US 5969108 A, McCafferty
- US 5702892 A, Mulligan-Kehoe
- WO 9920749 A, Tomlinson
- WO 0157065 A
- WO 9958655 A
- WO 9708320 A, Morphosys
- WO 9902671 A
- WO 0040712 A
- US 6297053 B
- WO 2005003156 A1
- WO 2007024715 A


### Non-patent literature cited in the description


- **MILLSTEIN et al.** *Nature,* 1983, vol. 305, 537-539
- **TRAUNECKER et al.** *EMBO,* 1991, vol. 10, 3655-3659
- **SURESH et al.** *Methods in Enzymology,* 1986, vol. 121, 210
- *Mol. Immunol.,* 2006, vol. 44, 656-665
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- Non-Antibody Scaffolds from Handbook of Therapeutic Antibodies. Protein Science. 2006, vol. 15, 14-27
- **REITER et al.** *J Mol Biol,* 1999, vol. 290, 685-698
- **EWERT et al.** *J Mol Biol,* 2003, vol. 325, 531-553
- **JESPERS et al.** *J Mol Biol,* 2004, vol. 337, 893-903
- **JESPERS et al.** *Nat Biotechnol,* 2004, vol. 22, 1161-1165
- **MARTIN et al.** *Protein Eng.,* 1997, vol. 10, 607-614
- **SEPULVADA et al.** *J Mol Biol,* 2003, vol. 333, 355-365
- **EPP et al.** *Biochemistry,* 1975, vol. 14, 4943-4952
- **HUAN et al.** *Biochemistry,* 1994, vol. 33, 14848-14857
- **HUANG et al.** *Mol immunol,* 1997, vol. 34, 1291-1301
- **JAMES et al.** *J Mol Biol.,* 2007, vol. 367, 603-8
- **PLÜCKTHUN, A.** *Immunol. Rev.,* 1992, vol. 130, 151-188
- **MILLER et al.** Genetic Engineering. Plenum Press, 1986, vol. 8, 277-298
- **LASMAR U ; PARKINS D.** The formulation of Biopharmaceutical products. *Pharma. Sci.Tech.today,* 03 April 2000, vol. 3, 129-137
- **WANG, W.** Instability, stabilisation and formulation of liquid protein pharmaceuticals. *Int. J. Pharm,* 1999, vol. 185, 129-188

- Stability of Protein Pharmaceuticals Part A and B. Plenum Press, 1992
- **AKERS,M.J.** Excipient-Drug interactions in Parenteral Formulations. *J.Pharm Sci,* 2002, vol. 91, 2283-2300
- **IMAMURA, K et al.** Effects of types of sugar on stabilization of Protein in the dried state. *J Pharm Sci,* 2003, vol. 92, 266-274
- **IZUTSU, KKOJIMA, S.** Excipient crystalinity and its protein-structure-stabilizing effect during freeze-drying. *J Pharm. Pharmacol,* 2002, vol. 54, 1033-1039
- **JOHNSON, R.** Mannitol-sucrose mixtures-versatile formulations for protein lyophilization. *J. Pharm. Sci,* 2002, vol. 91, 914-922
- **HA,E ; WANG W ; WANG Y.J.** Peroxide formation in polysorbate 80 and protein stability. *J. Pharm Sci,* 2002, vol. 91, 2252-2264
- **KABAT.** Sequences of Proteins of Immunological Interest. US Department of Health and Human Services
- **CHOTHIA ; LESK.** *J. Mol. Biol.,* 1987, vol. 196, 910-917
- **TATUSOVA, T. A. et al.** *FEMS Microbiol Lett,* 1999, vol. 174, 187-188
- **WINTER, G. et al.** *Annu. Rev. Immunol.,* 1994, vol. 12, 433-455
- **SOUMILLION, P. et al.** *Appl. Biochem. Biotechnol.,* 1994, vol. 47 (2-3), 175-189
- **CASTAGNOLI, L. et al.** *Comb. Chem. High Throughput Screen,* 2001, vol. 4 (2), 121-133
- **DENG et al.** *J. Biol. Chem.,* 1994, vol. 269, 9533
- **LOW et al.** *J. Mol. Biol.,* 1996, vol. 260, 359
- **CRAMERI et al.** *Nature Med.,* 1996, vol. 2, 100
- **RIECHMANN et al.** *Bio/Technology,* 1995, vol. 13, 475
- **LANDT, O. et al.** *Gene,* 1990, vol. 96, 125-128
- **HORTON, R.M. et al.** *Gene,* 1989, vol. 77, 61-68
- **GOLDMAN, L.A. et al.** *Biotechniques,* 1996, vol. 21, 1013-1015
- **MIZUSHIMA, S. et al.** *Nucleic Acids Res.,* 1990, vol. 18, 5322
- **TAWFIK ; GRIFFITHS.** *Nature Biotechnol,* 1998, vol. 16 (7), 652-6
- **WINTER et al.** *Ann. Rev. Immunology,* 1994, vol. 12, 433-55
- **TOMLINSON et al.** *J. Mol. Biol.,* 1992, vol. 227, 776
- **STEMMER.** *Nature,* 1994, vol. 370, 389-391
- **CHOTHIA ; LESK.** *J. Mol. Biol.,* 1987, vol. 196, 901
- **CHOTHIA et al.** *Nature,* 1989, vol. 342, 877
- **CHOTHIA et al.** *J. Mol. Biol.,* 1992, vol. 227, 799
- **TOMLINSON et al.** *EMBO J.,* 1995, vol. 14, 4628
- **WILLIAMS et al.** *J. Mol. Biol.,* 1996, vol. 264, 220
- **MARTIN et al.** *J. Mol. Biol.,* 1996, vol. 263, 800
- **SHIRAI et al.** *FEBS Letters,* 1996, vol. 399, 1
- **HOOD et al.** *Cold Spring Harbor Symp. Quant. Biol.,* 1967, vol. 48, 133
- **KABAT et al.** Sequences of proteins of immunological interest. U.S. Department of Health and Human Services, 1991
- **HAWKINS et al.** *J. Mol. Biol.,* 1992, vol. 226, 889
- **BARBAS et al.** *Proc. Natl. Acad. Sci. USA,* 1992, vol. 89, 4457
- **HOOGENBOOM ; WINTER.** *J. Mol. Biol.,* 1992, vol. 227, 381
- **NISSIM et al.** *EMBO J.,* 1994, vol. 13, 692
- **GRIFFITHS et al.** *EMBO J.,* 1994, vol. 13, 3245
- **DE KRUIF et al.** *J. Mol. Biol.,* 1995, vol. 248, 97
- **TOMLINSON et al.** *J. Mol. Biol.,* 1996, vol. 256, 813
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- **ZIMM.** *J. Chem. Phys.,* 1948, vol. 16, 1093-1099
- **BOYD et al.** *J. Imm. Meth.,* 1995, vol. 184, 29-38
- **ZAHND, C. et al.** *J. Mol. Biol.,* 2007, vol. 369, 1015-1028